# EUROPEAN PATENT APPLICATION

(11) **EP 1 438 973 A1**
(43) Date of publication of application: **21.07.2004**
(21) Application number: 02800776.3
(22) Date of filing: 04.10.2002
(51) Int. Cl.: A61K 45/00, A61K 31/166, A61K 31/167, A61K 31/277, A61K 31/381, A61K 31/4015, A61K 31/41, A61K 31/4427, A61K 31/47, A61K 31/517, A61K 31/519, A61P 1/00, A61P 1/02, A61P 3/00, A61P 3/04, A61P 3/10, A61P 11/00, A61P 11/06, A61P 15/00, A61P 15/10, A61P 15/12

(54) **REMEDIES FOR STRESS DISEASES COMPRISING MITOCHONDRIAL BENZODIAZEPINE RECEPTOR ANTAGONISTS**

(30) Priority: 05.10.2001 JP 2001310058
(71) Applicant: ONO PHARMACEUTICAL CO., LTD., Osaka-shi, Osaka 541-8526 (JP)
(72) Inventor: SEKO, Takuya; c/o Ono Pharmaceutical Co., Ltd., Osaka 541-8526 (JP); KATSUMATA, S.; c/o Minase Res. Institure, Shimamoto-cho, Mishima-gun, Osaka 618-8 (JP); KATO, M.; c/o Minase Res. Institute, Shimamoto-cho, Mishima-gun, Osaka 618-8 (JP); MANAKO, Jun-ichiro; c/o Minase Res. Institute, Shimamoto-cho, Mishima-gun, Osaka 618-8 (JP)
(74) Representative: Henkel, Feiler & Hänzel
(86) International application number: PCT/JP2002/010377
(87) International publication number: WO 2003/030937

(57) **Abstract**

A pharmaceutical composition for the prophylaxis and/or treatment of diseases induced, exacerbated or reignited by stressors comprising the compound of formula (I) 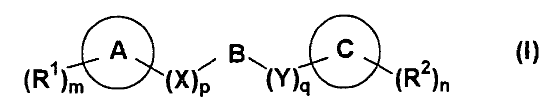 wherein all symbols have the same meanings as described in the specification, *etc*. as an active ingredient.

## Description

### Technical Field

The present invention relates to a pharmaceutical composition for the treatment of diseases induced, exacerbated or reignited by stressors, which comprises a mitochondrial benzodiazepine receptor antagonist as an active ingredient.

More specifically, the present invention relates to
(1) a mitochondrial benzodiazepine receptor antagonist comprising the compound of formula (I) 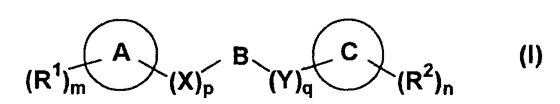 (wherein all symbols have the same meanings as hereafter.) or a non-toxic salt thereof,
(2) an agent for the prophylaxis and/or treatment of diseases induced, exacerbated or reignited by stressor, which is a mitochondrial benzodiazepine receptor antagonist comprising the compound of formula (I), the compound of formula (II) 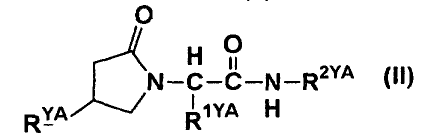 (wherein all symbols have the same meanings as hereafter.), the compound of formula (III) 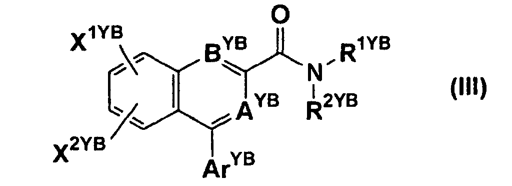 (wherein all symbols have the same meanings as hereafter.), and the compound of formula (IV) 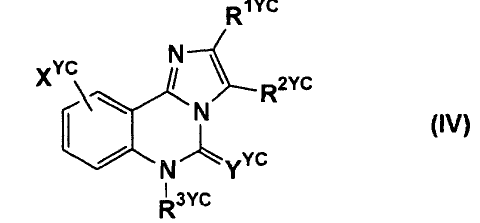 (wherein all symbols have the same meanings as hereafter.) and the compound of formula (V) 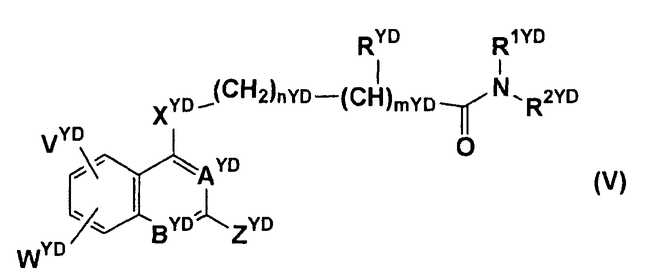 (wherein all symbols have the same meanings as hereafter.) and a non-toxic salt thereof and
(3) a tetrazole derivative of formula (I-a') 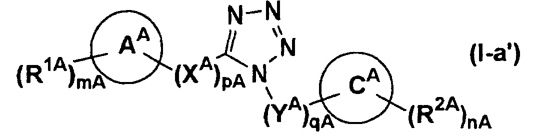 (wherein all symbols have the same meanings as hereafter.).

### Background of the invention and background art

A mitochondrial benzodiazepine receptor (which may be abbreviated to MBR hereafter) is identified in 1977 as a different receptor from the benzodiazepine binding cite which exists in the GABAA receptor which benzodiazepine(s) bind (*Science*, 198, 849-851 (1977); *Proc. Natl*. *Acad*. *Sci*., 89, 3805-3809 (1977)), Although its physiological function is not completely revealed, it is reported that it participates in steroid synthesis, differentiation and proliferation of cells, immunity functional regulation, *etc.* MBR exists in immunity system cells such as erythrocyte, blood platelets, a single ball, and macrophages besides the adrenal cortex, heart, a smooth muscle, kidneys, lungs, and a spermary, in a peripheral organization, while in a central nervous system, it exists in choroid plexus, a pineal body, olfactory bulb, the cerebral cortex, hippocampus, *etc*. It is known that the main expression cells in the central nervous system is a neuroglia, and since the amount of expression increases accompanying nerve denaturation diseases, such as Alzheimer's disease, cerebral ischemia, multiple sclerosis, and the Huntington disease, it is used as a marker of gliosis.

It is reported that, exposed to chronic stressors, morphological changes are recognized in hippocampus such as neurocyte death in CA3 region and atorophy of dendrite and that the glia fiber acidic protein positive cells increases in number (*Stress*, 3, 275-284, 2000); it implies that MBR is activated in glia cells when one is in the stress condition. MBR exists in the mitochondrial outer mcmbrane, and cholesterol is conveyed to the mitochondria inner membrane which is the activity cite of P-450scc from the inside the cells.

The steroid synthesized in a brain is called neurosteroid, the cholesterol which is a steroid precursor is metabolized by side-chain cutting enzyme P-450scc, and the stage changed into pregnenolone is the first phase of a steroid generation system. However, it is shown that that this transportation process is the rate-determining step of the steroid generation system rather than the metabolism by P-450scc. Therefore, if it is possible to adjust the function of MBR, it will be possible to adjust the neurosteroid content in a brain. It is actually reported that diazepam binding inhibiting protein (Diazepam binding inhibitor; which may be abbreviated to DBI hereafter), which is identified as endogenic ligand to GABAA receptor benzodiazepine binding site and MBR, promotes synthesis of pregnenolone in rat brain and mitochondria fraction derived from glioma cell.

It is shown that a DBI content increases in a hippocampus and that DBI concentration in cerebrospinal fluid in a depression patient is rising when sound stressor is loaded on a rat-- it is expected that the amount from neurosteroid of raw is increasing in a stress state, It is expected that the amount of neurosteroid production is increasing in a stress state. As an experimental result which supports it, it is reported that if stressor is loaded on a rat, such as compulsive swimming, a foot shock, carbon dioxide exposure, and a restraint, various neuro-steroid contents increase within a brain.

Neurosteroids adjust the functions of various receptors and ion channels positively or negatively according to the kind. According to the kind, neurosteroid adjusts the function of various receptors and ion channels to positive or negative. For example, pregnenolone sulfate and dehydroepiandrosterone sulfate suppresses the GABA_{A} receptor function, and progesterone activates it. And, pregnenolone sulfate suppresses AMPA / kainate-type glutamate receptor function, glycine receptor, voltage-dependent calcium channel function, but it activates NMDA-type glutamate receptor function. It suppresses kainite-type glutamate-type receptor, glycine receptor, charge-dependent calcium channel function, but it activates the function of NMDA type glutamate receptor. And progestelone suppresses the function of acetylcholine receptor as well as glycine receptor. Moreover, dehydroepiandrostelone sulfate activates the function of σreceptor, while progestelone suppresses it in the contrary. Thus, since the amount of neurosteroid in a brain changes in a stress state, activity of the nervous system currently variously adjusted by these nervous systems because the balance of excitement nature and a control nature communication-of-information system disrupts, an immunity system, and an internal secretion system also changes, it is thought that various stress-related diseases are caused. Furthermore, considering that it is reported that pregnenolone sulfate reinforces the NMDA induction cell death of a cultivation hippocampus nerve cell and that it causes delayed cell death accompanied by DNA fragmentation in retina nerve cells, it suggests a possibility that pregnenolone sulfate are participating also in the denaturation of hippocampus CA3 field at stress state at least in part.

Thus, it is effective to inhibit the increase of production of neurosteroid which is mediated by MBR which is overactivated by stressor loading and resume the balance of excitatory or inhibitory information transmission to normal condition in order to treat stress-related diseases. If MBR antagonist is provided for the purpose, it is expected it is extremely useful for the prophylaxis or treatment of these diseases.

On the other hand, as tetrazole compounds which possess substituents in 1- and 5-positions, the followings are known;
(A) In the specification of WO96/13489, it is described that the compound of formula (X^{A}) 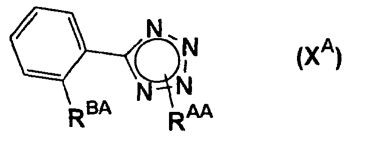 (wherein R^{AA} is benzyl, p-methoxybenzyl, diphenylmethyl or 2-phenyl-2-propyl and R^{BA} is p-methylphenyl or chloro.) is an intermediate for angiotensin-II antagonist.
(B) In the specification of WO 98/28269, the compound of formula (X^{B}) 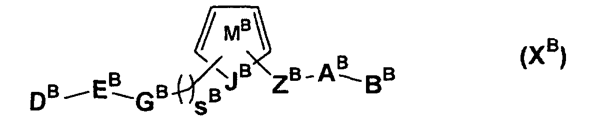 (wherein J^{B} is N or NH, ring M^{B} further has 0-3 of nitrogen, s is 0-2, G^{B} is a bond, NHCH₂, OCH₂, *etc*., E^{B} is phenyl, pyridyl, pyrimidyl, *etc.*, D^{B} is CN, C(=NR^{8B})NR^{7B}R^{9B}, NR^{8B}CH=NR^{7B}, CONR^{7B}R^{8B} or (CR^{8B}R^{9B})ₜNR^{7B}R^{8B}, *etc.,* Z^{B} is C1-4 alkylene *etc.*, A^{B} is 3-10C carboring or heteroring, B^{B} is a substituent. Only the closely related portion extracted explanation of the groups.) is described as a factor Xa inhibitor.
(C) In the specification of JP kokoku 6-62410, it is described that the compound of formula (X^{C}) 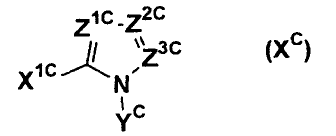 (wherein Z^{1C}, Z^{2C}, Z^{3C} are the same or different to represent nitrogen, =C(X^{2C})-, *etc.,* X^{1C} is optionally substituted phenyl (its substituent is hydroxy, halogen, methoxy, ethoxy, *etc.*), Y^{C} is optionally substituted benzyl (its substituent is hydrogen atom, halogen, nitro, cyano, formyl, alkoxy, *etc*.)) is useful as a medicament for the treatment and/or prophylaxis of hyperuricemia having angiotensin-II receptor antagonist activity.
(D) In the specification of JP1-117876, it is described that the compound of formula (X^{D}) 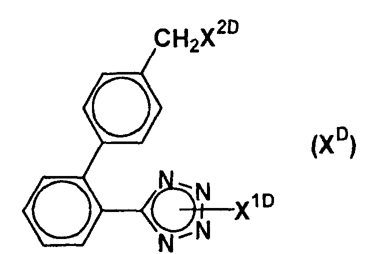 (wherein X^{1D} is H, Sn(R^{D})₃, triphenylmethyl, p-nitrobenzyl, β-propionitril, X^{2D} is hydrogen, chlorine, bromine, iodine, o-tosyl, hydroxy, o-mesyl, *etc*. (Only the closely related portion extracted explanation of the groups.)) is an intermediate for an agent for hypertension.
(E) In the specification of WO00/01666, the compound of formula (X^{E}) 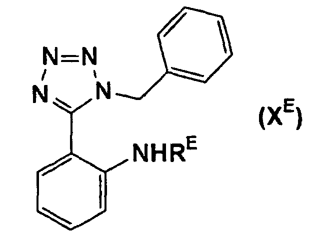 (wherein R^{E} is hydrogen atom, -COCOOH, -COCH₃ or -COCOOCH₃.) is an intermediate for interleukin-1β converting enzyme.
(F) In the specification of WO98/52929, it is described that the compound of formula (X^{F}) 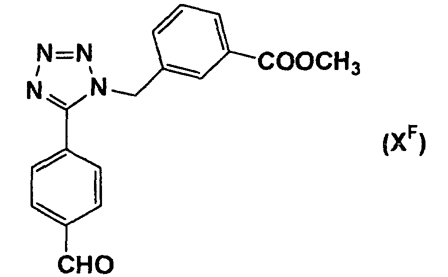 is an intermediate for δ-opioid agonist.

Furthermore, 1H-1,2,3,4-tetrazole derivatives which are substituted in its 1- or 5-position are described in Russ., *J. Org. Chem.,* 33(4), 524-531 (1997), *Heterocycles,* 40(2), 801-8 (1995), *Khim-Geterotsikl*. *Soedin*., 6, 851-3 (1984), *Khim-Farm Zh.,* 20(5), 559-63 (1986), *Tetrahedron Lett.,* 36(10), 1679-82 (1995), *Khim-Geterotsikl*. *Soedin*, (8) 1060-3 (1993), *J*.*C*.*S*. *Perkin Trans I,* (2), 323-7 (1991), *Synlett*., 7, 413-4 (1990), *Tetrahedron Lett*., 31(13), 1925-8 (1990), *J*. *Am. Chem. Soc*., 102(9), 2968-79 (1980), *J. Org. Chem*., 44(19), 3281-7 (1979), *J*. *Med. Chem*., 13(4), 777-9 (1970) and so on.

However, it is not known so far that these tetrazole derivatives have MBR antagonist activity or are useful for stress-related diseases.

And, concretely, the following tetrazole derivatives are known. The numbers described after the substance names are, registry numbers in chemical abstracts (CAS).
(1) 5-(4-chlorophenyl)-1-(3-chlorobenzyl)-1H-1,2,3,4-tetrazole (CAS No. 303144-83-4),
(2) 5-(4-chlorophenyl)-1-(3-trifluoromethylbenzyl)-1H-1,2,3,4-tetrazole (CAS No. 303144-87-8),
(3) 5-(3-chlorophenyl)-1-(3-chlorobenzyl)-1H-1,2,3,4-tetrazole (CAS No. 303144-92-5),
(4) 5-(3-chlorophenyl)-1-(3-trifluoromethylbenzyl)-1H-1,2,3,4-tetrazole (CAS No. 303144-93-6),
(5) 5-(3-trifluoromethylphenyl)-1-(3-chlorobenzyl)-1H-1,2,3,4-tetrazole (CAS No. 303144-94-7),
(6) 5-(3,5-dichlorophenyl)-1-(3-chlorobenzyl)-1H-1,2,3,4-tetrazole (CAS No. 303145-03-1),
(7) 5-(3-trifluoromethylphenyl)-1-(3-trifluoromethylbenzyl)-1H-1,2,3,4-tetrazole (CAS No. 303145-04-2),
(8) 5-(3,5-dichlorophenyl)-1-(3-trifluoromethylbenzyl)-1H-1,2,3,4-tetrazole (CAS No. 303145-07-5),
(9) 5-(4-bromophenyl)-1-benzyl-1H-1,2,3,4-tetrazole (CAS No. 304885-17-4),
(10) 5-(4-pyridyl)-1-(2,5-dimethyl-4-hydroxybenzyl)-1H-1,2,3,4-tetrazole (CAS No. 92595-42-1),
(11) 5-(4-bromophenyl)-1-(2,5-dimethyl-4-hydroxybenzyl)-1H-1,2,3,4-tetrazole (CAS No. 92595-44-3),
(12) 5-phenyl-1-(5-formyl-2-hydroxy-3-methoxybenzyl)-1H-1,2,3,4-tetrazole (CAS No. 104065-28-3),
(13) 5-(3,4-dimethoxyphenyl)-1-(5-formyl-2-hydroxy-3-methoxybenzyl)-1H-1,2,3,4-tetrazole (CAS No. 104065-30-7),
(14) 5-(4-bromophenyl)-1-(3,5-dimethyl-4-hydroxybenzyl)-1H-1,2,3,4-tetrazole (CAS No. 104186-20-1),
(15) 5-(4-pyridyl)-1-(3,5-dimethyl-4-hydroxybenzyl)-1H-1,2,3,4-tetrazole (CAS No. 104186-21-2),
(16) 5-cyclohexyl-1-cyclohexylmethyl-1H-1,2,3,4-tetrazole (CAS No. 107270-34-8),
(17) 5-(4-nitrobenzyl)-1-phenyl-1H-1,2,3,4-tetrazole (CAS No 118241-94-4),
(18) 5-(4-aminobenzyl)-1-phenyl-1H-1,2,3,4-tetrazole (CAS No. 118241-95-5),
(19) 5-(4-dimethylaminocarbonylaminobenzyl)-1-phenyl-1H-1,2,3,4-tetrazole (CAS No. 118259-52-2),
(20) 5-[2-(4-methylphenyl)phenyl]-1-(4-nitrobenzyl)-1H-1,2,3,4-tetrazole (CAS No. 120568-14-1),
(21) 5-phenyl-1-(4-methylbenzyl)-1H-1,2,3,4-tetrazole (CAS No. 131183-02-3),
(22) 5-[4-(4-chlorophenylhydroxymethyl)phenyl]-1-(4-fluorobenzyl)-1H-1,2,3,4-tetrazole (CAS No. 137814-78-9),
(23) 5-[4-(4-chlorobenzoyl)phenyl]-1-(4-fluorobenzyl)-1H-1,2,3,4-tetrazole (CAS No. 137814-80-3),
(24) 1-benzyl-5-(5-nitrofuran-2-yl)-1H-1,2,3,4-tetrazole (CAS No. 15182-14-6),
(25) 1-benzyl-5-[2-(4-bromomethylphenyl)phenyl]-1H-1,2,3,4-tetrazole (CAS No. 152732-25-7),
(26) -1-(3,5-dimethyl-4-hydroxybenzyl)-5-(4-pyridyl)1H-1,2,3,4-tetrazole (CAS No. 153872-97-0),
(27) 1-(3,5-dimethyl-4-hydroxybenzyl)-5-(4-nitrophenyl)-1H-1,2,3,4-tetrazole (CAS No. 153872-98-1),
(28) 5-(4-chlorophenyl)-1-(3,5-dimethyl-4-hydroxybenzyl)-1H-1,2,3,4-tetrazole (CAS No. 153872-99-2),
(29) 1-benzyl-5-(4-methylphenyl)-1H-1,2,3,4-tetrazole (CAS No. 163680-72-6),
(30) 1-benzyl-5-(4-fluorophenyl)-1H-1,2,3,4-tetrazole (CAS No. 163680-73-7),
(31) 1-benzyl-5-(4-methoxyphenyl)-1H-1,2,3,4-tetrazole (CAS No. 163680-74-8),
(32) 1-benzyl-5-(2-methoxyphenyl)-1H-1,2,3,4-tetrazole (CAS No. 163680-75-9),
(33) 1-benzyl-5-(4-benzyloxymethylphenyl)-1H-1,2,3,4-tetrazole (CAS No. 163680-76-0),
(34) 1-benzyl-5-(2-(4-methylphenyl)phenyl)-1H-1,2,3,4-tetrazole (CAS No. 168265-56-3),
(35) 5-(2-chlorophenyl)-1-(4-methoxybenzyl)-1H-1,2,3,4-tetrazole (CAS No. 179089-00-0),
(36) 1-benzyl-5-(2-chlorophenyl)-1H-1,2,3,4-tetrazole (CAS No. 179089-02-2),
(37) 1-(3,5-dimethyl-4-hydroxybenzyl)-5-phenyl-1H-1,2,3,4-tetrazole (CAS No. 203735-93-7),
(38) 1-(4-bromobenzyl)-5-(3-cyanophenyl)-1H-1,2,3,4-tetrazole (CAS No. 209959-39-7),
(39) 5-(3-cyanophenyl)-1-[4-(2-trifluoromethylphenyl)benzyl]-1H-1,2,3,4-tetrazole (CAS No. 209959-40-0),
(40) 5-(5-bromofuran-2-yl)-1-(4-methoxybenzyl)-1H-1,2,3,4-tetrazole (CAS No. 211940-52-2),
(41) 5-(6-bromopyridin-2-yl)-1-(4-methoxybenzyl)-1H-1,2,3,4-tetrazole (CAS No. 211940-58-8),
(42) 5-(6-iodopyridin-2-yl)-1-(4-methoxybenzyl)-1H-1,2,3,4-tetrazole (CAS No. 211940-60-2),
(43) 5-(3-ethoxycarbonylmethyloxy-5-iodophenyl)-1-(4-methoxybenzyl)-1H-1,2,3,4-tetrazole (CAS No. 211941-31-0),
(44) 5-(3-iodo-2-isopropyloxyphenyl)-1-(4-methoxybenzyl)-1H-1,2,3,4-tetrazole (CAS No. 211941-34-3),
(45) 5-(4-formylphenyl)-1-(3-methoxycarbonylbenzyl)-1H-1,2,3,4-tetrazole (CAS No. 216532-80-8),
(46) 5-(2-aminophenyl)-1-benzyl-1H-1,2,3,4-tetrazole (CAS No. 254751-02-5),
(47) 5-(2-acetylaminophenyl)-1-benzyl-1H-1,2,3,4-tetrazole (CAS No. 254751-14-9),
(48) 5-phenyl-1-benzyl-1H-1,2,3,4-tetrazole (CAS No. 28386-90-5),
(49) 5-benzyl-1-(4-mercaptophenyl)-1H-1,2,3,4-tetrazole (CAS No. 83211-81-8),
(50) 5-benzyl-1-phenyl-1H-1,2,3,4-tetrazole (CAS No. 96746-54-2),
(51) 5-benzyl-1-(3,5-dimethyl-4-hydroxybenzyl)-1H-1,2,3,4-tetrazole (CAS No. 104065-32-9),
(52) 1-benzyl-5-benzyl-1H-1,2,3,4-tetrazole (CAS No. 28386-91-6),
(53) 5-phenyl-1-[2-(2-pyridyl)ethyl]-1H-1,2,3,4-tetrazole (CAS No. 178904-79-5),
(54) 5-(2,4-dimethoxy-5-ethylphenethyl)-1-benzyl-1H-1,2,3,4-tetrazole (CAS No. 139032-49-8),
(55) 5-(2,4-diethoxy-5-ethylphenethyl)-1-benzyl-1H-1,2,3,4-tetrazole (CAS No. 139032-54-5),
(56) 5-(2,4-diethoxy-5-ethylphenethyl)-1-phenethyl-1H-1,2,3,4-tetrazole (CAS No. 139050-46-7),
(57) 5-(2,4-dimethoxy-5-ethylphenethyl)-1-phenethyl-1H-1,2,3,4-tetrazole (CAS No. 134032-53-4),
(58) 5-benzyl-1-N,N-diisopropylaminocarbonylbenzyl-1H-1,2,3,4-tetrazole (CAS No. 163680-68-0),
(59) 5-[4-(4-acetyl-3-hydroxy-2-propylphenyloxy)phenyl]-1-(4-cyanobenzyl)-1H-1,2,3,4-tetrazole (CAS No. 122010-30-4),
(60) 5-benzyl-1-(4-amino-2,5-dimethoxyphenyl)-1H-1,2,3,4-tetrazole (CAS No. 107778-57-4),
(61) 5-(2-aminophenyl)-1-benzyl-1H-1,2,3,4-tetrazole (CAS No. 108954-19-4),
(62) 5-(4-t-butylcarbonyloxyphenyl)-1-(4-aminomethylbenzyl)-1H-1,2,3,4-tetrazole (CAS No. 172353-62-7),
(63) 5-(4-t-butylcarbonyloxyphenyl)-1-(4-isopropylbenzyl)-1H-1,2,3,4-tetrazole (CAS No. 172353-69-4),
(64) 5-(4-t-butylcarbonyloxyphenyl)-1-(4-dimethylaminobenzyl)-1H-1,2,3,4-tetrazole (CAS No. 172353-72-9),
(65) 5-(4-t-butylcarbonyloxyphenyl)-1-(4-N-t-butoxycarbonyl-N-methylbenzyl)-1H-1,2,3,4-tetrazole (CAS No. 172353-71-6),
(66) 5-(1-hydroxycycloheptyl)-1-benzyl-1H-1,2,3,4-tetrazole (GAS No. 164589-84-8),
(67) 5-(1-naphthylmethyl)-1-(2-carboxyphenyl)-1H-1,2,3,4-tetrazole (CAS No. 144098-68-0),
(68) 5-(4-carboxy-2-methoxyphenyl)-1-(4-methoxybenzyl)-1H-1,2,3,4-tetrazole (CAS No. 211943-81-6),
(69) 5-(2-furyl)-1-benzyl-1H-1,2,3,4-tetrazole (CAS No. 97800-09-4),
(70) 5-[2-(5-nitro)thieno]-1-benzyl-1H-1,2,3,4-tetrazole (CAS No. 97800-10-7,
(71) 5-[4-methoxycarbonyl-3-(2-methylphenyl)benzyl]-1-benzyl-1H-1,2,3,4-tetrazole (CAS No. 215917-93-4),
(72) 5-[4-carboxy-3-(2-methylphenyl)benzyl]-1-benzyl-1H-1,2,3,4-tetrazole (CAS No. 215917-95-6) and
(73) 5-thieno-1-benzyl-1H-1,2,3,4-tetrazole (CAS No. 307552-97-2).

On the other hand, the followings are known ligands which bind to MBR.

In WO98/14213. it is described that the compound of formula (II) 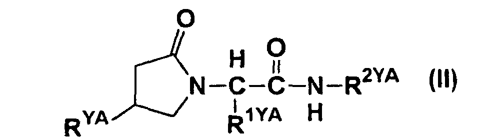 (wherein R^{E} is hydrogen atom or hydroxy, R^{1E} is hydrogen or methyl, R^{2E} is pyridyl or substituted phenyl which is substituted with 1-3 of the same or different substituent(s)) has a stabilizing effect on mitochondria membrane and that it is useful for the diseases resulting from mitochondria function disorders, and it acts on MBR as antagonist.

According to JP4-217682, it is known that the compound of formula (III) 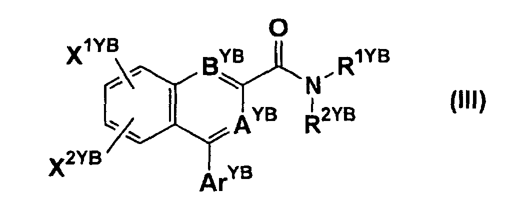 (wherein R^{1YB} and R^{2YB} are each independently straight or branched-chain alkyl having 1-6 carbon atom(s), cycloalkyl having 3-7 carbon atoms, phenylalkyl which has 1-3 carbon atom(s) in alkyl or alkyl substituted with cycloalkyl, or alkenyl having 3-6 carbon atoms or alkynyl (wherein double bond or triple bond is not located in 1-2 position to nitrogen.),
A^{YB} and B^{YB} are each independently N or CH,
X^{1YB} and X^{2YB} are each independently halogen, straight or branched-chain alkyl which has 1-3 of carbon, straight or branched-chain alkoxy which has 1-3 of carbon, nitro, or trifluoromethyl,
Ar^{YB} is phenyl, pyridyl, thienyl, or substituted phenyl (substituent on phenyl is 1 or 2 selected from a group consisting of, halogen, straight or branched-chain alkyl which has 1-4 of carbon atom, straight or branched-chain alkoxy which has 1-4 of carbon, straight or branched-chain alkylthio which has 1-4 of carbon, trifluoromethyl, and nitro.).) binds to MBR.

In JP kokoku hei 3-24467, it is described that the compound of formula (IV) 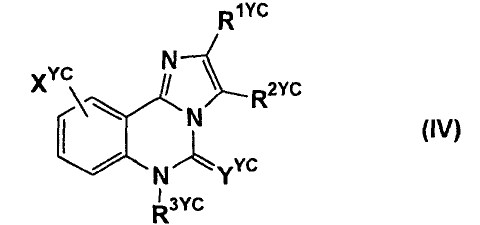 (wherein R^{1YC} is unsubstituted phenyl, or substituted phenyl (substituent on phenyl is 1-2 selected from halogen, straight or branched-chain alkyl having 1-6 of carbon, straight or branched-chain alkoxy having 1-6 of carbon.), or thienyl,
R^{2YC} is hydrogen or halogen, optionally substituted straight or branched-chain alkyl having 1-6 of carbon (substituent is selected from amino, alkylamino, and dialkylamino.),
R^{3YC} is a group represented by (R^{4YC})(R^{5YC})N-CO-Q^{YC} (wherein Q^{YC} is straight or branched-chain alkylene having 1-6 of carbon, R^{4YC} and R^{5YC} are each independently straight or branched-chain alkyl which has 1-6 of carbon, unsubstituted phenyl, or substituted phenyl (phenyl is substituted with 1-2 selected from halogen, straight or branched-chain alkyl having 1-6 of carbon, and straight or branched-chain alkoxy having 1-6 of carbon atom),
X^{YC} is hydrogen atom or halogen,
Y^{YC} is oxygen atom or sulfur atom.) binds to MBR.

In EP210084, it is described that the compound of formula (V) 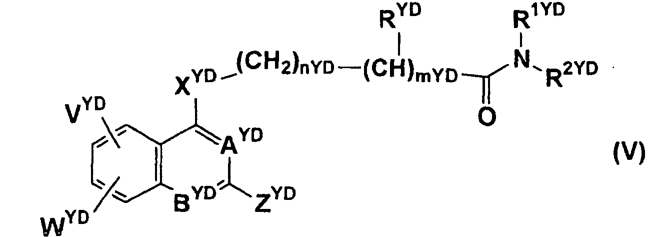 (wherein A^{YD} and B^{YD} are each independently N or CH,
V^{YD} and W^{YD} are, each independently hydrogen, halogen, C1-3 alkyl, C1-3 alkoxy, nitro or trifluoromethyl,
Z^{YD} is phenyl, thienyl or pyridyl, and above phenyl is unsubstituted or substituted with 1-2 of halogen, C1-4 alkyl, C1-4 alkoxy, trifluoromethyl and nitro, Z^{YD} is located ortho or para to B^{YD},
side chain -X^{YD}-(CH₂)_{nYD}-(CHR^{YD})_{mYD}-CONR^{1YD}R^{2YD} is located ortho or para to B^{YD}, R^{YD} is hydrogen or C1-3 alkyl,
R^{1YD} and R^{2YD} are each independently C1-6 alkyl, C3-6 cycloalkyl, phenyl, phenyl-C1-3 alkyl, C3-6 cycloalkyl-C1-3 alkyl or C3-6 alkenyl (with proviso that, double bond in alkenyl is not located in 1,2-position to N atom.), or
NR^{1YD}R^{2YD} is pyrrolidino, piperidino, morpholino or thiomorpholino,
X^{YD} is CHR^{3YD}, NR^{4YD}, SO, SO₂, O or S,
R^{3YD} is hydrogen or C1-3 alkyl,
R^{4YD} is C1-3 alkyl,
m^{YD} is 0 or 1,
n^{YD} is 0 or 1-2
(with proviso that,
(1) when X^{YD} is SO, SO₂, NR^{4YD}, m^{YD}+n^{YD} is one or more,
(2) when both A^{YD} and B^{YD} are N and Z^{YD} is para to B^{YD}, X^{YD} is not CHR^{3YD},
(3) A^{YD} is CH, B^{YD} is N, Z^{YD} is ortho to B^{YD}, X^{YD} is O, and when R^{YD} is hydrogen, m^{YD}+n^{YD} is not 1, and
(4) 2-phenylquinolin-4-yl-N,N'-dimethylcarbamate is excluded.)) binds to MBR and has an anti-anxiety effect.

In the specification of WO 99/28320, it is described that the compound of formula (VI) 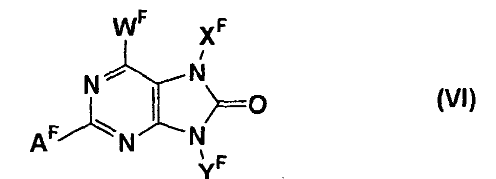 acts on peripheral BZω3 receptor selectively, and is useful for the treatment of central nervous system diseases such as anxiety-related diseases (neuropathy, psychosomatic disorder, anxiety disorder, *etc*.), depression, epilepsy, circulatory system diseases such as angina pectoris, hypertension, *etc*.

However, in the above specification, no implication is suggested about the relationship between MBR antagonist and diseases induced by stress and no experimental proof was carried out. Furthermore, as a result of the experiment which the present inventors tried on the compound described in above WO 99/28320 (The compound of Example 146; N-benzyl-N-ethyl-2-(7-methyl-8-oxo-2-phenyl-7,8-dihydro-9H-purin-9-yl)acetamide), the compound proved to be MBR antagonist and it did not show an effect on stress loaded model of example 5.

As a result of energetic investigation of the present inventors, the present inventors have found out that MBR antagonist, not MBR agonist, is useful for diseases induced, exacerbated or reignited by stressors and that the compound of formula (I) relieves stress as MBR antagonist, to complete the present invention.

### Disclosure of the invention

The present invention relates to a pharmaceutical composition of diseases for the prophylaxis and/or treatment of diseases induced, exacerbated or reignited by stressor comprising an MBR antagonist.

More particularly, the present invention relates to an agent for the prophylaxis and/or treatment of diseases induced, exacerbated or reignited by stressors, which comprises an MBR antagonist characterized by inhibiting neurosteroid production.

The present invention relates to,
(1) a mitochondrial benzodiazepine receptor antagonist comprising the compound of formula (I) 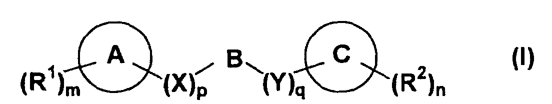 [wherein A and C are each independently 5-10 membered carboring or heteroring, B is 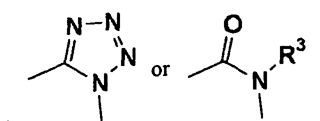 (wherein R³ is hydrogen atom, C1-8 alkyl which may be substituted with phenyl, C2-8 acyl which may be substituted with phenyl or C1-8 alkoxycarbonyl, or R² and R3 are taken together to represent C1-4 alkylene.),
   X and Y are each independently -CH₂-, -O- or -CHR⁴- (wherein R⁴ is C1-4 alkyl, or taken together with R¹ or R², represents C2-5 alkylene),
   p and q are each independently 0 or an integer of 1-2 (wherein p and q do not represent 0 at the same time.),
   R¹ and R² are each independently
   1) halogen,
   2) OR⁵,
   3) SR⁵,
   4) NR⁶R⁷,
   5) nitro,
   6) cyano,
   7) COR⁸,
   8) 3-10 membered carboring or heteroring which may be substituted with 1-5 group(s) selected from C1-8 alkyl (the alkyl may be substituted with 1-5 group(s) selected from halogen, OR⁵, SR⁵, NR⁶R⁷, nitro, cyano, COR⁸ and phenyl.), halogen, OR⁵, SR⁵, NR⁶R⁷, nitro, cyano, COR⁸, and 5-10 membered carboring or heteroring (the ring may be substituted with 1-5 group(s) selected from halogen, OR⁵, SR⁵, NR⁶R⁷, nitro, cyano, COR⁸, phenyl and C1-8 alkyl.), or
   9) C1-8 alkyl substituted with 1-5 group(s) selected from halogen, OR⁵, SR⁵, NR⁶R⁷, nitro, cyano, COR⁸, and 5-10 membered carboring or heteroring (the ring may be substituted with 1-5 group(s) selected from halogen, OR⁹, SR⁹, NR¹⁰R¹¹, nitro, cyano, COR¹², and C1-8 alkyl (the alkyl may be substituted with 1-5 group(s) selected from halogen, OR⁵, SR⁵, NR⁶R⁷, nitro, cyano, COR⁸ and phenyl.).)
   (wherein R⁵ is
   1) hydrogen,
   2) C1-8 alkyl which may be substituted with 1-5 group(s) selected from halogen, OR⁹, SR⁹, NR¹⁰R¹¹, nitro, cyano, COR¹², and 3-10 membered carboring or heteroring (the ring may be substituted with 1-5 group(s) selected from halogen, OR⁹, SR⁹, NR¹⁰R¹¹, nitro, cyano, COR¹², phenyl, and C1-8 alkyl (the alkyl may be substituted with 1-5 group(s) selected from halogen, OR⁹, SR⁹, NR¹⁰R¹¹, nitro, cyano, COR¹², and phenyl.).),
   3) 3-10 membered carboring or heteroring which may be substituted with 1-5 group(s) selected from C1-8 alkyl (the alkyl may be substituted with 1-5 group(s) selected from halogen, OR⁹, SR⁹, NR¹⁰R¹¹, nitro, cyano, COR¹², and phenyl.), halogen, OR⁹, SR⁹, NR¹⁰R¹¹, nitro, cyano, COR¹², and 3-10 membered carboring or heteroring (the ring may be substituted with 1-5 group(s) selected from, halogen, OR⁹, SR⁹, NR¹⁰R¹¹, nitro, cyano, COR¹², phenyl, and C1-8 alkyl.), or
   4) COR¹³ (R¹³ is C1-8 alkyl (the alkyl may be substituted with 1-5 group(s) selected from halogen, OR⁹, SR⁹, NR¹⁰R¹¹, nitro, cyano, COR¹², and phenyl.) or 3-10 membered carboring or heteroring (the ring may be substituted with 1-5 group(s) selected from halogen, OR⁹, SR⁹, NR¹⁰R¹¹, nitro, cyano, COR¹², phenyl, and C1-8 alkyl.).),
   R⁶ and R⁷ are each independently
   1) hydrogen,
   2) C2-8 acyl may be substituted with 1-5 group(s) selected from halogen, OR⁹, SR⁹, NR¹⁰R¹¹, nitro, cyano, COR¹², and 3-10 membered carboring or heteroring (the ring may be substituted with 1-5 group(s) selected from halogen, OR⁹, SR⁹, NR¹⁰R¹¹, nitro, cyano, COR¹², and 3-10 membered carboring or heteroring (the ring may be substituted with 1-5 group(s) selected from, halogen, OR⁹, SR⁹, NR¹⁰R¹¹, nitro, cyano, COR¹², and C1-8 alkyl (the alkyl may be substituted with 1-5 group(s) selected from halogen, OR⁹, SR⁹, NR¹⁰R¹¹, nitro, cyano, COR¹², and phenyl.).).),
   3) COOR¹²,
   4) CONR¹⁰R¹¹,
   5) 3-10 membered carboring which may substituted with 1-5 group(s) selected from C1-8 alkyl (the alkyl may be substituted with 1-5 group(s) selected from halogen, OR⁹, SR⁹, NR¹⁰R¹¹, nitro, cyano, COR¹² and phenyl.), halogen, OR⁹, SR⁹, NR¹⁰R¹¹, nitro, cyano, COR¹², and 3-10 membered carboring or heteroring (the ring may be substituted with 1-5 group(s) selected from, halogen, OR⁹, SR⁹, NR¹⁰R¹¹, nitro, cyano, COR¹², and C1-8 alkyl (the alkyl may be substituted with 1-5 group(s) selected from halogen, OR⁹, SR⁹, NR¹⁰R¹¹, nitro, cyano, and COR¹².).) or
   6) C1-8 alkyl which may be substituted with 1-5 group(s) selected from halogen, OR⁹, SR⁹, NR¹⁰R¹¹, nitro, cyano, COR¹², and 3-10 membered carboring or heteroring (the ring may be substituted with 1-5 group(s) selected from halogen, OR⁹, SR⁹, NR¹⁰R¹¹, nitro, cyano, COR¹², and C1-8 alkyl (the alkyl may be substituted with 1-5 group(s) selected from halogen, OR⁹, SR⁹, NR¹⁰R¹¹, nitro, cyano, and COR¹².).),
   R⁸ is
   1) hydrogen,
   2) OR⁹,
   3) NR¹⁰R¹¹,
   4) 3-10 membered carboring or heteroring which may be substituted with 1-5 group(s) selected from halogen, OR⁹, SR⁹, NR¹⁰R¹¹, nitro, cyano, COR¹², phenyl, C1-8 alkyl (the alkyl may be substituted with 1-5 group(s) selected from halogen, OR⁹, SR⁹, NR¹⁰R¹¹, nitro, cyano, COR¹², and phenyl.), and 3-10 membered carboring or heteroring (the ring may be substituted with 1-5 group(s) selected from halogen, OR⁹, SR⁹, NR¹⁰R¹¹, nitro, cyano, COR¹², C1-8 alkyl, and phenyl.), or
   5) C1-8 alkyl which may be substituted with 1-5 group(s) selected from halogen, OR⁹, SR⁹, NR¹⁰R¹¹, nitro, cyano, COR¹², and 3-10 membered carboring or heteroring (the ring may be substituted with 1-5 group(s) selected from halogen, OR⁹, SR⁹, NR¹⁰R¹¹, nitro, cyano, COR¹², and C1-8 alkyl (the alkyl may be substituted with 1-5 group(s) selected from halogen, OR⁹, SR⁹, NR¹⁰R¹¹, nitro, cyano, COR¹², and phenyl.).)
   (wherein R⁹ is hydrogen atom, C1-8 alkyl or C2-8 acyl (the alkyl and alkoxy may be substituted with C1-8 alkoxy, C1-8 alkylthio or 3-10 membered carboring or heteroring.),
   R¹⁰ and R¹¹ are each independently hydrogen atom, C1-8 alkyl or phenyl,
   R¹² is hydrogen atom, phenyl, C1-8 alkyl optionally substituted with phenyl, or C1-8 alkoxy optionally substituted with phenyl.).),
   m and n are each independently 0 or 1-5.] and
   a non-toxic salt thereof and
(2) a pharmaceutical composition for the treatment and/or prophylaxis of diseases induced, exacerbated or reignited by a stressor, which comprises the compound of formula (I) as an active ingredient,
(3) a pharmaceutical composition for the prophylaxis and/or treatment of diseases induced, exacerbated or reignited by stressors, which comprises the compound of formula 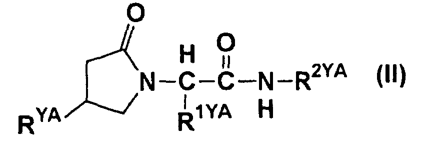 (wherein R^{YA} is hydrogen or hydroxy,
   R^{1YA} is hydrogen or methyl,
   R^{2YA} is pyridyl or phenyl substituted with 1-3 of the same or different substituent(s),
   the substituents on phenyl are selected from halogen, trifluoromethyl, nitro, acetyl, straight or branched-chain alkyl having 1-4 carbon atom(s), straight or branched-chain alkoxy having 1-4 carbon atom(s), straight or branched-chain alkylmercapto having 1-7 carbon atom(s), substituted alkylmercapto of formula: -S-(CH₂)_{nYA}-CH(R^{3YA})(R^{4YA}) (wherein n^{YA} is 1 or 2, R^{3YA} is hydrogen or methyl, R^{4YA} is hydroxy or substituted amino of formula: -NR^{8YA}R^{9YA} (wherein R^{8YA} is hydrogen or methyl, R^{9YA} is methyl, benzyl, or substituted benzyl, or R^{8YA} and R^{9YA} are taken together to form substituted pyrrolidine ring with the nitrogen atom in the formula.).), sulfonyl of formula: -SO₂R^{5YA} (wherein R^{5YA} is amino or alkyl having 1-3 carbon atom(s).), and aminoethoxycarbonyl of formula: -COO(CH₂)₂-NR^{6YA}R^{7YA} (wherein R^{6YA} and R^{7YA} are each independently hydrogen atom, methyl, or ethyl.).) as an active ingredient,
(4) a pharmaceutical composition for the prophylaxis and/or treatment of diseases induced, exacerbated or reignited bv stressors, which comprises the compound of formula (III) 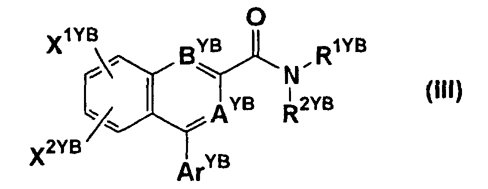 (wherein R^{1YB} and R^{2YB} are each independently straight or branched-chain alkyl having 1-6 of carbon atom, cycloalkyl having 3-7 of carbon atom, phenylalkyl which has 1-3 of carbon atom in alkyl or alkyl substituted with cycloalkyl, or alkenyl having 3-6 of carbon atoms or alkynyl (wherein double bond or triple bond is not located in 1-2 position to nitrogen atom.),
   A^{YB} and B^{YB} are each independently N or CH,
   X^{1YB} and X^{2YB} are each independently halogen, straight or branched-chain alkyl which has 1-3 of carbon atom, straight or branched-chain alkoxy which has 1-3 of carbon atom(s), nitro, or trifluoromethyl,
   Ar^{YB} is phenyl, pyridyl, thienyl, or substituted phenyl (substituent on phenyl is 1 or 2 selected from a group consisting of, halogen, straight or branched-chain alkyl which has 1-4 of carbon atom(s), straight or branched-chain alkoxy which has 1-4 of carbon atom(s), straight or branched-chain alkylthio which has 1-4 of carbon atom(s), trifluoromethyl, and nitro.).) as an active ingredient,
(5) a pharmaceutical composition for the prophylaxis and/or treatment of diseases induced, exacerbated or reignited bv stressors, which comprises the compound of formula (IV) 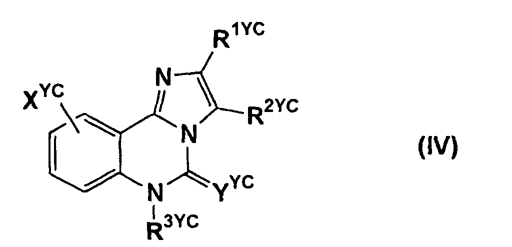 (wherein R^{1YC} is unsubstituted phenyl, substituted phenyl (wherein substituent on phenyl is I or 2 selected from halogen, straight or branched-chain alkyl having C1-6, and straight or branched-chain alkoxy having C1-6.), or thienyl,
   R^{2YC} is hydrogen atom or halogen, optionally substituted straight or branched-chain alkyl having C1-6 (substituent is selected from amino, alkylamino, and dialkylamino.),
   R^{3YC} is a group of formula (R^{4YC})(R^{5YC})N-CO-Q^{YC} (wherein Q^{YC} is straight or branched-chain alkylene having C1-6, R^{4YC} and R^{5YC} are each independently straight or branched-chain alkyl having C1-6, unsubstituted phenyl, or substituted phenyl (substituents on phenyl are 1 or 2 selected from halogen, straight or branched-chain alkyl having C1-6, and straight or branched-chain alkoxy having C1-6.),
   X^{YC} is hydrogen or halogen,
   Y^{YC} is oxygen or sulfur.) as an active ingredient,
(6) a pharmaceutical composition for the prophylaxis and/or treatment of diseases induced, exacerbated or reignited by stressors, which comprises the racemate or optical isomer, or a non-toxic salt of the compound of formula (V) 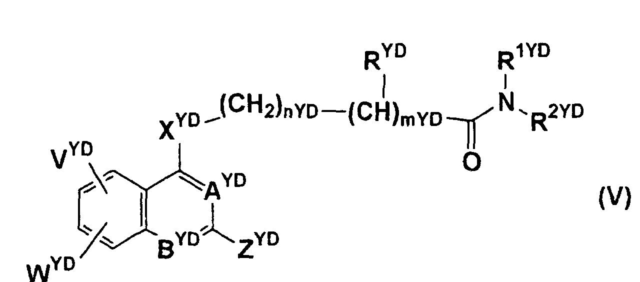 (wherein A^{YD} and B^{YD} are each independently N or CH,
   V^{YD} and W^{YD} are, each independently hydrogen, halogen, C1-3 alkyl, C1-3 alkoxy, nitro or trifluoromethyl,
   Z^{YD} is phenyl, thienyl or pyridyl, above phenyl is unsubstituted or substituted with 1-2 substituent(s) selected from halogen, C1-4 alkyl, C1-4 alkoxy, trifluoromethyl, and nitro, Z^{YD} is located to ortho- or para-position to B^{YD},
   side chain -X^{YD}-(CH₂)_{nYD}-(CHR^{YD})_{mYD}-CONR^{1YD}R^{2YD} is located to ortho- or para- to B^{YD},
   R^{YD} is hydrogen or C 1-3 alkyl,
   R^{1YD}, R^{2YD} is each independently C1-6 alkyl, C3-6 cycloalkyl, phenyl, phenyl-C1-3 alkyl, C3-6 cycloalkyl-C1-3 alkyl or C3-6 alkenyl (wherein the double bond in alkenyl is not located in the 1,2-position to N atom.), or
   NR^{1YD}R^{2YD} is pyrrolidino, piperidino, morpholino or thiomorpholino,
   X^{YD} is CHR^{3YD}, NR^{4YD}, SO, SO₂, O or S,
   R^{3YD} is hydrogen or C1-3 alkyl,
   R^{4YD} is C1-3 alkyl,
   m^{YD} is 0 or 1,
   n^{YD} is 0 or 1-2
   (wherein (1) when X^{YD} is SO, SO₂ or NR^{4YD}, m^{YD}+n^{YD} is 1 or more,

(2) when both A^{YD} and B^{YD} are N and Z^{YD} is para to B^{YD}, X^{YD} is not CHR^{3YD},
(3) when A^{YD} is CH, B^{YD} is N, Z^{YD} is ortho to B^{YD}, X^{YD} is O and R^{YD} is hydrogen, m^{YD}+n^{YD} is not 1,
(4) 2-phenylquinolin-4-yl-N,N'-dimethylcarbamate is excluded.)), its racemate or optical isomer, or a non-toxic salt thereof as an active ingredient
   And the present invention further relates to a compound of formula (I-a') 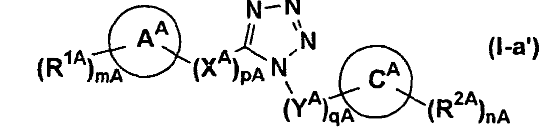 (wherein A^{A} and C^{A} are each independently 5-10 membered carboring or heteroring,
X^{A} and Y^{A} are each independently -CH₂-, -O- or -CHR^{4A}-(wherein R^{4A} is C1-4 alkyl, or taken together with R^{1A} or R^{2A} to form C2-5 alkylene),
p^{A} and q^{A} are each independently 0 or an integer of 1-2 (wherein p^{A} and q^{A} do not form 0 at the same time.),
R^{1A} and R^{2A} are each independently
1) halogen,
2) OR^{5A},
3) SR^{5A},
4) NR^{6A}R^{7A},
5) nitro,
6) cyano,
7) COR^{8A},
8) 3-10 membered carboring or heteroring which may be substituted with 1-5 group(s) selected from C1-8 alkyl (the alkyl may be substituted with 1-5 group(s) selected from halogen, OR^{5A}, SR^{5A}, NR^{6A}R^{7A}, nitro, cyano, COR^{8A}, and phenyl.), halogen, OR^{5A}, SR^{5A}, NR^{6A}R^{7A}, nitro, cyano, COR^{8A}, and 5-10 membered carboring or heteroring (the ring may be substituted with 1-5 group(s) selected from halogen, OR^{5A}, SR^{5A}, NR^{6A}R^{7A}, nitro, cyano, COR^{8A}, phenyl, and C1-8 alkyl.), or
9) C1-8 alkyl which may be substituted with 1-5 group(s) selected from halogen, OR^{5A}, SR^{5A}, NR^{6A}R^{7A}, nitro, cyano, COR^{8A}, and 5-10 membered carboring or heteroring (the ring may be substituted with 1-5 group(s) selected from halogen, OR^{9A}, SR^{9A}, NR^{10A}R^{11A}, nitro, cyano, COR^{12A}, and C1-8 alkyl (the alkyl may be substituted with 1-5 group(s) selected, halogen, OR^{5A}, SR^{5A}, NR^{6A}R^{7A}, nitro, cyano, COR^{8A}, and phenyl.).),
R^{5A} is
1) hydrogen,
2) C1-8 alkyl substituted with 1-5 group(s) selected from halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A}, phenyl, and C1-8 alkyl (the alkyl may be substituted with 1-5 group(s) selected from halogen, OR^{9A}, SR^{9A}, NR^{10A}R^{11A}, nitro, cyano, COR^{12A}, and phenyl.).),
3) 3-10 membered carboring or heteroring which may be substituted with 1-5 group(s) selected from C1-8 alkyl (the alkyl may be substituted with 1-5 group(s) selected from halogen, OR^{9A}, SR^{9A}, NR^{10A}R^{11A}, nitro, cyano, COR^{12A} and phenyl.), halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A}, phenyl, and C1-8 alkyl), or
4) COR^{13A} (R^{13A} is C1-8 alkyl (the alkyl may be substituted with 1-5 group(s) selected from halogen, OR^{9A}, SR^{9A}, NR^{10A}R^{11A}, nitro, cyano, COR^{12A}, and phenyl.) or 3-10 membered carboring or heteroring (the ring may be substituted with 1-5 group(s) selected from halogen, OR^{9A}, SR^{9A}, NR^{10A}R^{11A}, nitro, cyano, COR^{12A}, phenyl, and C1-8 alkyl.).),
R^{6A} and R^{7A} are each independently
1) hydrogen,
2) C2-8 acyl which may be substituted with 1-5 group(s) selected from halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A} and C1-8 alkyl (the alkyl may be substituted with 1-5 group(s) selected from halogen, OR^{9A}, SR^{9A}, NR^{10A}R^{11A}, nitro, cyano, COR^{12A}, and phenyl.).).),
3) COOR^{12A},
4) CONR^{10A}R^{11A},
5) 3-10 membered carboring or heteroring which may be substituted with 1-5 group(s) selected from C1-8 alkyl (the alkyl may be substituted with 1-5 group(s) selected from halogen, OR^{9A}, SR^{9A}, NR^{10A}R^{11A}, nitro, cyano, COR^{12A}, and phenyl.), halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A} and C1-8 alkyl (the alkyl may be substituted with 1-5 group(s) selected from halogen, OR^{9A}, SR^{9A}, NR^{10A}R^{11A}, nitro, cyano, and COR^{12A}.).) or
6) C1-8 alkyl which may be substituted with 1-5 group(s) selected from halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A}, and C1-8 alkyl (the alkyl may be substituted with 1-5 group(s) selected from halogen, OR^{9A}, SR^{9A}, NR^{10A}R^{11A}, nitro, cyano, and COR^{12A}.).),
R^{8A} is
1) hydrogen,
2) OR^{9A},
3) NR^{10A}R^{11A},
4) 3-10 membered carboring or heteroring which may be substituted with 1-5 group(s) selected from halogen, OR^{9A}, SR^{9A}, NR^{10A}R^{11A}, nitro, cyano, COR^{12A}, phenyl, C1-8 alkyl (the alkyl may be substituted with 1-5 group(s) selected from halogen, OR^{9A}, SR^{9A}, NR^{10A}R^{11A}, nitro, cyano, COR^{12A}, and phenyl.), and 3-10 membered carboring or heteroring (the ring may be substituted with 1-5 group(s) selected from halogen, OR^{9A}, SR^{9A}, NR^{10A}R^{11A}, nitro, cyano, COR^{12A}, C1-8 alkyl, and phenyl.) or
5) C1-8 alkyl which may be substituted with 1-5 group(s) selected from halogen, OR^{9A}, SR^{9A}, NR^{10A}R^{11A}, nitro, cyano, COR^{12A}, and 3-10 membered carboring or heteroring (the ring may be substituted with 1-5 group(s) selected from halogen, OR^{9A}, SR^{9A}, NR^{10A}R^{11A}, nitro, cyano, COR^{12A}, and C1-8 alkyl (the alkyl may be substituted with 1-5 group(s) selected from halogen, OR^{9A}, SR^{9A}, NR^{10A}R^{11A}, nitro, cyano, COR^{12A}, and phenyl.).),
R^{9A} is hydrogen, C1-8 alkyl or C2-8 acyl (the alkyl and alkoxy may be substituted with C1-8 alkoxy, C1-8 alkylthio or 3-10 membered carboring or heteroring.),
R^{10A} and R^{11A} are each independently hydrogen, C1-8 alkyl or phenyl,
R^{12A} is hydrogen, phenyl, C1-8 alkyl which may be substituted with phenyl, or C1-3 alkoxy which may be substituted with phenyl,
m^{A} and n^{A} are each independently 0 or an integer of 1-5;
wherein the compounds of following (1)-(73) are excluded;
(1) 5-(4-chlorophenyl)-1-(3-chlorobenzyl)-1H-1,2,3,4-tetrazole,
(2) 5-(4-chlorophenyl)-1-(3-trifluoromethylbenzyl)-1H-1,2,3,4-tetrazole,
(3) 5-(3-chlorophenyl)-1-(3-chlorobenzyl)-1H-1,2,3,4-tetrazole,
(4) 5-(3-chlorophenyl)-1-(3-trifluoromethylbenzyl)-1H-1,2,3,4-tetrazole,
(5) 5-(3-trifluoromethylphenyl)-1-(3-chlorobenzyl)-1H-1,2,3,4-tetrazole,
(6) 5-(3,5-dichlorophenyl)-1-(3-chlorobenzyl)-1H-1,2,3,4-tetrazole,
(7) 5-(3-trifluoromethylphenyl)-1-(3-trifluoromethylbenzyl)-1H-1,2,3,4-tetrazole,
(8) 5-(3,5-dichlorophenyl)-1-(3-trifluoromethylbenzyl)-1H-1,2,3,4-tetrazole,
(9) 5-(4-bromophenyl)-1-benzyl-1H-1,2,3,4-tetrazole,
(10) 5-(4-pyridyl)-1-(2,5-dimethyl-4-hydroxybenzyl)-1H-1,2,3,4-tetrazole,
(11) 5-(4-bromophenyl)-1-(2,5-dimethyl-4-hydroxybenzyl)-1H-1,2,3,4-tetrazole
(12) 5-phenyl-1-(5-formyl-2-hydroxy-3-methoxybenzyl)-1H-1,2,3,4-tetrazole,
(13) 5-(3,4-dimethoxyphenyl)-1-(5-formyl-2-hydroxy-3-methoxybenzyl)-1H-1,2,3,4-tetrazole,
(14) 5-(4-bromophenyl)-1-(3,5-dimethyl-4-hydroxybenzyl)-1H-1,2,3,4-tetrazole,
(15) 5-(4-pyridyl)-1-(3,5-dimethyl-4-hydroxybenzyl)-1H-1,2,3,4-tetrazole,
(16) 5-cyclohexyl-1-cyclohexylmethyl-1H-1,2,3,4-tetrazole,
(17) 5-(4-nitrobenzyl)-1-phenyl-1H-1,2,3,4-tetrazole,
(18) 5-(4-aminobenzyl)-1-phenyl-1H-1,2,3,4-tetrazole,
(19) 5-(4-dimethylaminocarbonylaminobenzyl)-1-phenyl-1H-1,2,3,4-tetrazole,
(20) 5-[2-(4-methylphenyl)phenyl]-1-(4-nitrobenzyl)-1H-1,2,3,4-tetrazole,
(21) 5-phenyl-1-(4-methylbenzyl)-1H-1,2,3,4-tetrazole,
(22) 5-[4-(4-chlorophenylhydroxymethyl)phenyl]-1-(4-fluorobenzyl)-1H-1,2,3,4-tetrazole,
(23) 5-[4-(4-chlorobenzoyl)phenyl]-1-(4-fluorobenzyl)-1H-1,2,3,4-tetrazole,
(24) 1-benzyl-5-(5-nitrofuran-2-yl)-1H-1,2,3,4-tetrazole,
(25) 1-benzyl-5-[2-(4-bromomethylphenyl)phenyl]-1H-1,2,3,4-tetrazole,
(26) -1-(3,5-dimethyl-4-hydroxybenzyl)-5-(4-pyridyl)1H-1,2,3,4-tetrazole,
(27) 1-(3,5-dimethyl-4-hydroxybenzyl)-5-(4-nitrophenyl)-1H-1,2,3,4-tetrazole,
(28) 5-(4-chlorophenyl)-1-(3,5-dimethyl-4-hydroxybenzyl)-1H-1,2,3,4-tetrazole,
(29) 1-benzyl-5-(4-methylphenyl)-1H-1,2,3,4-tetrazole,
(30) 1-benzyl-5-(4-fluorophenyl)-1H-1,2,3,4-tetrazole,
(31) 1-benzyl-5-(4-methoxyphenyl)-1H-1,2,3,4-tetrazole,
(32) 1-benzyl-5-(2-methoxyphenyl)-1H-1,2,3,4-tetrazole,
(33) 1-benzyl-5-(4-benzyloxymethylphenyl)-1H-1,2,3,4-tetrazole,
(34) 1-benzyl-5-(2-(4-methylphenyl)phenyl)-1H-1,2,3,4-tetrazole,
(35) 5-(2-chlorophenyl)-1-(4-methoxybenzyl)-1H-1,2,3,4-tetrazole,
(36) 1-benzyl-5-(2-chlorophenyl)-1H-1,2,3,4-tetrazole,
(37) 1-(3,5-dimethyl-4-hydroxybenzyl)-5-phenyl-1H-1,2,3,4-tetrazole,
(38) 1-(4-bromobenzyl)-5-(3-cyanophenyl)-1H-1,2,3,4-tetrazole,
(39) 5-(3-cyanophenyl)-1-[4-(2-trifluoromethylphenyl)benzyl]-1H-1,2,3,4-tetrazole,
(40) 5-(5-bromofuran-2-yl)-1-(4-methoxybenzyl)-1H-1,2,3,4-tetrazole,
(41) 5-(6-bromopyridin-2-yl)-1-(4-methoxybenzyl)-1H-1,2,3,4-tetrazole,
(42) 5-(6-iodopyridin-2-yl)-1-(4-methoxybenzyl)-1H-1,2,3,4-tetrazole,
(43) 5-(3-ethoxycarbonylmethyloxy-5-iodophenyl)-1-(4-methoxybenzyl)-1H-1,2,3,4-tetrazole,
(44) 5-(3-iodo-2-isopropyloxyphenyl)-1-(4-methoxybenzyl)-1H-1,2,3,4-tetrazolc,
(45) 5-(4-formylphenyl)-1-(3-methoxycarbonylbenzyl)-1H-1,2,3,4-tetrazole,
(46) 5-(2-aminophenyl)-1-benzyl-1H-1,2,3,4-tetrazole,
(47) 5-(2-acetylaminophenyl)-1-benzyl-1H-1,2,3,4-tetrazole,
(48) 5-phenyl-1-benzyl-1H-1,2,3,4-tetrazole,
(49) 5-benzyl-1-(4-mercaptophenyl)-1H-1,2,3,4-tetrazole,
(50) 5-benzyl-1-phenyl-1H-1,2,3,4-tetrazole,
(51) 5-benzyl-1-(3,5-dimethyl-4-hydroxybenzyl)-1H-1,2,3,4-tetrazole,
(52) 1-benzyl-5-benzyl-1H-1,2,3,4-tetrazole,
(53) 5-phenyl-1-[2-(2-pyridyl)ethyl]-1H-1,2,3,4-tetrazole,
(54) 5-(2,4-dimethoxy-5-ethylphenethyl)-1-benzyl-1H-1,2,3,4-tetrazole,
(55) 5-(2,4-diethoxy-5-ethylphenethyl)-1-benzyl-1H-1,2,3,4-tetrazole,
(56) 5-(2,4-diethoxy-5-ethylphenethyl)-1-phenethyl-1H-1,2,3,4-tetrazole,
(57) 5-(2,4-dimethoxy-5-ethylphenethyl)-1-phenethyl-1H-1,2,3,4-tetrazole,
(58) 5-benzyl-1-N,N-diisopropylaminocarbonylbenzyl-1H-1,2,3,4-tetrazole,
(59) 5-[4-(4-acetyl-3-hydroxy-2-propylphenyloxy)phenyl]-1-(4-cyanobenzyl)-1H-1,2,3,4-tetrazole,
(60) 5-benzyl-1-(4-amino-2,5-dimethoxyphenyl)-1H-1,2,3,4-tetrazole,
(61) 5-(2-aminophenyl)-1-benzyl-1H-1,2,3,4-tetrazole,
(62) 5-(4-t-butylcarbonyloxyphenyl)-1-(4-aminomethylbenzyl)-1H-1,2,3,4-tetrazole,
(63) 5-(4-t-butylcarbonyloxyphenyl)-1-(4-isopropylbenzyl)-1H-1,2,3,4-tetrazole,
(64) 5-(4-t-butylcarbonyloxyphenyl)-1-(4-dimethylaminobenzyl)-1H-1,2,3,4-tetrazole,
(65) 5-(4-t-butylcarbonyloxyphenyl)-1-(4-N-t-butoxycarbonyl-N-methylbenzyl)-1H-1,2,3,4-tetrazole,
(66) 5-(1-hydroxycycloheptyl)-1-benzyl-1H-1,2,3,4-tetrazole,
(67) 5-(1-naphthylmethyl)-1-(2-carboxyphenyl)-1H-1,2,3,4-tetrazole,
(68) 5-(4-carboxy-2-methoxyphenyl)-1-(4-methoxybenzyl)-1H-1,2,3,4-tetrazole,
(69) 5-(2-furyl)-1-benzyl-1H-1,2,3,4-tetrazole,
(70) 5-[2-(5-nitro)thieno]-1-benzyl-1H-1,2,3,4-tetrazole,
(71) 5-[4-methoxycarbonyl-3-(2-methylphenyl)benzyl]-1-benzyl-1H-1,2,3,4-tetrazole,
(72) 5-[4-carboxy-3-(2-methylphenyl)benzyl]-1-benzyl-1H-1,2,3,4-tetrazole and
(73) 5-thieno-1-benzyl-1H-1,2,3,4-tetrazole.)
or a non-toxic salt thereof.

In the formula (I), C1-8 alkyl is methyl, ethyl, propyl, butyl, pentyl, hexyl, heptyl, octyl and isomers thereof.

In the formula (I), C1-8 alkoxycarbonyl is methoxycarbonyl, ethoxycarbonyl, propoxycarbonyl, butoxycarbonyl, pentyloxycarbonyl, hexyloxycarbonyl, heptyloxycarbonyl, octyloxycarbonyl and isomers thereof.

In the formula (I), C1-4 alkylene is methylene, ethylene, propylene, butylene and isomers thereof.

In the formula (I), C2-5 alkylene is ethylene, propylene, butylenes, pentylene and isomers thereof.

In the formula (I), halogen is fluorine, chlorine, bromine and iodine.

In the formula (I), C2-8 acyl is acetyl, propanoyl, butanoyl, pentanoyl, hexanoyl, heptanoyl, octanoyl and isomers thereof.

In the formula (I), C1-8 alkoxy is methoxy, ethoxy, propoxy, butoxy, pentyloxy, hexyloxy, heptyloxy, octyloxy and isomers thereof.

In the formula (I), C1-8 alkylthio is methylthio, ethylthio, propylthio, butylthio, pentylthio, hexylthio, heptylthio, octylthio and isomers thereof.

In the formula (I), 5-10 membered carboring or heteroring which represents ring A and ring C is C5-10 mono-, bi- or tri-cyclic carboring or 5-10 membered mono- or bi-cyclic heteroring.

C5-10 mono-, bi- or tri-cyclic carboring which ring A or ring C represents includes, benzene, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, pentalene, indan, indene, naphthalene, dihydronaphthalene, tetrahydronaphthalene, azulene, perhydropentalene, perhydroindene, dihydronaphthalene, tetrahydronaphthalene, perhydronaphthalene, perhydroazulene, norbomane, [2.2.2]bicyclooctane, adamantan, *etc*.

5-10 Membered mono- or bi-cyclic heteroring which ring A or ring C represents includes, a 5-10 membered mono- or bi-cyclic heteroring comprising 1-4 of N, 1-2 of O and/or 1-2 of S, and the 5-10 membered mono- or bi-cyclic heteroring compnsing 1-4 ofN, 1-2 of O and/or 1-2 of S includes 5-10 membered mono- or bi-cyclic heteroaryl comprising 1-4 of N, 1-2 of O and/or 1-2 of S and its partially or completely saturated one.

Above 5-10 membered mono- or bi-cyclic heteroaryl comprising 1-4 of N, 1-2 of O and/or 1-2 of S includes, for example, pyrrole, imidazole, triazole, tetrazole, pyrazole, pyridine, pyrazine, pyrimidine, pyridazine, azepine, diazepine, furan, pyran, oxepin, oxazepine, thiophene, thiain(thiopyran), thiepine, oxazole, isoxazole, thiazole, isothiazole, oxadiazole, oxazine, oxadiazine, oxazepine, oxadiazepine, thiadiazole, thiazine, thiadiazine, thiazepine, thiadiazepine, isoindole, isobenzofuran, isobenzothiophene, indazole, isoquinoline, phthalazine, naphthyridine, quinoxaline, quinazoline, cinnoline, benzoxazole, benzothiazole, benzimidazole, carbazole, acridine, indoloxazepine, indoloxadiazepine, indolothiazepine, indolothiadiazepine, indoloazepine, indolodiazepine, benzofurazane, benzotriazole, imidazothiazole, *etc*.

Above partially or completely saturated 5-10 membered mono- or bi-cyclic heteroaryl comprising 1-4 of N, 1-2 of O and/or 1-2 of S includes, for example, pyrroline, pyrrolidine, imidazoline, imidazolidine, triazoline, triazolidine, tetrazoline, tetrazolidine, dihydropyridine, dihydropyrazine, dihydropyrimidine, dihydropyridazine, piperidine, tetrahydropyridine, piperazine, tetrahydropynmidine, tetrahydropyridazine, dihydrofuran, tetrahydrofuran, dihydropyran, tetrahydropyran, dihydrothiophene, tetrahydrothiophene, dihydrothiain (dihydrothiopyran), tetrahydrothiain (tetrahydrothiopyran), dihydroxazole, tetrahydroxazole, dihydroisoxazole, tetrahydroisoxazole, dihydrothiazole, tetrahydrothiazole, dihydroisothiazole, tetrahydroisothiazole, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzimidazole, perhydrobenzimidazole, dihydrobenzotriazole, camphor, tetrahydrocarbazole, perhydrocarbazole, dihydroacridine, tetrahydroacridine, perhydroacridine, dihydroimidazothiazole, perhydroimidazothiazole, 1,3-dioxaindan, 1,4-benzodioxane, quinuclidine, aziridine, dioxan, oxylan, thioxylane, azetidine, oxetane, thioxetane, *etc*.

In the formula (1), 3-10 membered carboring or heteroring in R¹, R², R⁵, R⁶, R⁷, R⁸ and R⁹, C3-10 mono-, bi- or tri- carboring or 3-10 membered mono- or bi-cyclic heteroring.

Above C3-10 mono-, bi- or tri-cyclic carboring includes, for example, benzene, cyclopropane, cyclobutane, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, pentalene, indan, indene, naphthalene, dihydronaphthalene, tetrahydronaphthalene, azulene, perhydropentalene, perhydroindene, dihydronaphthalene, tetrahydronaphthalene, perhydronaphthalene, perhydroazulene, norbomane, [2.2.2]bicyclooctane, adamantan, *etc*.

Above 3-10 membered mono- or bi-cyclic heteroring includes, 3-10 membered mono- or bi-cyclic heteroring comprising 1-4 of N, 1-2 of O, and/or 1-2 of S, and the 3-10 membered mono- or bi-cyclic heteroring comprising 1-4 of N, 1-2 of O, and/or 1-2 of S includes 3-10 membered mono- or bi-cyclic heteroaryl comprising 1-4 of N, 1-2 of O, and/or 1-2 of S and its partially or completely saturated one.

Above 3-10 membered mono- or bi-cyclic heteroaryl comprising 1-4 of N, 1-2 of O, and/or 1-2 of S includes, for example, pyrrole, imidazole, triazole, tetrazole, pyrazole, pyridine, pyrazine, pyrimidine, pyridazine, azepine, diazepine, furan, pyran, oxepin, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazane, benzothiadiazole, benzotriazole, *etc*.

Above partially or completely saturated 3-10 membered mono- or bi-cyclic heteroaryl comprising 1-4 of N, 1-2 of O, and/or 1-2 of S includes, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, pipendine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxylane, oxetane, dihydrofuran, tetrahydrofuran, dihydropyran, tetrahydropyran, dihydrooxepine, tetrahydroxepine, perhydroxepine, thiilane, thietane, dihydrothiophene, tetrahydrothiophene, dihydrothiopyran, tetrahydrothiopyran, dihydrothiepine, tetrahydrothiepine, perhydrothiepine, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydroxadiazole, tetrahydroxadiazole (oxadiazolidine), dihydroxazine, tetrahydroxazine, dihydroxadiazine, tetrahydroxadiazine, dihydroxazepine, tetrahydroxazepine, perhydroxazepine, dihydroxadiazepine, tetrahydroxadiazepine, perhydroxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathian, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazme, tetrahydrophthalazine, perhydrophthalazine, dihydronaphthyridine, tetrahydronaphthyridine, perhydronaphthyndine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxolane, dioxane, dithiolane, dithiane, dioxaindan, benzodioxane, chroman, benzodithiolane, benzodithiane, *etc*.

The definition of the groups in the compounds of formula (II), (III), (IV) and (V) are as described in WO 98/14213, JP hei 4-217682(A), JP 3-24467(B), and EP210084, respectively.

The definition of the groups in the compound of formula (I-a') is the same as the counterpart in the compound of formula (I), respectively.

In the compound of formula (I), ring A and ring C are preferably, above described C5-10 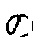 mono-, bi- or tri-cyclic carboring or 5-10 membered mono- or bi-cyclic heteroring, and more preferably benzene, cyclohexane, naphthalene, dihydronaphthalene, tetrahydronaphthalene, cyclopentane, cycloheptane, cyclooctane, indan, norbornane, adamantan, furan, thiophene, pyridine, pyran, pyrrole, pyrazole, triazole, tetrazole, thiazole, oxazole, imidazole, pyrrolidine, morpholine, thiomorpholine, 1,4-dioxaindan, benzofuran and benzothiophene.

B is preferably, both

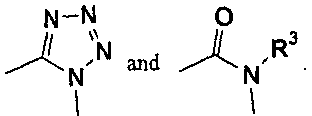

As the combination of p and q, p+q is preferably 1 or 2, and more preferably 1. (p,q) is preferably (0,1), (1,0), (1,1), (2,0), (0,2).

R¹ and R² are each preferably halogen, nitro, cyano, OR⁵, NR⁶R⁷, COR⁸, 5-10 membered carboring or heteroring, or C1-8 alkyl which may be substituted with 1-5 group(s) selected from halogen, OR⁵, NR⁶R⁷, COR⁸ or 5-10 membered carboring or heteroring, more preferably fluorine, chlorine, bromine, nitro, cyano, OR⁵, NR⁶R⁷, C2-8 acyl, COOR⁹, CONR¹⁰R¹¹, 5-10 membered carboring or heteroring, or halogen, hydroxy, OR⁵, NR⁶R⁷, C2-8 acyl, COOR⁹, CONR¹⁰R¹¹ or C1-8 alkyl which may be substituted with 5-10 membered carboring or heteroring.

R³ is all preferable, and more preferably, hydrogen and C1-4 alkyl, and taken together with R² to represent C1-4 alkylene.

X and Y are preferably -CH₂-, -CHR⁴- and -O-.

m and n are preferably 0 and 1-3, respectively.

Preferable compounds in the compound of formula (I-a') are the same as the corresponding ones in the compound of formula (I) shown above.

Preferable compounds in the present invention include the compounds in the following table 1 to table 64 in addition to the compound shown in the examples.

In the following tables, Ph is phenyl, iPr is isopropyl, and Bzl is benzyl.

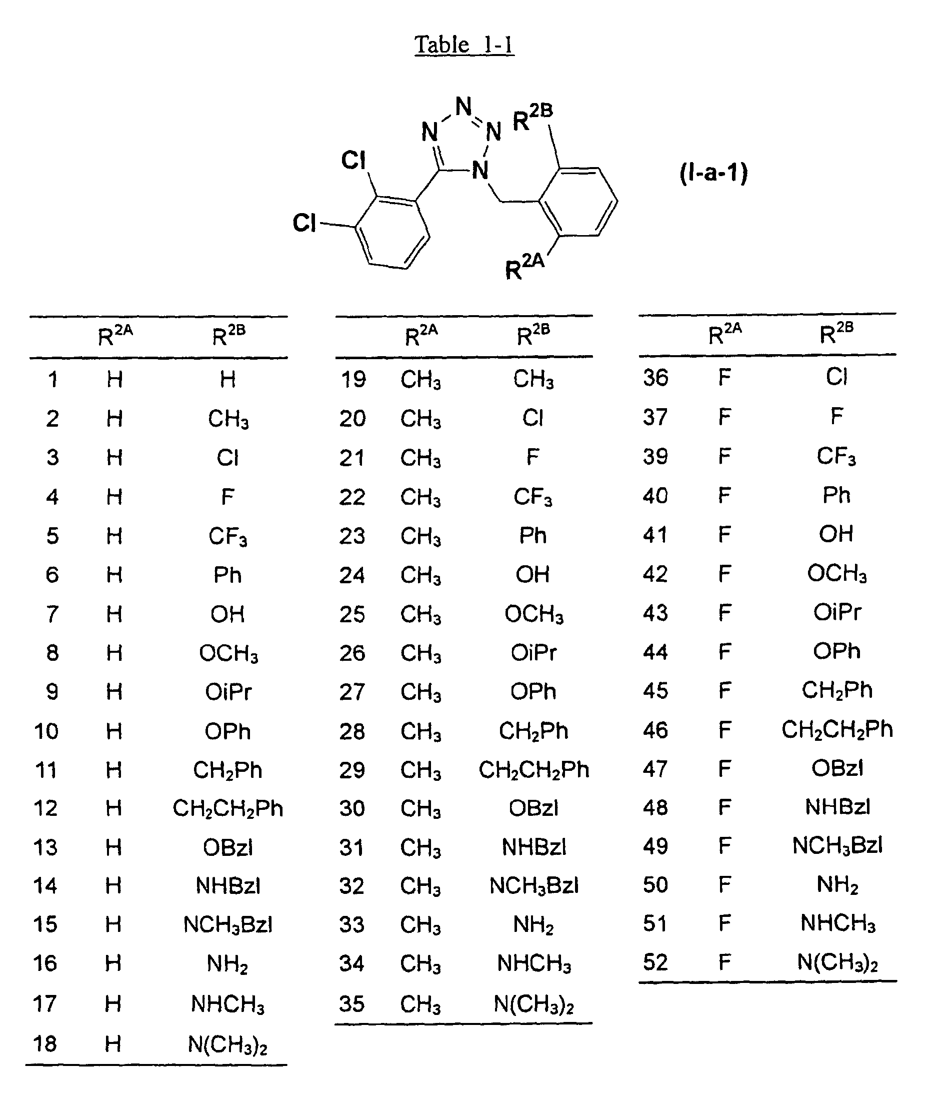

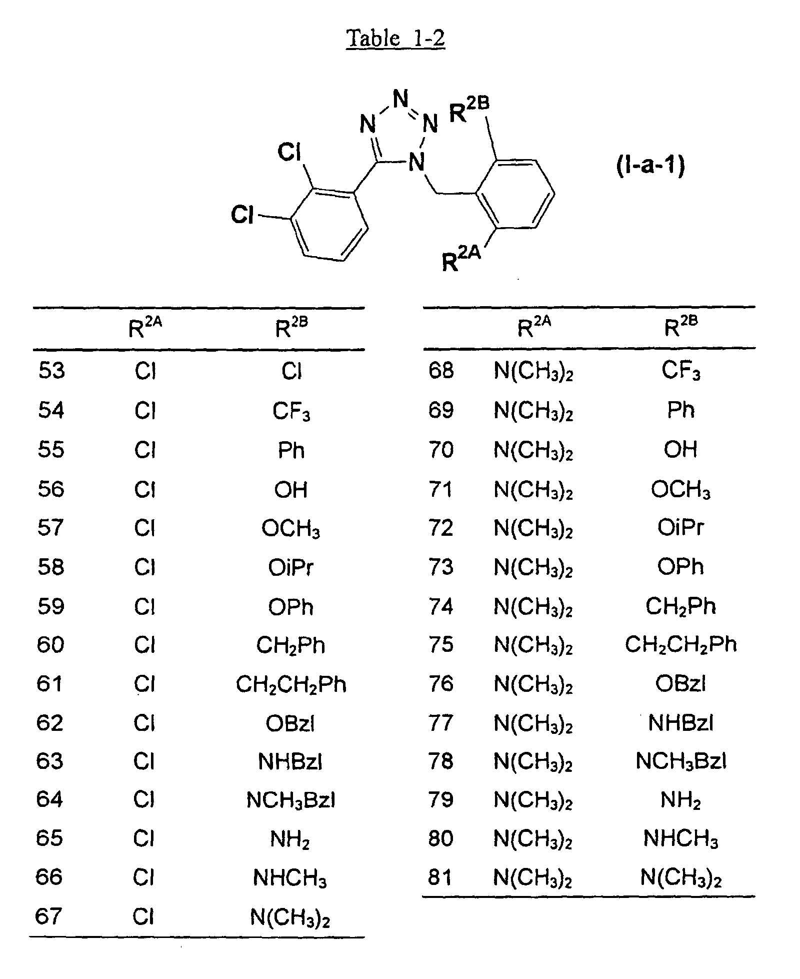

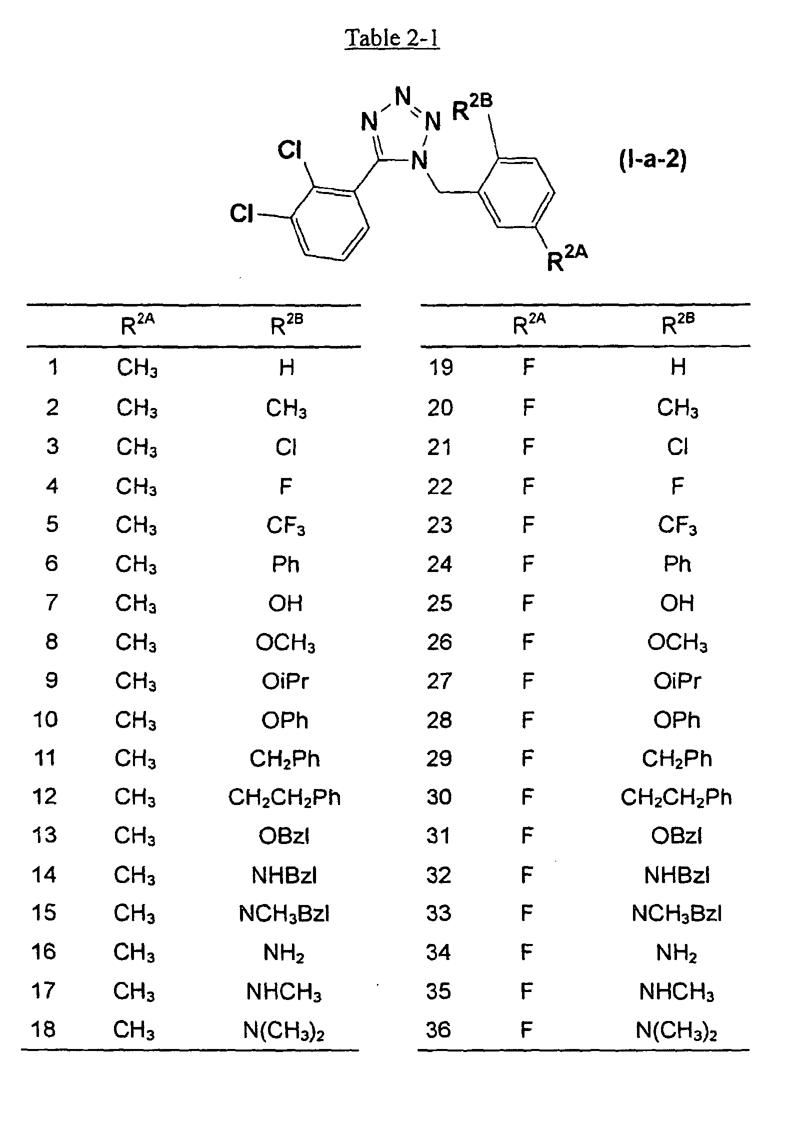

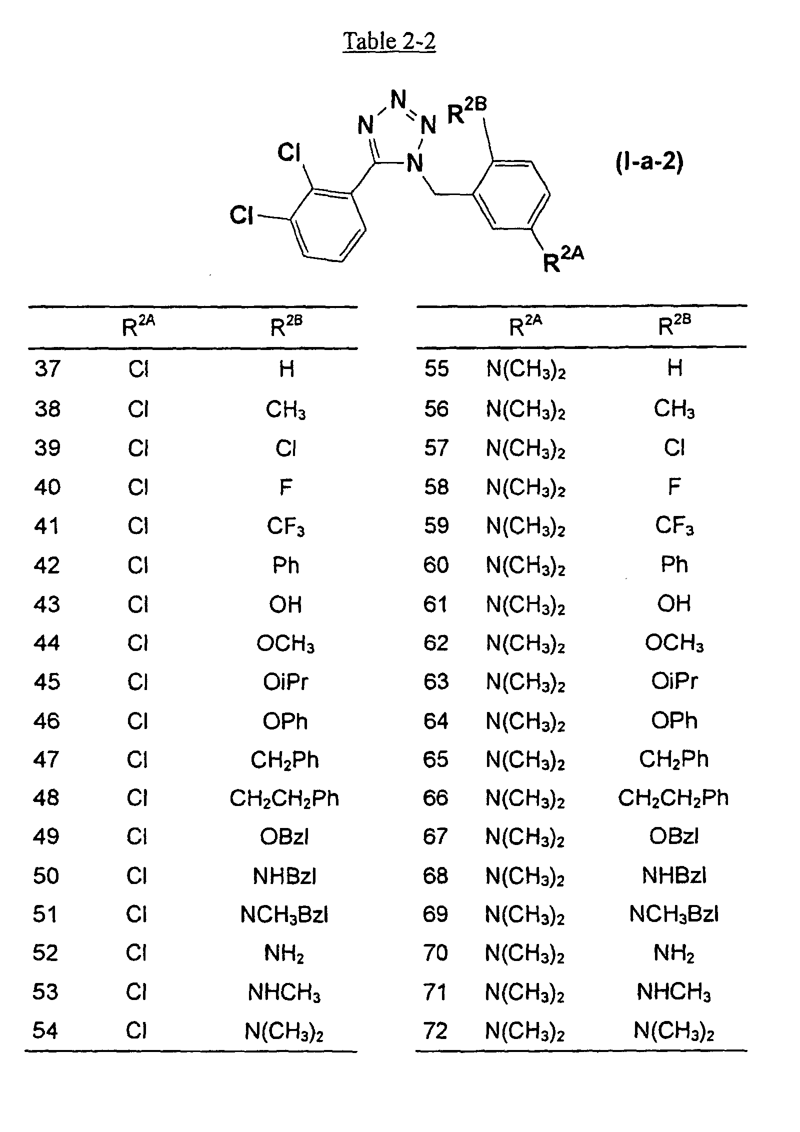

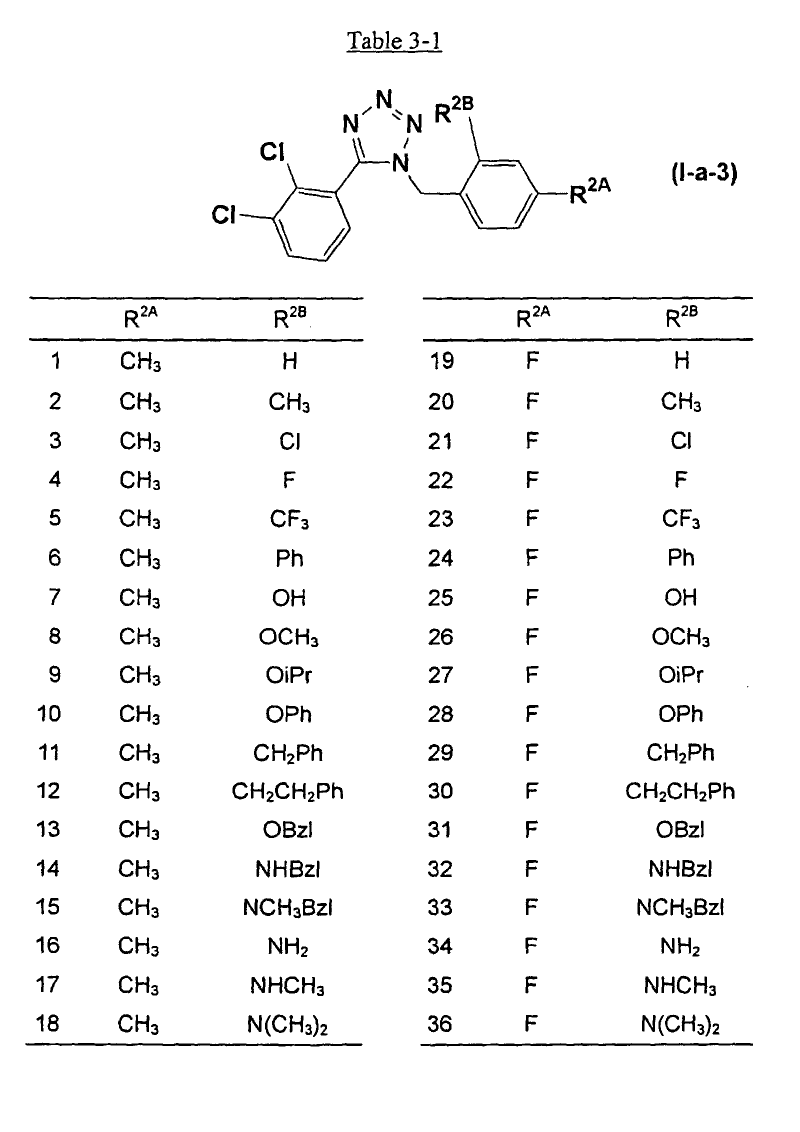

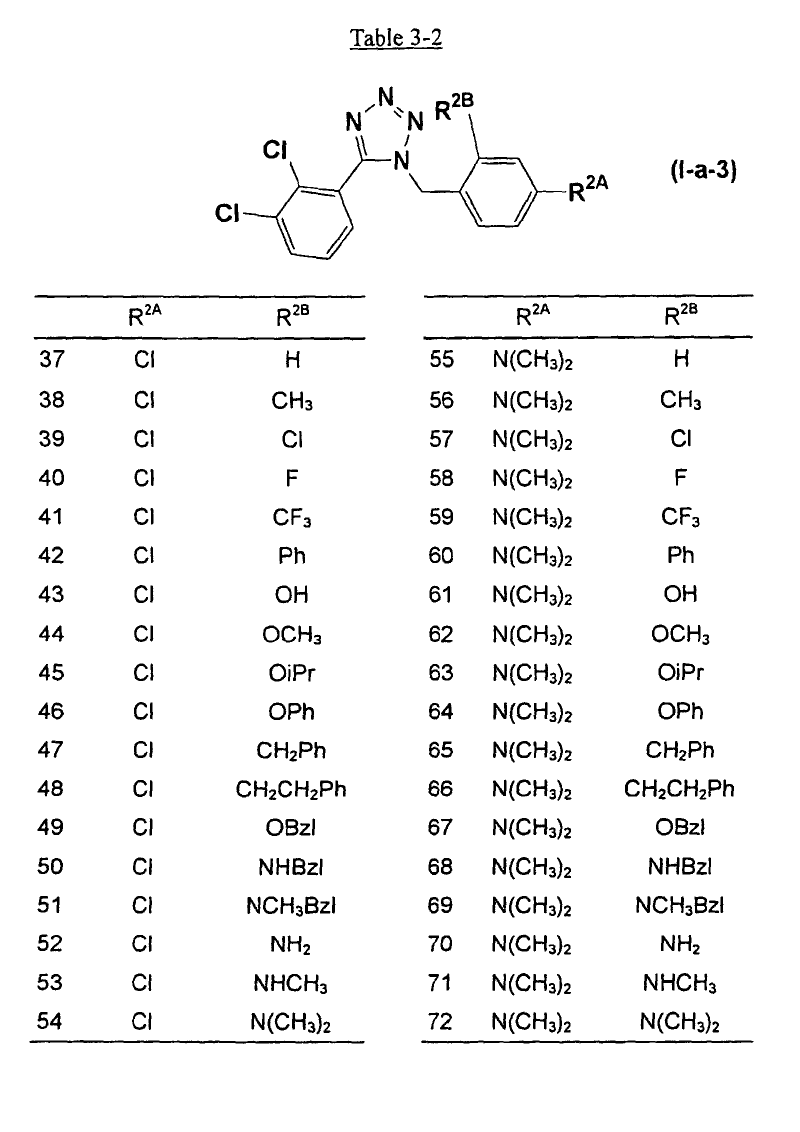

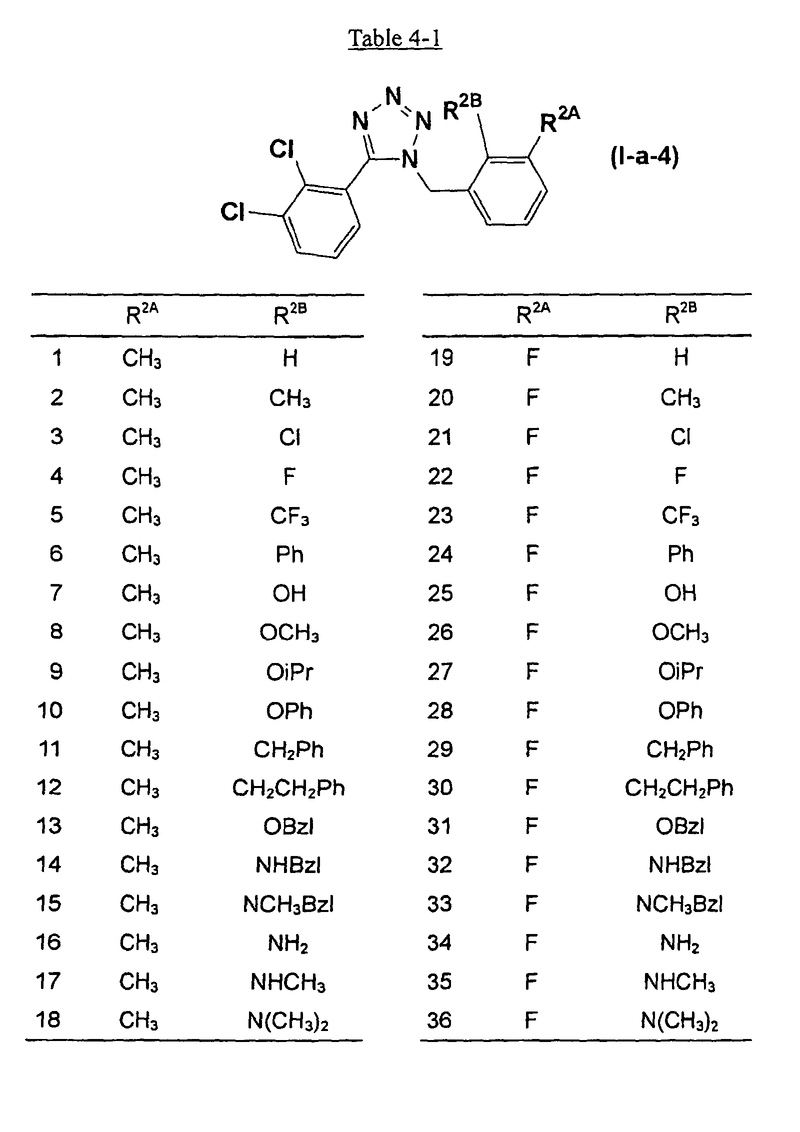

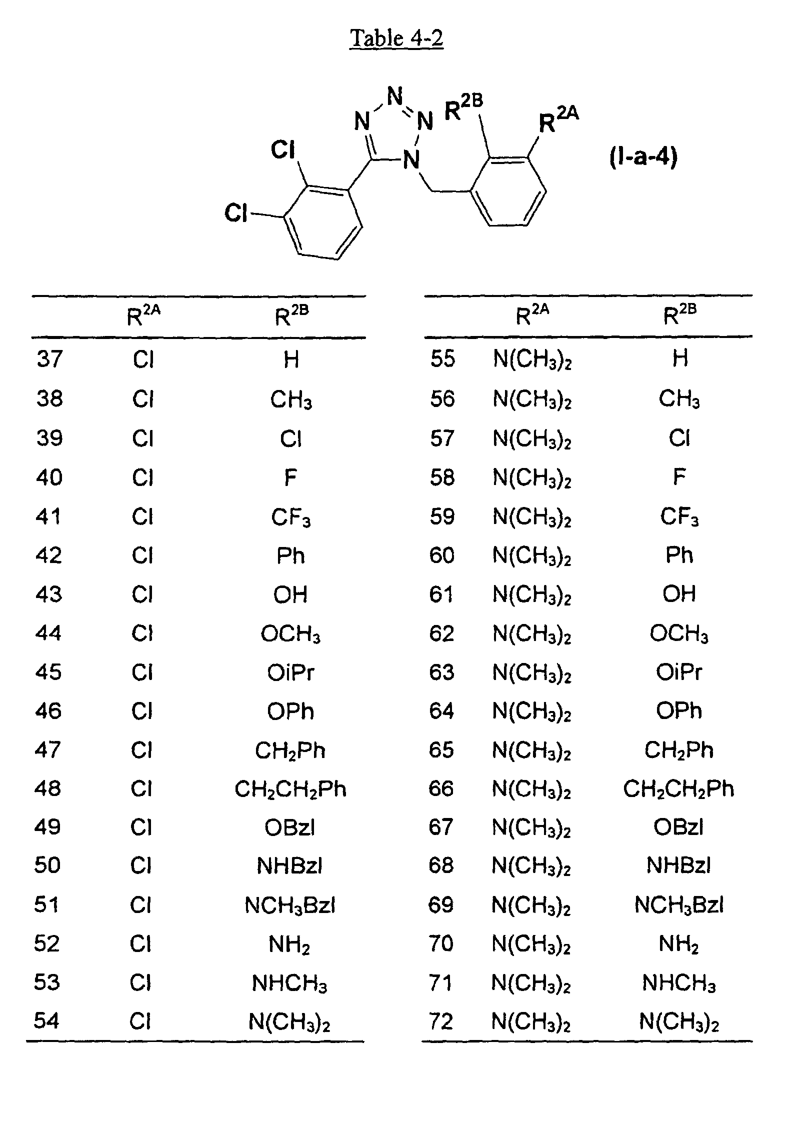

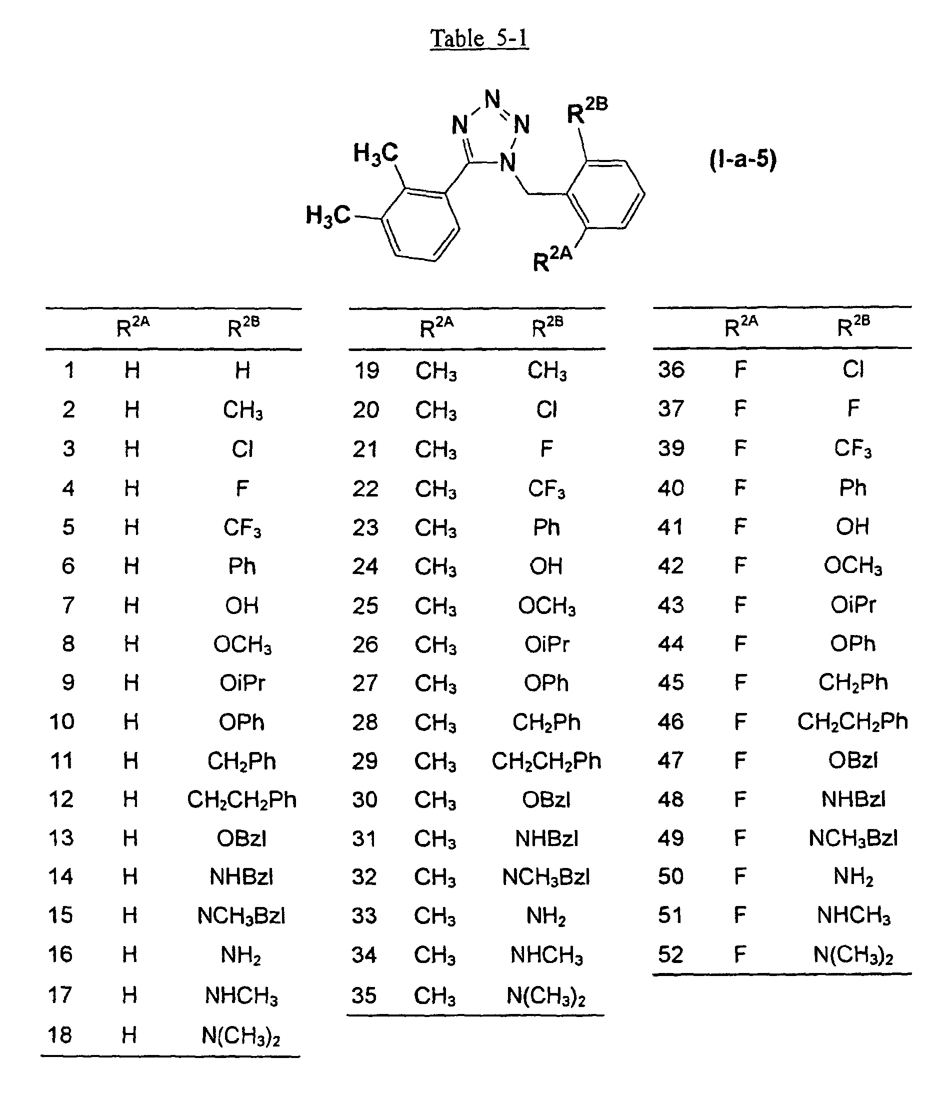

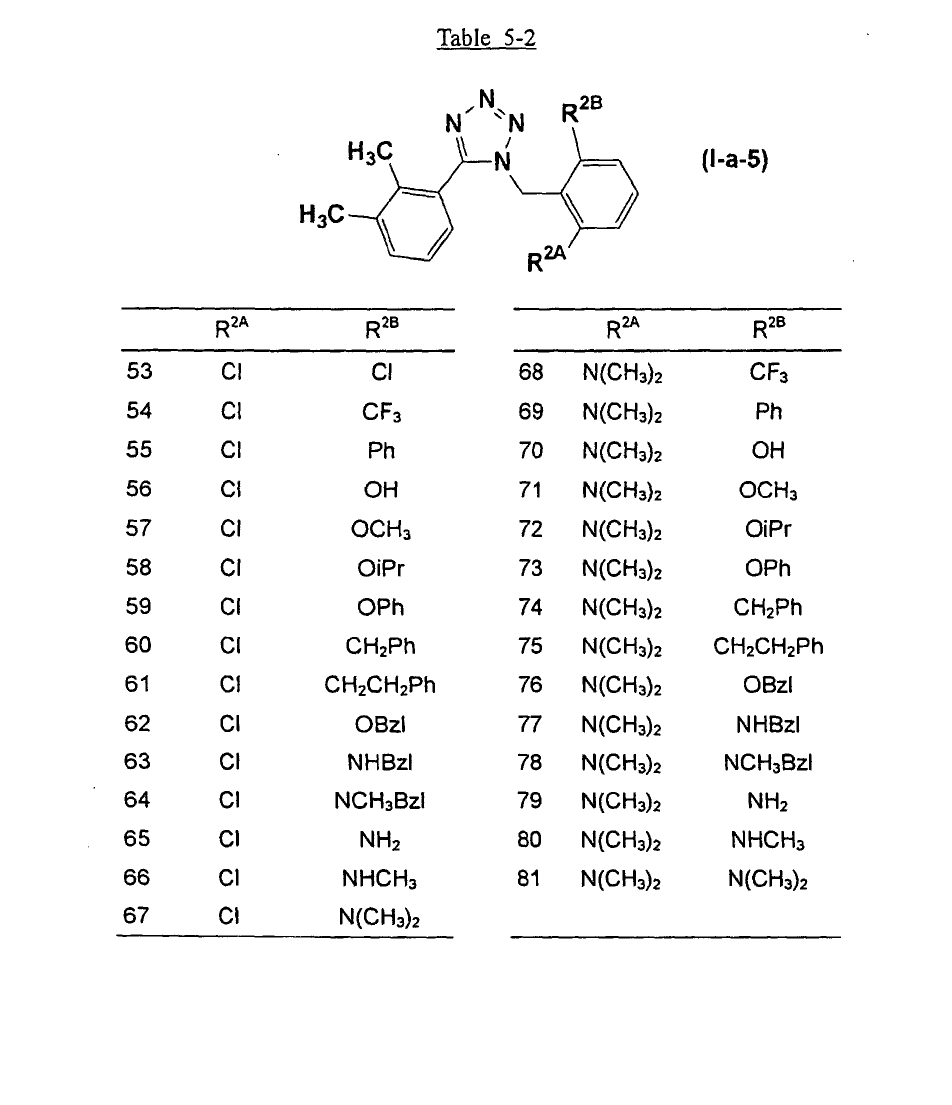

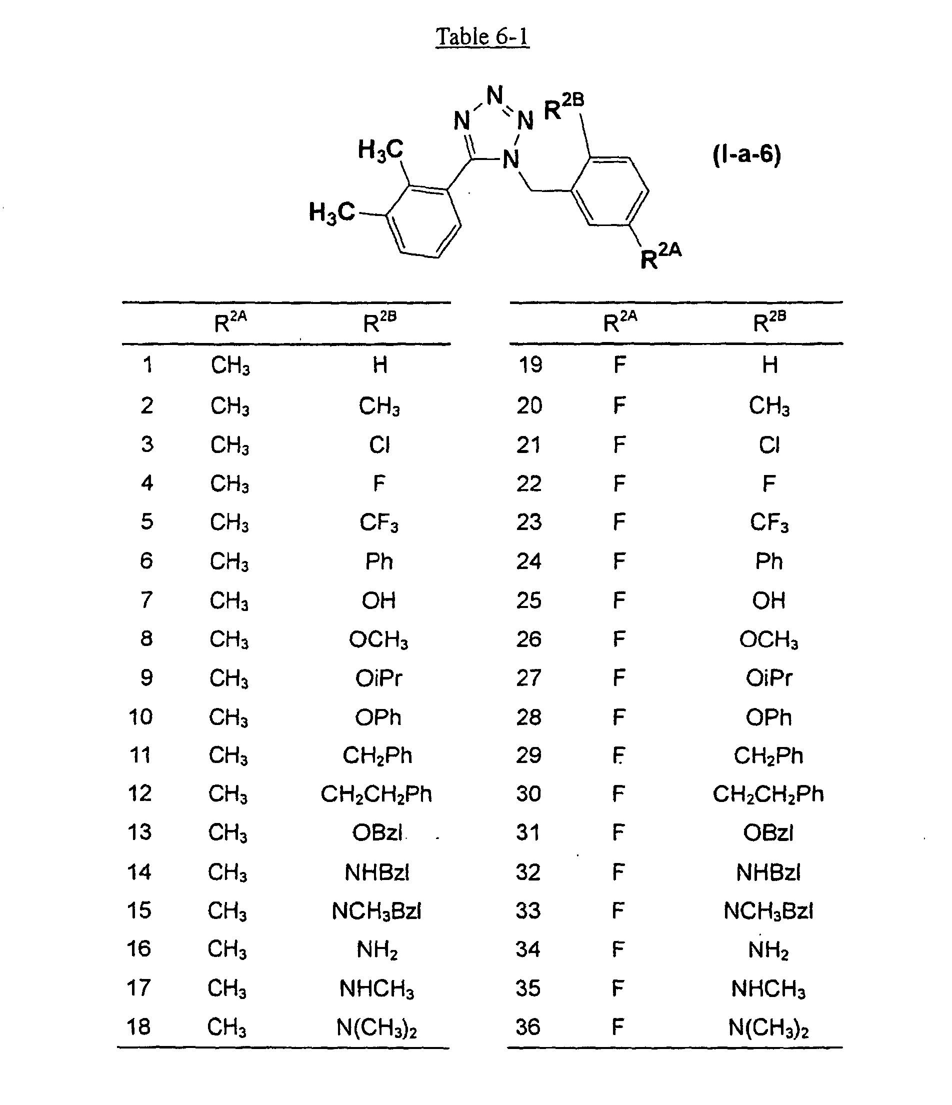

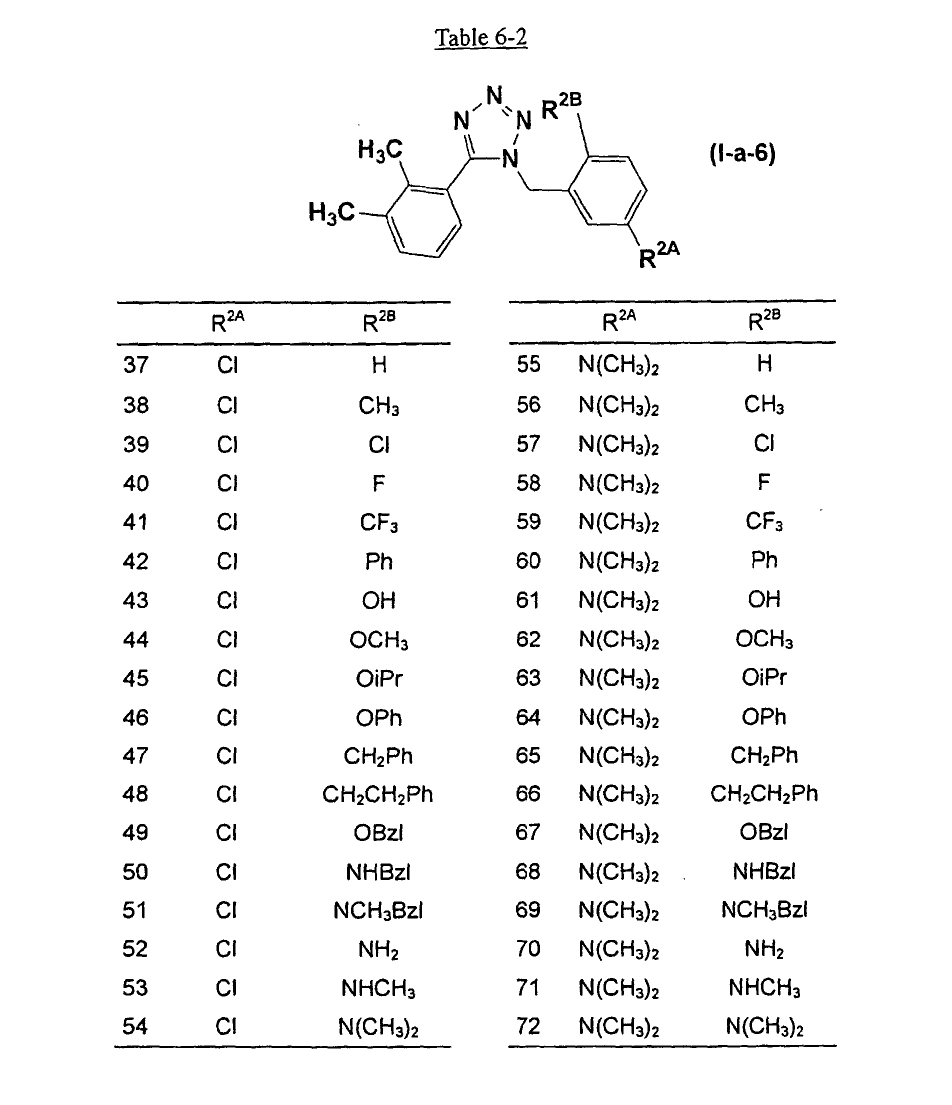

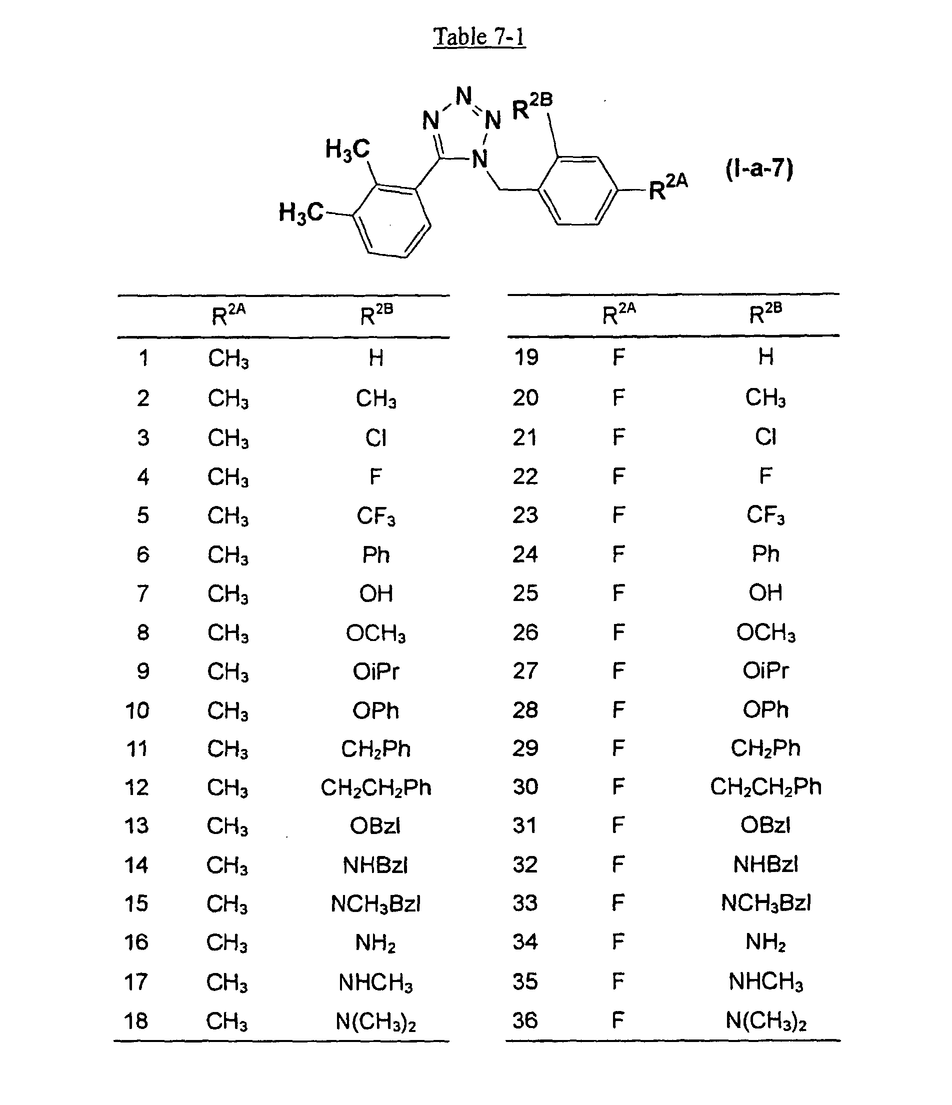

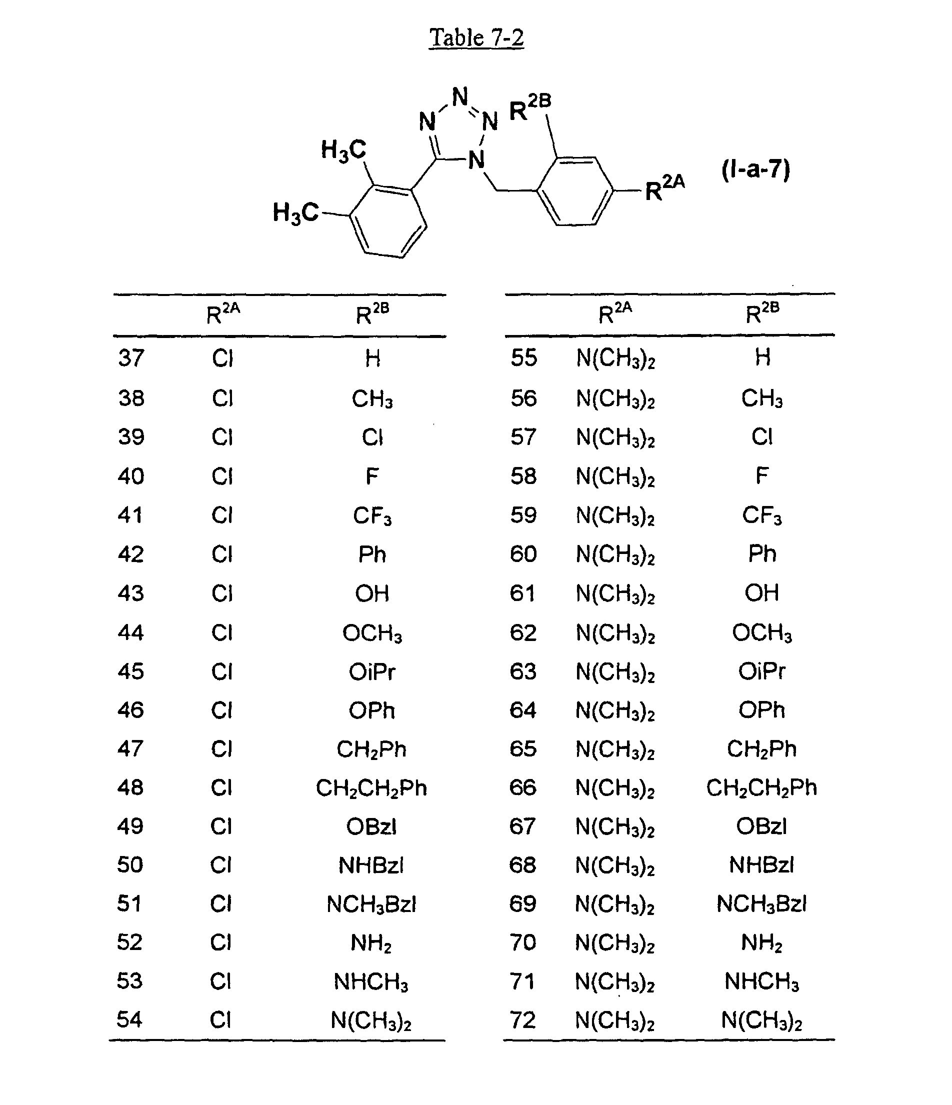

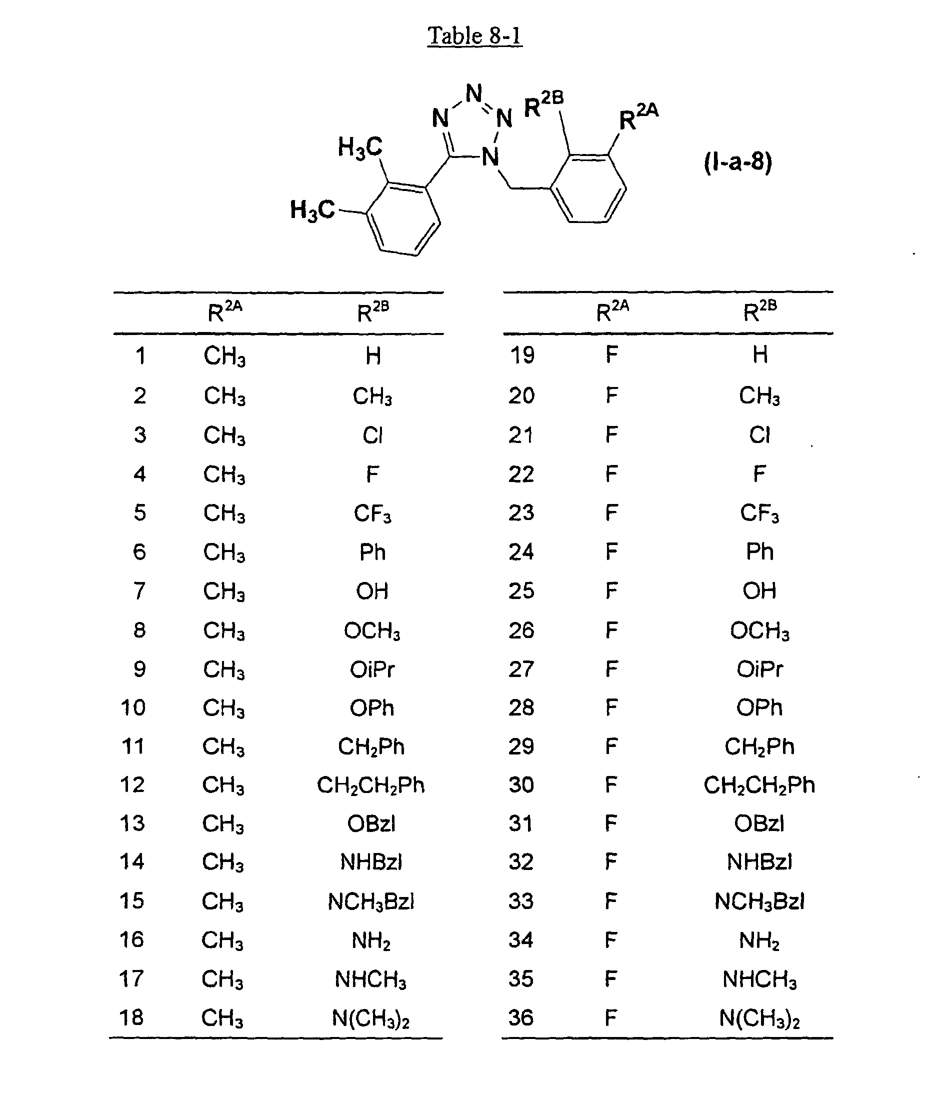

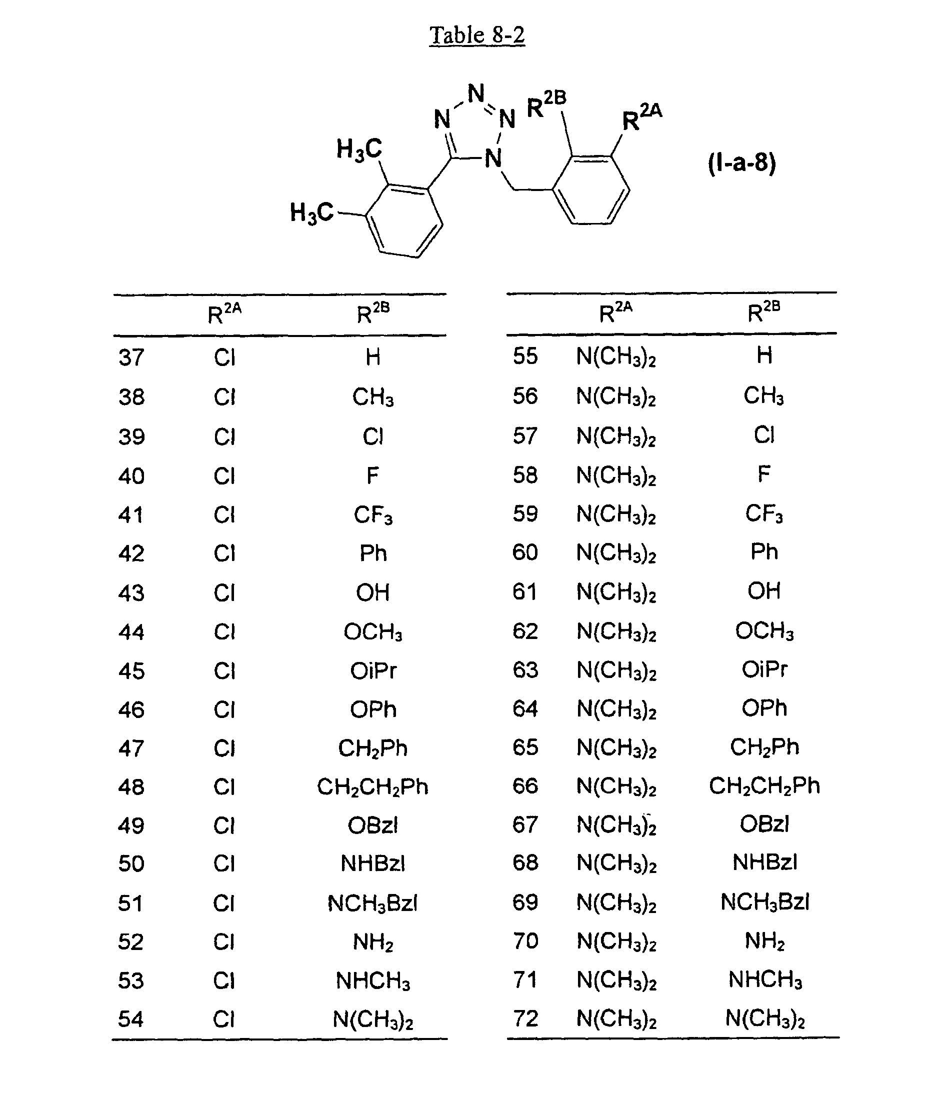

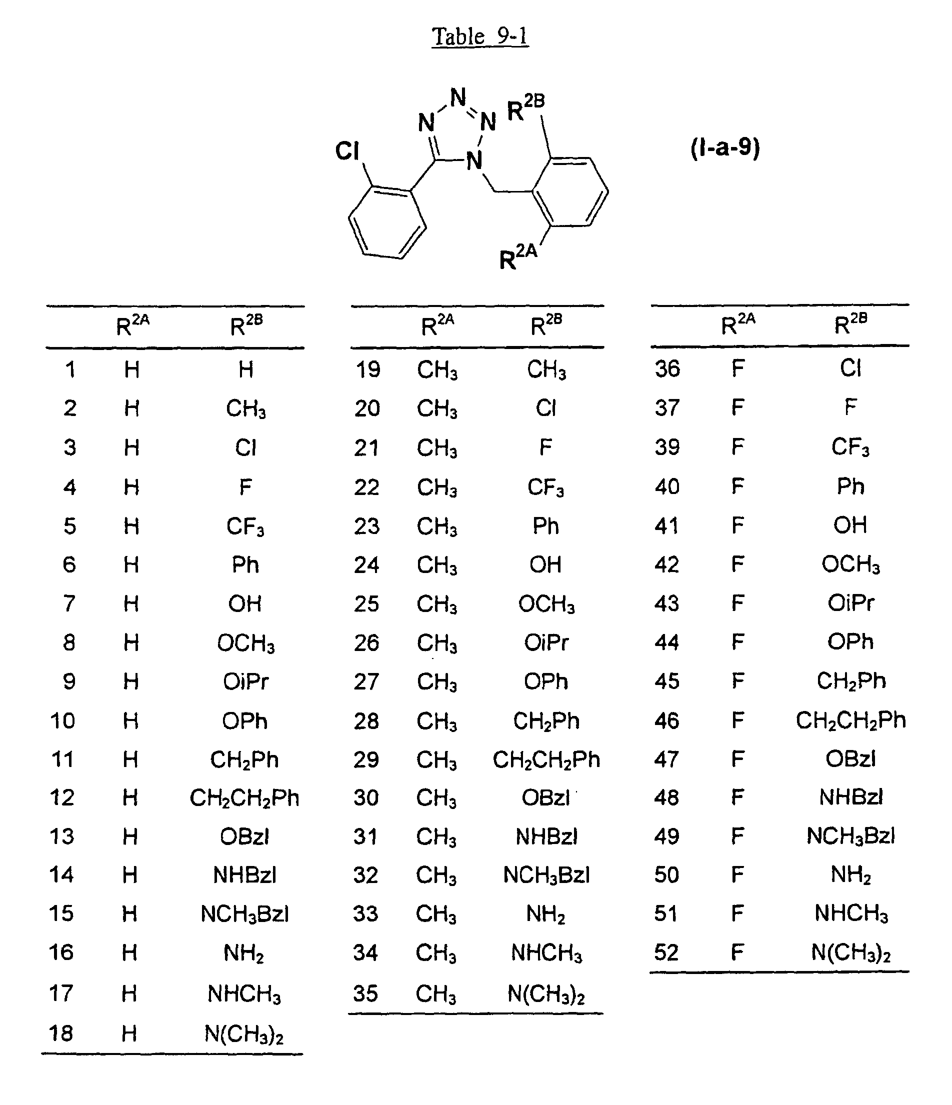

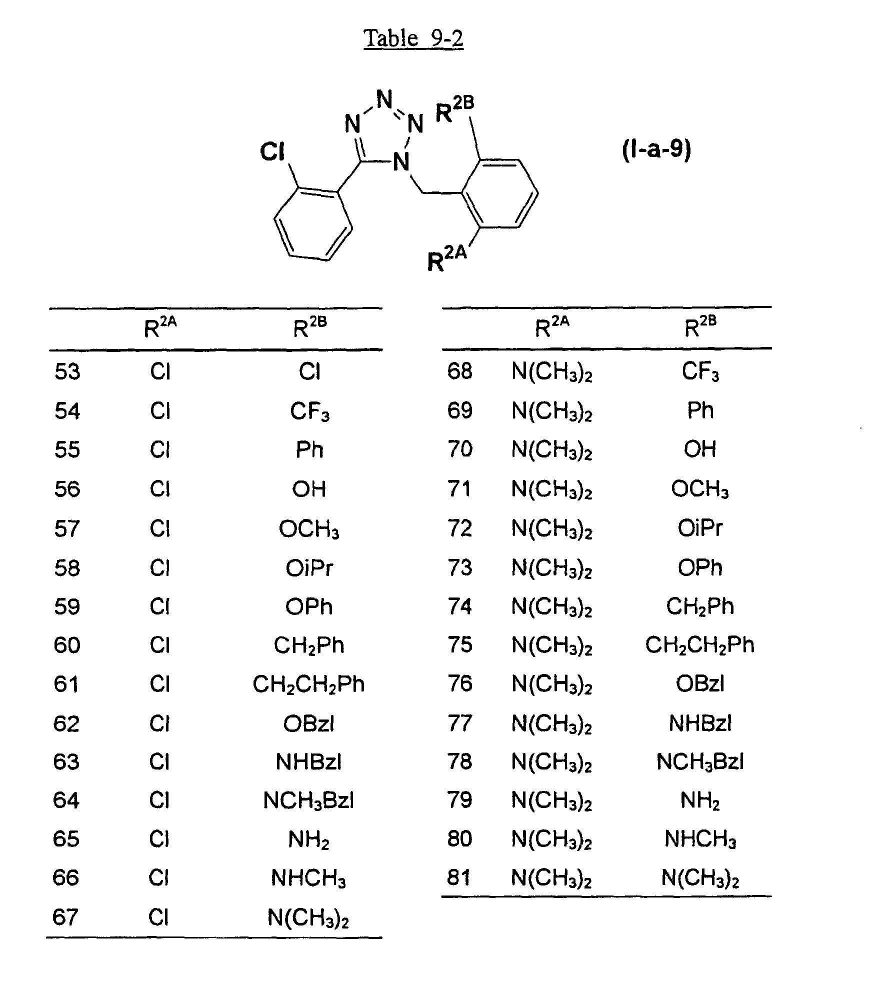

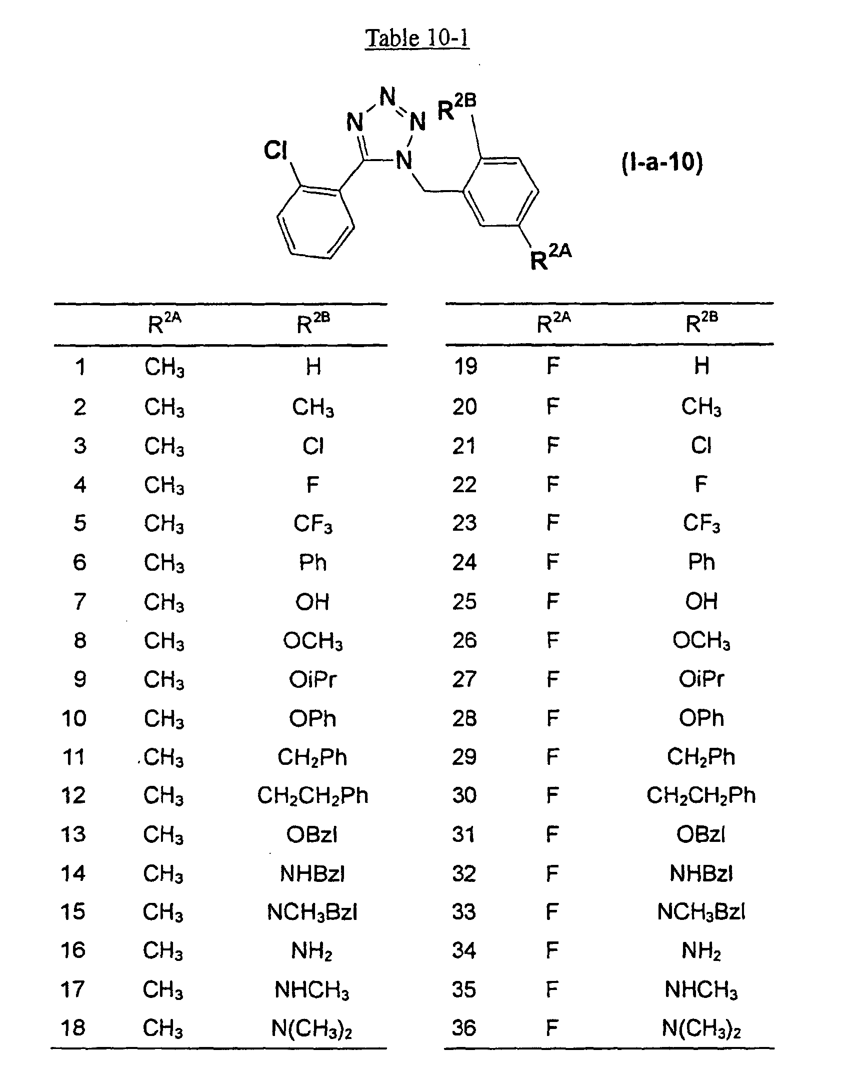

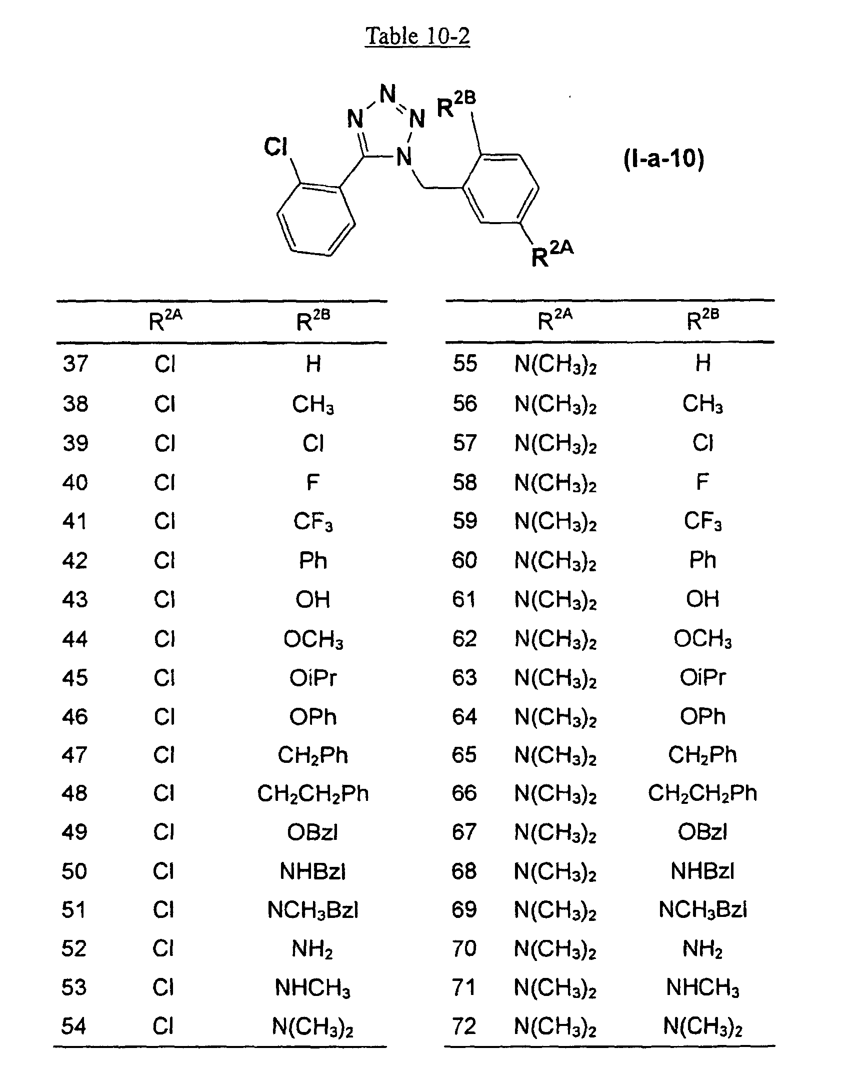

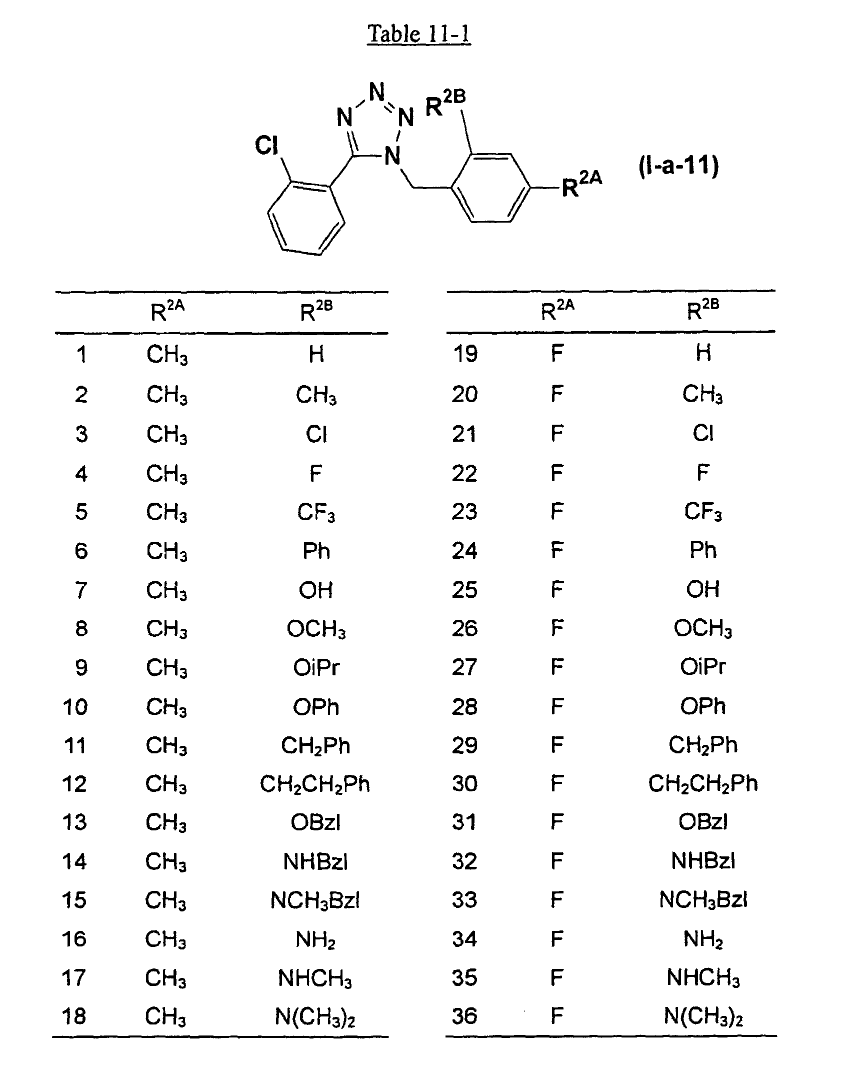

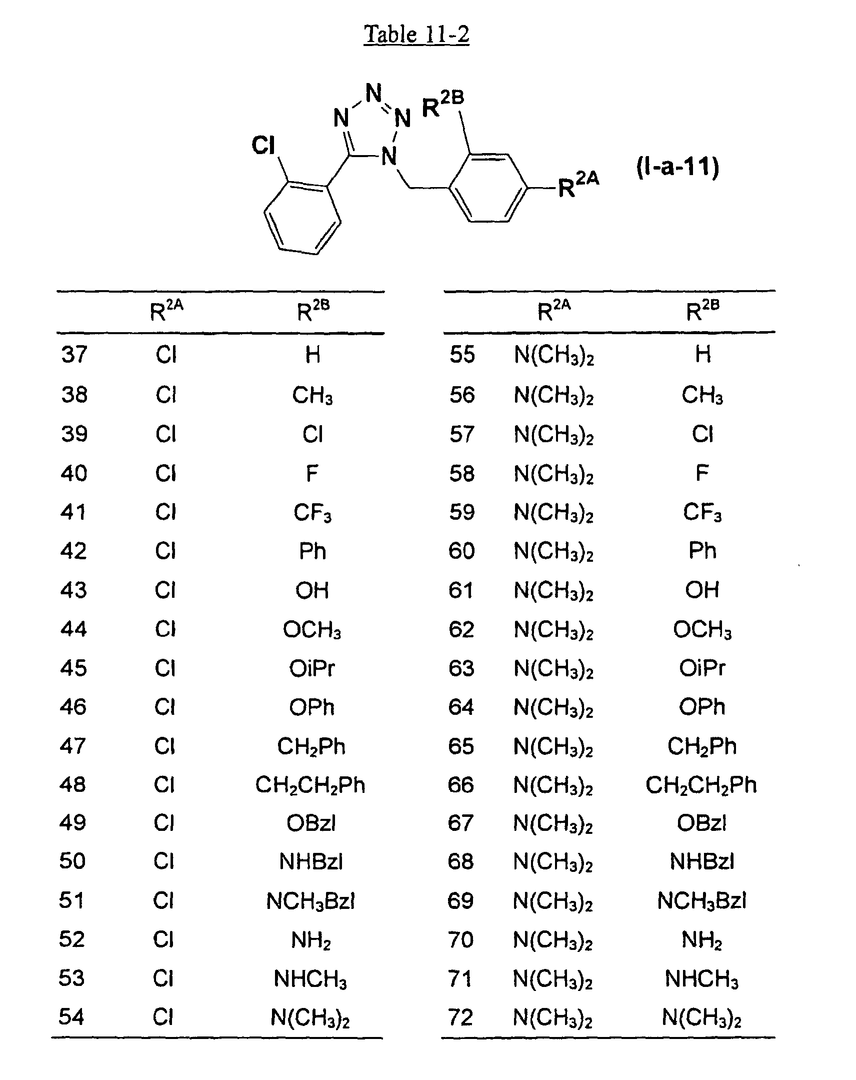

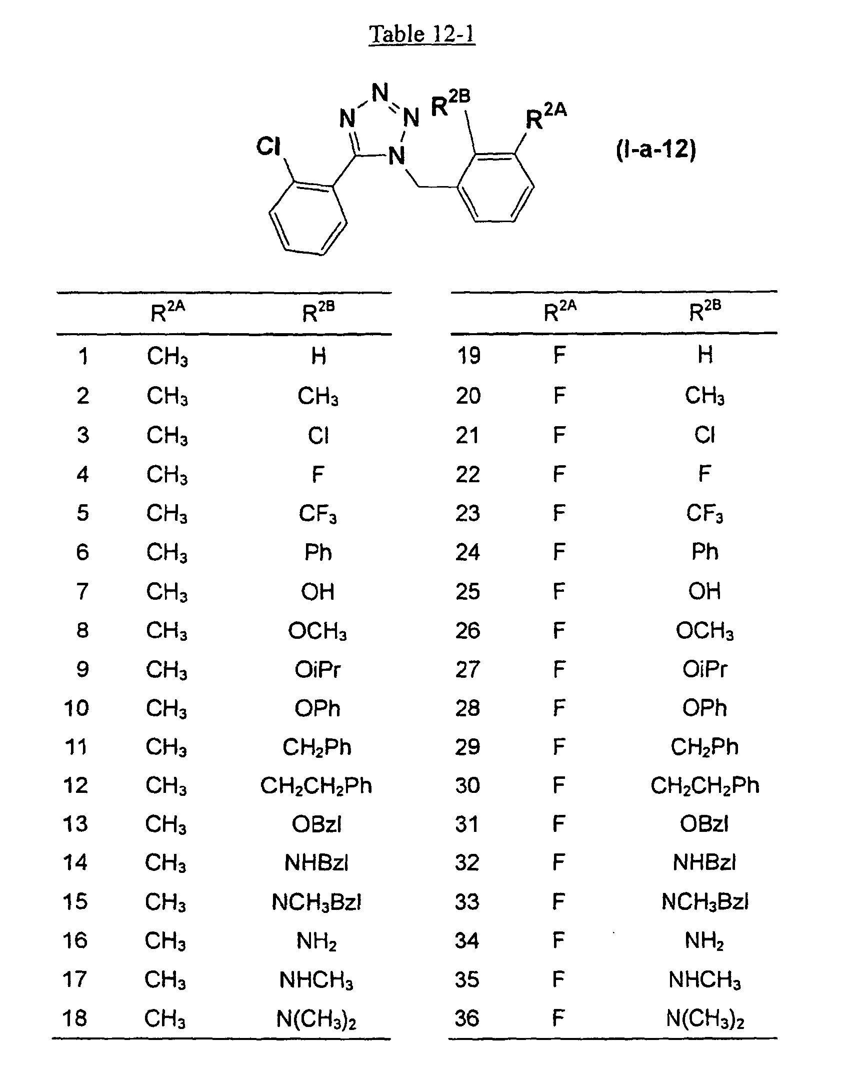

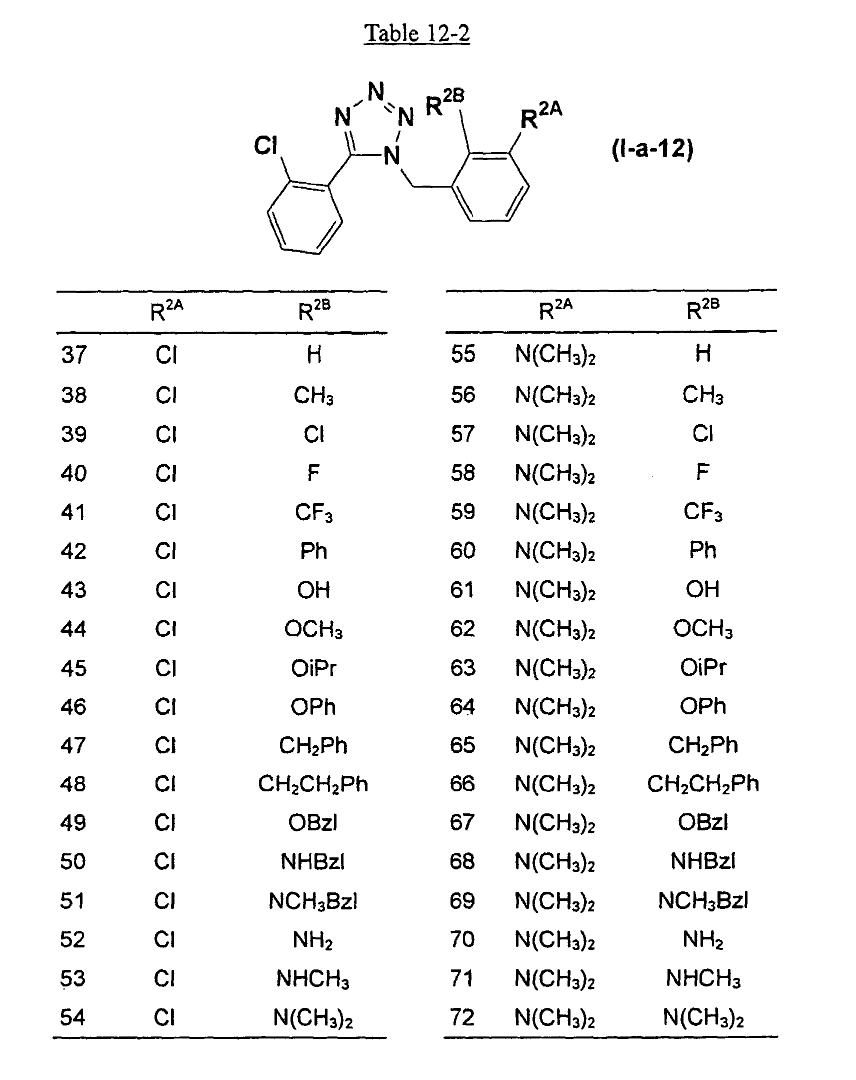

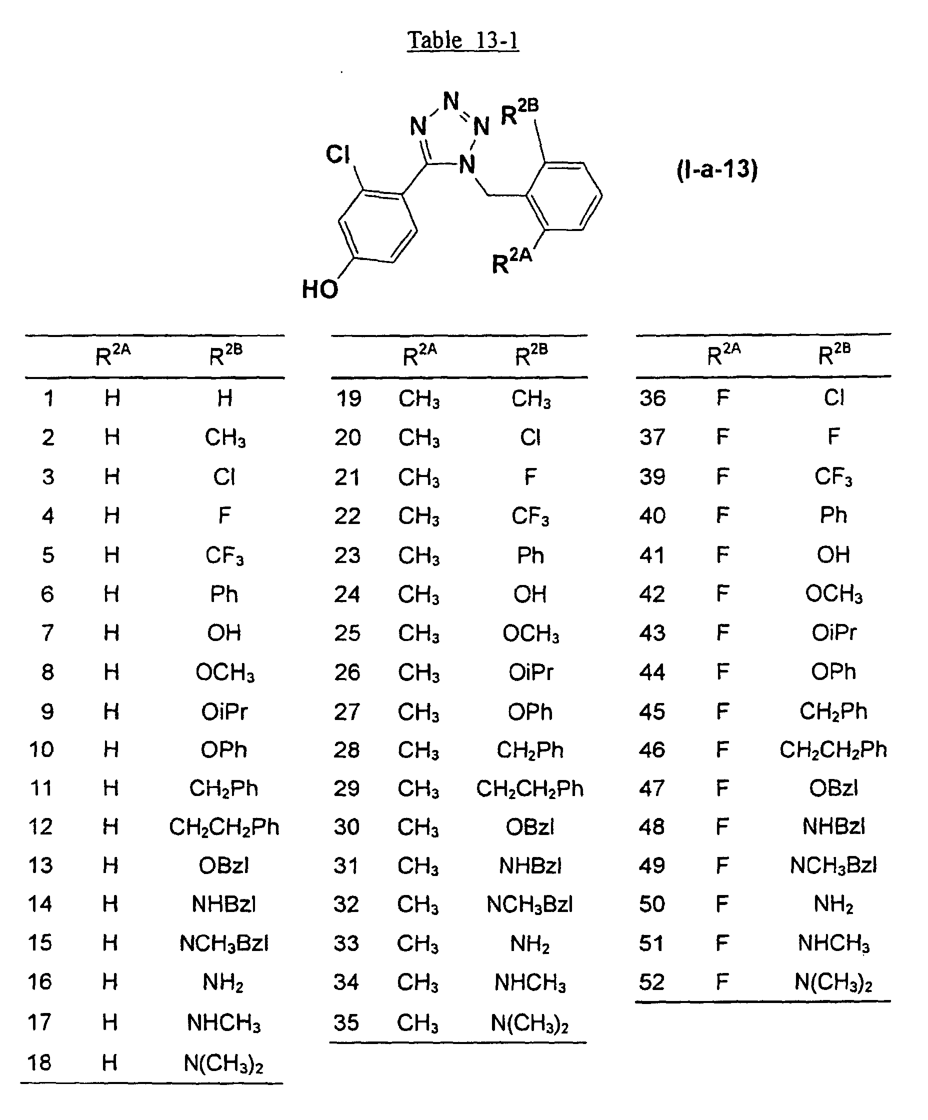

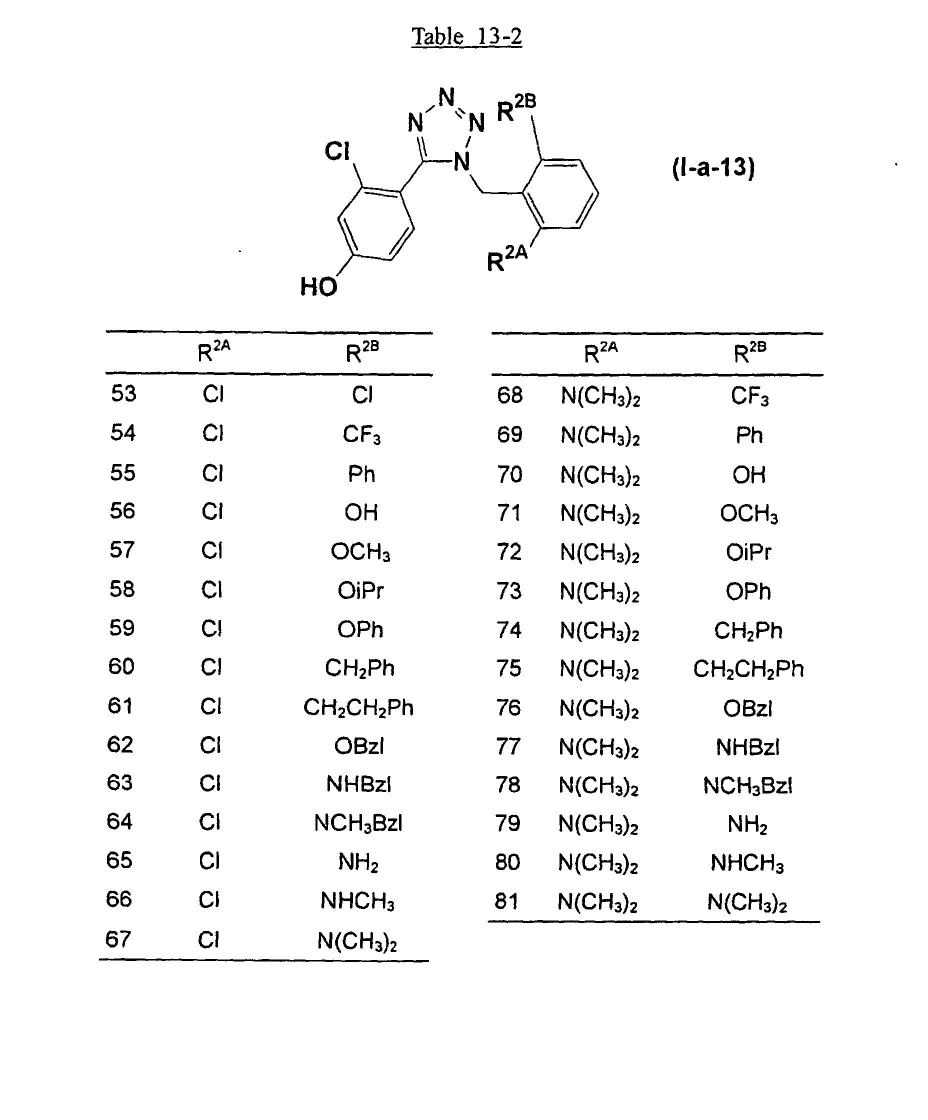

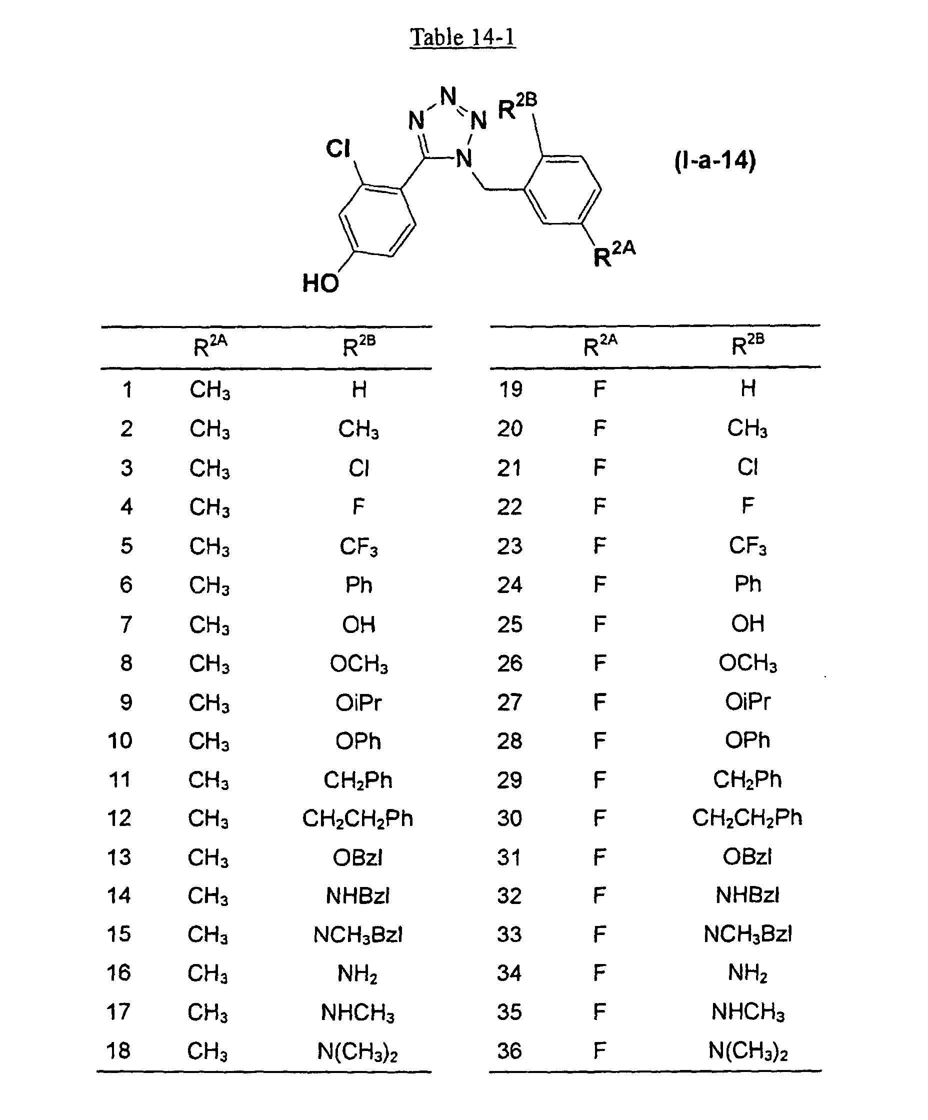

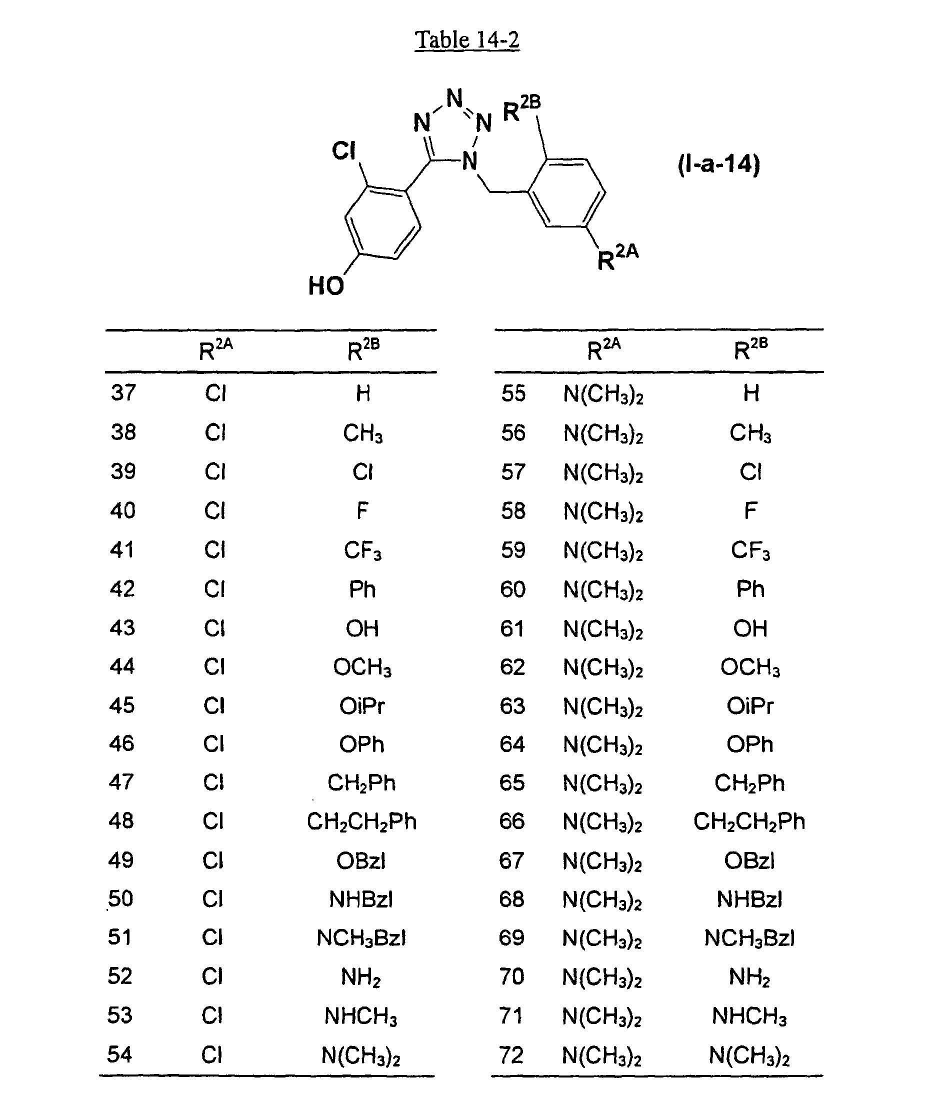

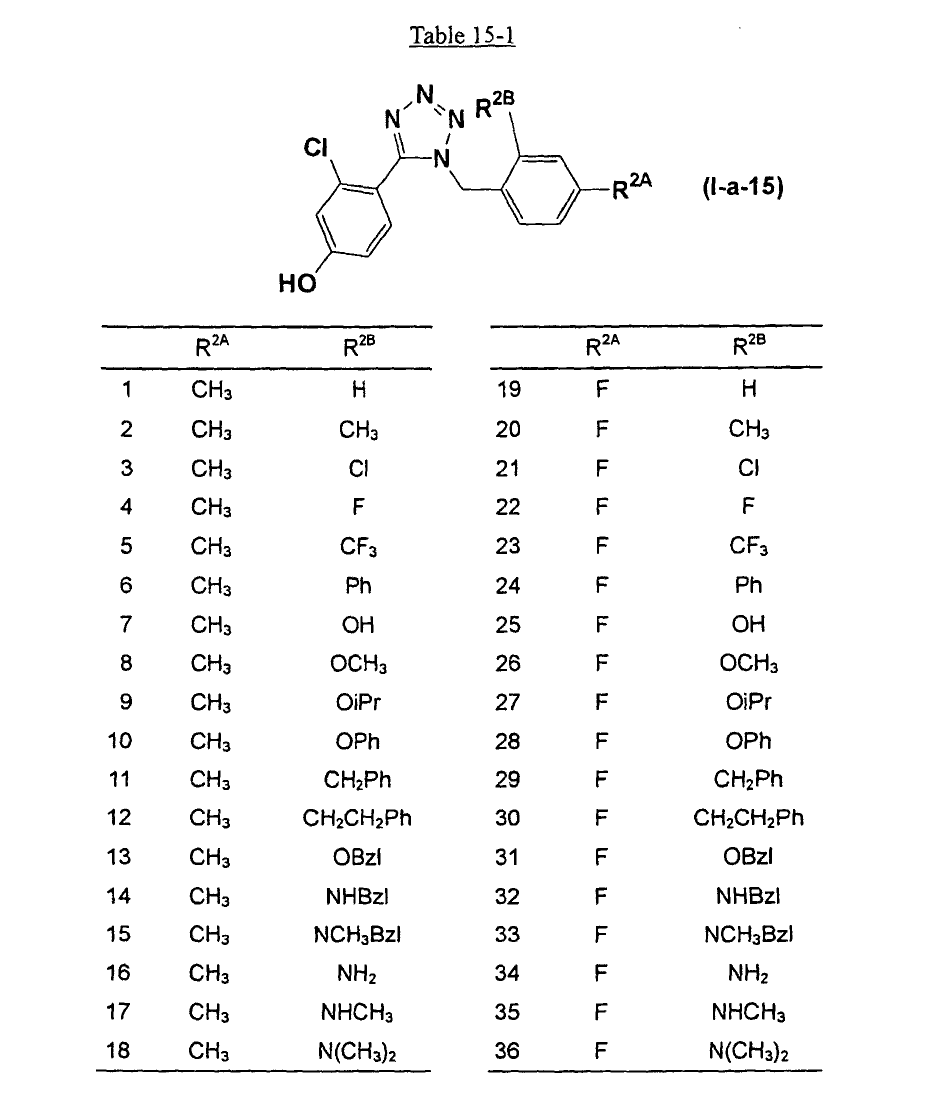

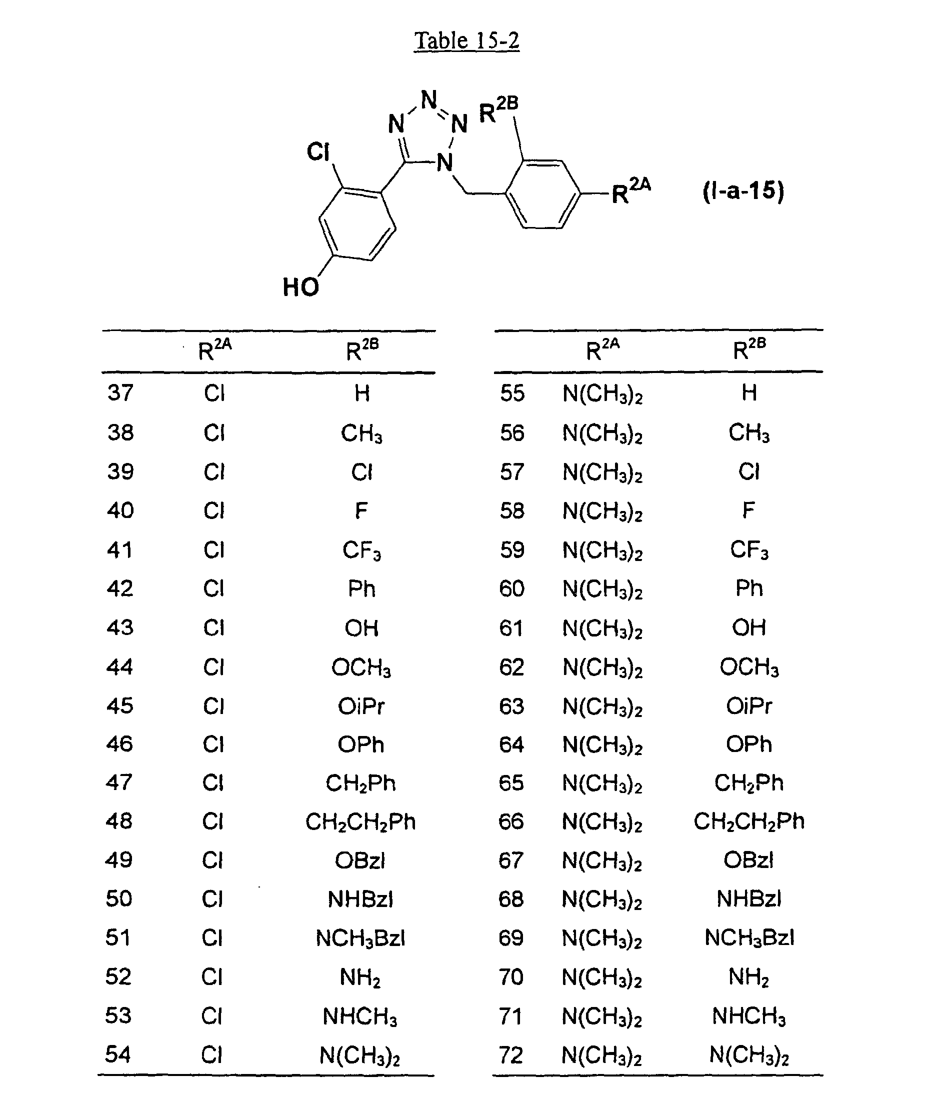

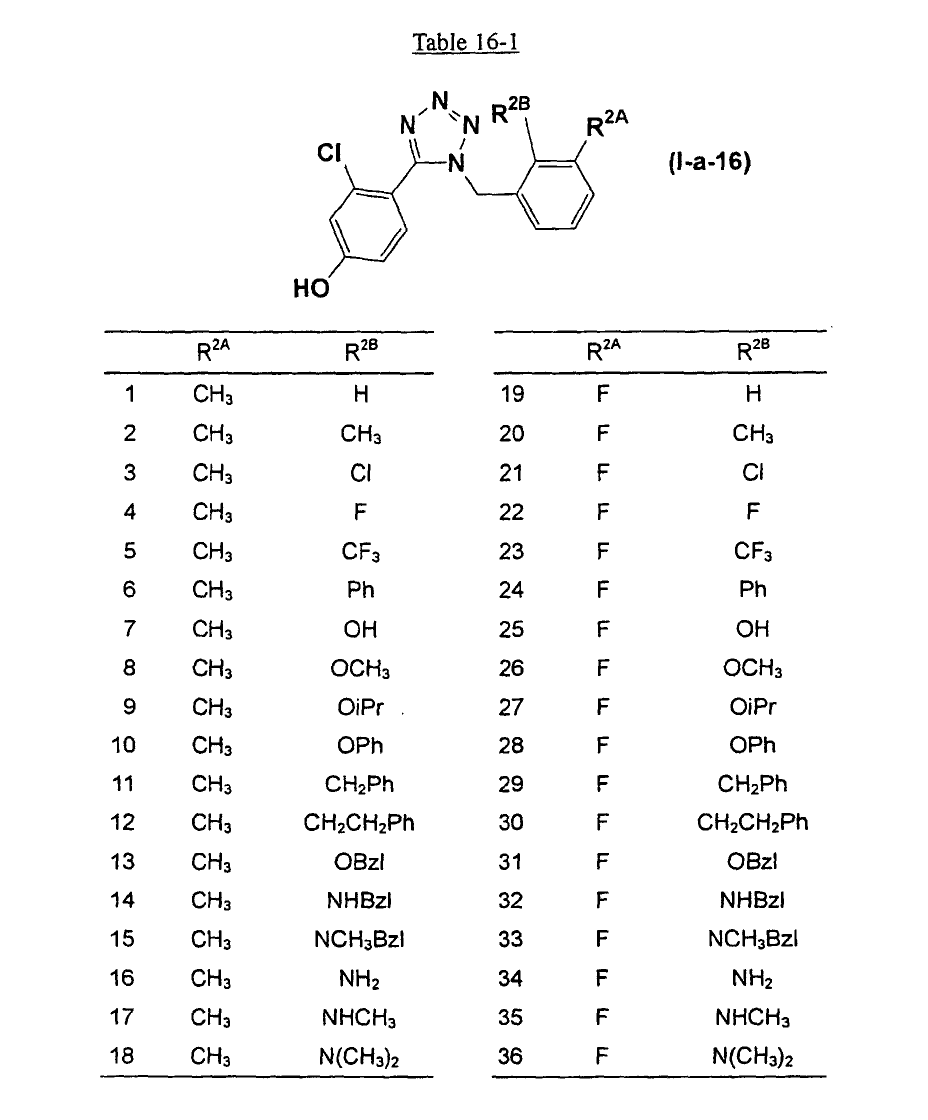

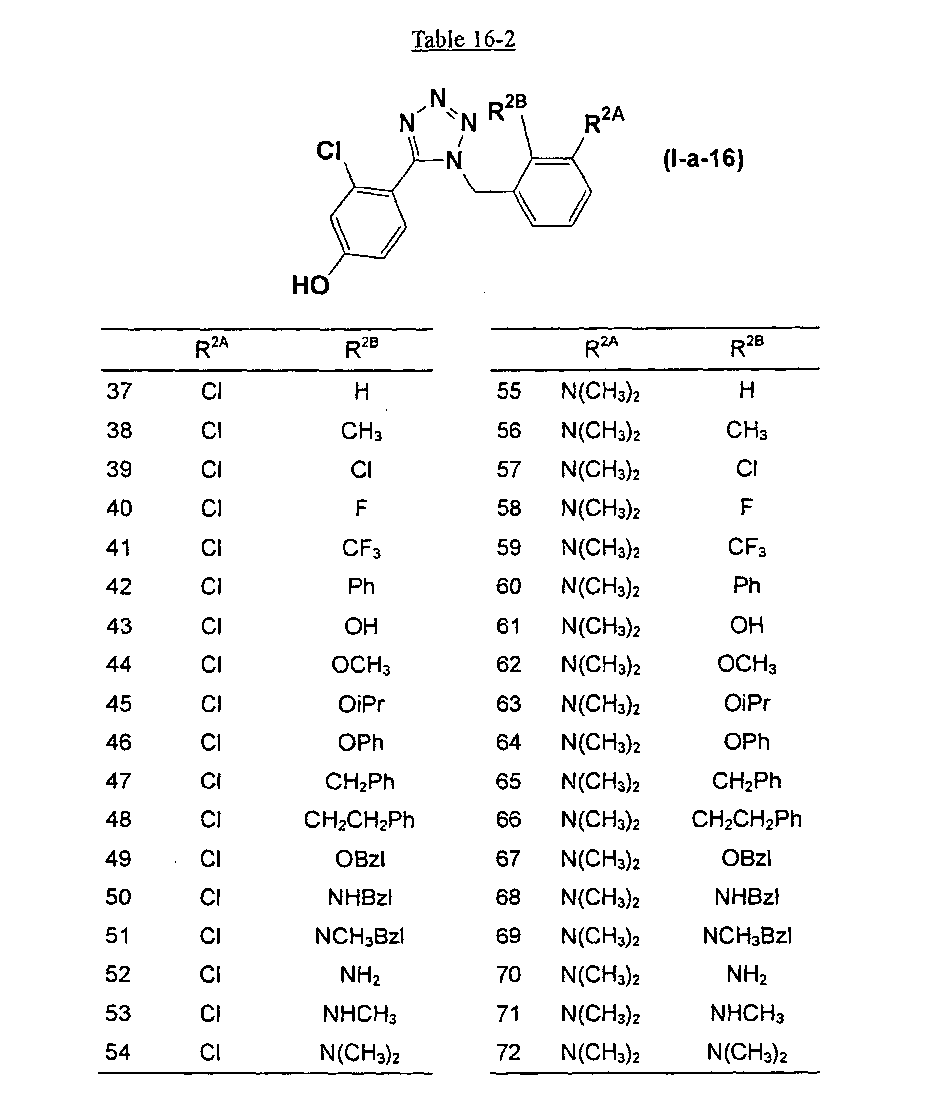

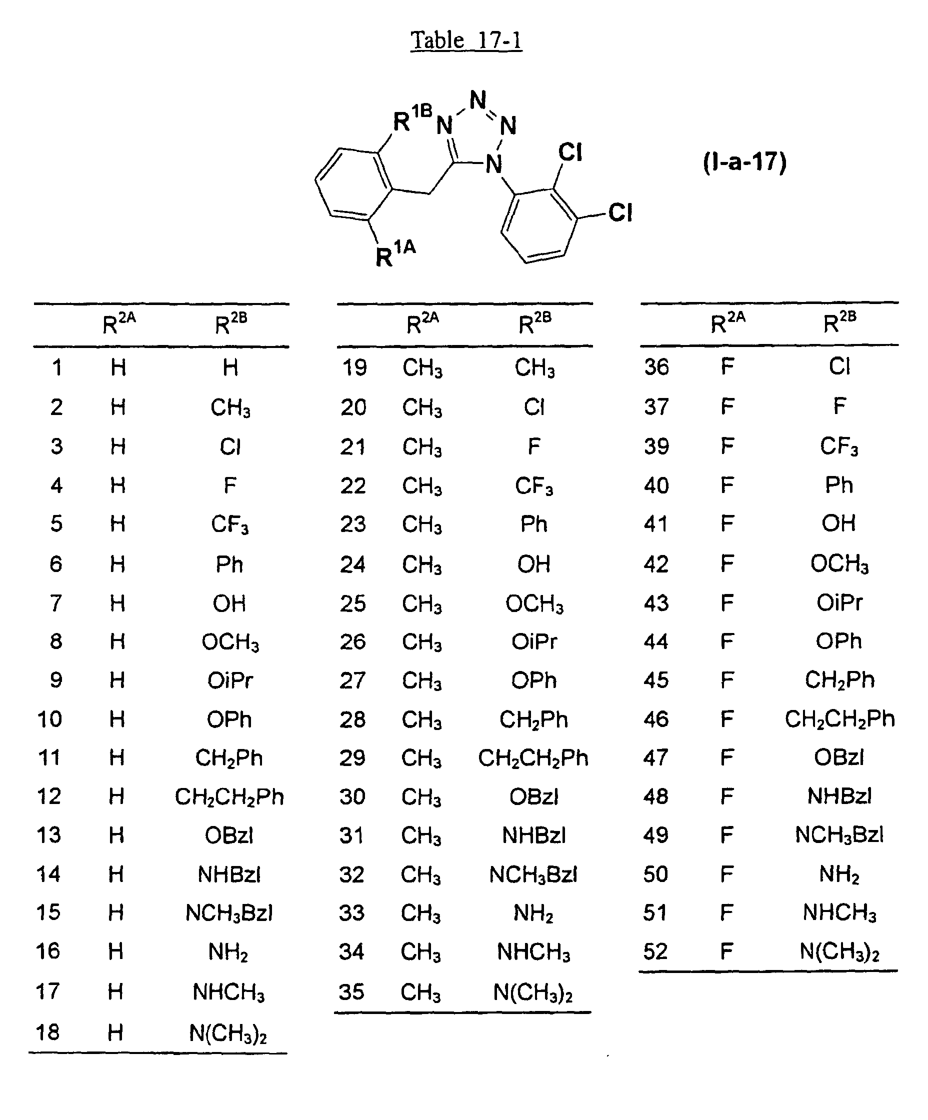

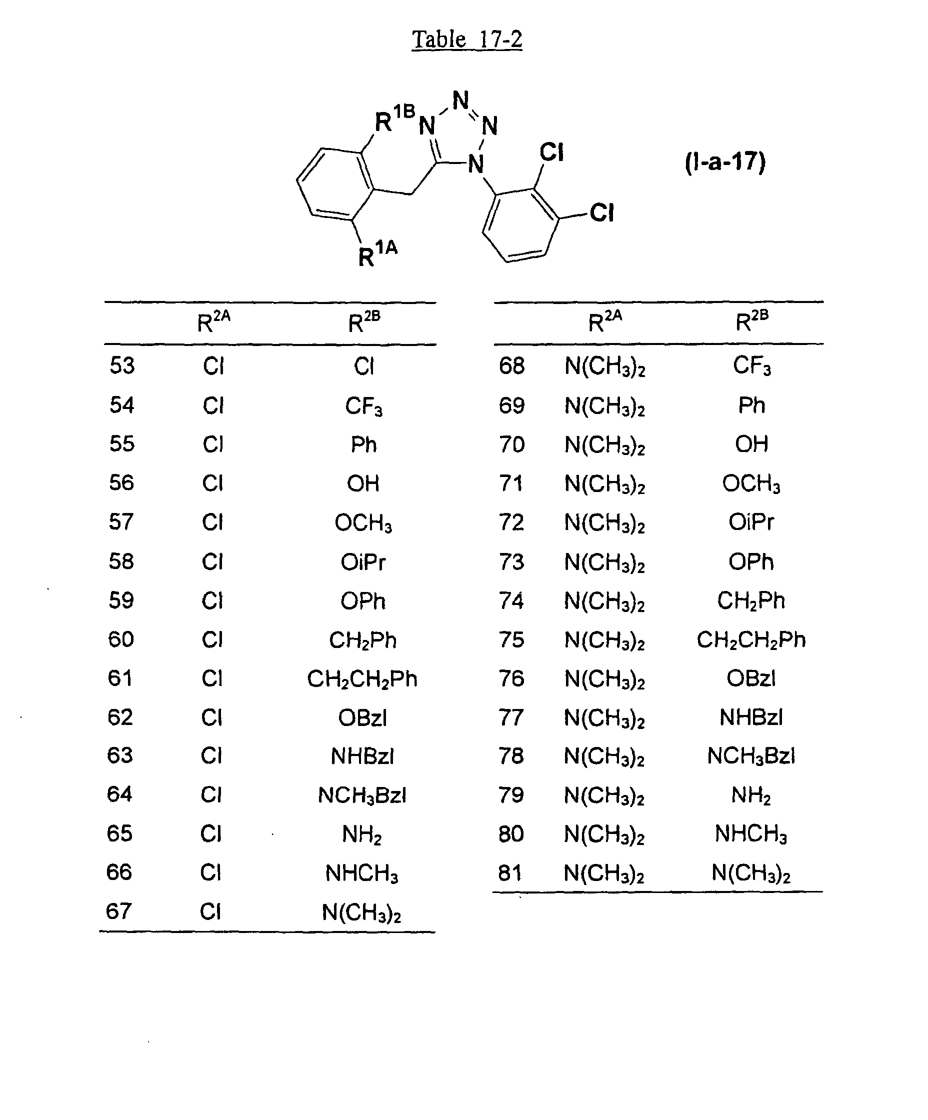

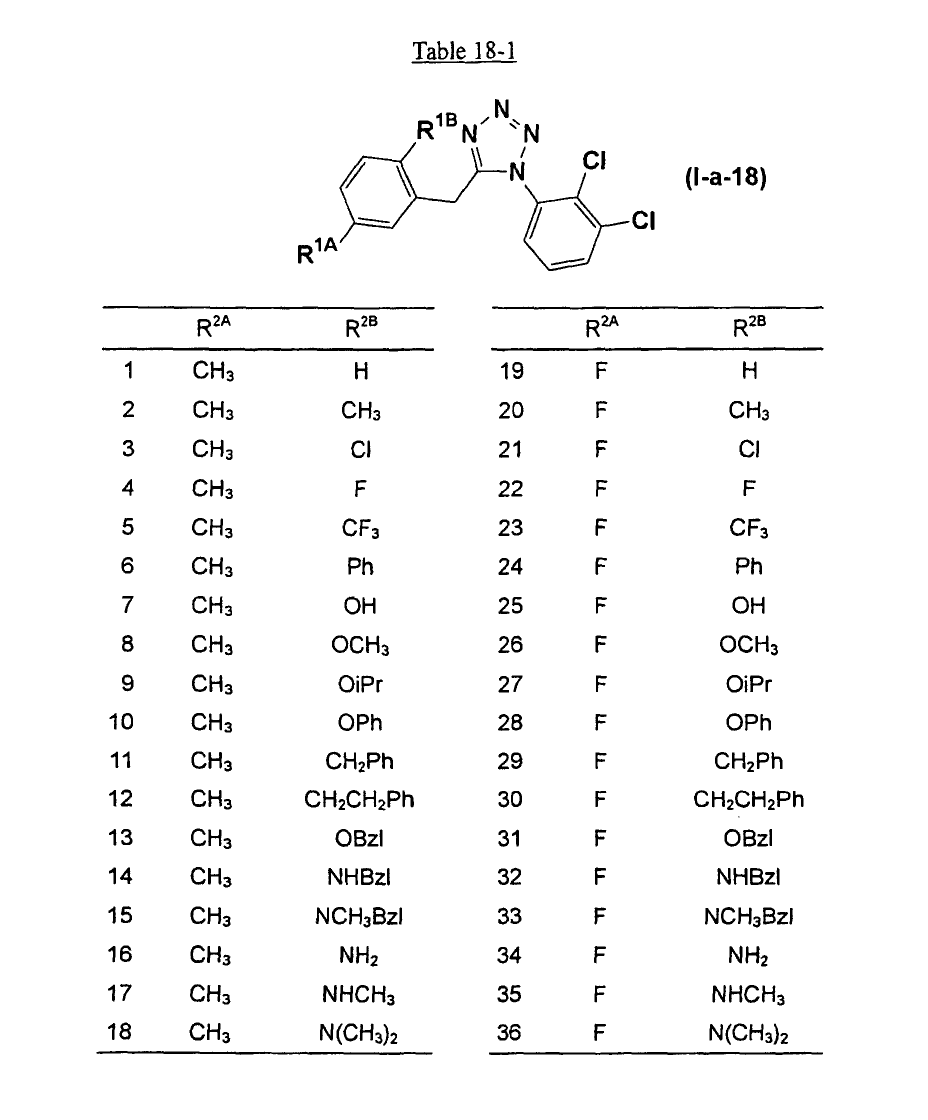

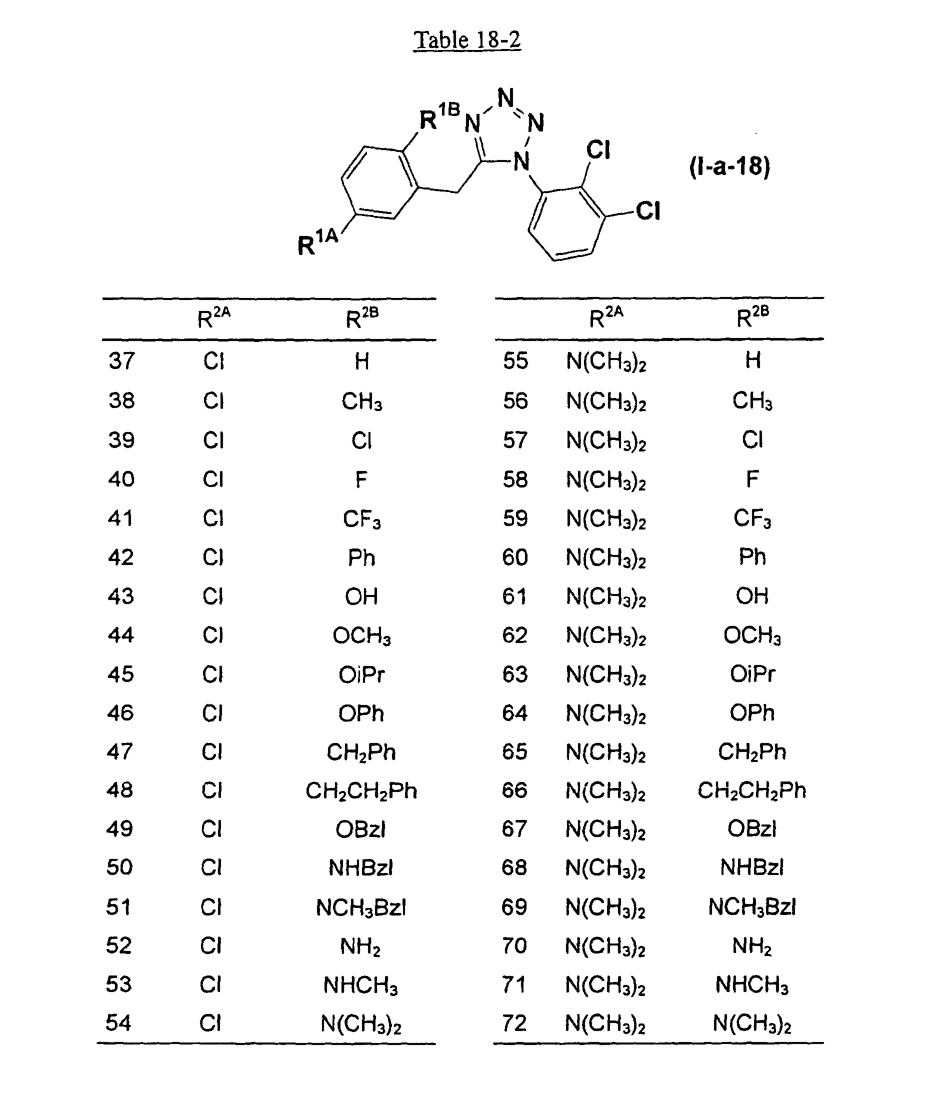

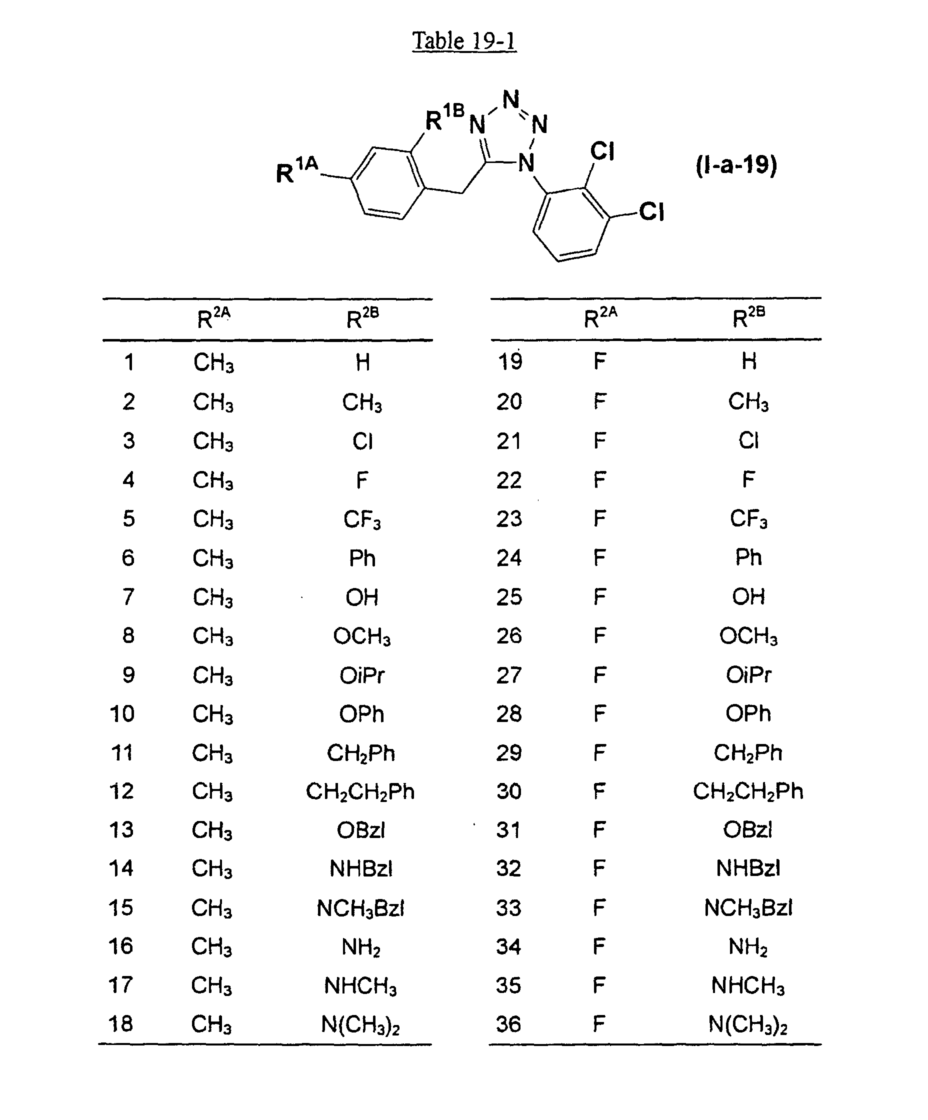

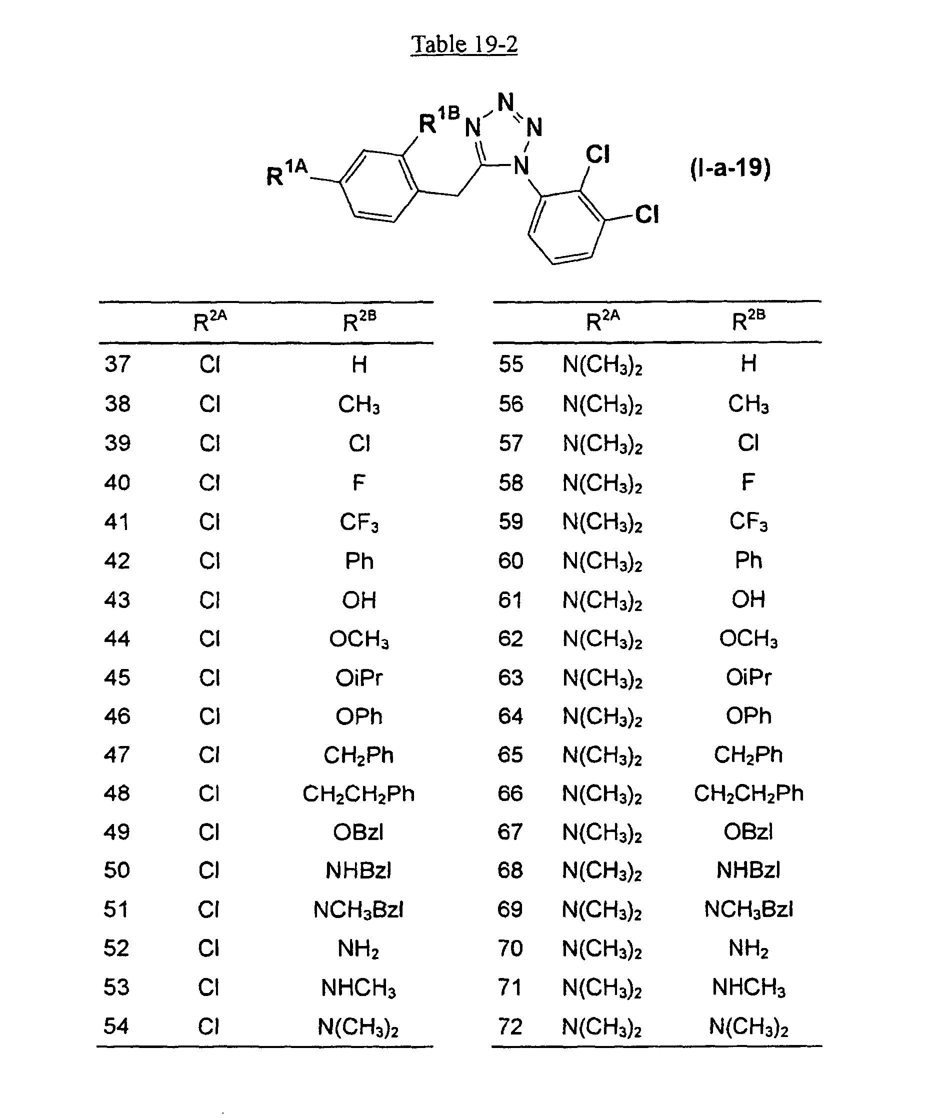

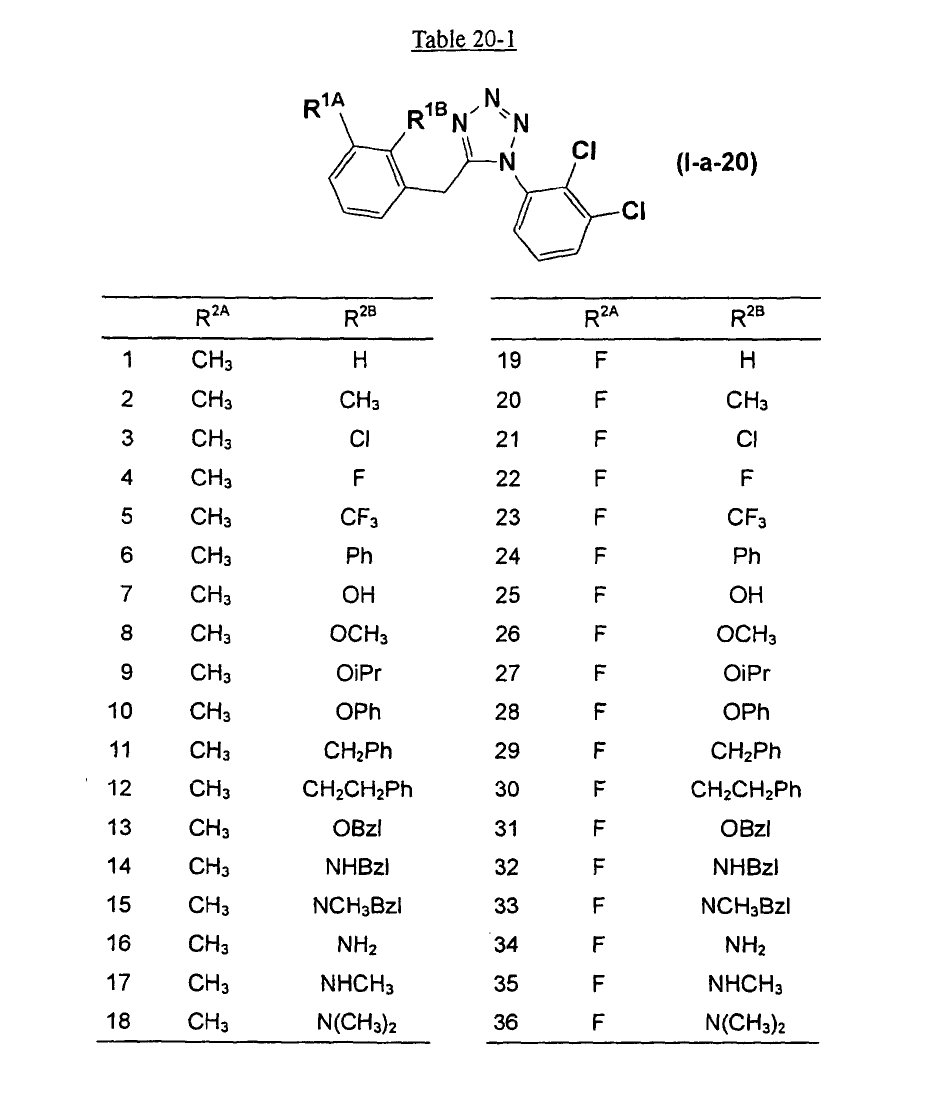

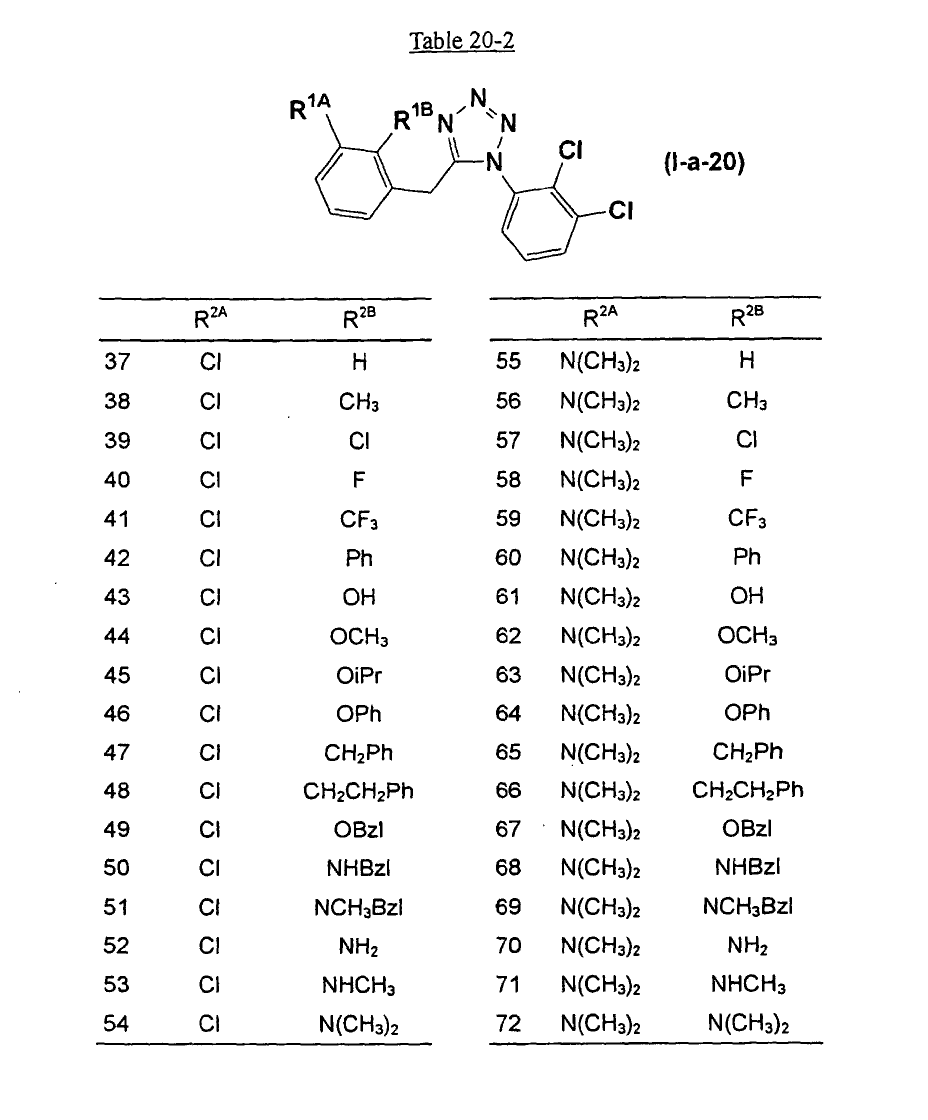

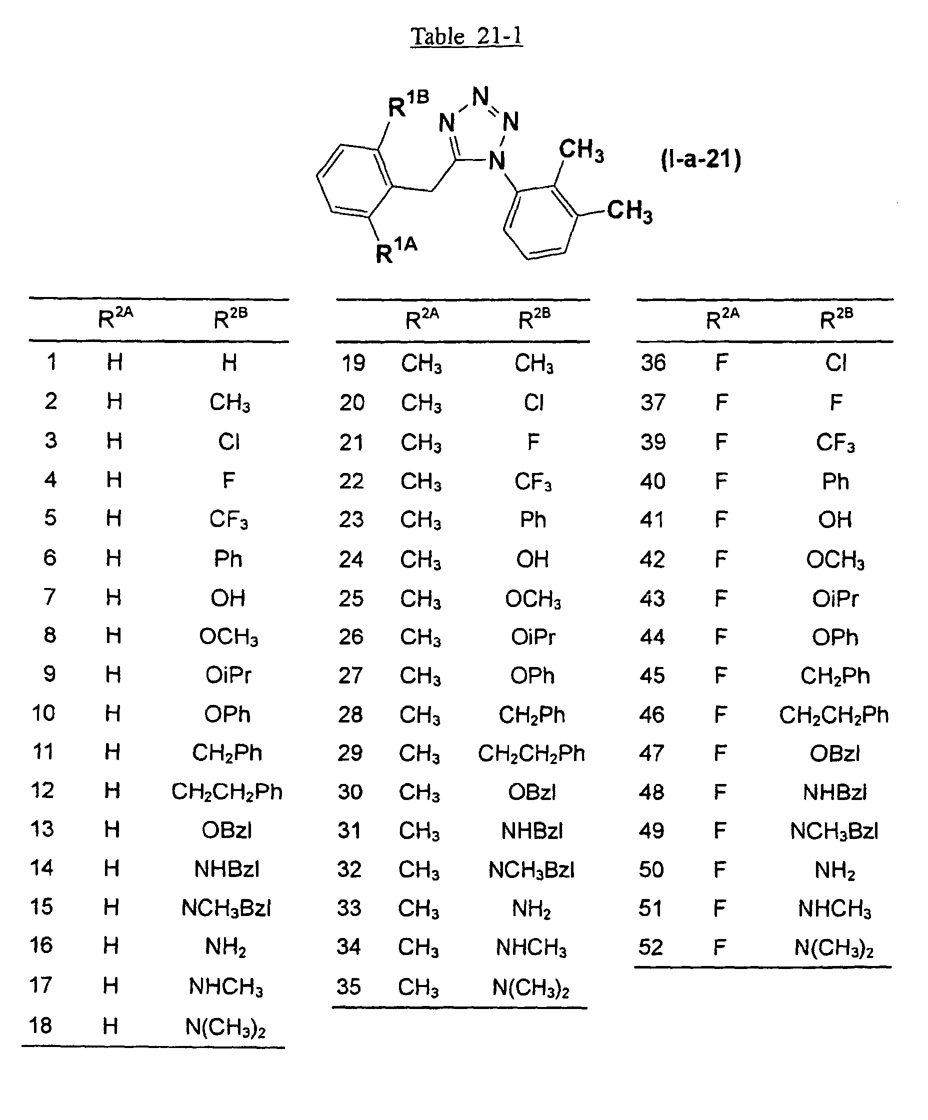

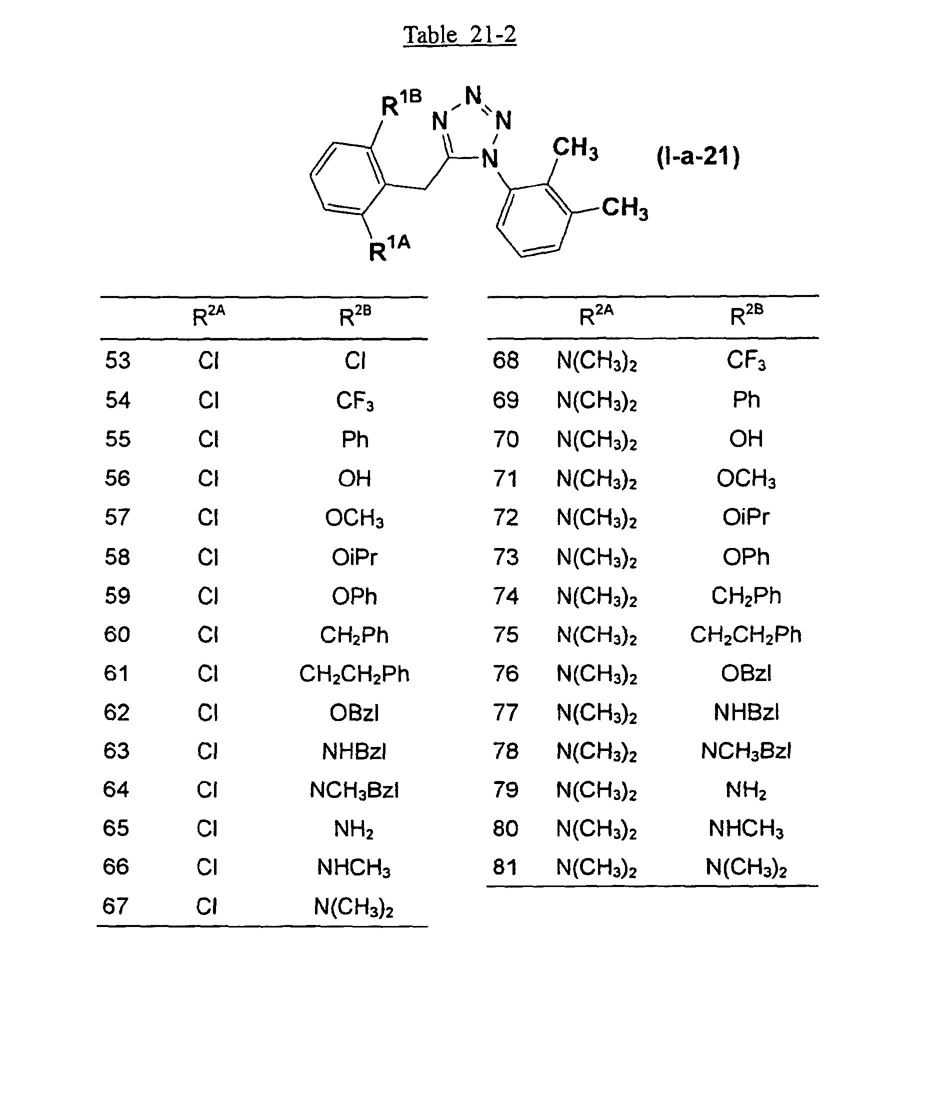

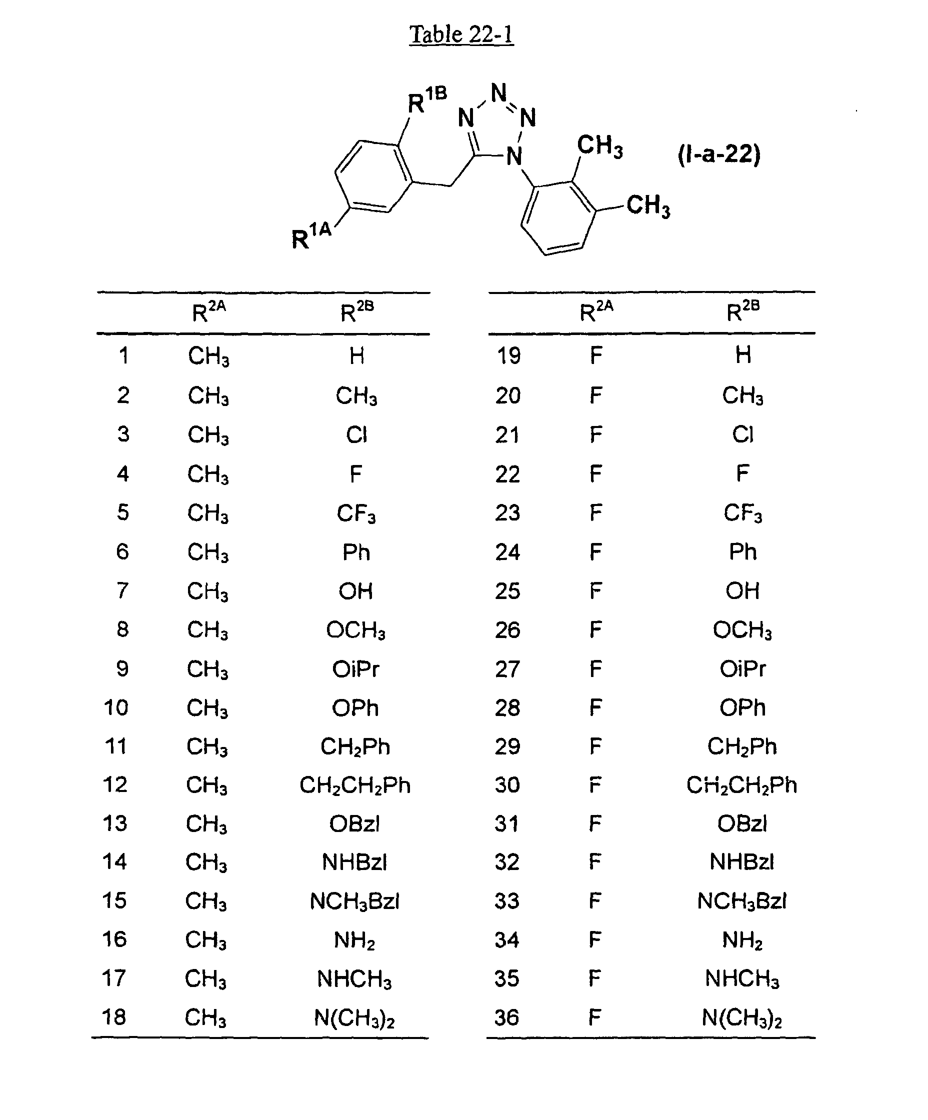

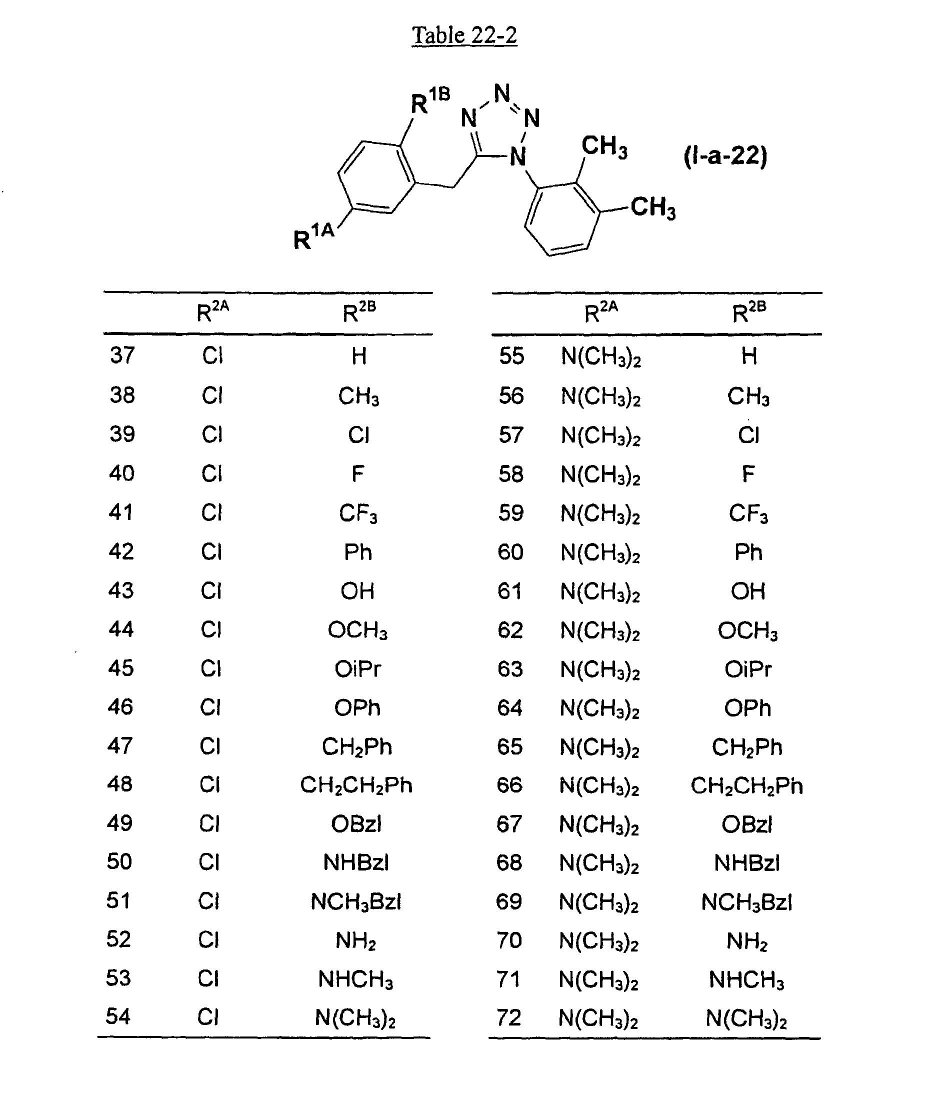

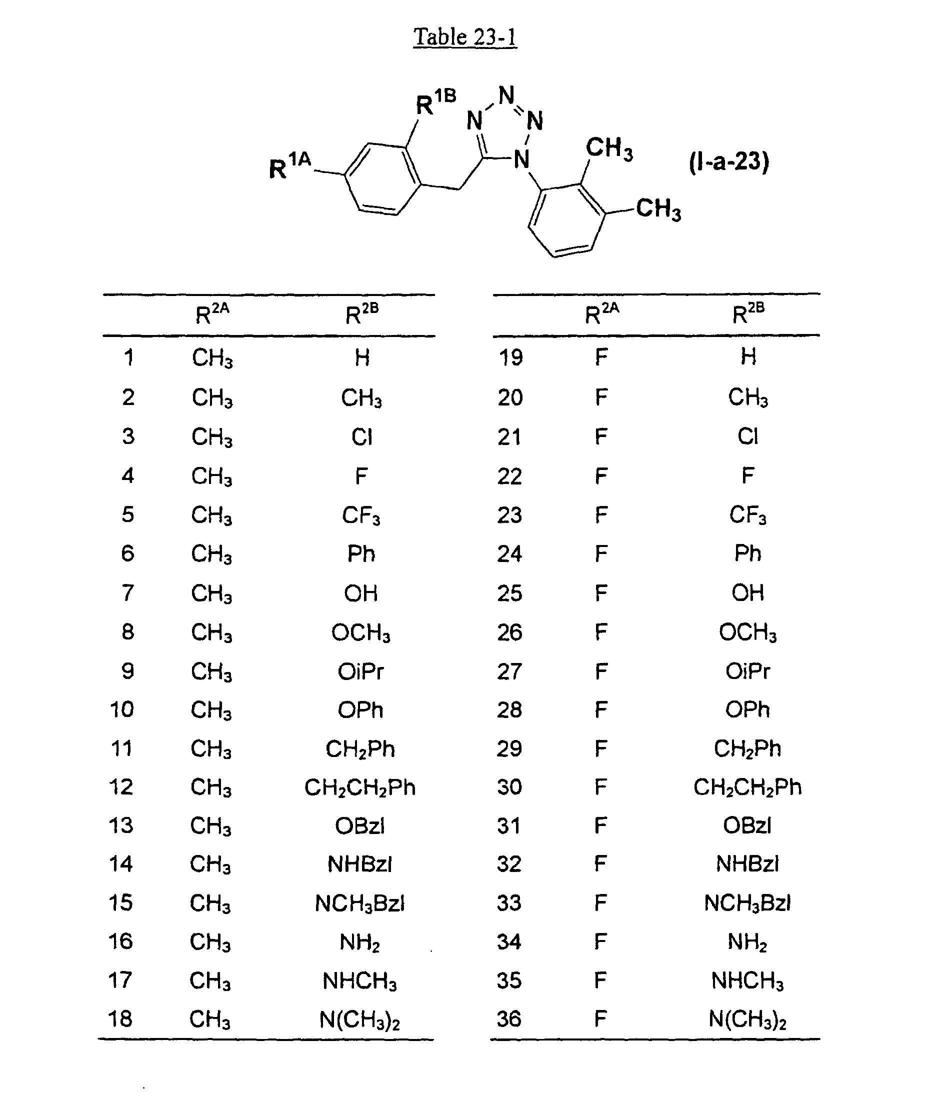

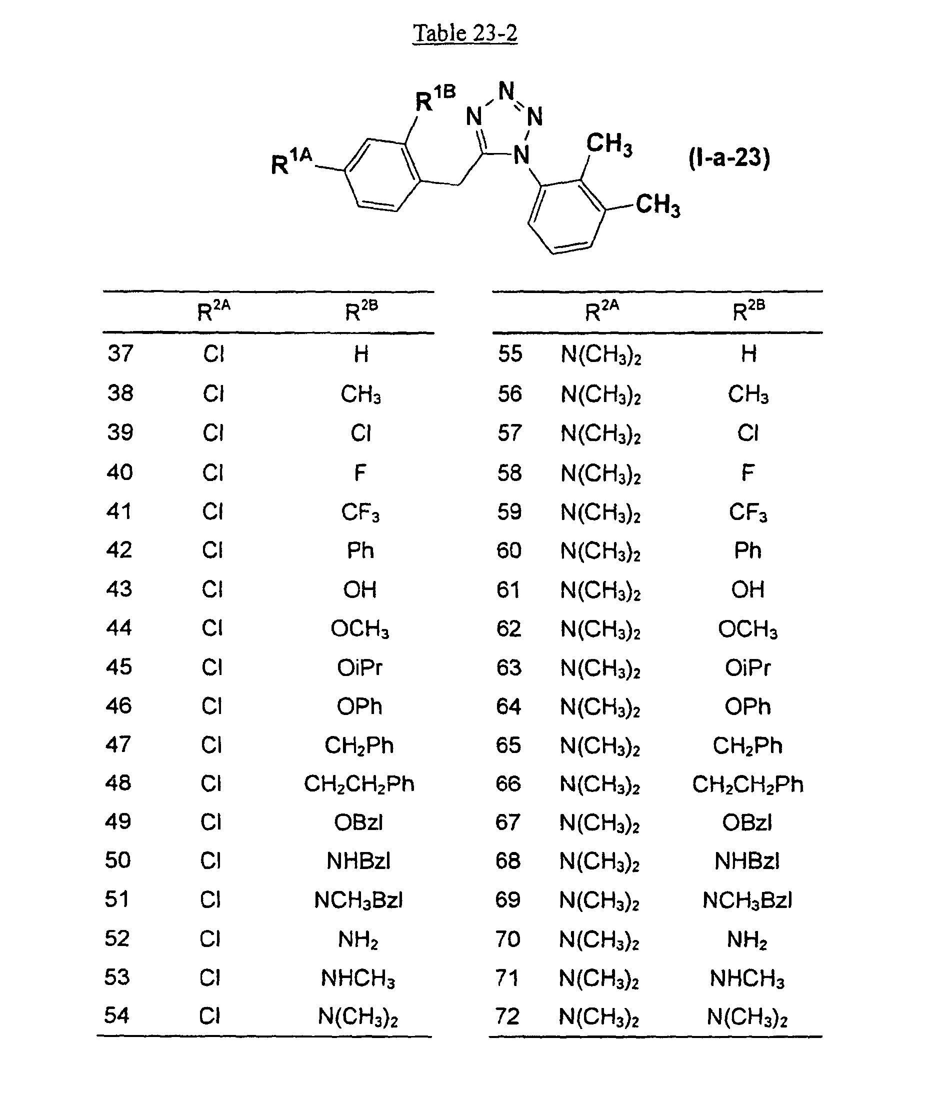

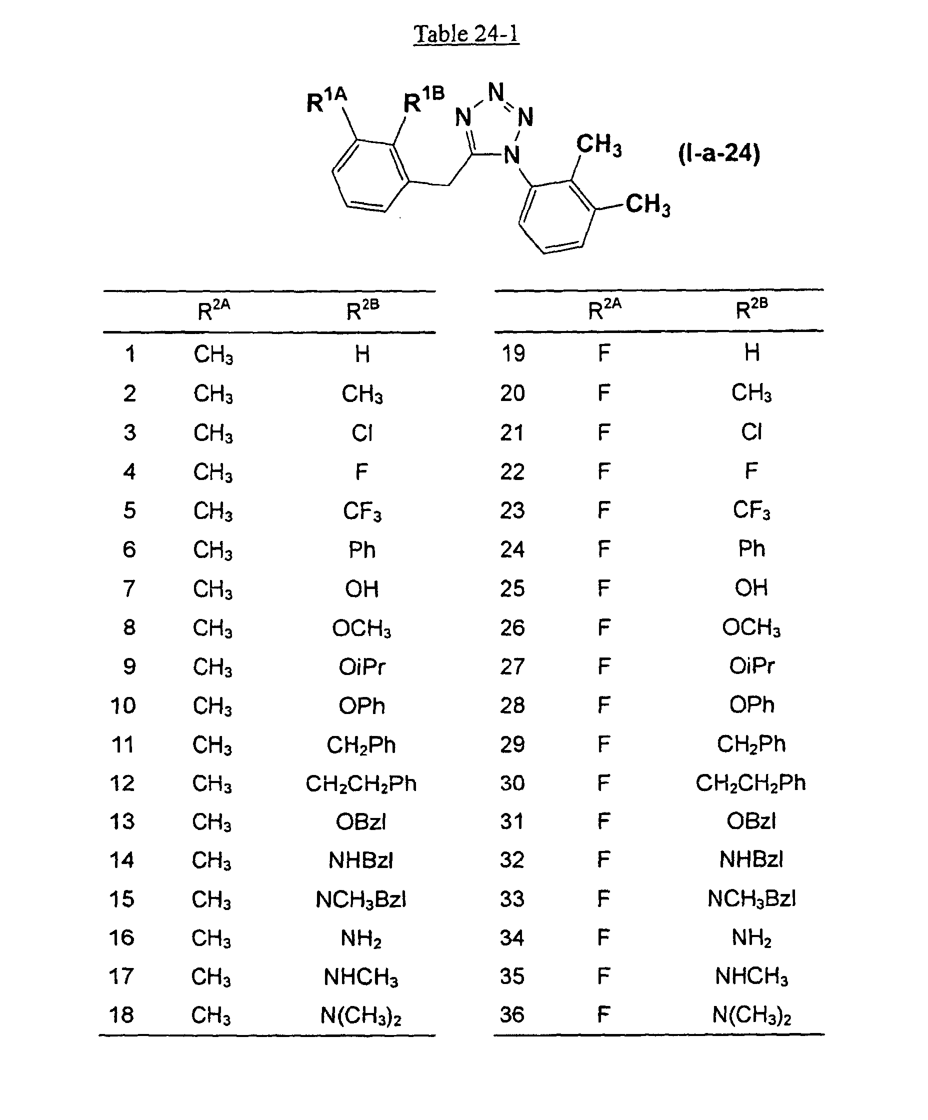

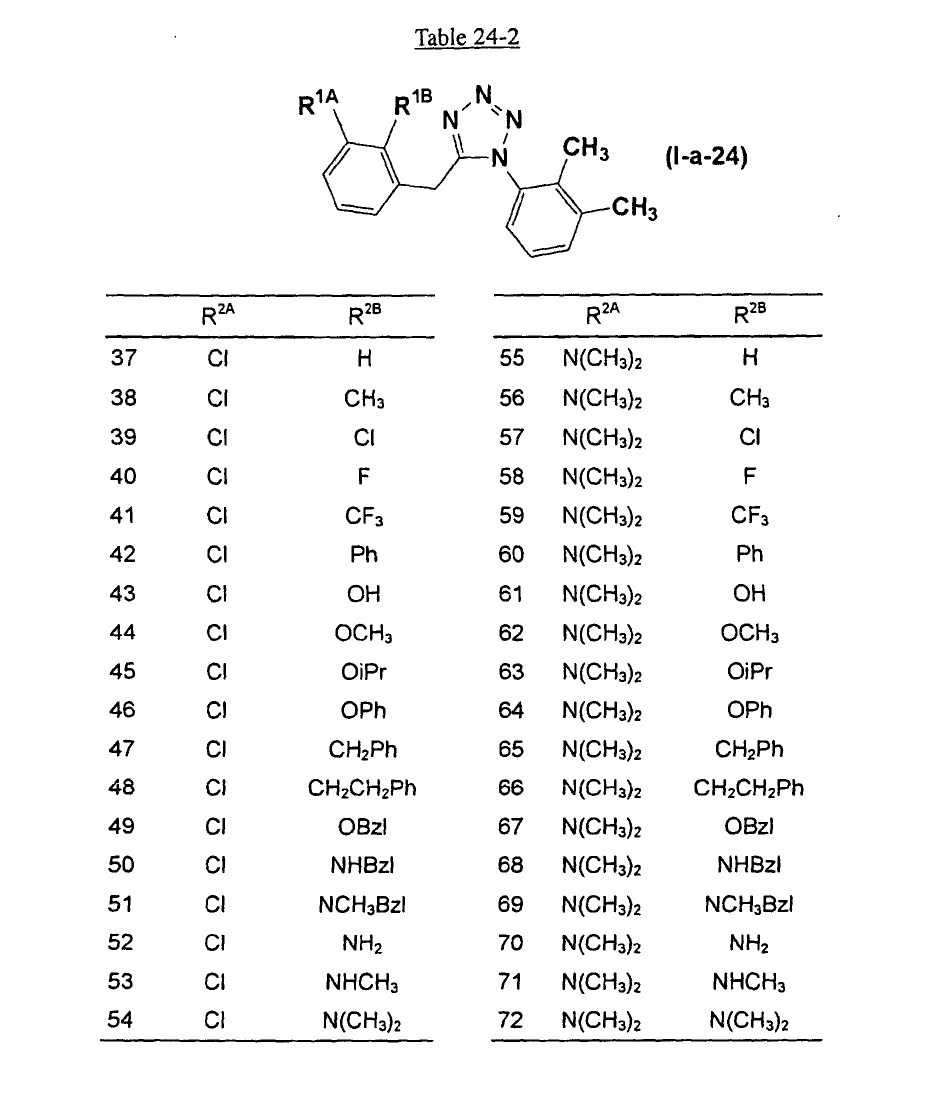

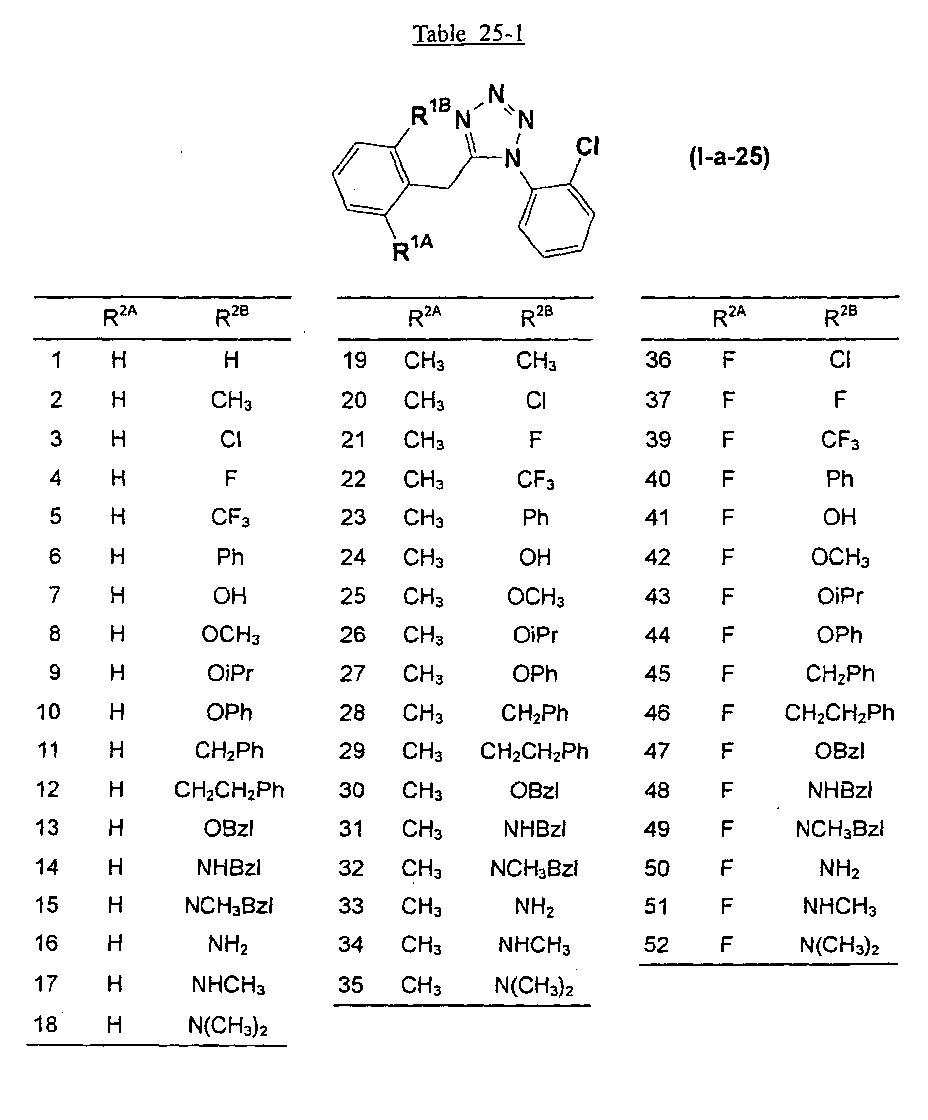

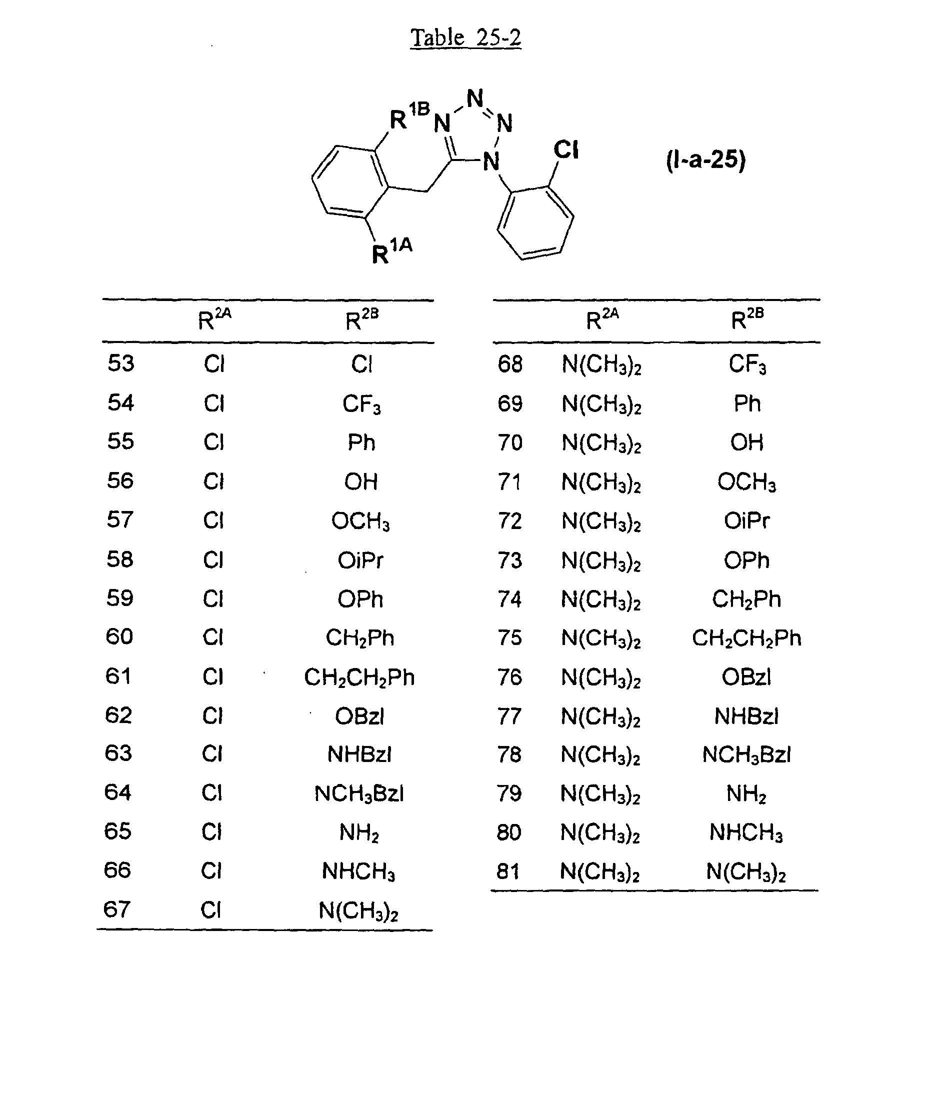

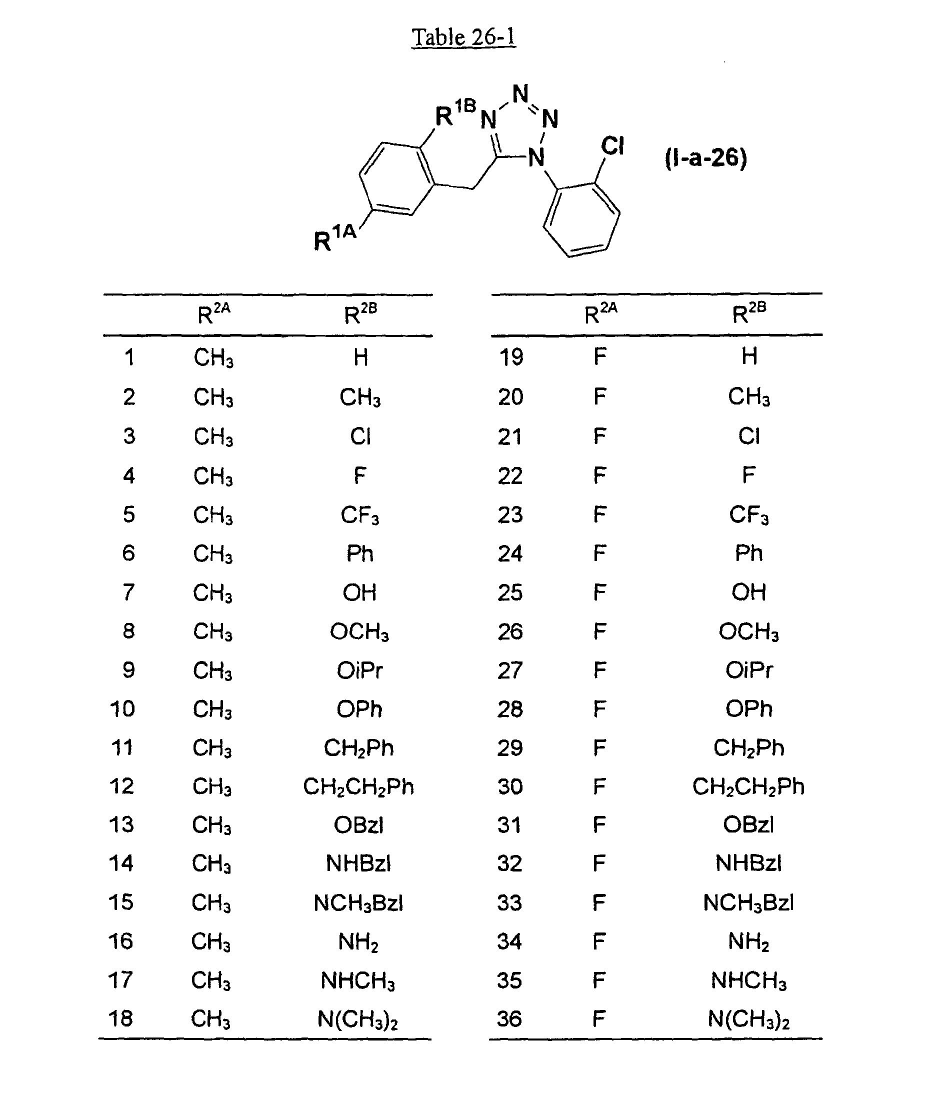

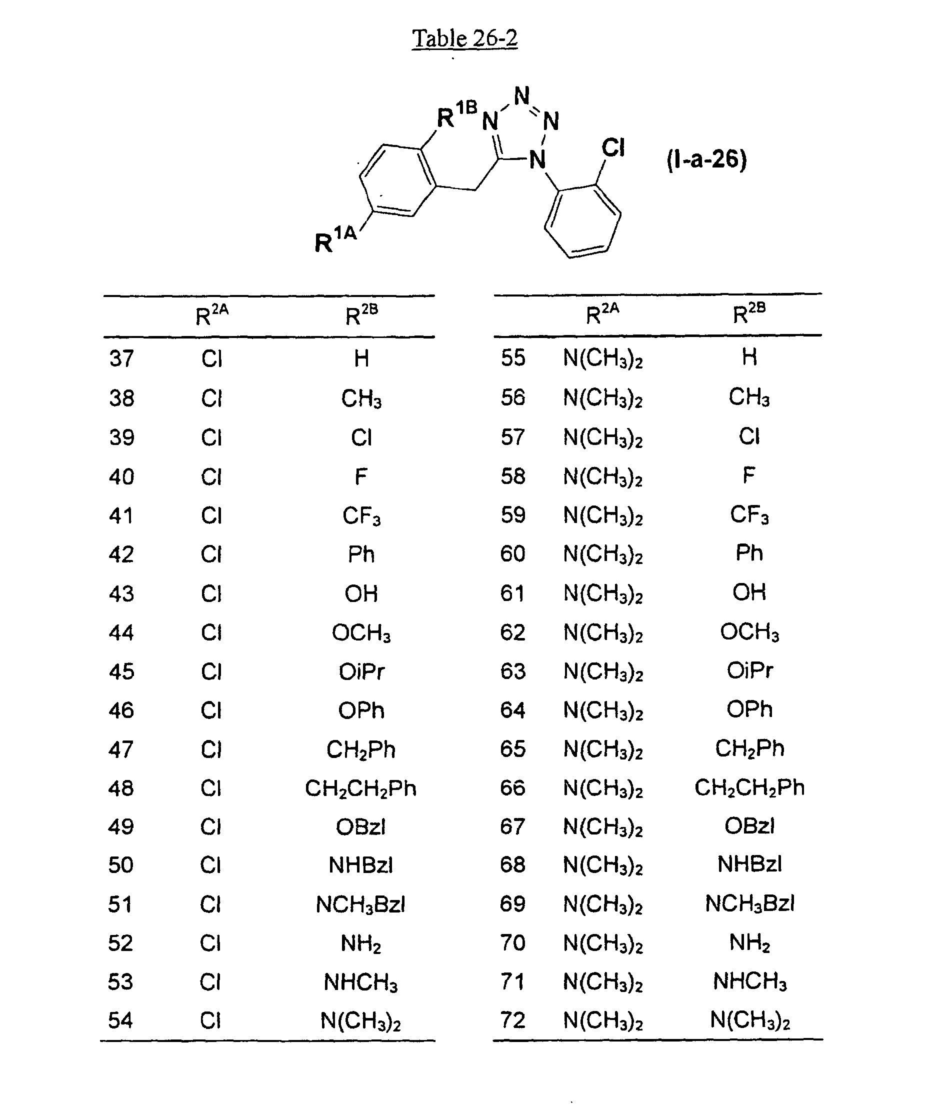

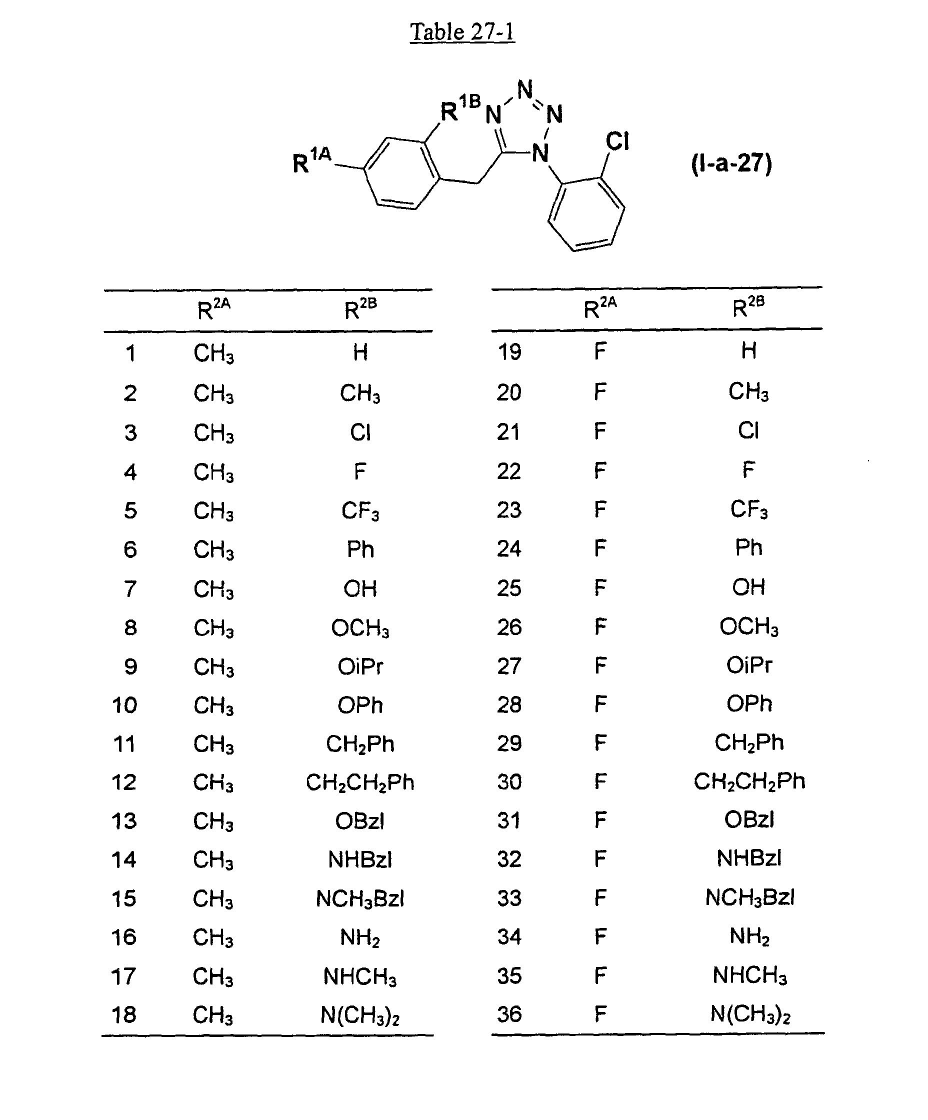

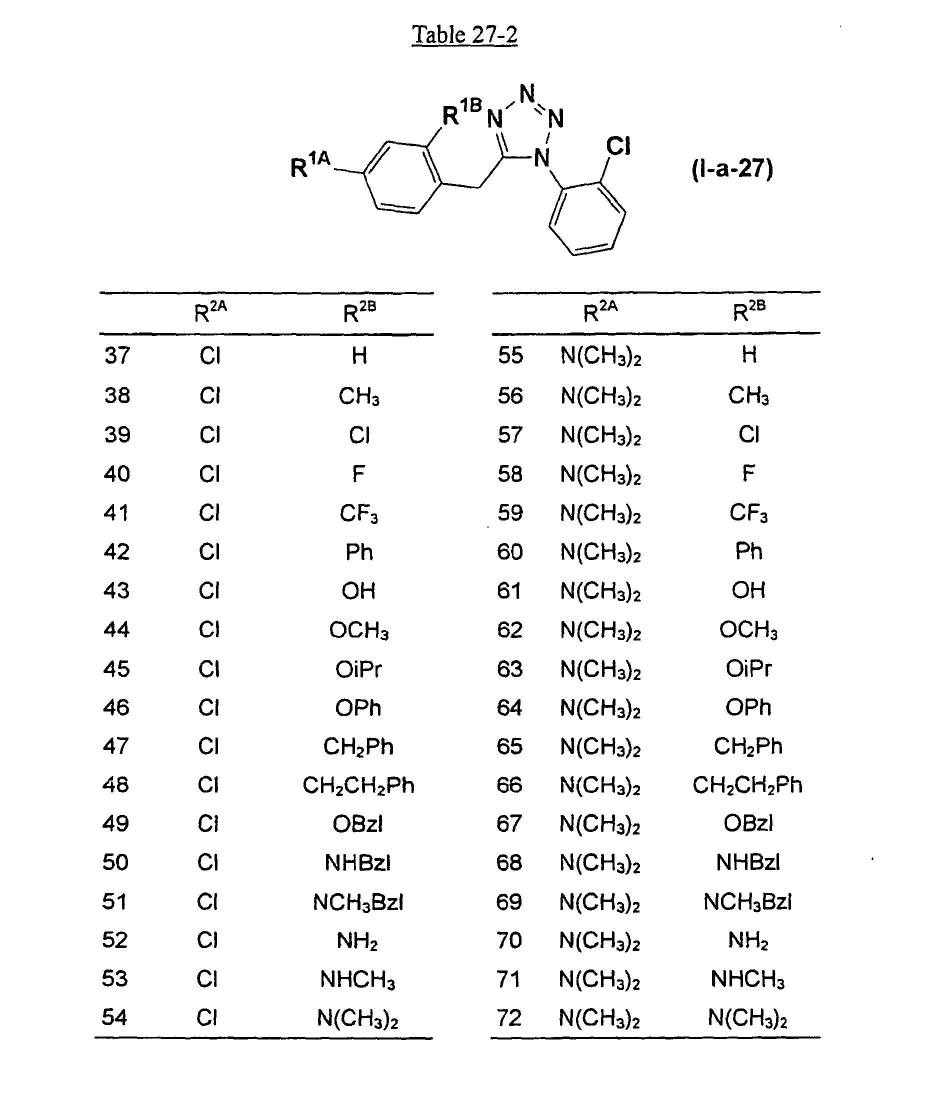

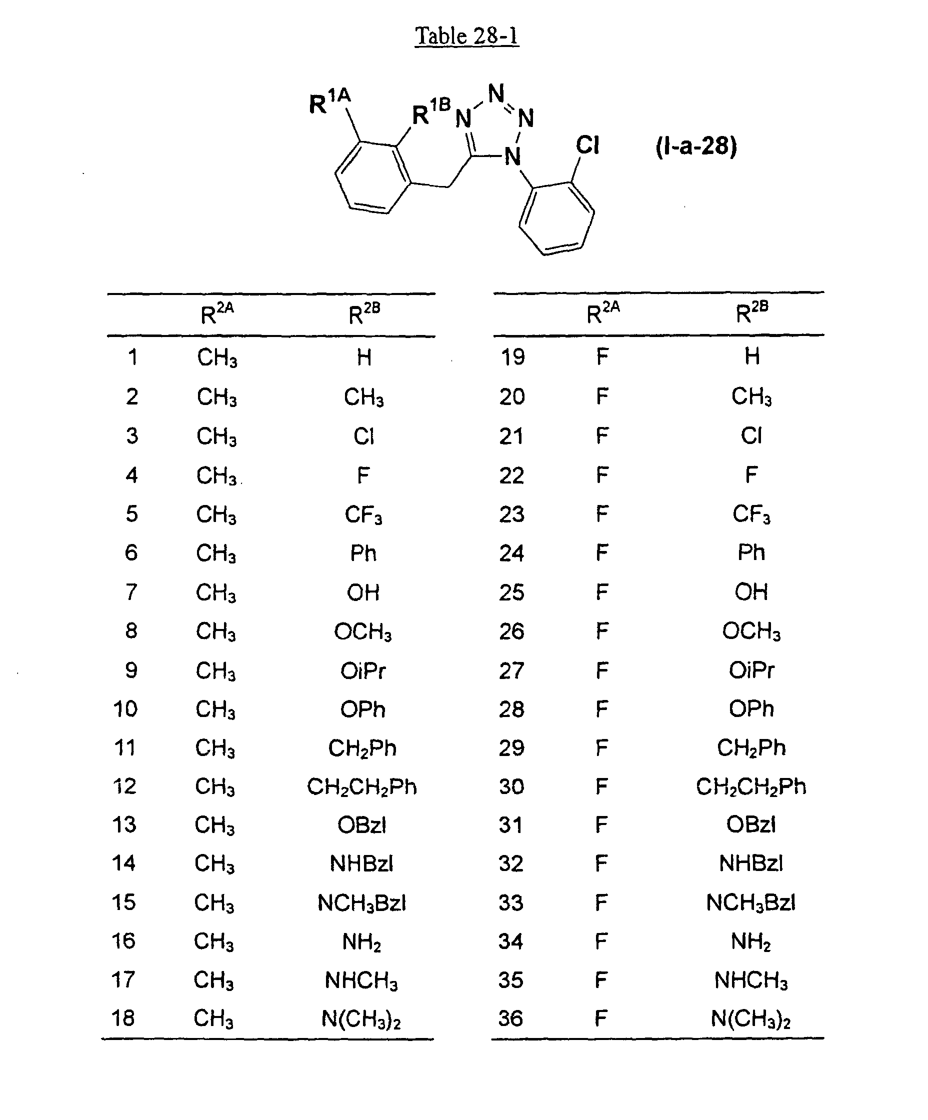

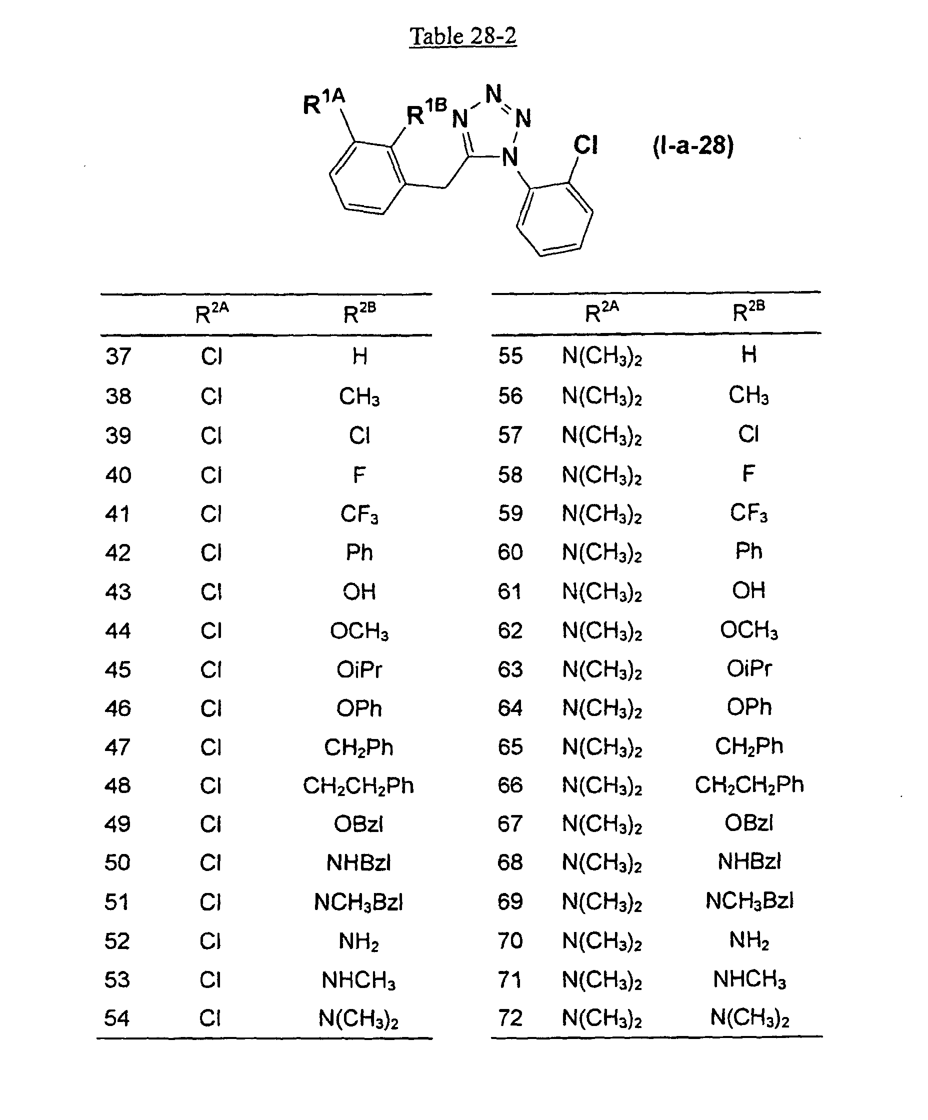

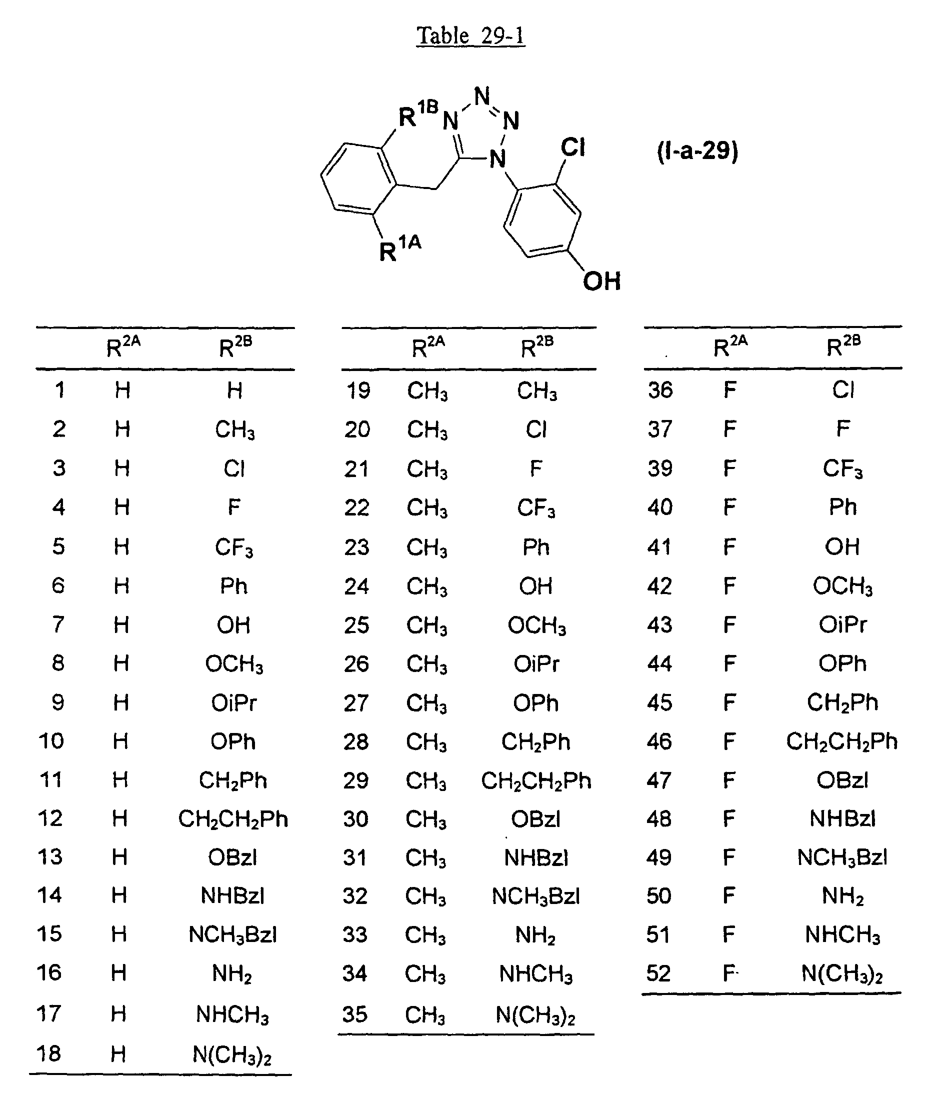

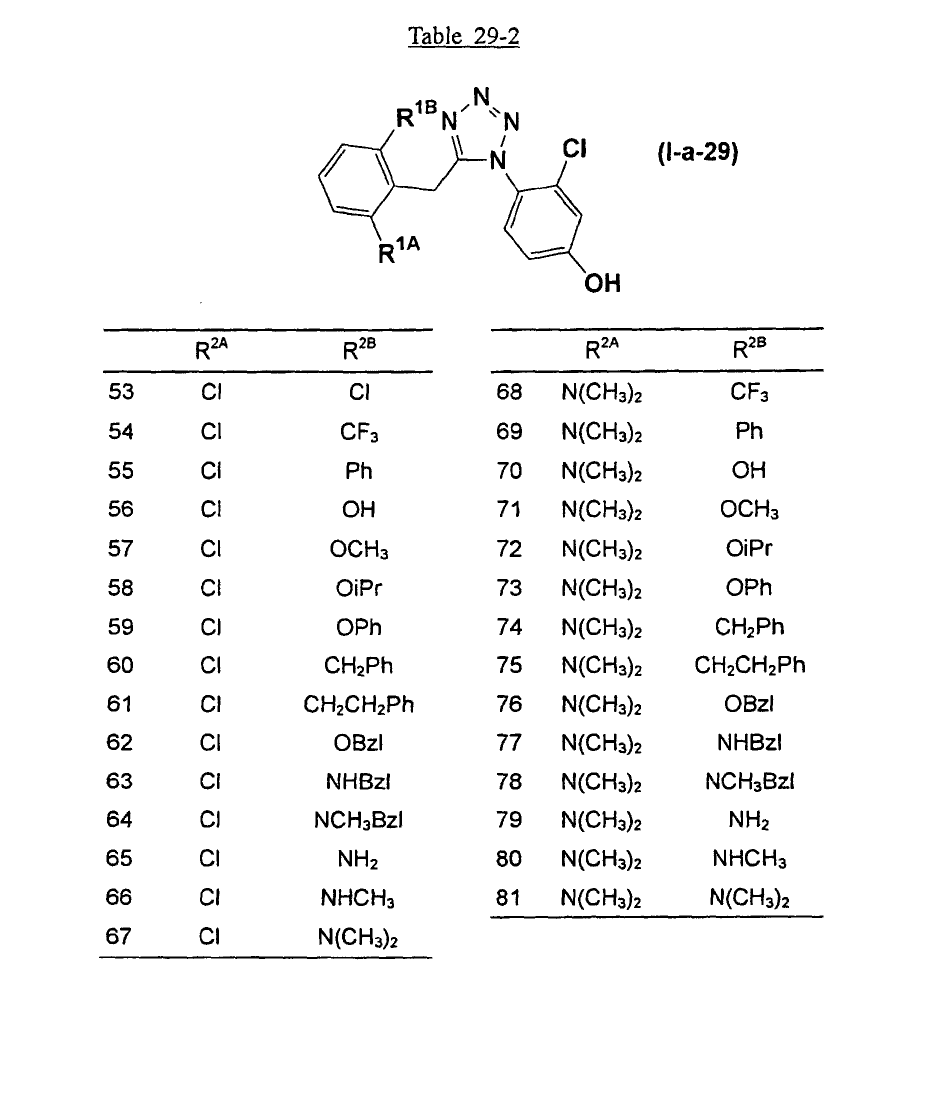

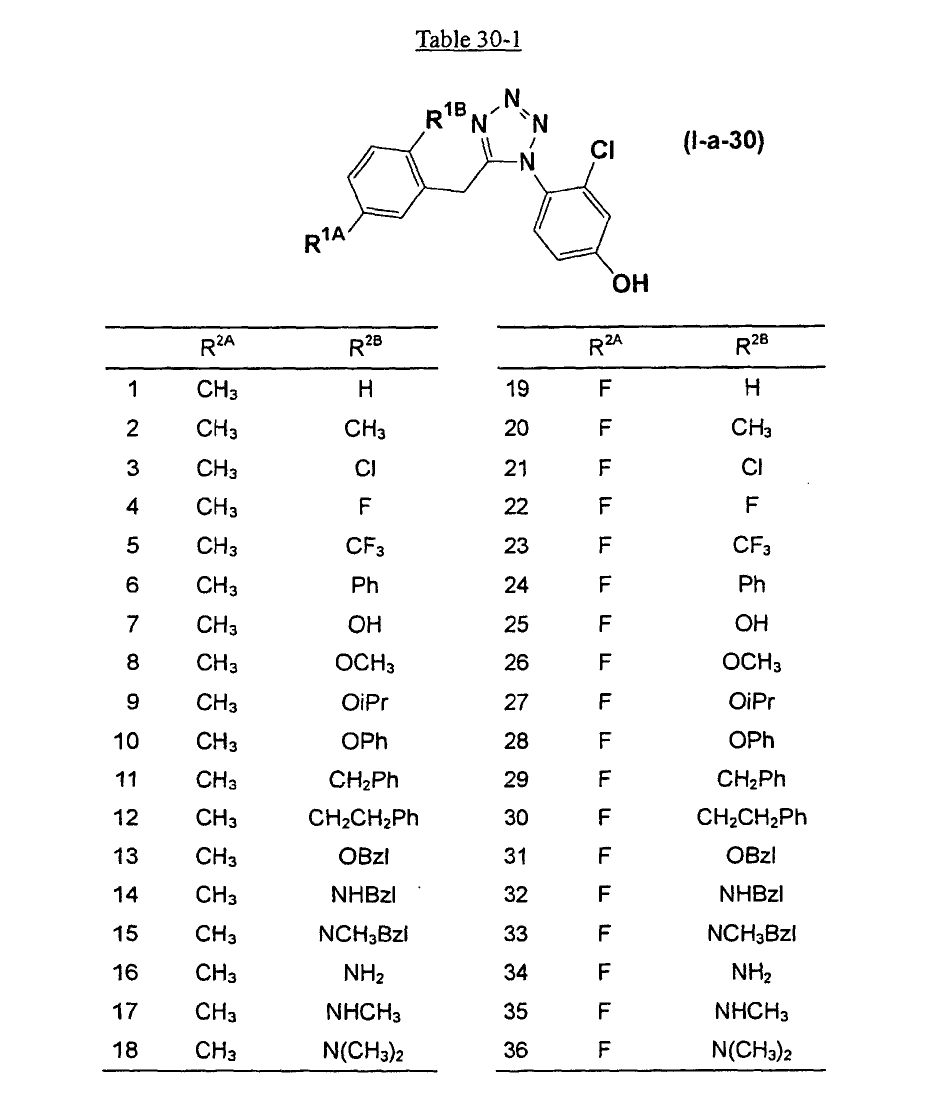

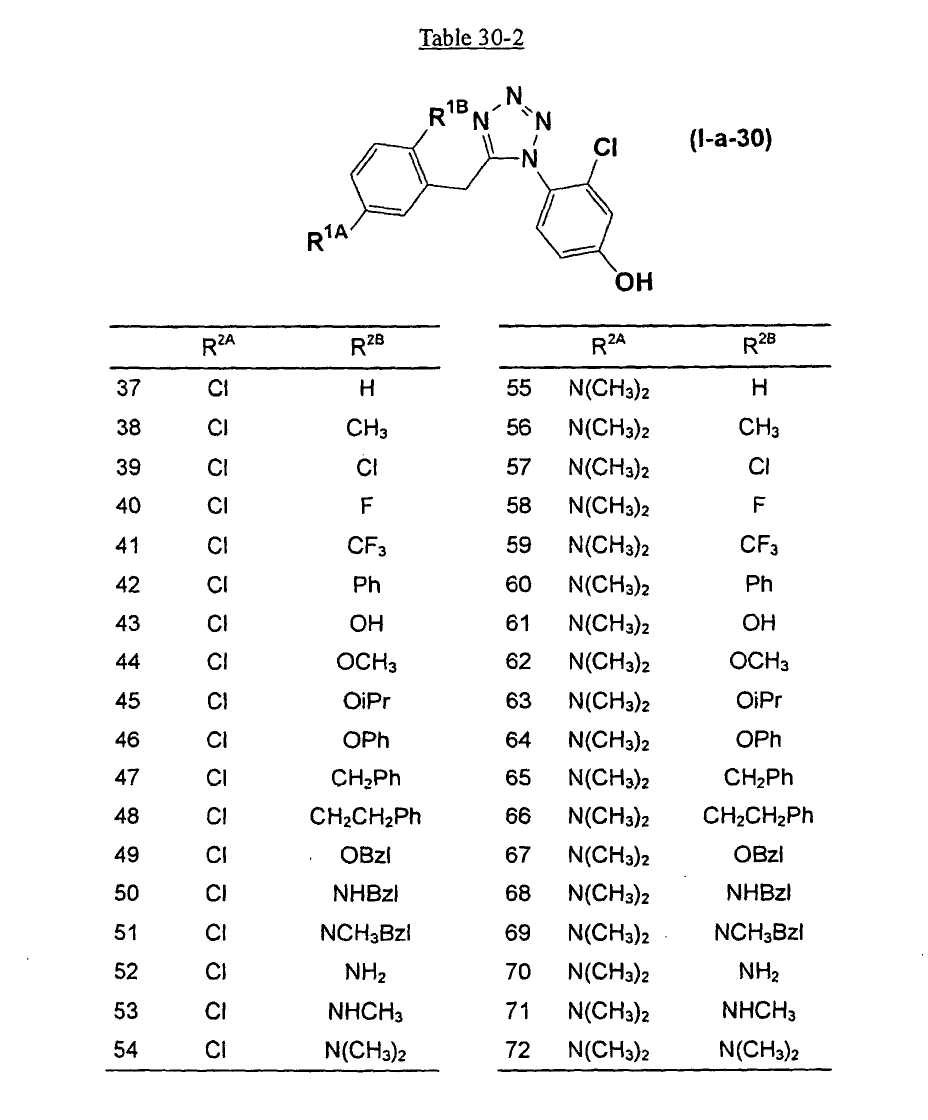

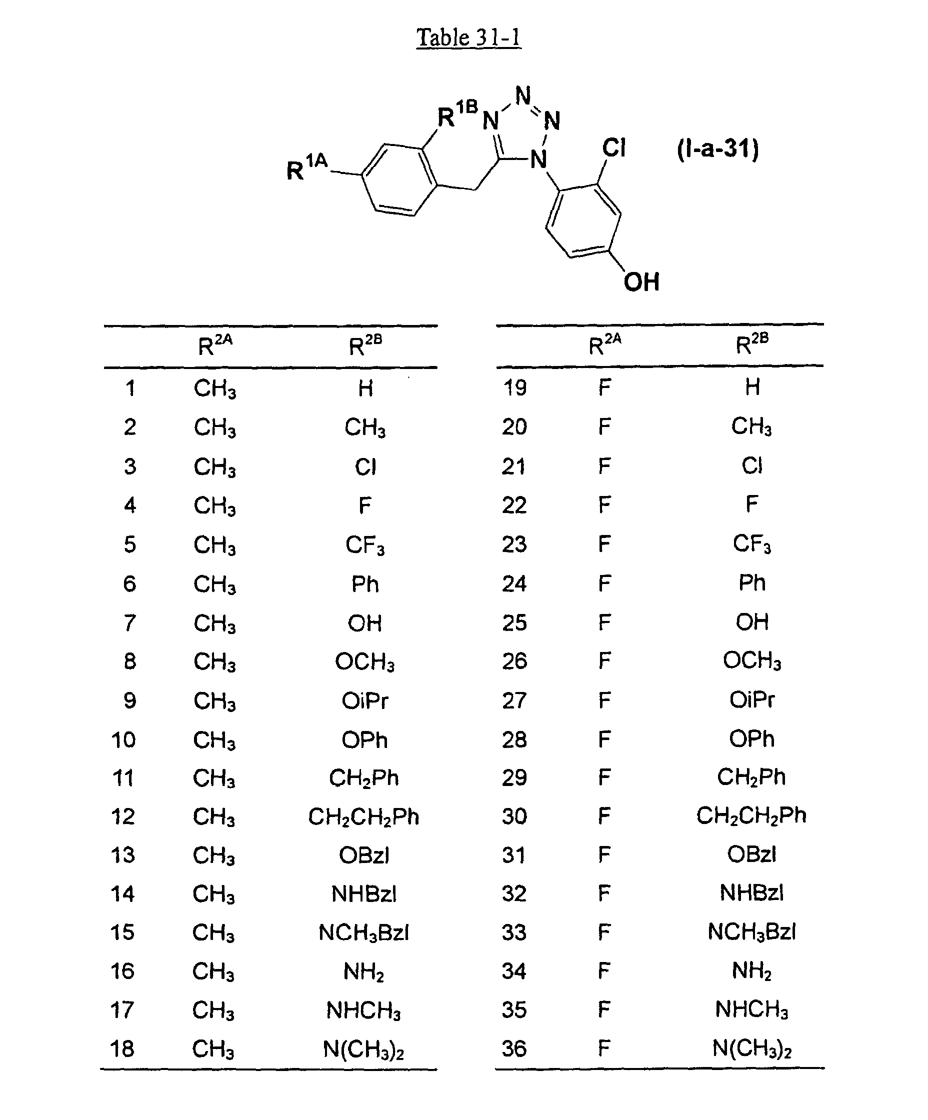

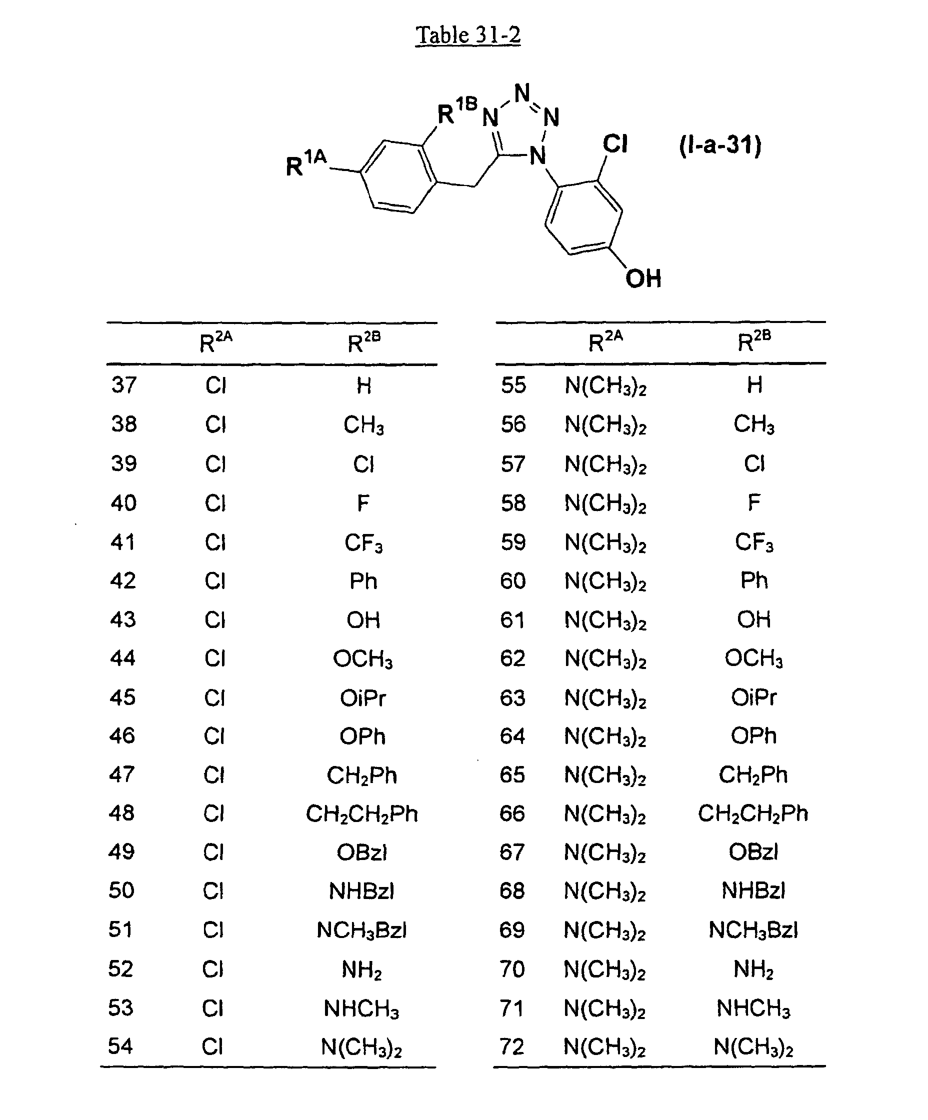

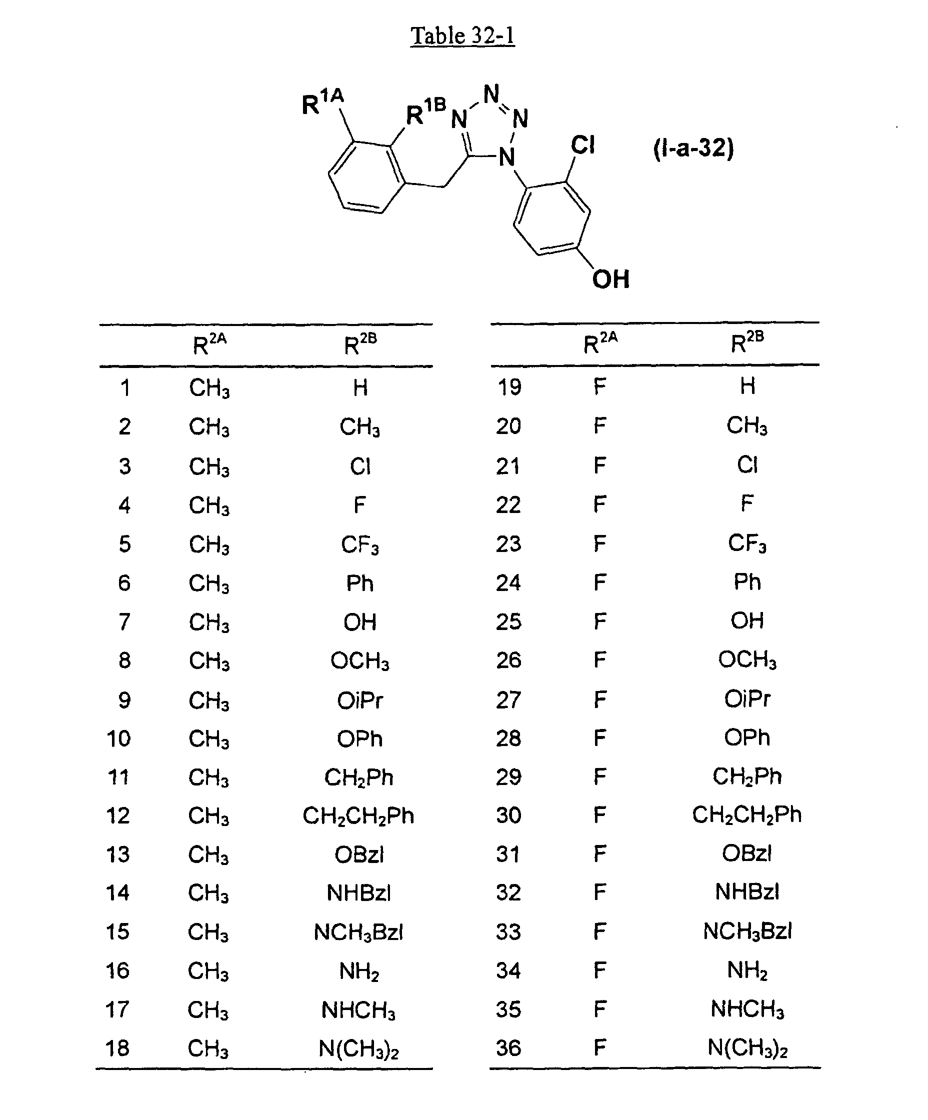

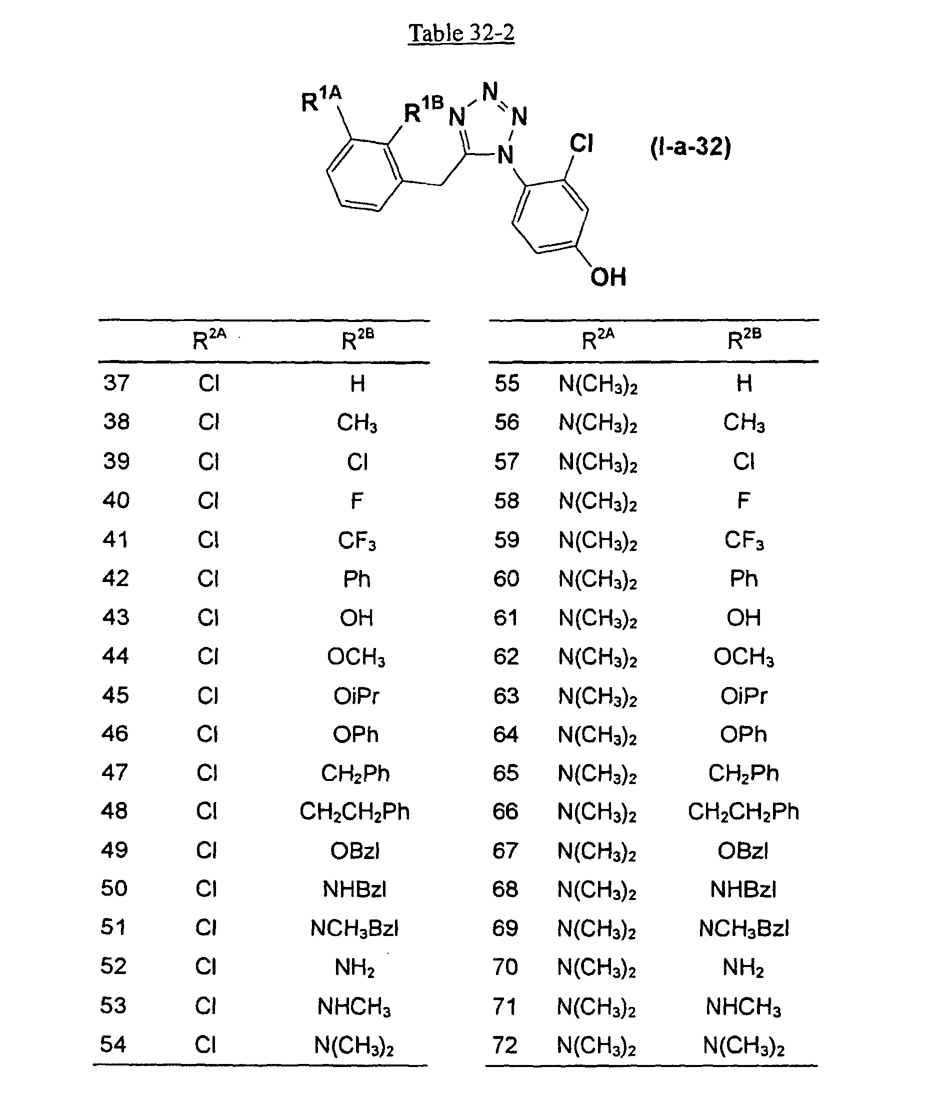

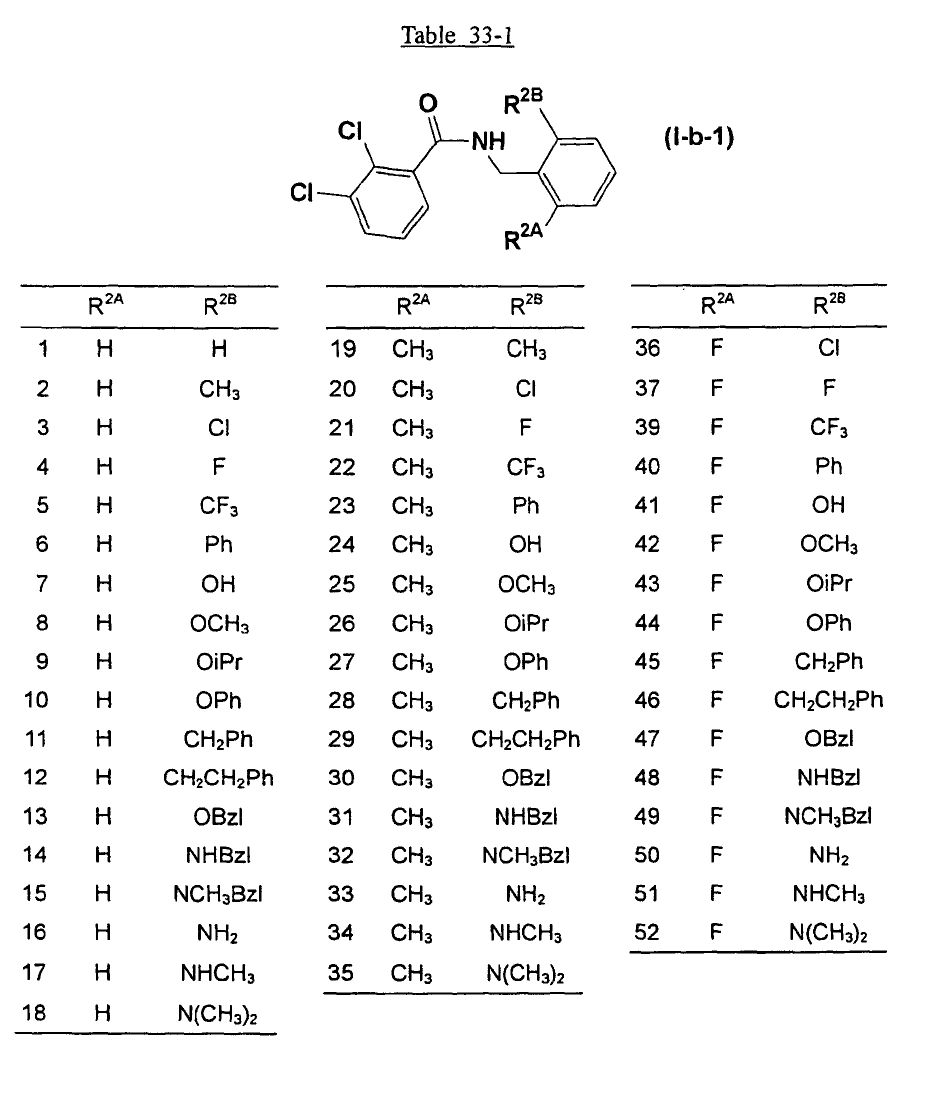

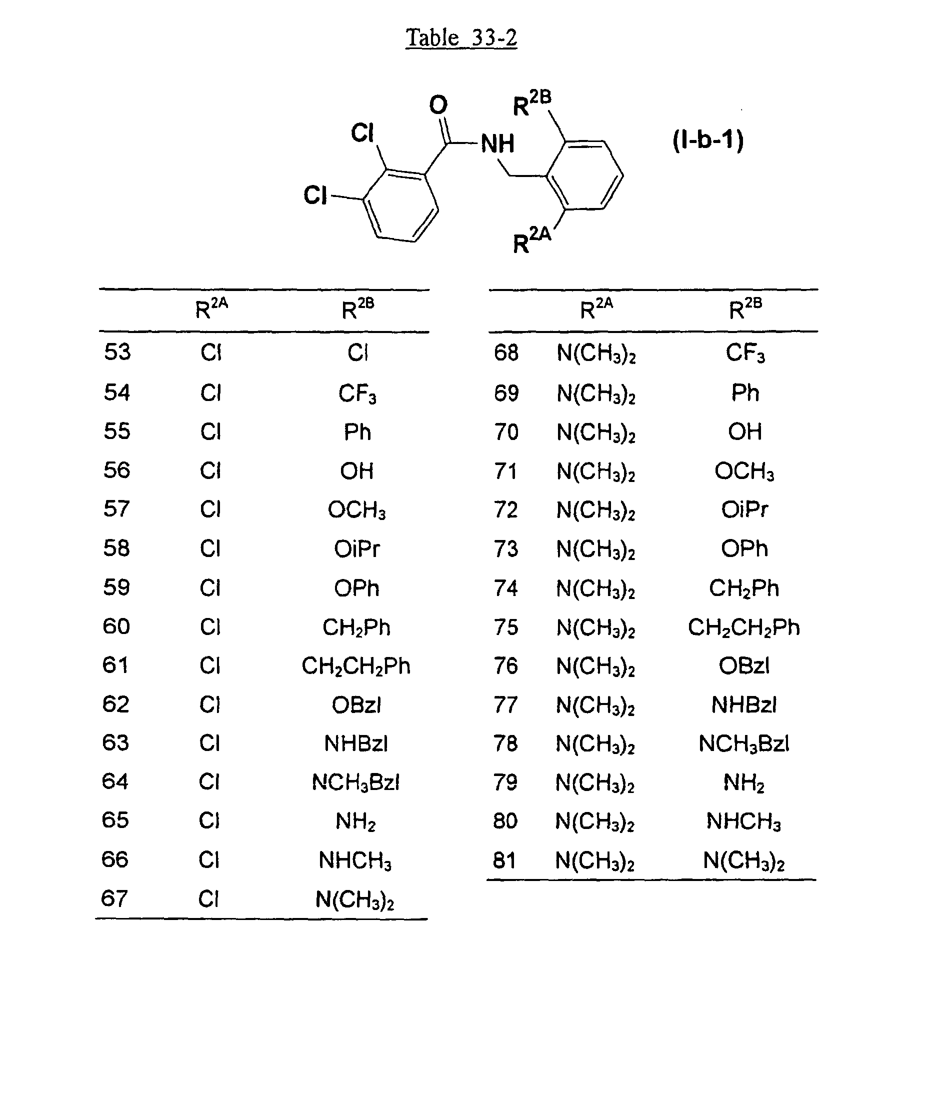

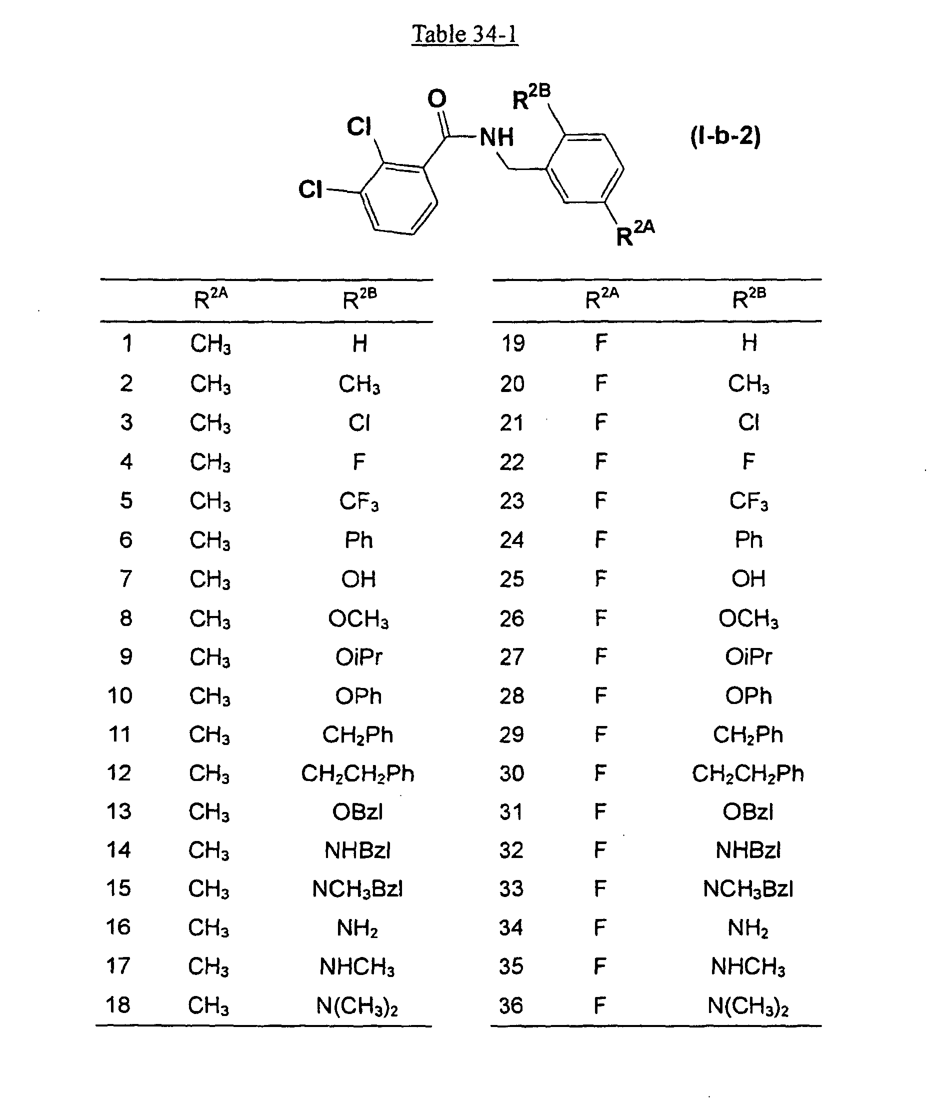

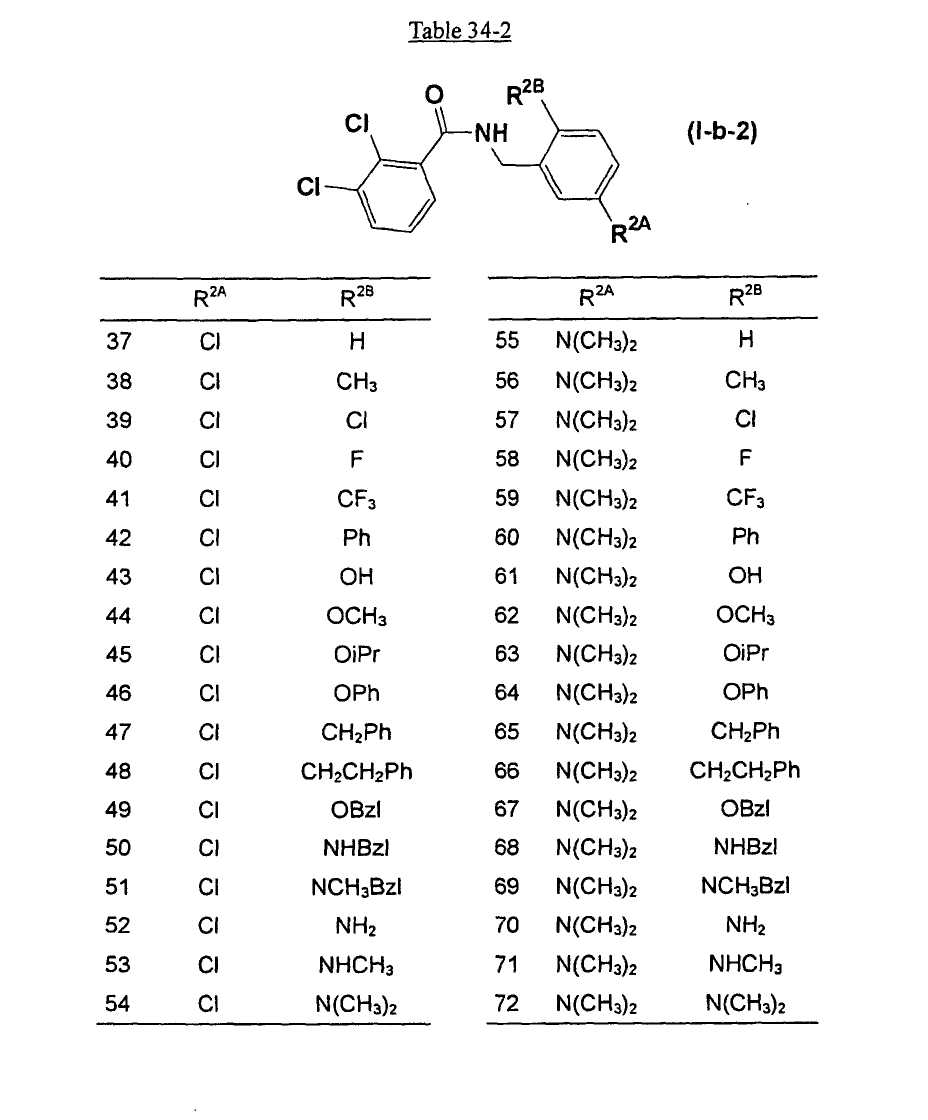

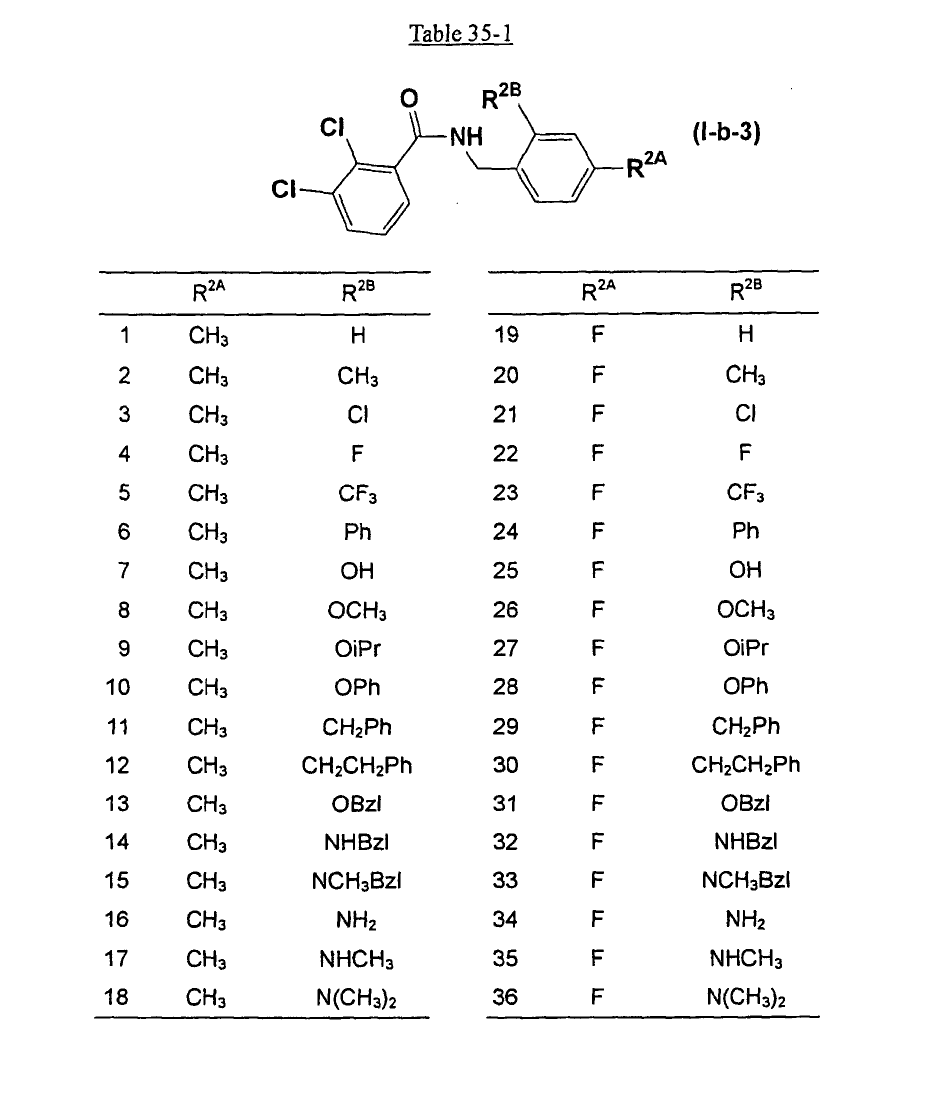

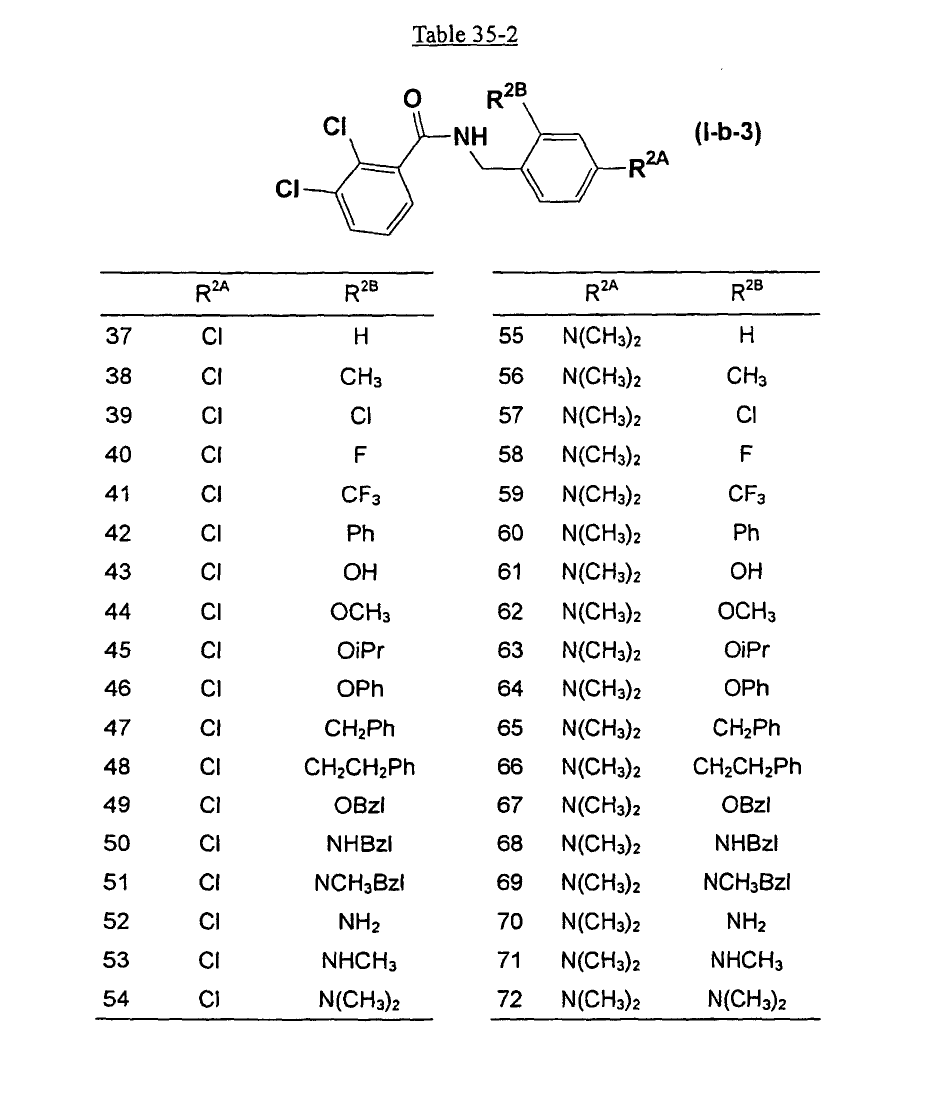

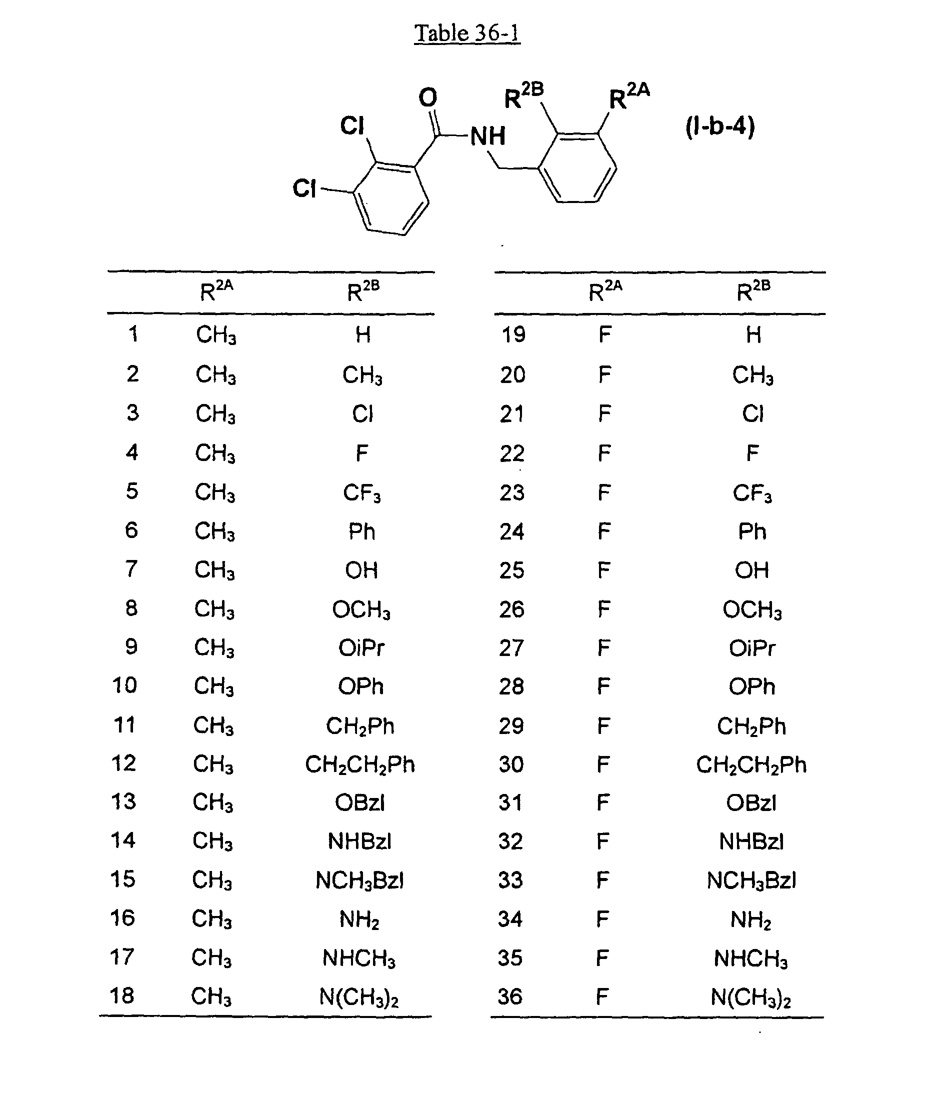

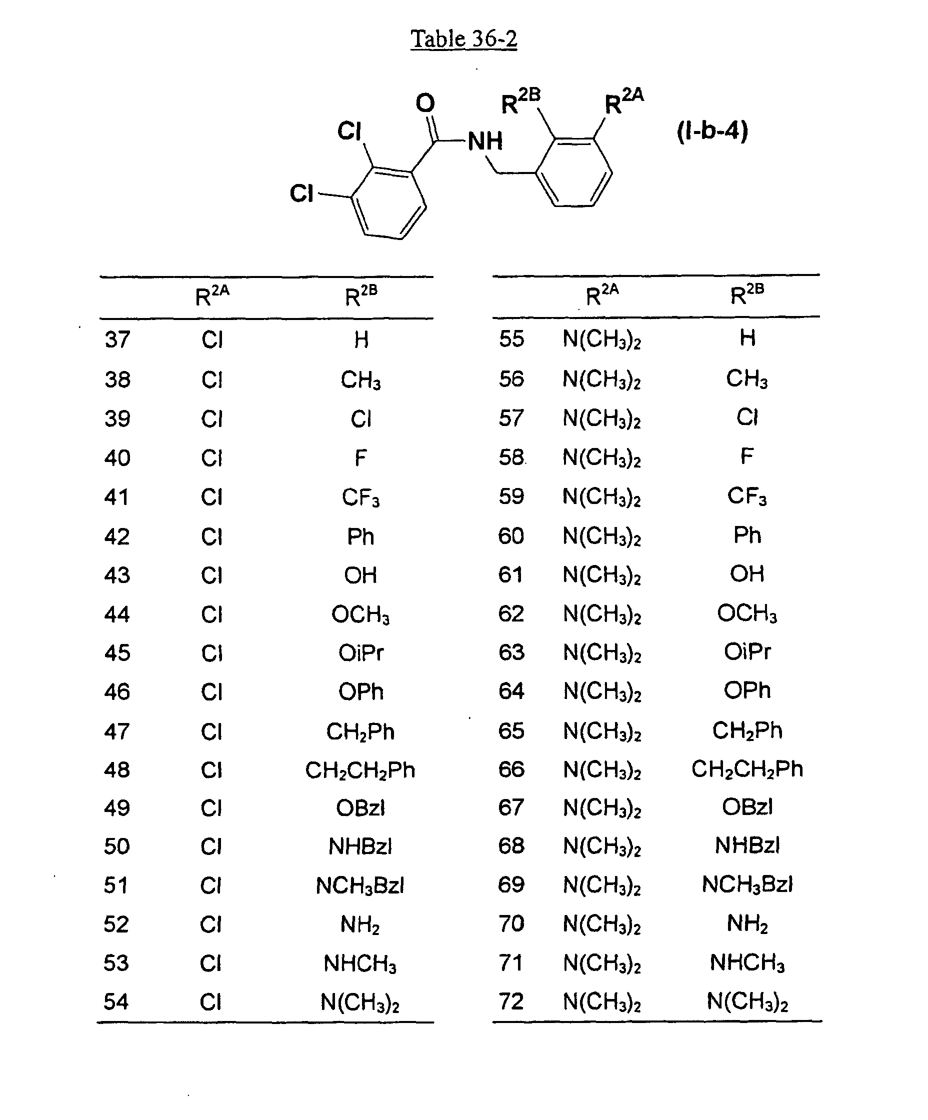

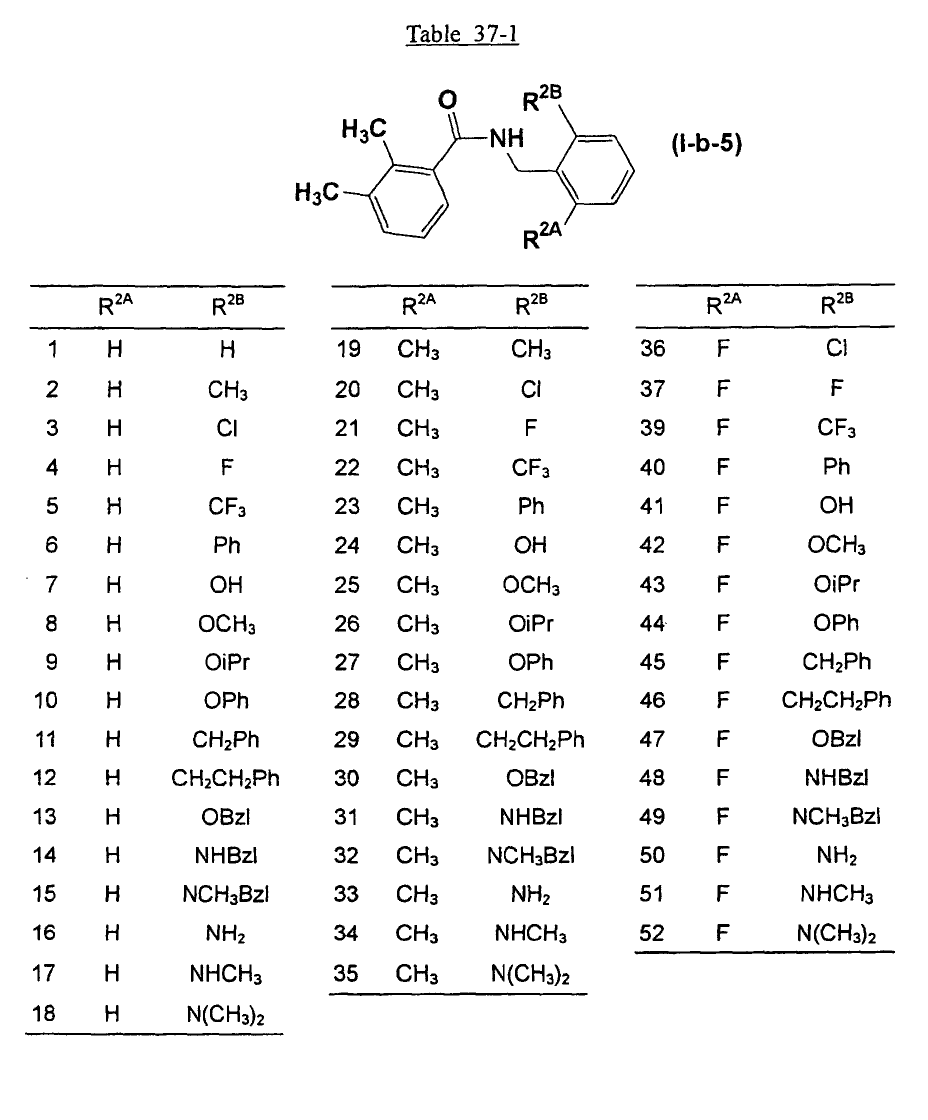

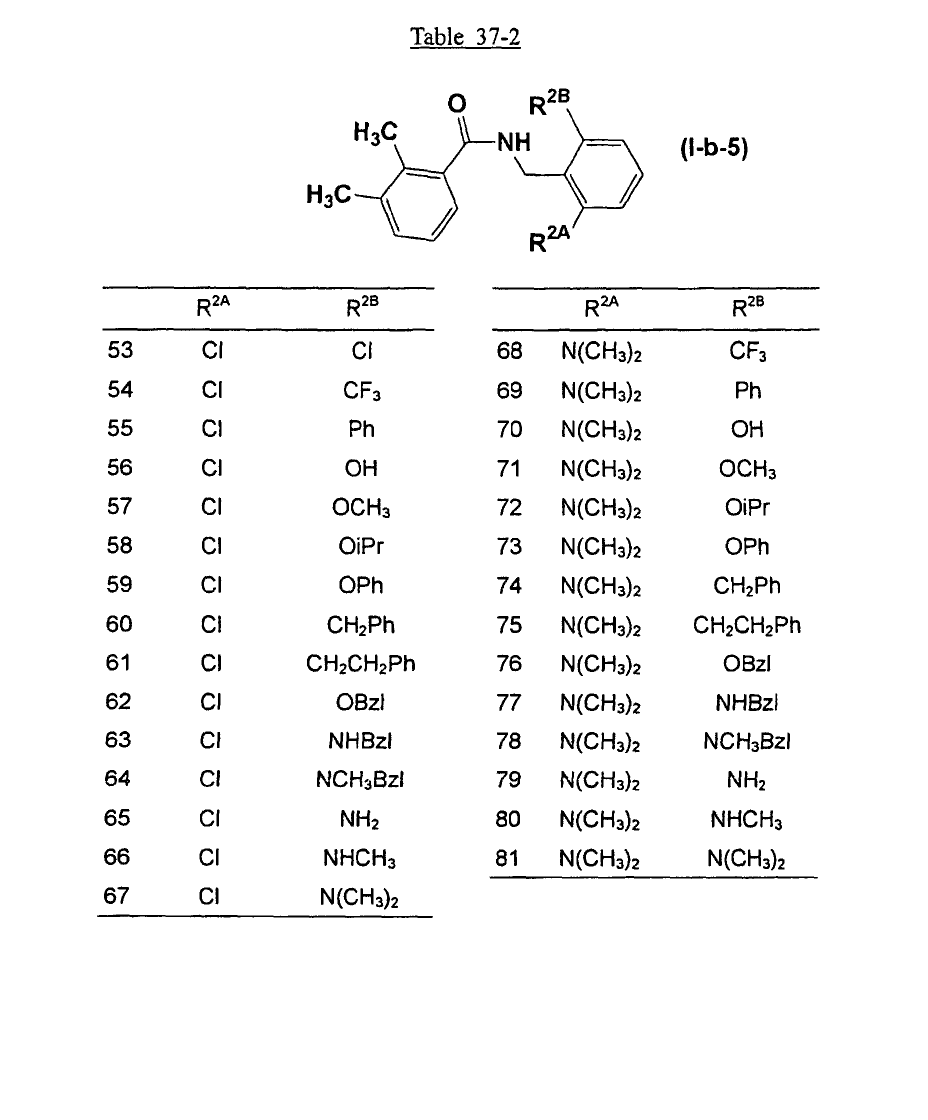

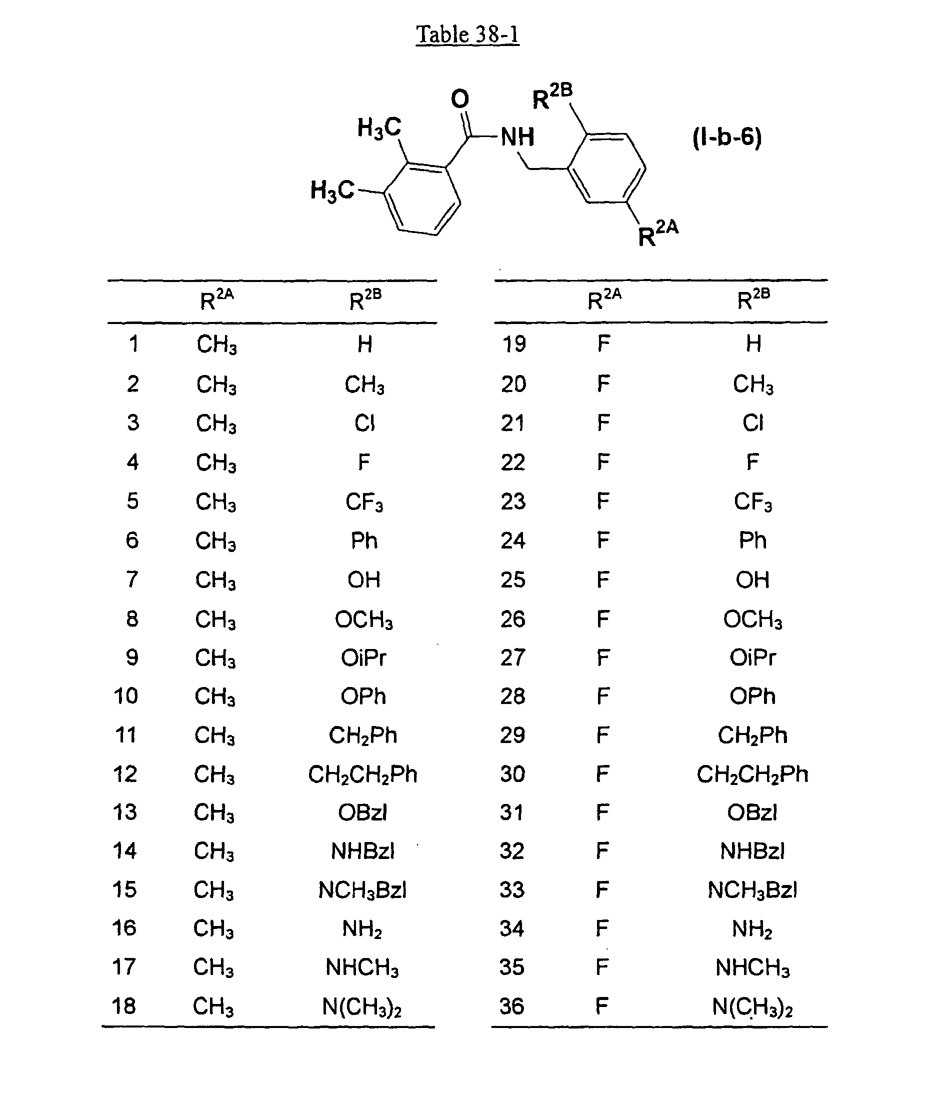

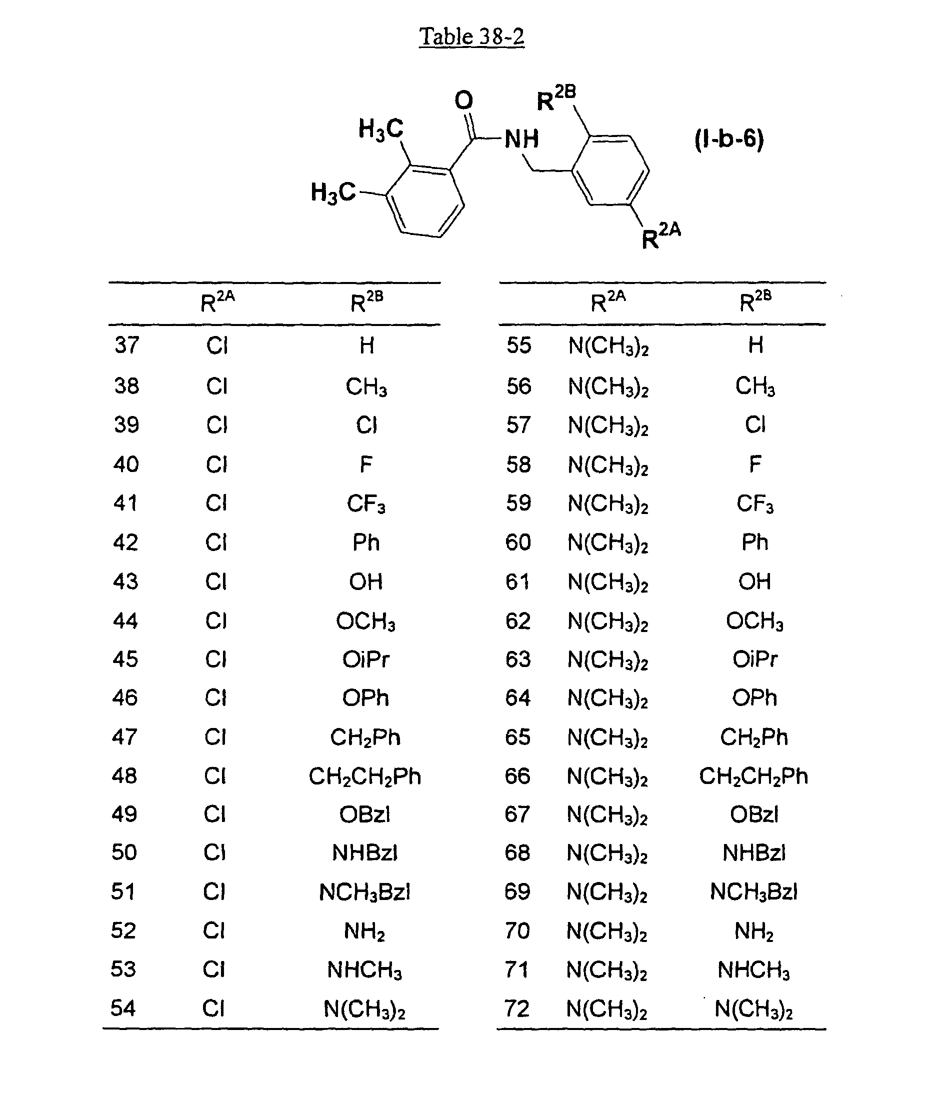

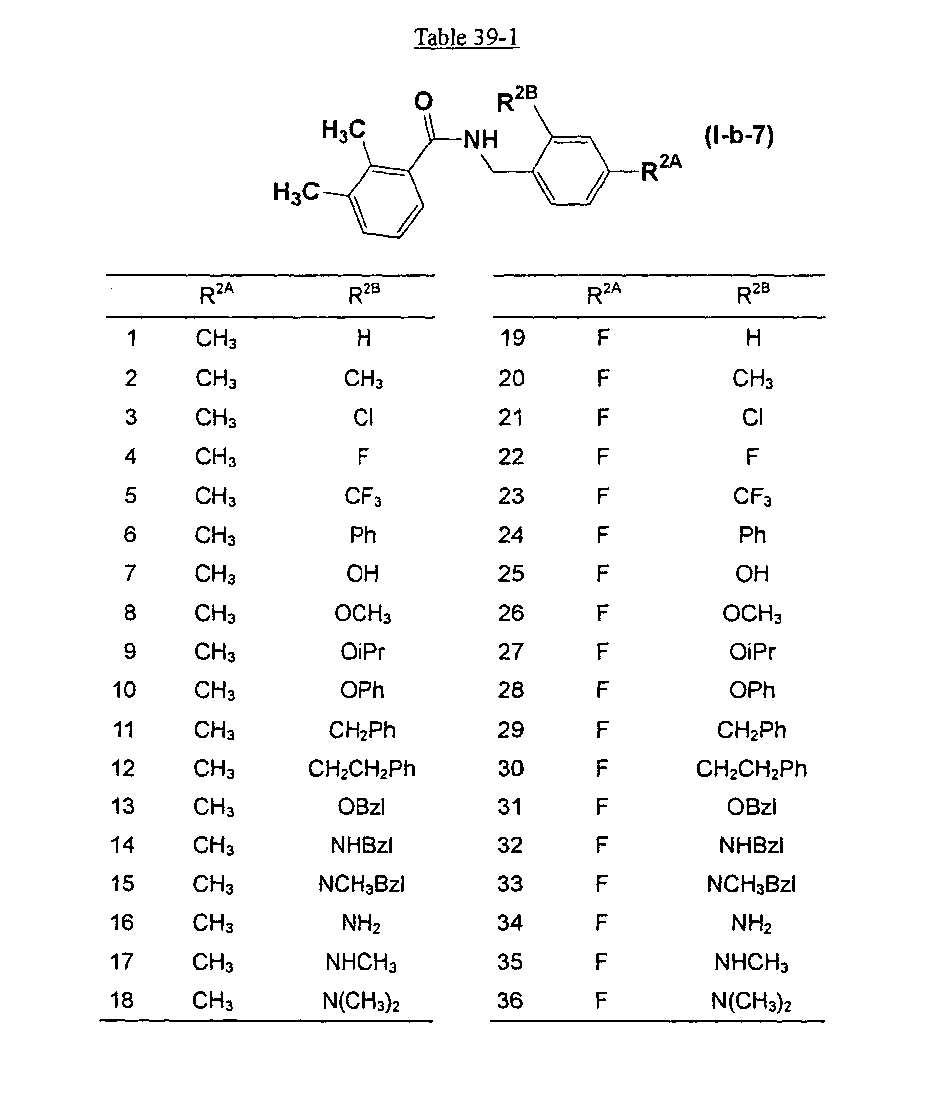

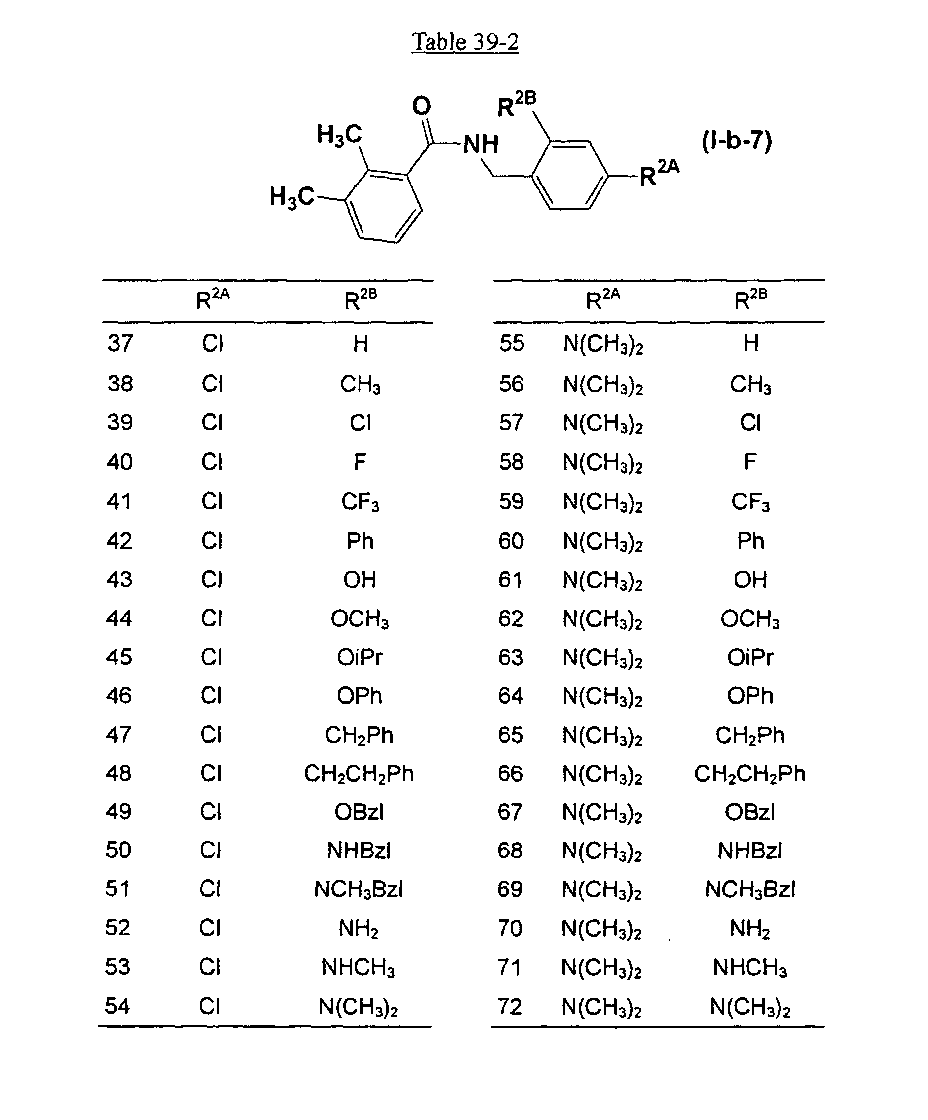

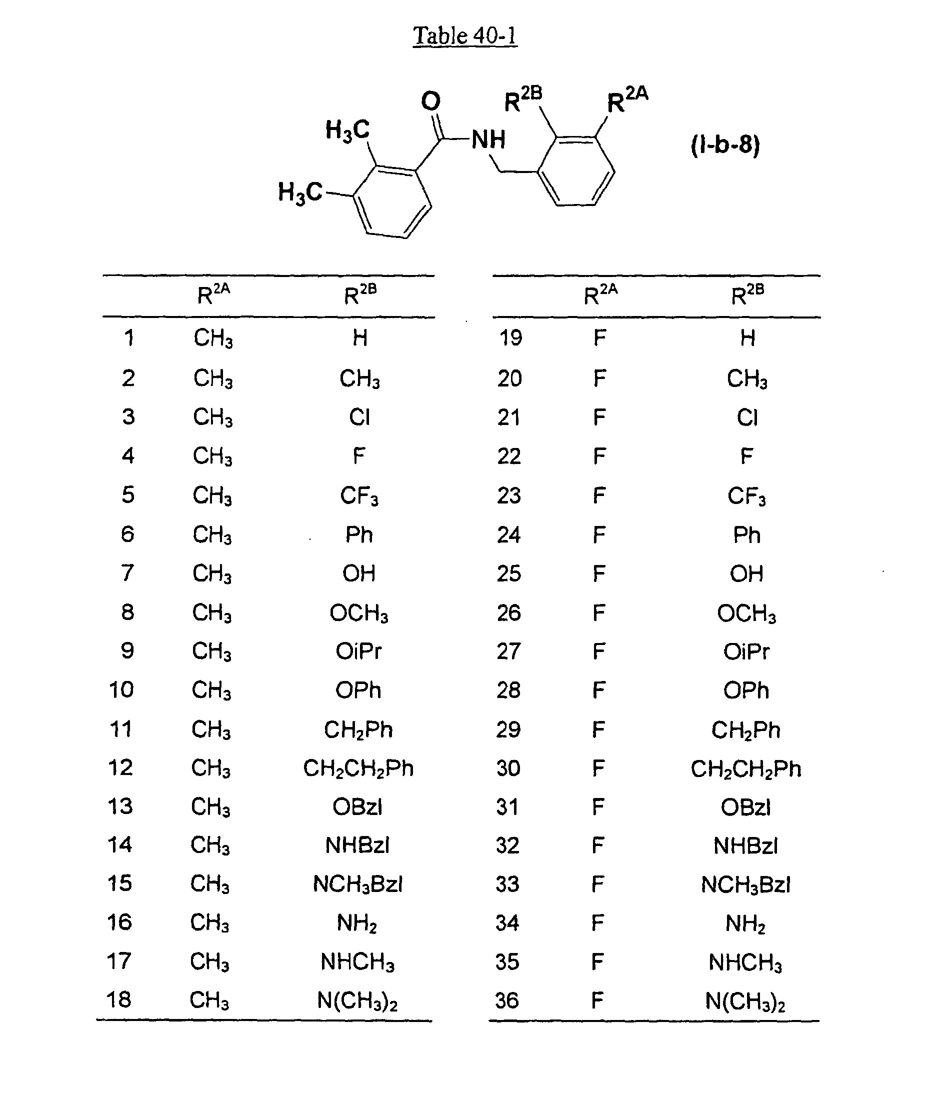

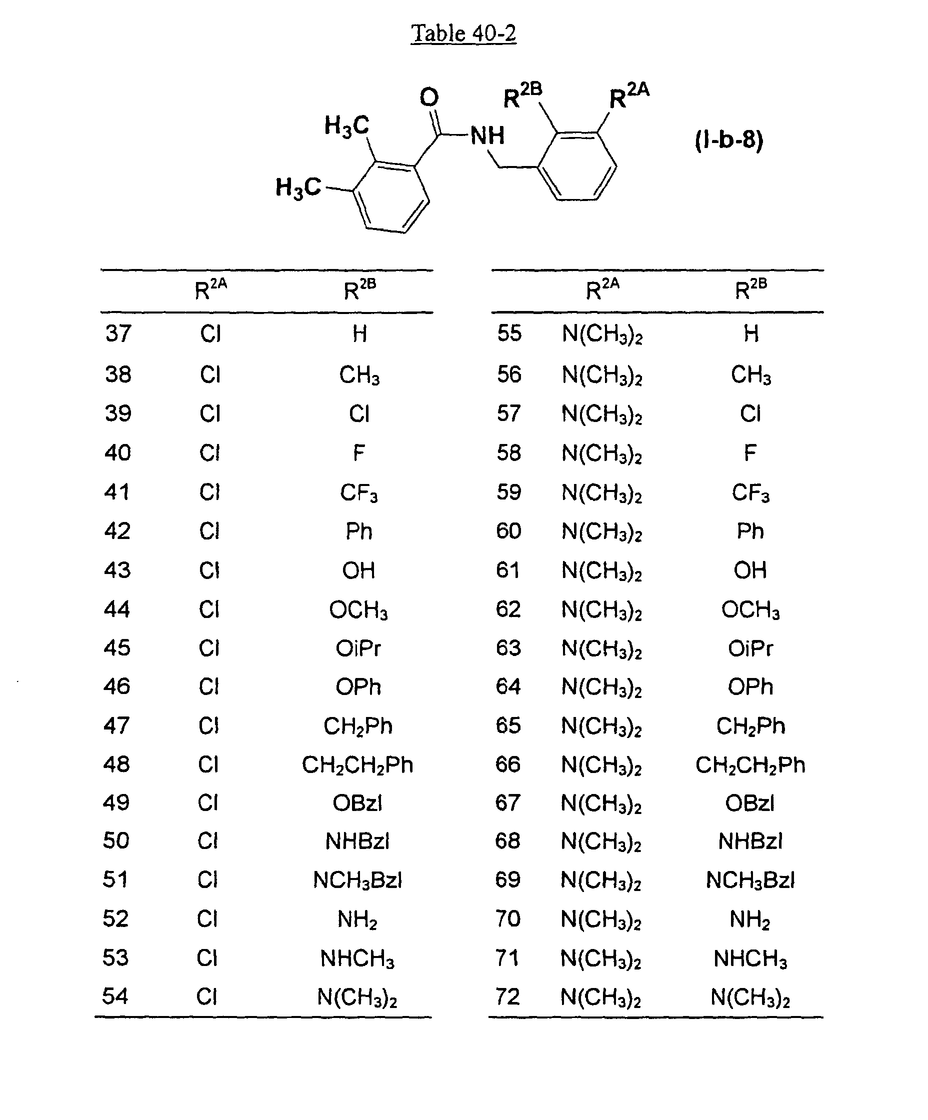

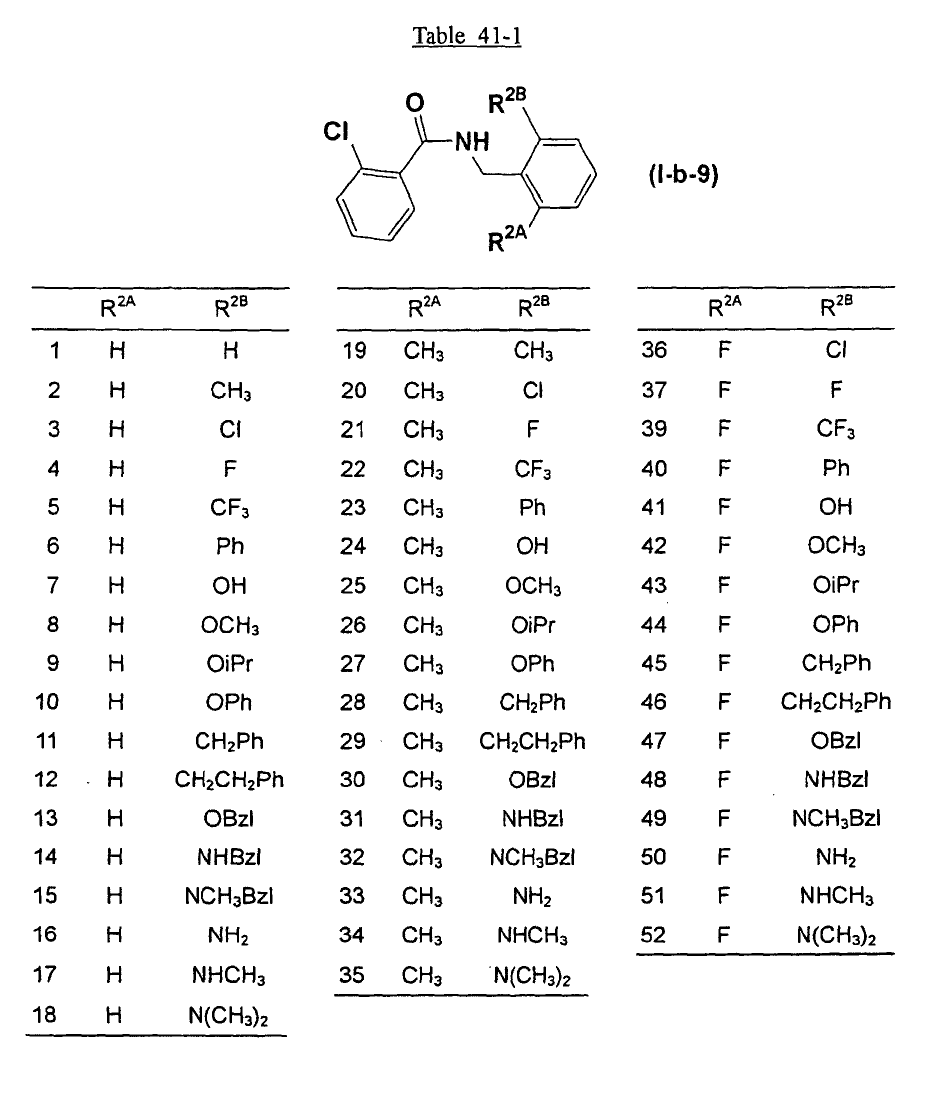

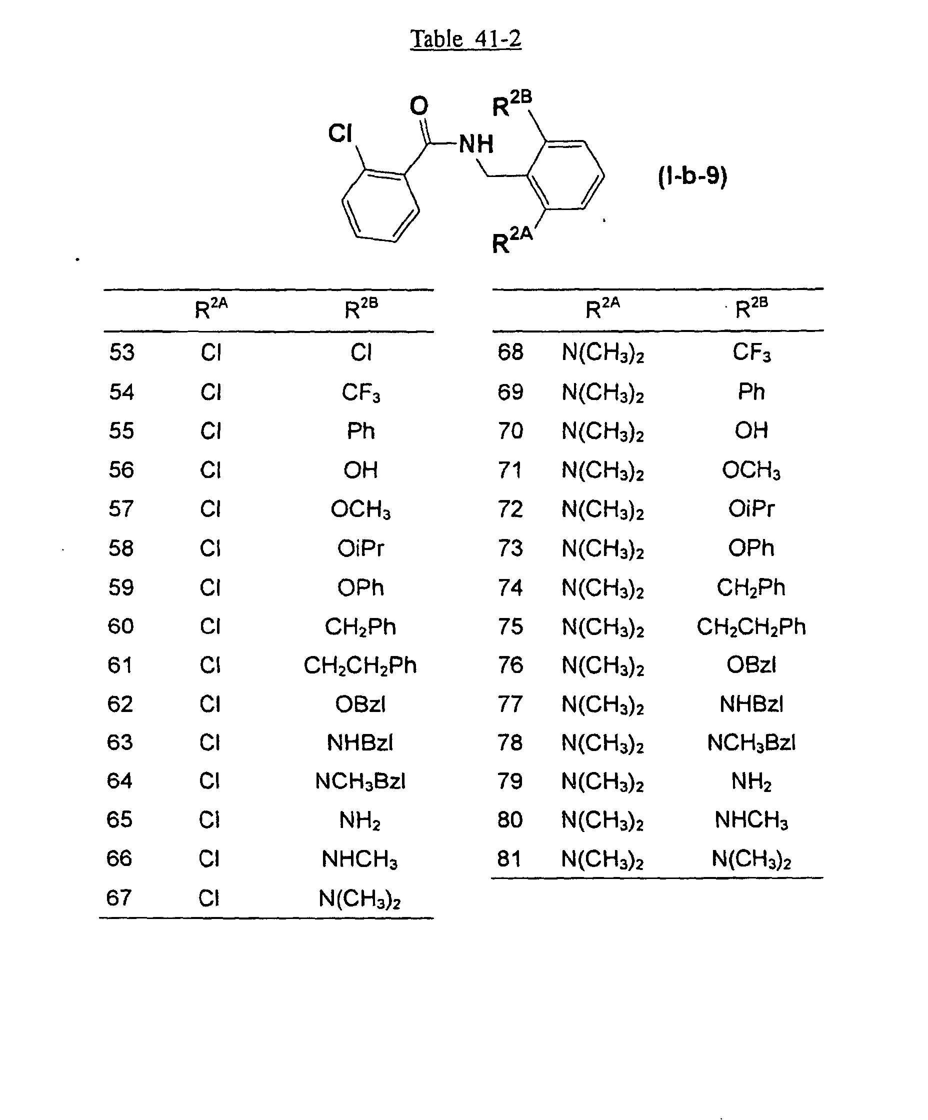

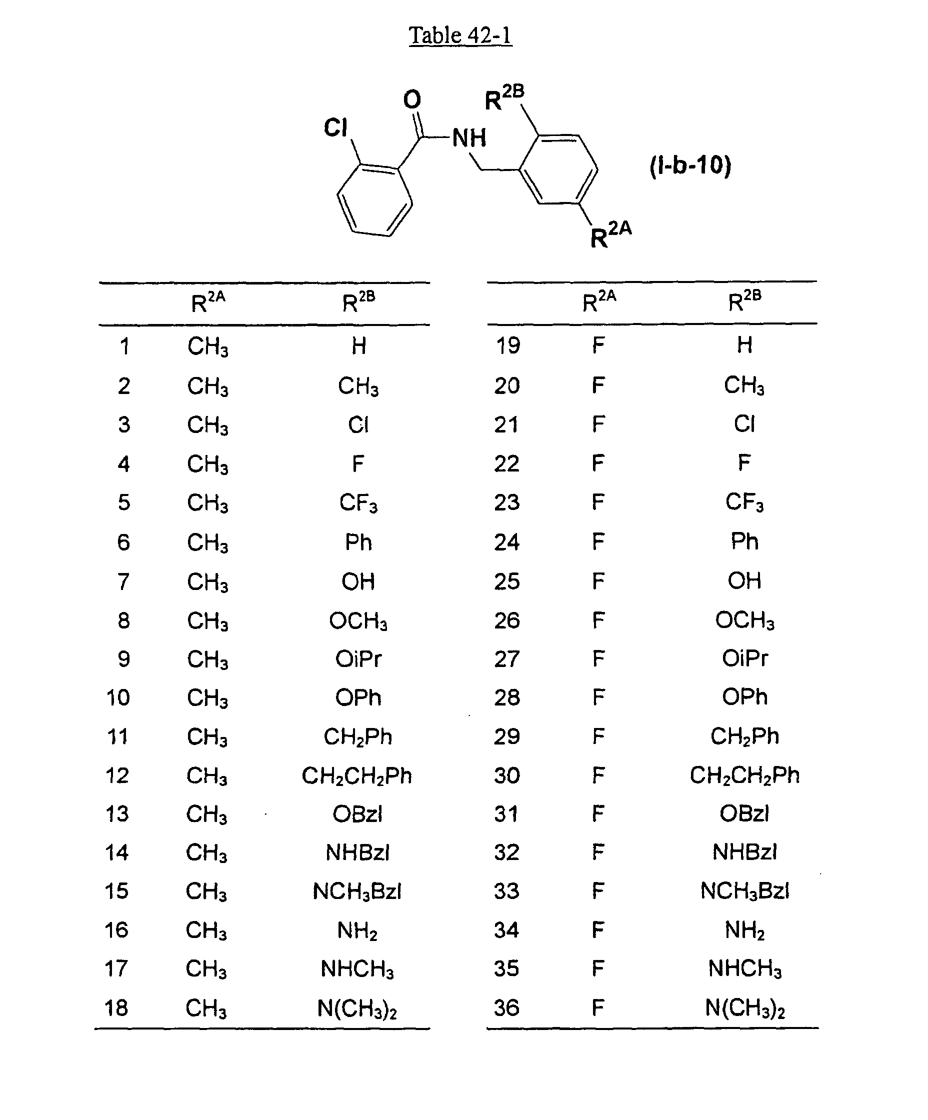

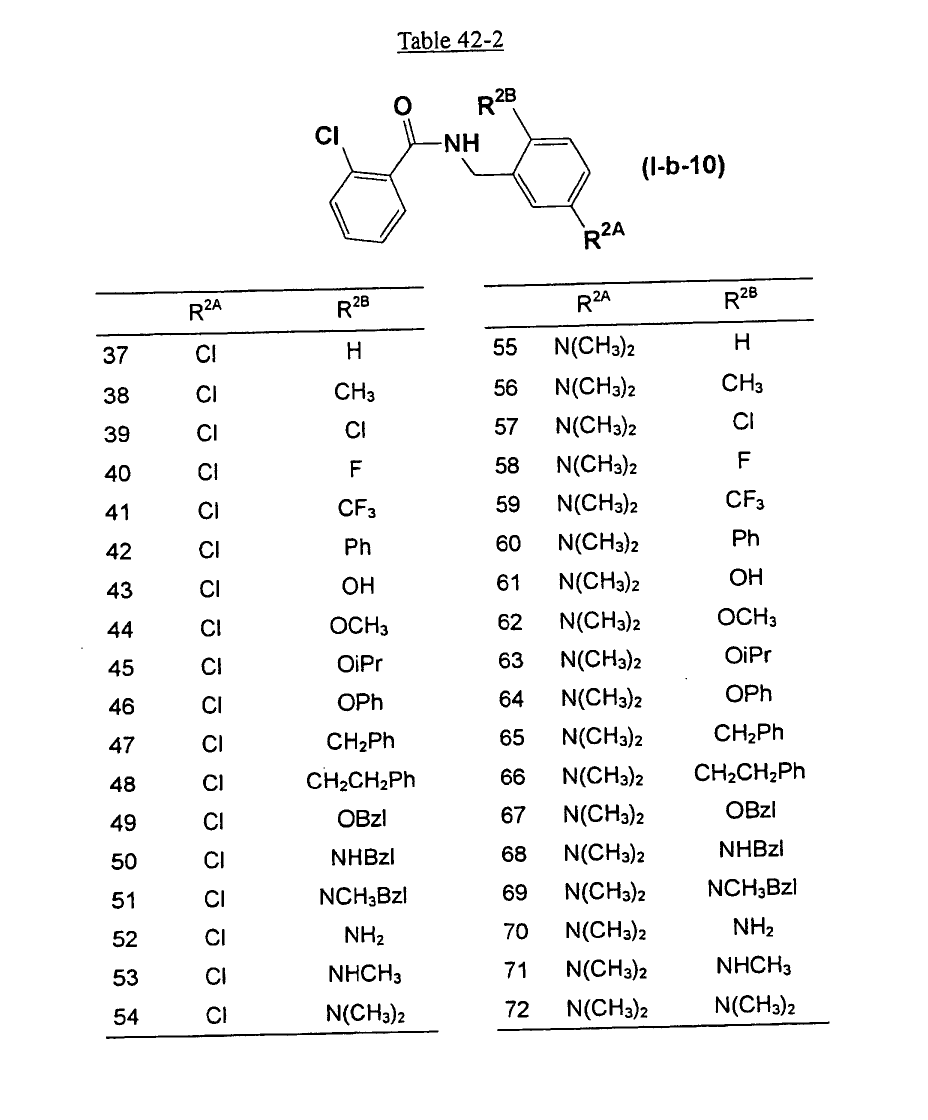

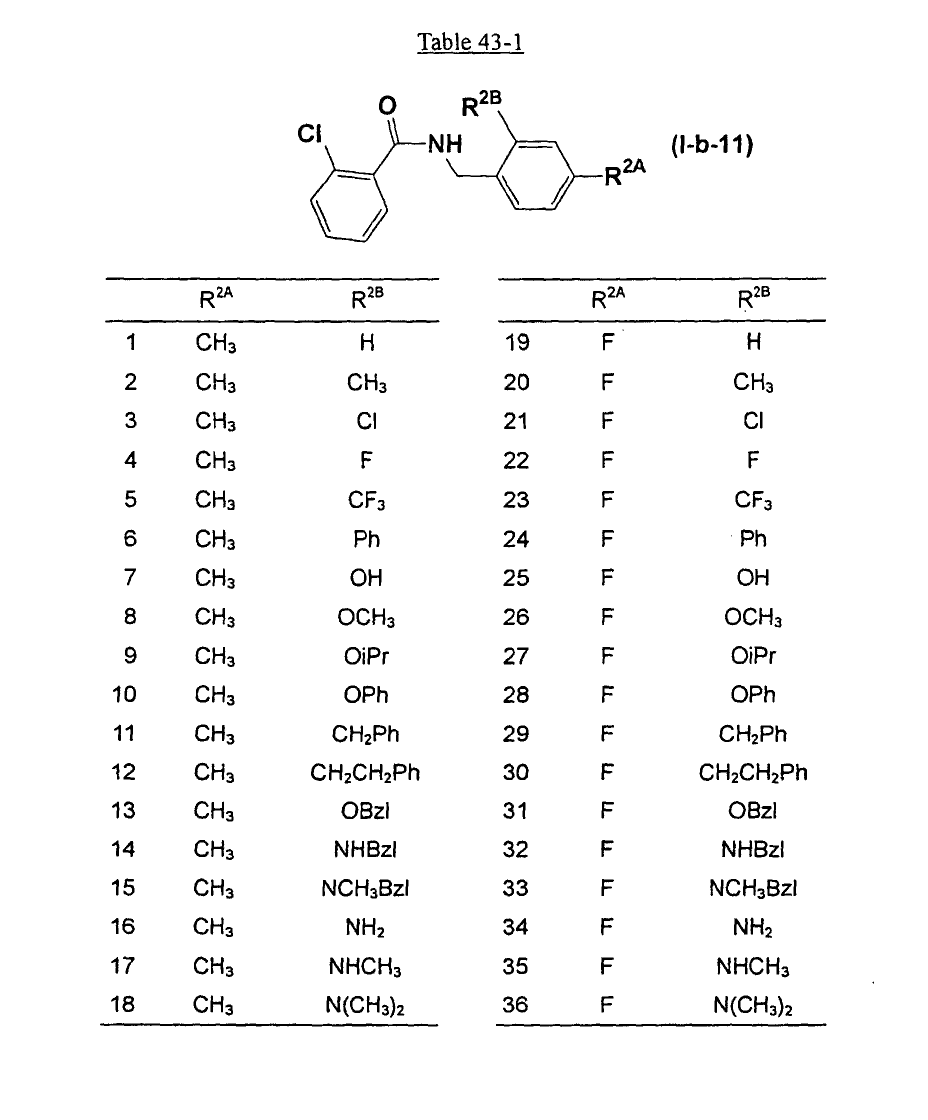

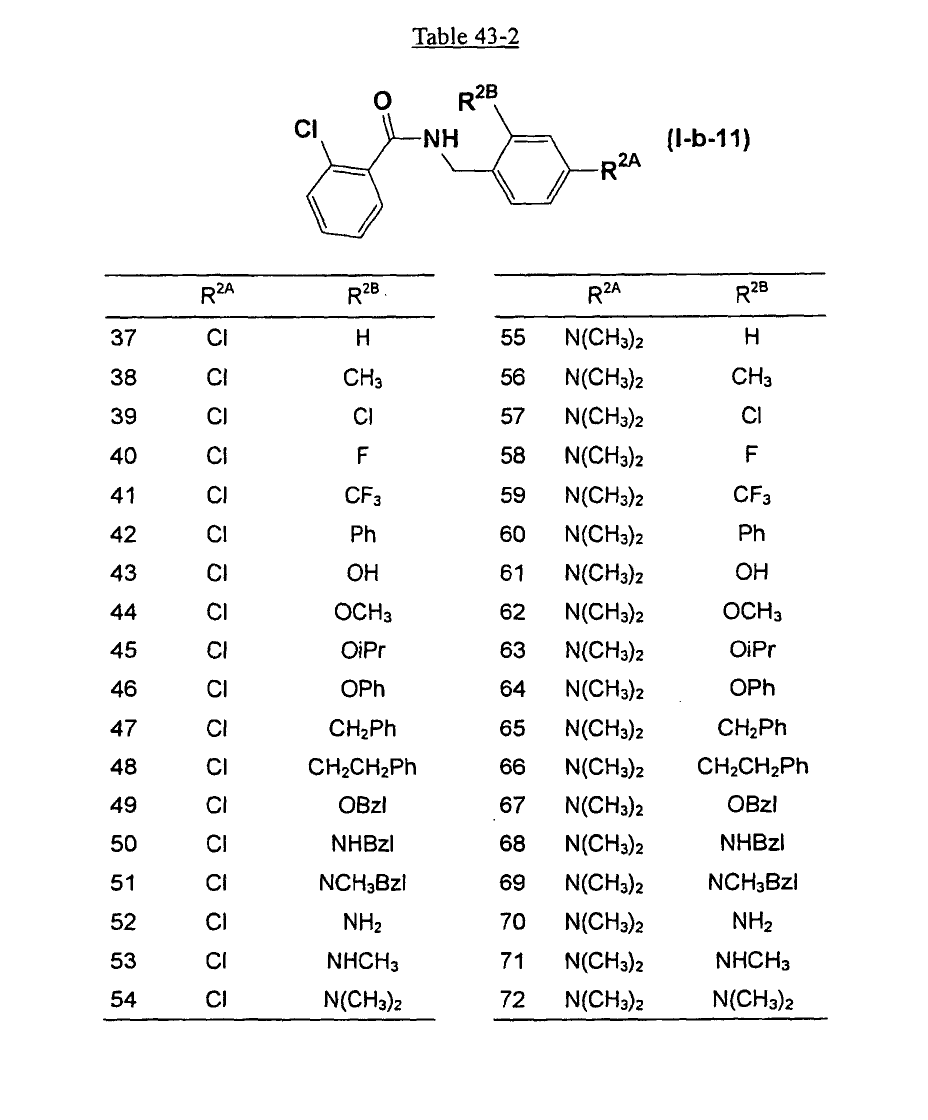

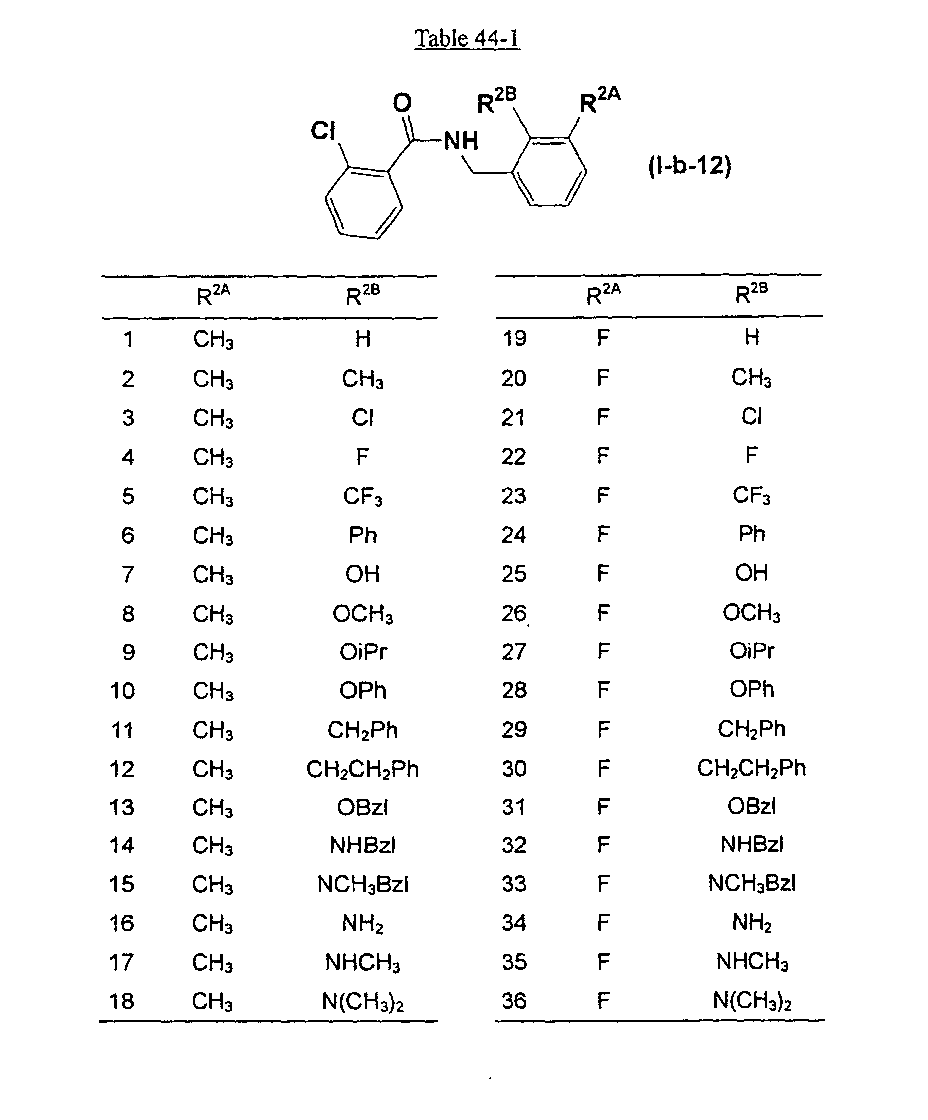

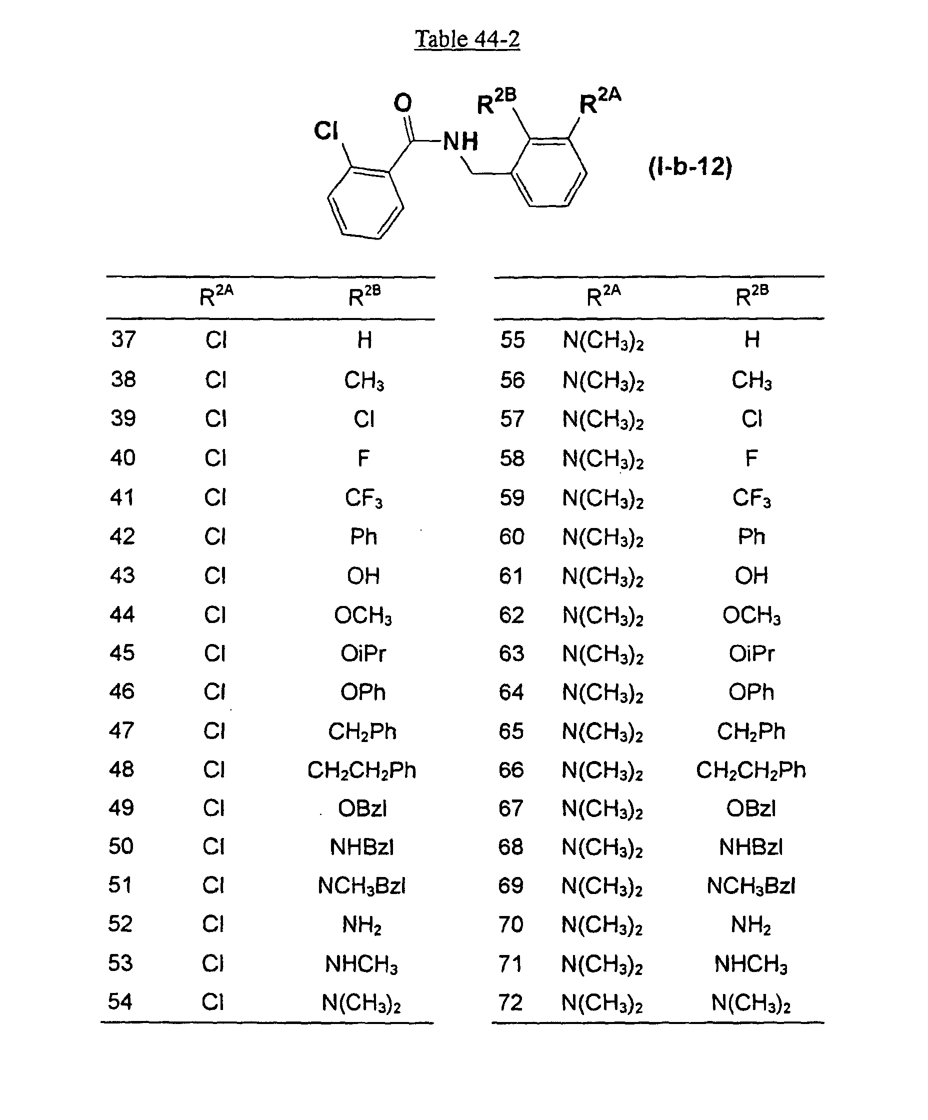

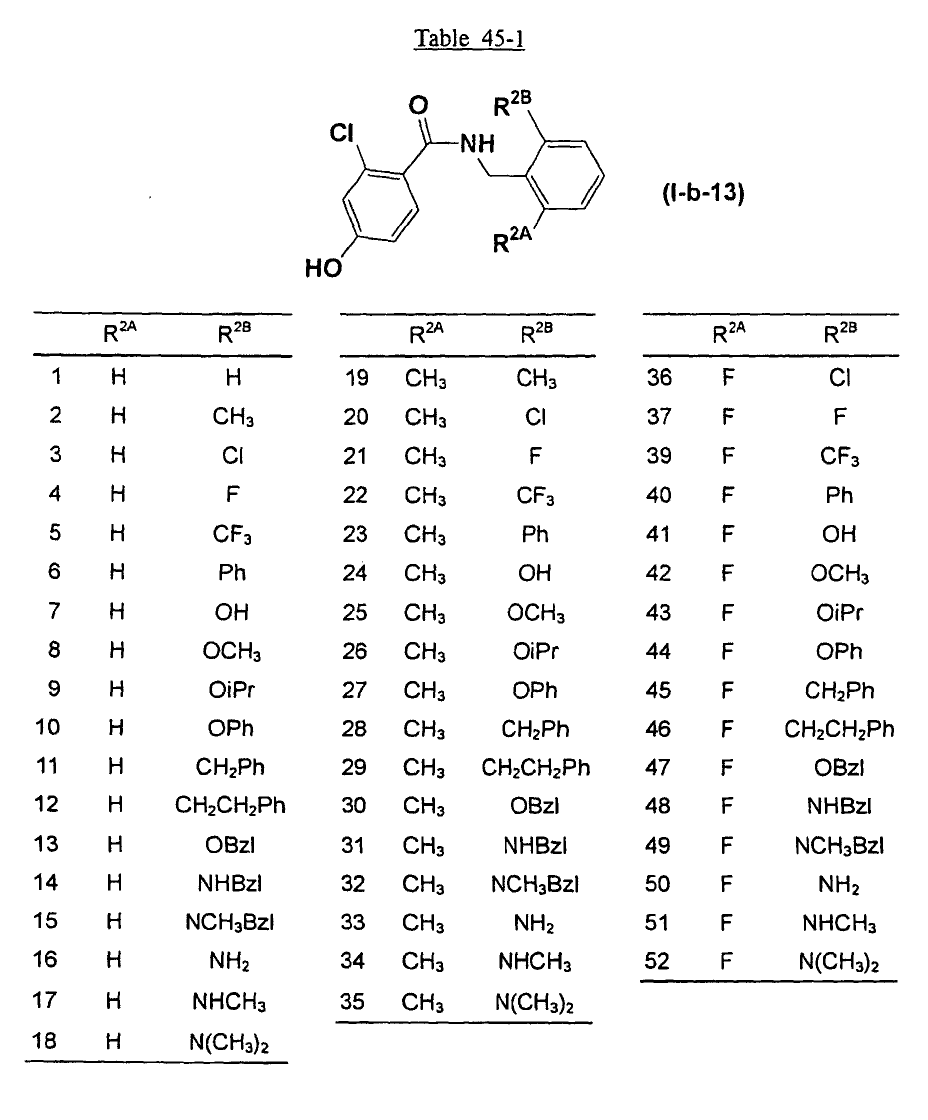

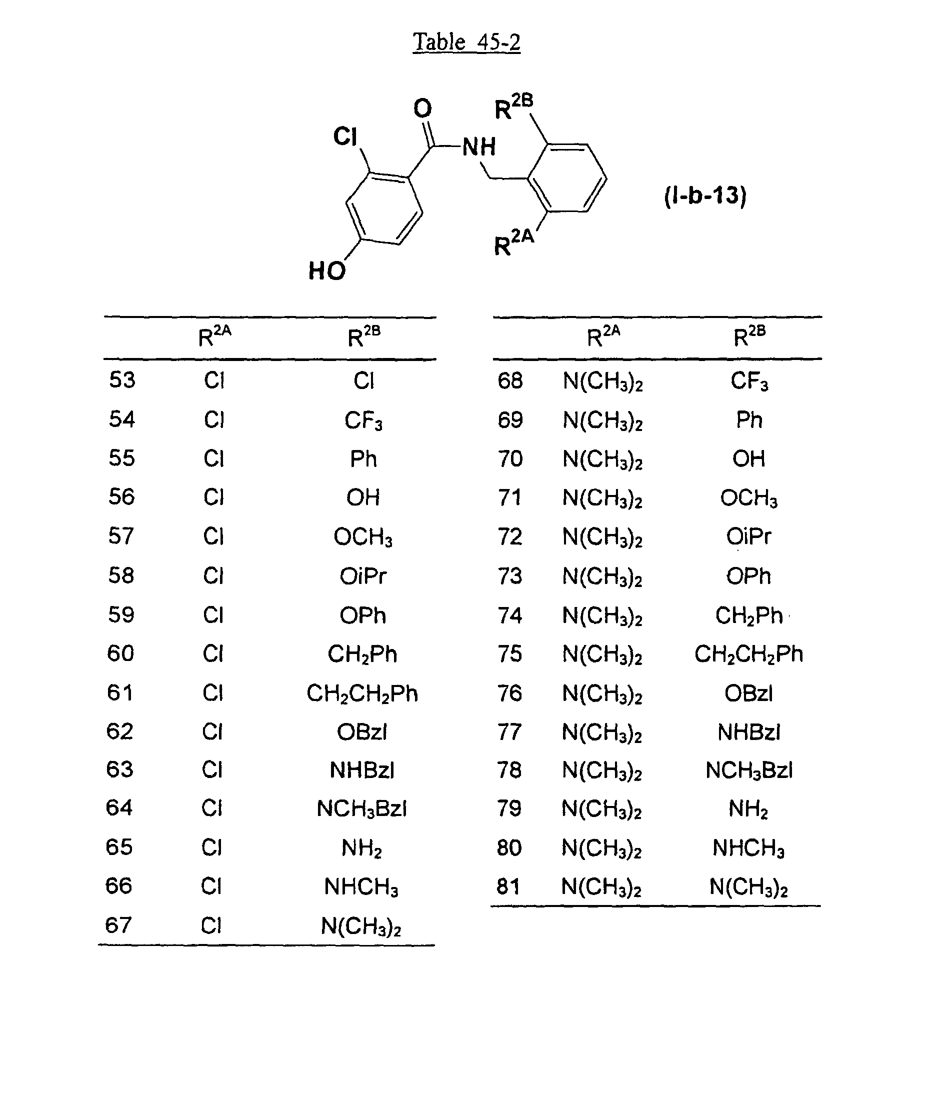

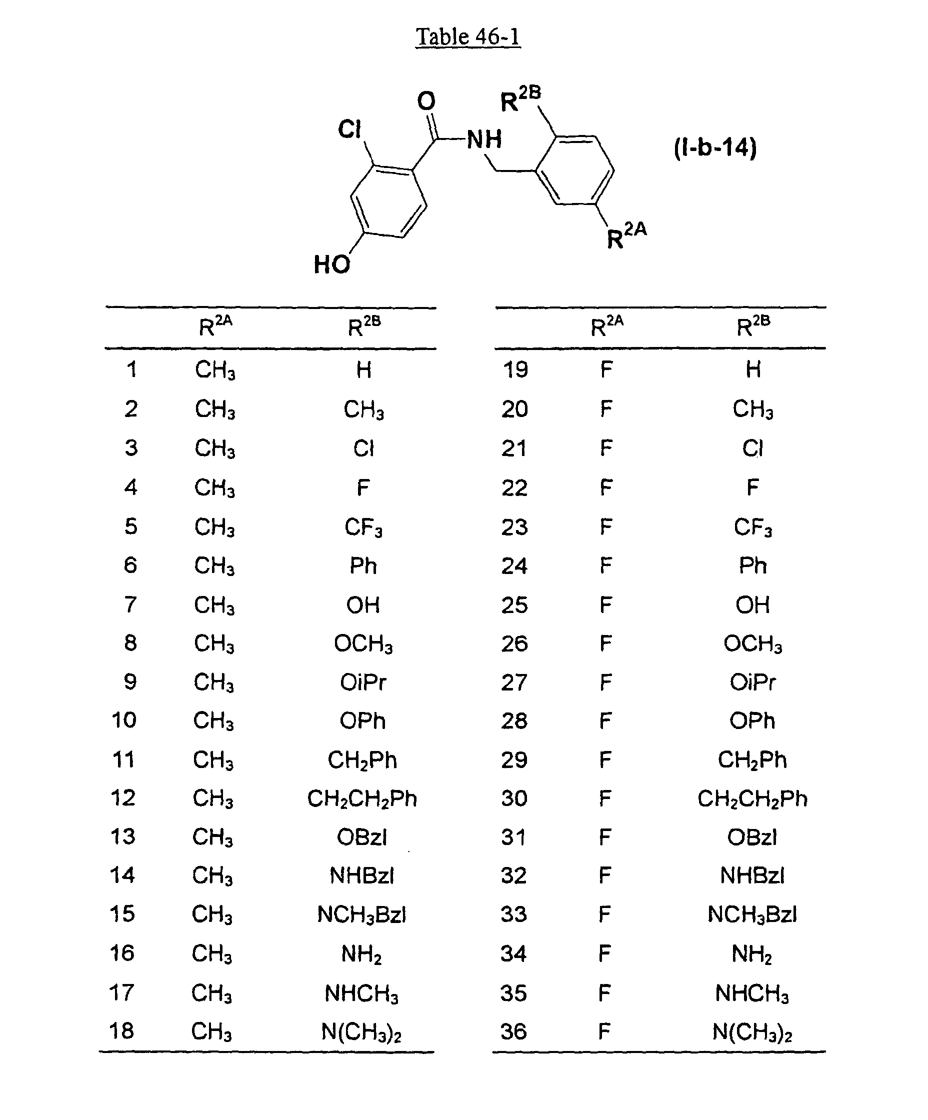

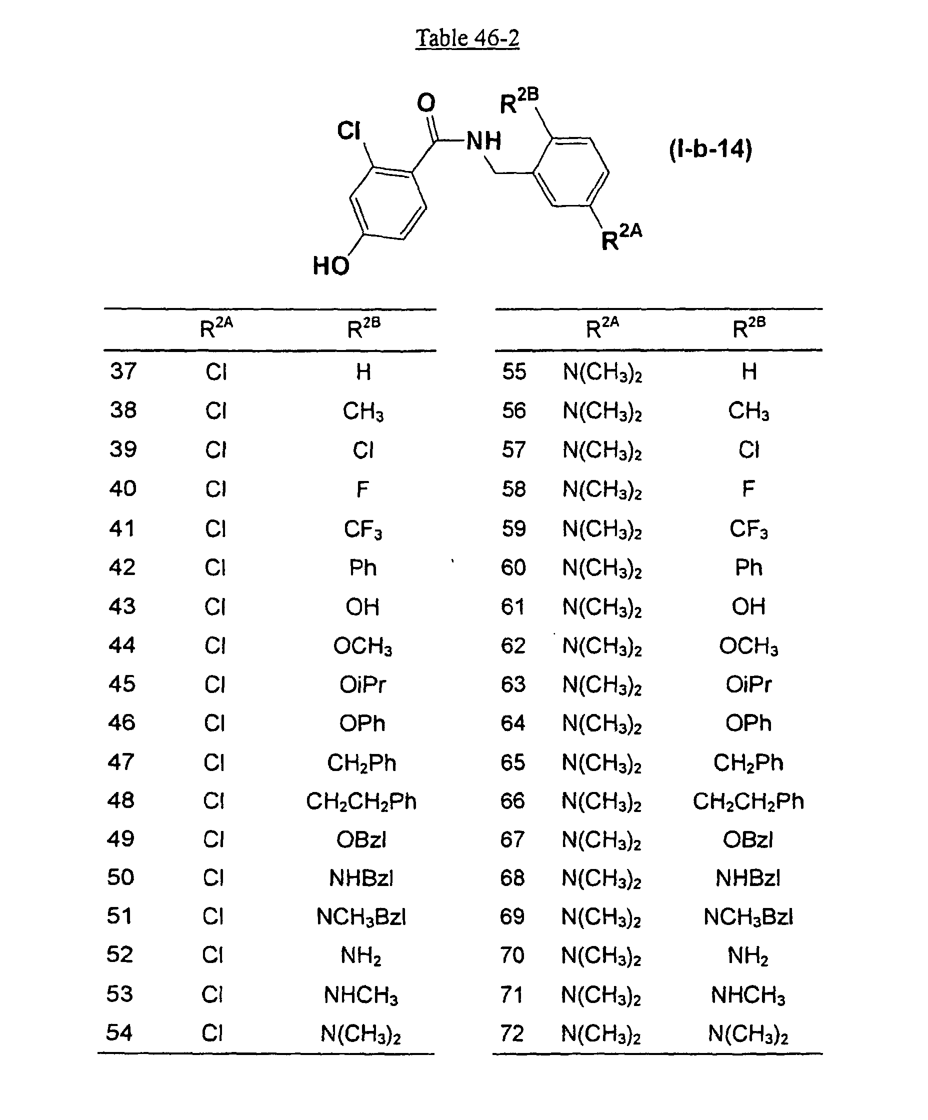

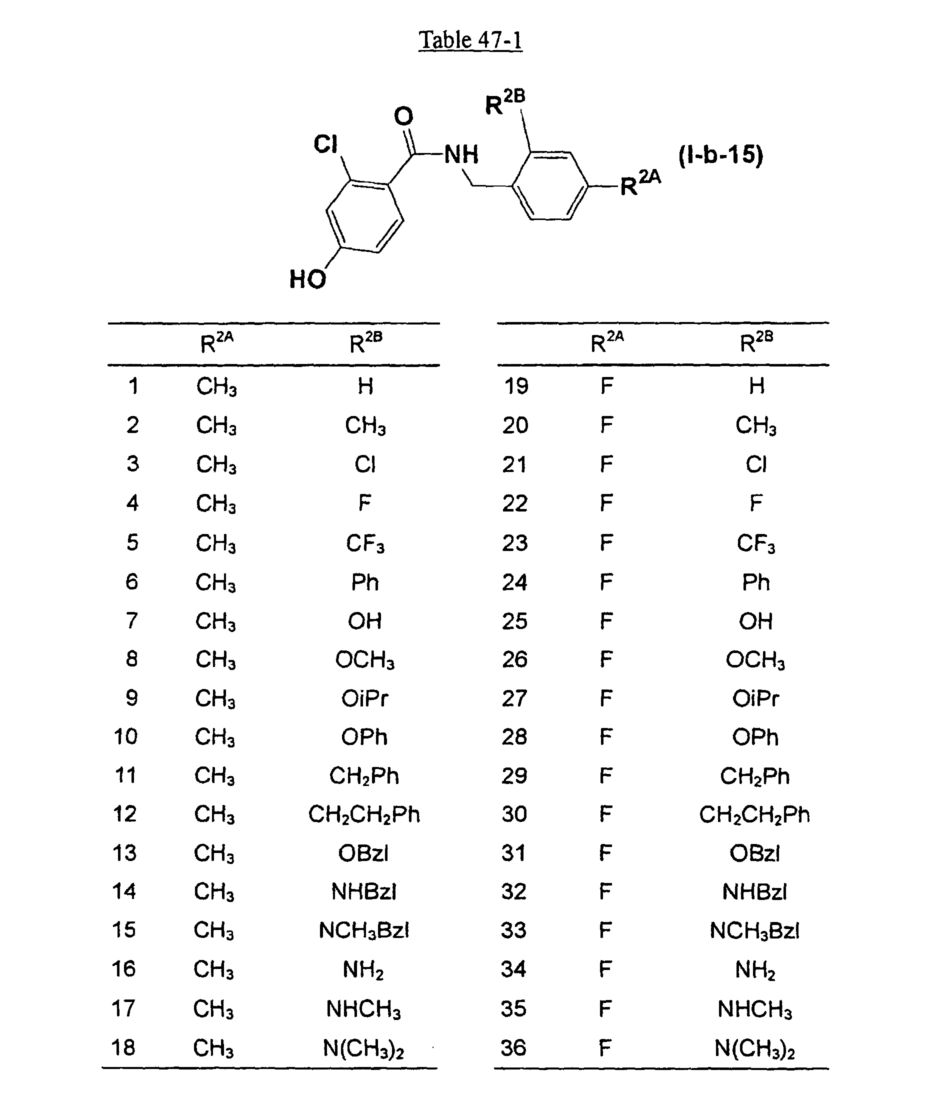

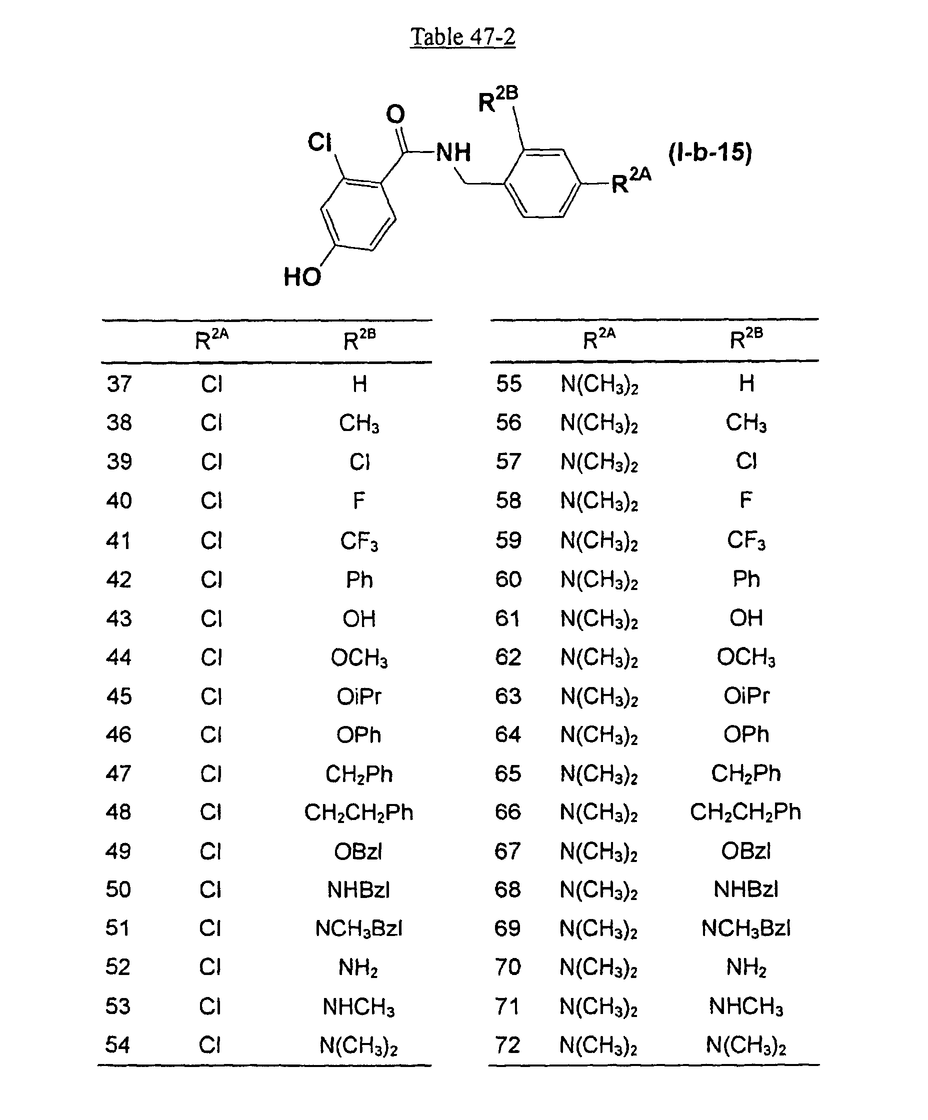

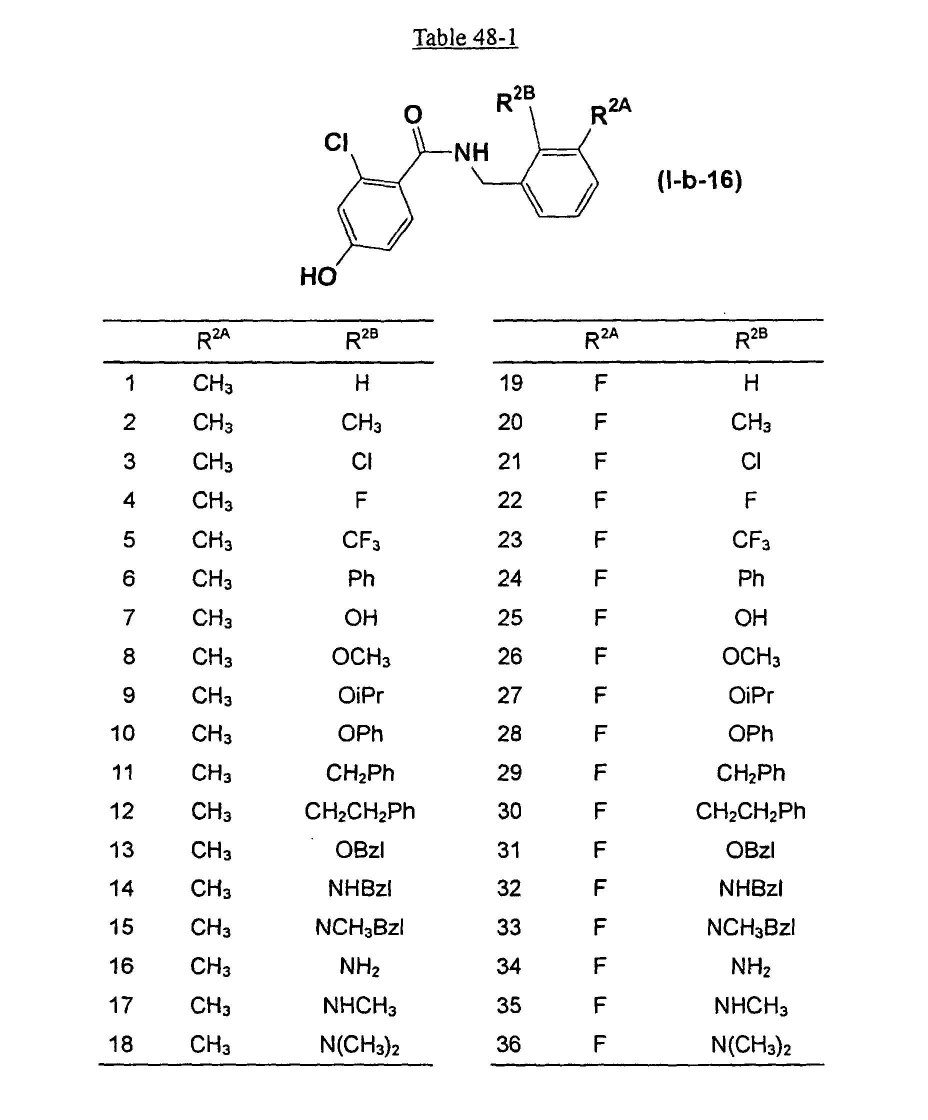

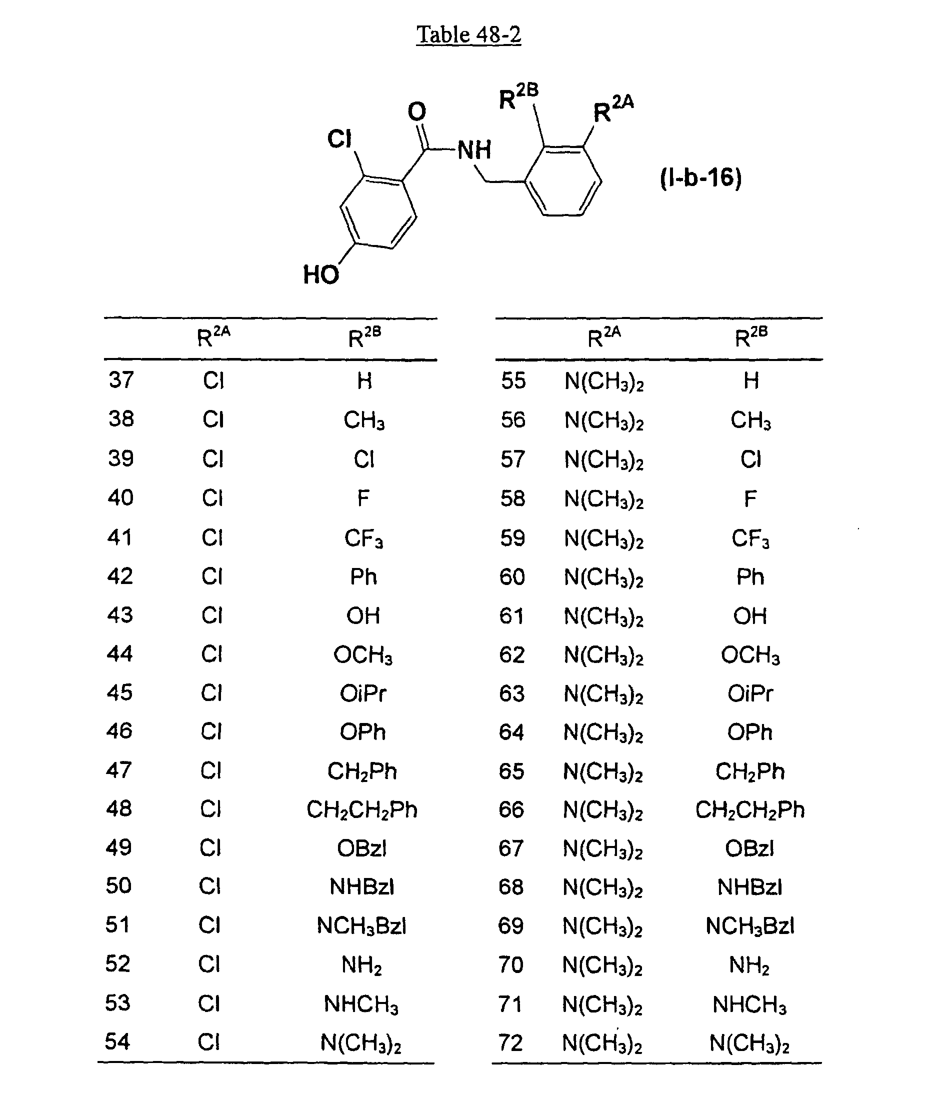

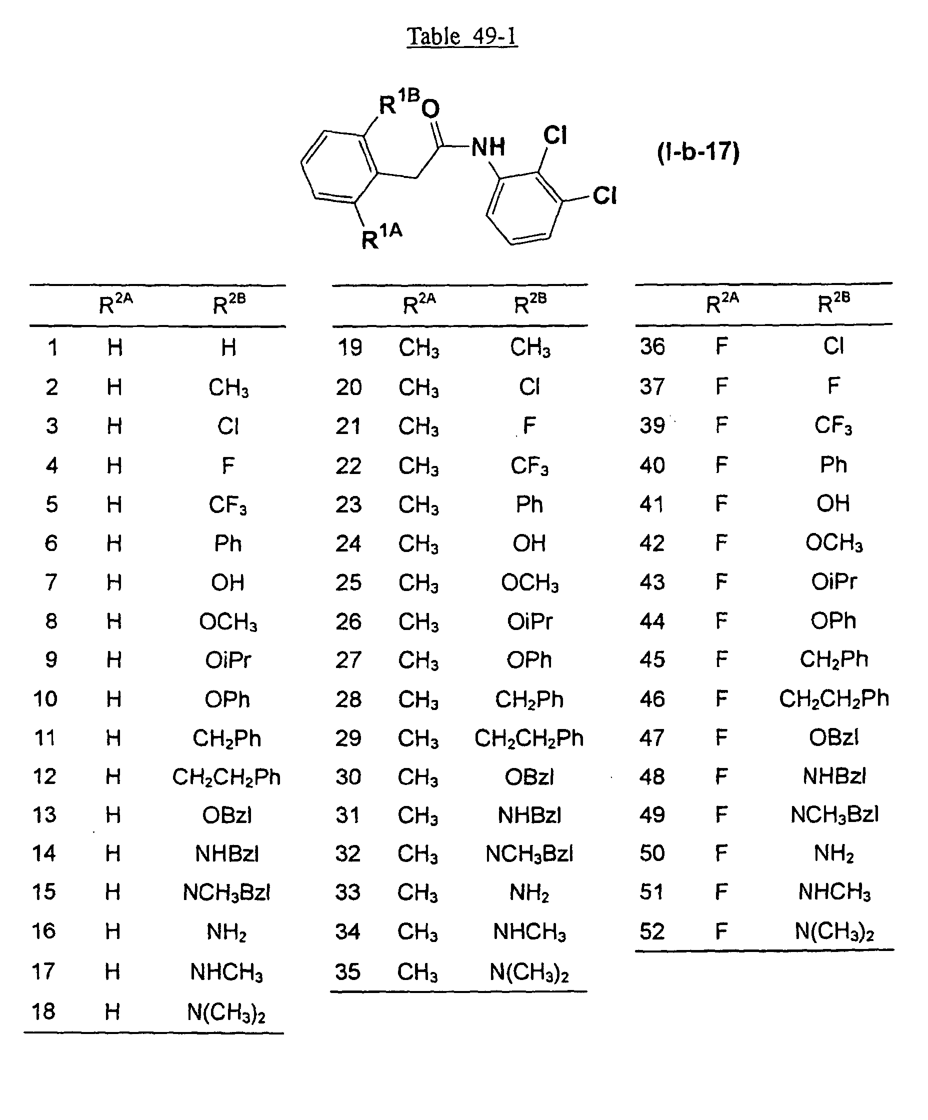

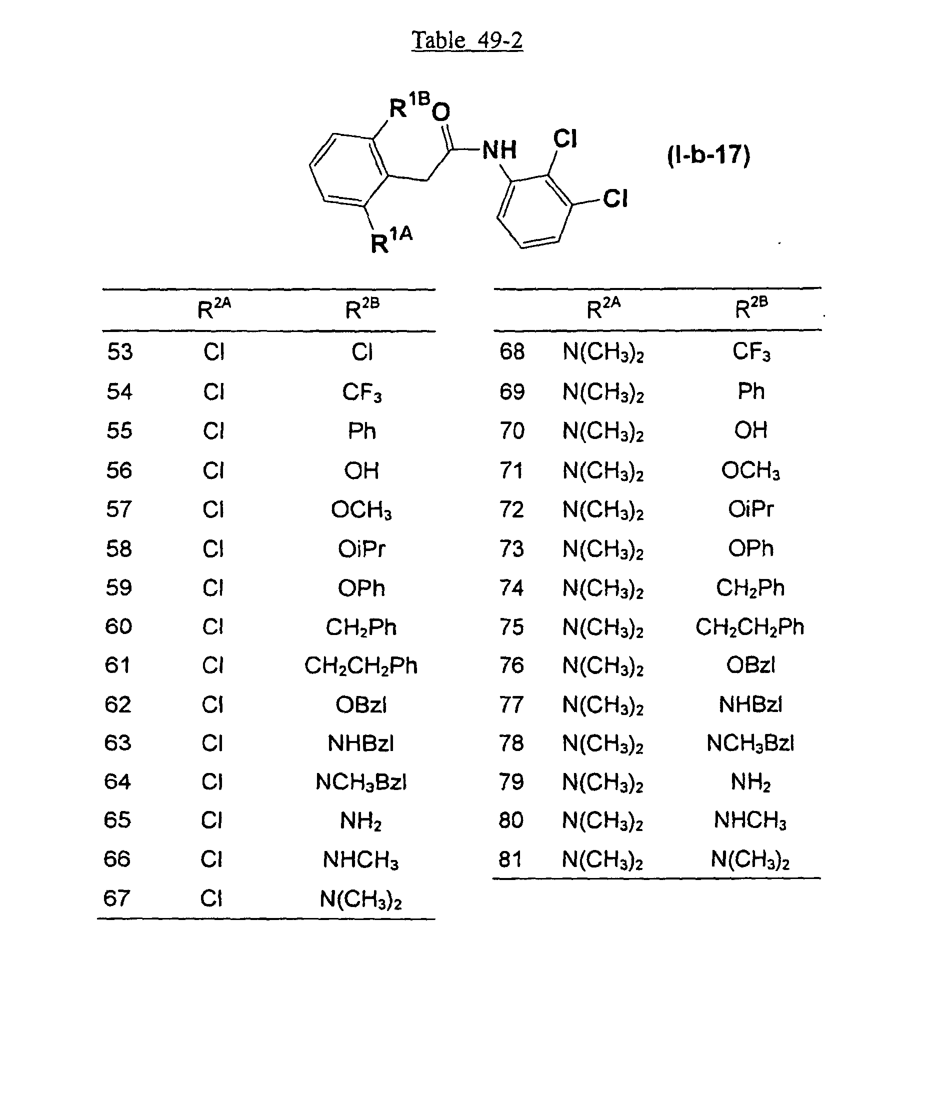

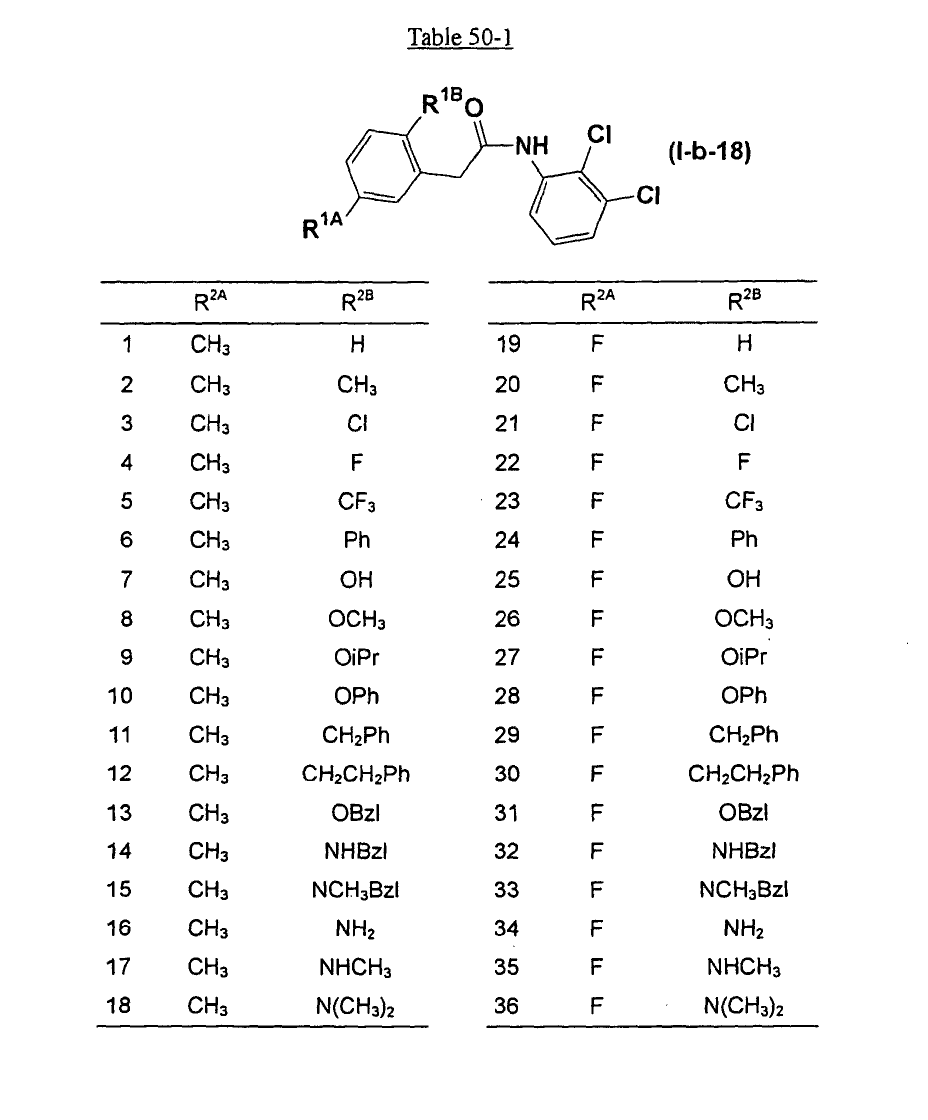

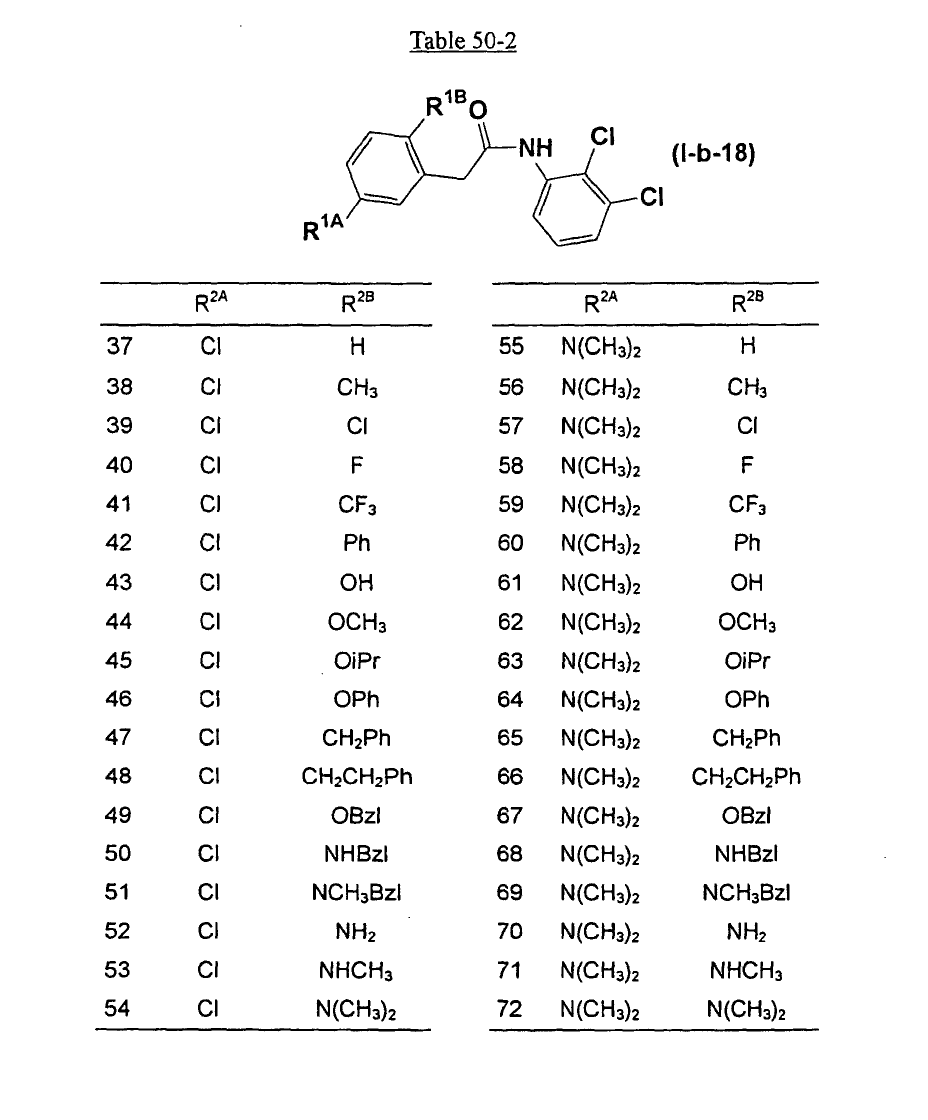

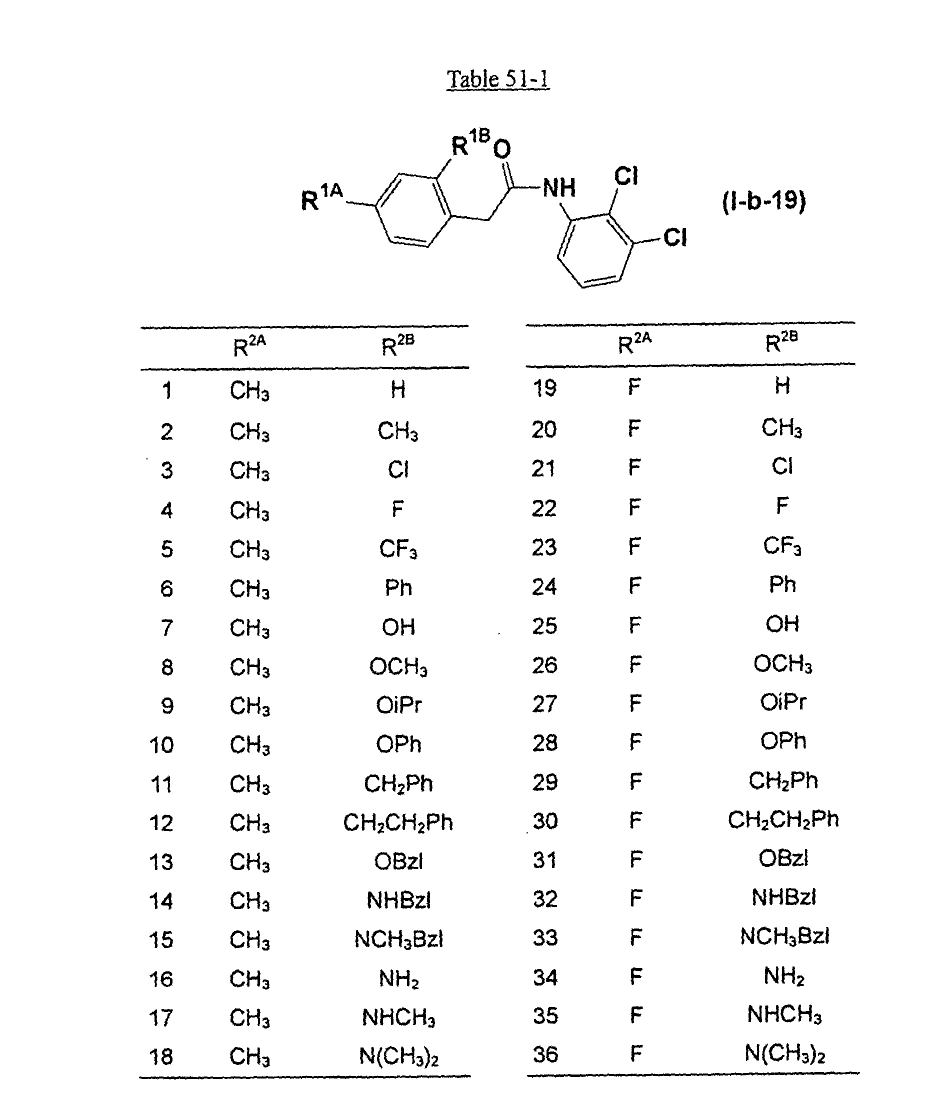

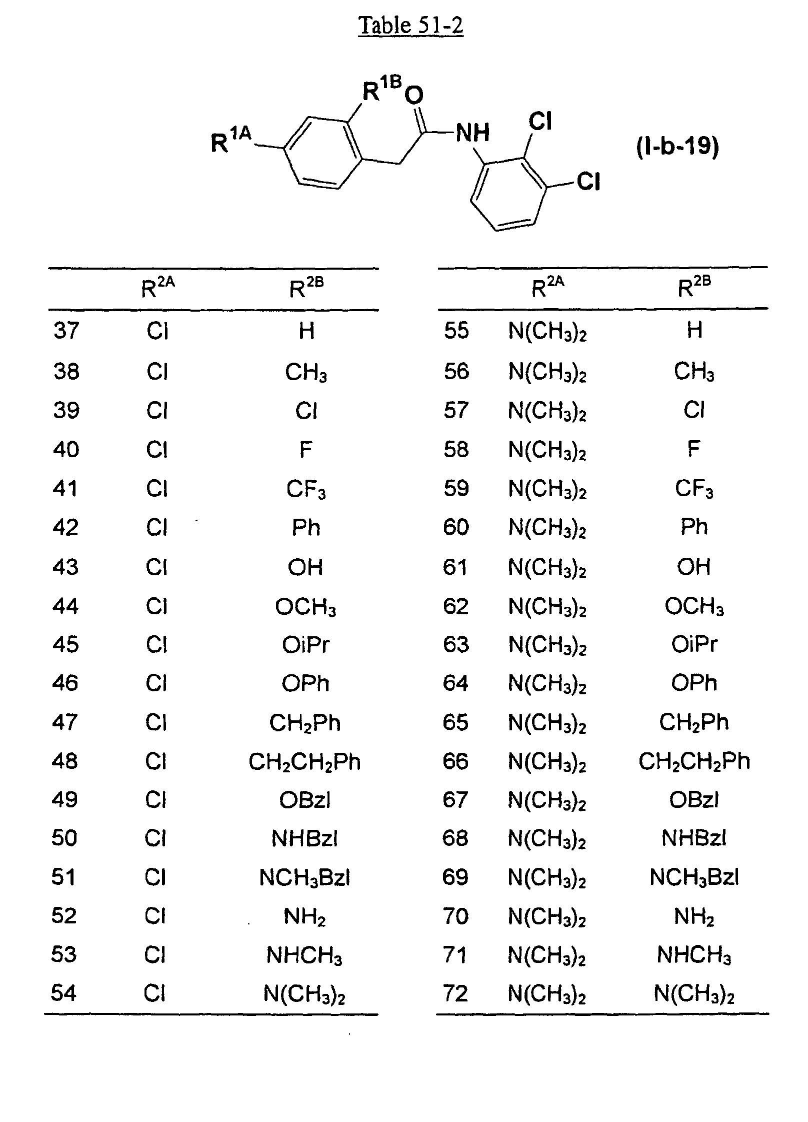

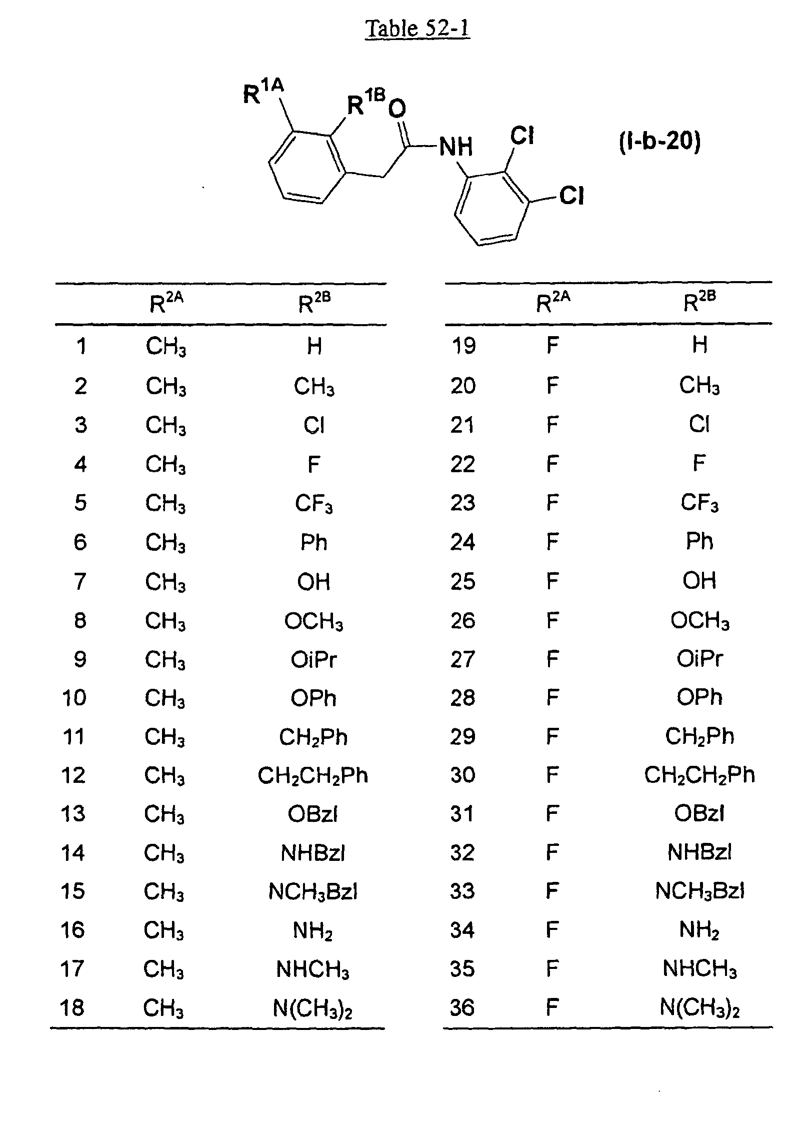

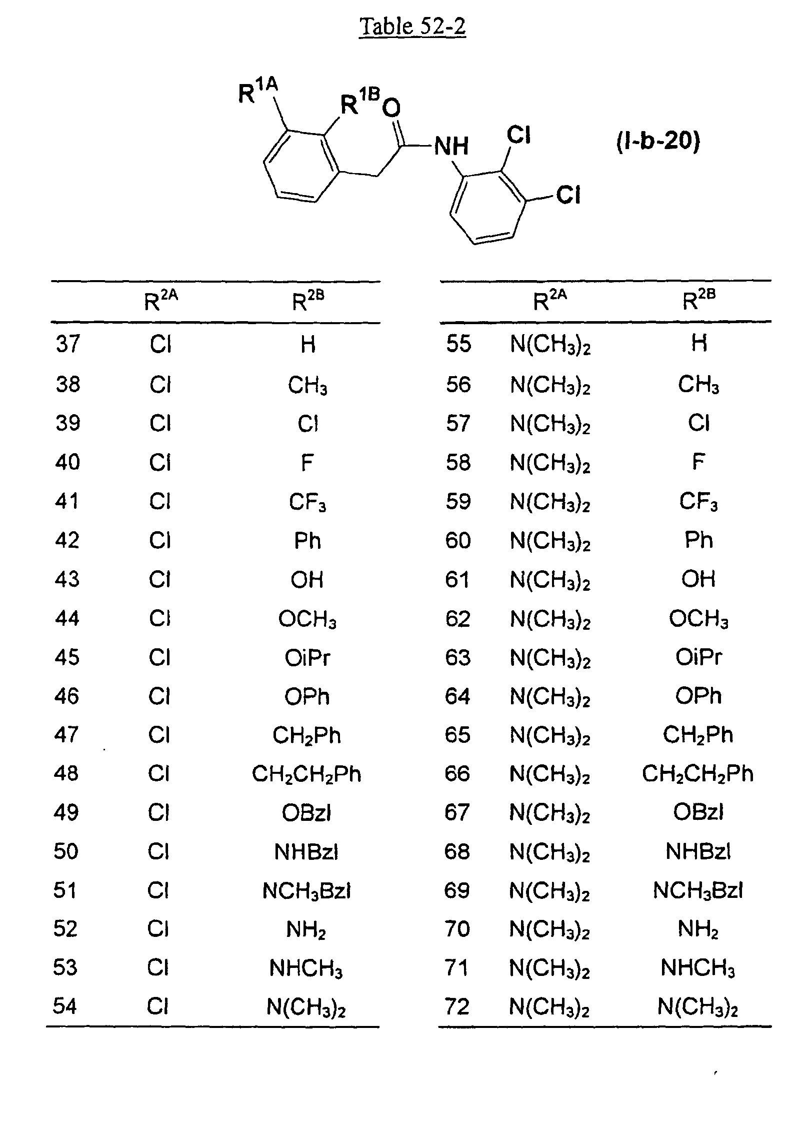

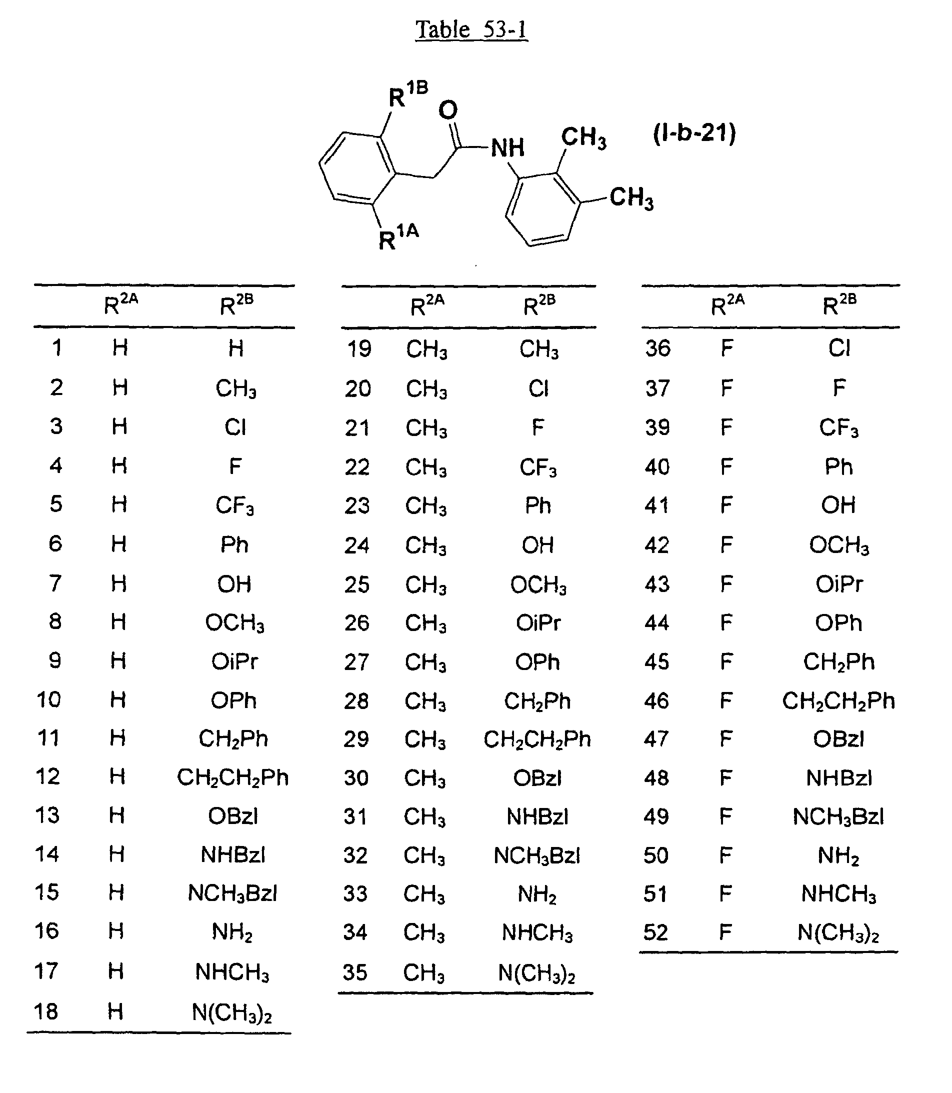

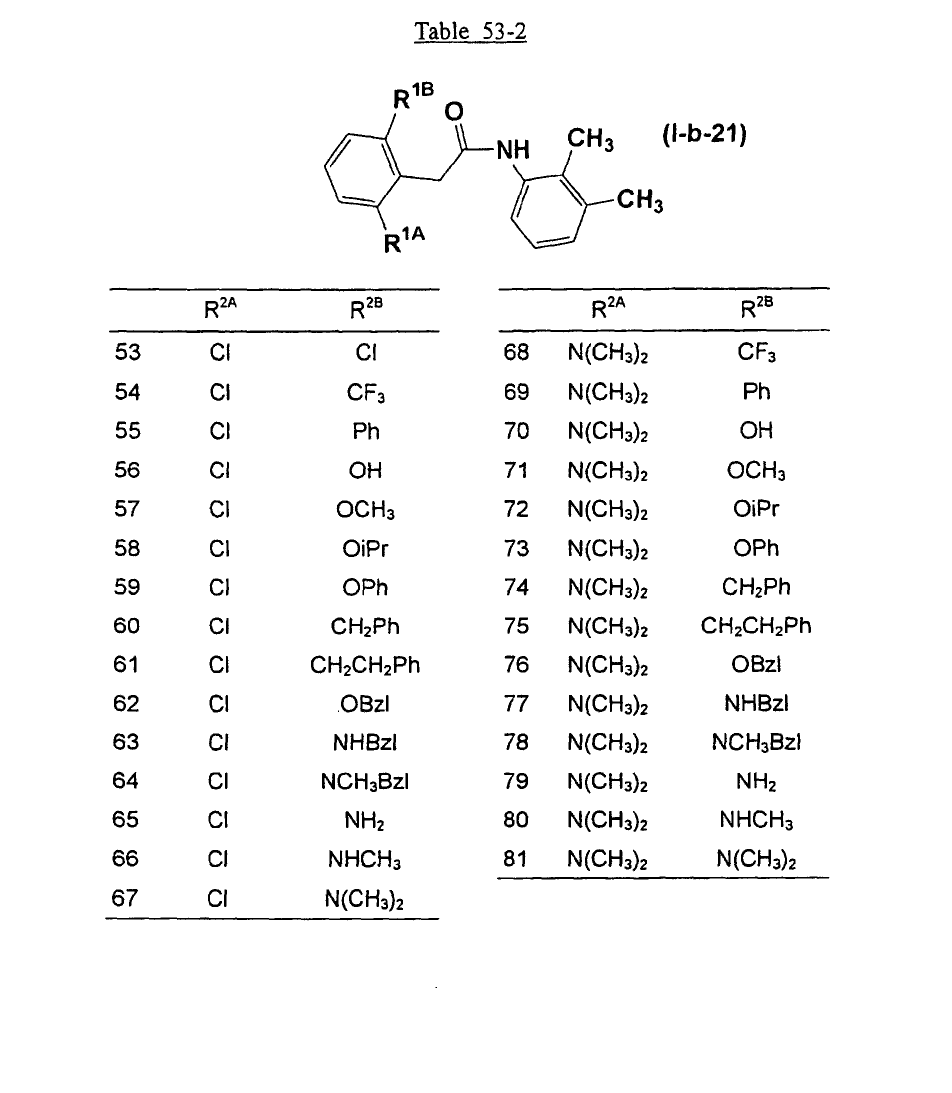

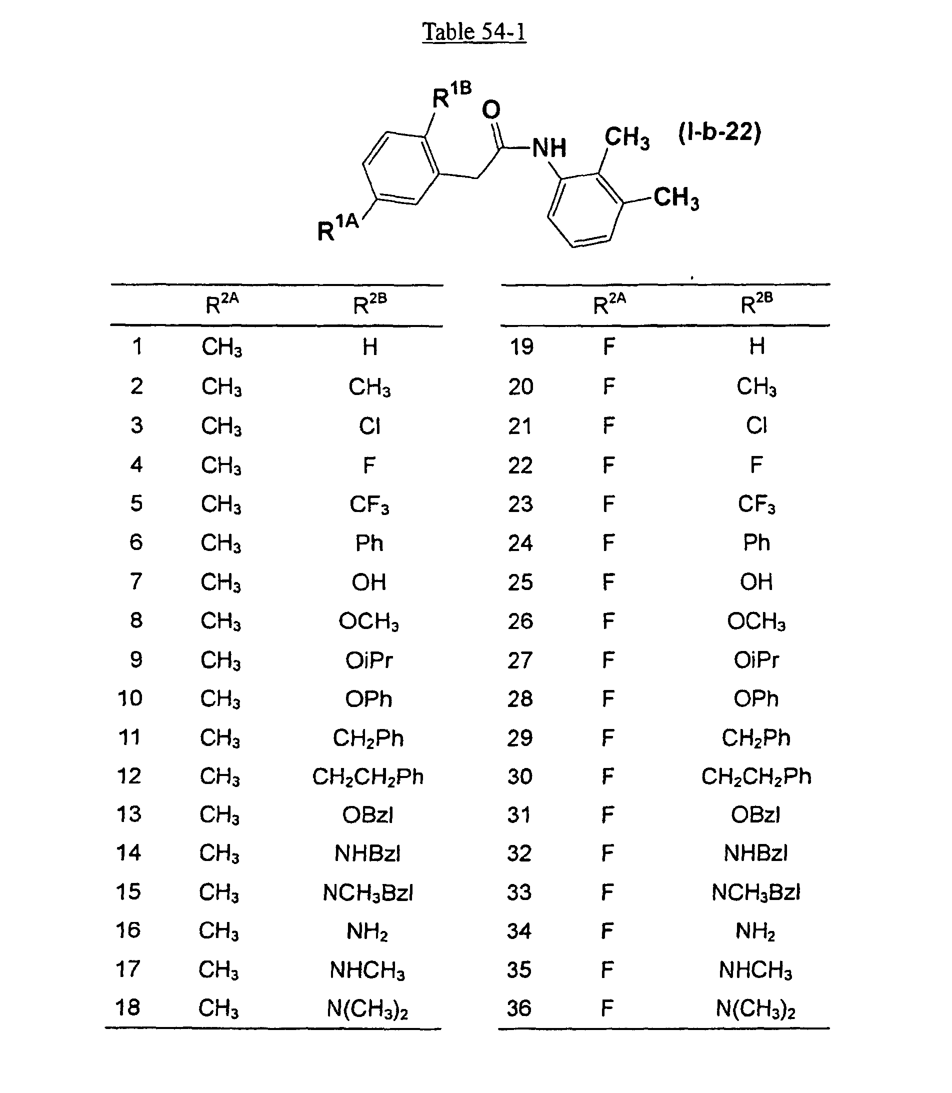

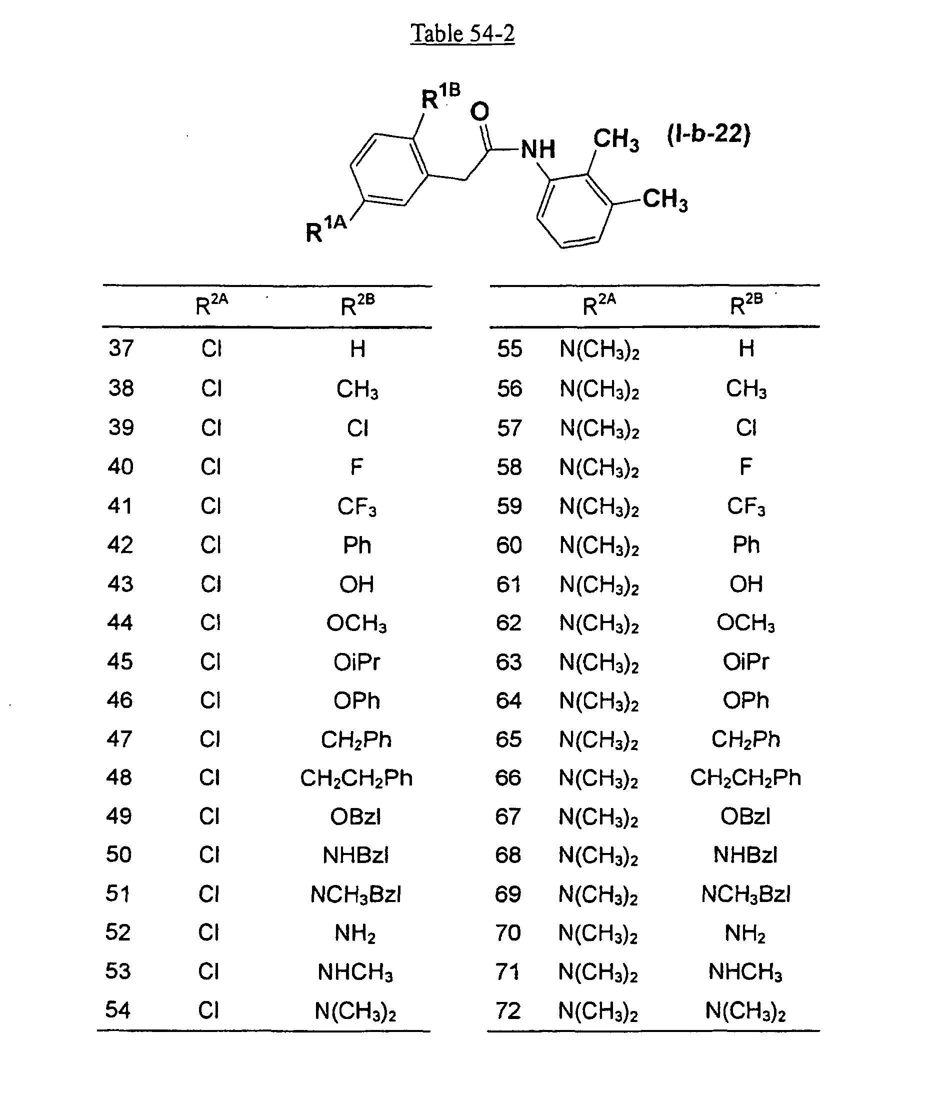

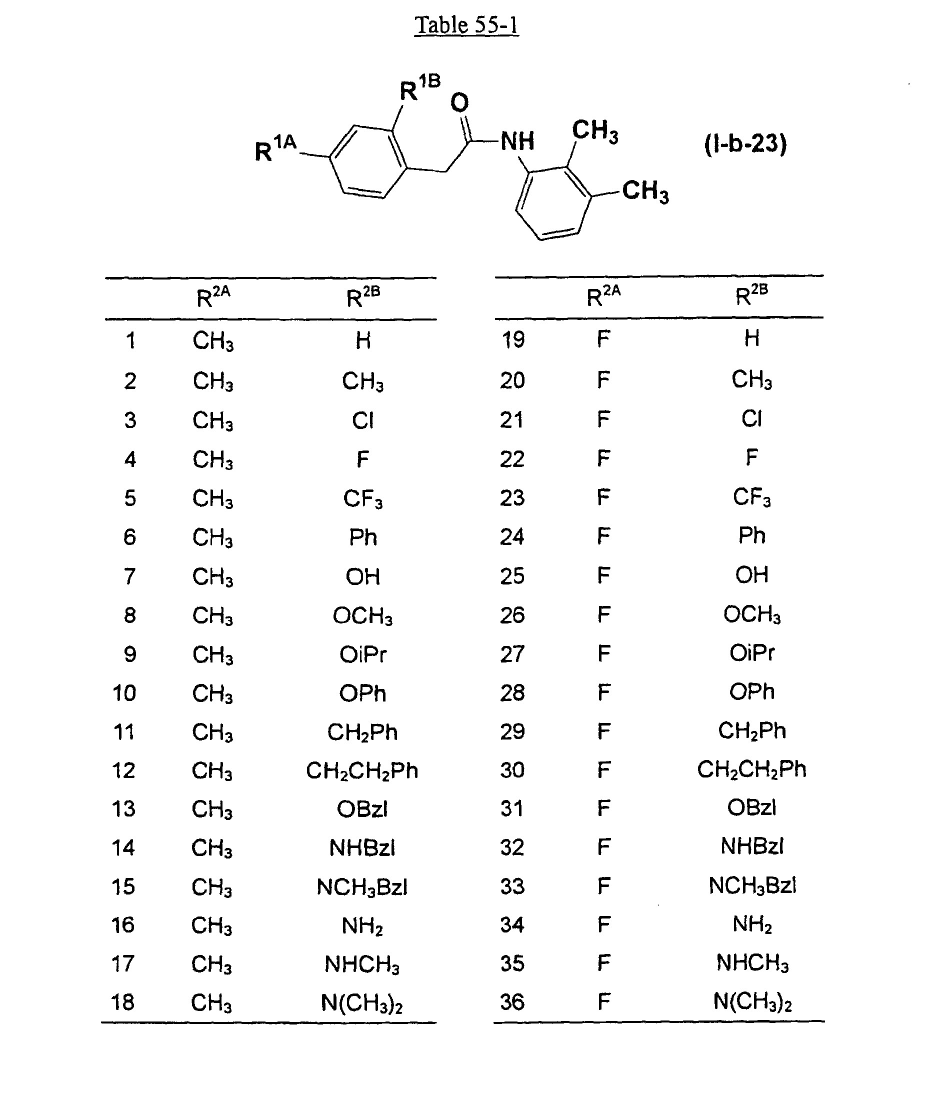

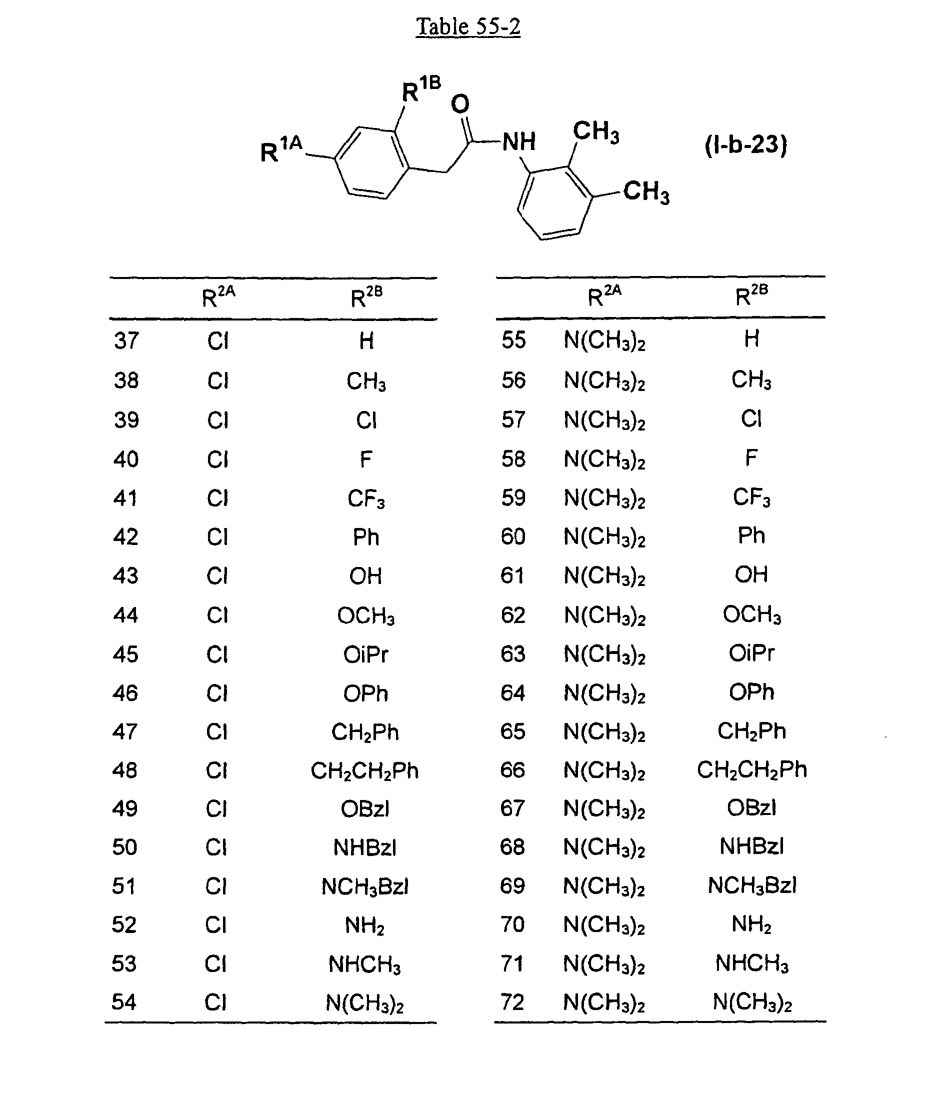

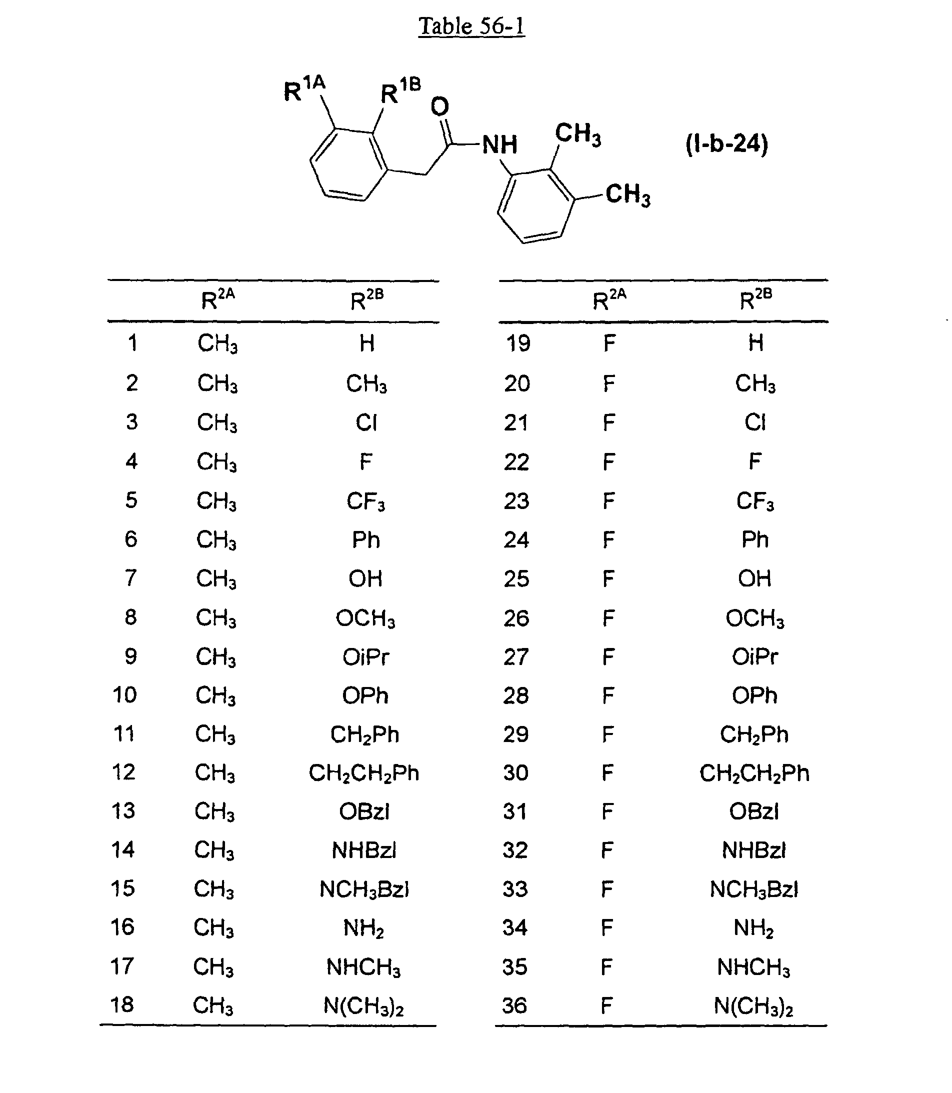

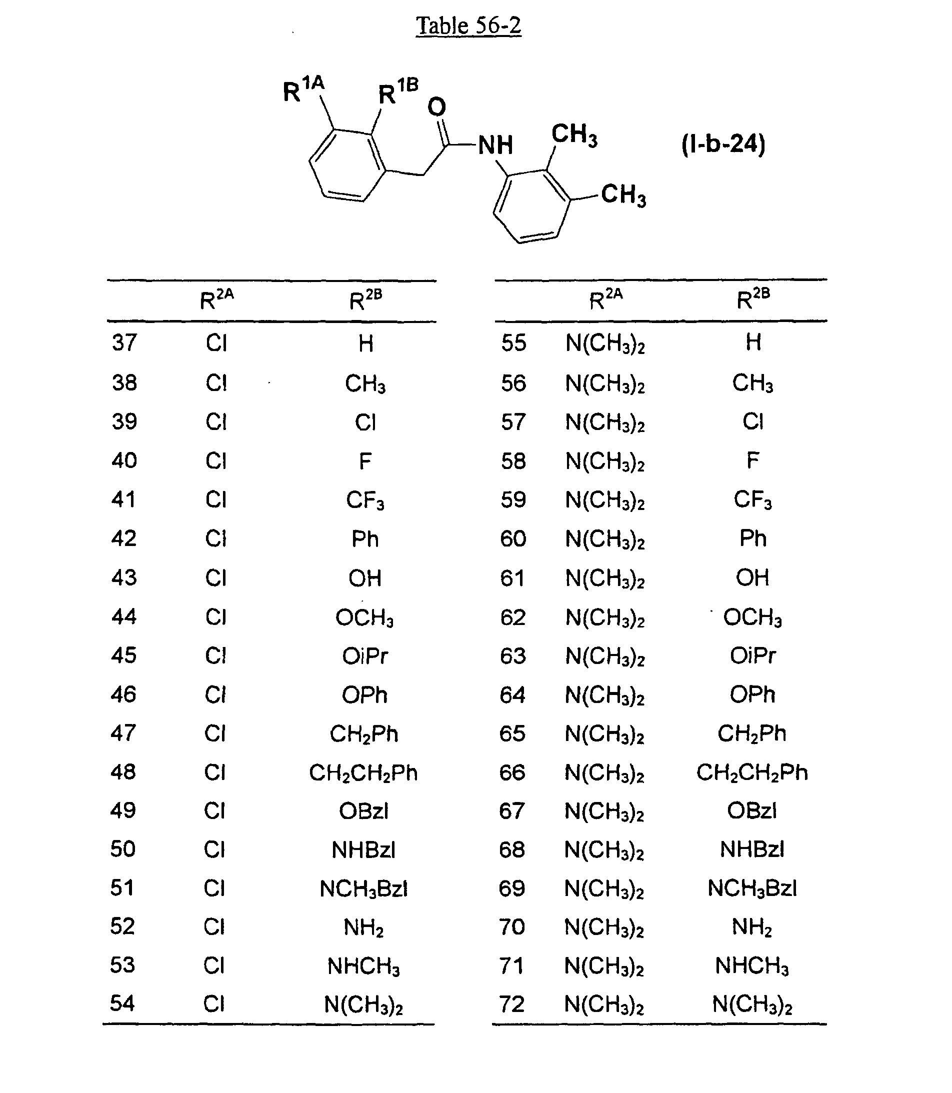

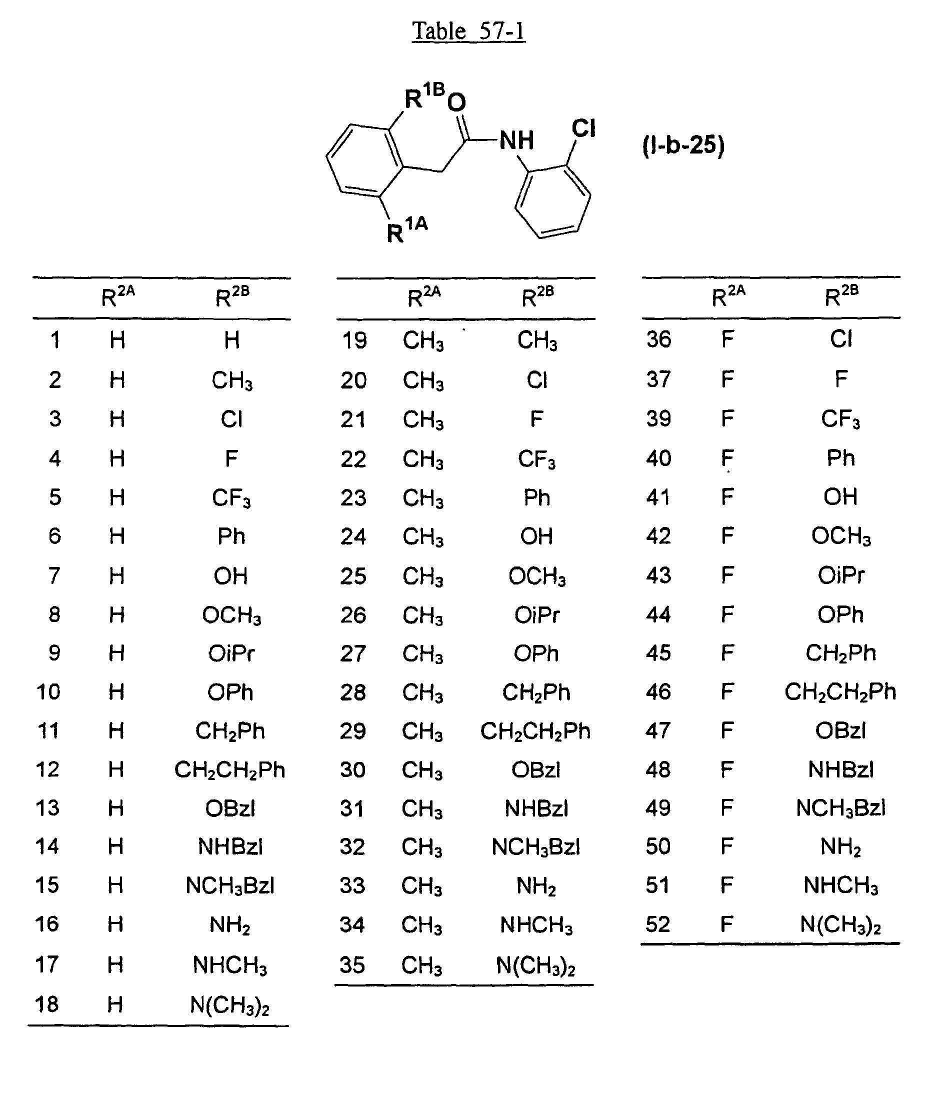

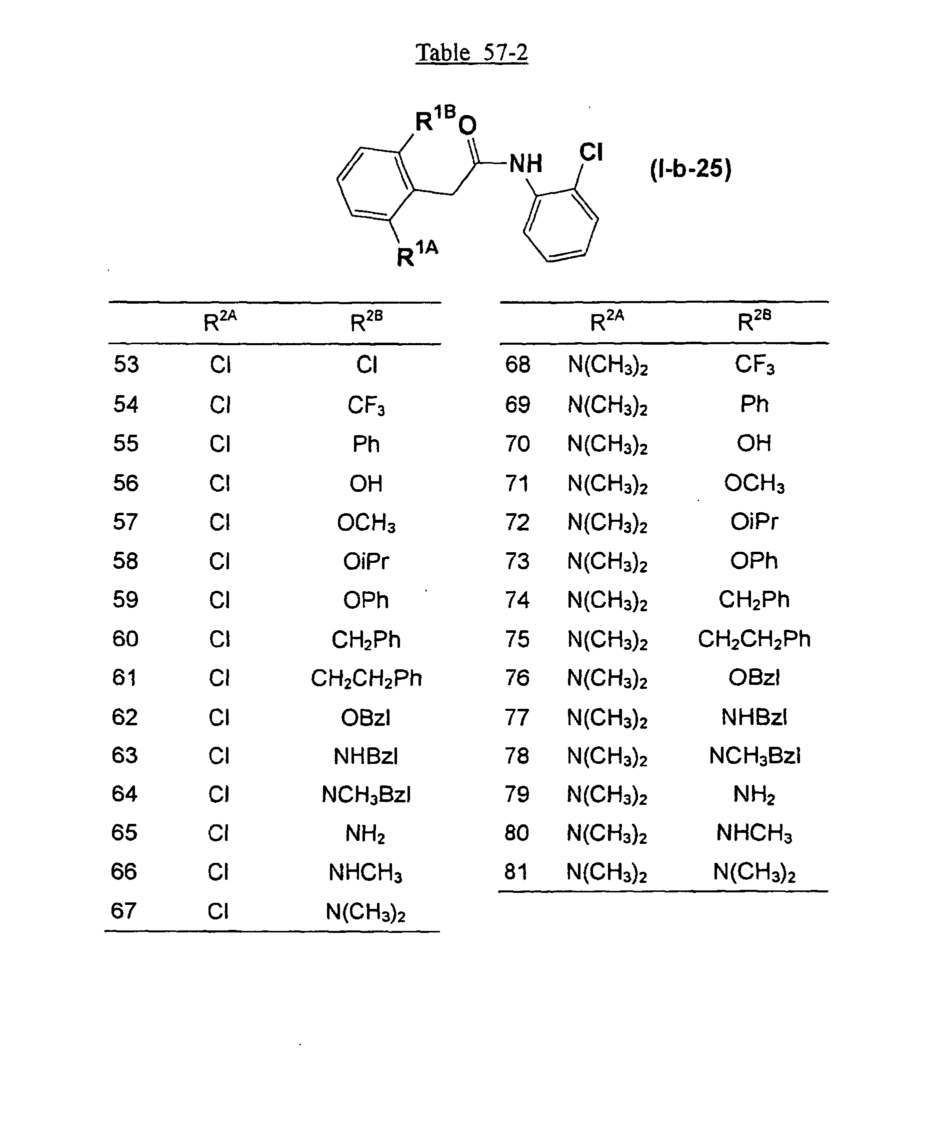

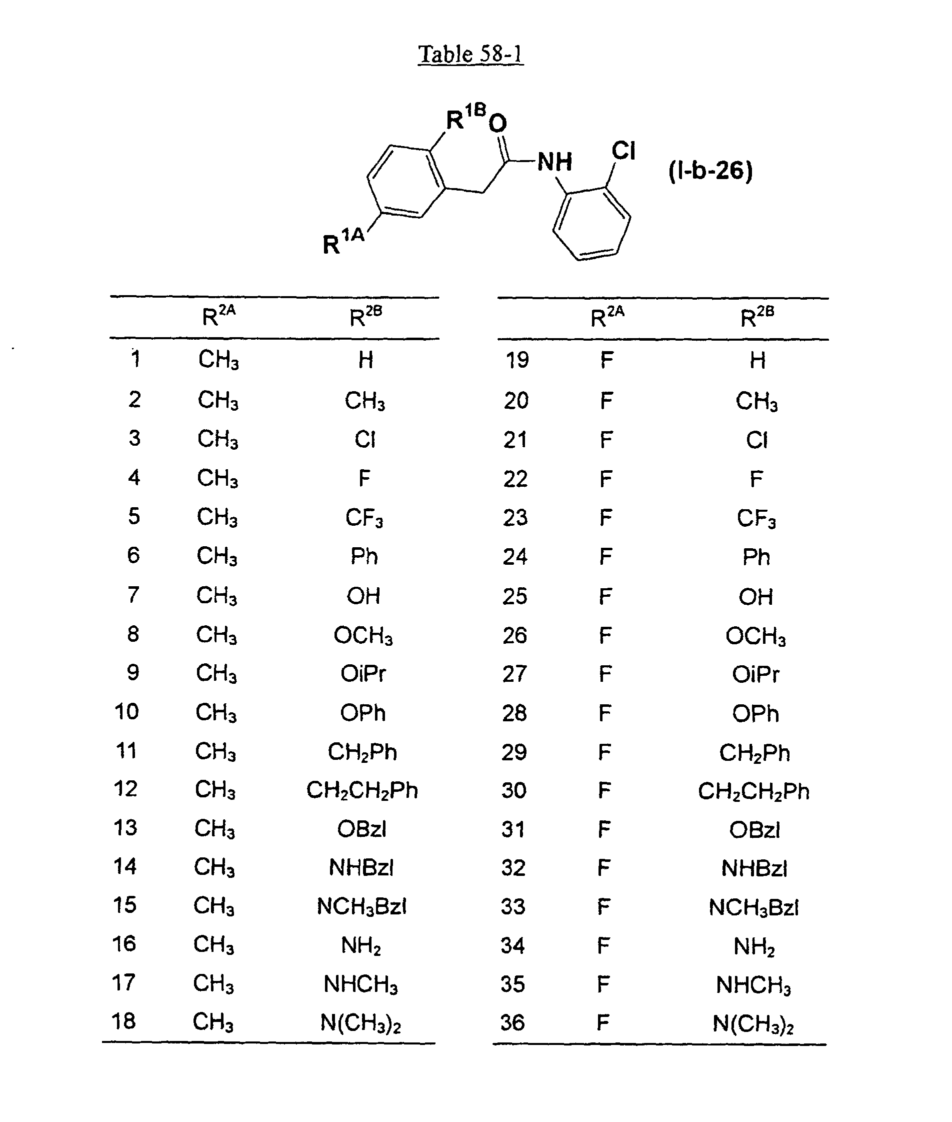

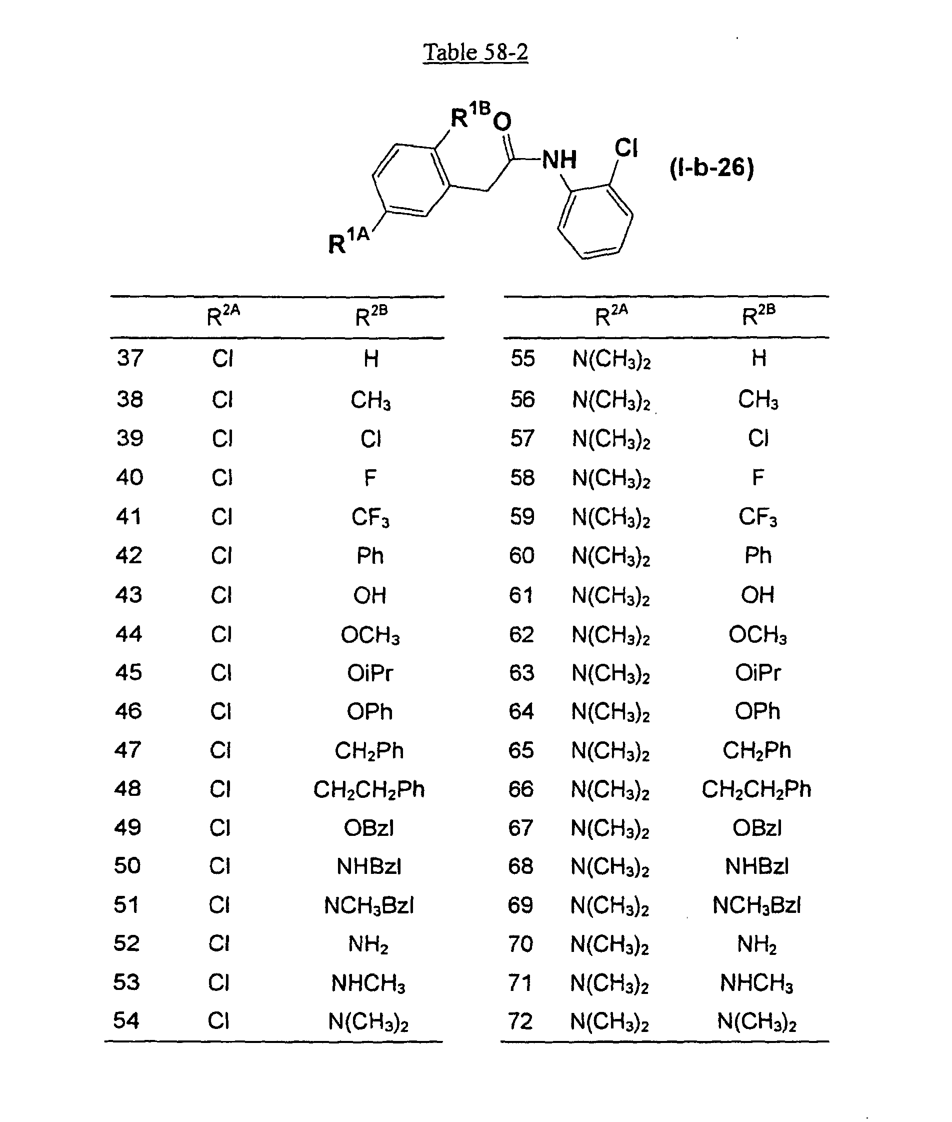

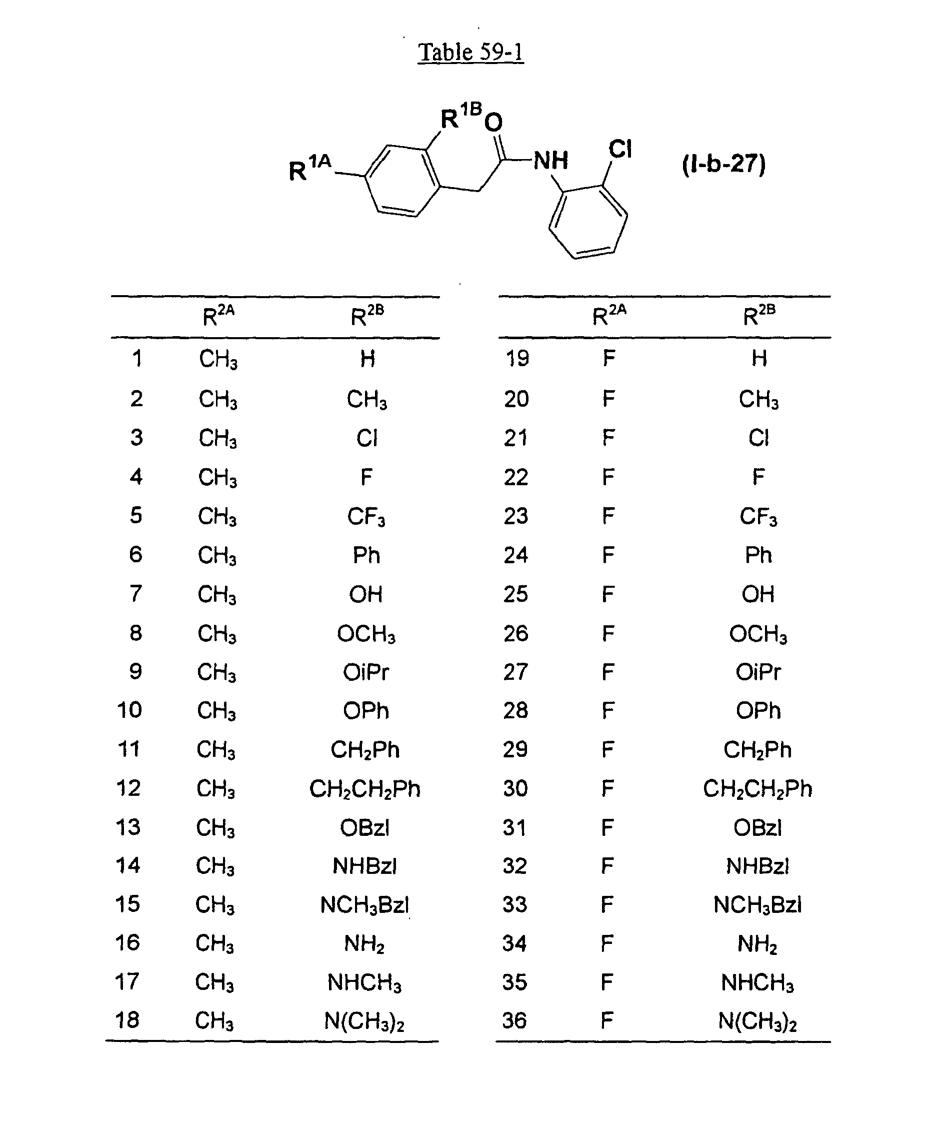

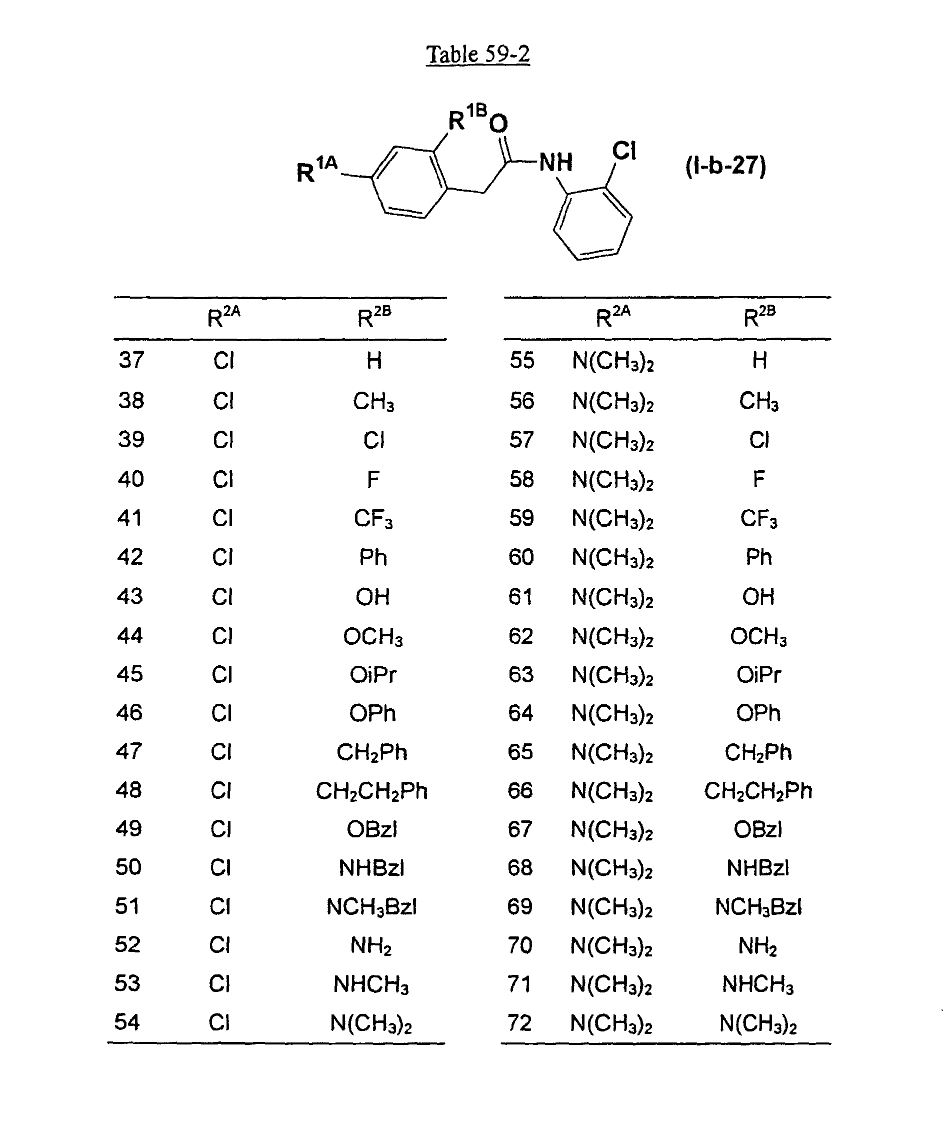

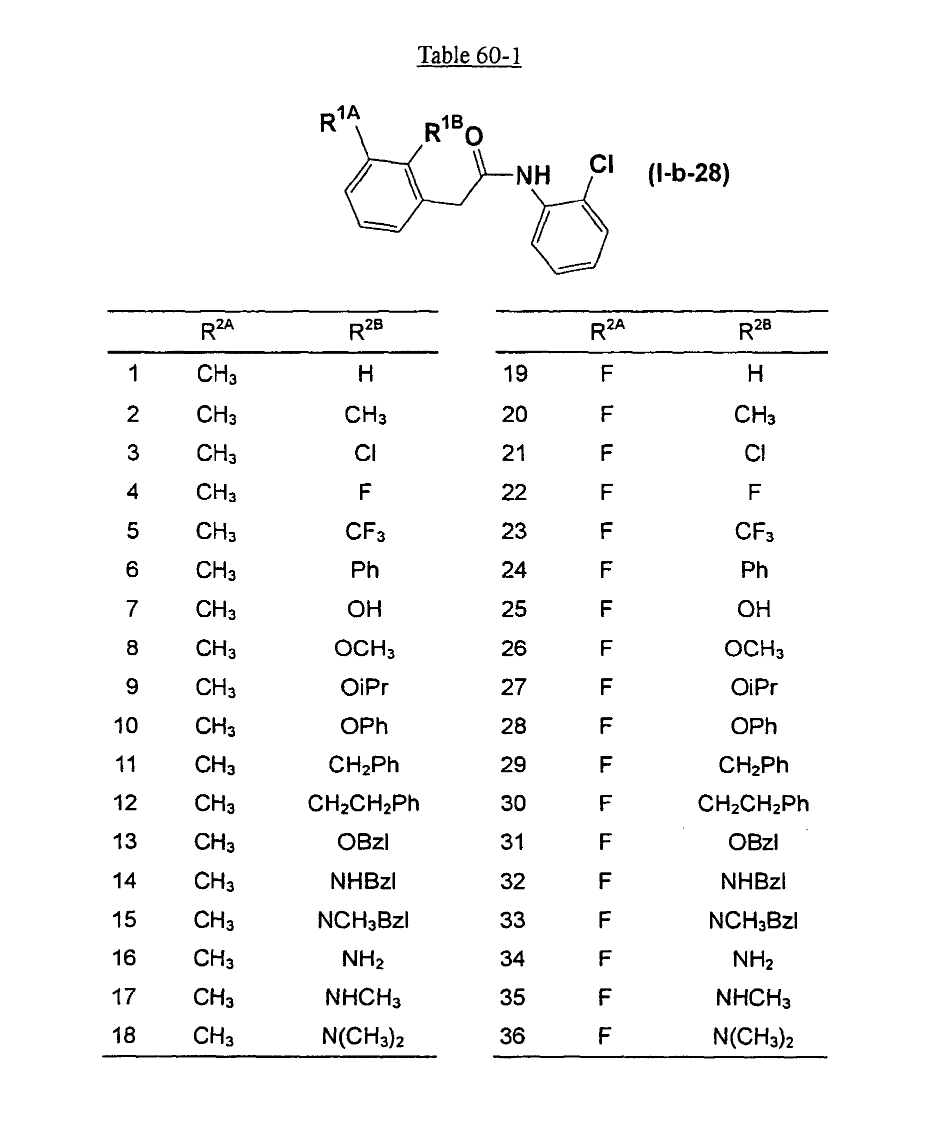

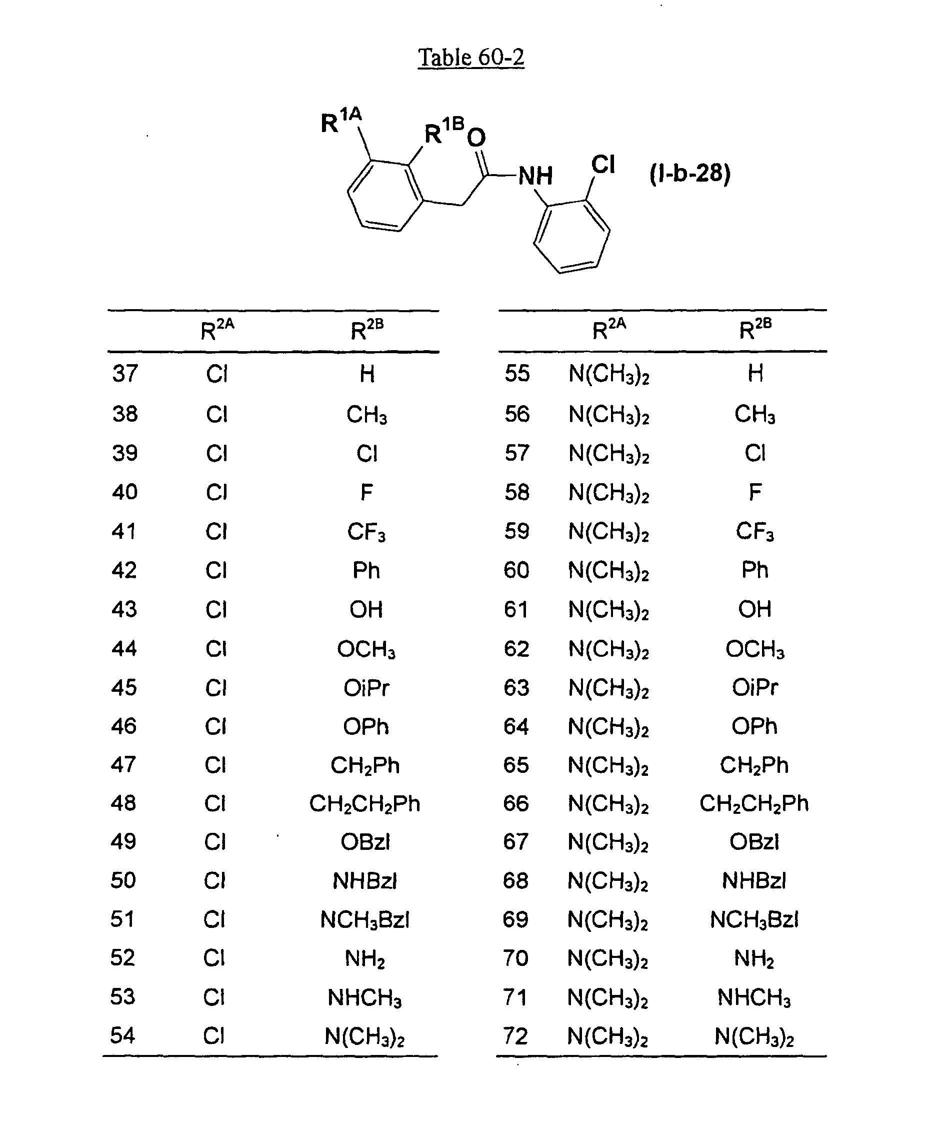

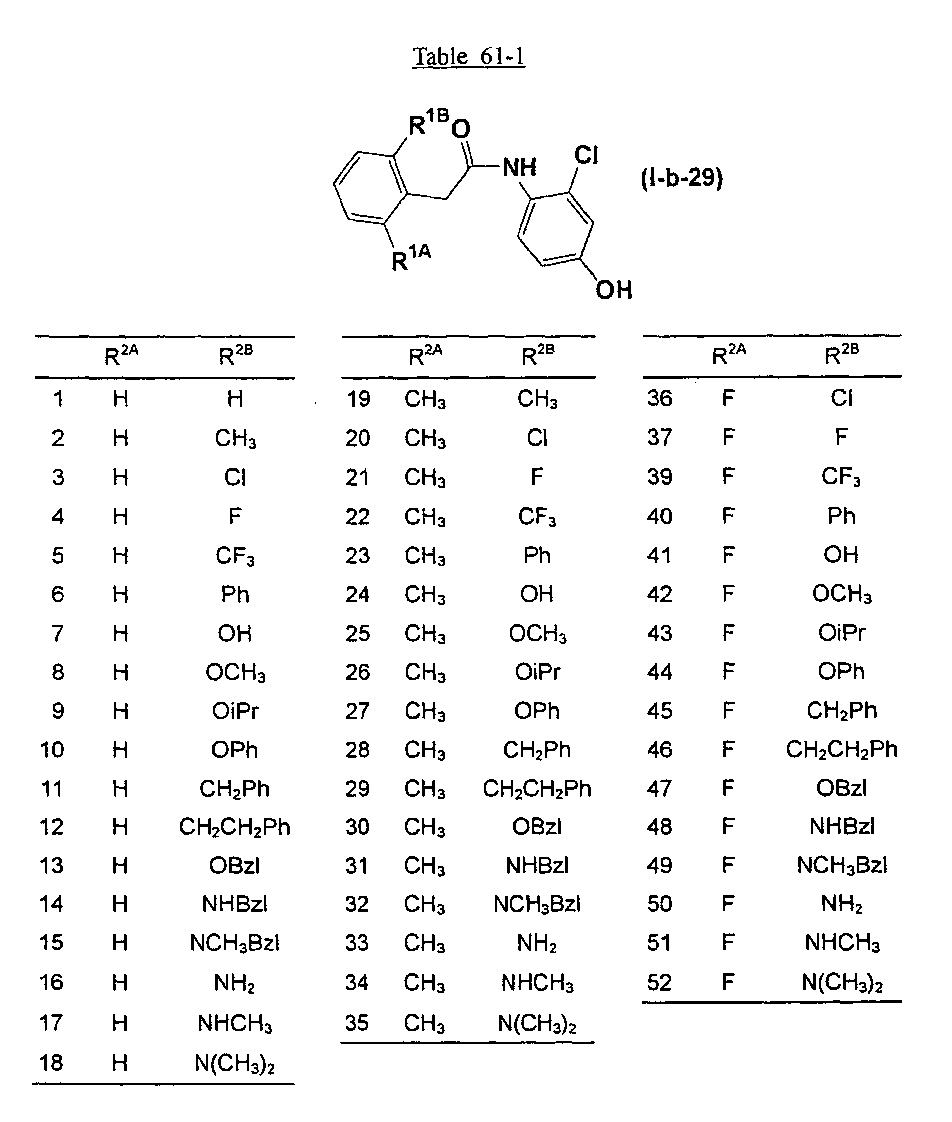

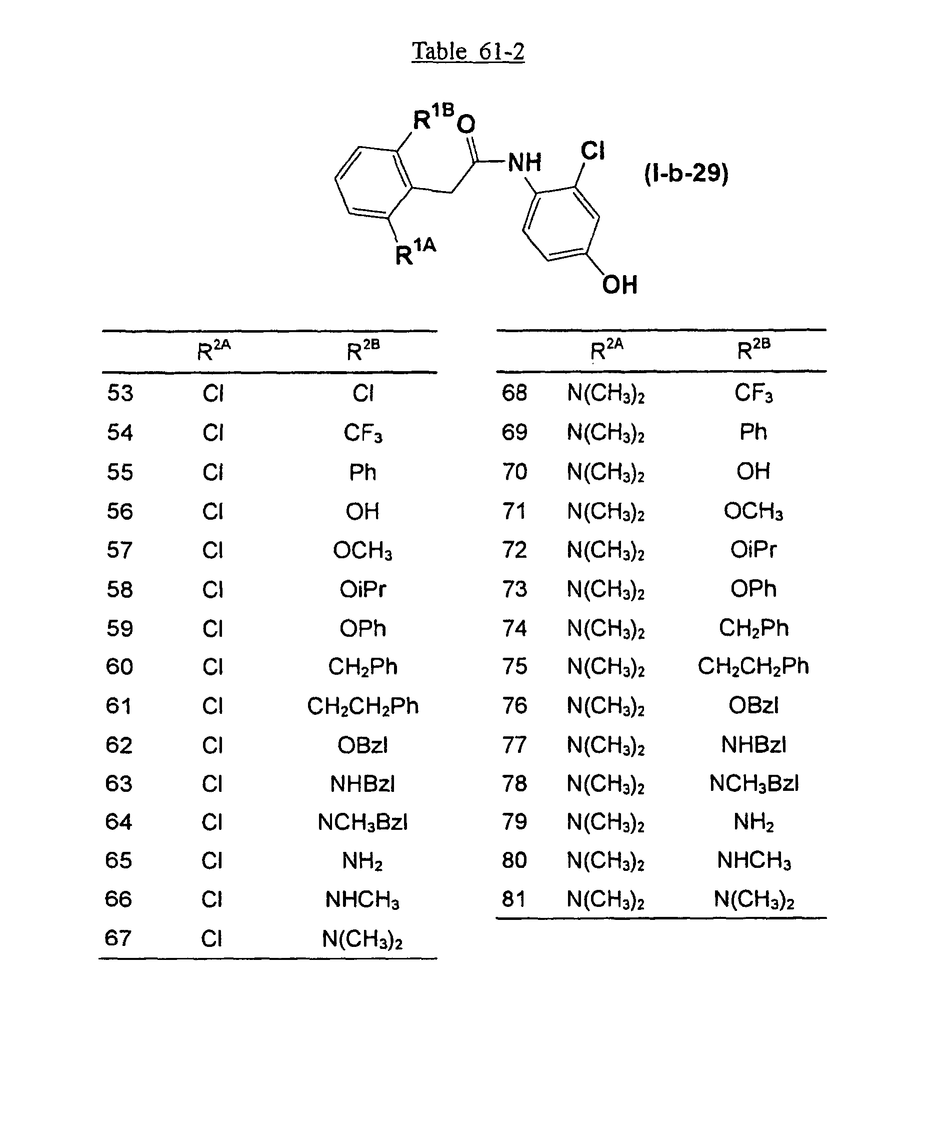

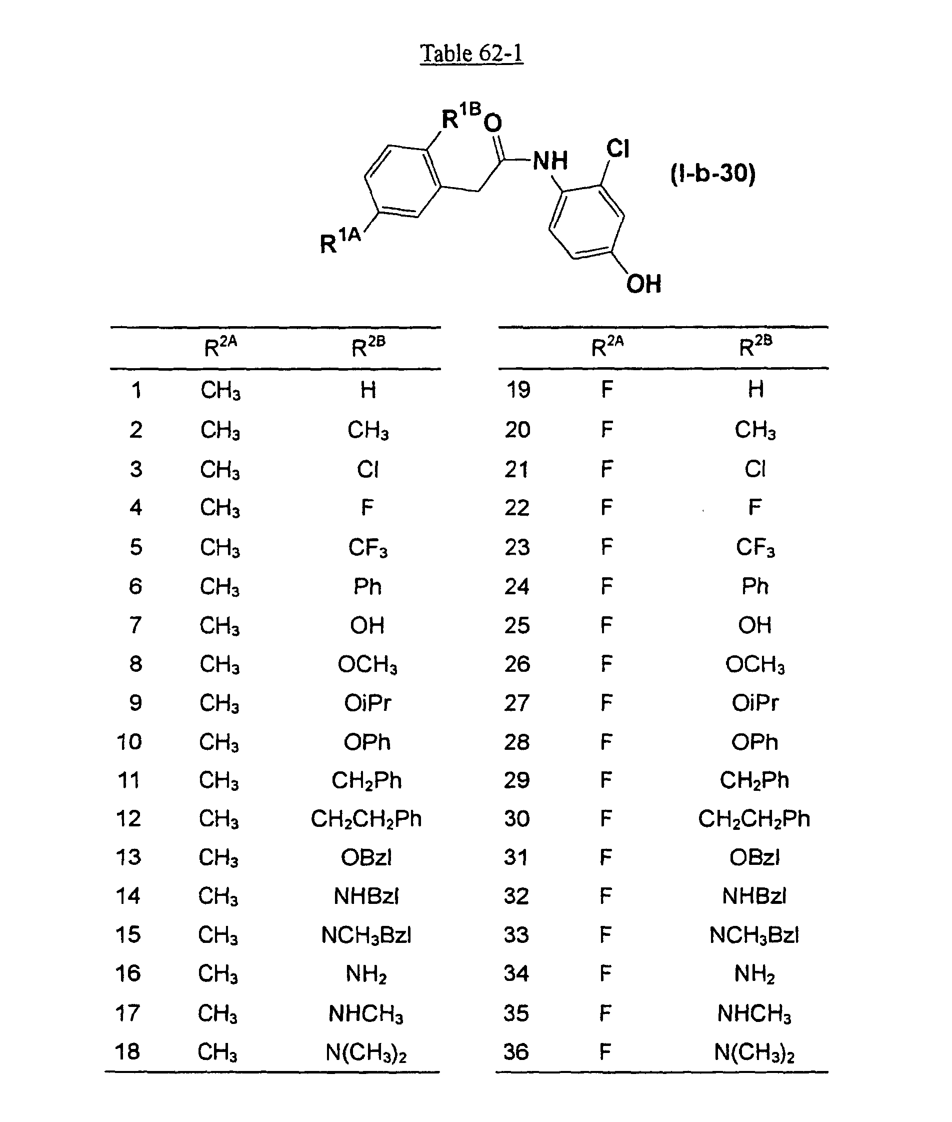

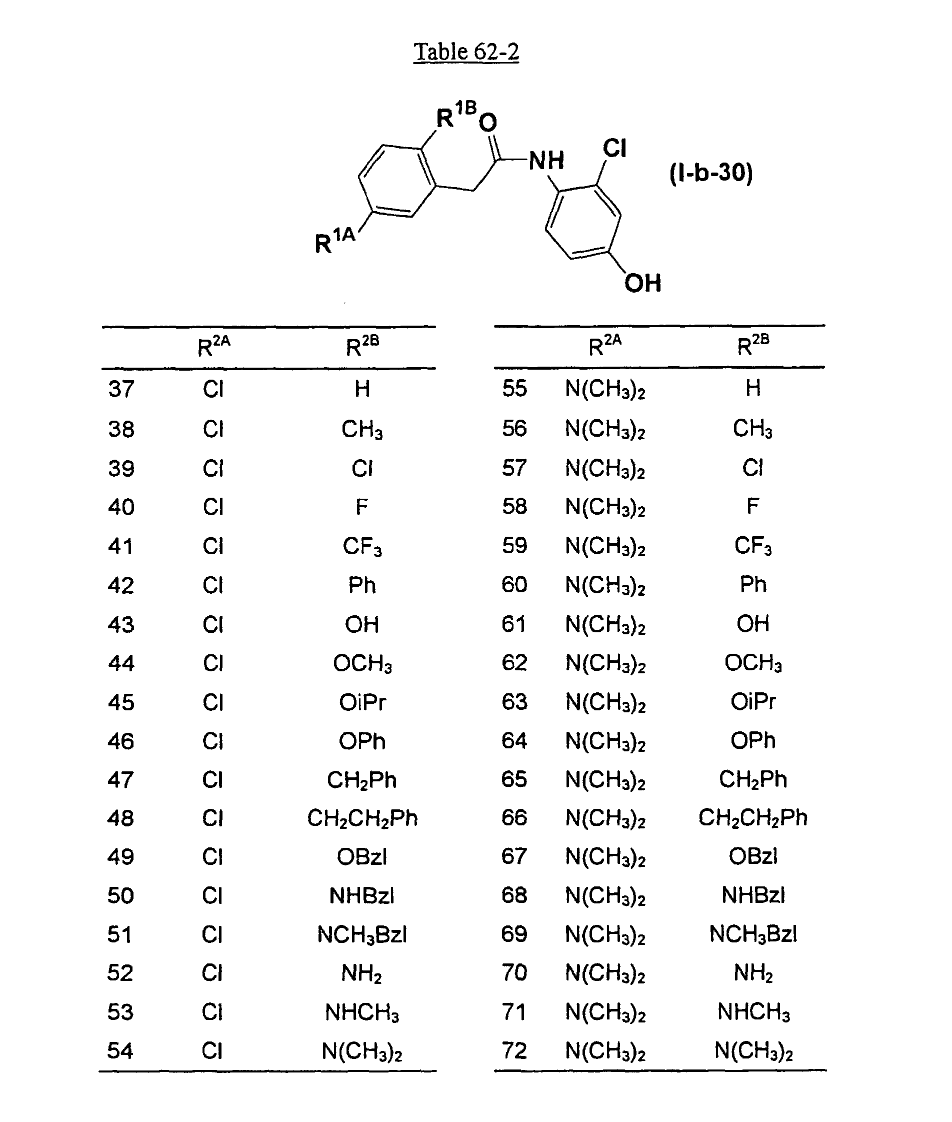

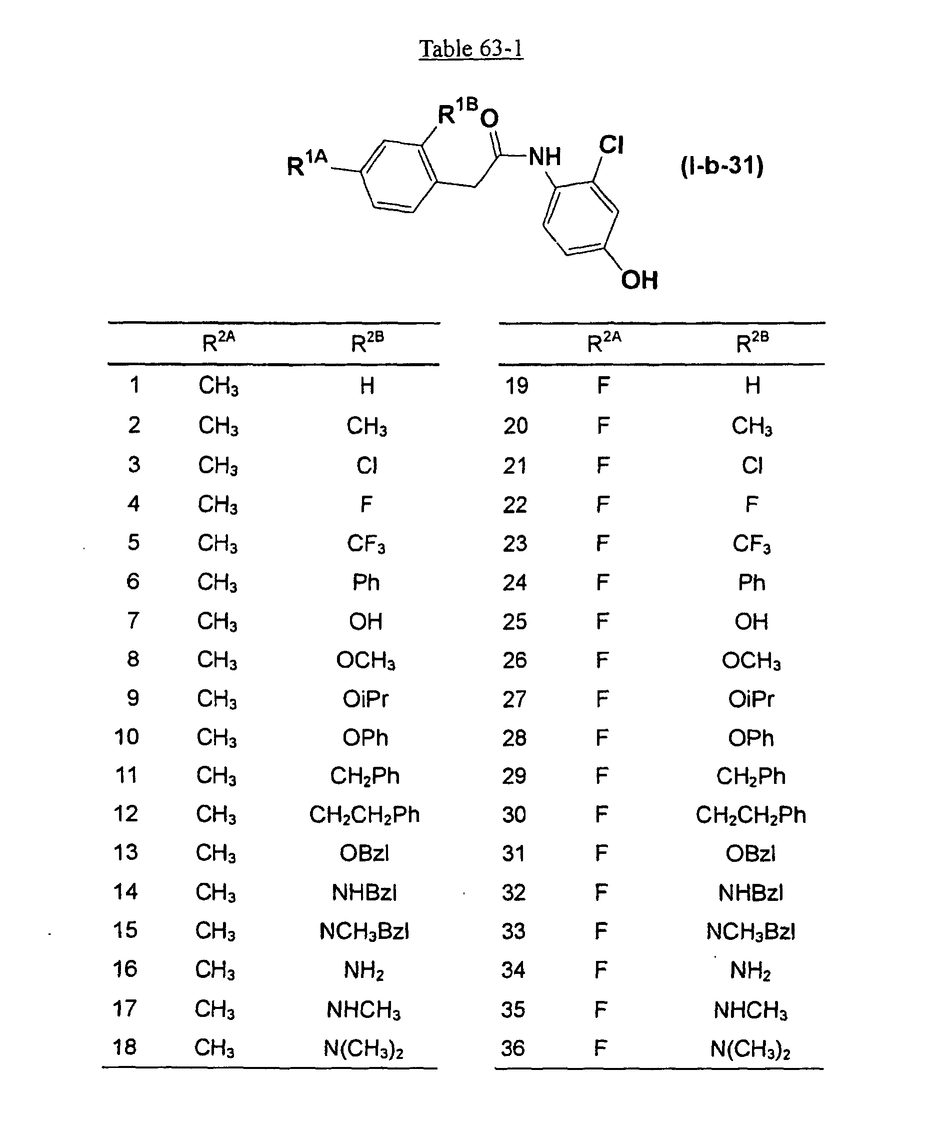

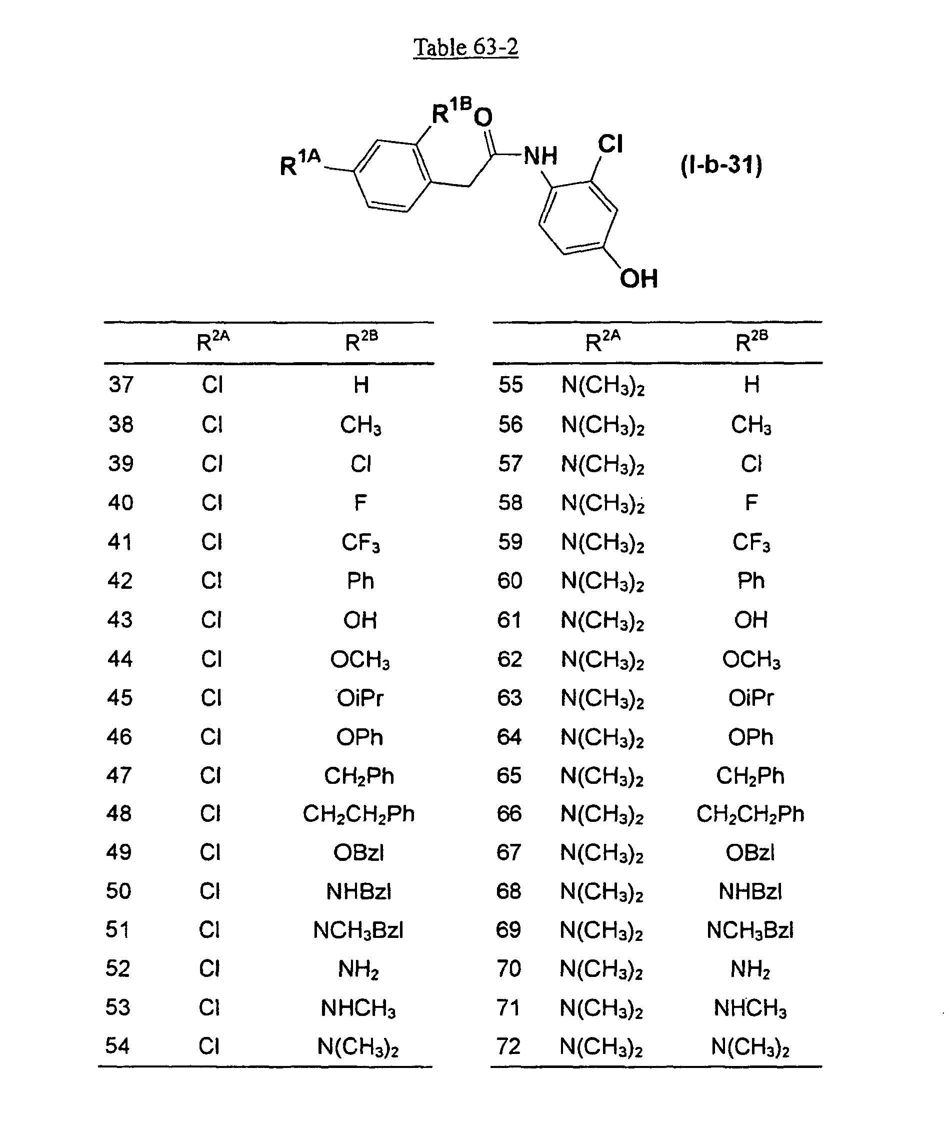

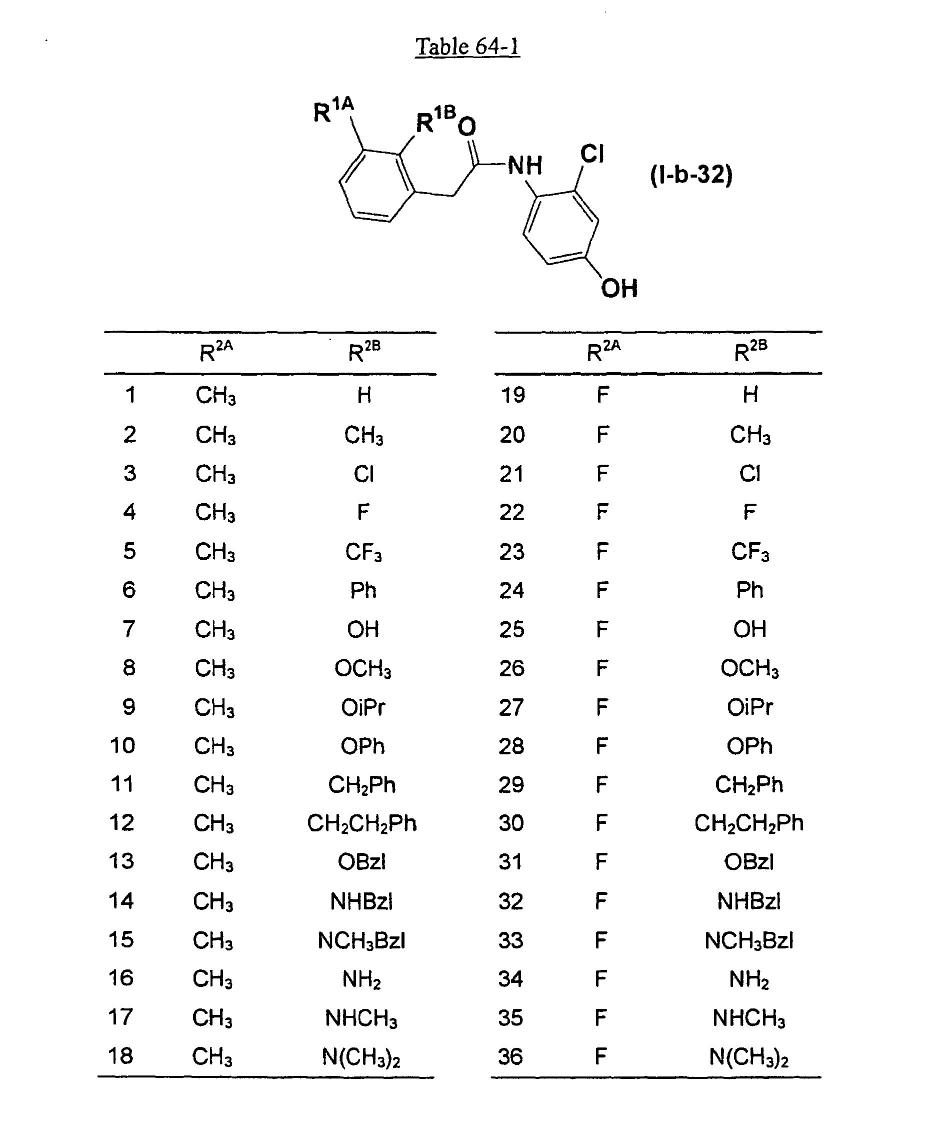

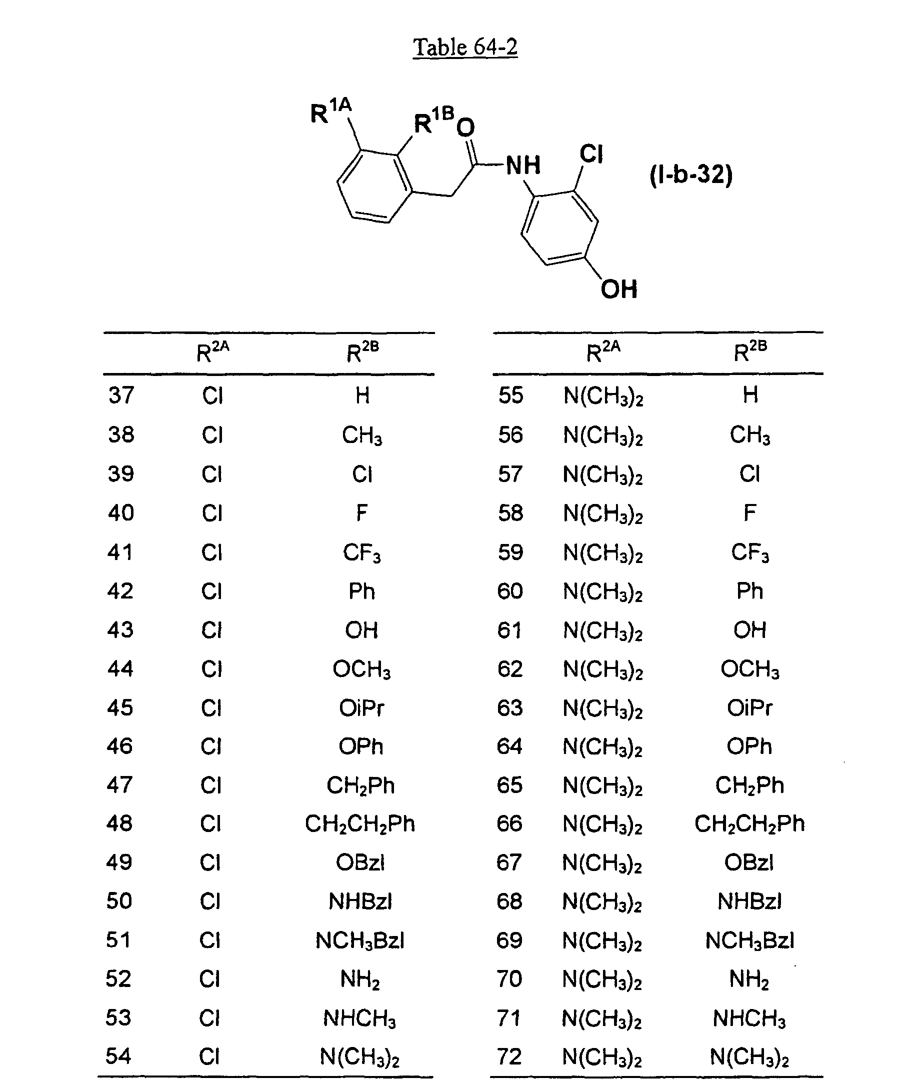

The present invention provides a substance possessing an effect of antagonizing diazepam binding inhibitor (DBI), which is increased by stressor and its metabolite on mitochondrial benzodiazepine receptor (MBR) and inhibits excessive neurosteroid production which mediates MBR and recover the excitory and inhibitory transmission to normal level, that is, MBR antagonist effect. The present invention further provides a pharmaceutical composition for the prophylaxis and/or treatment of diseases induced, exacerbated or reignited by stressor comprising a substance possessing MBR antagonistic effect.

The present invention provides a pharmaceutical composition for the prophylaxis and/or treatment of diseases induced, exacerbated or reignited by stressor comprising a substance acting on MBR possessing MBR antagonistic effect.

The present invention provides a pharmaceutical composition for the prophylaxis and/or treatment of diseases induced, exacerbated or reignited by stressor comprising a substance which acts on MBR and suppresses the increase of abnormal neurosteroid production in a stress state.

The compound of the present invention acts on MBR and suppresses the increase of neurosteroid production by MBR agonist. As a result, the compound of the present invention suppresses increased neurosteroid production and recovers excitory and inhibitory signaling pathway and then recovers the function of nervous, immune internal secretion.

When stressor is added to a living body, the production of pregnenolone, which mediates MBR in the mitochondria of a neuroglia increases, and as a result kinds of neurosteroid also increases, which are metabolites. Since neurosteroid adjusts the function of various receptors and ion channels to positive or negative according to the kind, the balance of excitatory and inhibitory signaling pathway collapses, and thereby the activity of nervous system, immunity system, and internal secretion system also changes, accordingly various stress related diseases are caused. The medicament provided by the present invention, when especially MBR is activated and neurosteroid production increases extraordinarily, suppresses its production, and recovers the function of a nerve, immunity, and an internal secretion system.

The compound of the present invention binds to MBR and it acts as competitive antagonist against MBR agonist which binds to this receptor and increases neurosteroid production [for example, FGIN 2-[2-(4-fluorophenyl)-1H-indole- 3-yl]-N,N-dihexylacetamide: *J*. *Pharmacol*. *Exp*. *Ther.,* 262, 971-978 (1992)]. It is considered that the compound of the present invention demonstrates anti-stress effect by acting as an antagonist to MBR and antagonizing the increasing effect of steroid production induced by endogenous MBR ligand.

Therefore, the compound of the present invention is characterized by (a) inhibiting steroid production induced by MBR agonist and by the steroid production inhibitory effect, it is characterized by (b) expressing anti-stress effect by decreasing neurosteroid and recovers the balance of excitory and inhibitory signaling pathway to the normal level. Therefore, the compound of the present invention is not limited to a particular chemical structure, but all compounds which have the above features should be included in the scope of the present invention. And the methods for confirming the above features are described in the examples of the present specification.

And the compound of the present invention includes MBR antagonist as an active ingredient and it accomplishes the prophylaxis and/or treatment of diseases induced, exacerbated or reignited by stressors effectively by inhibiting steroid production to recover the balance of excitory and inhibitory signaling pathway to the normal level and by recovering the function of a nerve, immunity, and an internal secretion system. The methods for the confirmation of the effect of MBR antagonist and anti-stress effect of MBR antagonist are described concretely in the experimental examples of the present specification.

Those diseases induced, exacerbated or reignited by stress include, digestive organ diseases (functional dyspepsia, gastric and duodenal ulcer, chronic ulcerative colitis, irritable bowel syndrome, biliary dyskinesia, esophagus spasm, gastric atony, aerophagia, chronic hepatitis, chronic pancreatitis, *etc.*), circulatory organ disease (primary hypertension, primaryhypotension, (nervous) angina pectoris, arrhythmia, orthostatic disturbance, myocardial infarction, arteriosclerosis, dizziness, *etc*.), internal secretion and a metabolism diseases (anorexia nervosa, bulimia, barter syndrome, cachexia exophthalmica, diabetes, psychogenic polydipsia, obesity, reflective hypoglycemia, *etc.*), respiratory disease (bronchial asthma, hyperventilation syndrome, larynx spasm, chronic obstructive pulmonary diseases, *etc.*), nervous and muscular diseases (migraine, tension-type headache, migrainous neuralgia, post-traumatic stress disorder, dissociative disorder, panic disorder, anxiety, depression, insomnia, nervous vomiting, nervous cough, neurosis, autonomic imbalance, reactive depression, psychogenic spasm, psychogenic faint spasm, maladjustment to job, burnout syndrome, chronic fatigue syndrome, writer's cramp, spasmodic torticollis, *etc.*), dermatological diseases (chronic urticaria, atopic dermatitis, hyperhidrosis, eczema, skin pruritus, alopecia areata, systemic lupus erythematosus, *etc.*), surgery diseases (postoperative abdominal neurosis, dumping syndrome, polysurgery, neurosis after formation, *etc.*), orthopedic diseases (rheumatoid arthritis, low back pain, cervico-omo-brachial syndrome, stiff neck, uniting callus, polyarthralgia, systemic muscle pain, gout, *etc*.), urologic and genital diseases (nervous pollakiuria (overactive bladder), bed-wetting, enuresis, psychogenic anuria, impotence, prostatism, urethra syndrome, *etc*.), gynecological diseases (menopausal disorders, menstrual pain, menstrual disorder, premenstrual syndrome, infertility, frigidity, serious vomiting of pregnancy, abortus pregnancy immature birth, *etc*.), ophthalmologic diseases (asthenopia, central retinitis, myodesopsia, palpebra spasm, primary glaucoma, *etc*.), otolaryngological diseases (tinnitus, dizziness, psychogenic deafness, chronic sinusitis, allergic rhinitis, anosmia, stammering, aphonia, *etc*.), oral surgery dentistry diseases (temporomandibular arthrosis, glossopharyngeal neuralgia, spasmodic glossalgia, stomatitis, toothache, ozostomia, abnormal salivation, teeth grinding, *etc.*), cancer, *etc*. are mentioned.

### The methods for the preparation of the compound of the present invention:

The compound of formula (I) used in the present invention is known or may be prepared by the following methods.

### (1) In the compound of formula (I-b), the compound wherein neither R¹ nor R² is amino, hydroxy, carboxy or mercapto, that is, the compound of formula (I-b-A)

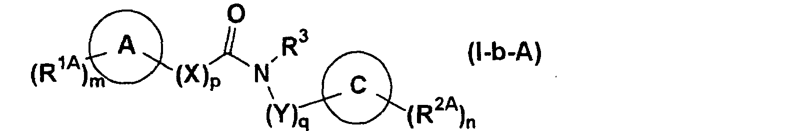

(wherein R^{1A} and R^{2A} are the same meanings as R¹ and R² respectively, but do not represent amino, hydroxy, carboxy or mercapto and the other symbols have the same meaning as above.) may be prepared by subjecting to amidation reaction the compound of formula (X^{A}) 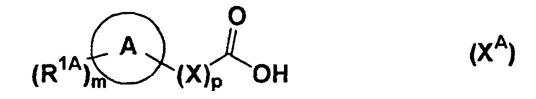 (wherein all symbols have the same meaning as hereinbefore.) and the compound of formula (X^{B}) 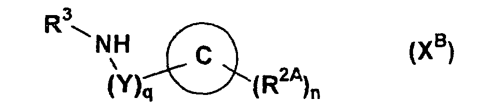 (wherein all symbols have the same meanings as hereinbefore.).

Methods for amidation reaction are known, for example,
(1) a method using acid halide,
(2) a method using mixed anhydride,
(3) a method using a condensing agent, *etc.*

To explain these methods concretely,
(1) The method using acid halide is, for example, carried out by subjecting to a reaction carboxylic acid in an organic solvent (chloroform, methylene chloride, diethyl ether, tetrahydrofuran, *etc.*) or without a solvent, and acid-halogenating agent (oxalyl chloride, thionyl chloride, *etc*.) at a temperature between -20 °C and refluxing temperature, and then subjecting to a reaction thus obtained acid halide in the presence of tertiary amine (pyridine, triethylamine, dimethylaniline, dimethylaminopyridine, *etc*.) at a temperature between 0 and 40 °C. And it is also carried out by subjecting to a reaction with an acid halide by using an alkaline aqueous solution (sodium bicarbonate or sodium hydroxide, *etc.*) in organic solvent (dioxane, tetrahydrofuran, *etc.*) at a temperature between 0 and 40 °C.
(2) The method using mixed anhydride is, for example, carried out by subjecting to a reaction in an organic solvent (chloroform, methylene chloride, diethyl ether, tetrahydrofuran, *etc.*) or without a solvent, in the presence of tertiary amine (pyridine, triethylamine, dimethylaniline, dimethyl aminopyridine, *etc*.) carboxylic acid with acid halide (pivaloyl chloride, tosyl chloride, mesylchloride, *etc*.) or acid derivative (chloroethyl formate, chloroisobutyl formate, *etc.)* at a temperature between -20 and 40 °C, and then subjecting to a reaction thus obtained mixed anhydride with amine at a temperature between 0 and 40 °C.
(3) The method using a condensing agent is carried out, for example, in an inert organic solvent (chloroform, methylene chloride, dimethylformamide, diethyl ether, tetrahydrofuran, *etc*.) or without a solvent, in the presence or absence of a tertiary amine (pyridine, triethylamine, dimethylaniline, dimethylaminopyridine, N-methylmorpholine, *etc.*), using a condensing reagent (1,3-dichlorohexyl carbodiimide (DCC), 1-ethyl-3-[3-(dimethylamino) propyl]carbodiimide (EDC), 1,1'-carbonyldi imizazole (CDI), 2-chloro-1-methylpyridinium iodide, *etc*.) in the presence or absence of 1-hydroxybenzotriazole, by subjecting to a reaction carboxylic acid and amine at a temperature between 0 and 40 °C.

These reactions (1), (2) and (3) are desirably carried out under atmosphere of an inert gas (argon, nitrogen, *etc*.) under anhydrous conditions.

### (2) In the compound of formula (I-b), the compound wherein at least one of R¹ or R² contains amino, hydroxy, carboxy or mercapto, that is, the compound of formula (I-b-B)

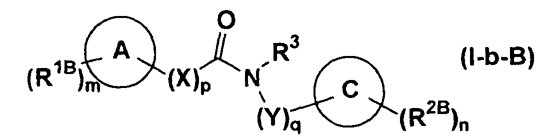

(wherein R^{1B} and R^{2B} are the same meanings as R¹ and R², respectively, but at least one of them contains amino, hydroxy or carboxy.) may be prepared by subjecting to a deprotection reaction the compound, which has a protected form of amino, hydroxy, carboxy or mercapto in the compound of formula (I-b-A), that is, the compound of formula (I-b-A-1) 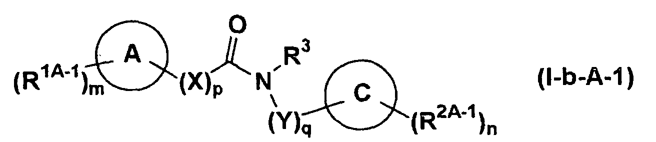 (wherein R^{1A-1} and R^{2A-1} are the same meaning as R^{1A} and R^{2A} respectively, and at least one of them include a protected form of amino, hydroxy, carboxy or mercapto.).

Protective groups for amino include, for example, benzyloxycarbonyl, t-butoxycarbonyl, trifluoroacetyl, 9-fluorenylmethoxycarbonyl, *etc.*

Protective groups for hydroxyl include, for example, methoxymethyl, 2-tetrahydropyranyl, t-butyldimethylsilyl, t-butyldiphenylsilyl, acetyl, benzyl.

Protective groups for carboxy include, for example, methyl, ethyl, t-butyl, benzyl.

Protective groups for mercapto include, for example, benzyl, methoxybenzyl, methoxymethyl, 2-tetrahydropyranyl, diphenylmethyl, acetyl.

Protective groups for carboxy, hydroxy or amino are not limited to above listed, but other groups may also be used instead, if easily and selectively eliminated. For example, the groups described in T. W. Greene, Protective Groups in Organic Synthesis 3rd edition, Wiley, New York, 1999 may be used.

Deprotection reactions of protective groups of carboxy, hydroxy or amino is known, for example,
(1) a deprotection reaction under alkaline conditions,
(2) a deprotection reaction under acidic conditions,
(3) a deprotection reaction by hydration,
(4) a deprotection reaction of silyl group, *etc* are known.

To describe these methods concretely,
(1) The deprotection reaction under alkaline conditions is, for example, carried out in an organic solvent (methanol, tetrahydrofuran, dioxane, *etc.*) using a hydroxide of alkali metals (sodium hydroxide, potassium hydroxide, lithium hydroxide, *etc.*), hydroxide of alkaline earth metals (barium hydroxide, calcium hydroxide, *etc*.), or carbonates (sodium carbonate, potassium carbonate, *etc.*) or a solution thereof or a mixture thereof at a temperature between 0 and 40 °C.
(2) The deprotection reaction under acidic conditions is, for example, carried out in or without an organic solvent (methylene chloride, chloroform, dioxane, ethyl acetate, anisole, *etc*.), using an organic acid (acetic acid, trifluoroacetic acid, methanesulfonic acid, *etc*.) or an inorganic acid (hydrochloric acid, sulfuric acid, *etc*.) or a mixture thereof (hydrobromic acid/acetic acid, *etc*.) at a temperature between 0 and 100 °C.
(3) The deprotection reaction by hydration is, for example, carried out in a solvent (ethers (tetrahydrofuran, dioxane, dimethoxyethane, diethyl ether, *etc*.), alcohols (methanol, ethanol, *etc*.), benzenes (benzene, toluene, *etc*.), ketones (acetone, methyl ethyl ketone, *etc*.), nitriles (acetonitrile *etc*.), amides (dimethylformamide *etc*.), water, ethyl acetate, acetic acid or a mixture of more than two from above *etc*.) in the presence of a catalyst (palladium-carbon, palladium black, palladium hydroxide, platinum oxide, Raney nickel, *etc*.) under atmosphere of hydrogen of normal or suppressed pressure, or in the presence of ammonium formate at a temperature between 0 and 200°C.
   As easily understood by those skilled in the art, the compounds of the present invention may be easily prepared by these reactions.
(4) The deprotection reaction of silyl group is, for example, carried out in an organic solbent which is miscible with water (tetrahydrofuran, acetonitrile, *etc*.) using tetrabutylammoniumfluoride at a temperature between 0 and 40°C.

As easily understood by those skilled in the art, the target compounds of the present invention may be easily prepared by these reactions.

### (3) In the compound of formula (I-a), the compound wherein neither R¹ nor R² includes amino, hydroxy, carboxy, or mercapto, that is, the compound of formula (I-a-A)

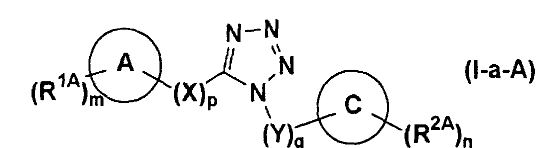

(wherein all symbols have the same meaning as hereinbefore.) may be prepared by subjecting to a reaction of converting amide to tetrazole, using the compound, wherein in the compound of formula (I-b-A) wherein R3 is hydrogen, that is, the compound of formula (I-b-A-2) 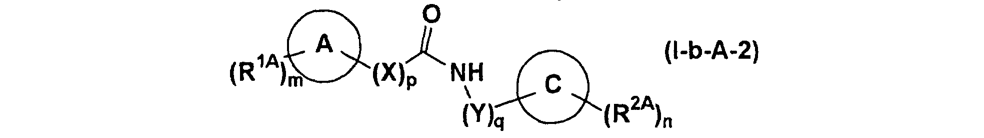 (wherein all symbols have the same meanings as hereinbefore.).

The reaction is known, for example
1) a reaction which go through iminochloride,
2) a reaction using an azo compound and a phosphine compound,
3) a reaction which goes through thioamide, *etc*. are included.

To explain these methods concretely,
1) The reaction which goes through iminochloride is, for example, carried out in an organic solvent or without a solvent, by subjecting to a reaction the compound of formula (I-b-A-2) with a halogenating agent (phosphine pentachloride, thionyl chloride, *etc*.) at a temperature between -20°C and refluxing temperature, and then subjecting to a reaction thus given iminochloride with
   (a) an azide compound (inorganic azide such as sodium azide, organic azide such as trimethylazide, *etc*.) in an organic solvent (N,N-dimethylformamide, dichloromethane, diethyl ether, tetrahydrofuran, acetonitrile, benzene, toluene, *etc*.) or without a solvent at a temperature between 0°C and refluxing temperature, or
   (b) hydrazine monohydrate in an organic solvent (N,N-dimethylformamide, dichloromethane, diethyl ether, tetrahydrofuran, acetonitrile, benzene, toluene, methanol, ethanol, *etc*.) or without a solvent, at a temperature between 0°C and refluxing temperature, and then subjecting to a reaction thus given hydrazoneamide in water or organic solvent (methanol, ethanol, acetic acid, *etc*.) or without a solvent, with nitrite salt(sodium nitrite, potassium nitrite, *etc*.) at a temperature between -20°C and refluxing temperature.
2) The reaction using an azo compound and a phosphine compound is, for example, carried out by subjecting to a reaction the compound of formula (I-b-A-2) with an azide compound (inorganic azide such as sodium azide, organic azide such as trimethylazide, *etc.*) in an organic solvent (dichloromethane, diethyl ether, tetrahydrofuran, acetonitrile, benzene, toluene, *etc.*) or without a solvent, in the presence of an azo compound (azo dicarboxylic acid diethyl, azo dicarboxylic acid diisopropyl, 1,1'-(azodicarbonyl)dipiperidine, 1,1'-azobis(N,N-dimethylformamide), *etc*.) and a phosphine compound (triphenyl phosphine, tributyl phosphine, trimethyl phosphine, *etc.*), at a temperature between -20°C and refluxing temperature.
3) The reaction which goes through thioamide is, for example, carried out by subjecting to a reaction the compound of formula (I-b-A-2) in an organic solvent (dichloromethane, diethyl ether, tetrahydrofuran, acetonitrile, benzene, toluene, *etc*.) or without a solvent, with a sulphidizing agent (phosphine pentasulfide, Lawesson reagent (2,4-bis(4-methoxyphenyl)-1,3-dithia-2,4-diphosphethan-2,4-disulfide), *etc*.) at a temperature between -20°C and refluxing temperature and then subjecting to a reaction thus given thioamide with
   (a) hydrazine monohydrate in an organic solvent (N,N-dimethylformamide, dichloromethane, diethyl ether, tetrahydrofuran, acetonitrile, benzene, toluene, *etc*.) or without a solvent, at a temperature between 0°C and refluxing temperature, and then subjecting to a reaction thus given hydrazone amide with nitrite salt (sodium nitrite, potassium nitrite, *etc.*), in an organic solvent (dichloromethane, diethyl ether, tetrahydrofuran, acetonitrile, benzene, toluene, methanol, ethanol, acetic acid, *etc*.) or without a solvent, at a temperature between -20°C and refluxing temperature, or
   (b) an azide compound (inorganic azide such as sodium azide, or organic azide such as trimethylazide, *etc*.) in an organic solvent (N,N-dimethylformamide, dichloromethane, diethyl ether, tetrahydrofuran, acetonitrile, benzene, toluene, methanol, ethanol, *etc*.) or without a solvent, in the presence or absence of Lewis acid (tin chloride *etc*.), at a temperature between 0°C and refluxing temperature.

   These reactions 1), 2) and 3) are desirably carried out under atmosphere of an inert gas (argon, nitrogen, *etc*.) in an anhydrous condition.
(4) In the compound of formula (I-a), the compound wherein at least one of R¹ or R² contains amino, hydroxy, carboxv or mercapto, that is, the compound of formula (I-a-B) 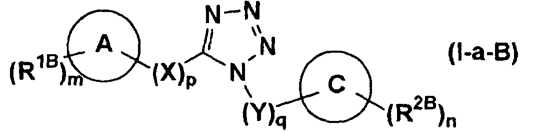 (wherein all symbols are the same meanings as hereinbefore)
   may be prepared by subjecting to a deprotection reaction the compound of formula (I-a-A-1) 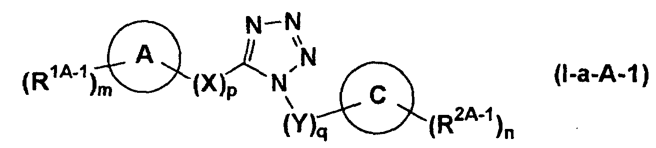 (wherein all symbols are the same meanings as hereinbefore).

The carboxylic acid compounds and amine compounds, which are used as starting materials in the above methods are known or may be prepared according to known methods.

The compound of formula (II) may be prepared according to the method described in the specification of WO98/14213.

The compound of formula (III) may be prepared according to the method described in the specification of JP4-217682(A).

The compound of formula (IV) may be prepared according to the method described in JP3-24467(B).

The compound of formula (V) may be prepared according to the method described in the specification of EP210084.

In each reaction of the present specification, obtained products may be purified by conventional techniques. For example, purification may be carried out by distillation at atmospheric or reduced pressure, by high performance liquid chromatography, by thin layer chromatography or by column chromatography using silica gel or magnesium silicate, by washing or by recrystallization. Purification may be carried out after each reaction, or after a series of reactions.

### Salt:

The compounds of formula (I), (II), (III), (IV) and (V) may be converted into non-toxic salts thereof by conventional techniques.

In the present specification, non-toxic salts include, alkali metal salt, alkaline earth metal salt, ammonium salt, amine salt, acid-addition salt, *etc*.

Non-toxic and water-soluble salts are preferable. Appropriate salts include salts of alkali metals (potassium, sodium, *etc.*), salts of alkaline-earth metals (calcium, magnesium, *etc*.), ammonium salts, pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, *etc*.).

Non-toxic and water-soluble acid-addition salts are preferable. Appropriate acid-addition salts include, salts of inorganic acid, e.g. hydrochloride, hydrobromide, hydroiodide, sulfate, phosphate, nitrate, or salts of organic acid, e.g. acatate, lactate, tartarate, benzoate, citrate, methanesulfonate, ethanesulfonate, benzenesulfonate, toluenesulfonate, isethionate, glucronate, gluconate, *etc*.

The compound of formula (I) and its salt may be converted to a solvate thereof.

### Non-toxic and water-soluble solvates are preferable. Appropriate ones are solvates of water and alcohol solvent (e.g. ethanol etc.)

Acid-addition salt is preferably non-toxic and water-soluble. Appropriate acid-addition salt include, for example, inorganic salt such as hydrochloride, hydrobromide, hydroiodide, sulfate, phosphate, or organic salt such as acetate, lactate, tartrate, benzoate, citrate, metanesulfonate, ethanesulfonate, benzenesulfonate, toluenesulfonate, isethionate, glucuronate, gluconate, *etc*.

The compound of the present invention of formula (I) and a salt thereof may be converted into a solvate thereof by a conventional technique.

Solvates are preferably non-toxic and water-soluble. Appropriate solvates include, for example, ones of water and alcohol solvents such as ethanol.

### Pharmacological activity:

It was proved by the following experiments that the compound of the present invention of formula (I) has an MBR antagonistic activity and that it is effective for the diseases induced, exacerbated or reignited by a stressor, and it was also proved by the following experiments that MBR antagonist is effective for stress-induced diseases.

In the following experimental examples, 'PK11195' is MBR ligand [1-(2-chlorophenyl)-N-methyl-N-(1-methylpropyl)-3-isoquinolinecarboxamide: *Eur. J*. *Pharmacol*., 119, 153-167 (1985)] (one among the compound of formula (III)), 'FGIN1-27' is MBR agonist [2-[2-(4-fluorophenyl)-1H-indole-3-yl]-N,N-dihexylacetamide:*J*. *Pharmacol. Exp. Ther*., 262, 971-978 (1992)], 'AC5216' is MBR agonist [N-benzyl-N-ethyl-2-(7-methyl-8-oxo-2-phenyl-7,8-dihydro-9H-purin-9-yl)acetamide; WO99/28320]. CB34 is MBR selective agonist [5,7-dichloro-2-(4-chlorophenyl)-3-dipropylaminocarbonylimidazo[1,2-a]pyridine; *British Journal of Pharmacology* (1999), 127, 177-187], Ro5-4864 is MBR agonist [7-chloro-5-(4-chlorophenyl)-1-methyl-2-oxo-2,3-dihydro-1H-benzodiazepine;*Peripheral Benzodiazepine Receptors,* E. Giesen-Crouse, p.62].

### Experiment 1: Receptor binding

The affinity of the compound to MBR was determined using rat brain membrane preparation. Male Wistar rats were decapitated. After extirpation of the brain, cerebellum was removed. The brains without cerebellum were homogenized in ice-cold 50 mmol/L Tris-HCl buffer (pH7.4). The homogenate was centrifuged at 12,000g for 20 min (4°C), then supernatant was discarded. The residual pellet was washed in the same buffer. The pellet resuspended in 50 mmol/L Tris-HCl buffer (pH7.4) and adjusted to about 1 mg/mL was used as the rat brain membrane preparation for binding assay. [³H]PK11195, a selective MBR ligand, was used for binding assay.

To determine the amount of total binding in saturation binding study, membrane preparation, various concentrations of [³H]PK11195, dimethylsulfoxide (DMSO, final concentration: 0.5 vol%) and 50 mmol/L Tris-HCl buffer (pH7.4) in a total volume of 200 µL were incubated for 1 h at room temperature. To determine the amount of non-specific binding, 20 µmol/L of PK11195 was added in place of DMSO and incubate for 1 h. The reaction was terminated by rapid filtration on GF/B filter soaked with 0.3% polyethyleneimine using cell harvester, then the filter was washed twice with 50 mmol/L Tris-HCl buffer (pH7.4). After dried in the oven, the radioactivity on the filer was measured by liquid scintillation counter. The data were analyzed using analysis soft KELL (Ver.6, BIOSOFT). The Scatchard analyses revealed that dissociation constant (K_{D} value) was 0.75 ± 0.054 nmol/L.

To determine the amount of total binding in competition binding study, membrane preparation, [³H]PK11195 (final concentration: 1 nmol/L), DMSO (final concentration. 0.5 vol%) and 50 mmol/L Tris-HCl buffer (pH7.4) in a total volume of 200 µL were incubated for 1 h at room temperature. To determine the amount of non-specific binding, 20 µmol/L of PK11195 was added in place of DMSO. And to determine the affinity of the invention, 10 pmol/L to 1 µmol/L of the compound dissolved in DMSO was added in place of DMSO. The reaction was terminated as above described, then radioactivity on the filer was measured by liquid scintillation counter. The concentration of the compound required to reduce [³H]PK11195 binding to 50% of the amount of specific binding of [³H]PK11195 (IC₅₀ value) were derived from the data obtained. The inhibitory constant (Kᵢ value) was derived according to the equation of Cheng & Prusoff (*Biochem*. *Pharmacol*., 22, 3099-3108, 1973). The Kᵢ values of the compounds in this invention are shown in Table 65. These results clearly indicate that these compounds have high affinity to MBR.

**Table 65**

| Example | Ki (nM) |
|---|---|
| 1(16) | 0. 19 |
| 1(110) | 0. 22 |
| 2(14) | 3. 30 |
| 2(71) | 0. 08 |
| 2(74) | 0. 16 |
| 2(105) | 0. 73 |

### Experiment 2: Determination of pregnenolone in rat adrenocortical mitochondria

The ability of steroid production of the compound was evaluated using mitochondrial fraction from rat adrenal cortex. Five min after intraperitoneal administration of 20 mg/mL (1 mL) of cycloheximide, 10 IU/mL (0.3 mL) of adrenocorticotropic hormone (ACTH) was intraperitoneally administered to Male SD rat. Twenty min after ACTH administration, rat was sacrificed by cervical dislocation and bilateral adrenal glands were extirpated. Adrenal cortexes were obtained and homogenized in buffer A (50 mmol/L Tris-HCl, 250 mmol/L Sucrose), then centrifuged at 2,000g for 3 min (4°C). The supernatant was centrifuged at 12,500g for 10 min (4°C). The residual pellet was resuspended in buffer A, then further centrifuged at 12,500g for 10 min (4°C). The pellet was washed again and finally suspended in buffer B (250 mmol/L Sucrose, 10 mmol/L potassium phosphate buffer, 15 mmol/L triethanolamine, 20 mmol/L potassium chloride, 5 mmol/L magnesium chloride, 10 µmol/L trilostane, 10 µmol/L SU10603) for experiments. Malate (150 mmol/L), β-NADP⁺ (5 mmol/L) and the compound in a total volume of 25 µL were incubated for 5 min at 37°C. Following the incubation, 225 µL of mitochondrial membrane fraction from rat adrenal cortexes in buffer B preincubated for 5 min at 37°C was added, then further incubated for 10 min at 37°C to produce pregnenolone (final concentration of the compound: 1 µmol/L). The reaction was terminated by addition of ether (1 mL) and pregnenolone was extracted with n-hexane (1.25 mL). The organic phase was collected and evaporated. The residual was dissolved in buffer C (0.1% gelatin in phosphate buffered saline). After centrifuged at 12,000g for 5 min, the supernatant was collected as a sample for determination of pregnenolone. [³H]Pregnenolone (10,000 cpm, 100 µL), pregnenolone antibody (ICN Biomedicals Inc, 100 µL) and sample (100 µL) were mixed and incubated overnight at 4°C. The reaction was terminated by adding dextran/charcoal (200 µL) and mixed well. After kept on ice for 10 min, the mixture was centrifuged at 1,000g for 10 min (4°C). The radioactivity of the supernatant was measured by liquid scintillation counter. The pregnenolone content in the sample was calculated from standard curve. The pregnenolone content in FFIN1-27 treated group was 1.37 times as high as that in DMSO treated group. But, the pregnenolone content in the invention (Compound (14) in Experiment 2) treated group was as 0.98 times as high as that in DMSO treated group. The Table 66 summarizes effects of some other compounds on pregnenolone production (n=5).

**Table 66**

| Compound (10 µ mol/L) | Amount of pregnenolone production(%) |
|---|---|
| DMSO | 100 ± 0.00 |
| CB34 | 131.36 ± 5.38 |
| Ro5-4864 | 123.59 ± 5.41 |
| AC-5216 | 122.65 ± 7.56 |
| PK11195 | 101.82 ± 1.64 |

The amount of pregnenolone production when treated with DMSO was assumed 100, and the amount of pregnenolone production when treated with the compound of the present invention was displayed as %.

Accordingly, these results indicate that FGIN1-27, CB34, Ro5-4864 and AC-5216 are MBR agonists which increase pregnenolone production, on the other hand, the inventions and PK11195 are MBR antagonists which hardly effect on it compared with DMSO treated group.

### Experiment 3: Measurement of MBR antagonist activity in rat adrenocortical mitochondria

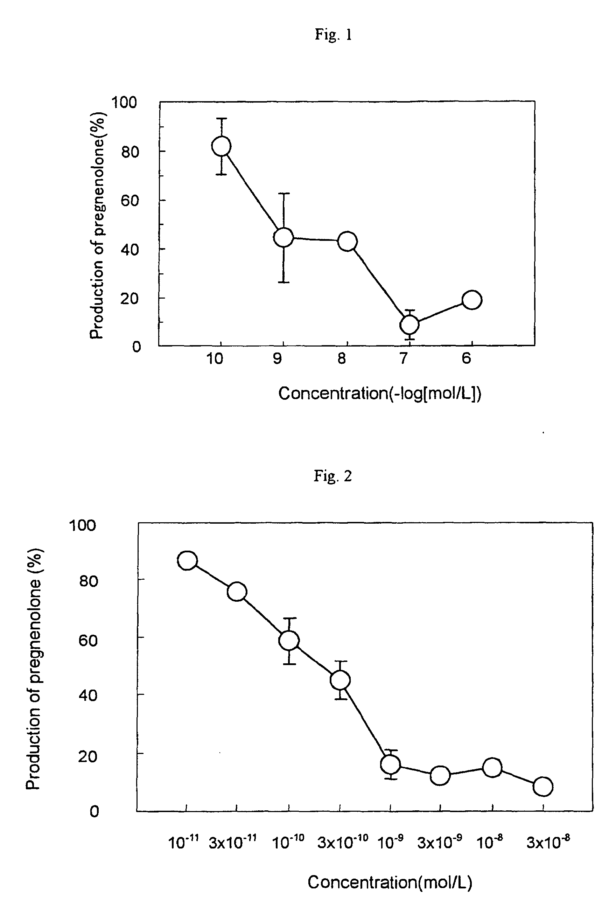

The results of the experiments evaluated the antagonistic effects of MBR antagonists on FGIN1-27 (1 nmol/L)-stimulated pregnenolone production according to the method described in Experiment 2 are shown in Figure 1 and 2.

Vertical axis represents% of pregnenolone production in the group treated with FGIN1-27 (1 nmol/L) and MBR antagonist (0.1 pmol/L to 1 µmol/L) simultaneously which is expressed taking that in DMSO treated group as 0% and that in FGIN1-27 (1 nmol/L) treated group as 100% (mean ± standard errors in 3 experiments). Horizontal axis represents the concentrations of the compound. These results indicate that the compounds antagonize the steroid production stimulated by MBR agonist. The MBR antagonists used in the experiments were the compound (Figure 1) in experiment 2 (14) and PK11195 (Figure 2).

### Experiment 4: Effect of the invention on stress-stimulated increase in pregnenolone production in the brain.

Psychological stress was loaded to male Wistar rats (Brain Res., 641, 21-28, 1994). Platform was located in the center of the container and water was collected to 10 cm in height. The rats in non-treated group were treated with microwave (output: about 6.5 kW, exposure time: 0.96 s, Muromachi Kikai Co., Ltd.) without administration and stress loading. On the other hand, the rats in stressed groups were rendered to stress loading by putted them on the platform for 1 h which started at 30 min after oral administration with vehicle or compound, then treated with microwave. Bilateral hippocampi were extirpated and weighted. Internal standard (D₄-pregnenolone, 20 ng), water (1 mL) and diethyl ether / n-hexane (9 : 1) (3 mL) were added to the crushed hippocampi. Following sonication and stir, the homogenate was centrifuged at 3,000 rpm for 5 min. The organic phase was collected to new tube with pipette. Water phase was extracted with diethyl ether / n-hexane (9 : 1) again, then the organic phase was added to the preserved one. The organic phase was evaporated. The residual was dissolved in 150 µL of H₂O/acetonitrile (1 : 9), then the pregnenolone content was measured by LC-MS. The measurement conditions are as follows.
LC: Hewlett Packard series 1100; Column: Inertsil ODS-3, 3µm, 2.1^{ϕ} x 100 mm; Temperature: room temp.; Mobile phase: 5 mmol/L CH₃CO₂NH₄/MeCN (10:90); Flow rate: 0.2 mL/min; Injection vol.: 40 µL
MS: Quattoro II (Micromass); Ionization mode: Atmosphere Pressure Chemical Ionization (APcI), positive; Corona: 3.4 kV; Sheath gas: N₂ (50 L/hr); Source temperature: 180°C; Probe temperature: 550°C; Detection: Pregnenolone: m/z 317.2 (cone: 10 V); D₄-pregnenolone: m/z 321.2 (cone: 10 V)

Pregnenolone content in hippocampus in vehicle-treated and stress-loaded group significantly increased compared with that in non-stressed group. On the other hand, the stress-stimulated increase in the pregnenolone content was inhibited in the compound (oral administration at doses from 3 to 30 mg/kg)-treated group. Significant effect was observed at 30 mg/kg (p<0.01, t test). These results indicate that the inventions inhibit stress-stimulated increase in pregnenolone content in hippocampus.

### Experiment 5: Evaluation of anti-stress effects of MBR antagonists.

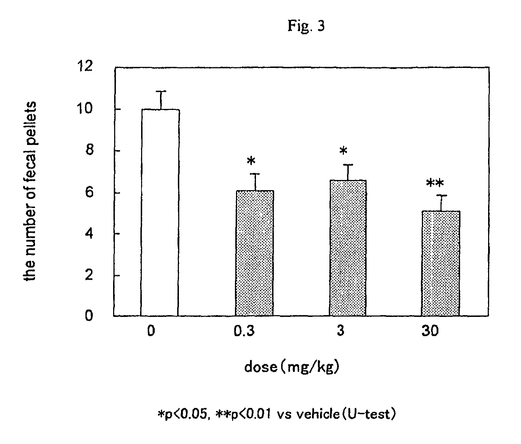

Psychological stress was loaded to male Wistar rats (*Brain Res.,* 641, 21-28 (1994)). Platform was located in the center of the container and water was collected to 10 cm in height. Thirty min after oral administration of vehicle or the compound, stress loading was started. One hour after, number of fecal pellets were counted (n=10/group). The rats without administration and stress-loading rarely defecated in 1 h. On the other hand, vehicle-treated and stress-loaded rats remarkably defecated as shown in Table 67 and Figure 3. But, the inventions significantly inhibited stress-induced increase in the number of fecal pellets compared with vehicle-treated group These results clearly indicate that MBR antagonists have anti-stress effects.

As described in Experiment 1 to 5, MBR antagonists whose structures are shown as (I) and (III) have anti-stress effects. So it is easily deduced that the compounds with the structures shown as (II), (IV) and (V) also have anti-stress effects.

Although MBR agonists, like AC-5261, Ro5-4864 and CB-34, did not produce significant effects, PK11195 and the compound with structure shown as (I) produced significant effects in water aversive stress model.

**Table 67**

| | | | | |
|---|---|---|---|---|
| AC-5216 | Vehicle | 0.3mg/kg, p.o. | 3mg/kg, p.o. | 30mg/kg, p.o. |
| | 8.6±0.99 | 9.6±0.74 | 7.5±0.63 | 9.3±0.94 |
| | n=10 | n=10 | n=10 | n=10 |
| | No significant effect in any dose. | | | |
| Ro5-4864 | Vehicle | 3mg/kg, p.o. | 10mg/kg, p.o. | 30mg/kg, p.o. |
| | 8.6±0.82 | 8.9±1.16 | 9.2±0.98 | 8.6±0.91 |
| | No significant effect in any dose. | | | |
| CB34 | Vehicle | 3mg/kg, p.o. | 10mg/kg, p.o. | 30mg/kg, p.o. |
| | 9.7±0.70 | 10.2±0.96 | 7.6±0.93 | 8.6±1.00 |
| | No significant effect in any dose. | | | |
| PK11195 | Vehicle | 0.3mg/kg, p.o. | 3mg/kg, p.o. | 30mg/kg, p.o. |
| | 8.9±0.82 | 6.4±0.76 | 6.6±0.97 | 5.5±0.81* |
| | *p<0.05 vs vehicle (U-test) | | | |
| The compound of example 2(14) | Vehicle | 0.3mg/kg, p.o. | 3mg/kg, p.o. | 30mg/kg, p.o. |
| | 10.0±0.88 | 6.1±0.82* | 6.6±0.76* | 5.1±0.78** |
| | **p<0.01, *p<0.05 vs vehicle (U-test) | | | |

### Toxicity:

The toxicity of the compound of the present invention is very low and it is thought to be safe enough for pharmaceutical use.

### Industrial Applicability

### Application to Pharmaceuticals:

The compound of the present invention of formula (I) antagonize MBR, and it is thought that it is useful for the prophylaxis and/or treatment of those diseases induced, exacerbated or reignited by stress.

Those diseases induced, exacerbated or reignited by stress include, digestive organ diseases (functional dyspepsia, gastric and duodenal ulcer, chronic ulcerative colitis, irritable bowel syndrome, biliary dyskinesia, esophagus spasm, gastric atony, aerophagia, chronic hepatitis, chronic pancreatitis, *etc.*), circulatory organ disease (primary hypertension, primaryhypotension, (nervous) angina pectoris, arrhythmia, orthostatic disturbance, myocardial infarction, arteriosclerosis, dizziness, *etc*.), internal secretion and a metabolism diseases (anorexia nervosa, bulimia, barter syndrome, cachexia exophthalmica, diabetes, psychogenic polydipsia, obesity, reflective hypoglycemia, *etc*.), respiratory disease (bronchial asthma, hyperventilation syndrome, larynx spasm, chronic obstructive pulmonary diseases, *etc*.), nervous and muscular diseases (migraine, tension-type headache, migrainous neuralgia, post-traumatic stress disorder, dissociative disorder, panic disorder, anxiety, depression, insomnia, nervous vomiting, nervous cough, neurosis, autonomic imbalance, reactive depression, psychogenic spasm, psychogenic faint spasm, maladjustment to job, burnout syndrome, chronic fatigue syndrome, writer's cramp, spasmodic torticollis, *etc*.), dermatological diseases (chronic urticaria, atopic dermatitis, hyperhidrosis, eczema, skin pruritus, alopecia areata, systemic lupus erythematosus, *etc*.), surgery diseases (postoperative abdominal neurosis, dumping syndrome, polysurgery, neurosis after formation, *etc*.), orthopedic diseases (rheumatoid arthritis, low back pain, cervico-omo-brachial syndrome, stiff neck, uniting callus, polyarthralgia, systemic muscle pain, gout, *etc*.), urologic and genital diseases (nervous pollakiuria (overactive bladder), bed-wetting, enuresis, psychogenic anuria, impotence, prostatism, urethra syndrome, *etc*.), gynecological diseases (menopausal disorders, menstrual pain, menstrual disorder, premenstrual syndrome, infertility, frigidity, serious vomiting of pregnancy, abortus pregnancy immature birth, *etc*.), ophthalmologic diseases (asthenopia, central retinitis, myodesopsia, palpebra spasm, primary glaucoma, *etc*.), otolaryngological diseases (tinnitus, dizziness, psychogenic deafness, chronic sinusitis, allergic rhinitis, anosmia, stammering, aphonia, *etc*.), oral surgery dentistry diseases (temporomandibular arthrosis, glossopharyngeal neuralgia, spasmodic glossalgia, stomatitis, toothache, ozostomia, abnormal salivation, teeth grinding, *etc*.), cancer, *etc.* are mentioned.

In the diseases described hereinbefore, preferable is irritable bowel syndrome.

The compound of formula (I) or non-toxic salt thereof may be administered in combination with other pharmaceutical preparations to accomplish the following purposes:
1) To compensate for and/or enhance the preventing and/or treatment effect of the compound to be combined;
2) To improve the kinetics/absorption of the compound to be combined and to reduce the dose of the compound; and/or
3) To eliminate the side effect of the compound to be combined

The compound of formula (I) and other pharmaceutical preparations may be administered in the form of formulation having these components incorporated in one preparation or may be administered in separate preparations. In the case where these pharmaceutical preparations are administered in separate preparations, they may be administered simultaneously or at different times. In the latter case, the compound represented of formula (I) may be administered before the other pharmaceutical preparations. Alternatively, the other pharmaceutical preparations may be administered before the compound represented of formula (I). The method for the administration of these pharmaceutical preparations may be the same or different.

The diseases on which the preventive and/or treatment effect of the aforementioned combined preparations works are not specifically limited but may be those for which the preventive and/or treatment effect of the compound represented by formula (I-1), (I-2) or (I-3) is compensated for and/or enhanced.

Examples of the other pharmaceutical preparations for compensating for and/or enhancing the preventive and/or treatment effect of the compound of formula (I) on irritable bowel syndrome include antidepressant (benzodiazepine drugs, thienodiazepine drugs, non-benzodiazepinedrugs, *etc*.), antidepressant (monoamine liberating agent, monoamine oxidase inhibitor, monoamine reuptake inhibitor (SNRI, SSRI), dopamine (D2) antagonist, CRF antagonist, β3 agonist, neurotensin antagonist, NK1 antagonist, tricyclic antidepressant, tetracyclic antidepressant, *etc*.), anticholinergic drugs, affinity polyacrylic resin, antidiarrheal drug, mucosal paralytic agent, bulk cathartic, saline purgative, fiber formulation, drug for controlling intestinal function, autonomic nervous system modulator, calcium blocker, phosphodiesterase inhibitor, serotonin antagonist (5-HT3 antagonist, 5-HT4 antagonist), serotonin agonist (5-HT4 agonist, 5-HT1A agonist), modulator of gastrointestinal tract function (CCK-A antagonist, β3 agonist, neurotensin, antagonist, opioid agonist, NK1 antagonist, NK2 antagonist, 5-HT1A agonist, muscarine agonist, 5-lipoxygenase inhibitor, CRF antagonist), *etc*.

Examples of the other pharmaceutical preparations for compensating for and/or enhancing the preventive and/or treatment effect of the compound of formula (I) on gastric and duodenal ulcer include, acid reducer, histamine (H2) receptor antagonist, proton-pump inhibitor, muscarine receptor antagonist, antiulcer drugs (defensive factor enhancer, anti-pepsin drugs, prostaglandin derivatives, mesalazine, salazosulfapyrizine, *etc.*), anticholinergic drugs, gastric mucosa anesthetics, antidepressant, dopamine antagonist, *etc*.

Examples of the other pharmaceutical preparations for compensating for and/or enhancing the preventive and/or treatment effect of the compound of formula (I) on ulcerative colitis include, mesalazine, salazosulfapyrizine, antiulcer drug, anticholinergic agent, steroid agent, 5-lipoxygenase inhibitor, antioxidant, LTB4 antagonist, local anesthetics, immunosuppressant, defensive factor enhancer, metalloprotease inhibitor, *etc*.

Examples of the other pharmaceutical preparations for compensating for and/or enhancing the preventive and/or treatment effect of the compound of formula (I) on bilary dyskinesia include, cerulein, spasmolytic agents, COMT (catechol-O-methyl transferase) inhibitor, cholinergic agent, anticholinergic agent, anxiolytic drug, cholagogue, antidepressant, CCK-A antagonist, *etc*.

Examples of the other pharmaceutical preparations for compensating for and/or enhancing the preventive and/or treatment effect of the compound of formula (I) on aerophagia include, drug for controlling intestinal function, anxiolytic drug, autonomic nerve modulator, fiber formulations, digestive enzyme formulation, gas absorption agent, intestinal tract motion promoter.

Examples of the other pharmaceutical preparations for compensating for and/or enhancing the preventive and/or treatment effect of the compound of formula (I) on chronic hepatitis include, hepatic hydrolysate drugs, polyenephosphatidylcholine, glycyrrhizinate formulations, protoporphyrin disodium, ursodeoxycholic acid, steroid drugs, anticholinergic agents, antacidum, propagermanium, lipid peroxisome inhibitor, *etc*.

Examples of the other pharmaceutical preparations for compensating for and/or enhancing the preventive and/or treatment effect of the compound of formula (I) on chronic pancreatitis include, protease inhibitor, gastric acid secretion suppressant,antispasmodicantispasmodic agents (COMT inhibitor, antiserotonin drug, *etc*.), non-steroidal anti-inflammatory drug, central analgesics, sedatives, digestive enzyme formulation, acid reducer, H2 receptor antagonist, antidepressant, gastric mucosa local anesthetic, digestive function modulator (CCK-A antagonist), *etc*.

Examples of the other pharmaceutical preparations for compensating for and/or enhancing the preventive and/or treatment effect of the compound of formula (I) on throat spasms include, throat mobile function modulator, anxiolytic drug, autonomic nerve modulator, *etc.*

Examples of the other pharmaceutical preparations for compensating for and/or enhancing the preventive and/or treatment effect of the compound of formula (I) on gastric atony include, digestive function promotor, digestive enzyme formulation, tranquilizers, *etc*.

Examples of the other pharmaceutical preparations for compensating for and/or enhancing the preventive and/or treatment effect of the compound of formula (I) on functional dyspepsia include, acid reducer, H2 receptor antagonist, digestive function modulator, digestive function promoters, anxiolytic drug, tranquilizers, digestive enzyme formulation, proton pump inhibitor, muscarine receptor antagonist, anticholinergic agent, defensive factor enhancer, dopamine antagonist, *etc*.

Anxiolytic drugs include, for example, diazepam, oxazolam, flutazolam, alprazolam, ethyl loflazepate, tofisopam, *etc*.

Tricyclic antidepressants include, for example, amitriptyline, imipramine, clomipramine, nortriptyline, amoxapine, *etc*.

Tetracyclic antidepressants include, for example, maprotiline, mianserin, *etc*.

Acid reducers include, for example, sodium bicarbonate, magnesium oxide, dry aluminum hydroxide, aluminum silicate, *etc.*

H2 receptor antagonist includes, for example, famotidine, lanitidine, cimetidine, *etc*.

Proton pump inhibitors include, for example, omeprazole, *etc*.

Muscarine receptor antagonists include, for example, pirenzepine hydrochloride, *etc*.

Defensive factor enhancers include, for example, sucralfatc, aldioxa, tcprenonc, cetraxate hydrochloride, omoprostil, *etc*.

Anti-pepsine drugs include, for example, sucralfate *etc*.

Prostaglandin derivatives include, for example, omoprostil, misoprostol, *etc*.

Anticholinergic drugs include, for example, mepenzolate bromide, ipratropium bromide, *etc*.

Steroid drus include, for example, prednisolone, *etc*.

Topical anesthetics include, for example, cocaine hydrochlonde, procaine hydrochloride, lidocaine, dibucaine hydrochloride, tetracaine hydrochloride, *etc*.

Immunosuppresants include, for example, cyclosporin, tacrolimus, azathiopurine, *etc*.

Autonomic nerve modulators include, for example, γ-oryzanol, *etc*.

Cholagogues include, for example, ursodesoxycholic acid, *etc.*

Drugs which adjust the function of gastrointestinal tract include, for example, metoclopramide, domperidone, trimebutine maleate, *etc*.

Drugs which assist the function of gastrointestinal tract include, for example, cisapride, bethanechol hydrochloride.

The weight proportion of the compound of formula (I) and the other pharmaceutical preparations is not specifically limited.

Arbitrary two or more of the other pharmaceutical preparations may be administered in combination.

Examples of the other pharmaceutical preparations for compensating for and/or enhancing the preventive and/or treatment effect of the compound of formula (I) include not only those which have so far been found but also those which will be found on the basis of the aforementioned mechanism.

For the purpose above described, the compounds of the present invention of formula (I), non-toxic salts, acid addition salts or hydrates thereof may normally be administered usually systemically or topically, orally or parenterally.

The doses to be administered are determined depending upon, for example, age, body weight, symptom, the desired therapeutic effect, the route of administration, and the duration of the treatment. In the human adult, the doses per person are generally in the range of from 1 mg to 1000 mg, by oral administration, up to several times per day, and in the range of from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered in the form of, for example, solid forms for oral administration, liquid forms for oral administration, injections, liniments or suppositories for parenteral administration.

Solid forms for oral administration include compressed tablets, pills, capsules, dispersible powders, and granules, *etc*.

Capsules include hard capsules and soft capsules.

In these solid forms, one or more of the active compound(s) may be admixed with excipients (e.g. lactose, mannitol, glucose, microcrystalline cellulose, starch), binders (e.g. hydroxypropyl cellulose, polyvinylpyrrolidone or magnesium metasilicate aluminate), disintegrants (e.g. cellulose calcium glycolate), lubricants (e.g. magnesium stearate), stabilizing agents, and adjuvants to assist dissolution (e.g. glutamic acid, aspartic acid) and prepared according to methods well known to those skilled in the art. The solid forms may, if desired, be coated with coating agents (e.g. sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include pharmaceutically acceptable aqueous solutions, suspensions, emulsions, syrups and elixirs, *etc.* In such forms, one or more of the active compound(s) may be dissolved, suspended or emulsified into diluent(s) commonly used in the art (e.g. purified water, ethanol or a mixture thereof). Besides such liquid forms may also comprise wetting agents, suspending agents, emulsifying agents, sweetening agents, flavoring agents, aroma, preservative or buffering agent, *etc*.

Sprays may comprise additional substances other than diluents, such as stabilizing agents (e.g. sodium bisulfite), isotonic buffers (e.g. sodium chloride, sodium citrate or citric acid). For the preparation of such sprays, for example, the method described in the United States Patent No. 2,868,691 or 3,095,355 may be used.

In the present invention, injections for parenteral administration include sterile aqueous and/or inaqueous solutions, suspensions and emulsions. Aqueous solutions and suspensions include, for example, distilled water and physiological saline. Inaqueous solutions and suspensions include, for example, propylene glycol, polyethylene glycol, vegetable oils such as olive oil, alcohols such as ethanol,polysorbate 80 (registered trademark), *etc*. Sterile aqueous and inaqueous solutions, suspensions and emulsions may be mixed in use. These compositions may further comprise some additives, such as antiseptic agents, wetting agents, emulsifying agents, dispersing agents, stabilizing agents (for example, lactose), solubilizing agents (e.g. glutamic acid, aspartic acid). They may be sterilized at the final step, or may be prepared and compensated according to sterile methods. They may also be manufactured in the form of sterile solid forms, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known per se.

Sprays may comprise additional substances other than diluents, such as stabilizing agents (e.g. sodium sulfate), isotonic buffers (e.g. sodium chloride, sodium citrate or citric acid). For preparation of such sprays, for example, the method described in the United States Patent No. 2,868,691 or 3,095,355 may be used.

### Brief description of the figures

Fig. 1 shows the effect of the compound of the present invention against the increase of pregnenolone production by FGIN1-27 in mitochondrium derived from rat adrenal cortex.
Fig. 2 shows the effect of PK11195 against the increase of pregnenolone production by FGIN1-27 in mitochondrium derived from rat adrenal cortex.
Fig. 3 shows an anti-stress effect of the compound of the present invention on a rat.

### Best mode for carrying out the present invention

The following examples and experimental examples illustrate the present invention, but the present invention is not limited to them.

The solvents in the parentheses show the developing or eluting solvents and the ratios of the solvents used are by volume in chromatographic separations and TLC.

Solvents in the parentheses of NMR show the solvents used for measurement.

### Example 1:

### N-(2-chlorobenzyl)-2-methoxybenzamide

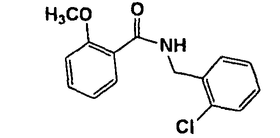

To a solution of 2-chlorobenzylamine (708 mg) and triethylamine (0.7 ml) in tetrahydrofuran (3.0 ml) was added, 2-methoxybenzoyl chloride (853 mg) under cooling with ice, and the mixture was stirred for 1 hour. The reaction mixture was poured into ice water and the precipitate was collected and dried to give the compound of the present invention (1.31 g) having the following physical data.
TLC:Rf 0.45(n-hexane : ethyl acetate = 2 : 1).

### Example 1(1)-Example 1(127)

By the same procedure as described in example 1 using corresponding compounds, the following physical data were given.

### Example 1(1):

### N-(2-chloro-6-fluorobenzyl)-2-trifluoromethylbenzamide

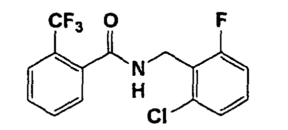

TLC:Rf 0.24(ethyl acetate : n-hexane = 3 : 7);
NMR (CDCl₃):δ 7.69-7.49 (m, 4H), 7.30-7.23 (m, 2H), 7.07-7.01 (m, 1H), 6.07 (brs, 1H), 4.83 (dd, J = 5.7, 1.8 Hz, 2H).

### Example 1(2):

### N-(2-chloro-6-fluorobenzyl)-N-methyl-2-chlorobenzamide

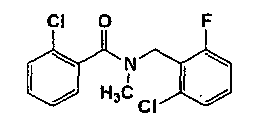

TLC:Rf 0.33 (ethyl acetate : n-hexane = 3 : 7);
NMR (CDCl₃):δ 7.49-6.96 (m, 7H), 5.15 (brd, J = 13.0 Hz, 1H x 3/5), 4.95 (brd, J = 13.0 Hz, 1H x 3/5), 4.69 (d, J = 14.4 Hz, 1H x 2/5), 4.49 (dd, J = 14.4, 1.8 Hz, 1H x 2/5), 2.96 (s, 3H x 2/5), 2.67 (s, 3H x 3/5).

### Example 1(3):

### N-benzyl-N-(2-chloro-6-fluorobenzyl)-2-chlorobenzamide

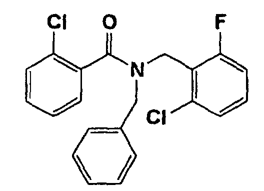

TLC:Rf 0.19(ethyl acetate : n-hexane = 1 : 9);
NMR (CDCl₃):δ 7.50-6.84 (m, 12H), 5.05 (d, J = 15.0 Hz, 1H), 4.94 (m, 1H x 1/2), 4.70 (d, J = 14.3 Hz, 1H x 1/2), 4.49 (d, J = 14.3 Hz, 1H x 1/2), 4.34-4.28 (m, 1.5H).

### Example 1(4):

### N-(2-methylbenzyl)-2,6-dichlorobenzamide

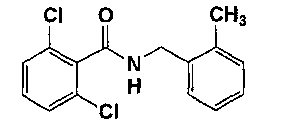

TLC:Rf 0.33 (ethyl acetate : n-hexane = 1 : 3);
NMR (CDCl₃):δ 7.37-7.18 (m, 7H), 5.81 (brs, 1H), 4.67 (d, J = 5.4 Hz, 2H), 2.42 (s, 3H).

### Example 1(5):

### N-(4-fluorobenzyl)-2-chloro-4-fluorobenzamide

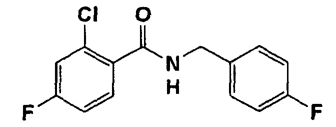

TLC:Rf 0.19(ethyl acetate : n-hexane = 1 : 4);
NMR (CDCl₃):δ 7.75 (dd, J = 8.7, 6.3 Hz, 1H), 7.35 (dd, J = 8.4, 5.4 Hz, 1H), 7.15 (dd, J = 8.4, 2.4 Hz, 1H), 7.09-7.02 (m, 4H), 6.53 (brs, 1H), 4.62 (d, J = 5.7 Hz, 2H).

### Example 1(6):

### N-(4-fluorobenzyl)-2,3-dichlorobenzamide

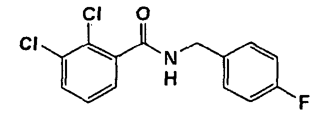

TLC:Rf 0.20 (ethyl acetate : n-hexane = 3 : 7);
NMR (CDCl₃):δ 7.53 (dd, J = 8.1, 1.5 Hz, 1H), 7.48 (dd, J = 8.1, 1.5 Hz, 1H), 7.37-7.24 (m, 3H), 7.09-7.02 (m, 2H), 6.29 (brs, 1H), 4.62 (d, J = 5.7 Hz, 2H).

### Example 1(7):

### N-(4-fluorobenzyl)-2,5-dichlorobenzamide

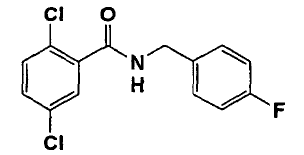

TLC:Rf 0.31 (ethyl acetate : n-hexane = 1 : 4);
NMR (CDCl₃):δ 7.68 (t, J = 1.5 Hz, 1H), 7.37-7.30 (m, 4H), 7.08-7.02 (m, 2H), 6.49 (brs, 1H), 4.62 (d, J = 6.0 Hz, 2H).

### Example 1(8):

### N-benzyl-2,5-dichlorobenzamide

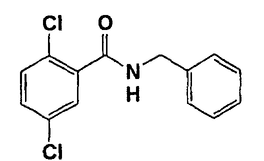

TLC:Rf 0.36 (ethyl acetate : n-hexane = 1 : 4),
NMR (CDCl₃):δ 7.68 (m, 1H), 7.38-7.26 (m, 7H), 6.48 (brs, 1H), 4.66 (d, J = 5.7 Hz, 2H).

### Example 1(9):

### 2-(4-fluorobenzylcarbamoyl)benzoic acid

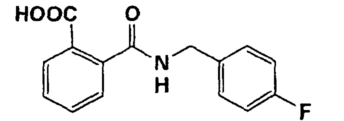

TLC:Rf 0.23(methanol : methylene chloride=1 : 10);
NMR (DMSO-d₆):δ 12.97 (brs, 1H), 8.85 (t, J = 6.0 Hz, 1H), 7.76 (d, J = 7.2 Hz, 1H), 7.60-7.39 (m, 5H), 7.13 (t, J = 9.0 Hz, 2H), 4.40 (d, J = 6.0 Hz, 2H).

### Example 1(10):

### 2-(2-methylbenzylcarbamoyl)benzoic acid

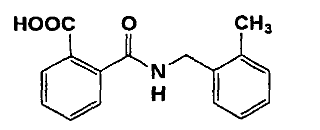

TLC:Rf 0.34(methanol : methylene chloride=1 : 9);
NMR (DMSO-d₆):δ 13.92 (brs, 1H), 8.76 (t, J = 5.4 Hz, 1H), 7.75 (dd, J = 7.5, 1.5 Hz, 1H), 7.59-7.35 (m, 4H), 7.15 (m, 3H), 4.38 (d, J = 5.4 Hz, 2H), 2.31 (s, 3H).

### Example 1(11):

### N-(2-methylbenzyl)-2-benzyloxybenzamide

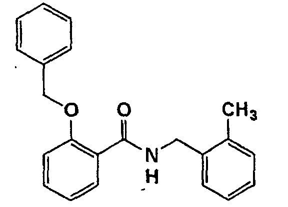

TLC Rf 0.49 (ethyl acetate : n-hexane = 3 : 7);
NMR (CDCl₃):δ 8.29 (dd, J = 7.8, 1.5 Hz, 1H), 8.08 (brs, 1H), 7.48-7.42 (m, 1H), 7.33-7.04 (m, 11H), 5.10 (s, 2H), 4.55 (d, J = 5.4 Hz, 2H), 2.18 (s, 3H).

### Example 1(12):

### N-(2-phenylbenzyl)-2,6-dichlorobenzamide

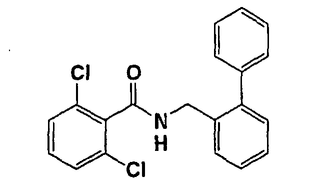

TLC:Rf 0.20(ethyl acetate : n-hexane = 1 : 4);
NMR (CDCl₃):δ 7.65-7.60 (m, 1H), 7.47-7.18 (m, 11H), 5.81 (bs, 1H), 4.65 (d, J = 5.4 Hz, 2H)..

### Example 1(13):

### N-(2-chloro-6-fluorobenzyl)-2-benzyloxybenzamide

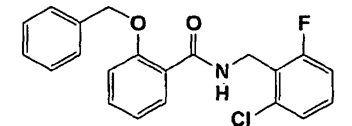

TLC:Rf 0.23(ethyl acetate : n-hexane = 1 : 4);
NMR (CDCl₃):δ 8.35-8.26 (m, 2H), 7.46-7.38 (m, 1H), 7.38-7.28 (m, 5H), 7.20-7.00 (m, 4H), 6.95-6.88 (m, 1H), 5.12 (s, 2H), 4.72 (dd, J = 5.1 Hz, 1.5 Hz, 2H).

### Example 1(14):

### N-(1,2,3,4-tetrahydronaphthalen-1-yl)-2,6-dichlorobenzamide

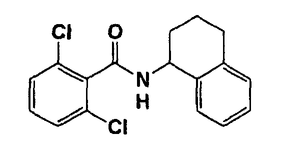

TLC:Rf 0.26 (n-hexane : ethyl acetate = 4 : 1);
NMR (CDCl₃):δ 7.49-7.42 (m, 1H), 7.35-7.28 (m, 2H), 7.25-7.15 (m, 3H), 7.14-7.07 (m, 1H), 6.00-5.90 (m, 1H), 5.46-5.38 (m, 1H), 2.90-2.71 (m, 2H), 2.26-2.14 (m, 1H), 2.12-2.00 (m, 1H), 1.94-1.84 (m, 2H).

### Example 1(15):

### N-(4-fluoro-2-trifluoromethylbenzyl)-2,5-dichlorobenzamide

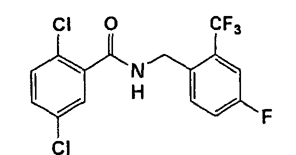

TLC.Rf 0.69(ethyl acetate : n-hexane = 1 : 2);
NMR (CDCl₃):δ 7.71 (dd, J = 8.4 HZ, 5.1 Hz, 1H), 7.69-7 66 (m, 1H), 7.38 (dd, J = 8.4 HZ, 3.0 Hz, 1H), 7.36-7.23 (m, 3H), 6.63 (bs, 1H), 4.79 (d, J = 6.3 Hz, 2H).

### Example 1(16):

### N-(4-fluoro-2-trifluoromethylhenzyl)-2,3-dichlorobenzamide

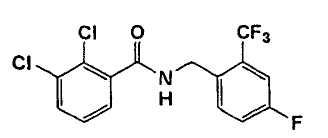

TLC:Rf 0.48(ethyl acetate : n-hexane = 1 : 2);
NMR (CDCl₃):δ 7.73 (dd, J = 8.4 HZ, 5.1 Hz, 1H), 7.54 (dd, J = 8.1 HZ, 1.8 Hz, 1H), 7.46 (dd, J = 7.5 HZ, 1.8 Hz, 1H), 7.39 (dd, J = 8.7 HZ, 2.4 Hz, 1H), 7.32-7.23 (m, 2H), 6.37 (bs, 1H), 4.79 (d, J = 6.0 Hz, 2H).

### Example 1(17):

### N-(2-chloro-4-fluorobenzyl)-2,5-dichlorobenzamide

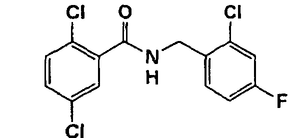

TLC:Rf 0.51 (ethyl acetate : n-hexane = 1 : 2)
NMR (CDCl₃):δ 7.71-7.67 (m, 1H), 7.50 (dd, J = 8.4 HZ, 6.0 Hz, 1H), 7.36-7.32 (m, 2H), 7.16 (dd, J = 8.4 HZ, 2.7 Hz, 1H), 7.00 (td, J = 8.4 HZ, 2.7 Hz, 1H), 6.70 (bs, 1H), 4.69 (d, J = 6.3 Hz, 2H).

### Example 1(18):

### N-(2-chloro-4-fluorobenzyl)-2,3-dichlorobenzamide

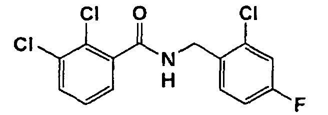

TLC:Rf 0.26(ethyl acetate : n-hexane = 1 : 2);
NMR (CDCl₃):δ 7.56-7.45 (m, 2H), 7.30-7.23 (m, 2H), 7.16 (dd, J = 8.4 HZ, 2.7 Hz, 1H), 7.00 (td, J = 8.4 HZ, 2.7 Hz, 1H), 6.47 (bs, 1H), 4.70 (d, J = 6.0 Hz, 2H).

### Example 1(19):

### N-(2,5-dichlorobenzyl)-2,5-dichlorobenzamide

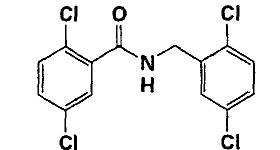

TLC:Rf 0.58(ethyl acetate : n-hexane = 1 : 3);
NMR (CDCl₃):δ 7.72-7.69 (m, 1H), 7.50 (d, J = 2.4 Hz, 1H), 7.38-7.30 (m, 2H), 7.28-7.21 (m, 2H), 6.74 (bs, 1H), 4.70 (d, J = 6.0 Hz, 2H).

### Example 1(20):

### N-(2,5-difluorobenzyl)-2,5-dichlorobenzamide

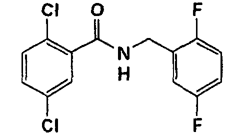

TLC:Rf 0.57(ethyl acetate : n-hexane = 1 : 3);
NMR (CDCl₃):δ 7.70-7.67 (m, 1H), 7.38-7.32 (m, 2H), 7.20-7.13 (m, 1H), 7.08-6.92 (m, 2H), 6.63 (bs, 1H), 4.67 (d, J = 6.3 Hz, 2H).

### Example 1(21):

### N-(2,5-dichlorobenzyl)-2-chlorobenzamide

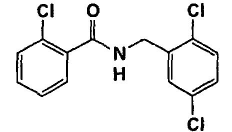

TLC:Rf 0.34(ethyl acetate : n-hexane = 1 : 3);
NMR (CDCl₃):δ 7.74-7.70 (m, 1H), 7.52 (d, J = 2.4 Hz, 1H), 7.44-7.30 (m, 4H), 7.23 (dd, J = 8.1, 2.4 Hz, 1H), 6.73 (bs, 1H), 4.71 (d, J = 6.3 Hz, 2H).

### Example 1(22):

### N-(2,5-difluorobenzyl)-2-chlorobenzamide

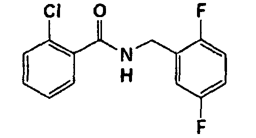

TLC:Rf 0.29(ethyl acetate : n-hexane = 1 : 3);
NMR (CDCl₃):δ 7.73-7.68 (m, 1H), 7.44-7.30 (m, 3H), 7.22-7.14 (m, 1H), 7.07-6.90 (m, 2H), 6.64 (bs, 1H), 4.69 (d, J = 6.0 Hz, 2H).

### Example 1(23):

### N-(4-fluoro-2-trifluoromethylbenzyl)-2-chlorobenzamide

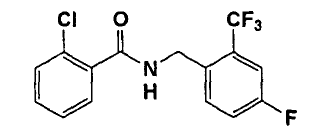

TLC:Rf 0.33(ethyl acetate : n-hexane = 1 : 3);
NMR (CDCl₃):δ 7.76-7.64 (m, 2H), 7.42-7.22 (m, 3H), 6.62 (bs, 1H), 4.80 (d, J = 6.3 Hz, 2H).

### Example 1(24):

### N-(2-chloro-4-fluorobenzyl)-2-chlorobenzamide

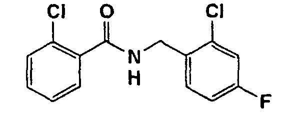

TLC:Rf 0.21 (ethyl acetate : n-hexane = 1 : 4);
NMR (CDCl₃):δ 7.70 (dd, J = 6.9 Hz, 1.8 Hz, 1H), 7.52 (dd, J = 8.7 Hz, 6.0 Hz, 1H), 7.42-7.29 (m, 3H), 7.15 (dd, J = 8.7 Hz, 2.4 Hz, 1H), 6.99 (td, J = 8.7 Hz, 2.4 Hz, 1H), 6.70 (bs, 1H), 4.71 (d, J = 6.0 Hz, 2H).

### Example 1(25):

### N-(4-fluoro-2-trifluoromethylbenzyl)-5-fluoro-2-trifluoromethylbenzamide

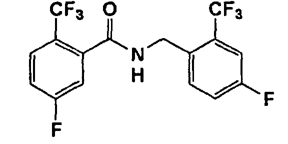

TLC:Rf 0.50(ethyl acetate : n-hexane = 1 : 3);
NMR (CDCl₃):δ 7.74-7.67 (m, 2H), 7.39 (dd, J = 8.7 Hz, 2.7 Hz, 1H), 7.32-7.18 (m, 3H), 6 13 (bs, 1H), 4.76 (d, J = 6.0 Hz, 2H).

### Example 1(26):

### N-(2-chloro-4-fluorobenzyl)-5-fluoro-2-trifluoromethylbenzamide

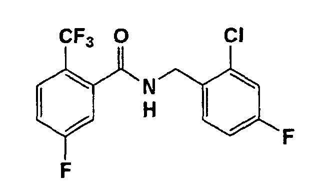

TLC:Rf 0.44(ethyl acetate : n-hexane = 1 : 3);
NMR (CDCl₃):δ 7.70 (dd, J = 8.1 Hz, 4.8 Hz, 1H), 7.50 (dd, J = 8.4 Hz, 6.0 Hz, 1H), 7.28-7.18 (m, 2H), 7.16 (dd, J = 8.4 Hz, 2.7 Hz, 1H), 7.01 (td, J = 8.4 Hz, 2.7 Hz, 1H), 6.22 (bs, 1H), 4.68 (d, J = 6.0 Hz, 2H).

### Example 1(27):

### N-(3,5-dimethoxybenzyl)-2-chlorobenzamide

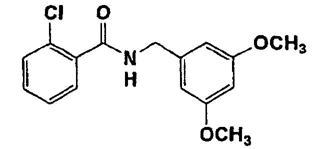

TLC:Rf 0.77 (methanol : chloroform=1 : 10);
NMR (CDCl₃):δ 7.68-7.64 (m, 1H), 7.38-7 26 (m, 4H), 6.69 (brs, 1H), 6.46-6.43 (m, 2H), 4.58 (d, J = 5.7 Hz, 2H), 3.83 (s, 3H), 3.80 (s, 3H).

### Example 1(28):

### N-phenethyl-2-chlorobenzamide

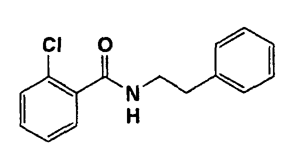

TLC:Rf 0.74 (methanol : chloroform=1 : 20);
NMR (CDCl₃):δ 7.63-7.58 (m, 1H), 7.38-7.19 (m, 8H), 6.21 (brs, 1H), 3.75 (q, J = 7.0 Hz, 2H), 2.96 (t, J = 7.0 Hz, 2H).

### Example 1(29):

### N-(1-phenylethyl)-2-chlorobenzamide

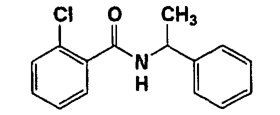

TLC:Rf 0.44 (ethyl acetate : n-hexane = 3 : 7);
NMR (CDCl₃):δ 7.68-7.65 (m, 1H), 7.43-7.26 (m, 8H), 6.46 (brs, 1H), 5.34 (m, 1H), 1.62 (d, J = 6.9 Hz, 3H).

### Example 1(30):

### N-(indan-1-yl)-2-chlorobenzamide

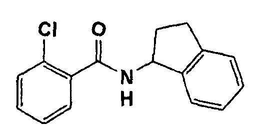

TLC:Rf 0.40 (ethyl acetate : n-hexane = 3 : 7);
NMR (CDCl₃):δ 7.73-7.70 (m, 1H), 7.43-7.21 (m, 7H), 6.38 (brd, 1H), 5.72 (td, J = 7.8, 7.8 Hz, 1H), 3 09-2.88 (m, 2H), 2.78-2.67 (m, 1H), 2.03-1.91 (m, 1H).

### Example 1(31):

### N-(2-methylbenzyl)-2-chlorobenzamide

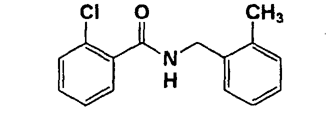

TLC:Rf 0.30 (ethyl acetate : n-hexane = 3 : 7);
NMR (CDCl₃):δ 7.69-7.66 (m, 1H), 7.40-7.19 (m, 7H), 6.32 (brs, 1H), 4.65 (d, J = 5.4 Hz, 2H), 2.40 (s, 3H).

### Example 1(32):

### N-(2,6-difluorobenzyl)-2-chlorobenzamide

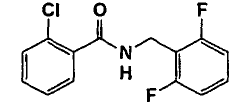

TLC:Rf 0.32 (ethyl acetate : n-hexane = 3 : 7);
NMR(CDCl₃):δ 7.69-7.66 (m, 1H), 7.40-7.22 (m, 4H), 6.95-6.89 (m, 2H), 6.59 (brs, 1H), 4.76 (d, J = 5.4 Hz, 2H).

### Example 1(33):

### N-(2-chloro-6-fluorobenzyl)-2-chlorobenzamide

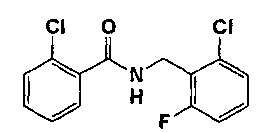

TLC:Rf 0.33 (ethyl acetate : n-hexane = 3 : 7);
NMR (CDCl₃):δ 7.71-7.68 (m, 1H), 7.39-7.21 (m, 5H), 7.07-7.01 (m, 1H), 6.58 (brs, 1H), 4 86-4.83 (m, 2H).

### Example 1(34):

### N-(2-chloro-6-fluorobenzyl)-2-methylbenzamide

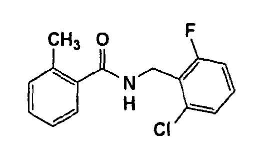

TLC:Rf 0.49 (ethyl acetate : n-hexane = 3 : 7);
NMR (CDCl₃):δ 7.35-7.14 (m, 6H), 7.07-7.01 (m, 1H), 6.04 (brs, 1H), 4.82-4.80 (m, 2H), 2.43 (s, 3H).

### Example 1(35):

### N-(2,6-dimethylbenzyl)-2-chlorobenzamide

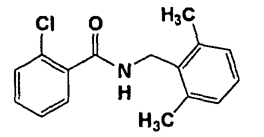

TLC:Rf 0.42 (ethyl acetate : n-hexane = 3 : 7);
NMR (CDCl₃):δ7.68-7.63 (m, 1H), 7.37-7.03 (m, 6H), 5.97 (brs, 1H), 4.68 (d, J = 4.8 Hz, 2H), 2.43 (s, 6H).

### Example 1(36):

### N-(2-chloro-6-fluorobenzyl)-2-methoxybenzamide

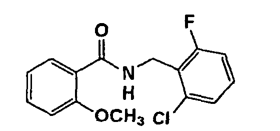

TLC:Rf 0.28 (ethyl acetate : n-hexane = 3 : 7);
NMR (CDCl₃):δ 8.47 (brs, 1H), 8.23 (dd, J = 8.0, 2.0 Hz, 1H), 7.47-7.38 (m, 1H), 7.27-6.93 (m, 5H), 4.86-4.83 (m, 2H), 3.94 (s, 3H).

### Example 1(37):

### N-(2-chloro-6-fluorobenzyl)-2-furylcarboxamide

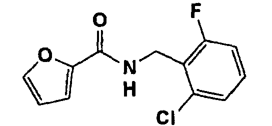

TLC:Rf 0.29 (ethyl acetate : n-hexane = 3 : 7);
NMR (CDCl₃):δ 7.41 (dd, J = 1.8, 0.9 Hz, 1H), 7.28-7.20 (m, 2H), 7.13 (dd, J = 3.3, 0.9 Hz, 1H), 7.06-7.00 (m, 1H), 6.67 (brs, 1H), 6.48 (dd, J = 3.3, 1.8 Hz, 1H), 4.80 (dd, J = 5.9, 1.8 Hz, 2H).

### Example 1(38):

### N-(2,4-dichlorobenzyl)-2-chlorobenzamide

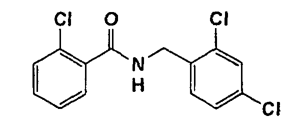

TLC:Rf 0.34 (ethyl acetate: n-hexane = 3 : 7);
NMR (CDCl₃):δ 7.73-7.68 (m, 1H), 7.50-7.23 (m, 6H), 6.73 (brs, 1H), 4.71 (d, J = 5.8 Hz, 2H).

### Example 1(39):

### N-(3,4-dichlorobenzyl)-2-chlorobenzamide

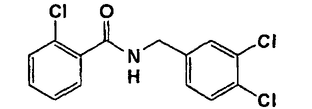

TLC:Rf 0.35 (ethyl acetate : n-hexane = 3 : 7),
NMR (CDCl₃):δ 7.72-7.67 (m, 1H), 7.48-7.20 (m, 6H), 6.62 (brs, 1H), 4.61 (d, J = 5.8 Hz, 2H).

### Example 1(40):

### N-(4-trifluoromethylbenzyl)-2-chlorobenzamide

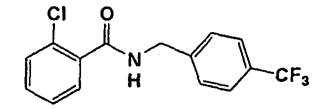

TLC:Rf 0.21 (ethyl acetate : n-hexane = 3 : 7);
NMR (CDCl₃):δ 7.75-7.26 (m, 8H), 6.63 (brs, 1H), 4.73 (d, J = 6.0 Hz, 2H).

### Example 1(41):

### N-(2-chloro-6-fluorobenzyl)phenylacetamide

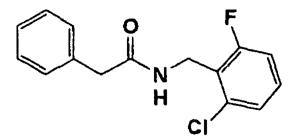

TLC:Rf 0.29 (ethyl acetate : n-hexane = 3 : 7);
NMR (CDCl₃):δ 7.39-7.16 (m, 7H), 7.02-6.93 (m, 1H), 5.76 (brs, 1H), 4.58 (dd, J = 5.6, 1.6 Hz, 2H), 3.57 (s, 2H).

### Example 1(42):

### N-(4-chlorobenzyl)-2-chlorobenzamide

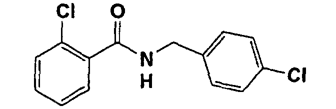

TLC:Rf 0.32 (ethyl acetate : n-hexane = 3 : 7);
NMR (CDCl₃):δ 7.73-7.68 (m, 1H), 7.43-7.26 (m, 7H), 6.52 (brs, 1H), 4.64 (d, J = 5.8 Hz, 2H).

### Example 1(43):

### N-(1-naphthylmethyl)-2-chlorobenzamide

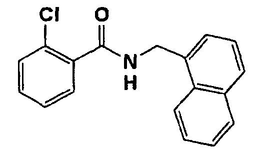

TLC:Rf0.36 (ethyl acetate : n-hexane = 3 : 7);
NMR (CDCl₃):δ 8.15-8.11 (m, 1H), 7.92-7.82 (m, 2H), 7.69-7.25 (m, 8H), 6.40 (brs, 1H), 5.12 (d, J = 5 0 Hz, 2H).

### Example 1(44):

### N-(2-chloro-6-fluorobenzyl)-2,6-dimethoxybenzamide

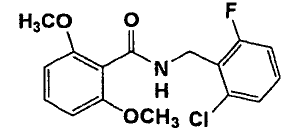

TLC:Rf 0.32 (ethyl acetate : n-hexane = 1 : 1);
NMR (CDCl₃):δ 7.27-6.99 (m, 4H), 6.52 (d, J = 8.4 Hz, 2H), 6.06 (brs, 1H), 4.81 (dd, J = 5.7, 1.5 Hz, 2H), 3.76 (s, 6H).

### Example 1(45):

### N-(2-chloro-6-fluorobenzyl)-2,6-dichlorobenzamide

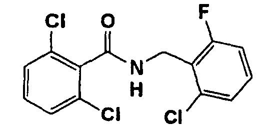

TLC:Rf 0.56 (ethyl acetate : n-hexane = 1 : 1);
NMR (CDCl₃):δ 7.35-7.23 (m, 5H), 7.07-7.01 (m, 1H), 6.01 (brs, 1H), 4.86 (dd, J = 5.7, 1.8 Hz, 2H).

### Example 1(46):

### N-(2-chloro-6-fluorobenzyl)-2-nitrobenzamide

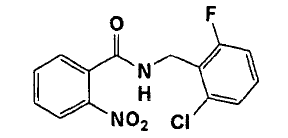

TLC:Rf 0.42 (ethyl acetate : n-hexane = 1 : 1);
NMR (CDCl₃):δ 8 06 (dd, J = 8.1, 1.2 Hz, 1H), 7.68-7.50 (m, 3H), 7.31-7.22 (m, 2H), 7.09-7.02 (m, 1H), 6.11 (brs, 1H), 4.85 (dd, J = 5.9, 1.7 Hz, 2H).

### Example 1(47):

### N-(2-chloro-6-fluorobenzyl)-2-benzoyloxymethylbenzamide

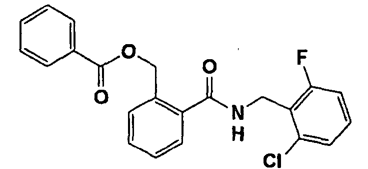

TLC:Rf 0.25 (ethyl acetate : n-hexane = 1 : 4);
NMR (CDCl₃):δ 7.96-7.93 (m, 2H), 7 58-7.13 (m, 9H), 6.98-6.92 (m, 1H), 6.59 (brs, 1H), 5.53 (s, 2H), 4.81 (dd, J = 5.4, 1.5 Hz, 2H).

### Example 1(48):

### N-(2-chloro-6-fluorobenzyl)-2-dimethylaminobenzamide

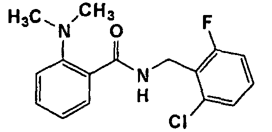

TLC:Rf 0.38 (ethyl acetate : n-hexane = 3 : 7);
NMR (CDCl₃):δ 10 33 (brs, 1H), 8.19-8.16 (m, 1H), 7.43-7.37 (m, 1H), 7.26-7.15 (m, 4H), 7.06-7.00 (m, 1H), 4.85 (dd, J = 5.7, 1.5 Hz, 2H), 2.63 (s, 6H).

### Example 1(49):

### N-(2-chloro-6-fluorobenzyl)-2-(4-methoxyphenoxymethyl)benzamide

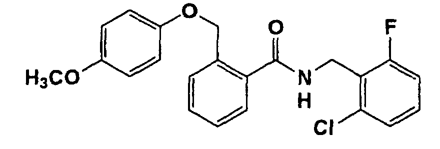

TLC:Rf 0.43 (ethyl acetate : n-hexane = 1 : 4);
NMR (CDCl₃):δ 7.71-7.68 (m, 1H), 7.49-7.38 (m, 3H), 7.15-7.03 (m, 2H), 6.89-6.83 (m, 2H), 6.74 (s, 4H), 5.05 (s, 2H), 4.76 (dd, J = 5.4, 1.5 Hz, 1H), 3.78 (s, 3H).

### Example 1(50):

### N-(2-chloro-6-fluorobenzyl)-2,6-dimethylbenzamide

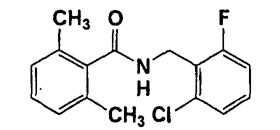

TLC:Rf 0.38 (ethyl acetate : n-hexane = 3 : 7);
NMR (CDCl₃):δ 7.29-6.97 (m, 6H), 5.88 (brs, 1H), 4.82 (dd, J = 5.4, 1.5 Hz, 2H), 2.27 (s, 6H).

### Example 1(51):

### N-(2-chloro-6-fluorobenzyl)-1-naphthylcarboxamide

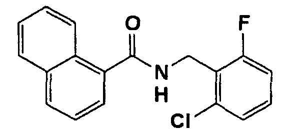

TLC:Rf 0.49 (ethyl acetate : n-hexane = 3 : 7);
NMR (CDCl₃):δ 8.34-8.31 (m, 1H), 7.92-7.84 (m, 2H), 7.61-7.41 (m, 4H), 7.28-7.23 (m, 2H), 7.09-7.03 (m, 1H), 6.26 (brs, 1H), 4.93 (dd, J = 5.7, 1.5 Hz, 2H)

### Example 1(52):

### 2-(2,6-dichlorobenzoyl)-1,2,3,4-tetrahydroisoquinoline

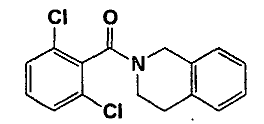

TLC:Rf0.43 (ethyl acetate : n-hexane = 3 : 7);
NMR (CDCl₃):δ 7.38-6.92 (m, 7H), 4.98 (s, 2H x 3/5), 4.38 (s, 2H x 2/5), 4.05 (t, J = 6.0 Hz, 2H x 2/5), 3.49 (t, J = 6.0 Hz, 2H x 3/5), 3.00 (t, J = 6.0 Hz, 2H x 2/5), 2.90 (t, J = 6.0 Hz, 2H x 3/5).

### Example 1(53):

### N-(2-chloro-6-fluorobenzyl)-2,3-dichlorobenzamide

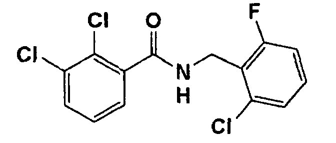

TLC:Rf 0.43 (ethyl acetate : n-hexane = 3 : 7);
NMR (CDCl₃):δ 7.53-7.46 (m, 2H), 7.30-7.22 (m, 3H), 7.08-7.01 (m, 1H), 6.33 (brs, 1H), 4.84 (dd, J = 5.7, 1.5 Hz, 2H).

### Example 1(54):

### N-(2-chloro-6-fluorobenzyl)-2,4-dichlorobenzamide

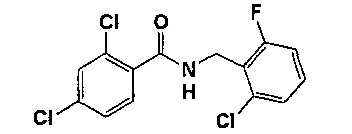

TLC:Rf 0.50 (ethyl acetate : n-hexane = 3 : 7);
NMR (CDCl₃):δ 7.66 (d, J = 8.1 Hz, 1H), 7.39 (d, J = 2.1 Hz, 1H), 7.31-7.21 (m, 3H), 7.07-7.01 (m, 1H), 6.64 (brs, 1H), 4.83 (dd, J = 5.7, 1.5 Hz, 2H).

### Example 1(55):

### N-(2-methoxybenzyl)-2,6-dichlorobenzamide

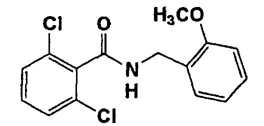

TLC:Rf 0.31 (ethyl acetate : n-hexane = 3 : 7);
NMR (CDCl₃):δ 7.43-7.20 (m, 5H), 6.95 (t, J = 7.2 Hz, 1H), 6.88 (d, J = 8.1 Hz, 1H), 6.18 (brs, 1H), 4.67 (d, J = 6.3 Hz, 2H), 3.85 (s, 3H).

### Example 1(56):

### N-(4-methylhenzyl)-2,6-dichlorobenzamide

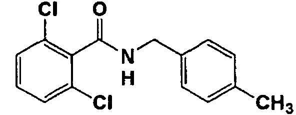

TLC.Rf 0.42 (ethyl acetate : n-hexane = 3 : 7);
NMR (CDCl₃):δ 7.35-7.21 (m, 5H), 7.16 (d, J = 8.4 Hz, 2H), 5.95 (brs, 1H), 4.64 (d, J = 5.4 Hz, 2H), 2.34 (s, 3H).

### Example 1(57):

### N-(2-trifluoromethylbenzyl)-2,6-dichlorobenzamide

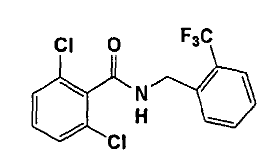

TLC:Rf 0.49 (ethyl acetate : n-hexane = 3 : 7);
NMR (CDCl₃):δ 7.80 (d, J = 7.8 Hz, 1H), 7.67 (d, J = 7.8 Hz, 1H), 7.58 (t, J = 7.8 Hz, 1H), 7.42 (t, J = 7.8 Hz, 1H), 7.33-7.22 (m, 3H), 6.07 (brs, 1H), 4.86 (d, J = 6.3 Hz, 2H).

### Example 1(58):

### N-(2-methoxvbenzvl)-2-chloro-6-fluorobenzamide

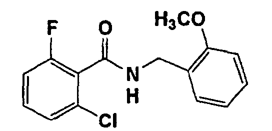

TLC:Rf 0.40 (ethyl acetate : n-hexane = 3 : 7);
NMR (CDCl₃):δ 7.38 (dd, J = 7.5, 1.5 Hz, 1H), 7.32-7.26 (m, 2H), 7.21-7.17 (m, 1H), 7.05-6.93 (m, 2H), 6.89 (d, J = 8.1 Hz, 1H), 6.27 (brs, 1H), 4.67 (d, J = 5.7 Hz, 2H), 3.85 (s, 3H).

### Example 1(59):

### N-(2-methoxybenzyl)-2,6-difluorobenzamide

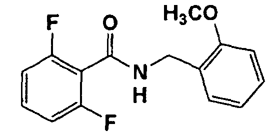

TLC:Rf 0.39 (ethyl acetate : n-hexane = 3 : 7);
NMR (CDCl₃):δ 7.37-7.26 (m, 3H), 6.97-6.88 (m, 4H), 6.45 (brs, 1H), 4.65 (d, J = 6.0 Hz, 2H), 3.87 (s, 3H).

### Example 1(60):

### N-(3-methoxybenzyl)-2,6-dichlorobenzamide

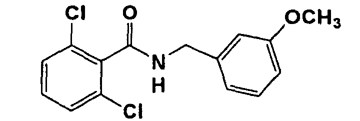

TLC:Rf 0.37 (ethyl acetate : n-hexane = 3 : 7);
NMR (CDCl₃):δ 7.34-7.22 (m, 4H), 6.98-6.96 (m, 2H), 6.85-6.81 (m, 1H), 6.02 (brs, 1H), 4.66 (d, J = 6.0 Hz, 2H), 3.80 (s, 3H).

### Example 1(61):

### N-(4-methoxybenzyl)-2,6-dichlorobenzamide

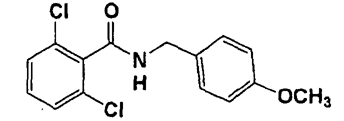

TLC:Rf 0.35 (ethyl acetate : n-hexane = 3 : 7);
NMR (CDCl₃):δ 7.35-7.21 (m, 5H), 6.90-6.86 (m, 2H), 5.96 (brs, 1H), 4.61 (d, J = 5 4 Hz, 2H), 3.80 (s, 3H).

### Example 1(62):

### N-(2-chloro-6-fluorobenzyl)-2,5-dichlorobenzamide

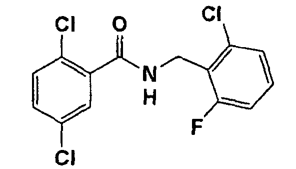

TLC:Rf 0.63 (ethyl acetate : n-hexane = 3 : 7);
NMR (CDCl₃):δ 7 69 (t, J = 1.5 Hz, 1H), 7.32-7.24 (m, 4H), 7.08-7.02 (m, 1H), 6.58 (brs, 1H), 4.84 (dd, J = 6.0, 1.5 Hz, 2H).

### Example 1(63):

### N-(4-fluorobenzyl)-2,6-dichlorobenzamide

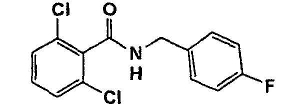

TLC:Rf 0.26 (ethyl acetate : n-hexane = 3 : 7);
NMR (CDCl₃):δ 7.40-7.22 (m, 5H), 7.07-7.01 (m, 2H), 6.03 (brs, 1H), 4.65 (d, J = 5.7 Hz, 2H).

### Example 1(64):

### N-(2-chloro-6-fluorobenzyl)-2,4,6-trichlorobenzamide

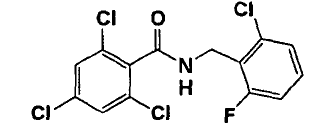

TLC:Rf 0.33 (ethyl acetate : n-hexane = 3 : 7);
NMR (CDCl₃):δ 7.38-7.23 (m, 4H), 7.07-7.01 (m, 1H), 6.63 (brs, 1H), 4.84 (dd, J = 5.4, 1.5 Hz, 2H).

### Example 1(65):

### N-(2-chloro-6-fluorobenzyl)-4-fluorobenzamide

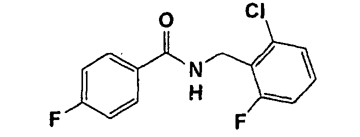

TLC:Rf 0.37 (ethyl acetate : n-hexane = 3 : 7);
NMR (CDCl₃):δ 7.80-7.75 (m, 2H), 7.29-7.21 (m, 2H), 7.13-7.01 (m, 3H), 6.42 (brs, 1H), 4.82 (dd, J = 5.4, 1.5 Hz, 2H).

### Example 1(66):

### N-(4-fluorobenzyl)-4-fluorobenzamide

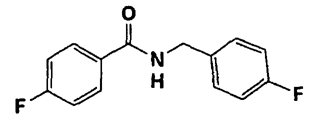

TLC:Rf 0.28 (ethyl acetate : n-hexane = 3 : 7);
NMR (CDCl₃):δ 7.82-7.77 (m, 2H), 7 35-7.30 (m, 2H), 7.14-7.01 (m, 4H), 6.37 (brs, 1H), 4.60 (d, J = 5.7 Hz, 2H).

### Example 1(67):

### N-(2-chloro-6-fluorobenzyl)cyclohexylcarboxamide

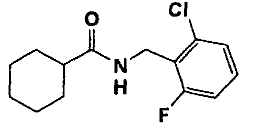

TLC:Rf 0.47 (ethyl acetate : n-hexane = 3 : 7);
NMR (CDCl₃):δ 7.27-7.18 (m, 2H), 7.04-6.97 (m, 1H), 5.73 (brs, 1H), 4.61 (dd, J = 5.4, 1.2 Hz, 2H), 2.11-2.02 (m, 1H), 1.87-1.20 (m, 10H).

### Example 1(68):

### N-(2-chloro-6-fluorobenzyl)cyclopentylcarboxamide

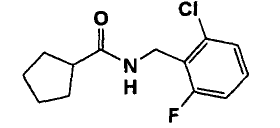

TLC Rf 0.44 (ethyl acetate : n-hexane = 3 : 7);
NMR (CDCl₃):δ 7.27-7.18 (m, 2H), 7.04-6.98 (m, 1H), 5.75 (brs, 1H), 4.62 (dd, J = 5.4, 1.5 Hz, 2H), 2.53-2.45 (m, 1H), 1.86-1.51 (m, 8H).

### Example 1(69):

### N-cyclohexylmethyl-2,3-dichlorobenzamide

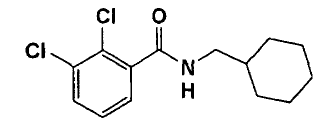

TLC:Rf 0.17 (ethyl acetate : n-hexane = 1 : 4);
NMR (CDCl₃):δ 7.51 (dd, J = 7.8, 1.5 Hz, 1H), 7.45 (dd, J = 7.8, 1.5 Hz, 1H), 7.25 (t, J = 7.8 Hz, 1H), 6.04 (brs, 1H), 3.31 (t, J = 6.3 Hz, 2H), 1.83-0.96 (m, 11H).

### Example 1(70):

### N-(2-furylmethyl)-2,3-dichlorobenzamide

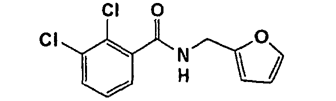

TLC:Rf 0.37 (ethyl acetate : n-hexane = 3 : 7);
NMR (CDCl₃):δ 7.52 (dd, J = 7.8, 1.5 Hz, 1H), 7.45 (dd, J = 7.8, 1.5 Hz, 1H), 7.38 (t, J = 0.9 Hz, 1H), 7.24 (t, J = 7.8 Hz, 1H), 6.36 (brs, 1H), 6.35-6.31 (m, 2H), 4.64 (d, J = 5.4 Hz, 2H).

### Example 1(71):

### N-(1-adamantylmethyl)-2,3-dichlorobenzamide

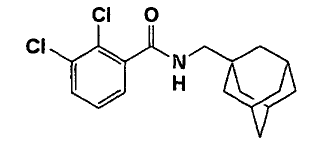

TLC:Rf 0.50 (ethyl acetate : n-hexane = 3 : 7);
NMR (CDCl₃):δ 7.52 (dd, J = 7.8, 1.5 Hz, 1H), 7 47 (dd, J = 7.8, 1.5 Hz, 1H), 7.26 (t, J = 7.8 Hz, 1H), 6.03 (brs, 1H), 3.17 (d, J = 6.6 Hz, 2H), 2.01-1.58 (m, 15H).

### Example 1(72):

### N-((2R)-2-norbornyl)-2,3-dichlorobenzamide

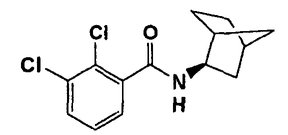

TLC:Rf0.44 (ethyl acetate : n-hexane = 3 : 7);
NMR (CDCl₃):δ 7.50 (dd, J = 7.8, 1.5 Hz, 1H), 7.43 (dd, J = 7.8, 1.5 Hz, 1H), 7.24 (t, J = 7.8 Hz, 1H), 5.88 (brs, 1H), 3.95-3.89 (m, 1H), 2.37-2.33 (m, 2H), 1.94-1.86 (m, 1H), 1.65-1.15 (m, 7H).

### Example 1(73):

### N-(2-chloro-6-phenoxybenzyl)-2,3-dichlorobenzamide

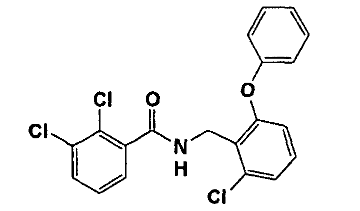

TLC:Rf 0.55 (ethyl acetate : n-hexane = 3 : 7);
NMR (CDCl₃):δ 7.49 (dd, J = 7.8, 1.5 Hz, 1H), 7.40-7.11 (m, 7H), 7.02-6.98 (m, 2H), 6.80 (t, J = 4.8 Hz, 1H), 6.33 (brs, 1H), 4.91 (d, J = 5.4 Hz, 2H).

### Example 1(74):

### N-(2-chloro-6-fluorobenzyl)-2-naphthylcarboxamide

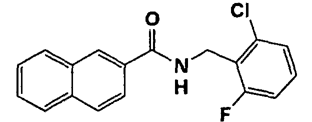

TLC:Rf 0.50 (ethyl acetate : n-hexane = 3 : 7);
NMR (CDCl₃):δ 8.28 (s, 1H), 7.92-7.80 (m, 4H), 7.59-7.50 (m, 2H), 7.27-7.04 (m, 3H), 6.62 (brs, 1H), 4.90 (dd, J = 5.7, 1.2 Hz, 2H).

### Example 1(75):

### N-(4-fluorobenzyl)-2,4-dichlorobenzamide

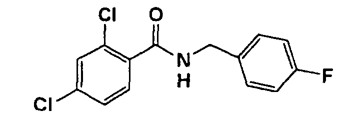

TLC:Rf 0.38 (ethyl acetate : n-hexane = 1 : 2).

### Example 1(76):

### N-(4-fluorobenzyl)-2-chloro-4-nitrobenzamide

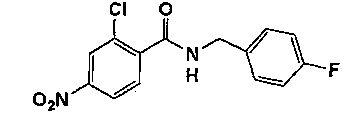

TLC:Rf 0.27 (ethyl acetate : n-hexane = 1 : 2).

### Example 1(77):

### N-(4-fluorobenzyl)-4-cyanobenzamide

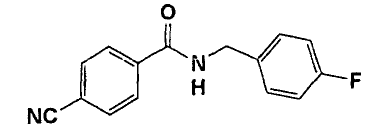

TLC:Rf 0.23 (ethyl acetate : n-hexane = 1 : 2).

### Example 1(78):

### N-(4-fluorobenzyl)-4-methylbenzamide

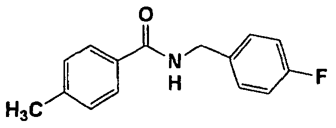

TLC:Rf 0.38 (ethyl acetate : n-hexane = 1 : 2).

### Example 1(79):

### N-(4-fluorobenzyl)-4-chlorobenzamide

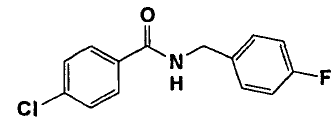

TLC:Rf 0.43 (ethyl acetate : n-hexane = 1 : 2).

### Example 1(80):

### N-(4-fluorobenzyl)-4-nitrobenzamide

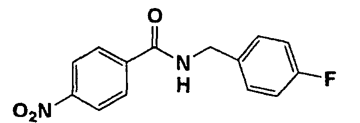

TLC:Rf 0.12 (ethyl acetate:toluene=1 : 9).

### Example 1(81):

### N-(4-fluorobenzyl)-2-nitrobenzamide

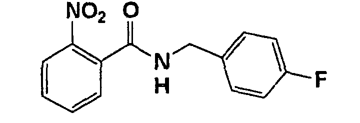

TLC:Rf 0.10 (ethyl acetate:toluene=1 : 9).

### Example 1(82):

### N-(4-fluorobenzyl)-2-thienylcarboxamide

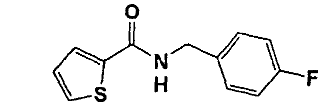

TLC:Rf 0.29 (ethyl acetate : n-hexane = 1 : 2);
NMR (CDCl₃):δ 7.52-7.46 (m, 2H), 7.36-7.28 (m, 2H), 7.10-7.00 (m, 3H), 6.26 (bs, 1H), 4 59 (d, J = 5.7 Hz, 2H).

### Example 1 (83):

### N-(4-fluorobenzyl)-[2-(3-chlorothienyl)]carboxamide

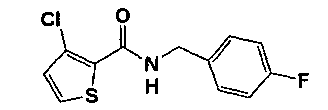

TLC:Rf 0.42 (ethyl acetate : n-hexane = 1 : 3);
NMR (CDCl₃):δ 7.48 (d, J = 5.4 Hz, 1H), 7.38-7.30 (m, 2H), 7.30-7.22 (b, 1H), 7.08-7.00 (m, 2H), 6.98 (d, J = 5.4 Hz, 1H), 4.63 (d, J = 6.0 Hz, 2H).

### Example 1(84):

### N-(4-fluorobenzyl)-3-thienylcarboxamide

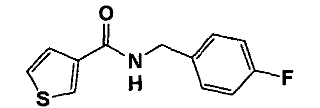

TLC:Rf 0.30 (ethyl acetate : n-hexane = 1 : 2);
NMR (CDCl₃):
δ 7.88 (dd, J = 2.7 Hz, 1.5 Hz, 1H), 7.40-7.28 (m, 4H), 7.08-6.99 (m, 2H), 6.24 (bs, 1H), 4.59 (d, J = 6.0 Hz, 2H).

### Example 1(85):

### N-(2-phenoxybenzyl)-2,3-dichlorobenzamide

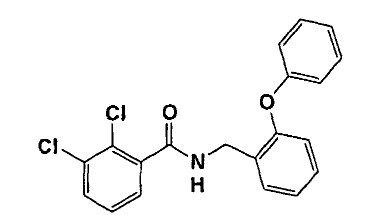

TLC:Rf 0.26 (ethyl acetate : n-hexane = 1 : 4);
NMR (CDCl₃):δ 7.54-7.48 (m, 2H), 7.41-7.07 (m, 7H), 7.00-6.94 (m, 2H), 6.90 (dd, J = 7.8 Hz, 1.5 Hz, 1H), 6.50-6.42 (m, 1H), 4.70 (d, J = 5.7 Hz, 2H).

### Example 1(86):

### N-(2-phenoxybenzyl)-2,4-dichlorobenzamide

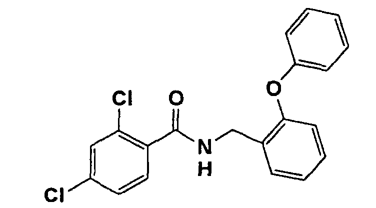

TLC:Rf 0.23 (ethyl acetate : n-hexane = 1 : 4);
NMR (CDCl₃):δ 7.59 (d, J = 8.4 Hz, 1H), 7.50 (dd, J = 7.2 Hz, 1.5 Hz, 1H), 7.39 (d, J = 2.1 Hz, 1H), 7.36-7.23 (m, 4H), 7.16-7.07 (m, 2H), 7.00-6.94 (m, 2H), 6.90 (dd, J = 8.1 Hz, 1.0 Hz, 1H), 6.76-6.68 (m, 1H), 4.70 (d, J = 6.0 Hz, 2H).

### Example 1(87):

### N-(2-phenoxybenzyl)-2,5-dichlorobenzamide

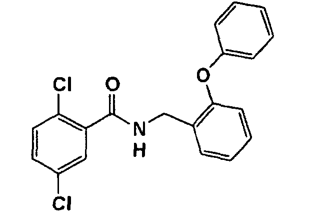

TLC:Rf 0.35 (ethyl acetate : n-hexane = 1 : 4);
NMR (CDCl₃):δ 7.62-7.58 (m, 1H), 7.51 (dd, J = 7.8 Hz, 1.5 Hz, 1H), 7.38-7.22 (m, 5H), 7.18-7.07 (m, 2H), 7.01-6.94 (m, 2H), 6.90 (d, J = 7.8 Hz, 1H), 6.74-6.62 (m, 1H), 4.70 (d, J = 6.0 Hz, 2H).

### Example 1(88):

### N-(4-fluoro-2-trifluoromethylbenzyl)-2-trifluoromethylbenzamide

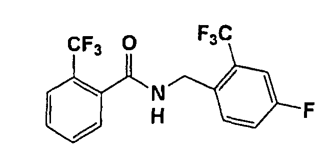

TLC:Rf 0.27 (ethyl acetate : n-hexane = 1 : 3);
NMR (CDCl₃):δ 7.76-7.67 (m, 2H), 7.63-7.50 (m, 3H), 7.38 (dd, J = 9 Hz, 3 Hz, 1H), 7.32-7.24 (m, 1H), 6.20-6.00 (m, 1H), 4.77 (d, J = 6.3 Hz, 2H).

### Example 1(89):

### N-(2-chloro-4-fluorobenzyl)-2-trifluoromethylbenzamide

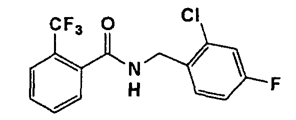

TLC:Rf 0.23 (ethyl acetate : n-hexane = 1 : 3);
NMR (CDCl₃):δ 7.70 (d, J = 6.9 Hz, 1H), 7.64-7.48 (m, 4H), 7.15 (dd, J = 8.4 Hz, 3.0 Hz, 1H), 7.00 (dt, J = 3.0 Hz, 8.7 Hz, 1H) 6.21 (bs, 1H), 4.69 (d, J = 6.0 Hz, 2H).

### Example 1(90):

### N-(4-fluoro-2-trifluoromethylbenzyl)-2-benzyloxy-3-methoxybenzamide

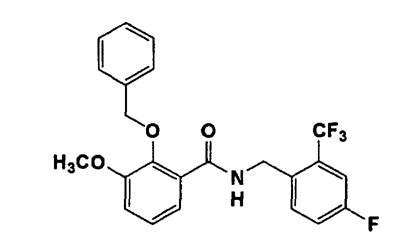

TLC:Rf0.31 (ethyl acetate : n-hexane = 1 : 3);
NMR (CDCl₃):δ 8.38-8.29 (m, 1H), 7.76-7.71 (m, 1H), 7.48 (dd, J = 9.0 Hz, 6.0 Hz, 1H), 7.32-7.07 (m, 9H), 5.03 (s, 2H), 4.58 (d, J = 6.0Hz, 2H), 3.93 (s, 3H).

### Example 1(91):

### N-(2,4-dichlorobenzyl)-2,3-dichlorobenzamide

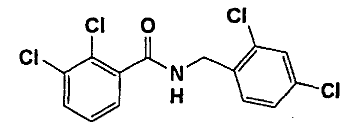

TLC:Rf 0.33 (ethyl acetate : n-hexane = 1 : 3);
NMR (CDCl₃):δ 7.53 (dd, J = 7.8 Hz, 1.5 Hz, 1H), 7.50-7.44 (m, 2H), 7.42 (dd, J = 2.4 Hz, 1H), 7.29-7.23 (m, 2H), 6.50 (bs, 1H), 4.69 (d, J = 6.0 Hz, 2H).

### Example 1(92):

### N-(2,5-dichlorobenzyl)-2,3-dichlorobenzamide

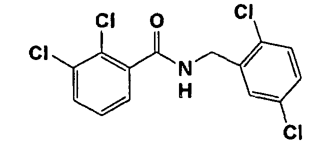

TLC:Rf 0.37 (ethyl acetate : n-hexane = 1 : 3);
NMR (CDCl₃):δ 7.54 (dd, J = 8.1 Hz, 1.5 Hz, 1H), 7.52-7.47 (m, 2H), 7.35-7.22 (m, 3H), 6.51 (bs, 1H), 4.70 (d, J = 6.0 Hz, 2H).

### Example 1(93):

### N-(2-benzyloxybenzyl)-2,3-dichlorobenzamide

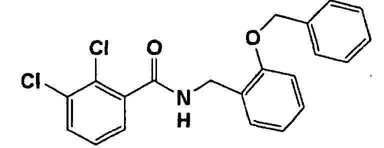

TLC:Rf 0.37 (ethyl acetate : n-hexane = 1 : 3);
NMR (CDCl₃):δ 7.49 (dd, J = 8.1 Hz, 1.5 Hz, 1H), 7.42-7.26 (m, 8H), 7.20 (t, J = 8.1 Hz, 1H), 7.01-6.94 (m, 2H), 6.56-6.46 (m, 1H), 5.11 (s, 2H), 4.69 (d, J = 5.7 Hz, 2H).

### Example 1(94):

### N-(2-benzyloxy-5-chlorobenzyl)-2,3-dichlorobenzamide

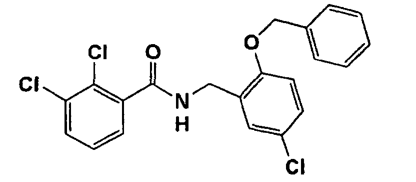

TLC:Rf 0.22 (ethyl acetate : n-hexane = 1 : 3);
NMR (CDCl₃):δ 7.51 (dd, J = 8.1 Hz, 2.7 Hz, 1H), 7.42-7.19 (m, 9H), 6.89 (d, J = 9.0 Hz, 1H), 6.52-6.44 (m, 1H), 5.09 (s, 2H), 4.64 (d, J = 6.0 Hz, 2H).

### Example 1(95):

### N-(2,3-dichlorobenzyl)-2,3-dichlorobenzamide

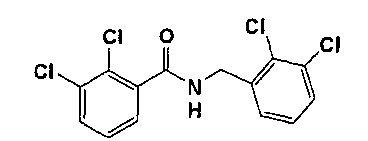

TLC:Rf 0.28 (ethyl acetate : n-hexane = 1 : 3);
NMR (CDCl₃):δ 7.53 (dd, J = 7.8 Hz, 1.5 Hz, 1H), 7.48 (dd, J = 7.8 Hz, 1.5 Hz, 1H), 7.44 (d, J = 8.1 Hz, 1H), 7.30-7.19 (m, 3H), 6.54 (bs, 1H), 4.76 (d, J = 6.3 Hz, 2H).

### Example 1(96):

### N-(2,3-dichlorophenyl)-2,5-dichlorophenylacetamide

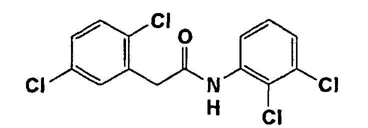

TLC:Rf 0.34 (ethyl acetate : n-hexane = 1 : 5);
NMR (CDCl₃):δ 8.35-8.28 (m, 1H), 7.81 (bs, 1H), 7.44 (d, J = 2.4 Hz, 1H), 7.40 (d, J = 8.4 Hz, 1H), 7.29 (dd, J = 8.4 Hz, 2.4 Hz, 1H), 7.24-7.18 (m, 2H), 3.89 (s, 2H).

### Example 1(97):

### N-(2-benzylbenzyl)-2,3-dichlorobenzamide

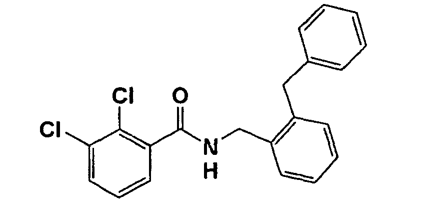

TLC:Rf 0.38 (ethyl acetate : n-hexane = 1 : 3);
NMR (CDCl₃):δ 7.48 (dd, J = 6.6, 3.0 Hz, 1H), 7.41 (dd, J = 6.6, 3.0 Hz, 1H), 7.31-7.12 (m, 10H), 5.71 (brs, 1H), 4.60 (d, J = 5.7 Hz, 2H), 4.10 (s, 2H).

### Example 1(98):

### N-(2,3-dichlorobenzyl)-2,3-dichlorophenylacetamide

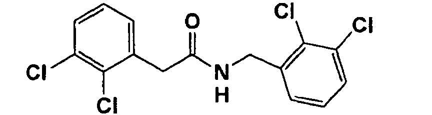

TLC:Rf 0.18 (ethyl acetate : n-hexane = 1 : 3);
NMR (CDCl₃):δ 7.43 (dd, J = 7.8 Hz, 1.5 Hz, 1H), 7.39 (dd, J = 7.8 Hz, 1.5 Hz, 1H), 7.29-7.13 (m, 4H), 6.05-5.90 (b, 1H), 4.52 (d, J = 6.0 Hz, 2H), 3.76 (s, 2H).

### Example 1(99):

### N-(2,3-dichlorophenyl)-2,3-dichlorophenylacetamide

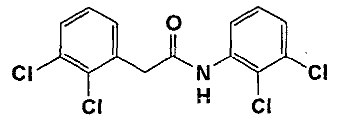

TLC:Rf 0.51 (ethyl acetate : n-hexane = 1 : 3);
NMR (CDCl₃):δ 8.34-8.28 (m, 1H), 7.78 (s, 1H), 7.49 (dd, J = 7.8 Hz, 1.5 Hz, 1H), 7.35 (dd, J = 7.8 Hz, 1.5 Hz, 1H), 7.27 (t, J = 7.8 Hz, 1H), 7.23-7.18 (m, 2H), 3.96 (s, 2H).

### Example 1(100):

### N-(2-phenylbenzyl)-2,3-dichlorobenzamide

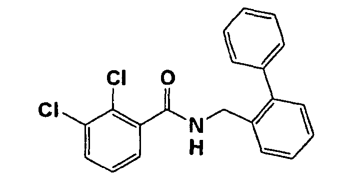

TLC:Rf 0.30 (ethyl acetate : n-hexane = 1 : 3);
NMR (CDCl₃):δ 7.56-7.19 (m, 12H), 6.08-5.98 (m, 1H), 4.64 (d, J = 5.4 Hz, 2H).

### Example 1(101):

### N-(2-phenoxymethylbenzyl)-2,3-dichlorobenzamide

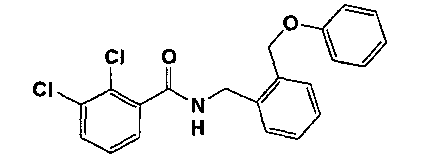

TLC:Rf 0.31 (ethyl acetate : n-hexane = 1 : 3);
NMR (CDCl₃):δ 7.54-7.14 (m, 7H), 7.02-6.95 (m, 1H), 6.94-6.87 (m, 2H), 6.54-6.44 (m, 1H), 5.13 (s, 2H), 4.74 (d, J = 5.4 Hz, 2H).

### Example 1(102):

### N-(2-phenethylbenzyl)-2,3-dichlorobenzamide

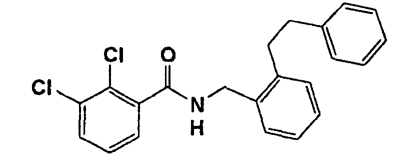

TLC:Rf 0.42 (ethyl acetate : n-hexane = 1 : 3);
NMR (CDCl₃):δ 7.51 (dd, J = 8.1 Hz, 1.5 Hz, 1H), 7.39-7.15 (m, 8H), 7.13-7.08 (m, 2H), 7.03-6.97 (m, 1H), 5.58 (b, 1H), 4.56 (d, J = 5.4 Hz, 2H), 3.05-2.88 (m, 4H).

### Example 1(103):

### N-(2-phenylaminobenzyl)-2,3-dichlorobenzamide

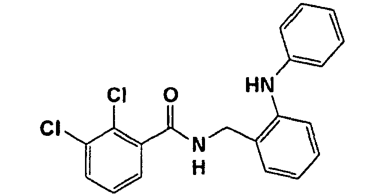

TLC:Rf 0.19 (ethyl acetate : n-hexane = 1 : 4);
NMR (CDCl₃):δ 7.49 (dd, J = 7.8, 1.8 Hz, 1H), 7.39 (d, J = 7.8 Hz, 1H), 7.31-7.16 (m, 5H), 7.11-7 08 (m, 3H), 6.94-6.88 (m, 2H), 6.42 (bs, 1H), 4.65 (d, J = 6.3 Hz, 2H).

### Example 1(104):

### N-(2-benzylaminobenzyl)-2,3-dichlorobenzamide

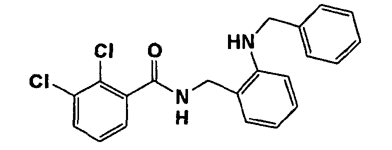

TLC:Rf 0.38 (ethyl acetate : n-hexane = 1 : 3);
NMR (CDCl₃):δ 7.51 (dd, J = 7.8, 1.5 Hz, 1H), 7.37-7.11 (m, 9H), 6.66 (dd, J = 7.8, 1.5 Hz, 1H), 6.61 (d, J = 7.8 Hz, 1H), 6.28-6.18 (br, 1H), 5.37-5.18 (br, 1H), 4.63 (d, J = 6.0 Hz, 1H), 4.42 (s, 2H).

### Example 1(105):

### N-(2-chloro-6-methoxybenzyl)-2,3-dichlorobenzamide

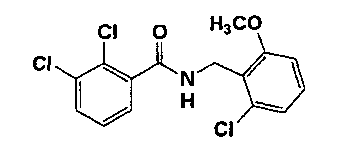

TLC:Rf 0.24 (ethyl acetate : n-hexane = 1 : 3);
NMR (CDCl₃):δ 7.51-7.46 (m, 2H), 7.27-7.19 (m, 2H), 7.03 (d, J = 8.1 Hz, 1H), 6.82 (d, J = 8.1 Hz, 1H), 6.32 (s, 2H), 4.86 (d, J = 5.4 Hz, 2H), 3.88 (s, 3H).

### Example 1(106):

### N-[2-(N-methyl-N-phenylamino)benzyl]-2,3-dichlorobenzamide

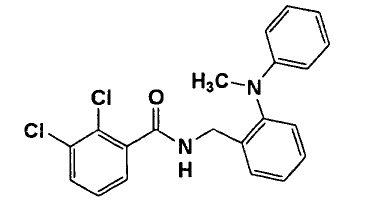

TLC:Rf 0.38 (ethyl acetate : n-hexane = 1 : 3);
NMR (CDCl₃):δ 7.56 (dd, J = 7.5, 1.5 Hz, 1H), 7.47 (dd, J = 7.8, 1.8 Hz, 1H), 7.41-7.28 (m, 2H), 7.24-7.11 (m, 5H), 6.74-6.68 (m, 1H), 6.57-6.53 (m, 2H), 6.24-6.13 (br, 1H), 4.55 (d, J = 6 3 Hz, 2H), 3.28 (s, 3H).

### Example 1(107):

### N-(2-benzyloxy-4-chlorobenzyl)-2,3-dichlorobenzamide

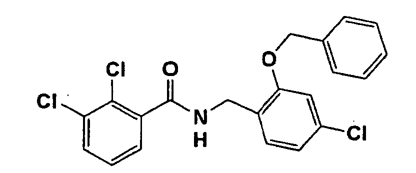

TLC:Rf0.32 (ethyl acetate : n-hexane = 1 : 2);
NMR (CDCl₃):δ 7.52-7.46 (m, 1H), 7.40-7.28 (m, 7H), 7.21 (t, J = 7.8 Hz, 1H), 7.00-6.94 (m, 2H), 6.50-6.40 (m, 1H), 5.08 (s, 2H), 4.63 (d, J = 5.7 Hz, 2H).

### Example 1(108):

### N-(2-benzyloxy-6-chlorobenzyl)-2,3-dichlorobenzamide

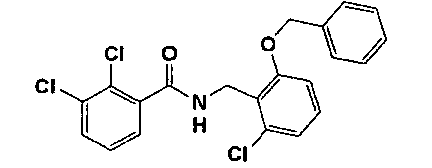

TLC:Rf 0.50 (ethyl acetate : n-hexane = 1 : 2);
NMR (CDCl₃):δ 7.48 (dd, J = 8.1 Hz, 1.5 Hz, 1H), 7.43-7.28 (m, 6H), 7.24-7.16 (m, 2H), 7.05 (dd, J = 8.1 Hz, 1.2 Hz, 1H), 6.89 (d, J = 8.1 Hz, 1H), 6.34-6.24 (m, 1H), 5.13 (s, 2H), 4.91 (d, J = 5.4 Hz, 2H).

### Example 1(109):

### N-(benzocycloheptane-1-yl)-2,3-dichlorobenzamide

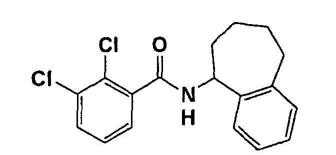

TLC:Rf 0.40 (ethyl acetate : n-hexane = 1 : 4);
NMR (CDCl₃):δ 7.55-7.51 (m, 2H), 7.33-7.22 (m, 2H), 7.20-7.11 (m, 3H), 6.44 (d, J = 6.9 Hz, 1H), 5.43-5.38 (m, 1H), 2.97-2.81 (m, 2H), 2.07-1 92 (m, 4H), 1.84-1.60 (m, 2H).

### Example 1(110):

### N-(2-benzyloxy-5-fluorobenzyl)-2,3-dichlorobenzamide

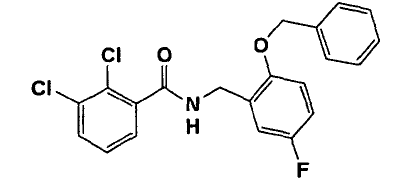

TLC:Rf 0.35 (ethyl acetate : n-hexane = 1 : 3);
NMR (CDCl₃):δ 7.51 (dd, J = 7.5, 1.5 Hz, 1H), 7.41-7.30 (m, 6H), 7.22 (t, J = 7.5 Hz, 1H), 7.13 (dd, J = 8.4, 3.0 Hz, 1H), 7.00-6.86 (m, 2H), 6.56-6.42 (m, 1H), 5.07 (s, 2H), 4.64 (d, J = 5.7 Hz, 2H).

### Example 1(111):

### N-(2-benzyloxy-5-methylbenzyl)-2,3-dichlorobenzamide

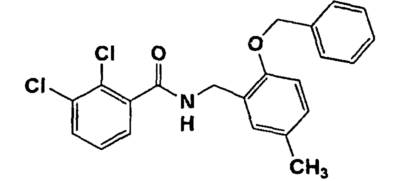

TLC:Rf 0.30 (ethyl acetate : n-hexane = 1 : 3);
NMR (CDCl₃):δ 7.49 (dd, J = 7.8, 1.5 Hz, 1H), 7.40-7.28 (m, 6H), 7.22-7.16 (m, 2H), 7.09-7.06(m, 1H), 6.86 (d, J = 8.1 Hz, 1H), 6.58-6.45 (m, 1H), 5.07 (s, 2H), 4.64 (d, J = 5.7 Hz, 2H), 2.29 (s, 3H).

### Example 1(112):

### N-(2-benzyloxy-5-nitrobenzyl)-2,3-dichlorobenzamide

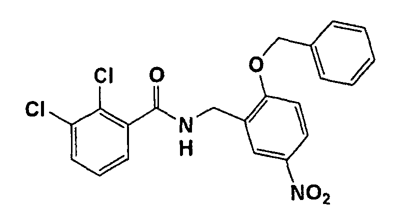

TLC:Rf 0.36 (ethyl acetate : n-hexane = 1 : 2);
NMR (CDCl₃):δ 8.32 (d, J = 2.7 Hz, 1H), 8.20 (dd, J = 9.0, 2.7 Hz, 1H), 7.53 (dd, J = 8 1, 1.5 Hz, 1H), 7.45 (dd, J = 8.1, 1.5 Hz, 1H), 7.42-7.35 (m, 5H), 7.26 (t, J = 8.1 Hz, 1H), 7.03 (d, J = 9.0 Hz, 1H), 6.60-6.00 (m, 1H), 5.22 (s, 2H), 4.73 (d, J = 6.0 Hz, 2H).

### Example 1(113):

### N-(2-benzyloxy-5-methoxybenzyl)-2,3-dichlorobenzamide

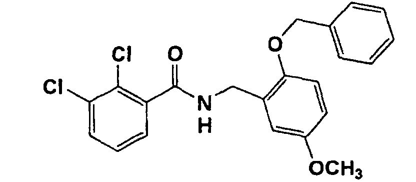

TLC:Rf0.44 (ethyl acetate : n-hexane = 1 : 2);
NMR (CDCl₃):δ 7.49 (dd, J = 8.0, 1.8 Hz, 1H), 7.40-7.28 (m, 6H), 7.20 (t, J = 8.0 Hz, 1H), 6.97 (d, J = 3.0 Hz, 1H), 6.90 (d, J = 8.7 Hz, 1H), 6.79 (dd, J = 8.7, 3.0 Hz, 1H), 6.49-6.41 (m, 1H), 5.05 (s, 2H), 4.65 (d, J = 5.7 Hz, 2H), 3.78 (s, 3H).

### Example 1(114):

### N-(2,3-dichlorophenyl)-2-benzyloxyphenylacetamide

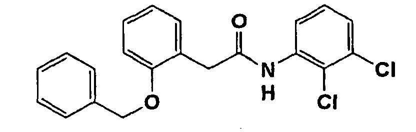

TLC:Rf 0.22 (ethyl acetate : n-hexane = 1 : 9);
NMR (CDCl₃):δ 8.36-8.28 (m, 1H), 8.12 (bs, 1H), 7.39-7.28 (m, 7H), 7.19-7.12 (m, 2H), 7.04-6.98 (m, 2H), 5.16 (s, 2H), 3.82 (s, 2H).

### Example 1(115):

### N-(2,3-dichlorophenyl)-2-benzyloxy-5-chlorophenylacetamide

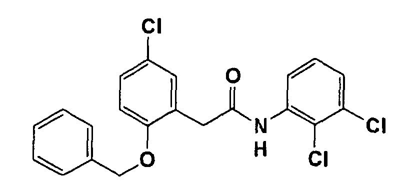

TLC:Rf 0.23 (ethyl acetate : n-hexane = 1 : 9);
NMR (CDCl₃):δ 8.33-8.27 (m, 1H), 8.07 (bs, 1H), 7.37-7.24 (m, 7H), 7.20-7.14 (m, 2H), 6.94 (d, J = 9.0 Hz, 1H), 5.14 (s, 2H), 3.77 (s, 2H)

### Example 1(116):

### N-(5-chloro-2-methylbenzyl)-2,3-dichlorobenzamide

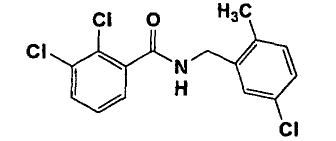

TLC:Rf 0.42 (ethyl acetate : n-hexane = 1 : 2);
NMR (CDCl₃):δ 7.54 (dd, J = 7.8, 1.5 Hz, 1H), 7.49 (dd, J = 7.8, 1.5 Hz, 1H), 7.31 (d, J = 2.1 Hz, 1H), 7.27 (t, J = 7.8 Hz, 1H), 7.19 (dd, J = 8.1, 2.1 Hz, 1H), 7.13 (d, J = 8.1 Hz, 1H), 6 21-6.10 (m, 1H), 4.61 (d, J = 5.7 Hz, 2H), 2.35 (s, 3H).

### Example 1(117):

### N-(2,4-dimethylbenzyl)-2,3-dichlorobenzamide

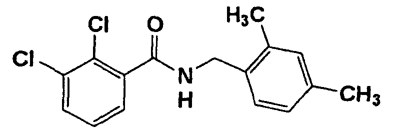

TLC:Rf 0.50 (ethyl acetate : n-hexane = 1 : 2);
NMR (CDCl₃):δ 7.51 (dd, J = 8.1, 1.5 Hz, 1H), 7.47 (dd, J = 8.1, 1.5 Hz, 1H), 7.25 (t, J = 8.1 Hz, 1H), 7.20 (d, J = 7.5 Hz, 1H), 7.05-6.98 (m, 2H), 6.08-5.97 (m, 1H), 4.61 (d, J = 5.4 Hz, 2H), 2.36 (s, 3H), 2.31 (s, 3H).

### Example 1(118):

### N-(2-benzyloxy-5-chlorobenzyl)benzamide

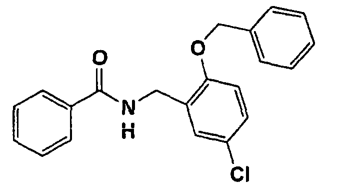

TLC.Rf 0.48 (ethyl acetate : n-hexane = 1 : 2);
NMR (CDCl₃):δ 7.68-7.62 (m, 2H), 7.51-7.33 (m, 9H), 7.21 (dd, J = 9.0, 2.7 Hz, 1H), 6.89 (d, J = 9.0 Hz, 1H), 6.72-6.60 (m, 1H), 5.10 (s, 2H), 4 64 (d, J = 6.0 Hz, 2H).

### Example 1(119):

### N-(2-benzyloxy-5-chlorobenzyl)-2-chlorobenzamide

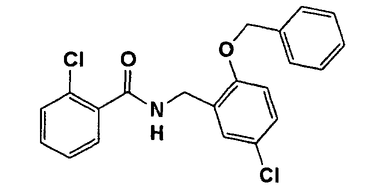

TLC:Rf0.43 (ethyl acetate : n-hexane = 1 : 2);
NMR (CDCl₃):δ 7.63-7.58 (m, 1H), 7.43-7.24 (m, 9H), 7.20 (dd, J = 9.0, 2.7 Hz, 1H), 6.87 (d, J = 9.0 Hz, 1H), 6.80-6.69 (m, 1H), 5.08 (s, 2H), 4.64 (d, J = 6 0 Hz, 2H).

### Example 1(120):

### N-(2-benzyloxy-5-chlorobenzyl)-2-methylbenzamide

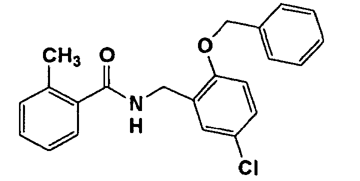

TLC:Rf 0.50 (ethyl acetate : n-hexane = 1 : 2);
NMR (CDCl₃):δ 7.39-7.12 (m, 11H), 6.88 (d, J = 8.7 Hz, 1H), 6.24-6.17 (m, 1H), 5.08 (s, 2H), 4.62 (d, J = 6.0 Hz, 2H), 2.38 (s, 3H).

### Example 1(121):

### N-(2-benzyloxy-5-chlorobenzyl)-3-methylbenzamide

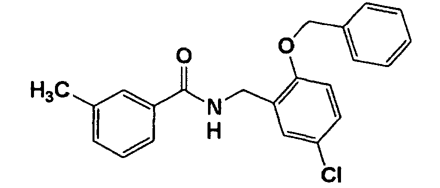

TLC:Rf 0.50 (ethyl acetate : n-hexane = 1 : 2);
NMR (CDCl₃):δ 7.54-7.51 (m, 1H), 7.45-7.22 (m, 9H), 7.20 (dd, J = 8.7, 2.7 Hz, 1H), 6.87 (d, J = 8.7 Hz, 1H), 6.70-6.61 (m, 1H), 5.09 (s, 2H), 4.62 (d, J = 6.0 Hz, 2H), 2.35 (s, 3H).

### Example 1(122):

### N-(2-benzyloxy-5-chlorobenzyl)-2,3-dimethylbenzamide

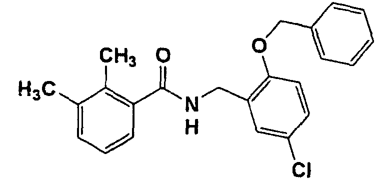

TLC:Rf 0.35 (ethyl acetate:toluene=1 : 19);
NMR (CDCl₃):δ 7.44-7.26 (m, 6H), 7.24-7.16 (m, 2H), 7.14-7.02 (m, 2H), 6.88 (d, J = 8.4 Hz, 1H), 6.20-6.10 (m, 1H), 5.08 (s, 2H), 4.62 (d, J = 6.3 Hz, 2H), 2.27 (s, 3H), 2.24 (s, 3H).

### Example 1(123):

### N-(2-nitrobenzyl)-2,3-dichlorobenzamide

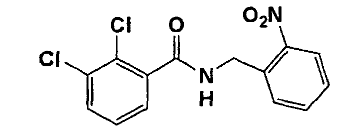

TLC:Rf 0.35 (ethyl acetate : n-hexane = 1 : 2);
NMR (CDCl₃):δ 9.14 (t, J = 5.7 Hz, 1H), 8.05 (dd, J = 8.1, 1.5 Hz, 1H), 7.79-7.67 (m, 3H), 7.58-7.53 (m, 1H), 7.46-7.40 (m, 2H), 4.73 (d, J = 5.7 Hz, 2H).

### Example 1(124):

### N-(2,5-dimethylbenzyl)-2,3-dichlorobenzamide

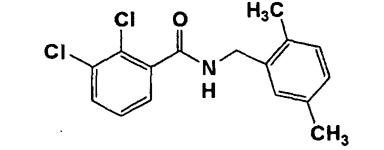

TLC:Rf 0.49 (ethyl acetate : n-hexane = 1 : 2);
NMR (CDCl₃):δ 7.52 (dd, J = 8.1, 1.5 Hz, 1H), 7.48 (dd, J = 8.1, 1.5 Hz, 1H), 7.25 (t, J = 8 1 Hz, 1H), 7.13 (d, J = 1.5 Hz, 1H), 7.10 (d, J = 7.8 Hz, 1H), 7.03 (dd, J = 7.8, 1.5 Hz, 1H), 6.09-5.98 (m, 1H), 4.61 (d, J = 5.4 Hz, 1H), 2.35 (s, 3H), 2.31 (s, 3H).

### Example 1(125):

### N-(2-benzyloxy-5-chlorobenzyl)-3-chlorobenzamide

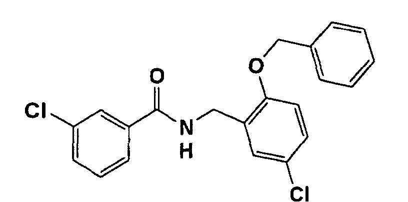

TLC:Rf 0.42 (ethyl acetate : n-hexane = 1 : 2);
NMR (CDCl₃):δ 7 66 (t, J = 2.1 Hz, 1H), 7.52-7.28 (m, 9H), 7.22 (dd, J = 8.7, 2.7 Hz, 1H), 6 90 (d, J = 8.7 Hz, 1H), 6.68-6.57 (m, 1H), 5.10 (s, 2H), 4.61 (d, J = 6.0 Hz, 2H).

### Example 1(126):

### N-(2-benzyloxy-5-chlorobenzyl)-2-chloro-4-methoxymethoxybenzamide

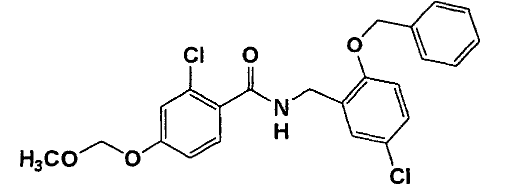

TLC:Rf 0.47 (ethyl acetate : n-hexane = 1 : 2);
NMR (CDCl₃):δ 7.70 (d, J = 9.0 Hz, 1H), 7.42-7.29 (m, 6H), 7.20 (dd, J = 8.7 Hz, 2.4 Hz, 1H), 7.04 (d, J = 2.4 Hz, 1H), 6.96 (dd, J = 9.0 Hz, 2.4 Hz, 1H), 6.92-6.84 (m, 2H), 5.17 (s, 2H), 5.09 (s, 2H), 4.65 (d, J = 5.7 Hz, 2H), 3.46 (s, 3H).

### Example 1(127):

### N-(4-benzyloxy-2-chlorobenzyl)-2,3-dichlorobenzamide

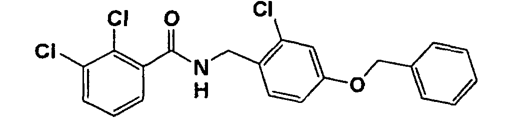

TLC:Rf 0.48 (ethyl acetate : n-hexane = 1 : 2);
NMR (CDCl₃):δ 7.51 (dd, J = 7.8 Hz, 1.8 Hz, 1H), 7.49 (dd, J = 7.8 Hz, 1.8 Hz, 1H), 7.44-7.30 (m, 6H), 7.24 (t, J = 7.8 Hz, 1H), 7.03 (d, J = 2.4 Hz, 1H), 6.87 (dd, J = 8.4 Hz, 2.4 Hz, 1H), 6.44-6.36 (m, 1H), 5.05 (s, 2H), 4.66 (d, J = 6.0 Hz, 2H).

### Example 2:

### 1-(2-chlorobenzyl)-5-(2-methoxyphenyl)-1H-1,2,3,4-tetrazole

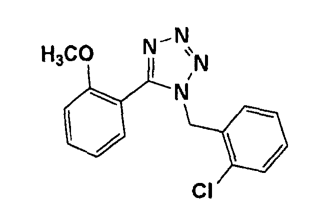

To a solution of the compound prepared in Example 1(413 mg) in toluene (3.0 ml) was added phosphorus pentachloride (344 mg) and the mixture was refluxed for 3 hours. The mixture was concentrated and the residue was dissolved in acetonitrile (3.0 ml). To the mixture was added trimethylsilyl azide (0.22 ml) and the mixture was stirred overnight. To the reaction mixture was added water, and the mixture was extracted with ethyl acetate. The organic layer was washed with 1N hydrochloric acid, a saturated aqueous solution of sodium bicarbonate, and brine successively, dried over anhydrous sodium sulfate and was concentrated. The residue was purified by column chromatography on silica gel (hexane : ethyl acetate = 7 : 3) to give the compound of the present invention (238 mg) having the following physical data.
TLC:Rf 0.37 (ethyl acetate : n-hexane = 1 : 2);
NMR (CDCl₃):δ 7.58-7.40 (m, 2H), 7.36-7.14 (m, 3H), 7.11-6.96 (m, 3H), 5.56 (s, 2H), 3.64 (s, 3H).

### Example 2(1)-Example 2(112)

By the same procedure as described in example 2 using a corresponding compound, optionally followed by converting into a corresponding salt by a conventional method, the compounds of the present invention having the following physical data were given.

### Example 2(1):

### 1-(3-methoxybenzyl)-5-phenyl-1H-1,2,3,4-tetrazole

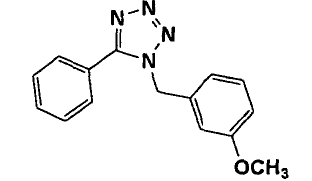

TLC:Rf0.48 (ethyl acetate:toluene=1 : 4);
NMR (CDCl₃):δ 7.64-7.45 (m, 5H), 7.32-7.21 (m, 1H), 6.91-6.84 (m, 1H), 6.76-6.67 (m, 2H), 5.59 (s, 2H), 3.76 (s, 3H).

### Example 2(2):

### 1-(2,6-dichlorobenzyl)-5-phenyl-1H-1,2,3,4-tetrazole

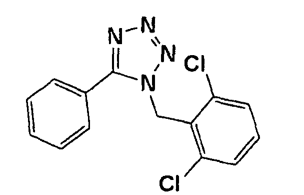

TLC:Rf 0.34 (ethyl acetate : n-hexane = 1 : 2);
NMR (CDCl₃):δ 7.76-7.68 (m, 2H), 7.63-7.52 (m, 3H), 7.38-7.22 (m, 3H), 5.80 (s, 2H).

### Example 2(3)

### 1-benzyl-5-phenyl-1H-1,2,3,4-tetrazole

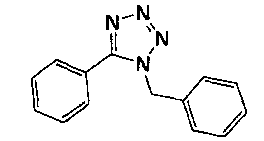

TLC.Rf 0.31 (ethyl acetate : n-hexane = 1 : 2);
NMR (CDCl₃):δ 7.63-7.45 (m, 5H), 7.40-7.31 (m, 3H), 7.19-7.13 (m, 2H), 5.62 (s, 2H).

### Example 2(4):

### 1-benzyl-5-(2-trifluoromethylphenyl)-1H-1,2,3,4-tetrazole

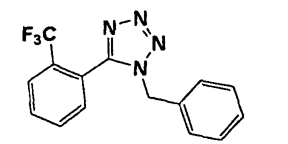

TLC:Rf 0.29 (ethyl acetate : n-hexane = 1 : 2);
NMR (CDCl₃):δ 7.91-7.84 (m, 1H), 7.76-7.66 (m, 1H), 7.64-7.53 (m, 1H), 7.31-7.20 (m, 3H), 7.12-7.06 (m, 1H), 7.06-6.98 (m, 2H), 5.37 (s, 2H).

### Example 2(5):

### 1-benzyl-5-(2-chlorophenyl)-1H-1,2,3,4-tetrazole

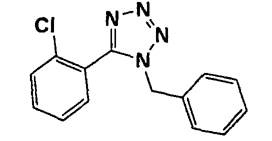

TLC:Rf 0.34 (ethyl acetate : n-hexane = 1 : 2);
NMR (CDCl₃):δ 7.60-7.46 (m, 2H), 7.38-7.15 (m, 5H), 7.06-6.96 (m, 2H), 5.47(s, 2H).

### Example 2(6):

### 5-(2-chlorophenyl)-1-(2,6-dichlorobenzyl)-1H-1,2,3,4-tetrazole

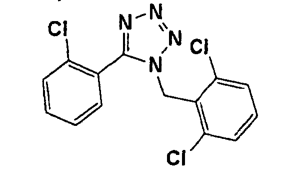

TLC:Rf 0.36 (ethyl acetate : n-hexane = 1 : 2);
NMR (CDCl₃):δ 7.61-7.19 (m, 7H), 5.67 (s, 2H).

### Example 2(7):

### 5-(2-chlorophenyl)-1-(pyridin-2-ylmethyl)-1H-1,2,3,4-tetrazole hydrochloride

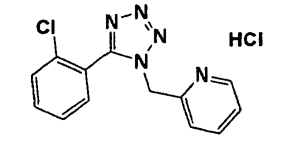

TLC:Rf0.43 (n-hexane : ethyl acetate = 1 : 4);
NMR (CD₃OD):δ 8.78 (bd, J = 6 Hz, 1H), 8.41 (td, J = 8, 2 Hz, 1H), 7.95-7 86 (m, 1H), 7.74 (d, J = 8 Hz, 1H), 7.69-7.48 (m, 4H), 5.95 (s, 2H).

### Example 2(8):

### 5-(2-chlorophenyl)-1-(pyridin-3-ylmethyl)-1H-1,2,3,4-tetrazole hydrochloride

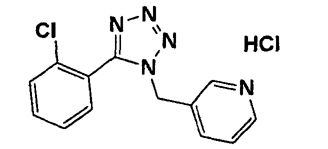

TLC:Rf 0.19 (n-hexane : ethyl acetate = 1 : 4);
NMR (CD₃OD):δ 8.90-8.81 (m, 2H), 8.48 (d, J = 8 Hz, 1H), 8.07 (dd, J = 8, 6 Hz, 1H), 7.76-7.64 (m, 2H), 7.64-7.52 (m, 2H), 5.85 (s, 2H).

### Example 2(9):

### 5-(2-chlorophenyl)-1-(pyridin-4-ylmethyl)-1H-1,2,3,4-tetrazole hydrochloride

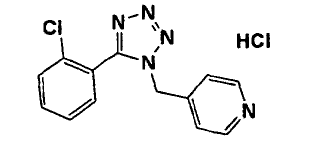

TLC:Rf 0.20 (n-hexane : ethyl acetate = 1 : 4);
NMR (CD₃OD):δ 8.88-8.81 (m, 2H), 7.90-7.85 (m, 2H), 7.70-7.49 (m, 4H), 5.97 (s, 2H).

### Example 2(10):

### 1-(4-methylbenzyl)-5-(2-trifluoromethylphenyl)-1H-1,2,3,4-tetrazole

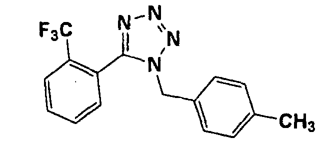

TLC:Rf 0.29 (ethyl acetate : n-hexane = 1 : 2);
NMR (CDCl₃):δ 7.85 (bd, J = 7 Hz, 1H), 7.72 (bt, J = 7 Hz, 1H), 7.59 (bt, J = 7 Hz, 1H), 7.14-7.03 (m, 3H), 6.94-6.87 (m, 2H), 5.32 (s, 2H), 2.31 (s, 3H).

### Example 2(11):

### 1-(2,6-dichlorobenzyl)-5-(2-pyridyl)-1H-1,2,3,4-tetrazole

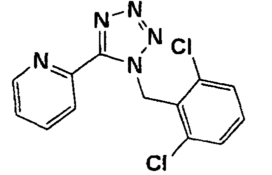

TLC:Rf 0.26 (n-hexane : ethyl acetate = 2 : 1);
NMR (CDCl₃):δ 8.81-8.76 (m, 1H), 8.25-8.19 (m, 1H), 7.84 (td, J = 8.0, 2.0 Hz, 1H), 7.45-7.25 (m, 4H), 6.17 (s, 2H).

### Example 2(12):

### 1-(2-chlorobenzyl)-5-phenyl-1H-1,2,3,4-tetrazole

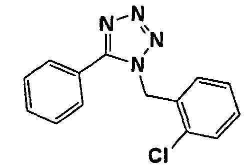

TLC:Rf0.44 (ethyl acetate:toluene=1 : 2);
NMR (CDCl₃):δ 7.64-7.41 (m, 6H), 7.37-7.20 (m, 2H), 6.94-6.88 (m, 1H), 5.74 (s, 2H).

### Example 2(13):

### 1-(2-chlorobenzyl)-5-(3-pyridyl)-1H-1,2,3,4-tetrazole hydrochloride

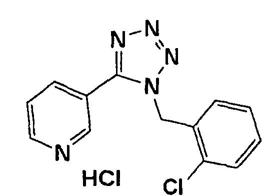

TLC:Rf 0.36 (n-hexane : ethyl acetate = 1 : 3);
NMR (CD₃OD):δ 9.27-9.20 (m, 1H), 9.08-9.00 (m, 1H), 8.86-8.73 (m, 1H), 8.23-8.09 (m, 1H), 7.46-7.24 (m, 4H), 5.94 (s, 2H).

### Example 2(14):

### 1-(2-chloro-6-fluorobenzyl)-5-(2-trifluoromethylphenyl)-1H-1,2,3,4-tetrazole

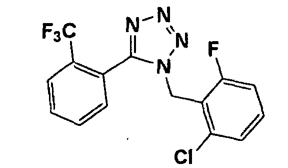

TLC:Rf 0.34 (ethyl acetate : n-hexane = 1 : 2);
NMR (CDCl₃):δ 7.86 (d, J = 7.8 Hz, 1H), 7.78-7.69 (m, 1H), 7.68-7.60 (m, 1H), 7.34-7.24 (m, 2H), 7.19 (d, J = 8.1 Hz, 1H), 6.97-6.89 (m, 1H), 5.52 (d, J = 1.2 Hz, 2H).

### Example 2(15):

### 1-(2-chlorobenzyl)-5-(3-methoxyphenyl)-1H-1,2,3,4-tetrazole

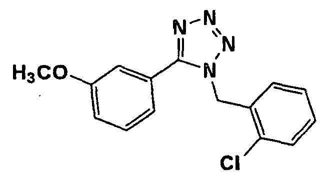

TLC:Rf 0.46 (ethyl acetate:toluene=1 : 9);
NMR (CDCl₃):δ 7.46-7.42 (m, 1H), 7.41-7.36 (m, 1H), 7.32 (td, J = 7.5, 2.1 Hz, 1H), 7.26 (td, J = 7.5, 1.5 Hz, 1H), 7.14-7.06 (m, 3H), 6.90 (dd, J = 7.5, 2.1 Hz, 1H), 5.75 (s, 2H), 3.77 (s, 3H).

### Example 2(16):

### 1-(2-chlorobenzyl)-5-(4-methoxyphenyl)-1H-1,2,3,4-tetrazole

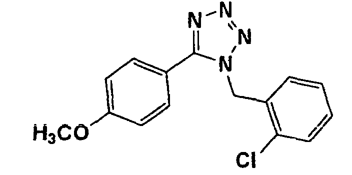

TLC:Rf0.43 (ethyl acetate:toluene=1 : 9);
NMR (CDCl₃):δ 7.57-7.51 (m, 2H), 7.45 (dd, J = 7.8, 1.5 Hz, 1H), 7.32 (td, J = 7.8, 1.5 Hz, 1H), 7.25 (td, J = 7 8, 1.5 Hz, 1H), 7.03-6.97 (m, 2H), 6.89 (dd, J = 7.8, 1 5 Hz, 1H), 5.73 (s, 2H), 3.86 (s, 3H).

### Example 2(17):

### 1-(2-methoxybenzyl)-5-phenyl-1H-1,2,3,4-tetrazole

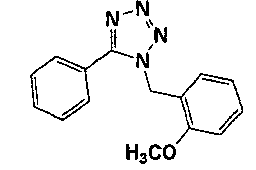

TLC:Rf0.35 (ethyl acetate : n-hexane = 1 : 2);
NMR (CDCl₃):δ 7.70-7.62 (m, 2H), 7.62-7.44 (m, 3H), 7.36-7.26 (m, 1H), 7.02-6.84 (m, 3H), 5.63 (s, 2H), 3.71 (s, 3H).

### Example 2(18):

### 1-(4-methoxybenzyl)-5-phenyl-1H-1,2,3,4-tetrazole

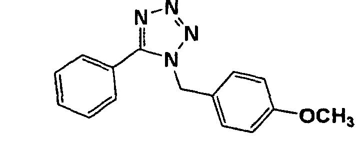

TLC:Rf 0.22 (ethyl acetate : n-hexane = 1 : 3);
NMR (CDCl₃):δ 7.64-7.46 (m, 5H), 7.14-7.06 (m, 2H), 6.91-6.82 (m, 2H), 5.55 (s, 2H), 3.79 (s, 3H).

### Example 2(19):

### 1-(2-chlorobenzyl)-5-(4-dimethylaminophenyl)-1H-1,2,3,4-tetrazole

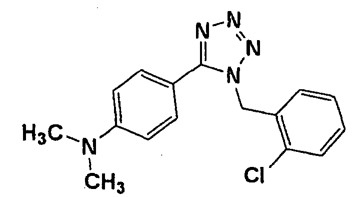

TLC:Rf 0.41 (ethyl acetate:toluene=1 : 4);
NMR (CDCl₃):δ 7.54-7.43 (m, 3H), 7.36-7.18 (m, 2H), 6.87-6.80 (m, 1H), 6.76-6.66 (m, 2H), 5.74 (s, 2H), 3.02 (s, 6H).

### Example 2(20):

### 1-(2-chlorobenzyl)-5-(3-dimethylaminophenyl)-1H-1,2,3,4-tetrazole

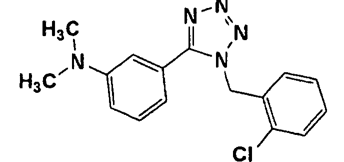

TLC:Rf 0.51 (ethyl acetate:toluene=1 : 4);
NMR (CDCl₃):δ 7.48-7.41 (m, 1H), 7.37-7.20 (m, 3H), 6.90-6.80 (m, 4H), 5.75 (s, 2H), 2.89 (s, 6H).

### Example 2(21):

### 1-(2-chlorobenzyl)-5-(2-dimethylaminophenyl)-1H-1,2,3,4-tetrazole

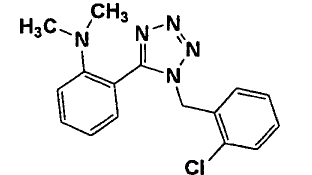

TLC:Rf 0.32 (ethyl acetate:toluene=1 : 10);
NMR (CDCl₃):δ 7.51-6.89 (m, 8H), 5.57 (s, 2H), 2.47 (s, 6H).

### Example 2(22):

### 5-(2-chlorophenyl)-1-(2-methylbenzyl)-1H-1,2,3,4-tetrazole

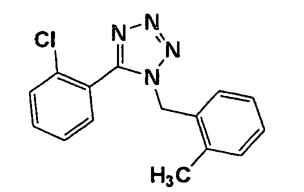

TLC.Rf 0.32 (ethyl acetate : n-hexane = 3 : 7);
NMR (CDCl₃):δ 7.56-7.45 (m, 2H), 7.35-7.28 (m, 1H), 7.20-6.96 (m, 4H), 6.76 (d, J = 7.4 Hz, 1H), 5.51 (s, 2H), 2.14 (s, 3H).

### Example 2(23):

### 5-(2-chlorophenyl)-1-(2,6-difluorobenzyl)-1H-1,2,3,4-tetrazole

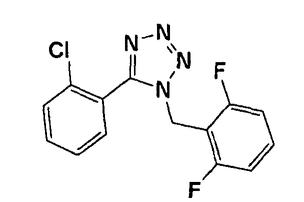

TLC:Rf 0.23 (ethyl acetate : n-hexane = 3 : 7);
NMR (CDCl₃):δ 7.57-7.30 (m, 5H), 6.88-6.80 (m, 2H), 5.53 (s, 2H).

### Example 2(24):

### 1-(3-dimethylaminobenzyl)-5-phenyl-1H-1,2,3,4-tetrazole

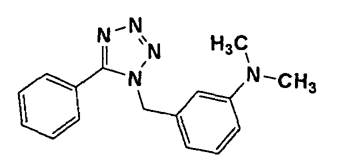

TLC:Rf 0.36 (ethyl acetate : n-hexane = 1 : 2);
NMR (CDCl₃):δ 7.65-7.60 (m, 2H), 7.60-7.46 (m, 3H), 7.19 (t, J = 8.4 Hz, 1H), 6.70-6.64 (m, 1H), 6.49-6.44 (m, 2H), 5.56 (s, 2H), 2.81 (s, 6H).

### Example 2(25):

### 1-(2-chloro-6-fluorobenzyl)-5-(2-methylphenyl)-1H-1,2,3,4-tetrazole

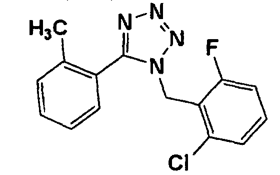

TLC:Rf 0.38 (ethyl acetate : n-hexane = 3 : 7);
NMR (CDCl₃):δ 7.48-7.42 (m, 1H), 7.35-7.24 (m, 4H), 7.17 (d, J = 8.4 Hz, 1H), 6.95 (td, J = 9.0, 1.2 Hz, 1H), 5.54 (d, J = 1.2 Hz, 2H), 2.18 (s, 3H).

### Example 2(26):

### 1-(2-chloro-6-fluorobenzyl)-5-(2-chlorophenyl)-1H-1,2,3,4-tetrazole

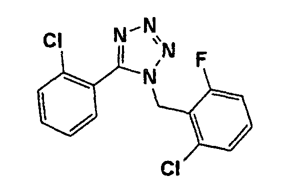

TLC:Rf 0.25 (ethyl acetate : n-hexane = 1 : 4);
NMR (CDCl₃):δ 7.58-7.50 (m, 2H), 7.41-7.39 (m, 2H), 7.31-7.24 (m, 1H), 7.16(d, J = 8.1 Hz, 1H), 6.96 (td, J = 8.7, 1.5 Hz, 1H), 5.59 (d, J = 1.5 Hz, 2H).

### Example 2(27):

### 1-(2-chloro-6-fluorobenzyl)-5-(2-methoxyphenyl)-1H-1,2,3,4-tetrazole

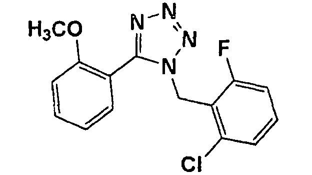

TLC:Rf 0.27 (ethyl acetate : n-hexane = 3 : 7);
NMR (CDCl₃):δ 7.56-7.50 (m, 1H), 7.43 (dd, J = 7.5, 1.8 Hz, 1H), 7.28-6.92 (m, 5H), 5.55 (d, J = 1.2 Hz, 2H), 3.86 (s, 3H).

### Example 2(28):

### 1-(2-chloro-6-fluorobenzyl)-5-(2-furyl)-1H-1,2,3,4-tetrazole

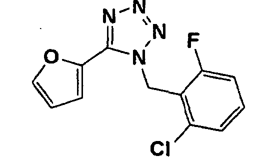

TLC:Rf 0.27 (n-hexane : ethyl acetate = 7: 3);
NMR (CDCl₃):δ 7.72 (dd, J = 2.0, 0.8 Hz, 1H), 7.35-7.26 (m, 3H), 7.10-7.04 (m, 1H), 6.67 (dd, J = 3.6, 2.0 Hz, 1H), 6.00 (d, J = 1.5 Hz, 2H).

### Example 2(29):

### 5-(2-chlorophenyl)-1-(3,4-dichlorobenzyl)-1H-1,2,3,4-tetrazole

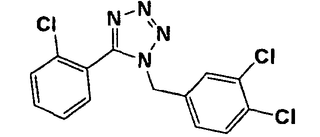

TLC:Rf 0.34 (ethyl acetate : n-hexane = 3 : 7);
NMR (CDCl₃):δ 7.59-7.22 (m, 5H), 7.09 (d, J = 2.2 Hz, 1H), 6.90 (dd, J = 8.2, 2.2 Hz, 1H), 5.42 (s, 2H).

### Example 2(30):

### 5-(2-chlorophenyl)-1-(4-trifluoromethylbenzyl)-1H-1,2,3,4-tetrazole

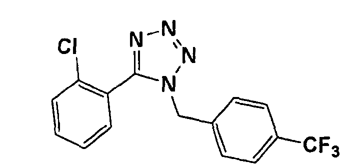

TLC:Rf 0.35 (ethyl acetate : n-hexane = 3 : 7);
NMR (CDCl₃):δ 7.57-7.50 (m, 4H), 7.41-7.33 (m, 1H), 7.27-7.21 (m, 1H), 7.16 (d, J = 8.0 Hz, 2H), 5.53 (s, 2H).

### Example 2(31):

### 1-(2-chloro-6-fluorobenzyl)-5-[2-(4-methoxyphenyloxymethyl)phenyl]-1H-1,2,3,4-tetrazole

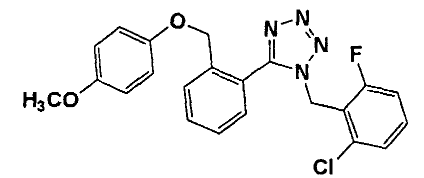

TLC:Rf 0.38 (ethyl acetate : n-hexane = 3 : 7);
NMR (CDCl₃):δ 7.71 (d, J = 7.8 Hz, 1H), 7.61 (td, J = 7.8, 1.5 Hz, 1H), 7.47 (td, J = 7.8, 1.5 Hz, 1H), 7.36-7.25 (m, 2H), 7.18 (d, J = 7.8 Hz, 1H), 6.97 (t, J = 9.0, 1.5 Hz, 1H), 6.81-6.72 (m, 4H), 5.55 (d, J = 1.5 Hz, 2H), 4.91 (s, 2H), 3.75 (s, 3H).

### Example 2(32):

### 5-(2-benzyloxyphenyl)-1-(2-chloro-6-fluorobenzyl)-1H-1,2,3,4-tetrazole

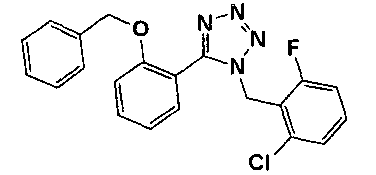

TLC:Rf 0.37 (ethyl acetate : n-hexane = 3 : 7);
NMR (CDCl₃):δ 7.56-7.46 (m, 2H), 7.37-7.07 (m, 9H), 6.92 (td, J = 9.0, 1.2 Hz, 1H), 5.47 (d, J = 1.2 Hz, 2H), 5.12 (s, 2H).

### Example 2(33):

### 1-(2-chloro-6-fluorobenzyl)-5-(2,3-dichlorophenyl)-1H-1,2,3,4-tetrazole

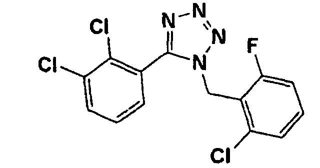

TLC:Rf0.43 (ethyl acetate : n-hexane = 3 : 7);
NMR (CDCl₃):δ 7.70-7.67 (m, 1H), 7.37-7.24 (m, 3H), 7.17 (d, J = 8.1 Hz, 1H), 6.96 (td, J = 8.7, 1.2 Hz, 1H), 5.60 (d, J = 1.5 Hz, 2H).

### Example 2(34):

### 1-(4-fluorobenzyl)-5-(4-fluorophenyl)-1H-1,2,3,4-tetrazole

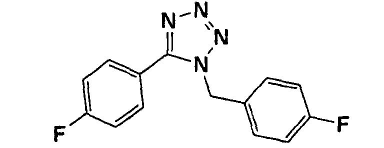

TLC:Rf0.40 (ethyl acetate : n-hexane = 3 : 7);
NMR (CDCl₃):δ 7.60-7.55 (m, 2H), 7.27-7.02 (m, 6H), 5.58 (s, 2H).

### Example 2(35):

### 5-(2,6-dichlorophenyl)-1-(4-fluorobenzyl)-1H-1,2,3,4-tetrazole

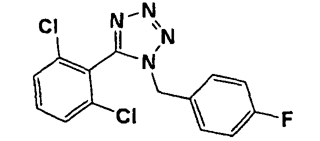

TLC:Rf 0.43 (ethyl acetate : n-hexane = 3 : 7);
NMR (CDCl₃):δ 7.50-7.40 (m, 3H), 7.07-7.03 (m, 2H), 6.96-6.90 (m, 2H), 5.40 (s, 2H).

### Example 2(36):

### 5-(2,5-dichlorophenyl)-1-(4-fluorobenzyl)-1H-1,2,3,4-tetrazole

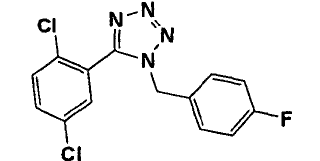

TLC.Rf 0.24 (ethyl acetate : n-hexane = 1 : 4);
NMR (CDCl₃):δ 7.50 (m, 2H), 7.17-6.94 (m, 5H), 5.45 (s, 2H).

### Example 2(37):

### 5-(2-chloro-4-fluorophenyl)-1-(4-fluorobenzyl)-1H-1,2,3,4-tetrazole

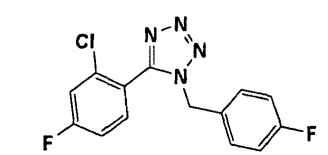

TLC:Rf 0.21 (ethyl acetate : n-hexane = 1 : 4);
NMR (CDCl₃):δ 7.33 (dd, J = 8.1, 2.7 Hz, 1H), 7.19 (dd, J = 9.0, 5.7 Hz, 1H), 7.12-6.92 (m, 5H), 5.44 (s, 2H).

### Example 2(38):

### 5-(2,3-dichlorophenyl)-1-cyclohexylmethyl-1H-1,2,3,4-tetrazole

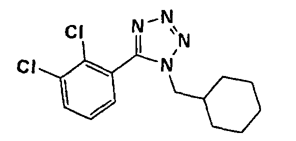

TLC:Rf 0.29 (n-hexane : ethyl acetate = 8 : 2);
NMR (CDCl₃):δ 7.72 (dd, J = 8.1, 1.5 Hz, 1H), 7.42 (t, J = 8.1 Hz, 1H), 7.34 (dd, J = 8.1, 1.5 Hz, 1H), 4.04 (d, J = 7.5 Hz, 2H), 1.92 (m, 1H), 1.70-1.06 (m, 8H), 0.88-0.80 (m, 2H).

### Example 2(39):

### 5-(2,3-dichlorophenyl)-1-(4-fluorobenzyl)-1H-1,2,3,4-tetrazole

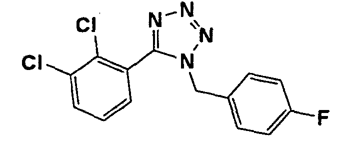

TLC:Rf 0.23 (ethyl acetate : n-hexane = 3 : 7);
NMR (CDCl₃):δ 7.71-7.67 (m, 1H), 7.31 (t, J = 8.1 Hz, 1H), 7.11-6.91 (m, 5H), 5.44 (s, 2H).

### Example 2(40):

### 1-(2-chloro-6-fluorobenzyl)-5-cyclohexyl-1H-1,2,3,4-tetrazole

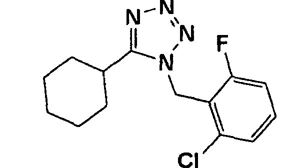

TLC:Rf 0.39 (n-hexane : ethyl acetate = 7 : 3);
NMR (CDCl₃):δ 7.40-7.25 (m, 2H), 7.09 (td, J = 8.7, 1.2 Hz, 1H), 5.59 (d, J = 1.2 Hz, 2H), 2.95-2.87 (m, 1H), 1.91-1.34 (m, 10H).

### Example 2(41):

### 1-(2-chloro-6-fluorobenzyl)-5-(1-naphthyl)-1H-1,2,3,4-tetrazole

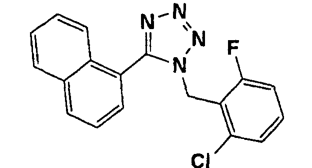

TLC:Rf 0.21 (ethyl acetate : n-hexane = 1 : 4);
NMR (CDCl₃):δ 8.07-8.04 (m, 1H), 7.93 (d, J = 7.8 Hz, 1H), 7.61-7.44 (m, 5H), 7.15 (td, J = 8.1, 6.0 Hz, 1H), 7.03 (d, J = 7.8 Hz, 1H), 6.83 (t, J = 8.9 Hz, 1H), 5.54 (s, 2H).

### Example 2(42):

### 1-benzyl-5-(2,5-dichlorophenyl)-1H-1,2,3,4-tetrazole

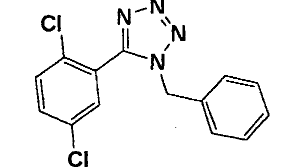

TLC:Rf0.45 (ethyl acetate : n-hexane = 3 : 7);
NMR(CDCl₃):δ 7.47 (m, 2H), 7.37-7.24 (m, 3H), 7.12 (m, 1H), 7.03-7.00 (m, 2H), 5.49 (s, 2H).

### Example 2(43):

### 1-(2-chloro-6-fluorobenzyl)-5-(2,5-dichlorophenyl)-1H-1,2,3,4-tetrazole

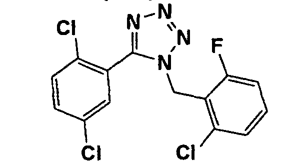

TLC:Rf 0.39 (ethyl acetate : n-hexane = 1 : 4);
NMR (CDCl₃):δ 7.49-7.25 (m, 4H), 7.18 (d, J = 8.7 Hz, 1H), 6.96 (td, J = 8.7, 0.9 Hz, 1H), 5.63 (d, J = 1.2 Hz, 2H).

### Example 2(44):

### 1-(2-chloro-6-phenoxybenzyl)-5-(2,3-dichlorophenyl)-1H-1,2,3,4-tetrazole

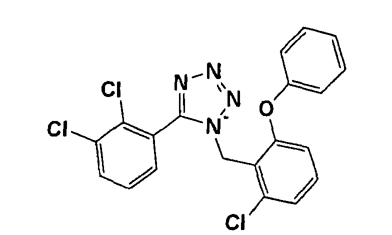

TLC:Rf 0.31 (ethyl acetate : n-hexane = 1 : 4);
NMR (CDCl₃):δ 7.64 (dd, J = 8.1, 1.5 Hz, 1H), 7.37-7.08 (m, 7H), 6.84-6.81 (m, 2H), 6.62 (dd, J = 8.1, 1.2 Hz, 1H), 5.67 (s, 2H).

### Example 2(45):

### 1-(2-chloro-6-fluorobenzyl)-5-(2-naphthyl)-1H-1,2,3,4-tetrazole

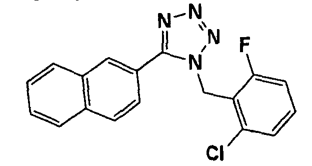

TLC:Rf 0.24 (ethyl acetate : n-hexane = 1 : 4);
NMR (CDCl₃):δ 8.21 (s, 1H), 8.03 (d, J = 8.4 Hz, 1H), 7.97-7.94 (m, 2H), 7.77 (dd, J = 8.4, 1.5 Hz, 1H), 7.67-7.61 (m, 2H), 7.33-7.25 (m, 1H), 7.19 (d, J = 8.1 Hz, 1H), 7.00 (t, J = 8.4 Hz, 1H), 5.79 (d, J = 1.2 Hz, 2H).

### Example 2(46):

### 5-(2,4-dichlorophenyl)-1-(4-fluorobenzyl)-1H-1,2,3,4-tetrazole

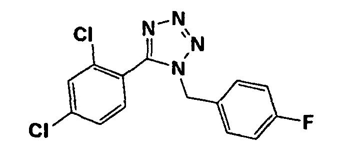

TLC:Rf 0.38 (ethyl acetate:toluene=1 : 9);
NMR (CDCl₃):δ 7.59 (d, J = 1.8 Hz, 1H), 7.36 (dd, J = 8.4 Hz, 1.8 Hz, 1H), 7.14 (d, J = 8.4 Hz, 1H), 7.06-6.90 (m, 4H), 5.44 (s, 2H).

### Example 2(47):

### 5-(2-chloro-4-nitrophenyl)-1-(4-fluorobenzyl)-1H-1,2,3,4-tetrazole

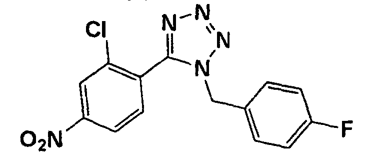

TLC:Rf 0.38 (ethyl acetate : n-hexane = 1 : 2);
NMR (CDCl₃):δ 8.43 (d, J = 2.1 Hz, 1H), 8.21 (dd, J = 8.4 Hz, 2.1 Hz, 1H), 7.41 (d, J = 8.4 Hz, 1H), 7.06-6.91 (m, 4H), 5.50 (s, 2H).

### Example 2(48):

### 5-(4-cyanophenyl)-1-(4-fluorobenzyl)-1H-1,2,3,4-tetrazole

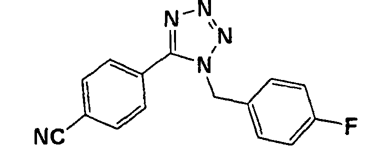

TLC:Rf 0.25 (ethyl acetate : n-hexane = 1 : 2);
NMR (CDCl₃):δ 7.86-7.79 (m, 2H), 7.75-7.69 (m, 2H), 7.18-7.02 (m, 4H), 5.62 (s, 2H).

### Example 2(49):

### 1-(4-fluorobenzyl)-5-(4-methylphenyl)-1H-1,2,3,4-tetrazole

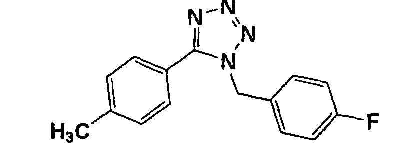

TLC:Rf 0.43 (ethyl acetate : n-hexane = 1 : 2);
NMR (CDCl₃):δ 7.49-7.44 (m, 2H), 7.35-7.30 (m, 2H), 7.19-7.12 (m, 2H), 7.08-7.00 (m, 2H), 5.57 (s, 2H), 2.44 (s, 3H).

### Example 2(50):

### 5-(2,5-dichlorophenyl)-1-(4-fluoro-2-trifluoromethylbenzyl)-1H-1,2,3,4-tetrazole

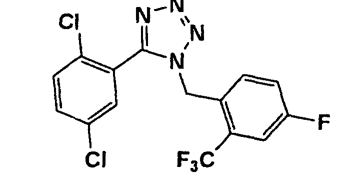

TLC:Rf 0.33 (ethyl acetate : n-hexane = 1 : 4);
NMR (CDCl₃):δ 7.53-7.45 (m, 2H), 7.37 (dd, J = 8.7 Hz, 2.7 HZ, 1H), 7.28-7.16 (m, 3H), 5.63 (s, 2H).

### Example 2(51):

### 5-(2,3-dichlorophenyl)-1-(4-fluoro-2-trifluoromethylbenzyl)-1H-1,2,3,4-tetrazole

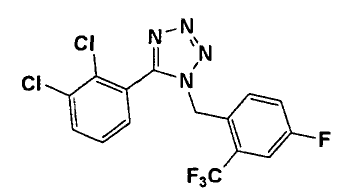

TLC:Rf0.49 (ethyl acetate : n-hexane = 1 : 2);
NMR (CDCl₃):δ 7.68 (d, J = 8.1 Hz, 1.8 Hz, 1H), 7.38-7.29 (m, 2H), 7.24-7.16 (m, 3H), 5.60 (s, 2H).

### Example 2(52):

### 1-(2-chloro-4-fluorobenzyl)-5-(2,5-dichlorophenyl)-1H-1,2,3,4-tetrazole

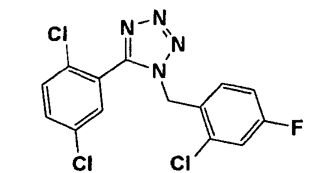

TLC:Rf 0.26 (ethyl acetate : n-hexane = 1 : 5);
NMR (CDCl₃):δ 7.53-7.45 (m, 2H), 7.28-7.24 (m, 1H), 7.20 (dd, J = 8.4, 2.4 Hz, 1H), 7.09 (td, J = 8.4 Hz, 6.0 Hz, 1H), 6.98-6.90 (m, 1H), 5.56 (s, 2H).

### Example 2(53):

### 1-(2-chloro-4-fluorobenzyl)-5-(2,3-dichlorophenyl)-1H-1,2,3,4-tetrazole

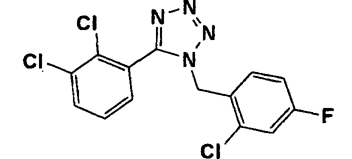

TLC:Rf 0.35 ethyl acetate:toluene=1 : 9);
NMR (CDCl₃):δ 7.68 (dd, J = 8.1, 1.5 Hz, 1H), 7.33 (t, J = 7.5 Hz, 1H), 7.22-7.15 (m, 2H), 7.05 (dd, J = 8.1, 2.7 Hz, 1H), 6.94 (td, J = 8.1, 2.7 Hz, 1H), 5.55 (s, 2H).

### Example 2(54):

### 5-(2,5-dichlorophenyl)-1-(2,5-difluorobenzyl)-1H-1,2,3,4-tetrazole

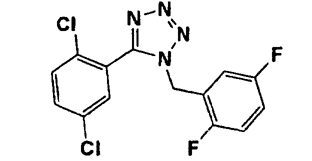

TLC:Rf 0.33 (ethyl acetate : n-hexane = 1 : 4);
NMR (CDCl₃):δ 7.55-7.48 (m, 2H), 7.31-7.28 (m, 1H), 7.07-6.87 (m, 3H), 5.48 (s, 2H).

### Example 2(55):

### 5-(2-chlorophenyl)-1-(2,5-dichlorobenzyl)-1H-1,2,3,4-tetrazole

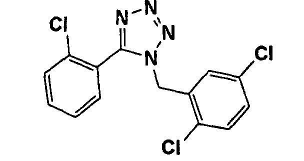

TLC:Rf 0.21 (ethyl acetate : n-hexane = 1 : 4);
NMR (CDCl₃):δ 7.58-7.50 (m, 2H), 7.41-7.35 (m, 1H), 7.34-7.28 (m, 1H), 7.23 (s, 2H), 7.07 (s, 1H), 5.55 (s, 2H).

### Example 2(56):

### 5-(2-chlorophenyl)-1-(2,5-difluorobenzyl)-1H-1,2,3,4-tetrazole

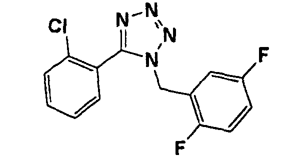

TLC:Rf 0.26 (ethyl acetate : n-hexane = 1 : 3);
NMR (CDCl₃):δ 7.60-7.52 (m, 2H), 7.44-7.38 (m, 1H), 7.36-7.31 (m, 1H), 7.04-6.90 (m, 2H), 6.88-6.81 (m, 1H), 5.48 (s, 2H).

### Example 2(57):

### 1-(4-fluorobenzyl)-5-(3-thienyl)-1H-1,2,3,4-tetrazole

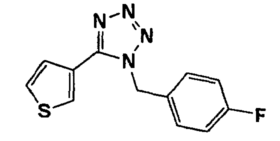

TLC:Rf 0.33 (toluene : ethyl acetate = 9: 1);
NMR (CDCl₃):δ 7.70 (dd, J = 3.0 Hz, 1.2 Hz, 1H), 7.51 (dd, J = 5.1 Hz, 3.0 Hz, 1H), 7.43 (dd, J = 5.1 Hz, 1.2 Hz, 1H), 7.20-7.12 (m, 2H), 7.12-7.03 (m, 2H), 5.67 (s, 2H).

### Example 2(58):

### 5-[2-(3-chlorothienyl)]-1-(4-fluorobenzyl)-1H-1,2,3,4-tetrazole

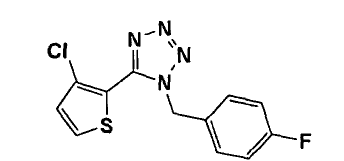

TLC:Rf 0.29 (n-hexane : ethyl acetate = 3 : 1);
NMR (CDCl₃):δ 7.61 (d, J = 5.4 Hz, 1H), 7.14-7 04 (m, 3H), 7.02-6.93 (m, 2H), 5.59 (s, 2H).

### Example 2(59):

### 1-(2-chloro-4-fluorobenzyl)-5-(2-trifluoromethylphenyl)-1H-1,2,3,4-tetrazole

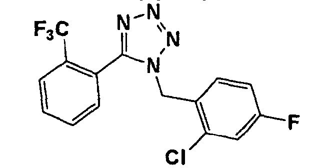

TLC:Rf 0.26 (ethyl acetate : n-hexane = 1 : 3);
NMR (CDCl₃):δ 7.87 (d, J = 8.1 Hz, 1H), 7.73 (t, J = 8.1 Hz, 1H), 7.64 (t, J = 8.1 Hz, 1H), 7.21 (d, J = 8.1 Hz, 1H), 7.15 (dd, J = 8.7 Hz, 6.0 HZ, 1H), 7.07 (dd, J = 8.4 Hz, 2.7 HZ, 1H), 6.97-6.90 (m, 1H), 5.47 (s, 2H).

### Example 2(60):

### 1-(4-fluoro-2-trifluoromethylbenzyl)-5-(5-fluoro-2-trifluoromethylphenyl)-1H-1,2,3,4-tetrazole

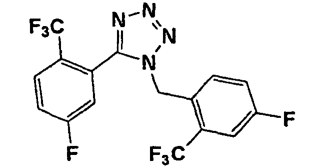

TLC:Rf 0.37 (ethyl acetate : n-hexane = 1 : 4);
NMR (CDCl₃):δ 7.88 (dd, J = 9.0 Hz, 5.1 Hz, 1H), 7.45-7.34 (m, 2H), 7.28-7.23 (m, 2H), 6.86 (dd, J = 8.1 Hz, 2.7 Hz, 1H), 5.55 (s, 2H).

### Example 2(61):

### 1-(2-chloro-4-fluorobenzyl)-5-(5-fluoro-2-trifluoromethylphenyl)-1H-1,2,3,4-tetrazole

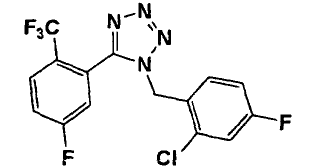

TLC.Rf 0.20 (ethyl acetate : n-hexane = 1 : 5);
NMR (CDCl₃):δ 7.88 (dd, J = 8.4 Hz, 5.4 Hz, 1H), 7.46-7.38 (m, 1H), 7.20 (dd, J = 8.7 Hz, 5.7 Hz, 1H), 7.10 (dd, J = 8.1 Hz, 2.7 Hz, 1H), 7.00-6.88 (m, 2H), 5.50 (s, 2H).

### Example 2(62):

### 1-(2,4-dichlorobenzyl)-5-(2,3-dichlorophenyl)-1H-1,2,3,4-tetrazole

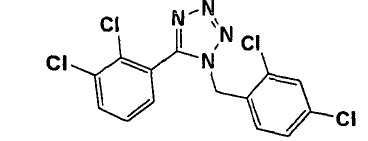

TLC:Rf 0.40 (ethyl acetate : n-hexane = 1 : 3);
NMR (CDCl₃):δ 7.68 (dd, J = 8.4 Hz, 1.8 Hz, 1H), 7.36-7.30 (m, 2H), 7.23-7.17 (m, 2H), 7.11 (d, J = 8.4 Hz, 1H), 5.54 (s, 2H).

### Example 2(63):

### 1-(2,5-dichlorobenzyl)-5-(2,3-dichlorophenyl)-1H-1,2,3,4-tetrazole

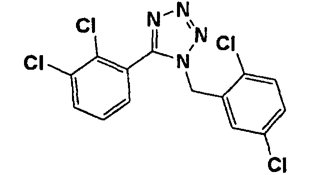

TLC:Rf 0.31 (ethyl acetate : n-hexane = 1 : 3);
NMR (CDCl₃):δ 7.69 (dd, J = 8.1 Hz, 1.8 Hz, 1H), 7.32 (t, J = 8.1 Hz, 1H), 7.25-7.23 (m, 2H), 7.20 (dd, J = 8.1 Hz, 1.8 Hz, 1H), 7.11-7.09 (m, 1H), 5.54 (s, 2H).

### Example 2(64):

### 5-(2,3-dichlorophenyl)-1-(2-phenoxybenzyl)-1H-1,2,3,4-tetrazole

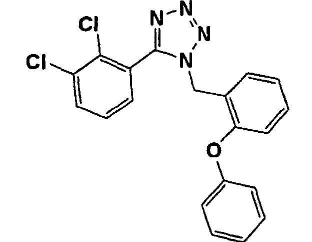

TLC:Rf0.37 (ethyl acetate : n-hexane = 1 : 3);
NMR (CDCl₃):δ 7.60 (dd, J = 7.8 Hz, 1.5 Hz, 1H), 7.33-7.00 (m, 8H), 6.73-6.68 (m, 3H), 5.54 (s, 2H).

### Example 2(65):

### 1-(2-benzyloxybenzyl)-5-(2,3-dichlorophenyl)-1H-1,2,3,4-tetrazole

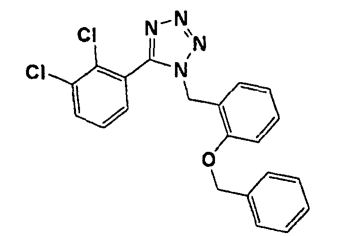

TLC:Rf 0.37 (ethyl acetate : n-hexane = 1 : 2);
NMR (CDCl₃):δ 7.53 (dd, J = 7.5 Hz, 1.5 Hz, 1H), 7.41-7.31 (m, 3H), 7.29-7.22 (m, 1H), 7.20-7.12 (m, 2H), 7.11-7.04 (m, 2H), 6.90-6.78 (m, 3H), 5.48 (s, 2H), 4.89 (s, 2H).

### Example 2(66):

### 1-(2-benzyloxy-5-chlorobenzyl)-5-(2,3-dichlorophenyl)-1H-1,2,3,4-tetrazole

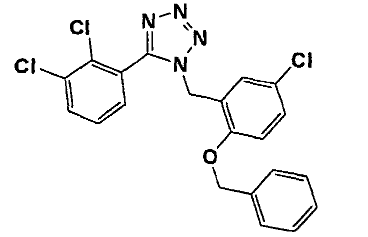

TLC.Rf0.47 (ethyl acetate : n-hexane = 1 : 2);
NMR (CDCl₃):δ 7.57 (dd, J = 8.1 Hz, 1.5 Hz, 1H), 7.42-7.33 (m, 3H), 7.20 (dd, J = 8.7 Hz, 2.4 Hz, 1H), 7.16-7.08 (m, 3H), 7.01 (d, J = 2.4 Hz, 1H), 6.82 (dd, J = 7.5 Hz, 1.5 Hz, 1H), 6.77 (d, J = 8.7 Hz, 1H), 5.42 (s, 2H), 4.88 (s, 2H).

### Example 2(67):

### 1-(5-chloro-2-methoxybenzyl)-5-(2,3-dichlorophenyl)-1H-1,2,3,4-tetrazole

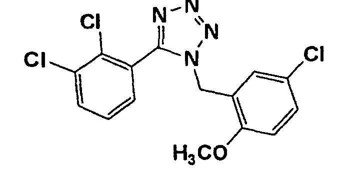

TLC:Rf 0.28 (ethyl acetate : n-hexane = 1 : 2);
NMR (CDCl₃):δ 7.68 (dd, J = 8.0 Hz, 1.5 Hz, 1H), 7.31 (t, J = 8.0 Hz, 1H), 7.21 (dd, J = 9.0 Hz, 2.4 Hz, 1H), 7.17 (dd, J = 8.0 Hz, 1.5 Hz, 1H), 7 02 (d, J = 2.4 Hz, 1H), 6.67 (d, J = 9.0 Hz, 1H), 5.43 (s, 2H), 3.58 (s, 3H).

### Example 2(68):

### 1-(5-chloro-2-isopropyloxybenzyl)-5-(2,3-dichlorophenyl)-1H-1,2,3,4-tetrazole

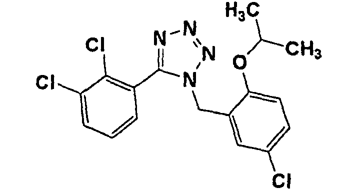

TLC:Rf 0.41 (ethyl acetate : n-hexane = 1 : 2);
NMR (CDCl₃):δ 7.65 (dd, J = 7.8 Hz, 1.8 Hz, 1H), 7.25 (t, J = 7.8 Hz, 1H), 7.15 (dd, J = 9.0 Hz, 2.7 Hz, 1H), 7.09 (dd, J = 7.8 Hz, 1.8 Hz, 1H), 6.84 (d, J = 2.7 Hz, 1H), 6.67 (d, J = 9.0 Hz, 1H), 5.46 (s, 2H), 4.39 (quintet, J = 6.0 Hz, 1H), 1.15 (d, J = 6.0, 6H).

### Example 2(69):

### 1-(5-chloro-2-ethoxycarbonylmethyloxybenzyl)-5-(2,3-dichlorophenyl)-1H-1,2,3,4-tetrazole

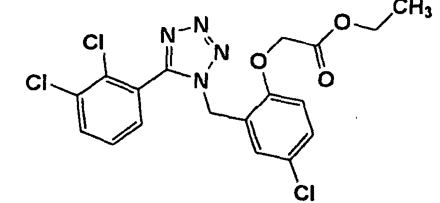

TLC:Rf 0.26 (ethyl acetate : n-hexane = 1 : 2);
NMR (CDCl₃):δ 7.65 (dd, J = 7.8 Hz, 1.5 Hz, 1H), 7.28 (t, J = 7.8 Hz, 1H), 7.22-7.16 (m, 2H), 7.00 (d, J = 2.7 Hz, 1H), 6.58 (d, J = 9.0 Hz, 1H), 5.54 (s, 2H), 4.42 (s, 2H), 4.19 (q, J = 7.2 Hz, 2H), 1.25 (t, J = 7.2, 3H).

### Example 2(70):

### 1-(2,3-dichlorobenzyl)-5-(2,3-dichlorophenyl)-1H-1,2,3,4-tetrazole

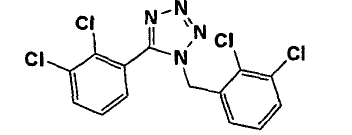

TLC:Rf 0.27 (ethyl acetate : n-hexane = 1 : 3);
NMR (CDCl₃):δ 7.68 (dd, J = 8.1 Hz, 1.8 Hz, 1H), 7.43 (dd, J = 8.1 Hz, 1.8 Hz, 1H), 7.32 (t, J = 8.1 Hz, 1H), 7.21 (dd, J = 8.1 Hz, 1.8 Hz, 1H), 7.16 (t, J = 8.1 Hz, 1H), 7.03 (dd, J = 8.1 Hz, 1.8 Hz, 1H), 5.60 (s, 2H).

### Example 2(71):

### 5-(2,5-dichlorobenzyl)-1-(2,3-dichlorophenyl)-1H-1,2,3,4-tetrazole

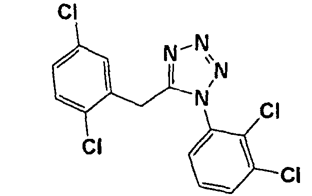

TLC:Rf 0.49 (n-hexane : ethyl acetate = 2 : 1);
NMR (CDCl₃):δ 7.71 (dd, J = 8.1 Hz, 1.5 Hz, 1H), 7.35 (t, J = 8.1 Hz, 1H), 7.23-7.12 (m, 4H), 4.29 (s, 2H).

### Example 2(72):

### 1-(2-benzylbenzyl)-5-(2,3-dichlorophenyl)-1H-1,2,3,4-tetrazole

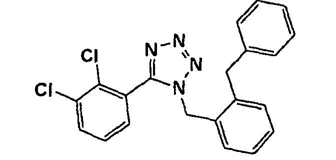

TLC:Rf 0.36 (toluene : ethyl acetate = 20 : 1);
NMR (CDCl₃):δ 7.64 (dd, J = 8.1, 1.5 Hz, 1H), 7.28-7.06 (m, 7H), 7.02 (dd, J = 7.6, 1.5 Hz, 1H), 6.96-6.91 (m, 2H), 6.77-6.73 (m, 1H), 5.38 (s, 2H), 3.84 (s, 2H).

### Example 2(73):

### 1-(2,3-dichlorobenzyl)-5-(2,3-dichlorobenzyl)-1H-1,2,3,4-tetrazole

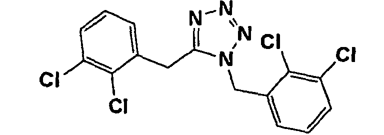

TLC:Rf 0.37 (n-hexane : ethyl acetate = 2 : 1);
NMR (CDCl₃):δ 7.41 (dd, J = 7.8 Hz, 1.5 Hz, 1H), 7.34 (dd, J = 7.8 Hz, 1.5 Hz, 1H), 7.10 (t, J = 7.8 Hz, 1H), 7.09 (t, J = 7.8 Hz, 1H), 6.96 (dd, J = 7.8 Hz, 1.5 Hz, 1H), 6.63 (dd, J = 7.8 Hz, 1.5 Hz, 1H), 5.60 (s, 2H), 4.39 (s, 2H).

### Example 2(74):

### 5-(2,3-dichlorobenzyl)-1-(2,3-dichlorophenyl)-1H-1,2,3,4-tetrazole

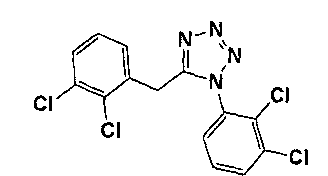

TLC:Rf 0.37 (n-hexane : ethyl acetate = 2 : 1);
NMR (CDCl₃):δ 7.69 (dd, J = 8.1 Hz, 1.5 Hz, 1H), 7.40-7.30 (m, 2H), 7.15 (dd, J = 8.1 Hz, 1.5 Hz, 1H), 7.13-7.09 (m, 2H), 4.36 (s, 2H).

### Example 2(75):

### 5-(2,3-dichlorophenyl)-1-(2-phenoxymethylbenzyl)-1H-1,2,3,4-tetrazole

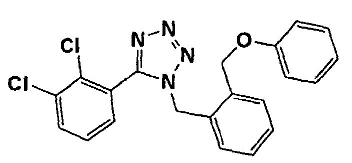

TLC:Rf 0.34 (toluene : ethyl acetate = 19 : 1);
NMR (CDCl₃):δ 7.61 (dd, J = 8.1 Hz, 1.5 Hz, 1H), 7.40-7.17 (m, 6H), 7.08 (dd, J = 7.5 Hz, 1.5 Hz, 1H), 7.03-6.96 (m, 1H), 6.93 (d, J = 7.5 Hz 1H), 6.86-6.80 (m, 2H), 5.63 (s, 2H), 4.87 (s, 2H).

### Example 2(76):

### 5-(2,3-dichlorophenyl)-1-(2-phenethylbenzyl)-1H-1,2,3,4-tetrazole

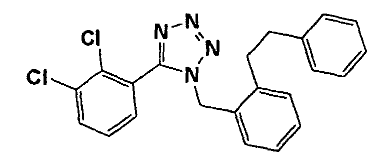

TLC:Rf 0.37 (toluene : ethyl acetate = 19 : 1);
NMR (CDCl₃):δ 7.63 (dd, J = 8.1 Hz, 1.5 Hz, 1H), 7.28-7.12 (m, 6H), 7.08-6.96 (m, 4H), 6.77 (d, J = 7.8 Hz 1H), 5.21 (s, 2H), 2.70 (s, 4H).

### Example 2(77):

### 1-(2-chloro-6-methoxybenzyl)-5-(2,3-dichlorophenyl)-1H-1,2,3,4-tetrazole

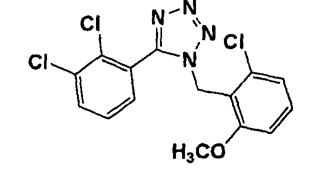

TLC:Rf 0.29(n-hexane : ethyl acetate = 2 : 1);
NMR (CDCl₃):δ 7.64 (dd, J = 7.8 Hz, 1.5 Hz, 1H), 7.27 (t, J = 7.8 Hz, 1H), 7.20 (t, J = 8.4 Hz, 1H), 7.18 (dd, J = 7.8 Hz, 1.5 Hz, 1H), 6.93 (d, J = 8.4 Hz, 1H), 6.67 (d, J = 8.4 Hz, 1H), 5.63 (s, 2H), 3.64 (s, 3H).

### Example 2(78):

### 5-(2,3-dichlorophenyl)-1-[2-(N-methyl-N-phenylamino)benzyl]-1H-1,2,3,4-tetrazole

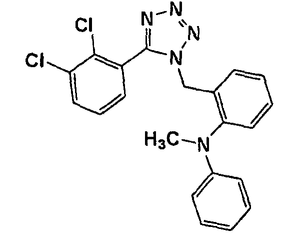

TLC:Rf0.44 (toluene : ethyl acetate = 9 : 1);
NMR (CDCl₃):δ 7.61 (dd, J = 8.4, 1.5 Hz, 1H), 7.37 (dt, J = 7.8, 1.5 Hz, 1H), 7.23-7 06 (m, 6H), 6.91 (dd, J = 7.8, 1.5 Hz, 1H), 6.79-6.74 (m, 1H), 6.35-6.31 (m, 2H), 5.33 (s, 2H), 2.94 (s, 3H).

### Example 2(79):

### 1-(2-benzyloxy-6-chlorobenzyl)-5-(2,3-dichlorophenyl)-1H-1,2,3,4-tetrazole

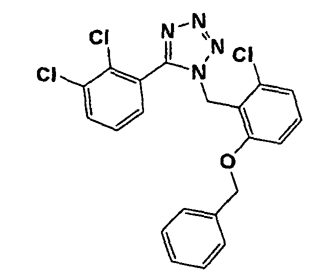

TLC:Rf 0.35 (n-hexane : ethyl acetate = 3 : 1);
NMR (CDCl₃):δ 7.58 (dd, J = 8.1 Hz, 1.5 Hz, 1H), 7.42-7.32 (m, 3H), 7.23-7.16 (m, 3H), 7.12 (t, J = 8.1 Hz, 1H), 6.95 (d, J = 8.1 Hz, 1H), 6.88 (dd, J = 8.1 Hz, 1.5 Hz, 1H), 6.78 (d, J = 8.1 Hz, 1H), 5.61 (s, 2H), 4.95 (s, 2H).

### Example 2(80):

### 1-(2-benzyloxy-4-chlorobenzyl)-5-(2,3-dichlorophenyl)-1H-1,2,3,4-tetrazole

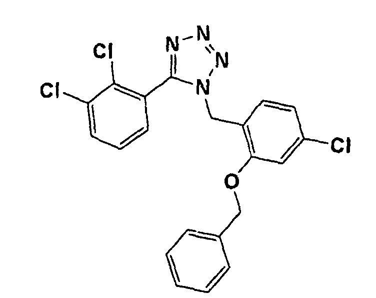

TLC:Rf 0.38 (n-hexane : ethyl acetate = 3 : 1);
NMR (CDCl₃):δ 7.55 (dd, J = 7.8 Hz, 1.5 Hz, 1H), 7.43-7.34 (m, 3H), 7.18-7.13 (m, 2H), 7.10 (t, J = 7.8 Hz, 1H), 7.03 (d, J = 7.8 Hz, 1H), 6.90-6.85 (m, 2H), 6.79 (dd, J = 8.1 Hz, 1.5 Hz, 1H), 5.41 (s, 2H), 4.87 (s, 2H).

### Example 2(81):

### 1-(benzocycloheptene-1-yl)-5-(2,3-dichlorophenyl)-1H-1,2,3,4-tetrazole

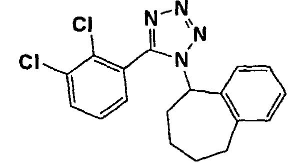

TLC:Rf0.48 (toluene : ethyl acetate = 9 : 1);
NMR (CDCl₃):δ 7.62 (dd, J = 8.1, 1.5 Hz, 1H), 7.23 (t, J = 7.8 Hz, 1H), 7.18-7.02 (m, 4H), 6.21 (d, J = 7.5 Hz, 1H), 5.51 (dd, J = 9.0, 3.0 Hz, 1H), 2.88-2.81 (m, 1H), 2.68-2.59 (m, 1H), 2.55-2.34 (m, 2H), 2.20-2.10 (m, 1H), 1.97-1.75 (m, 2H), 1.56-1.44 (m, 1H).

### Example 2(82):

### 1-(2-benzyloxy-5-nitrobenzyl)-5-(2,3-dichlorophenyl)-1H-1,2,3,4-tetrazole

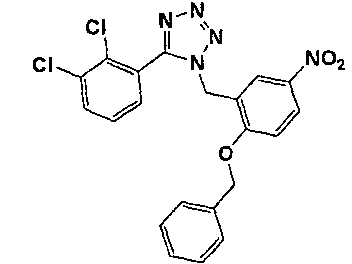

TLC:Rf 0.31 (n-hexane : ethyl acetate = 2 : 1);
NMR (CDCl₃):δ 8.20 (dd, J = 9.0 Hz, 2.7 Hz, 1H), 7.95 (d, J = 2.7 Hz, 1H), 7.59 (dd, J = 8.1 Hz, 1.5 Hz, 1H), 7.43-7.38 (m, 3H), 7.20-7.12 (m, 3H), 6.97 (d, J = 9.0 Hz, 1H), 6.83 (dd, J = 8.1 Hz, 1.5 Hz, 1H), 5.49 (s, 2H), 5.05 (s, 2H).

### Example 2(83):

### 1-(2-benzyloxy-5-fluorobenzyl)-5-(2,3-dichlorophenyl)-1H-1,2,3,4-tetrazole

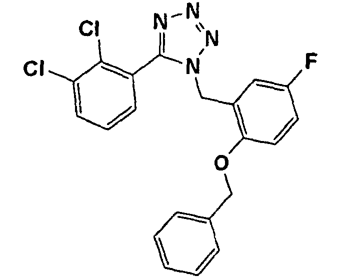

TLC:Rf 0.48 (n-hexane : ethyl acetate = 2 : 1);
NMR (CDCl₃):δ 7.56 (dd, J = 7.8, 1.8 Hz, 1H), 7.41-7.33 (m, 3H), 7.17-7.12 (m, 2H), 7.12 (t, J = 7.8 Hz, 1H), 6 95 (ddd, J = 9.0, 8.1, 3.0 Hz, 1H), 6.86 (dd, J = 7.8, 1.8 Hz, 1H), 6.82 (dd, J = 9.0, 3.0 Hz, 1H), 6.78 (dd, J = 9.0, 3.9 Hz, 1H), 5.43 (s, 2H), 4.86 (s, 2H).

### Example 2(84):

### 1-(2-benzyloxy-5-methylbenzyl)-5-(2,3-dichlorophenyl)-1H-1,2,3,4-tetrazole

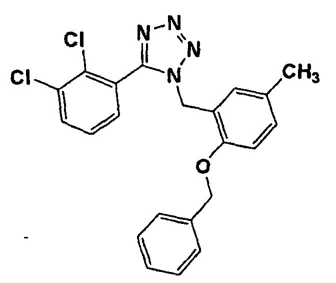

TLC:Rf 0.49 (n-hexane : ethyl acetate = 2 : 1);
NMR (CDCl₃):δ 7.52 (dd, J = 7.8, 1.8 Hz, 1H), 7.40-7.29 (m, 3H), 7 17-7.12 (m, 2H), 7.07 (t, J = 7.8, 1H), 7.02 (dd, J = 8.4, 2.1 Hz, 1H), 6.84 (d, J = 2.1 Hz, 1H), 6.80 (dd, J = 7.8, 1.8 Hz, 1H), 6.71 (d, J = 8.4 Hz, 1H), 5.44 (s, 2H), 4.85 (s, 2H), 2.18 (s, 3H).

### Example 2(85):

### 1-(2-benzyloxy-5-dimethylaminobenzyl)-5-(2,3-dichlorophenyl)-1H-1,2,3,4-tetrazole

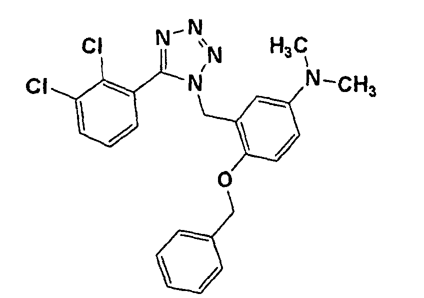

TLC:Rf 0.33 (ethyl acetate : n-hexane = 1 : 1);
NMR (CDCl₃):δ 7.51 (dd, J = 8.1 Hz, 1.5 Hz, 1H), 7.39-7.28 (m, 3H), 7.16-7.12 (m, 2H), 7.07 (t, J = 8.1 Hz, 1H), 6.83 (dd, J = 8.1 Hz, 1.5 Hz, 1H), 6.73 (d, J = 9.0 Hz, 1H), 6.61 (dd, J = 9.0 Hz, 3.3 Hz, 1H), 6.48 (d, J = 3.3 Hz, 1H), 5.45 (s, 2H), 4.79 (s, 2H), 2.80 (s, 6H).

### Example 2(86):

### 5-(2-benzyloxybenzyl)-1-(2,3-dichlorophenyl)-1H-1,2,3,4-tetrazole

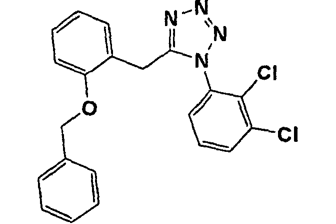

TLC:Rf 0.21 (n-hexane : ethyl acetate = 4 : 1);
NMR (CDCl₃):δ 7.53 (dd, J = 8.4, 1.2 Hz, 1H), 7.40-7.31 (m, 3H), 7.23-7.15 (m, 3H), 7.12-7.03 (m, 2H), 6.88-6.81 (m, 2H), 6.57 (dd, J = 8.4, 1.2 Hz, 1H), 4.86 (s, 2H), 4.15 (s, 2H).

### Example 2(87):

### 5-(2,3-dichlorophenyl)-1-(2-nitrobenzyl)-1H-1,2,3,4-tetrazole

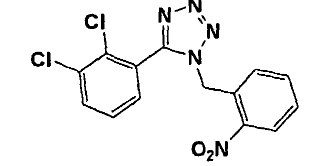

TLC:Rf 0.31 (n-hexane : ethyl acetate = 2 : 1);
NMR (CDCl₃):δ 8.13 (dd, J = 8.1, 1.5 Hz, 1H), 7.70 (dd, J = 7.2, 2.7 Hz, 1H), 7.65 (dd, J = 7.8, 1.5 Hz, 1H), 7.56 (dt, J = 7.8, 1.5 Hz, 1H), 7.41-7.33 (m, 2H), 7.23 (dd, J = 8.1, 1.5 Hz, 1H), 5.82 (s, 2H).

### Example 2(88):

### 5-(2-benzyloxy-5-chlorobenzyl)-1-(2,3-dichlorophenyl)-1H-1,2,3,4-tetrazole

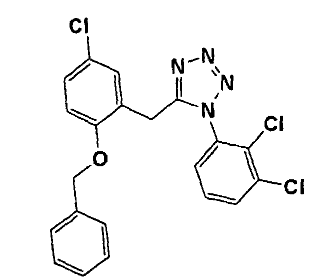

TLC:Rf 0.26 (n-hexane : ethyl acetate = 4 : 1);
NMR (CDCl₃):δ 7.58 (dd, J = 8.1, 1.8 Hz, 1H), 7.41-7.33 (m, 3H), 7.18-7.08 (m, 4H), 7.04 (d, J = 2.4 Hz, 1H), 6.76 (d, J = 7.8 Hz, 1H), 6.59 (dd, J = 7.8, 1.5 Hz, 1H), 4.85 (s, 2H), 4.10 (s, 2H).

### Example 2(89):

### 1-(2-benzyloxy-5-chlorobenzyl)-5-(2,3-dimethylphenyl)-1H-1,2,3,4-tetrazole

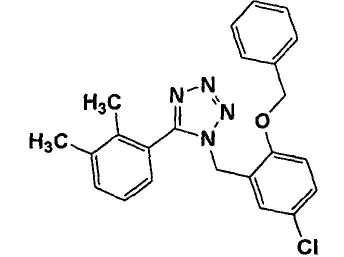

TLC:Rf 0.38 (n-hexane : ethyl acetate = 3 : 1);
NMR (CDCl₃):δ 7.39-7.26 (m, 4H), 7.22-7.16 (m, 3H), 7.11 (t, J = 7.8 Hz, 1H), 6.89 (d, J = 7.8 Hz, 1H), 6.80-6.74 (m, 2H), 5.38 (s, 2H), 4.93 (s, 2H), 2.26 (s, 3H), 1.83 (s, 3H).

### Example 2(90):

### 5-(2,3-dichlorophenyl)-1-(2,5-dimethylbenzyl)-1H-1,2,3,4-tetrazole

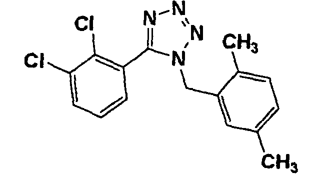

TLC:Rf 0.43 (toluene : ethyl acetate = 10 : 1);
NMR (CDCl₃):δ 7.63 (dd, J = 7.8, 1.8 Hz, 1H), 7.22 (t, J = 7.8 Hz, 1H), 7.02 (dd, J = 7.8, 1.8 Hz, 1H), 7.00-6.89 (m, 2H), 6.51 (brs, 1H), 5.47 (s, 2H), 2.13 (s, 3H), 2.07 (s, 3H).

### Example 2(91):

### 1-(2-benzyloxy-5-chlorobenzyl)-5-(2-chlorophenyl)-1H-1,2,3,4-tetrazole

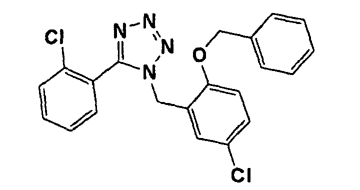

TLC:Rf 0.25 (n-hexane : ethyl acetate = 3 : 1);
NMR (CDCl₃):δ 7.47-7.33 (m, 5H), 7.24-7.13 (m, 4H), 6.99-6.94 (m, 2H), 6.76 (d, J = 8.7 Hz, 1H), 5.45 (s, 2H), 4.88 (s, 2H).

### Example 2(92):

### 1-(2-benzyloxy-5-chlorobenzyl)-5-(2-methylphenyl)-1H-1,2,3,4-tetrazole

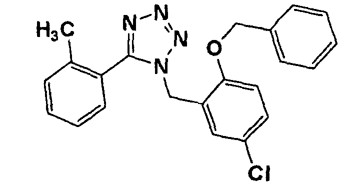

TLC:Rf 0.30 (n-hexane : ethyl acetate = 3 : 1);
NMR (CDCl₃):δ 7.43-7.25 (m, 5H), 7.22-7.16 (m, 4H), 7.03 (dd, J = 7.5, 1.2 Hz, 1H), 6.84 (d, J = 2.7 Hz, 1H), 6.78 (d, J = 8.7 Hz, 1H), 5.40 (s, 2H), 4.92 (s, 2H), 2.03 (s, 3H).

### Example 2(93):

### 1-(2-benzylaminobenzyl)-5-(2,3-dichlorophenyl)-1H-1,2,3,4-tetrazole

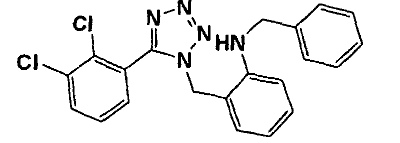

TLC:Rf 0.33(n-hexane : ethyl acetate = 3 : 1);
NMR (CDCl₃):δ 7.67 (dd, J = 8.1, 1.5 Hz, 1H), 7.39-7.33 (m, 2H), 7.31-7.22 (m, 4H), 7.14-7.09 (m, 1H), 7.02 (dd, J = 7.8, 1.5 Hz, 1H), 6.60 (d, J = 8.1 Hz, 1H), 6.49-6.42 (m, 2H), 5.42 (s, 2H), 4.89-4.83 (br, 1H), 4.33 (d, J = 5.1 Hz, 2H).

### Example 2(94):

### 5-(2,3-dichlorophenyl)-1-(2,4-dimethylbenzyl)-1H-1,2,3,4-tetrazole

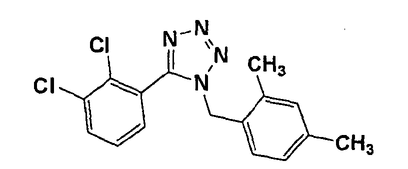

TLC:Rf0.49 (toluene : ethyl acetate = 10 : 1);
NMR (CDCl₃):δ 7 63 (dd, J = 8.4, 1 8 Hz, 1H), 7.25 (t, J = 8.4 Hz, 1H), 7.06 (dd, J = 8.4, 1.8 Hz, 1H), 6.91 (d, J = 1.8 Hz, 1H), 6.79 (dd, J = 7.5, 1.8 Hz, 1H), 6.63 (d, J = 7.5 Hz, 1H), 5.45 (s, 2H), 2.24 (s, 3H), 2.10 (s, 3H).

### Example 2(95):

### 1-(2-benzyloxy-5-chlorobenryl)-5-(3-methylphenyl)-1H-1,2,3,4-tetrazole

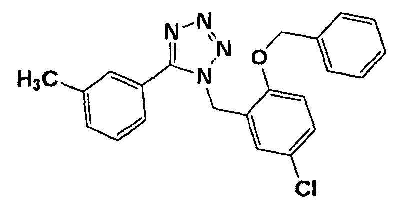

TLC:Rf0.48 (n-hexane : ethyl acetate = 2 : 1);
NMR (CDCl₃):δ 7.73-7.18 (m, 10H), 6.98 (d, J = 2.4 Hz, 1H), 6.89 (d, J = 8.7 Hz, 1H), 5.59 (s, 2H), 4.99 (s, 2H), 2.35 (s, 3H).

### Example 2(96):

### 1-(2-chloro-6-fluorobenzyl)-5-(2-nitrophenyl)-1H-1,2,3,4-tetrazole

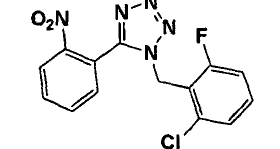

TLC:RF 0.19 (n-hexane : ethyl acetate = 3 : 2);
NMR (CDCl₃):δ 8.35 (dd, J = 7.8, 1.2 Hz, 1H), 7.81-7.68 (m, 2H), 7.34-7.22 (m, 2H), 7.14 (dt, J = 7.8, 1.2 Hz, 1H), 6.89 (td, J = 9.0, 1.2 Hz, 1H), 5.60 (d, J = 1.2 Hz, 2H).

### Example 2(97):

### 1-(4-fluorobenzyl)-5-(4-nitrophenyl)-1H-1,2,3,4-tetrazole

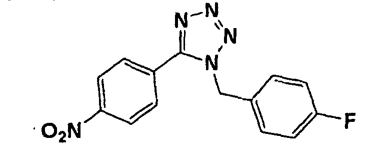

TLC:Rf 0.25 (toluene : ethyl acetate = 9 : 1).

### Example 2(98):

### 1-(4-fluorobenzyl)-5-(2-nitrophenyl)-1H-1,2,3,4-tetrazole

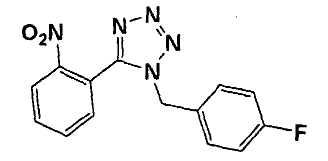

TLC:Rf 0.23 (toluene : ethyl acetate = 9 : 1).

### Example 2(99):

### 5-(2-benzyloxy-3-methoxyphenyl)-1-(4-fluoro-2-trifluoromethylbenzyl)-1H-1,2,3,4-tetrazole

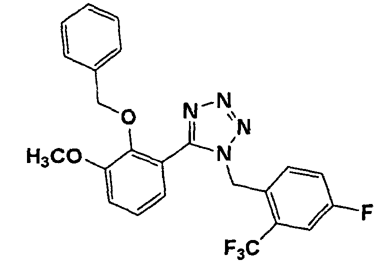

TLC:Rf 0.30 (n-hexane : ethyl acetate = 3 : 1);
NMR (CDCl₃):δ 7.33-7.25 (m, 4H), 7.22-7.12 (m, 2H), 7.08-6.86 (m, 5H), 5.44 (s, 2H), 4.87 (s, 2H), 3.96 (s, 3H).

### Example 2(100):

### 5-(2,3-dichlorophenyl)-1-(2-phenylbenzyl)-1H-1,2,3,4-tetrazole

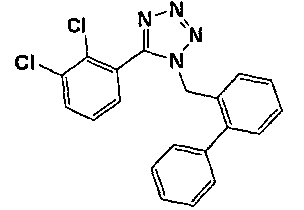

TLC:Rf 0.25 (toluene : ethyl acetate = 20 : 1);
NMR (CDCl₃):δ 7.61-7.11 (m, 9H), 6.95-6.84 (m, 3H), 5.47 (s, 2H).

### Example 2(101):

### 5-(2,4-dichlorophenyl)-1-(2-phenoxybenzyl)-1H-1,2,3,4-tetrazole

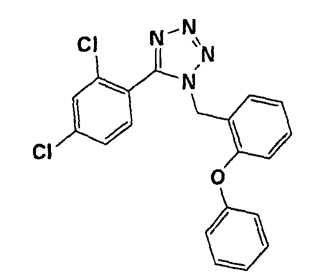

TLC:Rf 0.56 (n-hexane : ethyl acetate = 3 : 1);
NMR (CDCl₃):δ 7.53-7.50 (m, 1H), 7.34-7.01 (m, 8H), 6.74-6.61 (m, 3H), 5.55 (s, 2H).

### Example 2(102):

### 5-(2,5-dichlorophenyl)-1-(2-phenoxybenzyl)-1H-1,2,3,4-tetrazole

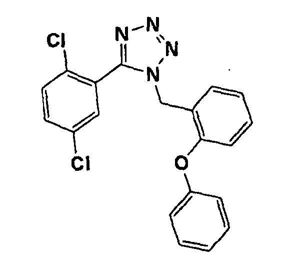

TLC:Rf 0.53 (n-hexane : ethyl acetate = 3 : 1);
NMR (CDCl₃):δ 7.44-7.40 (m, 1H), 7.34-7.21 (m, 5H), 7.14-7.02 (m, 3H), 6.76-6.62 (m, 3H), 5.54 (s, 2H).

### Example 2(103):

### 5-(2-chloro-5-methoxymethoxyphenyl)-1-(5-chloro-2-benzyloxybenzyl)-1H-1,2,3,4-tetrazole

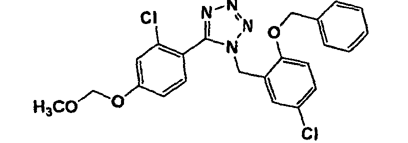

TLC:Rf 0.44 (ethyl acetate : n-hexane = 1 : 2);
NMR (CDCl₃):δ 7.41-7.30 (m, 3H), 7.22-7.12 (m, 4H), 6.97 (d, J = 2.7 Hz, 1H), 6.90-6.83 (m, 2H), 6.77 (d, J = 9.0 Hz, 1H), 5.43 (s, 2H), 5.18 (s, 2H), 4.90 (s, 2H), 3.48 (s, 3H).

### Example 2(104):

### 1-(2-benzyloxy-5-chlorobenzyl)-5-phenyl-1H-1,2,3,4-tetrazole

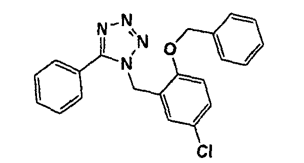

TLC:Rf 0.48 (n-hexane : ethyl acetate = 2 : 1);
NMR (CDCl₃):δ 7.57-7.19 (m, 11H), 6.98 (d, J = 2.4 Hz, 1H), 6.87 (d, J = 9.0 Hz, 1H), 5.59 (s, 2H), 4.99 (s, 2H).

### Example 2(105):

### 1-(2-benzyloxy-5-chlorobenzyl)-5-(3-chlorophenyl)-1H-1,2,3,4-tetrazole

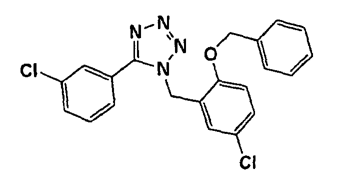

TLC:Rf 0.53 (n-hexane : ethyl acetate = 2 : 1);
NMR (CDCl₃):δ 7.52-7.25 (m, 8H), 7.22-7.17 (m, 2H), 7.05 (d, J = 2.4 Hz, 1H), 6.90 (d, J = 9.0 Hz, 1H), 5.58 (s, 2H), 4.99 (s, 2H).

### Example 2(106):

### 1-(5-chloro-2-cyclohexylmethyloxybenzyl)-5-(2,3-dichlorophenyl)-1H-1,2,3,4-tetrazole

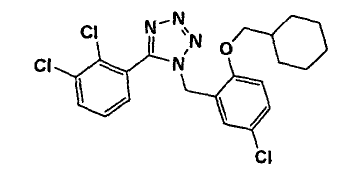

TLC:Rf 0.39 (n-hexane : ethyl acetate = 4 : 1);
NMR (CDCl₃):δ 7.63 (dd, J = 7.8, 1.5 Hz, 1H), 7.24 (t, J = 7.8 Hz, 1H), 7.16 (dd, J = 8.7, 2.7 Hz, 1H), 7.06 (dd, J = 7.8, 1.5 Hz, 1H), 6.86 (d, J = 2.7 Hz, 1H), 6.65 (d, J = 8.7 Hz, 1H), 5.48 (s, 2H), 3.57 (d, J = 6.0 Hz, 2H), 1.76-1.52 (m, 6H), 1.33-1.14 (m, 3H), 0.96-0.84 (m, 2H).

### Example 2(107):

### 1-[5-chloro-2-(4-pyridylmethyloxy)benzyl]-5-(2,3-dichlorophenyl)-1H-1,2,3,4-tetrazole

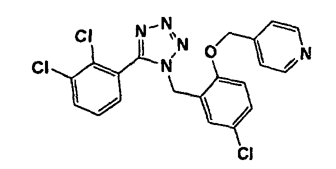

TLC:Rf 0.54 (chloroform:methanol=9 : 1);
NMR (CDCl₃):δ 8.62 (dd, J = 4.5, 1.5 Hz, 2H), 7.60 (dd, J = 7.8, 1.5 Hz, 1H), 7.23-7.18 (m, 2H), 7.14-7.12 (m, 2H), 7.02 (dd, J = 7.8, 1.5 Hz, 1H), 6.97 (d, J = 2.4 Hz, 1H), 6.70 (d, J = 9.0 Hz, 1H), 5.52 (s, 2H), 4.93 (s, 2H).

### Example 2(108):

### 1-[2-(2H,3H-benzo[e]1,4-dioxan-2-ylmethyloxy)-5-chlorobenzyl]-5-(2,3-dichlorophenyl)-1H-1,2,3,4-tetrazole

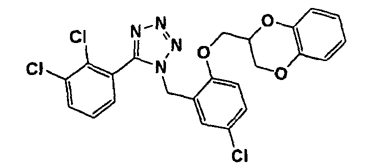

TLC:Rf 0.20(n-hexane : ethyl acetate = 4 : 1);
NMR (CDCl₃):δ 7.62 (dd, J = 7.8, 1.5 Hz, 1H), 7.25-7.18 (m, 2H), 7.12 (dd, J = 7.8, 1.5 Hz, 1H), 6.92-6.85 (m, 5H), 6.72 (d, J = 8.7 Hz, 1H), 5.47 (s, 2H), 4.41-4.35 (m, 1H), 4.26 (dd, J = 11.7, 2.1 Hz, 1H), 4.10-3.99 (m, 3H).

### Example 2(109):

### 1-(2-benzyloxy-5-chlorobenzyl)-5-(2-chloro-3-methylphenyl)-1H-1,2,3,4-tetrazole

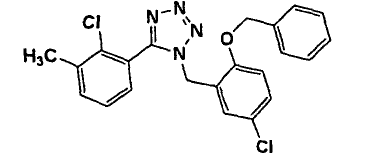

TLC:Rf 0.47 (n-hexane : ethyl acetate = 2 : 1);
NMR (CDCl₃):δ 7.41-7.32 (m, 4H), 7.20-7.08 (m, 4H), 6.93 (d, J = 2.7 Hz, 1H), 6.84-6.79 (m, 1H), 6.75 (d, J = 8.7 Hz, 1H), 5.43 (s, 2H), 4.89 (s, 2H), 2.39 (s, 3H).

### Example 2(110):

### 5-(2,3-chlorophenyl)-1-(1-phenylethyl)-1H-1,2,3,4-tetrazole

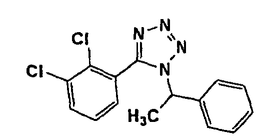

TLC:Rf 0.45 (n-hexane : ethyl acetate = 2 : 1);
NMR (CDCl₃):δ 7.66 (dd, J = 8.1, 1.5 Hz, 1H), 7.30-7.23 (m, 4H), 7.11-7.04 (m, 2H), 6.98 (dd, J = 8.1, 1.5 Hz, 1H), 5.39 (q, J = 6.9 Hz, 1H), 2.06 (d, J = 6.9 Hz, 3H).

### Example 2(111):

### 1-(5-chloro-2-phenethyloxybenzyl)-5-(2,3-dichlorophenyl)-1H-1,2,3,4-tetrazole

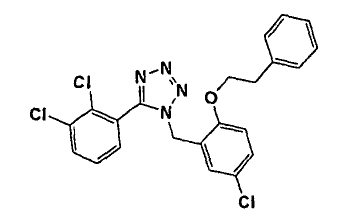

TLC:Rf 0.25 (n-hexane : ethyl acetate = 4 : 1);
NMR (CDCl₃):δ 7.63 (dd, J = 7.8, 1.5 Hz, 1H), 7.36-7.14 (m, 7H), 7.01 (dd, J = 7.8, 1.5 Hz, 1H), 6.85 (d, J = 2.4 Hz, 1H), 6.65 (d, J = 9.0 Hz, 1H), 5.40 (s, 2H), 3.99 (t, J = 6.9 Hz, 2H), 2.91 (t, J = 6.9 Hz, 2H).

### Example 2(112):

### 5-(2,3-dichlorophenyl)-1-(2-cyclohexyloxy-5-chlorobenzyl)-1H-1,2,3,4-tetrazole

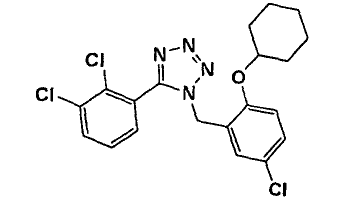

TLC:Rf 0.34 (n-hexane : ethyl acetate = 4 : 1);
NMR (CDCl₃):δ 7.63 (dd, J = 7.8, 1.5 Hz, 1H), 7.23 (t, J = 7.8 Hz, 1H), 7.13 (dd, J = 8.7, 2.4 Hz, 1H), 7.06 (dd, J = 7.8, 1.5 Hz, 1H), 6.80 (d, J = 2.4 Hz, 1H), 6.67 (d, J = 8.7 Hz, 1H), 5.49 (s, 2H), 4.16-4.06 (m, 1H), 1.79-1.48 (m, 5H), 1.34-1.22 (m, 5H).

### Example 3

### 5-(2-aminophenyl)-1-(2-chloro-6-fluorobenzyl)-1H-1,2,3,4-tetrazole hvdrochloride

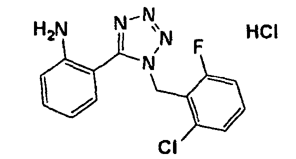

A solution of the compound prepared in Example 2(96) (276 mg) and tin chloride dehydrate (373 mg) in ethanol (3.00 ml) were refluxed for 45 minutes. The reaction mixture was cooled and the mixture was neutralized with a saturated sodium carbonate and was extracted with methylene chloride. The organic layer was washed with brine, dried over magnesium sulfate and was concentrated. The residue was purified by column chromatography on silica gel (n-hexane : ethyl acetate = 7 : 3) to give a free compound (82.6 mg) of the compound present invention. To a solution of the compound in methanol under cooling with ice, was added a solution of 4N hydrochloric acid in dioxane (0.140 ml) dropwise and the mixture was stirred for 30 minutes at room temperature. The precipitates were collected to give the compound of the present invention (63 mg) having the following physical data.
TLC:Rf0.37 (ethyl acetate : n-hexane = 3 : 7);
NMR (DMSO-d₆):δ 7.48-7.23 (m, 5H), 6.87 (dd, J = 8.4, 1.2 Hz, 1H), 6.69 (td, J = 7.8, 1.2 Hz, 1H), 5.62 (s, 2H).

### Example 3(1)-Example 3(2)

By the same procedure as described in example 3 using the compound prepared in Example 2(82) or Example 2(87), the compounds of the present invention having the following physical data were given.

### Example 3(1):

### 1-(5-amino-2-benzyloxybenzyl)-5-(2,3-dichlorophenyl)-1H-1,2,3,4-tetrazole

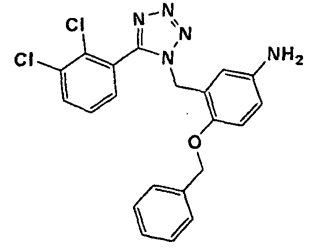

TLC:Rf 0.31 (ethyl acetate : n-hexane = 1 : 1);
NMR (CDCl₃):δ 7.53 (dd, J = 7.8 Hz, 1.5 Hz, 1H), 7.39-7.28 (m, 3H), 7.16-7.06 (m, 3H), 6.87 (dd, J = 7.8 Hz, 1.5 Hz, 1H), 6.65 (d, J = 8.4 Hz, 1H), 6.56 (dd, J = 8.4 Hz, 2.7 Hz, 1H), 6.44 (d, J = 2.7 Hz, 1H), 5.38 (s, 2H), 4.77 (s, 2H), 3.41 (bs, 2H).

### Example 3(2):

### 1-(2-aminobenzyl)-5-(2,3-dichlorophenyl)-1H-1,2,3,4-tetrazole

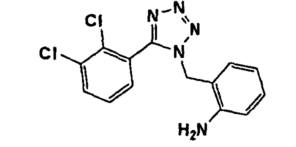

TLC:Rf 0.34(n-hexane : ethyl acetate = 2 : 1);
NMR (CDCl₃):δ 7.70 (dd, J = 8.1, 1.5 Hz, 1H), 7.31 (t, J = 8.1 Hz, 1H), 7.14-7.06 (m, 2H), 6.65 (dd, J = 8.1, 1.2 Hz, 1H), 6.53-6.43 (m, 2H), 5.39 (s, 2H), 4.13 (bs, 2H).

### Example 4:

### 1-(2-chloro-6-fluorobenzyl)-5-(2-hydroxyphenyl)-1H-1,2,3,4-tetrazole

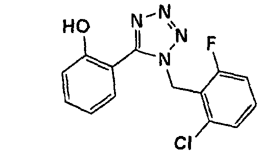

A solution of the compound prepared in example 2(32) (197 mg) and trimethylsilane iodide(0.14 ml) in acetonitrile (3.00 ml) was stirred for 2 hours at 50°C. The reaction mixture was concentrated. To the residue was added water, and the mixture was extracted with methylene chloride The organic layer was washed with water, dried over anhydrous magnesium sulfate and was concentrated. The residue was washed with n-hexane-ethyl acetate and dried to give the compound of the present invention (109 mg) having the following physical data.
TLC:RF 0.19 (ethyl acetate : n-hexane = 3 : 7);
NMR (CDCl₃):δ 9.82 (brs, 1H), 7.69 (dd, J = 8.1, 1.8 Hz, 1H), 7.49 (td, J = 7.8, 1.5 Hz, 1H), 7.40-7.33 (m, 1H), 7.26 (d, J = 7.8 Hz, 1H), 7.20 (d, J = 8.1 Hz, 1H), 7.11-7.05 (m, 2H), 5.87 (d, J = 0.9 Hz, 2H).

### Example 5:

### 1-(2-chloro-6-fluorobenzyl)-5-(2-hydroxymethylphenyl)-1H-1,2,3,4-tetrazole

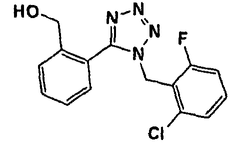

The solution of the compound prepared in Example 2(31) (38.8 mg) and cerium ammonium nitrate (250 mg) in acetonitrile (3.00 ml)-water (1.00 ml) was stirred for 1 hour at room temperature. The reaction mixture was extracted with methylene chloride, and the organic layer was washed with water and brine successively, dried over magnesium suldate and concentrated. The residue was purified by column chromatography on silica gel (n-hexane : ethyl acetate = 7 : 3) to give the compound of the present invention (22.0 mg) having the following physical data.
TLC:Rf 0.57 (ethyl acetate : n-hexane = 1 : 1);
NMR (CDCl₃):δ 7.61-7.45 (m, 4H), 7.34-7.26 (m, 1H), 7.19 (d, J = 7.8 Hz, 1H), 7.00 (td, J = 8.7, 1.2 Hz, 1H), 5.69 (d, J = 1.5 Hz, 2H), 4.42 (d, J = 6.9 Hz, 2H), 3.77 (t, J = 6.9 Hz, 1H).

### Example 6:

### 1-(5-chloro-2-hydroxybenzyl)-5-(2,3-dichlorophenyl)-1H-1,2,3,4-tetrazole

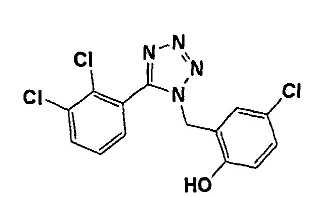

To a solution of the compound prepared in Example 2(66) (1.43 g) in methylene chloride (10 ml) under cooling with ice in dry ice-methanol bath, was added tribromoboron (1M methylene chloride solution, 9.7 ml) and the mixture was stirred for 2 hours. To the reaction mixture was added a saturated aqueous solution of sodium bicarbonate, and extracted with ethyl acetate. The organic layer was washed with brine, dried over anhydrous sodium sulfate and was concentrated. The residue was purified by column chromatography on silica gel (n-hexane : ethyl acetate = 2 : 1 → 1 : 1) and recrystallization (n-hexane / ethyl acetate), to give the compound of the present invention having the following physical data.
TLC:Rf0.33 (ethyl acetate : n-hexane = 1 : 1);
NMR (CDCl₃ + CD₃OD):δ 7.66 (dd, J = 7.8 Hz, 1 5 Hz, 1H), 7.29 (t, J = 7.8 Hz, 1H), 7.15 (dd, J = 7.8 Hz, 1.5 Hz, 1H), 7.07 (dd, J = 8.7 Hz, 2.7 Hz, 1H), 6.93 (d, J = 2.7 Hz, 1H), 6.62 (d, J = 8.7 Hz, 1H), 5.40 (s, 2H).

### Example 6(1)-Example 6(2)

By the same procedure as described in example 6 using corresponding compounds, the compounds of the present invention having the following physical data were given.

### Example 6(1):

### 1-(4-fluoro-2-trifluoromethylbenzyl)-5-(2-hydroxy-3-methoxyphenyl)-1H-1,2,3,4-tetrazole

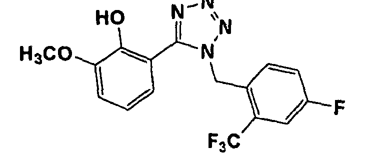

TLC:Rf 0.20(ethyl acetate : n-hexane = 1 : 2);
NMR (CDCl₃):δ 7.42-7.38 (m, 2H), 7.22-7.15 (m, 1H), 7.03 (dd, J = 7.5 Hz, 1.8 Hz, 1H), 7.00-6.90 (m, 3H), 5.82 (s, 2H), 3.95 (s, 3H).

### Example 6(2):

### 1-(2-chloro-4-hydroxybenzyl)-5-(2,3-dichlorophenyl)-1H-1,2,3,4-tetrazole

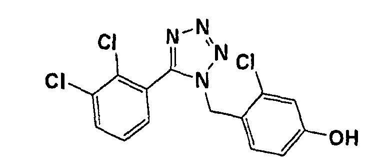

TLC:Rf 0.21 (ethyl acetate : n-hexane = 1 : 2);
NMR (CDCl₃+CD₃OD):δ 7.66 (dd, J = 8.1 Hz, 1.8 Hz, 1H), 7.30 (t, J = 8.1 Hz, 1H), 7.16 (dd, J = 8.1 Hz, 1.8 Hz, 1H), 7.00 (d, J = 8.7 Hz, 1H), 6.74 (d, J = 2.4 Hz, 1H), 6.62 (dd, J = 8.7 Hz, 2.4 Hz, 1H), 5.50 (s, 2H).

### Example 7:

### 1-(2-benzyloxy-5-chlorobenzyl)-5-(2-chloro-4-hydroxyphenyl)-1H-1,2,3,4-tetrazole

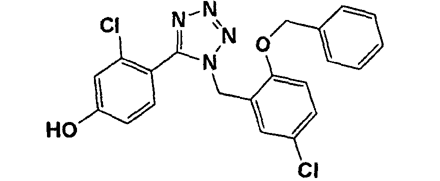

To a solution of the compound prepared in Example 2(104) (342 mg) in methanol (5 ml)-tetrahydrofuran (5 ml) was added 2N hydrochloric acid (2 ml) and the mixture was stirred for 3 hour at 80°C. To the reaction mixture was added water, the precipitate was collected by filtration, dried and recrystallized by n-hexane-ethyl acetate, the compound of the present invention having the following physical data (249 mg) was given.
TLC:Rf 0.20 (ethyl acetate : n-hexane = 1 : 2);
NMR (CDCl₃+CD₃OD) :δ 7.40-7.32 (m, 3H), 7.22-7.13 (m, 3H), 6.99 (d, J = 2.7 Hz, 1H), 6.93 (d, J = 2.4 Hz, 1H), 6.79 (d, J = 8.4 Hz, 1H), 6.78 (d, J = 9.0 Hz, 1H), 6.67 (dd, J = 8.4 Hz, 2.4 Hz, 1H), 5.43 (s, 2H), 4.91 (s, 2H).

### Example 8:

### 5-(2,6-dimethoxyphenoxy)-1-phenyl-1H-1,2,3,4-tetrazole

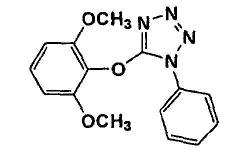

To a solution of 5-chloro-1-phenyl-1H-1,2,3,4-tetrazole (542 mg) in acetone (10.0 ml) were added potassium carbonate (622 mg) and 2,6-dimethoxyphenol (463 mg) and the mixture was refluxed for 10 hours. The reaction mixture was concentrated. To the residue was water added and the mixture was extracted with ethyl acetate. The organic layer was washed with a saturated aqueous ammonium chloride and brine, dried over anhydrous magnesium sulfate and was concentrated. The residue was purified by column chromatography on silica gel (n-hexane : ethyl acetate = 8 : 2 → 1 : 1) to give the compound of the present invention(338 mg) having the following physical data.
TLC:Rf 0.29 (n-hexane : ethyl acetate = 7 : 3);
NMR (CDCl₃):δ 7.91-7.88 (m, 2H), 7.60-7.45 (m, 3H), 7.26-7.19 (m, 1H), 6.67 (d, J = 8.4 Hz, 2H), 3.82 (s, 6H).

### Example 8(1):

### 5-phenoxy-1-phenyl-1H-1,2,3,4-tetrazole

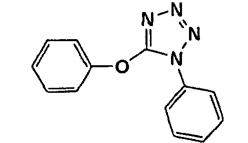

By the same procedure as described in example 8 using a corresponding compound, the compound of the present invention having the following physical data was given.
TLC:Rf 0.29 (n-hexane : ethyl acetate = 5 : 1);
NMR (CDCl₃):δ 7.83-7.80 (m, 2H), 7.62-7.29 (m, 8H).

### Example 9(1)-Example 9(3)

By the same procedure as described in example 5 using a corresponding compound, the compounds of the present invention having the following physical data were given.

### Example 9(1):

### N-(2-chloro-6-fluorobenzyl)-2-hydroxymethylbenzamide

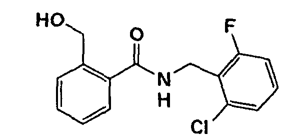

TLC:Rf 0.15 (ethyl acetate : n-hexane = 3 : 7).
NMR (CDCl₃):δ 7.53-7.23 (m, 6H), 7.09-7.03 (m, 1H), 6.61 (brs, 1H), 4.84 (dd, J = 5.7, 1.5 Hz, 2H), 4.61 (d, J = 6.9 Hz, 2H), 4.23 (t, J = 6.9 Hz, 1H).

### Example 9(2):

### N-(2-chloro-6-hydroxybenzyl)-2,3-dichlorobenzamide

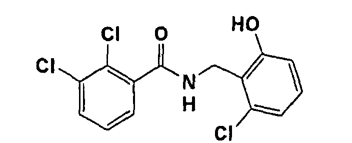

TLC:Rf0.40 (ethyl acetate : n-hexane = 1 : 2);
NMR (CDCl₃):δ 9.43 (s, 1H), 7.59-7.52 (m, 2H), 7.27 (t, J = 7.8 Hz, 1H), 7.22-7.13 (m, 2H), 6.98-6.92 (m, 2H), 4.71 (d, J = 6.6 Hz, 2H).

### Example 9(3):

### N-(4-fluoro-2-trifluoromethylbenzyl)-2-hydroxy-3-methoxybenzamide

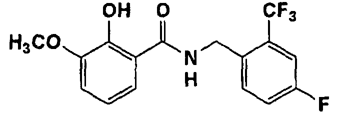

TLC:Rf 0.25(ethyl acetate : n-hexane = 1 : 3);
NMR (CDCl₃):δ 11.05 (s, 1H), 7.66 (dd, J = 8.4 Hz, 5.7 Hz, 1H), 7.39 (dd, J = 8.7 Hz, 2.7 Hz, 1H), 7.28-7.20 (m, 1H), 7.12 (dd, J = 8.1 Hz, 1.2 Hz, 1H), 7.10-7.02 (m, 1H), 6.99 (dd, J = 8.1 Hz, 1.2 Hz, 1H), 6.83 (t, J = 8 1 Hz, 1H), 4.79 (d, J = 6.0 Hz, 2H), 3.91 (s, 3H).

### Example 10(1)-Example 10(34)

By the same procedure as described in example 2 using a corresponding compound (optionally followed by converting into a corresponding salt by a conventional method), the following compounds having the following physical data were given.

### Example 10(1):

### 1-[5-chloro-2-(2,2-dimethyl-1,3-dioxolan-4-ylmethyloxy)benzyl]-5-(2,3-dichlorophenyl)-1H-1,2,3,4-tetrazole

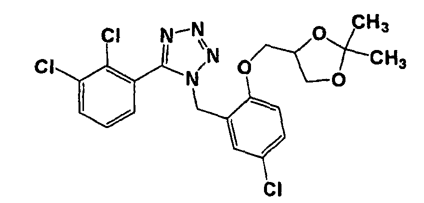

TLC:Rf 0.46 (n-hexane : ethyl acetate = 3 : 2);
NMR (CDCl3):δ 7.66 (dd, J = 7.8, 1.5 Hz, 1H), 7.27 (t, J = 7.8 Hz, 1H), 7.19 (dd, J = 8.7, 2.4 Hz, 1H), 7.12 (dd, J = 7.8, 1.5 Hz, 1H), 6.80 (d, J = 2.4 Hz, 1H), 6.71 (d, J = 8.7 Hz, 1H), 5.49 (d, J = 15.0 Hz, 1H), 5.46 (d, J = 15.0 Hz, □1H), 4.29-4.22 (m, 1H), 4.09 (dd, J = 8.7, 6.3 Hz, 1H), 3.89 (dd, J = 9.6, 5.7 Hz, 1H), 3.78 (dd, J = 9.6, 6.3 Hz, 1H), 3.70 (dd, J = 8.7, 5.4 Hz, 1H), 1.40 (s, 3H), 1.37 (s, 3H).

### Example 10(2):

### 1-[5-chloro-2-(4-dimethylaminobenzyloxy)benzyl]-5-(2,3-dichlorophenyl)-1H-1,2,3,4-tetrazole

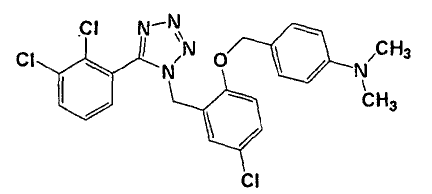

TLC:Rf 0.44 (n-hexane : ethyl acetate = 2 : 1);
NMR (CDCl₃):δ 7.57 (dd, J = 7.8, 1.5 Hz, 1H), 7.20 (dd, J = 8.7, 2.4 Hz, 1H), 7.09 (t, J = 7.8 Hz, 1H), 7.02 (d, J = 2.4 Hz, 1H), 7.01-6.96 (m, 2H), 6.80 (d, J = 8.7 Hz, 1H), 6.76 (dd, J = 7.8, 1.5 Hz, 1H), 6.71-6.66 (m, 2H), 5.35 (s, 2H), 4.75 (s, 2H), 2.97 (s, 6H).

### Example 10(3):

### 1-[5-chloro-2-(2-morpholinoethyloxy)benzyl]-5-(2,3-dichlorophenyl)-1H-1,2,3,4-tetrazole hydrochloride

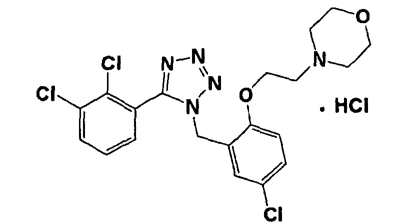

TLC:Rf0.42 (chloroform : methanol=19: 1);
NMR (DMSO-d₆):δ 11.42-11.26 (br, 1H), 7.86 (dd, J = 7.8, 1.5 Hz, 1H), 7.61 (dd, J = 7.8, 1.5 Hz, 1H), 7.53 (t, J = 7.8 Hz, 1H), 7.33 (dd, J = 8.7, 2.7 Hz, 1H), 6.97 (d, J = 8.7 Hz, 1H), 6.86 (d, J = 2.7 Hz, 1H), 5.63 (s, 2H), 4.36-4.26 (br, 2H), 3.93-3.75 (m, 4H), 3.38-3.25 (m, 4H), 3.16-3.01 (br, 2H).

### Example 10(4):

### 1-(2-benzyloxy-5-chlorobenzyl)-5-(2,4-dichlorophenyl)-1H-1,2,3,4-tetrazole

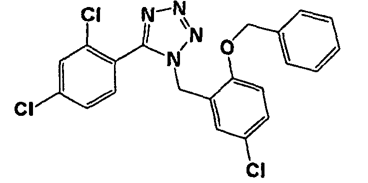

TLC:Rf 0.58 (n-hexane : ethyl acetate = 2 : 1);
NMR (CDCl₃):δ 7.44 (d, J = 2.1 Hz, 1H), 7.41-7.35 (m, 3H), 7.22 (dd, J = 8.7, 2.4 Hz, 1H), 7.17-7.12 (m, 3H), 7.05 (d, J = 2.4 Hz, 1H), 6.80 (d, J = 8.1 Hz, 1H), 6.78 (d, J = 8.7 Hz, 1H), 5.42 (s, 2H), 4.86 (s, 2H).

### Example 10(5):

### 1-[5-chloro-2-(3-pyridylmethyloxy)benzyl]-5-(2,3-dichlorophenyl)-1H-1,2,3,4-tetrazole

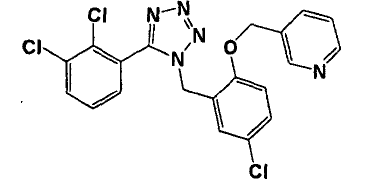

TLC:Rf 0.40 (chloroform : methanol=19 : 1);
NMR (CDCl₃):δ 8.62 (dd, J = 5.1, 1.5 Hz, 1H), 8.52 (d, J = 1.5 Hz, 1H), 7.59 (dd, J = 7.8, 1.5 Hz, 1H), 7.53-7.50 (m, 1H), 7.37-7.33 (m, 1H), 7.23 (dd, J = 8.7, 2.4 Hz, 1H), 7.19 (t, J = 7.8 Hz, 1H), 6.96 (d, J = 2.4 Hz, 1H), 6.92 (dd, J = 7.8, 1.5 Hz, 1H), 6.79 (d, J = 8.7 Hz, 1H), 5.45 (s, 2H), 4.92 (s, 2H).

### Example 10(6):

### 1-[5-chloro-2-(2-pyridylmethyloxy)benzyl]-5-(2,3-dichlorophenyl)-1H-1,2,3,4-tetrazole

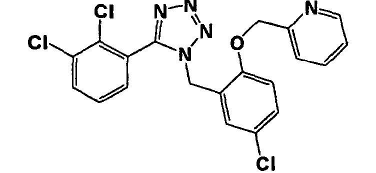

TLC:Rf 0.23 (n-hexane : ethyl acetate = 3 : 2);
NMR (CDCl₃):δ 8.58-8.55 (m, 1H), 7.72 (dt, J = 7.8, 1.8 Hz, 1H), 7 60 (dd, J = 7.8, 1.5 Hz, 1H), 7.25-7.13 (m, 4H), 7.01 (dd, J = 7.8, 1.5 Hz, 1H), 6.93 (d, J = 3.0 Hz, 1H), 6.75 (d, J = 7.8 Hz, 1H), 5.54 (s, 2H), 5.02 (s, 2H).

### Example 10(7):

### 1-(2-benzyloxy-5-chlorobenzyl)-5-(2-chloro-3-nitrophenyl)-1H-1,2,3,4-tetrazole

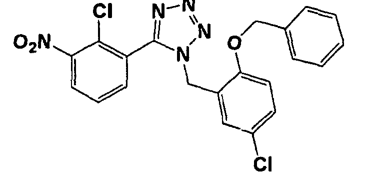

TLC:Rf 0.29 (n-hexane : ethyl acetate = 2 : 1);
NMR (CDCl₃):δ 7 88 (dd, J = 8.4, 1.8 Hz, 1H), 7.40-7.34 (m, 3H), 7.26 (t, J = 8.4 Hz, 1H), 7.23 (dd, J = 8.7, 2.4 Hz, 1H), 7.15-7.09 (m, 3H), 7.02 (dd, J = 8.4, 1.8 Hz, 1H), 6.80 (d, J = 8.7 Hz, 1H), 5.45 (s, 2H), 4.87 (s, 2H)

### Example 10(8):

### 1-(2-benzyloxy-5-chlorobenzyl)-5-(2,5-dichlorophenyl)-1H-1,2,3,4-tetrazole

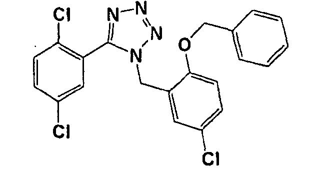

TLC:Rf 0.56 (n-hexane : ethyl acetate = 2: 1);
NMR (CDCl₃):δ 7.41-7.32 (m, 5H), 7.22 (dd, J = 9.0, 2.4 Hz, 1H), 7.14-7.10 (m, 2H), 7.06 (d, J = 2.4 Hz, 1H), 6.83 (dd, J = 2.1, 0.9 Hz, 1H), 6.79 (d, J = 9.0 Hz, 1H), 5.45 (s, 2H), 4.89 (s, 2H).

### Example 10(9):

### 1-(2-benzyloxy-5-chlorobenzyl)-5-(2,6-dichlorophenyl)-1H-1,2,3,4-tetrazole

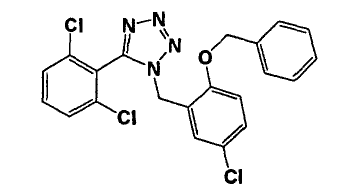

TLC:Rf0.47 (n-hexane : ethyl acetate = 2 : 1);
NMR (CDCl₃):δ 7.40-7.27 (m, 6H), 7.23-7.19 (m, 2H), 7.16 (dd, J = 9.0, 2.7 Hz, 1H), 6.83 (d, J = 2.7 Hz, 1H), 6.76 (d, J = 9.0 Hz, 1H), 5.51 (s, 2H), 4.92 (s, 2H).

### Example 10(10):

### 1-[5-chloro-2-(N-methyl-2,3,4,5,6,7-hexahydroazepine-3-yl)benzyl]-5-(2,3-dichlorophenyl)-1H-1,2,3,4-tetrazole hydrochloride

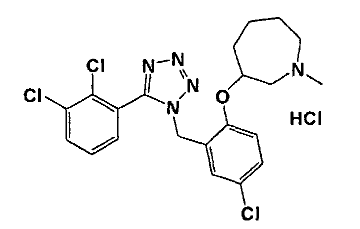

TLC:Rf 0.29 (chloroform : methanol=19 : 1);
NMR (CD₃OD):δ 7.81 (dd, J = 7.8, 1.5 Hz, 1H), 7.47 (t, J = 7.8 Hz, 1H), 7.35 (dd, J = 7.8, 1.5 Hz, 1H), 7.26 (dd, J = 9.0, 2 7 Hz, 1H), 6 96 (d, J = 9.0 Hz, 1H), 6.63 (bs, 1H), 5.77-5.61 (m, 2H), 4.89-4.81 (m, 1H), 3.70-3.38 (m, 4H), 3 09 (bs, 3H), 2 05-1.91 (m, 4H), 1.73-1.65 (m, 2H)

### Example 10(11):

### 1-[5-chloro-2-(N-methylpiperidin-2-ylmethyloxy)benzyl]-5-(2,3-dichlorophenyl)-1H-1,2,3,4-tetrazole hydrochloride

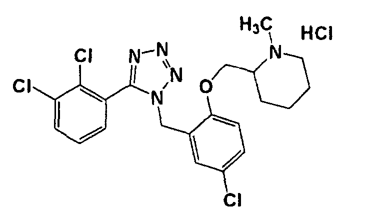

TLC:Rf0.23 (chloroform : methanol = 19: 1);
NMR (CD₃OD):δ 7.80 (dd, J = 7.8, 1.5 Hz, 1H), 7.47 (t, J = 7.8 Hz, 1H), 7.35 (dd, J = 7.8, 1.5 Hz, 1H), 7.28 (dd, J = 8.7, 2.7 Hz, 1H), 6.97 (d, J = 8.7 Hz, 1H), 6.67 (d, J = 2.7 Hz, 1H), 5 92-5.80 (br, 1H), 5.67-5.54 (br, 1H), 4.40-4.24 (br, 1H), 4.20-4.06 (br, 1H), 3.64-3.34 (br, 2H), 3.26-3.12 (br, 1H), 2.93 (s, 3H), 2.10-1.55 (br, 6H).

### Example 10(12):

### 1-(2-benzyloxy-5-chlorobenzyl)-5-(3-chloro-2-methylphenyl)-1H-1,2,3,4-tetrazole

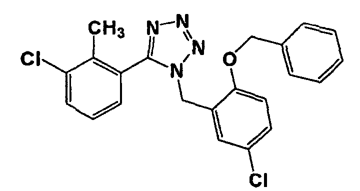

TLC:Rf 0.54 (n-hexane : ethyl acetate = 2 : 1);
NMR (CDCl₃):δ 7.49 (dd, J = 8.1, 1.2 Hz, 1H), 7.40-7.32 (m, 3H), 7.22 (dd, J = 8.7, 3.0 Hz, 1H), 7.20-7.16 (m, 2H), 7.11 (t, J = 8.1 Hz, 1H), 6.91 (dd, J = 8.1, 1.2 Hz, 1H), 6.88 (d, J = 3 0 Hz, 1H), 6.80 (d, J = 8.7 Hz, 1H), 5.38 (s, 2H), 4.91 (s, 2H), 1.97 (s, 3H).

### Example 10(13):

### 1-[5-chloro-2-(2-methylphenylamino)ethyloxybenzyl]-5-(2,3-dichlorophenyl)-1H-1,2,3,4-tetrazole

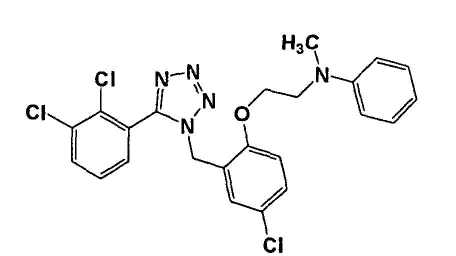

TLC:Rf 0.27 (n-hexane : ethyl acetate = 3 : 1);
NMR (CDCl₃):δ 7.63 (dd, J = 8.1, 1.5 Hz, 1H), 7.25-7.18 (m, 3H), 7.15 (dd, J = 8.7, 2.7 Hz, 1H), 7.05 (dd, J = 7.8, 1.5 Hz, 1H), 6.78 (d, J = 2.7 Hz, 1H), 6.73-6.63 (m, 4H), 5.35 (s, 2H), 3.98 (t, J = 5.7 Hz, 2H), 3.63 (t, J = 5.7 Hz, 2H), 2.95 (s, 3H).

### Example 10(14):

### 1-(2-benzyloxy-5-chlorobenzyl)-5-(3-methoxymethoxy-2-methylphenyl)-1H-1,2,3,4-tetrazole

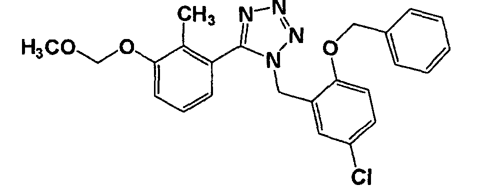

TLC:Rf 0.53 (ethyl acetate : n-hexane = 1 : 2);
NMR (CDCl₃) 7.40-7.29 (m, 3H), 7.23-7.10 (m, 5H), 6.81 (d, J = 3.0 Hz, 1H), 6.78 (d, J = 8.7 Hz, 1H), 6.70 (dd, J = 7.2 Hz, 1.5 Hz, 1H), 5.40 (s, 2H), 5.21 (s, 2H), 4.94 (s, 2H), 3.48 (s, 3H), 1.88 (s, 3H).

### Example 10(15):

### 1-[5-chloro-2-(tetrahydropyran-2-ylmethyloxy)-benzyl]-5-(2,3-dichlorophenyl)-1H-1,2,3,4-tetrazole

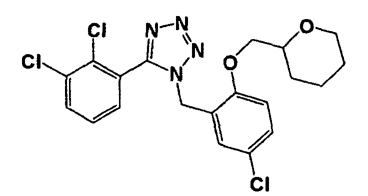

TLC:Rf 0.38 (n-hexane : ethyl acetate = 2 : 1);
NMR (CDCl₃):δ 7.63 (dd, J = 7.8, 1.5 Hz, 1H), 7.24 (t, J = 7.8 Hz, 1H), 7.18-7.11 (m, 2H), 6.87 (d, J = 2.7 Hz, 1H), 6.68 (d, J = 8.7 Hz, 1H), 5.52 (d, J = 15.0 Hz, 1H), 5.46 (d, J = 15.0 Hz, 1H), 3.98-3.93 (m, 1H), 3.80 (dd, J = 9.9, 6.3 Hz, 1H), 3.67 (dd, J = 9.9, 4.2 Hz, 1H), 3.47-3.37 (m, 2H), 1.91-1.85 (m, 1H), 1.60-1.49 (m, 4H), 1.30-1.20 (m, 1H).

### Example 10(16):

### 1-(2-benzyloxy-5-chlorobenzyl)-5-(4-methoxymethoxyphenyl)-1H-1,2,3,4-tetrazole

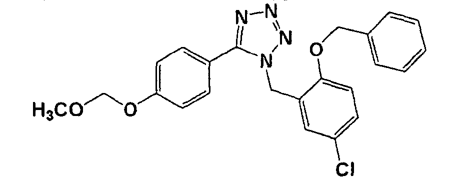

TLC:Rf 0.47 (ethyl acetate : n-hexane = 1 : 2);
NMR (CDCl₃):δ 7.52-7.46 (m, 2H), 7.40-7.32 (m, 3H), 7.28-7.22 (m, 3H), 7.09-7.03 (m, 2H), 6.94 (d, J = 2.7 Hz, 1H), 6.89 (d, J = 8.7 Hz, 1H), 5.59 (s, 2H), 5.20 (s, 2H), 5.02 (s, 2H), 3.48 (s, 3H).

### Example 10(17):

### 1-(2-benzyloxy-5-chlorobenzyl)-5-(4-chlorophenyl)-1H-1,2,3,4-tetrazole

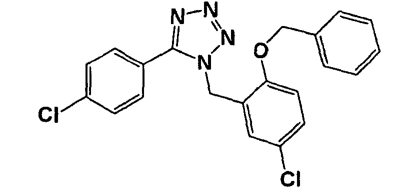

TLC:Rf 0.49 (ethyl acetate : n-hexane = 1 : 2);
NMR (CDCl₃):δ 7.48-7.41 (m, 2H), 7.39-7.32 (m, 5H), 7.28 (dd, J = 9.0, 2.4 Hz, 1H), 7.24-7.18 (m, 2H), 7.03 (d, J = 2.4 Hz, 1H), 6.90 (d, J = 9.0 Hz, 1H), 5.56 (s, 2H), 4.98 (s, 2H).

### Example 10(18):

### 5-(2,3-dichlorophenyl)1-(2-phenethyloxybenzyl)-1H-1,2,3,4-tetrazole

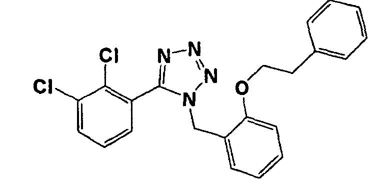

TLC:Rf 0.32 (n-hexane : ethyl acetate = 3 : 1);
NMR (CDCl₃):δ 7.59 (dd, J = 7.8, 1.5 Hz, 1H), 7.33-7.15 (m, 7H), 6.97 (dd, J = 7.8, 1.5 Hz, 1H), 6.92 (dd, J = 7.8, 1.5 Hz, 1H), 6.79 (dt, J = 7.8, 1.2 Hz, 1H), 6.71 (d, J = 8.1 Hz, 1H), 5.46 (s, 2H), 4.00 (t, J = 6.9 Hz, 2H), 2.91 (t, J = 6.9 Hz, 2H).

### Example 10(19):

### 1-(2-cyclohexylmethyloxybenzyl)-5-(2,3-dichlorophenyl)-1H-1,2,3,4-tetrazole

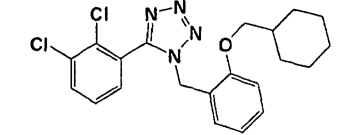

TLC:Rf 0.36 (n-hexane : ethyl acetate = 4 : 1);
NMR (CDCl₃):δ 7.58 (dd, J = 7.8, 1.5 Hz, 1H), 7.23-7.17 (m, 2H), 7.03 (dd, J = 7 8, 1.5 Hz, 1H), 6.93 (dd, J = 7.8, 1.5 Hz, 1H), 6.79-6.69 (m, 2H), 5.54 (s, 2H), 3.57 (d, J = 6.3 Hz, 2H), 1.76-1.51 (m, 6H), 1.34-1.11 (m, 3H), 0.96-0.85 (m, 2H).

### Example 10(20):

### 5-(2,3-dichlorophenyl)-1-[2-(4-pyridylmethyloxy)benzyl]-1H-1,2,3,4-tetrazole

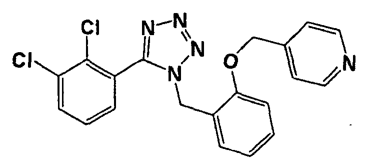

TLC:Rf 0.33 (chloroform : methanol=19 : 1);
NMR (CDCl₃):δ 8.63-8.60 (m, 2H), 7.55 (dd, J = 8.1, 1.8 Hz, 1H), 7.28-7.22 (m, 1H), 7.18-7.13 (m, 3H), 7.04 (dd, J = 7.8, 1.8 Hz, 1H), 7.00 (dd, J = 7.8, 1.8 Hz, 1H), 6.88 (dt, J = 7.8, 1.2 Hz, 1H), 6.75 (d, J = 8.1 Hz, 1H), 5.57 (s, 2H), 4.93 (s, 2H).

### Example 10(21):

### 5-(2,3-dichlorophenyl)-1-[2-(3-pyridylmethyloxy)benzyl]-1H-1,2,3,4-tetrazole

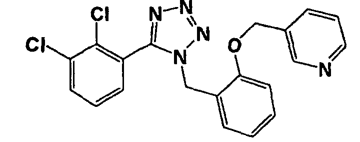

TLC:Rf 0.33 (chloroform : methanol=19 : 1);
NMR (CDCl₃):δ 8.61 (dd, J = 4.5, 1.5 Hz, 1H), 8.53 (d, J = 2.1 Hz, 1H), 7.57-7.51 (m, 2H), 7.37-7.25 (m, 2H), 7.14 (t, J = 7.8 Hz, 1H), 7.03 (dd, J = 7.5, 1.8 Hz, 1H), 6.92-6.84 (m, 3H), 5.50 (s, 2H), 4.93 (s, 2H).

### Example 10(22):

### 1-(2,3-dichlorophenyl)-5-[2-(2-morpholinoethyloxy)benzyl]-1H-1,2,3,4-tetrazole hydrochloride

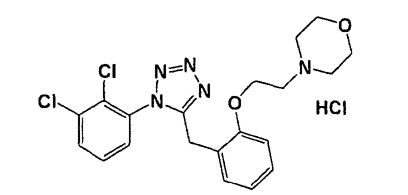

TLC:Rf 0.48 (methanol: ethyl acetate = 1 : 19);
NMR (CD₃OD):δ 7.88 (dd, J = 8.1 Hz, 1.5 Hz, 1H), 7.56 (t, J = 8.1 Hz, 1H), 7.48 (dd, J = 8.1 Hz, 1.5 Hz, 1H), 7.28-7.21 (m, 1H), 6.96 (d, J = 8.1 Hz, 1H), 6.75 (t, J = 7.5 Hz, 1H), 6.61 (dd, J = 7.5 Hz, 1 8 Hz, 1H), 4.42-4.36 (m, 2H), 4.31 (s, 2H), 4.18-4.10 (m, 4H), 3.80-3.30 (m, 6H).

### Example 10(23):

### 1-[2-(2H,3H-benzo[e]1,4-dioxan-2-ylmethyloxy)benzyl]-5-(2,3-dichlorophenyl)-1H-1,2,3,4-tetrazole

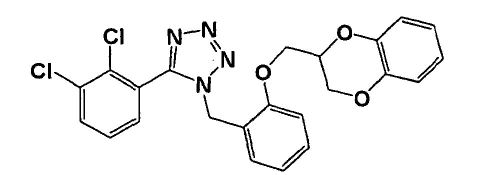

TLC:Rf 0.35 (n-hexane : ethyl acetate = 2 : 1);
NMR (CDCl₃):δ 7.57 (dd, J = 7.8, 1.5 Hz, 1H), 7.27-7.21 (m, 1H), 7.17 (t, J = 7.8 Hz, 1H), 7.10 (dd, J = 7.8, 1.5 Hz, 1H), 6.94-6.76 (m, 7H), 5.54 (d, J = 14.7 Hz, 1H), 5.52 (d, J = 14.7 Hz, 1H), 4.42-4.35 (m, 1H), 4.26 (dd, J = 11.4, 2.4 Hz, 1H), 4.12-4.00 (m, 3H).

### Example 10(24):

### 5-(2,3-dichlorophenyl)-1-[2-(2-morpholinoethyloxy)benzyl]-1H-1,2,3,4-tetrazole hydrochloride

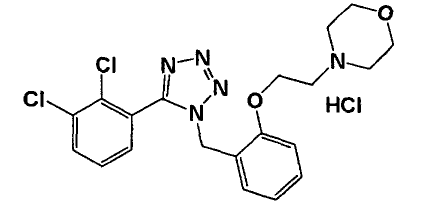

TLC:Rf 0.34 (methanol: ethyl acetate = 1 : 49);
NMR (CD₃OD):δ 7.77 (dd, J = 8.1 Hz, 1.5 Hz, 1H), 7.44 (t, J = 8.1 Hz, 1H), 7.36-7.26 (m, 2H), 6.98 (d, J = 8.1 Hz, 1H), 6.77 (dt, J = 1.2Hz, 7.5 Hz, 1H), 6.71 (dd, J = 7.5 Hz, 1.8 Hz, 1H), 5.71 (s, 2H), 4.39-4.32 (m, 2H), 4.12-3.90 (m, 4H), 3.60-3.34 (m, 6H).

### Example 10(25):

### 5-(2,3-dichlorophenyl)-1-[2-(2-pyridylmethyloxy)benzyl]-1H-1,2,3,4-tetrazole

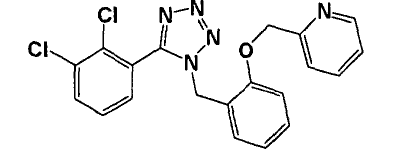

TLC:Rf 0.22 (ethyl acetate : n-hexane = 3 : 2);
NMR (CDCl₃):δ 8.59-8.55 (m, 1H), 7.71 (dt, J = 1.8 Hz, 7.8 Hz, 1H), 7.55 (dd, J = 8.1 Hz, 1.8Hz, 1H), 7.27-7.12 (m, 4H), 7.04-6.97 (m, 2H), 6.87-6.78 (m, 2H), 5.60 (s, 2H), 5.03 (s, 2H).

### Example 10(26):

### 1-(2-chloro-6-phenoxybenzyl)-5-(2,3-dimethylphenyl)-1H-1,2,3,4-tetrazole

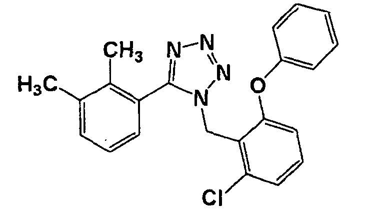

TLC:Rf 0.50 (ethyl acetate : n-hexane = 1 : 3);
NMR (CDCl₃):δ 7.38-7.28 (m, 3H), 7.21-7.04 (m, 5H), 6.90-6.84 (m, 2H), 6.65 (dd, J = 8.2, 1.1 Hz, 1H), 5.57 (s, 2H), 2.30 (s, 3H), 2.01 (s, 3H).

### Example 10(27):

### 1-(2-benzyloxybenzyl)-5-(2,3-dimethylphenyl)-1H-1,2,3,4-tetrazole

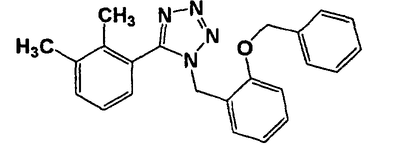

TLC:Rf 0.40 (ethyl acetate : n-hexane = 1 : 3);
NMR (CDCl₃):δ 7.39-7.30 (m, 3H), 7.30-7.18 (m, 4H), 7.08 (t, J = 7.5 Hz, 1H), 6.91-6.82 (m, 4H), 5.43 (s, 2H), 4.93 (s, 2H), 2.24 (s, 3H), 1.84 (s, 3H).

### Example 10(28):

### 5-(2,3-dichlorophenyl)-1-[2-(2,2-dimethyl-1,3-dioxolan-4-ylmethyloxy)benzyl]-1H-1,2,3,4-tetrazole

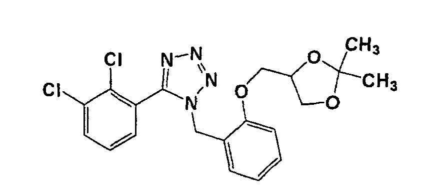

TLC:Rf 0.36 (n-hexane : ethyl acetate = 3 : 2);
NMR (CDCl₃):δ 7.61 (dd, J = 8.1, 1.5 Hz, 1H), 7.26-7.19 (m, 2H), 7.09 (dd, J = 7.8, 1.5 Hz, 1H), 6.87 (dd, J = 7.5, 2.1 Hz, 1H), 6.81 (dd, J = 7.5, 1.2 Hz, 1H), 6.76 (d, J = 8.1 Hz, 1H), 5.54 (d, J = 14.7 Hz, 1H), 5.51 (d, J = 14.7 Hz, 1H), 4.30-4.22 (m, 1H), 4.10 (dd, J = 8.4, 6.3 Hz, 1H), 3.91 (dd, J = 9.6, 5.4 Hz, 1H), 3.78 (dd, J = 9.6, 6.3 Hz, 1H), 3.71 (dd, J = 8.4, 5.4 Hz, 1H), 1.41 (s, 3H), 1.38 (s, 3H).

### Example 10(29):

### 5-(2,3-dichlorophenyl)-1-[2-[2-(N-methyl-N-phenylamino)ethyloxy]benzyl]-1H-1,2,3,4-tetrazole

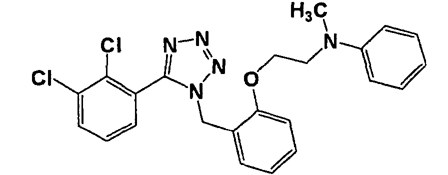

TLC:Rf 0.43 (n-hexane : ethyl acetate = 2 : 1);
NMR (CDCl₃):δ 7.59 (dd, J = 8.1, 1.5 Hz, 1H), 7.25-7.15 (m, 4H), 7.02 (dd, J = 7.5, 1.5 Hz, 1H), 6.85 (dd, J =7.5, 2.1 Hz, 1H), 6.78 (dt, J = 7.5, 0.9 Hz, 1H), 6.73-6.67 (m, 4H), 5.42 (s, 2H), 3.99 (t, J = 5.7 Hz, 2H), 3.63 (t, J = 5.7 Hz, 2H), 2.96 (s, 3H).

### Example 10(30):

### 1-(2-cyclohexyloxybenzyl)-5-(2,3-dichlorophenyl)-1H-1,2,3,4-tetrazole

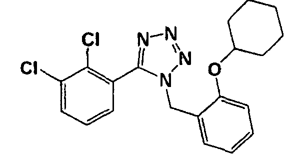

TLC:Rf0.44 (n-hexane : ethyl acetate = 3 : 1);
NMR (CDCl₃):δ 7.58 (dd, J = 7.8, 1.5 Hz, 1H), 7.21-7.14 (m, 2H), 7.04 (dd, J = 7.8, 1.5 Hz, 1H), 6.87 (dd, J = 7.5, 1.5 Hz, 1H), 6.75-6.68 (m, 2H), 5.54 (s, 2H), 4.18-4.09 (m, 1H), 1.81-1.48 (m, 5H), 1.37-1.22 (m, 5H).

### Example 10(31):

### 1-(2-benzyloxyphenethyl)-5-(2,3-dimethylphenyl)-1H-1,2,3,4-tetrazole

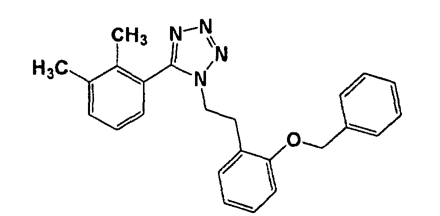

TLC:Rf 0.52 (ethyl acetate : n-hexane = 1 : 2);
NMR (CDCl₃):δ 7.39-7.30 (m, 3H), 7.30-7.22 (m, 1H), 7.20-7.14 (m, 3H), 7.03 (t, J = 7.8 Hz, 1H), 6.84-6.72 (m, 3H), 6.51 (d, J = 7.2 Hz, 1H), 4.72 (s, 2H), 4.44 (t, J = 6.6 Hz, 2H), 3.22 (t, J = 6.6 Hz, 2H), 2.25 (s, 3H), 1.81 (s, 3H).

### Example 10(32):

### 1-(4-benzyloxy-2-chlorobenzyl)-5-(2,3-dichlorophenyl)-1H-1,2,3,4-tetrazole

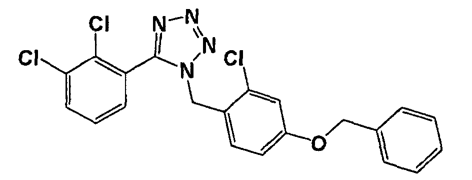

TLC:Rf 0.51(ethyl acetate: n-hexane = 1 : 2);
NMR (CDCl₃):δ 7.62 (dd, J = 8, 1 Hz, 1H), 7.44-7.32 (m, 5H), 7.25 (t, J = 8 Hz, 1H), 7.16 (dd, J = 8, 1 Hz, 1H), 7.08 (d, J = 9 Hz, 1H), 6.89 (d, J = 2 Hz, 1H), 6.77 (dd, J = 9, 2 Hz, 1H), 5.52 (s, 2H), 5.05 (s, 2H).

### Example 10(33):

### 1-[5-chloro-2-(4-methoxymethyloxybenzyloxy)benzyl]-5-(2,3-dichlorophenyl)-1H-1,2,3,4-tetrazole

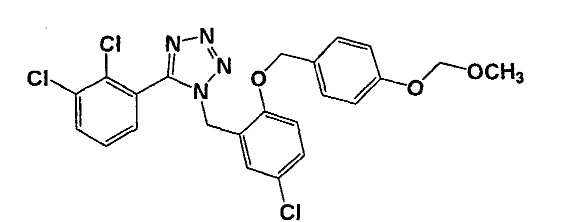

TLC:Rf 0.53(ethyl acetate : n-hexane = 1 : 2);
NMR (CDCl₃):δ 7.58 (dd, J = 7.8, 1.5 Hz, 1H), 7.19 (dd, J = 9.0, 2.7 Hz, 1H), 7.14 (t, J = 7.8 Hz, 1H), 7.09-6.98 (m, 5H), 6 82 (dd, J = 7.8, 1.5 Hz, 1H), 6.78 (d, J = 9.0 Hz, 1H), 5.39 (s, 2H), 5.19 (s, 2H), 4.81 (s, 2H), 3.49 (s, 3H).

### Example 10(34):

### 1-(2-benzyloxy-5-chlorobenzyl)-5-(2,3-dihydrobenzofuran-7-yl)-1H-1,2,3,4-tetrazole

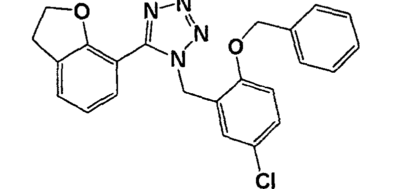

TLC:Rf 0.31 (n-hexane : ethyl acetate = 2 : 1);
NMR (CDCl₃):δ 7.38-7.29 (m, 4H), 7.20-7.11 (m, 4H), 7.01 (d, J = 2.7 Hz, 1H), 6.88 (t, J = 7.8 Hz, 1H), 6.76 (d, J = 8.7 Hz, 1H), 5.57 (s, 2H), 4.90 (s, 2H), 4.56 (t, J = 9.0 Hz, 2H), 3.23 (t, J = 9.0 Hz, 2H).

### Example 11(1)-Example 11(2)

By the same procedure as described in example 6 using the compound prepared in example 2(65) or example 2(86), the compounds of the present invention having the following physical data.

### Example 11(1):

### 5-(2,3-dichlorophenyl)-1-(2-hydroxybenzyl)-1H-1,2,3,4-tetrazole

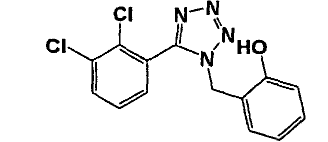

TLC:Rf 0.34 (n-hexane : ethyl acetate = 3 : 2);
NMR (CDCl₃):δ 7.64 (dd, J = 7.8, 1.5 Hz, 1H), 7.27 (t, J = 7.8 Hz, 1H), 7.18-7.13 (m, 2H), 7.00 (dd, J = 7.5, 1.8 Hz, 1H), 6.80 (dt, J = 7.8, 1.2 Hz, 1H), 6.74 (dd, J = 7.8, 1.2 Hz, 1H), 6.28 (s, 1H), 5.45 (s, 2H).

### Example 11(2):

### 1-(2,3-dichlorophenyl)-5-(2-hydroxybenzyl)-1H-1,2,3,4-tetrazole

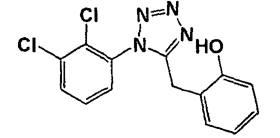

TLC:Rf 0.33 (ethyl acetate : n-hexane = 2:3);
NMR (CDCl₃+CD₃OD):δ 7.65 (dd, J = 8.1 Hz, 1.5 Hz, 1H), 7.30 (t, J = 8.1 Hz, 1H), 7.16 (dd, J = 8.1 Hz, 1.5 Hz, 1H), 7 09-7.02 (m, 1H), 6.91-6.86 (m, 1H), 6.73-6.64 (m, 2H), 4.16 (s, 2H).

### Example 12(1)-Example 12(2)

By the same procedure as described in example 7 using the compound prepared in example 10(14) or example 10(16), the compounds of the present invention having the following physical data were given.

### Example 12(1):

### 1-(2-benzyloxy-5-chlorobenzyl)-5-(3-hydroxy-2-methylphenyl)-1H-1,2,3,4-tetrazole

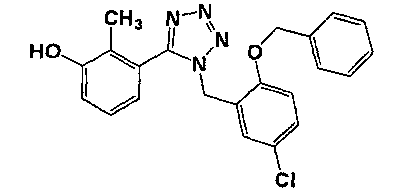

TLC:Rf 0.34 (ethyl acetate : n-hexane = 1 : 2);
NMR (CDCl₃+CD₃OD):δ 7.39-7.30 (m, 3H), 7.23-7.16 (m, 3H), 7.07-7.00 (m, 1H), 6.91 (dd, J = 8.4 Hz, 1.2 Hz, 1H), 6.84 (d, J = 2.4 Hz, 1H), 6.79 (d, J = 9.0 Hz, 1H), 6.57 (dd, J = 7.8 Hz, 1.2 Hz, 1H), 5.39 (s, 2H), 4.94 (s, 2H), 1.88 (s, 3H).

### Example 12(2):

### 1-(2-benzyloxy-5-chlorobenzyl)-5-(4-hydroxyphenyl)-1H-1,2,3,4-tetrazole

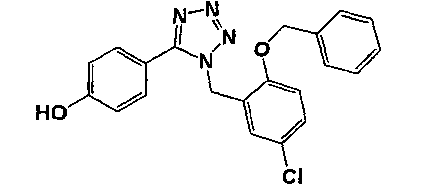

TLC:Rf 0.39 (ethyl acetate : n-hexane = 1 : 1);
NMR (CDCl₃+CD₃OD):δ 7.42-7.31 (m, 5H), 7.28-7.20 (m, 3H), 6.94 (d, J = 2.7 Hz, 1H), 6.92-6.83 (m, 3H), 5.58 (s, 2H), 5.02 (s, 2H).

### Example 13:

### 5-(3-amino-2-chlorophenyl)-1-(2-benzyloxy-5-chlorobenzyl)-1H-1,2,3,4-tetrazole

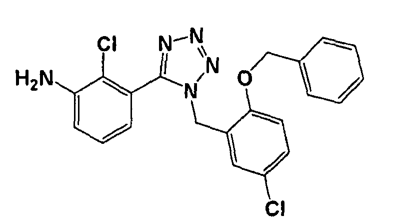

By the same procedure as described in Example 3 using the compound prepared in Example 10(7), the compound of the present invention having the following physical data was given.
TLC:Rf 0.53 (n-hexane : ethyl acetate =2:1);
NMR (CDCl₃):δ 7.40-7.32 (m, 3H), 7.21-7.16 (m, 3H), 7.03 (dd, J = 8.1, 7.5 Hz, 1H), 6.94 (d, J = 2.4 Hz, 1H), 6.88 (dd, J = 8.1, 1.5 Hz, 1H), 6.76 (d, J = 8.7 Hz, 1H), 6.42 (dd, J = 7.5, 1.5 Hz, 1H), 5.43 (s, 2H), 4.92 (s, 2H), 4.19 (brs, 2H).

### Example 14(1)-Example 14(23)

By the same procedure as described in example 1 using a corresponding compound, the compounds of the present invention were given.

### Example 14(1):

### N-(2-benzyloxy-5-chlorobenzyl)-2-chloro-3-methylbenzamide

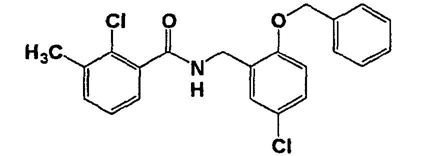

TLC:Rf 0.53 (n-hexane : ethyl acetate = 2 : 1);
NMR (CDCl₃):δ 7.40-7.14 (m, 10H), 6.87 (d, J = 8.7 Hz, 1H), 6.53-6.42 (m, 1H), 5.09 (s, 2H), 4.65 (d, J = 6.0 Hz, 2H), 2.39 (s, 3H).

### Example 14(2):

### N-(2-benzyloxy-5-chlorobenzyl)-2,4-dichlorobenzamide

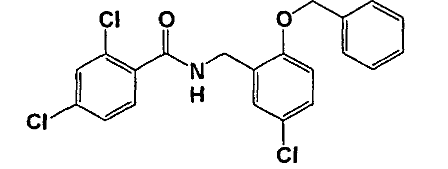

TLC:Rf 0.59 (n-hexane : ethyl acetate = 2 : 1);
NMR (CDCl₃):δ 7.60 (d, J = 8.4 Hz, 1H), 7.41-7.32 (m, 7H), 7.28 (dd, J = 8.4, 2.4 Hz, 1H), 7.22 (dd, J = 8.7, 2.7 Hz, 1H), 6.89 (d, J = 8.7 Hz, 1H), 6.80-6 69 (m, 1H), 5.09 (s, 2H), 4.64 (d, J = 6 0 Hz, 2H).

### Example 14(3)

### N-(2-benzyloxy-5-chlorobenzyl)-2,5-dichlorobenzamide

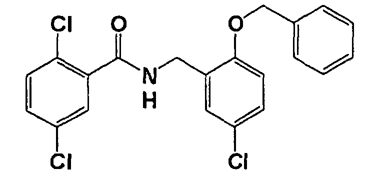

TLC:Rf 0.57 (n-hexane : ethyl acetate = 2 : 1);
NMR (CDCl₃):δ 7.62 (dd, J = 2.1, 0.9 Hz, 1H), 7.41-7.29 (m, 8H), 7.23 (dd, J = 9.0, 2.7 Hz, 1H), 6.89 (d, J = 9.0 Hz, 1H), 6.73-6.64 (m, 1H), 5 09 (s, 2H), 4.64 (d, J = 6.0 Hz, 2H)..

### Example 14(4):

### N-(2-benzyloxy-5-chlorobenzyl)-2,6-dichlorobenzamide

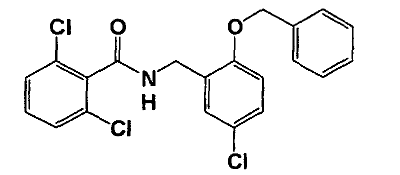

TLC:Rf0.47 (n-hexane : ethyl acetate = 2 : 1);
NMR (CDCl₃):δ 7.47-7.18 (m, 10H), 6.87 (d, J = 8.7 Hz, 1H), 6.18-6.07 (m, 1H), 5.08 (s, 2H), 4.67 (d, J = 6.0 Hz, 2H).

### Example 14(5):

### 2,3-dichloro-N-(1-phenylethyl)benzamide

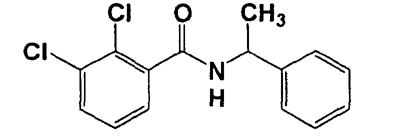

TLC:Rf0.45 (n-hexane : ethyl acetate = 2 : 1);
NMR (CDCl₃):δ 7.51 (dd, J = 7.5, 1.8 Hz, 1H), 7.46 (dd, J = 7 5, 1.8 Hz, 1H), 7.43-7 28 (m, 5H), 7.25 (t, J = 7.5 Hz, 1H), 6.25-6.16 (m, 1H), 5.40-5.28 (m, 1H), 1.63 (d, J = 6.9 Hz, 3H).

### Example 14(6):

### N-(2-benzyloxy-5-chlorobenzyl)-2-chloro-3-nitrobenzamide

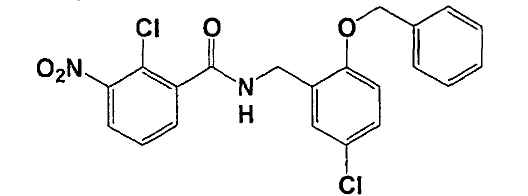

TLC:Rf 0.48 (n-hexane : ethyl acetate = 2 : 1);
NMR (CDCl₃):δ 7.80 (dd, J = 8.1, 1.5 Hz, 1H), 7.66 (dd, J = 8.1, 1.5 Hz, 1H), 7.45 (t, J = 8.1 Hz, 1H), 7.39-7.29 (m, 6H), 7.25 (dd, J = 8.7, 2.4 Hz, 1H), 6.91 (d, J = 8.7 Hz, 1H), 6.56-6.45 (m, 1H), 5.08 (s, 2H), 4.64 (d, J = 6.3 Hz, 2H).

### Example 14(7):

### N-(2-benzyloxy-5-chlorobenzyl)-2-methyl-3-nitrobenzamide

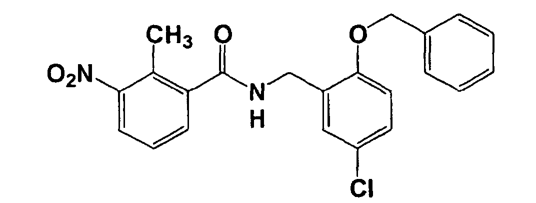

TLC:Rf 0.48 (n-hexane : ethyl acetate = 2: 1);
NMR (CDCl₃):δ 7.83 (dd, J = 8.4, 1.2 Hz, 1H), 7.42 (dd, J = 8.4, 1.2 Hz, 1H), 7.37-7.22 (m, 8H), 6.92 (d, J = 8.7 Hz, 1H), 6.24-6.18 (m, 1H), 5.08 (s, 2H), 4.61 (d, J = 6.0 Hz, 2H), 2.41 (s, 3H).

### Example 14(8):

### N-(2-benzyloxy-5-chlorobenzyl)-2,3-dihydrobenzofuran-7-ylcarbamide

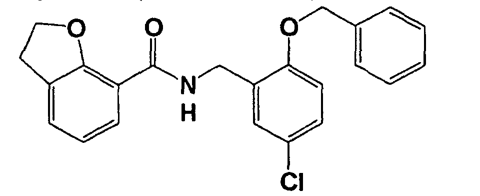

TLC:Rf 0.50 (n-hexane : ethyl acetate = 2 : 1);
NMR (CDCl₃):δ 8.18-8.10 (m, 1H), 7.96-7.91 (m, 1H), 7.48-7.26 (m, 7H), 7.17 (dd, J = 8.4, 2.7 Hz, 1H), 6.95 (t, J = 8.4 Hz, 1H), 6.84 (d, J = 8.7 Hz, 1H), 5.13 (s, 2H), 4.69 (d, J = 6.0 Hz, 2H), 4.49 (t, J = 9.0 Hz, 2H), 3.20 (t, J = 9.0 Hz, 2H).

### Example 14(9):

### N-(2-benzyloxy-5-chlorobenzyl)-3-chloro-2-methylbenzamide

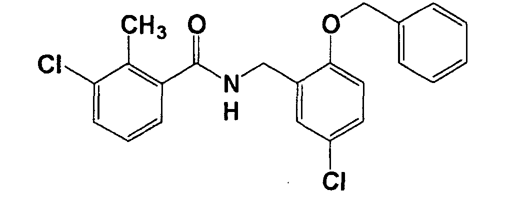

TLC:Rf 0.65 (n-hexane : ethyl acetate = 2 : 1);
NMR (CDCl₃):δ 7.42-7.,30 (m, 7H), 7.23 (dd, J = 8.7,2.7 Hz, 1H), 7.16-7.06 (m, 2H), 6.89 (d, J = 8.7 Hz, 1H), 6.22-6.16 (m, 1H), 5.08 (s, 2H), 4.60 (d, J = 5.7 Hz, 2H), 2.35 (s, 3H).

### Example 14(10):

### N-(2-benzyloxy-5-chlorobenzyl)-3-methoxymethyloxy-2-methylbenzamide

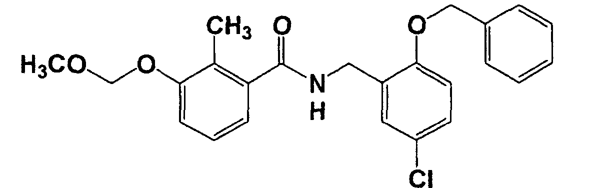

TLC:Rf 0.34 (ethyl acetate : n-hexane = 1 : 2);
NMR (CDCl₃):δ 7.38-7.30 (m, 6H), 7.21 (dd, J = 8.7, 2.7 Hz, 1H), 7.14-7.07 (m, 2H), 6.92 (dd, J = 9.0, 3.0 Hz, 1H), 6.88 (d, J = 8.7 Hz, 1H), 6.20-6.14 (m, 1H), 5.20 (s, 2H), 5.08 (s, 2H), 4.62 (d, J = 5.7 Hz, 2H), 3.47 (s, 3H), 2.25 (s, 3H).

### Example 14(11):

### N-(2-benzyloxy-5-chlorobenzyl)-4-methoxymethoxy-2-methylbenzamide

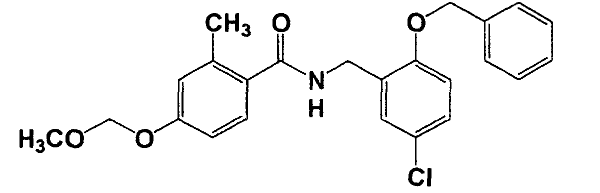

TLC:Rf 0.41 (ethyl acetate : n-hexane = 1 : 2);
NMR (CDCl₃):δ 7.64-7.58 (m, 2H), 7.44-7.36 (m, 5H), 7.33 (d, J = 2.7 Hz, 1H), 7.20 (dd, J = 8.7 Hz, 2.7 Hz, 1H), 7.04-6.98 (m, 2H), 6.88 (d, J = 8.7 Hz, 1H), 6.62-6.54 (m, 1H), 5.20 (s, 2H), 5.10 (s, 2H), 4.62 (d, J = 6.0 Hz, 2H), 3.48 (s, 3H).

### Example 14(12):

### 2,3-dichloro-N-(2-phenethyloxybenzyl)benzamide

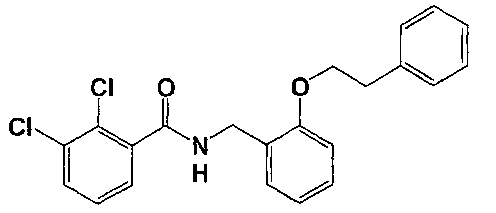

TLC:Rf 0.46 (n-hexane : ethyl acetate = 3 : 1);
NMR (CDCl₃):δ 7.50 (dd, J = 7.5, 1.5 Hz, 1H), 7.35 (dd, J = 7.5, 1.5 Hz, 1H), 7.29-7.18 (m, 5H), 7.14-7.09 (m, 2H), 6.97-6.86 (m, 3H), 6.13-6.03 (br, 1H), 4.53 (d, J = 6.0 Hz, 2H), 4.25 (t, J = 6.3 Hz, 2H), 3.10 (t, J = 6.3 Hz, 2H).

### Example 14(13):

### N-(2-benzyloxy-5-chlorobenzyl)-4-chlorobenzamide

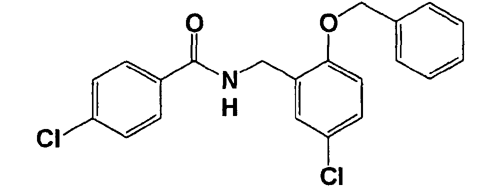

TLC:Rf 0.66 (ethyl acetate : n-hexane = 1 : 2);
NMR (CDCl₃):δ 7.58-7.52 (m, 2H), 7.44-7.30 (m, 8H), 7.22 (dd, J = 9.0 Hz, 3.0 Hz, 1H), 6.90 (d, J = 9.0 Hz, 1H), 5.10 (s, 2H), 4.62 (d, J = 5.7 Hz, 2H).

### Example 14(14):

### N-(2-cyclohexylmethyloxybenzyl)-2,3-dichlorobenzamide

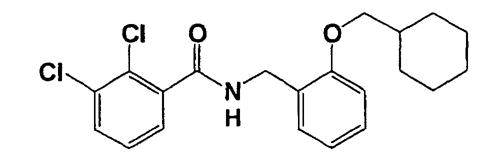

TLC:Rf 0.39 (n-hexane : ethyl acetate = 4 : 1);
NMR (CDCl₃):δ 7.50 (dd, J = 7.8, 1.5 Hz, 1H), 7.46 (dd, J = 7.8, 1.5 Hz, 1H), 7.35 (dd, J = 7.5, 1.2 Hz, 1H), 7.29-7.21 (m, 2H), 6.96-6.85 (m, 2H), 6.60-6.50 (br, 1H), 4.67 (d, J = 5.7 Hz, 2H), 3.80 (d, J = 5.7 Hz, 2H), 1.86-1.67 (m, 6H), 1.35-0.98 (m, 5H).

### Example 14(15):

### N-(2-cyclohcxyloxybenzyl)-2,3-dichlorobenzamide

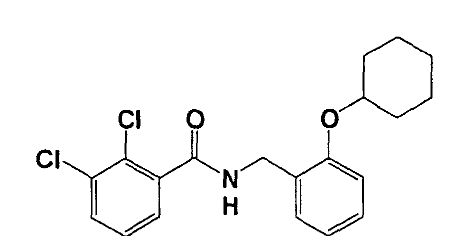

TLC:Rf 0.47 (n-hexane : ethyl acetate = 3 : 1);
NMR (CDCl₃):δ 7.52-7.46 (m, 2H), 7.36-7.34 (m, 1H), 7.28-7.21 (m, 2H), 6.94-6.88 (m, 2H), 6.64-6.54 (br, 1H), 4.65 (d, J = 5.7 Hz, 1H), 4.39-4.31 (m, 1H), 1.99-1.93 (m, 2H), 1.82-1.71 (m, 2H), 1.65-1.52 (m, 3H), 1.46-1.30 (m, 3H).

### Example 14(16):

### 2,3-dichloro-N-[2-(2-morpholinoethyloxy)benzyl]benzamide

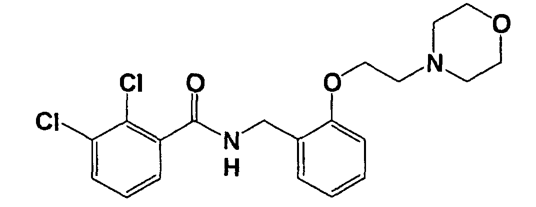

TLC:Rf 0.42 (chloroform : methanol = 19 : 1);
NMR (CDCl₃):δ 7.51 (dd, J = 7.8, 1.5 Hz, 1H), 7.43 (dd, J = 7.8, 1.5 Hz, 1H), 7.38 (dd, J = 7.5, 1.8 Hz, 1H), 7.31-7.22 (m, 2H), 6.99-6.87 (m, 3H), 4.65 (d, J = 6.0 Hz, 2H), 4.14 (t, J = 5.4 Hz, 2H), 3.48 (t, J = 4.8 Hz, 4H), 2.78 (t, J = 5.4 Hz, 2H), 2.47 (t, J = 4.8 Hz, 4H).

### Example 14(17):

### N-(2-chloro-6-phenoxybenzyl)-2,3-dimethylbenzamide

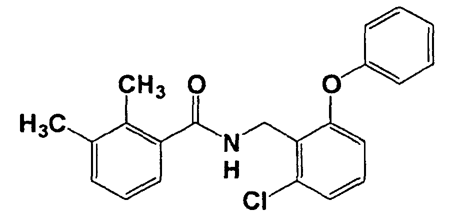

TLC:Rf 0.50(ethyl acetate : n-hexane = 1 : 2);
NMR (CDCl₃):δ 7.38-7.30 (m, 2H), 7.20-7.10 (m, 4H), 7.10-7.03 (m, 2H), 7.02-6.92 (m, 2H), 6.84-6.77 (m, 1H), 5.98-5.90 (m, 1H), 4.80 (d, J = 5.7 Hz, 2H), 2.27 (s, 3H), 2.26 (s, 3H).

### Example 14(18):

### N-(2-benzyloxybenzyl)-2,3-dimethylbenzamide

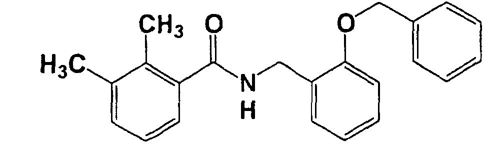

TLC:Rf 0.50 (ethyl acetate : n-hexane = 1 : 2);
NMR (CDCl₃):δ 7.41-7.24 (m, 7H), 7.18-7.14 (m, 1H), 7.12-6.94 (m, 4H), 6.25-6.16 (m, 1H), 5.10 (s, 2H), 4.67 (d, J = 5.7 Hz, 2H), 2.26 (s, 3H), 2.22 (s, 3H).

### Example 14(19):

### N-(2-benzyloxyphenethyl)-2,3-dimethylbenzamide

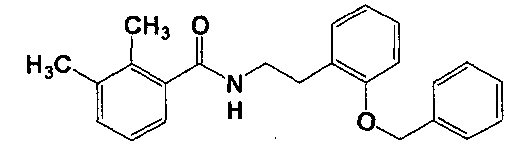

TLC:Rf 0.55(ethyl acetate : n-hexane = 1 : 2);
NMR (CDCl₃):δ 7.38-7.13 (m, 8H), 7.06-6.91 (m, 4H), 6.00-5.88 (m, 1H), 5.03 (s, 2H), 3.71 (q, J = 7 Hz, 2H), 2.99 (t, J = 7 Hz, 2H), 2.26 (s, 3H), 2.20 (s, 3H).

### Example 14(20):

### N-[2-(2H,3H-benzo[e]1,4-dioxan-2-ylmethyloxy)benzyl]-2,3-dichlorophenyl benzamide

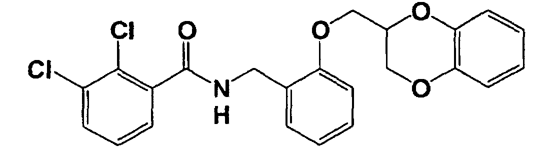

TLC:Rf 0.53(n-hexane : ethyl acetate = 3 : 2);
NMR (CDCl₃):δ 7.50 (dd, J = 7.8, 1.5 Hz, 1H), 7.42-7.38 (m, 2H), 7.32-7.27 (m, 1H), 7.22 (t, J = 7.8 Hz, 1H), 6.99 (dt, J = 7.5, 0.9 Hz, 1H), 6.91-6.72 (m, 4H), 6.69-6.59 (br, 1H), 6.49 (dd, J = 7.8, 1.5 Hz, 1H), 4.66-4.55 (m, 3H), 4.38 (dd, J = 11.4, 2.4 Hz, 1H), 4.28-4.22 (m, 3H).

### Example 14(21):

### 2,3-dichloro-N-[2-(2,2-dimethyl-1,3-dioxolan-4-ylmethyloxy)benzyl]benzamide

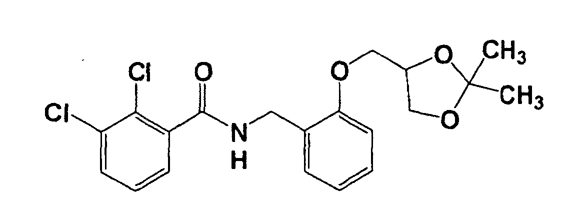

TLC:Rf 0.25(n-hexane : ethyl acetate = 2 : 1);
NMR (CDCl₃):δ 7.49 (dd, J = 7.8, 1.5 Hz, 1H), 7.41-7.38 (m, 2H), 7.31-7.19 (m, 2H), 7.00-6.95 (m, 2H), 6 87 (d, J = 8.1 Hz, 1H), 4.71 (dd, J = 14.4, 6.6 Hz, 1H), 4.59 (dd, J = 14.4, 5.4 Hz, 1H), 4.52-4.44 (m, 1H), 4.13-4.07 (m, 2H), 3.98 (dd, J = 9.6, 6.0 Hz, 1H), 3.82 (dd, J = 14.4, 5.7 Hz, 1H), 1.24 (s, 3H), 1.23 (s, 3H).

### Example 14(22):

### 2,3-dichloro-N-[2-[2-(N-methyl-N-phenylamino)ethyloxy]benzyl]benzamide

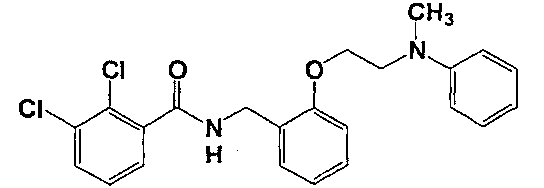

TLC:Rf 0.34(n-hexane : ethyl acetate = 3 : 1);
NMR (CDCl₃):δ 7.47 (dd, J = 7.5, 2.1 Hz, 1H), 7.39-7.35 (m, 1H), 7.29-7.07 (m, 5H), 6.97-6.85 (m, 2H), 6.69-6.61 (m, 3H), 6.26 (bs, 1H), 4.50 (d, J = 6.3 Hz, 2H), 4.19 (t, J = 5.4 Hz, 2H), 3.76 (t, J = 5.4 Hz, 2H), 2.91 (s, 3H).

### Example 14(23):

### 2,3-dichloro-N-(2-hydroxybenzyl)benzamide

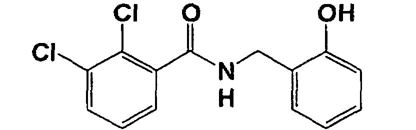

TLC.Rf 0.24(n-hexane : ethyl acetate = 3 : 1);
NMR (CDCl₃):δ 8.67 (s, 1H), 7.54 (dd, J = 7.8, 1.5 Hz, 1H), 7.50 (dd, J = 7.8, 1.5 Hz, 1H), 7.27-7.21 (m, 2H), 7.15 (dd, J = 7.8, 1.5 Hz, 1H), 6.98-6.95 (m, 2H), 6.85 (dt, J = 7.5, 1.2 Hz, 1H), 4.57 (d, J = 6.6 Hz, 2H).

### Formulation Example 1

The following components were mixed by a conventional technique and punched out to give 100 tablets each containing 50 mg of active ingredient.

| | |
|---|---|
| 1-(2-chloro-6-fluorobenzyl)-5-(2-trifluoromethylphenyl)-1H-1,2,3,4-tetrazole | 5.0 g |
| carboxymethylcellulose calcium (disintegrating agent) | 2 g |
| magnesium stearate(lubricating agent) | 0.1 g |
| microcrystalline cellulose | 4.7 g |

### Formulation Example 2

The following components were mixed by conventional technique, and the given solution was sterilized by a conventional technique, filled into ampoules 5 ml each, freeze-dried by a conventional technique, to give 100 ampoules each containing 20 mg of active ingredient.

| | |
|---|---|
| 1-(2-chloro-6-fluorobenzyl)-5-(2-trifluoromethylphenyl)-1H-1,2,3,4-tetrazole | 2.0 g |
| mannitol | 20 g |
| distilled water | 500 ml |

## Claims

1. A pharmaceutical composition for the prophylaxis and/or treatment of diseases induced, exacerbated or reignited by a stressor, which comprises a mitochondrial benzodiazepine receptor antagonist.

2. The pharmaceutical composition according to claim 1, which acts on mitochondrial benzodiazepine receptor and suppresses neurosteroid production.

3. A mitochondrial benzodiazepine receptor antagonist, which comprises a compound represented by formula (I): 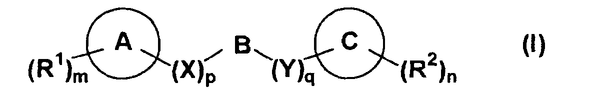 wherein A and C are each independently a 5-10 membered carboring or heteroring, B is 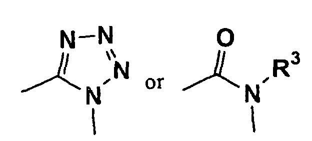 wherein R³ is hydrogen atom, C1-8 alkyl which may be substituted with phenyl, C2-8 acyl which may be substituted with phenyl or C1-8 alkoxycarbonyl, or R² and R3 are taken together to represent C1-4 alkylene,
X and Y are each independently -CH₂-; -O-; or -CHR⁴- wherein R⁴ is C1-4 alkyl, or taken together with R¹ or R², represents C2-5 alkylene,
p and q are each independently 0 or an integer of 1-2, wherein p and q do not represent 0 at the same time,
R¹ and R² are each independently
1) halogen,
2) OR⁵,
3) SR⁵,
4) NR⁶R⁷,
5) nitro,
6) cyano,
7) COR⁸,
8) 3-10 membered carboring or heteroring which may be substituted with 1-5 group(s) selected from C1-8 alkyl wherein the alkyl may be substituted with 1-5 group(s) selected from halogen, OR⁵, SR⁵, NR⁶R⁷, nitro, cyano, COR⁸, and phenyl; halogen; OR⁵; SR⁵; NR⁶R⁷; nitro; cyano, COR⁸; and 5-10 membered carboring or heteroring wherem the ring may be substituted with 1-5 group(s) selected from halogen, OR⁵, SR⁵, NR⁶R⁷, nitro, cyano, COR⁸, phenyl, and C1-8 alkyl, or
9) C1-8 alkyl substituted with 1-5 group(s) selected from halogen; OR⁵; SR⁵; NR⁶R⁷; nitro; cyano; COR⁸; and 5-10 membered carboring or heteroring wherein the nng may be substituted with 1-5 group(s) selected from halogen, OR⁹, SR⁹, NR¹⁰R¹¹, nitro, cyano, COR¹², and C1-8 alkyl wherein the alkyl may be substituted with 1-5 group(s) selected from halogen, OR⁵, SR⁵, NR⁶R⁷, nitro, cyano, COR⁸, and phenyl;
R⁵ is
1) hydrogen,
2) C1-8 alkyl which may be substituted with 1-5 group(s) selected from halogen; OR⁹; SR⁹; NR¹⁰R¹¹; nitro; cyano; COR¹²; and 3-10 membered carboring or heteroring wherein the ring may be substituted with 1-5 group(s) selected from halogen, OR⁹, SR⁹, NR¹⁰R¹¹, nitro, cyano, COR¹², phenyl, and C1-8 alkyl wherein the alkyl may be substituted with 1-5 group(s) selected from halogen, OR⁹, SR⁹, NR¹⁰R¹¹, nitro, cyano, COR¹², and phenyl;
3) 3-10 membered carboring or heteroring which may be substituted with 1-5 group(s) selected from C1-8 alkyl wherein the alkyl may be substituted with 1-5 group(s) selected from halogen, OR⁹, SR⁹, NR¹⁰R¹¹, nitro, cyano, COR¹², and phenyl; halogen; OR⁹; SR⁹; NR¹⁰R¹¹; nitro; cyano; COR¹²; and 3-10 membered carboring or heteroring wherein the ring may be substituted with 1-5 group(s) selected from, halogen, OR⁹, SR⁹, NR¹⁰R¹¹, nitro, cyano, COR¹², phenyl, and C1-8 alkyl, or
4) COR¹³, wherein R¹³ is C1-8 alkyl wherein the alkyl may be substituted with 1-5 group(s) selected from halogen, OR⁹, SR⁹, NR¹⁰R¹¹, nitro, cyano, COR¹², and phenyl; or 3-10 membered carboring or heteroring wherein the ring may be substituted with 1-5 selected from halogen, OR⁹, SR⁹, NR¹⁰R¹¹, nitro, cyano, COR¹², phenyl, and C1-8 alkyl,
R⁶ and R⁷ are each independently
1) hydrogen,
2) C2-8 acyl which may be substituted with 1-5 group(s) selected from halogen, OR⁹, SR⁹, NR¹⁰R¹¹, nitro, cyano, COR¹², and 3-10 membered carboring or heteroring wherein the ring may be substituted with 1-5 group(s) selected from halogen, OR⁹, SR⁹, NR¹⁰R¹¹, nitro, cyano, COR¹², and 3-10 membered carboring or heteroring wherein the ring may be substituted with 1-5 group selected from, halogen, OR⁹, SR⁹, NR¹⁰R¹¹, nitro, cyano, COR¹², and C1-8 alkyl wherein the alkyl may be substituted with 1-5 selected from halogen, OR⁹, SR⁹, NR¹⁰R¹¹, nitro, cyano, COR¹², and phenyl,
3) COOR¹²,
4) CONR¹⁰R¹¹,
5) 3-10 membered carbonng which may substituted with 1-5 group(s) selected from C1-8 alkyl wherein the alkyl may be substituted with 1-5 group(s) selected from halogen, OR⁹, SR⁹, NR¹⁰R¹¹, nitro, cyano, COR¹², and phenyl; halogen; OR⁹; SR⁹; NR¹⁰R¹¹; nitro; cyano; COR¹²; and 3-10 membered carboring or heteroring wherein the ring may be substituted with 1-5 group(s) selected from, halogen, OR⁹, SR⁹, NR¹⁰R¹¹, nitro, cyano, COR¹², and C1-8 alkyl wherein the alkyl may be substituted with 1-5 group(s) selected from halogen, OR⁹, SR⁹, NR¹⁰R¹¹, nitro, cyano, and COR¹², or
6) C1-8 alkyl which may be substituted with 1-5 group(s) selected from halogen; OR⁹; SR⁹; NR¹⁰R¹¹; nitro; cyano; COR¹²; and 3-10 membered carboring or heteroring wherein the ring may be substituted with 1-5 group(s) selected from halogen, OR⁹, SR⁹, NR¹⁰R¹¹, nitro, cyano, COR¹², and C1-8 alkyl wherein the alkyl may be substituted with 1-5 group(s) selected from halogen, OR⁹, SR⁹, NR¹⁰R¹¹, nitro, cyano, and COR¹²,
R⁸ is
1) hydrogen,
2) OR⁹,
3) NR¹⁰R¹¹,
4) 3-10 membered carbonng or heteroring which may be substituted with 1-5 group(s) selected from halogen; OR⁹; SR⁹; NR¹⁰R¹¹; nitro; cyano; COR¹²; phenyl; C1-8 alkyl wherein the alkyl may be substituted with 1-5 group(s) selected from halogen, OR⁹, SR⁹, NR¹⁰R¹¹, nitro, cyano, COR¹², and phenyl; and 3-10 membered carboring or heteroring wherein the ring may be substitutcd with 1-5 group(s) selected from halogen, OR⁹, SR⁹, NR¹⁰R¹¹, nitro, cyano, COR¹², C1-8 alkyl, and phenyl, or
5) C1-8 alkyl which may be substituted with 1-5 group(s) selected from halogen; OR⁹; SR⁹; NR¹⁰R¹¹; nitro; cyano; COR¹²; and 3-10 membered carboring or heteroring wherein the ring may be substituted with 1-5 group(s) selected from halogen, OR⁹, SR⁹, NR¹⁰R¹¹, nitro, cyano, COR¹², and C1-8 alkyl wherein the alkyl may be substituted with 1-5 group(s) selected from halogen, OR⁹, SR⁹, NR¹⁰R¹¹, nitro, cyano, COR¹², and phenyl,
R⁹ is hydrogen atom, C1-8 alkyl or C2-8 acyl wherein the alkyl and alkoxy may be substituted with C1-8 alkoxy, C1-8 alkylthio or 3-10 membered carboring or heteroring,
R¹⁰ and R¹¹ are each independently hydrogen atom, C1-8 alkyl or phenyl,
R¹² is hydrogen atom, phenyl, C1-8 alkyl optionally substituted with phenyl, or C1-8 alkoxy optionally substituted with phenyl,
m and n are each independently 0 or 1-5, or
a non-toxic salt thereof.

4. The pharmaceutical composition according to claim 1, which comprises the mitochondrial benzodiazepine receptor antagonist of formula (I) according to claim 3, as an active ingredient.

5. The mitochondrial benzodiazepine receptor antagonist according to claim 3, which comprises a tetrazole derivative compound, in the compound of formula (I), A is 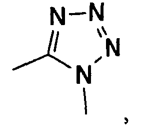 which is the compound of formula (I-a): 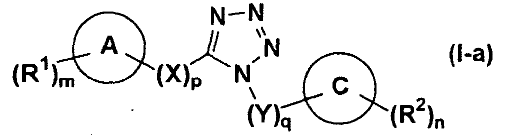 wherein all symbols have the same meaning as hereinbefore.

6. The mitochondrial benzodiazepine receptor antagonist according to claim 3, which comprises, as an active ingredient, a compound among the compound of formula (I), wherein A is -CONR¹⁰-, that is, an amide derivative of formula (I-b): 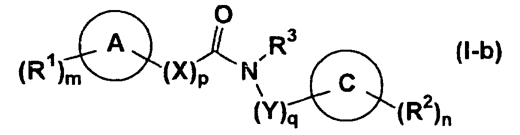 wherein all symbols have the same meaning as above

7. The pharmaceutical composition for the prophylaxis and/or treatment of diseases induced, exacerbated or reignited by stressors according to claim 1, which comprises, as an active ingredient, a compound of formula (II): 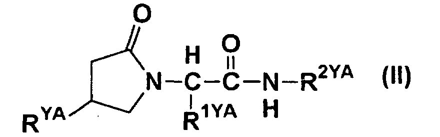 wherein R^{YA} is hydrogen or hydroxy,
R^{1YA} is hydrogen or methyl,
R^{2YA} is pyridyl or phenyl substituted with 1-3 of the same or different substituent(s),
the substituents on phenyl are selected from halogen; trifluoromethyl; nitro; acetyl; straight or branched-chain alkyl having 1-4 carbon atom(s); straight or branched-chain alkoxy having 1-4 carbon atom(s); straight or branched-chain alkylmercapto having 1-7 carbon atom(s); substituted alkylmercapto of formula: -S-(CH₂)_{nYA}-CH(R^{3YA})(R^{4YA}) wherein n^{YA} is 1 or 2, R^{3YA} is hydrogen or methyl, R^{4YA} is hydroxy or substituted amino of formula: -NR^{8YA}R^{9YA} wherein R^{8YA} is hydrogen or methyl, R^{9YA} is methyl, benzyl, or substituted benzyl, or R^{8YA} and R^{9YA} are taken together to form substituted pyrrolidine ring with the nitrogen atom in the formula; sulfonyl of formula: -SO₂R^{5YA} wherein R^{5YA} is amino or alkyl having 1-3 carbon atom(s); and aminoethoxycarbonyl of formula: -COO(CH₂)₂-NR^{6YA}R^{7YA} wherein R^{6YA} and R^{7YA} are each independently hydrogen atom, methyl, or ethyl.

8. The pharmaceutical composition for the prophylaxis and/or treatment of diseases induced, exacerbated or reignited by a stressor according to claim 1, which comprises, as an active ingredient, a compound of formula (III): 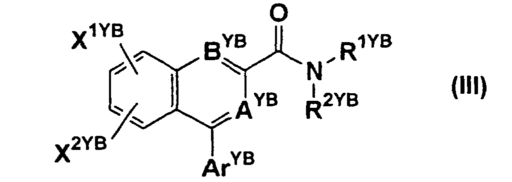 wherein R^{1YB} and R^{2YB} are each independently straight or branched chain C1-6 alkyl, C3-7 cycloalkyl, phenylalkyl which has C1-3 in alkyl or cycloalkyl-substituted alkyl, or C3-6 alkenyl or C3-6 alkynyl wherein neither double bond nor triple bond is not located in 1-2 position to N atom,
A^{YB} and B^{YB} are each independently N or CH,
X^{1YB} and X^{2YB} are each independently halogen, straight or branched-chain C1-3 alkyl, straight or branched-chain C1-3 alkoxy, nitro, or trifluoromethyl,
Ar^{YB} is phenyl, pyridyl, thienyl, or substituted phenyl wherein the substituent on phenyl is 1 or 2 selected from halogen, straight or branched-chain C1-4 alkyl, straight or branched-chain C1-4 alkoxy, straight or branched-chain C1-4 alkylthio, trifluoromethyl, and nitro, or
a non-toxic salt thereof.

9. The pharmaceutical composition for the prophylaxis and/or treatment of diseases induced, exacerbated or reignited by a stressor according to claim 1, which comprises, as an active ingredient, a compound of formula (IV): 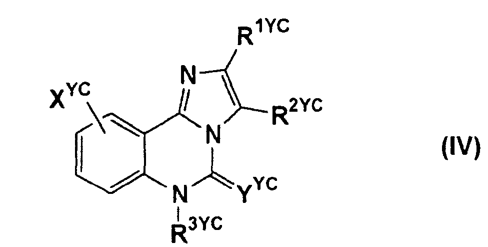 wherein R^{1YC} is unsubstituted phenyl; substituted phenyl wherein substituent on phenyl is 1-2 group(s) selected from halogen, straight or branched chain alkyl having 1-6 carbon atom(s), and straight or branched chain alkoxy having 1-6 carbon atom(s); or thienyl,
R^{2YC} is hydrogen; halogen; or optionally substituted straight or branched chain alkyl having 1-6 carbon atoms(s) wherein substituent is selected from amino, alkylamino and dialkylamino.),
R^{3YC} is (R^{4YC})(R^{5YC})N-CO-Q^{YC} wherein Q^{YC} is straight or branched chain alkylene having 1-6 carbon atom(s), R^{4YC} and R^{5YC} are each independently straight or branched chain alkyl having 1-6 carbon atom(s), unsubstituted phenyl, or substituted phenyl wherein the substituent on phenyl is 1 or 2 selected from halogen, straight or branched-chain C1-6 alkyl, and straight or branched-chain C1-6 alkoxy,
X^{YC} is hydrogen or halogen,
Y^{YC} is oxygen or sulfur, or
a non-toxic salt thereof.

10. The pharmaceutical composition for the prophylaxis and/or treatment of diseases induced, exacerbated or reignited by a stressor according to claim 1, which comprises, as an active ingredient, a compound of formula (V) 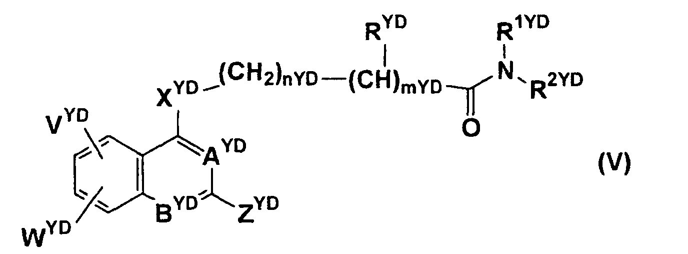 wherein A^{YD} and B^{YD} are each independently N or CH,
V^{YD} and W^{YD} are each independently hydrogen, halogen, C1-3 alkyl, C1-3 alkoxy, nitro or trifluoromethyl,
Z^{YD} is phenyl, thienyl or pyridyl, above phenyl is unsubstituted or substituted with 1-2 selected from halogen, C1-4 alkyl, C1-4 alkoxy, trifluoromethyl, and nitro, and Z^{YD} is located in ortho or para to B^{YD},
side chain -X^{YD}-(CH₂)_{nYD}-(CHR^{YD})_{mYD}-CONR^{1YD}R^{2YD} is located in ortho or para to B^{YD},
R^{YD} is hydrogen or C1-3 alkyl,
R^{1YD}, R^{2YD} are each independently C1-6 alkyl, C3-6 cycloalkyl, phenyl, phenyl-C1-3 alkyl, C3-6 cycloalkyl-C1-3 alkyl or C3-6 alkenyl wherein the double bond in alkenyl is not located in 1-2 position, or NR^{1YD}R^{2YD} is pyrrolidino, piperidino, morpholino or thiomorpholino,
X^{YD} is CHR^{3YD}, NR^{4YD}, SO, SO₂, O or S,
R^{3YD} is hydrogen or C 1-3 alkyl,
R^{4YD} is C1-3 alkyl,
m^{YD} is 0 or 1,
n^{YD} is 0 or an integer of 1-2,
wherein
(1) when X^{YD} is SO, SO₂ or NR^{4YD}, m^{YD}+n^{YD} is 1 or more,
(2) when A^{YD} and B^{YD} are each N and Z^{YD} is located in para to B^{YD}, X^{YD} is not CHR^{3YD},
(3) when A^{YD} is CH, B^{YD} is N, Z^{YD} is ortho to B^{YD}, X^{YD} is O, R^{YD} is hydrogen, m^{YD}+n^{YD} is not 1, and
(4) 2-phenylquinolin-4-yl-N,N'-dimethylcarbamate is excluded, or
a non-toxic salt thereof.

11. A compound of formula (I-a'): 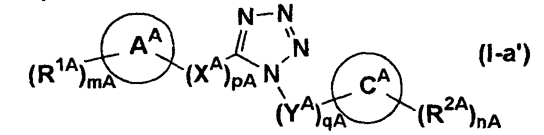 wherein A^{A} and C^{A} are each independently 5-10 membered carboring or heteroring,
X^{A} and Y^{A} are each independently -CH₂-, -O- or -CHR^{4A}- wherein R^{4A} is C1-4 alkyl, or taken together with R^{1A} or R^{2A} to form C2-5 alkylene,
p^{A} and q^{A} are each independently 0 or an integer of 1-2 wherein p^{A} and q^{A} do not form 0 at the same time,
R^{1A} and R^{2A} are each independently
1) halogen,
2) OR^{5A},
3) SR^{5A},
4) NR^{6A}R^{7A},
5) nitro,
6) cyano,
7) COR^{8A},
8) 3-10 membered carboring or heteroring which may be substituted with 1-5 group(s) selected from C1-8 alkyl wherein the alkyl may be substituted with 1-5 group(s) selected from halogen, OR^{5A}, SR^{5A}, NR^{6A}R^{7A}, nitro, cyano, COR^{8A}, and phenyl; halogen; OR^{5A}; SR^{5A}; NR^{6A}R^{7A}; nitro; cyano; COR^{8A}; and 5-10 membered carboring or heteroring wherein the rings may be substituted with 1-5 group(s) selected from halogen, OR^{5A}, SR^{5A}, NR^{6A}R^{7A}, nitro, cyano, COR^{8A}, phenyl, and C1-8 alkyl, or
9) C1-8 alkyl which may be substituted with 1-5 group(s) selected from halogen; OR^{5A}; SR^{5A}; NR^{6A}R^{7A}; nitro; cyano; COR^{8A}; and 5-10 membered carboring or heteroring wherein the ring may be substituted with 1-5 group(s) selected from halogen, OR^{9A}, SR^{9A}, NR^{10A}R^{11A}, nitro, cyano, COR^{12A}, and C1-8 alkyl wherein the alkyl may be substituted with 1-5 group(s) selected from halogen, OR^{5A}, SR^{5A}, NR^{6A}R^{7A}, nitro, cyano, COR^{8A}, and phenyl,
R^{5A} is
1) hydrogen,
2) C1-8 alkyl substituted with 1-5 group(s) selected from halogen; OR^{9A}; SR^{9A}; NR^{10A}R^{11A}; nitro; cyano; COR^{12A}; and 3-10 membered carboring or heteroring wherein the rings may be substituted with 1-5 group(s) selected from halogen, OR^{9A}, SR^{9A}, NR^{10A}R^{11A}, nitro, cyano, COR^{12A}, phenyl, and C1-8 alkyl wherein the alkyl may be substituted with 1-5 group(s) selected from halogen, OR^{9A}, SR^{9A}, NR^{10A}R^{11A}, nitro, cyano, COR^{12A}, and phenyl,
3) 3-10 membered carboring or heteroring which may be substituted with 1-5 group(s) selected from C1-8 alkyl wherein the alkyl may be substituted with 1-5 group(s) selected from halogen, OR^{9A}, SR^{9A}, NR^{10A}R^{11A}, nitro, cyano, COR^{12A}, and phenyl; halogen; OR^{9A}; SR^{9A}; NR^{10A}R^{11A}; nitro; cyano; COR^{12A}; and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, phenyl, and C1-8 alkyl, or
4) COR^{13A}, wherein R^{13A} is C1-8 alkyl wherein the alkyl may be substituted with 1-5 group(s) selected from halogen, OR^{9A}, SR^{9A}, NR^{10A}R^{11A}, nitro, cyano, COR^{12A}, and phenyl; or 3-10 membered carboring or heteroring wherein the ring may be substituted with 1-5 group(s) selected from halogen, OR^{9A}, SR^{9A}, NR^{10A}R^{11A}, nitro, cyano, COR^{12A}, phenyl, and C1-8 alkyl,
R^{6A} and R^{7A} are each independently
1) hydrogen,
2) C2-8 acyl which may be substituted with 1-5 group(s) selected from halogen; OR^{9A}; SR^{9A}; NR^{10A}R^{11A}; nitro; cyano; COR^{12A}; and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, and C1-8 alkyl wherein the alkyl may be substituted with 1-5 group(s) selected from halogen, OR^{9A}, SR^{9A}, NR^{10A}R^{11A}, nitro, cyano, COR^{12A}, and phenyl,
3) COOR^{12A},
4) CONR^{10A}R^{11A},
5) 3-10 membered carboring or heteronng which may be substituted with 1-5 group(s) selected from C1-8 alkyl wherein the alkyl may be substituted with 1-5 group(s) selected from 1-5 group selected from halogen, OR^{9A}, SR^{9A}, NR^{10A}R^{11A}, nitro, cyano, COR^{12A}, and phenyl; halogen; OR^{9A}; SR^{9A}; NR^{10A}R^{11A}; nitro; cyano; COR^{12A}; and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, and C1-8 alkyl wherein the alkyl may be substituted with 1-5 group(s) selected from halogen, OR^{9A}, SR^{9A}, NR^{10A}R^{11A}, nitro, cyano, and COR^{12A}, or
6) C1-8 alkyl which may be substituted with 1-5 group(s) selected from halogen; OR^{9A}; SR^{9A}; NR^{10A}R^{11A}; nitro; cyano; COR^{12A}; and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, and C1-8 alkyl wherein the alkyl may be substituted with 1-5 group(s) selected from halogen, OR^{9A}, SR^{9A}, NR^{10A}R^{11A}, nitro, cyano, and COR^{12A},
R^{8A} is
1) hydrogen,
2) OR^{9A},
3) NR^{10A}R^{11A},
4) 3-10 membered carboring or heteroring which may be substituted with 1-5 group(s) selected from halogen; OR^{9A}; SR^{9A}; NR^{10A}R^{11A}; nitro; cyano; COR^{12A}; phenyl; C1-8 alkyl wherein the alkyl may be substituted with 1-5 group(s) selected from halogen, OR^{9A}, SR^{9A}, NR^{10A}R^{11A}, nitro, cyano, COR^{12A}, and phenyl; and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, C1-8 alkyl, and phenyl, or
5) C1-8 alkyl which may be substituted with 1-5 group(s) selected from halogen; OR^{9A}; SR^{9A}; NR^{10A}R^{11A}; nitro; cyano; COR^{12A}; and 3-10 membered carboring or heteroring wherein the ring may be substituted with 1-5 group(s) selected from halogen, OR^{9A}, SR^{9A}, NR^{10A}R^{11A}, nitro, cyano, COR^{12A}, and C1-8 alkyl wherein the alkyl may be substituted with 1-5 group(s) selected from halogen, OR^{9A}, SR^{9A}, NR^{10A}R^{11A}, nitro, cyano, COR^{12A}, and phenyl,
R^{9A} is hydrogen, C1-8 alkyl or C2-8 acyl wherein the alkyl and alkoxy may be substituted with C1-8 alkoxy, C1-8 alkylthio or 3-10 membered carboring or heteroring,
R^{10A} and R^{11A} are each independently hydrogen, C1-8 alkyl or phenyl,
R^{12A} is hydrogen, phenyl, C1-8 alkyl which may be substituted with phenyl, or C1-3 alkoxy which may be substituted with phenyl,
m^{A} and n^{A} are each independently 0 or an integer of 1-5; or
a non-toxic salt thereof,
wherein the compounds of following (1)-(73) are excluded;
(1) 5-(4-chlorophenyl)-1-(3-chlorobenzyl)-1H-1,2,3,4-tetrazole,
(2) 5-(4-chlorophenyl)-1-(3-trifluoromethylbenzyl)-1H-1,2,3,4-tetrazole,
(3) 5-(3-chlorophenyl)-1-(3-chlorobenzyl)-1H-1,2,3,4-tetrazole,
(4) 5-(3-chlorophenyl)-1-(3-trifluoromethylbenzyl)-1H-1,2,3,4-tetrazole,
(5) 5-(3-trifluoromethylphenyl)-1-(3-chlorobenzyl)-1H-1,2,3,4-tetrazole,
(6) 5-(3,5-dichlorophenyl)-1-(3-chlorobenzyl)-1H-1,2,3,4-tetrazole,
(7) 5-(3-trifluoromethylphenyl)-1-(3-trifluoromethylbenzyl)-1H-1,2,3,4-tetrazole,
(8) 5-(3,5-dichlorophenyl)-1-(3-trifluoromethylbenzyl)-1H-1,2,3,4-tetrazole,
(9) 5-(4-bromophenyl)-1-benzyl-1H-1,2,3,4-tetrazole,
(10) 5-(4-pyridyl)-1-(2,5-dimethyl-4-hydroxybenzyl)-1H-1,2,3,4-tetrazole,
(11) 5-(4-bromophenyl)-1-(2,5-dimethyl-4-hydroxybenzyl)-1H-1,2,3,4-tetrazole
(12) 5-phenyl-1-(5-formyl-2-hydroxy-3-methoxybenzyl)-1H-1,2,3,4-tetrazole,
(13) 5-(3,4-dimethoxyphenyl)-1-(5-formyl-2-hydroxy-3-methoxybenzyl)-1H-1,2,3,4-tetrazole,
(14) 5-(4-bromophenyl)-1-(3,5-dimethyl-4-hydroxybenzyl)-1H-1,2,3,4-tetrazole,
(15) 5-(4-pyridyl)-1-(3,5-dimethyl-4-hydroxybenzyl)-1H-1,2,3,4-tetrazole,
(16) 5-cyclohexyl-1-cyclohexylmethyl-1H-1,2,3,4-tetrazole,
(17) 5-(4-nitrobenzyl)-1-phenyl-1H-1,2,3,4-tetrazole,
(18) 5-(4-aminobenzyl)-1-phenyl-1H-1,2,3,4-tetrazole,
(19) 5-(4-dimethylaminocarbonylaminobenzyl)-1-phenyl-1H-1,2,3,4-tetrazole,
(20) 5-[2-(4-methylphenyl)phenyl]-1-(4-nitrobenzyl)-1H-1,2,3,4-tetrazole,
(21) 5-phenyl-1-(4-methylbenzyl)-1H-1,2,3,4-tetrazole,
(22) 5-[4-(4-chlorophenylhydroxymethyl)phenyl]-1-(4-fluorobenzyl)-1H-1,2,3,4-tetrazole,
(23) 5-[4-(4-chlorobenzoyl)phenyl]-1-(4-fluorobenzyl)-1H-1,2,3,4-tetrazole,
(24) 1-benzyl-5-(5-nitrofuran-2-yl)-1H-1,2,3,4-tetrazole,
(25) 1-benzyl-5-[2-(4-bromomethylphenyl)phenyl]-1H-1,2,3,4-tetrazole,
(26) -1-(3,5-dimethyl-4-hydroxybenzyl)-5-(4-pyridyl)1H-1,2,3,4-tetrazole,
(27) 1-(3,5-dimethyl-4-hydroxybenzyl)-5-(4-nitrophenyl)-1H-1,2,3,4-tetrazole,
(28) 5-(4-chlorophenyl)-1-(3,5-dimethyl-4-hydroxybenzyl)-1H-1,2,3,4-tetrazole,
(29) 1-benzyl-5-(4-methylphenyl)-1H-1,2,3,4-tetrazole,
(30) 1-benzyl-5-(4-fluorophenyl)-1H-1,2,3,4-tetrazole,
(31) 1-benzyl-5-(4-methoxyphenyl)-1H-1,2,3,4-tetrazole,
(32) 1-benzyl-5-(2-methoxyphenyl)-1H-1,2,3,4-tetrazole,
(33) 1-benzyl-5-(4-benzyloxymethylphenyl)-1H-1,2,3,4-tetrazole,
(34) 1-benzyl-5-(2-(4-methylphenyl)phenyl)-1H-1,2,3,4-tetrazole,
(35) 5-(2-chlorophenyl)-1-(4-methoxybenzyl)-1H-1,2,3,4-tetrazole,
(36) 1-benzyl-5-(2-chlorophenyl)-1H-1,2,3,4-tetrazole,
(37) 1-(3,5-dimethyl-4-hydroxybenzyl)-5-phenyl-1H-1,2,3,4-tetrazole,
(38) 1-(4-bromobenzyl)-5-(3-cyanophenyl)-1H-1,2,3,4-tetrazole,
(39) 5-(3-cyanophenyl)-1-[4-(2-trifluoromethylphenyl)benzyl]-1H-1,2,3,4-tetrazole,
(40) 5-(5-bromofuran-2-yl)-1-(4-methoxybenzyl)-1H-1,2,3,4-tetrazole,
(41) 5-(6-bromopyridin-2-yl)-1-(4-methoxybenzyl)-1H-1,2,3,4-tetrazole,
(42) 5-(6-iodopyridin-2-yl)-1-(4-methoxybenzyl)-1H-1,2,3,4-tetrazole,
(43) 5-(3-ethoxycarbonylmethyloxy-5-iodophenyl)-1-(4-methoxybenzyl)-1H-1,2,3,4-tetrazole,
(44) 5-(3-iodo-2-isopropyloxyphenyl)-1-(4-methoxybenzyl)-1H-1,2,3,4-tetrazole,
(45) 5-(4-formylphenyl)- 1-(3-methoxycarbonylbenzyl)-1H-1,2,3,4-tetrazole,
(46) 5-(2-aminophenyl)-1-benzyl-1H-1,2,3,4-tetrazole,
(47) 5-(2-acetylaminophenyl)-1-benzyl-1H-1,2,3,4-tetrazole,
(48) 5-phenyl-1-benzyl-1H-1,2,3,4-tetrazole,
(49) 5-benzyl-1-(4-mercaptophenyl)-1H-1,2,3,4-tetrazole,
(50) 5-benzyl-1-phenyl-1H-1,2,3,4-tetrazole,
(51) 5-benzyl-1-(3,5-dimethyl-4-hydroxybenzyl)-1H-1,2,3,4-tetrazole,
(52) 1-benzyl-5-benzyl-1H-1,2,3,4-tetrazole,
(53) 5-phenyl-1-[2-(2-pyridyl)ethyl]-1H-1,2,3,4-tetrazole,
(54) 5-(2,4-dimethoxy-5-ethylphenethyl)-1-benzyl-1H-1,2,3,4-tetrazole,
(55) 5-(2,4-diethoxy-5-ethylphenethyl)-1-benzyl-1H-1,2,3,4-tetrazole,
(56) 5-(2,4-diethoxy-5-ethylphenethyl)- 1-phenethyl-1H-1,2,3,4-tetrazole,
(57) 5-(2,4-dimethoxy-5-ethylphenethyl)-1-phenethyl-1H-1,2,3,4-tetrazole,
(58) 5-benzyl-1-N,N-diisopropylaminocarbonylbenzyl-1H-1,2,3,4-tetrazole,
(59) 5-[4-(4-acetyl-3-hydroxy-2-propylphenyloxy)phenyl]-1-(4-cyanobenzyl)-1H-1,2,3,4-tetrazole,
(60) 5-benzyl-1-(4-amino-2,5-dimethoxyphenyl)-1H-1,2,3,4-tetrazole,
(61) 5-(2-aminophenyl)-1-benzyl-1H-1,2,3,4-tetrazole,
(62) 5-(4-t-butylcarbonyloxyphenyl)-1-(4-aminomethylbenzyl)-1H-1,2,3,4-tetrazole,
(63) 5-(4-t-butylcarbonyloxyphenyl)-1-(4-isopropylbenzyl)-1H-1,2,3,4-tetrazole,
(64) 5-(4-t-butylcarbonyloxyphenyl)-1-(4-dimethylaminobenzyl)-1H-1,2,3,4-tetrazole,
(65) 5-(4-t-butylcarbonyloxyphenyl)-1-(4-N-t-butoxycarbonyl-N-methylbenzyl)-1H-1,2,3,4-tetrazole,
(66) 5-(1-hydroxycycloheptyl)-1-benzyl-1H-1,2,3,4-tetrazole,
(67) 5-(1-naphthylmethyl)-1-(2-carboxyphenyl)-1H-1,2,3,4-tetrazole,
(68) 5-(4-carboxy-2-methoxyphenyl)-1-(4-methoxybenzyl)-1H-1,2,3,4-tetrazole,
(69) 5-(2-furyl)-1-benzyl-1H-1,2,3,4-tetrazole,
(70) 5-[2-(5-nitro)thieno]-1-benzyl-1H-1,2,3,4-tetrazole,
(71) 5-[4-methoxycarbonyl-3-(2-methylphenyl)benzyl]-1-benzyl-1H-1,2,3,4-tetrazole,
(72) 5-[4-carboxy-3-(2-methylphenyl)benzyl]-1-benzyl-1H-1,2,3,4-tetrazole,
(73) 5-thieno-1-benzyl-1H-1,2,3,4-tetrazole.

12. The compound according to claim 11, wherein both A ring and B ring are both carboring in formula (I-a'), or a non-toxic salt thereof,
wherein the following compounds (1)-(65) are excluded;
(1) 5-(4-chlorophenyl)-1-(3-chlorobenzyl)-1H-1,2,3,4-tetrazole,
(2) 5-(4-chlorophenyl)-1-(3-trifluoromethylbenzyl)-1H-1,2,3,4-tetrazole,
(3) 5-(3-chlorophenyl)-1-(3-chlorobenzyl)-1H-1,2,3,4-tetrazole,
(4) 5-(3-chlorophenyl)-1-(3-trifluoromethylbenzyl)-1H-1,2,3,4-tetrazole,
(5) 5-(3-trifluoromethylphenyl)-1-(3-chlorobenzyl)-1H-1,2,3,4-tetrazole,
(6) 5-(3,5-dichlorophenyl)-1-(3-chlorobenzyl)-1H-1,2,3,4-tetrazole,
(7) 5-(3-trifluoromethylphenyl)-1-(3-trifluoromethylbenzyl)-1H-1,2,3,4-tetrazole,
(8) 5-(3,5-dichlorophenyl)-1-(3-trifluoromethylbenzyl)-1H-1,2,3,4-tetrazole,
(9) 5-(4-bromophenyl)-1-benzyl-1H-1,2,3,4-tetrazole,
(10) 5-(4-pyridyl)-1-(2,5-dimethyl-4-hydroxybenzyl)-1H-1,2,3,4-tetrazole,
(11) 5-(4-bromophenyl)-1-(2,5-dimethyl-4-hydroxybenzyl)-1H-1,2,3,4-tetrazole,
(12) 5-phenyl-1-(5-formyl-2-hydroxy-3-methoxybenzyl)-1H-1,2,3,4-tetrazole,
(13) 5-(3,4-dimethoxyphenyl)-1-(5-formyl-2-hydroxy-3-methoxybenzyl)-1H-1,2,3,4-tetrazole,
(14) 5-(4-bromophenyl)-1-(3,5-dimethyl-4-hydroxybenzyl)-1H-1,2,3,4-tetrazole,
(15) 5-(4-pyridyl)-1-(3,5-dimethyl-4-hydroxybenzyl)-1H-1,2,3,4-tetrazole,
(16) 5-cyclohexyl-1-cyclohexylmethyl-1H-1,2,3,4-tetrazole,
(17) 5-(4-nitrobenzyl)-1-phenyl-1H-1,2,3,4-tetrazole,
(18) 5-(4-aminobenzyl)-1-phenyl-1H-1,2,3,4-tetrazole,
(19) 5-(4-dimethylaminocarbonylaminobenzyl)-1-phenyl-1H-1,2,3,4-tetrazole,
(20) 5-[2-(4-methylphenyl)phenyl]-1-(4-nitrobenzyl)-1H-1,2,3,4-tetrazole,
(21) 5-phenyl-1-(4-methylbenzyl)-1H-1,2,3,4-tetrazole,
(22) 5-[4-(4-chlorophenylhydroxymethyl)phenyl]-1-(4-fluorobenzyl)-1H-1,2,3,4-tetrazole,
(23) 5-[4-(4-chlorobenzoyl)phenyl]-1-(4-fluorobenzyl)-1H-1,2,3,4-tetrazole,
(24) 1-benzyl-5-[2-(4-bromomethylphenyl)phenyl]-1H-1,2,3,4-tetrazole,
(25) 1-(3,5-dimethyl-4-hydroxybenzyl)-5-(4-nitrophenyl)-1H-1,2,3,4-tetrazole,
(26) 5-(4-chlorophenyl)-1-(3,5-dimethyl-4-hydroxybenzyl)-1H-1,2,3,4-tetrazole,
(27) 1-benzyl-5-(4-methylphenyl)-1H-1,2,3,4-tetrazole,
(28) 1-benzyl-5-(4-fluorophenyl)-1H-1,2,3,4-tetrazole,
(29) 1-benzyl-5-(4-methoxyphenyl)-1H-1,2,3,4-tetrazole,
(30) 1-benzyl-5-(2-methoxyphenyl)-1H-1,2,3,4-tetrazole,
(31) 1-benzyl-5-(4-benzyloxymethylphenyl)-1H-1,2,3,4-tetrazole,
(32) 1-benzyl-5-(2-(4-methylphenyl)phenyl)-1H-1,2,3,4-tetrazole,
(33) 5-(2-chlorophenyl)-1-(4-methoxybenzyl)-1H-1,2,3,4-tetrazole,
(34) 1-benzyl-5-(2-chlorophenyl)-1H-1,2,3,4-tetrazole,
(35) 1-(3,5-dimethyl-4-hydroxybenzyl)-5-phenyl-1H-1,2,3,4-tetrazole,
(36) 1-(4-bromobenzyl)-5-(3-cyanophenyl)-1H-1,2,3,4-tetrazole,
(37) 5-(3-cyanophenyl)-1-[4-(2-trifluoromethylphenyl)benzyl]-1H-1,2,3,4-tetrazole,
(38) 5-(3-ethoxycarbonylmethyloxy-5-iodophenyl)-1-(4-methoxybenzyl)-1H-1,2,3,4-tetrazole,
(39) 5-(3-iodo-2-isopropyloxyphenyl)-1-(4-methoxybenzyl)-1H-1,2,3,4-tetrazole,
(40) 5-(4-formylphenyl)-1-(3-methoxycarbonylbenzyl)-1H-1,2,3,4-tetrazole,
(41) 5-(2-aminophenyl)-1-benzyl-1H-1,2,3,4-tetrazole,
(42) 5-(2-acetylaminophenyl)-1-benzyl-1H-1,2,3,4-tetrazole,
(43) 5-phenyl-1-benzyl-1H-1,2,3,4-tetrazole,
(44) 5-benzyl-1-(4-mercaptophenyl)-1H-1,2,3,4-tetrazole,
(45) 5-benzyl-1-phenyl-1H-1,2,3,4-tetrazole,
(46) 5-benzyl-1-(3,5-dimethyl-4-hydroxybenzyl)-1H-1,2,3,4-tetrazole,
(47) 1-benzyl-5-benzyl-1H-1,2,3,4-tetrazole,
(48) 5-phenyl-1-[2-(2-pyridyl)ethyl)-1H-1,2,3,4-tetrazole,
(49) 5-(2,4-dimethoxy-5-ethylphenethyl)-1-benzyl-1H-1,2,3,4-tetrazole,
(50) 5-(2,4-diethoxy-5-ethylphenethyl)-1-benzyl-1H-1,2,3,4-tetrazole,
(51) 5-(2,4-diethoxy-5-ethylphenethyl)-1-phenethyl-1H-1,2,3,4-tetrazole,
(52) 5-(2,4-dimethoxy-5-ethylphenethyl)-1-phenethyl-1H-1,2,3,4-tetrazole,
(53) 5-benzyl-1-N,N-diisopropylaminocarbonylbenzyl-1H-1,2,3,4-tetrazole,
(54) 5-[4-(4-acetyl-3-hydroxy-2-propylphenyloxy)phenyl]-1-(4-cyanobenzyl)-1H-1,2,3,4-tetrazole,
(55) 5-benzyl-1-(4-amino-2,5-dimethoxyphenyl)-1H-1,2,3,4-tetrazole,
(56) 5-(2-aminophenyl)-1-benzyl-1H-1,2,3,4-tetrazole,
(57) 5-(4-t-butylcarbonyloxyphenyl)-1-(4-aminomethylbenzyl)-1H-1,2,3,4-tetrazole,
(58) 5-(4-t-butylcarbonyloxyphenyl)-1-(4-isopropylbenzyl)-1H-1,2,3,4-tetrazole,
(59) 5-(4-t-butylcarbonyloxyphenyl)-1-(4-dimethylaminobenzyl)-1H-1,2,3,4-tetrazole,
(60) 5-(4-t-butylcarbonyloxyphenyl)-1-(4-N-t-butoxycarbonyl-N-methylbenzyl)-1H-1,2,3,4-tetrazole,
(61) 5-(1-hydroxycycloheptyl)-1-benzyl-1H-1,2,3,4-tetrazole,
(62) 5-(1-naphthylmethyl)-1-(2-carboxyphenyl)-1H-1,2,3,4-tetrazole,
(63) 5-(4-carboxy-2-methoxyphenyl)-1-(4-methoxybenzyl)-1H-1,2,3,4-tetrazole,
(64) 5-[4-methoxycarbonyl-3-(2-methylphenyl)benzyl]-1-benzyl-1H-1,2,3,4-tetrazole,
(65) 5-[4-carboxy-3-(2-methylphenyl)benzyl]-1-benzyl-1H-1,2,3,4-tetrazole.

13. The compound according to claim 11, wherein either ring A or ring B is a carboring and the other is a heteroring, or a non-toxic salt thereof, wherein the following (1) to (8) are excluded;
(1) 1-benzyl-5-(5-nitrofuran-2-yl)-1H-1,2,3,4-tetrazole,
(2) 1-(3,5-dimethyl-4-hydroxybenzyl)-5-(4-pyridyl)1H-1,2,3,4-tetrazole,
(3) 5-(5-bromofuran-2-yl)-1-(4-methoxybenzyl)-1H-1,2,3,4-tetrazole,
(4) 5-(6-bromopyridin-2-yl)-1-(4-methoxybenzyl)-1H-1,2,3,4-tetrazole,
(5) 5-(6-iodopyridin-2-yl)-1-(4-methoxybenzyl)-1H-1,2,3,4-tetrazole,
(6) 5-(2-furyl)-1-benzyl-1H-1,2,3,4-tetrazole,
(7) 5-[2-(5-nitro)thieno]-1-benzyl-1H-1,2,3,4-tetrazole,
(8) 5-thieno-1-benzyl-1H-1,2,3,4-tetrazole.

14. The compound according to claim 11, wherein both ring A and ring B are heterorings among the compound of formula (I-a') or a non-toxic salt thereof.

15. The compound according to claim 11, which is selected from
(1) 1-(2-chlorobenzyl)-5-(2-methoxyphenyl)-1H-1,2,3,4-tetrazole,
(2) 1-(3-methoxybenzyl)-5-phenyl-1H-1,2,3,4-tetrazole,
(3) 1-(2,6-dichlorobenzyl)-5-phenyl-1H-1,2,3,4-tetrazole,
(4) 1-benzyl-5-(2-trifluoromethylphenyl)-1H-1,2,3,4-tetrazole,
(5) 5-(2-chlorophenyl)-1-(2,6-dichlorobenzyl)-1H-1,2,3,4-tetrazole,
(6) 1-(4-methylbenzyl)-5-(2-trifluoromethylphenyl)-1H-1,2,3,4-tetrazole,
(7) 1-(2-chlorobenzyl)-5-phenyl-1H-1,2,3,4-tetrazole,
(8) 1-(2-chloro-6-fluorobenzyl)-5-(2-trifluoromethylphenyl)-1H-1,2,3,4-tetrazole,
(9) 1-(2-chlorobenzyl)-5-(3-methoxyphenyl)-1H-1,2,3,4-tetrazole,
(10) 1-(2-chlorobenzyl)-5-(4-methoxyphenyl)-1H-1,2,3,4-tetrazole,
(11) 1-(2-methoxybenzyl)-5-phenyl-1H-1,2,3,4-tetrazole,
(12) 1-(4-methoxybenzyl)-5-phenyl-1H-1,2,3,4-tetrazole,
(13) 1-(2-chlorobenzyl)-5-(4-dimethylaminophenyl)-1H-1,2,3,4-tetrazole,
(14) 1-(2-chlorobenzyl)-5-(3-dimethylaminophenyl)-1H-1,2,3,4-tetrazole,
(15) 1-(2-chlorobenzyl)-5-(2-dimethylaminophenyl)-1H-1,2,3,4-tetrazole,
(16) 5-(2-chlorophenyl)-1-(2-methylbenzyl)-1H-1,2,3,4-tetrazole,
(17) 5-(2-chlorophenyl)-1-(2,6-difluorobenzyl)-1H-1,2,3,4-tetrazole,
(18) 1-(3-dimethylaminobenzyl)-5-phenyl-1H-1,2,3,4-tetrazole,
(19) 1-(2-chloro-6-fluorobenzyl)-5-(2-methylphenyl)-1H-1,2,3,4-tetrazole,
(20) 1-(2-chloro-6-fluorobenzyl)-5-(2-chlorophenyl)-1H-1,2,3,4-tetrazole,
(21) 1-(2-chloro-6-fluorobenzyl)-5-(2-methoxyphenyl)-1H-1,2,3,4-tetrazole,
(22) 5-(2-chlorophenyl)-1-(3,4-dichlorobenzyl)-1H-1,2,3,4-tetrazole,
(23) 5-(2-chlorophenyl)-1-(4-trifluoromethylbenzyl)-1H-1,2,3,4-tetrazole,
(24) 1-(2-chloro-6-fluorobenzyl)-5-[2-(4-methoxyphenyloxymethyl)phenyl]-1H-1,2,3,4-tetrazole,
(25) 5-(2-benzyloxyphenyl)-1-(2-chloro-6-fluorobenzyl)-1H-1,2,3,4-tetrazole,
(26) 1-(2-chloro-6-fluorobenzyl)-5-(2,3-dichlorophenyl)-1H-1,2,3,4-tetrazole,
(27) 1-(4-fluorobenzyl)-5-(4-fluorophenyl)-1H-1,2,3,4-tetrazole,
(28) 5-(2,6-dichlorophenyl)-1-(4-fluorobenzyl)-1H-1,2,3,4-tetrazole,
(29) 5-(2,5-dichlorophenyl)-1-(4-fluorobenzyl)-1H-1,2,3,4-tetrazole,
(30) 5-(2-chloro-4-fluorophenyl)-1-(4-fluorobenzyl)-1H-1,2,3,4-tetrazole,
(31) 5-(2,3-dichlorophenyl)-1-cyclohexylinethyl-1H-1,2,3,4-tetrazole,
(32) 5-(2,3-dichlorophenyl)-1-(4-fluorobenzyl)-1H-1,2,3,4-tetrazole,
(33) 1-(2-chloro-6-fluorobenzyl)-5-cyclohexyl-1H-1,2,3,4-tetrazole,
(34) 1-(2-chloro-6-fluorobenzyl)-5-(1-naphthyl)-1H-1,2,3,4-tetrazole,
(35) 1-benzyl-5-(2,5-dichlorophenyl)-1H-1,2,3,4-tetrazole,
(36) 1-(2-chloro-6-fluorobenzyl)-5-(2,5-dichlorophenyl)-1H-1,2,3,4-tetrazole,
(37) 1-(2-chloro-6-phenoxybenzyl)-5-(2,3-dichlorophenyl)-1H-1,2,3,4-tetrazole,
(38) 1-(2-chloro-6-fluorobenzyl)-5-(2-naphthyl)-1H-1,2,3,4-tetrazole,
(39) 5-(2,4-dichlorophenyl)-1-(4-fluorobenzyl)-1H-1,2,3,4-tetrazole,
(40) 5-(2-chloro-4-nitrophenyl)-1-(4-fluorobenzyl)-1H-1,2,3,4-tetrazole,
(41) 5-(4-cyanophenyl)-1-(4-fluorobenzyl)-1H-1,2,3,4-tetrazole,
(42) 1-(4-fluorobenzyl)-5-(4-methylphenyl)-1H-1,2,3,4-tetrazole,
(43) 5-(2,5-dichlorophenyl)-1-(4-fluoro-2-trifluoromethylbenzyl)-1H-1,2,3,4-tetrazole,
(44) 5-(2,3-dichlorophenyl)-1-(4-fluoro-2-trifluoromethylbenzyl)-1H-1,2,3,4-tetrazole,
(45) 1-(2-chloro-4-fluorobenzyl)-5-(2,5-dichlorophenyl)-1H-1,2,3,4-tetrazole,
(46) 1-(2-chloro-4-fluorobenzyl)-5-(2,3-dichlorophenyl)-1H-1,2,3,4-tetrazole,
(47) 5-(2,5-dichlorophenyl)-1-(2,5-difluorobenzyl)-1H-1,2,3,4-tetrazole,
(48) 5-(2-chlorophenyl)-1-(2,5-dichlorobenzyl)-1H-1,2,3,4-tetrazole,
(49) 5-(2-chlorophenyl)-1-(2,5-difluorobenzyl)-1H-1,2,3,4-tetrazole,
(50) 1-(2-chloro-4-fluorobenzyl)-5-(2-trifluoromethylphenyl)-1H-1,2,3,4-tetrazole,
(51) 1-(4-fluoro-2-trifluoromethylbenzyl)-5-(5-fluoro-2-trifluoromethylphenyl)-1H-1,2,3,4-tetrazole,
(52) 1-(2-chloro-4-fluorobenzyl)-5-(5-fluoro-2-trifluoromethylphenyl)-1H-1,2,3,4-tetrazole,
(53) 1-(2,4-dichlorobenzyl)-5-(2,3-dichlorophenyl)-1H-1,2,3,4-tetrazole,
(54) 1-(2,5-dichlorobenzyl)-5-(2,3-dichlorophenyl)-1H-1,2,3,4-tetrazole,
(55) 5-(2,3-dichlorophenyl)-1-(2-phenoxybenzyl)-1H-1,2,3,4-tetrazole,
(56) 1-(2-benzyloxybenzyl)-5-(2,3-dichlorophenyl)-1H-1,2,3,4-tetrazole,
(57) 1-(2-benzyloxy-5-chlorobenzyl)-5-(2,3-dichlorophenyl)-1H-1,2,3,4-tetrazole,
(58) 1-(5-chloro-2-methoxybenzyl)-5-(2,3-dichlorophenyl)-1H-1,2,3,4-tetrazole,
(59) 1-(5-chloro-2-isopropyloxybenzyl)-5-(2,3-dichlorophenyl)-1H-1,2,3,4-tetrazole,
(60) 1-(5-chloro-2-ethoxycarbonylmethyloxybenzyl)-5-(2,3-dichlorophenyl)-1H-1,2,3,4-tetrazole,
(61) 1-(2,3-dichlorobenzyl)-5-(2,3-dichlorophenyl)-1H-1,2,3,4-tetrazole,
(62) 5-(2,5-dichlorobenzyl)-1-(2,3-dichlorophenyl)-1H-1,2,3,4-tetrazole,
(63) 1-(2-benzylbenzyl)-5-(2,3-dichlorophenyl)-1H-1,2,3,4-tetrazole,
(64) 1-(2,3-dichlorobenzyl)-5-(2,3-dichlorobenzyl)-1H-1,2,3,4-tetrazole,
(65) 5-(2,3-dichlorobenzyl)-1-(2,3-dichlorophenyl)-1H-1,2,3,4-tetrazole,
(66) 5-(2,3-dichlorophenyl)-1-(2-phenoxymethylbenzyl)-1H-1,2,3,4-tetrazole,
(67) 5-(2,3-dichlorophenyl)-1-(2-phenethylbenzyl)-1H-1,2,3,4-tetrazole,
(68) 1-(2-chloro-6-methoxybenzyl)-5-(2,3-dichlorophenyl)-1H-1,2,3,4-tetrazole,
(69) 5-(2,3-dichlorophenyl)-1-[2-(N-methyl-N-phenylamino)benzyl]-1H-1,2,3,4-tetrazole,
(70) 1-(2-benzyloxy-6-chlorobenzyl)-5-(2,3-dichlorophenyl)-1H-1,2,3,4-tetrazole,
(71) 1-(2-benzyloxy-4-chlorobenzyl)-5-(2,3-dichlorophenyl)-1H-1,2,3,4-tetrazole,
(72) 1-(benzocyclohepten-5-yl)-5-(2,3-dichlorophenyl)-1H-1,2,3,4-tetrazole,
(73) 1-(2-benzyloxy-5-nitrobenzyl)-5-(2,3-dichlorophenyl)-1H-1,2,3,4-tetrazole,
(74) 1-(2-benzyloxy-5-fluorobenzyl)-5-(2,3-dichlorophenyl)-1H-1,2,3,4-tetrazole,
(75) 1-(2-benzyloxy-5-methylbenzyl)-5-(2,3-dichlorophenyl)-1H-1,2,3,4-tetrazole,
(76) 1-(2-benzyloxy-5-dimethylaminobenzyl)-5-(2,3-dichlorophenyl)-1H-1,2,3,4-tetrazole,
(77) 1-(2-benzyloxybenzyl)-5-(2,3-dichlorophenyl)-1H-1,2,3,4-tetrazole,
(78) 5-(2,3-dichlorophenyl)-1-(2-nitrobenzyl)-1H-1,2,3,4-tetrazole,
(79) 5-(2-benzyloxy-5-chlorophenyl)-1-(2,3-dichlorophenyl)-1H-1,2,3,4-tetrazole,
(80) 1-(2-benzyloxy-5-chlorobenzyl)-5-(2,3-dimethylphenyl)-1H-1,2,3,4-tetrazole,
(81) 5-(2,3-dichlorophenyl)-1-(2,5-dimethylbenzyl)-1H-1,2,3,4-tetrazole,
(82) 1-(2-benzyloxy-5-chlorobenzyl)-5-(2-chlorophenyl)-1H-1,2,3,4-tetrazole,
(83) 1-(2-benzyloxy-5-chlorobenzyl)-5-(2-methylphenyl)-1H-1,2,3,4-tetrazole,
(84) 1-(2-benzylaminobenzyl)-5-(2,3-dichlorophenyl)-1H-1,2,3,4-tetrazole,
(85) 5-(2,3-dichlorophenyl)-1-(2,4-dimethylbenzyl)-1H-1,2,3,4-tetrazole,
(86) 1-(2-benzyloxy-5-chlorobenzyl)-5-(3-methylphenyl)-1H-1,2,3,4-tetrazole,
(87) 1-(2-chloro-6-fluorobenzyl)-5-(2-nitrophenyl)-1H-1,2,3,4-tetrazole,
(88) 1-(4-fluorobenzyl)-5-(4-nitrophenyl)-1H-1,2,3,4-tetrazole,
(89) 1-(4-fluorobenzyl)-5-(2-nitrophenyl)-1H-1,2,3,4-tetrazole,
(90) 5-(2-benzyloxy-3-methoxyphenyl)-1-(4-fluoro-2-trifluoromethylbenzyl)-1H-1,2,3,4-tetrazole,
(91) 5-(2,3-dichlorophenyl)-1-(2-phenylbenzyl)-1H-1,2,3,4-tetrazole,
(92) 5-(2,4-dichlorophenyl)-1-(2-phenoxybenzyl)-1H-1,2,3,4-tetrazole,
(93) 5-(2,5-dichlorophenyl)-1-(2-phenoxybenzyl)-1H-1,2,3,4-tetrazole,
(94) 5-(2-chloro-5-methoxymethoxyphenyl)-1-(5-chloro-2-phenoxybenzyl)-1H-1,2,3,4-tetrazole,
(95) 1-(2-benzyloxy-5-chlorobenzyl)-5-phenyl-1H-1,2,3,4-tetrazole,
(96) 1-(2-benzyloxy-5-chlorobenzyl)-5-(3-chlorophenyl)-1H-1,2,3,4-tetrazole,
(97) 1-(5-chloro-2-cyclohexylmethyloxybenzyl)-5-(2,3-dichlorophenyl)-1H-2,2,3,4-tetrazole,
(98) 1-[5-chloro-2-(4-pyridylmethyloxy)benzyl]-5-(2,3-dichlorophenyl)-1H-1,2,3,4-tetrazole,
(99) 1-[2-(benzodioxan-2-ylmethyloxy)-5-chlorobenzyl]-5-(2,3-dichlorophenyl)-1H-1,2,3,4-tetrazole,
(100) 1-(2-benzyloxy-5-chlorobenzyl)-5-(2-chloro-3-methylphenyl)-1H-1,2,3,4-tetrazole,
(101) 5-(2,3-chlorophenyl)-1-(1-phenylethyl)-1H-1,2,3,4-tetrazole,
(102) 1-(5-chloro-2-phenethyloxybenzyl)-5-(2,3-dichlorophenyl)-1H-1,2,3,4-tetrazole,
(103) 1-(2-cyclohexyloxy-5-chlorobenzyl)-5-(2,3-dichlorophenyl)-1H-1,2,3,4-tetrazole,
(104) 5-(2-aminophenyl)-1-(2-chloro-6-fluorobenzyl)-1H-1,2,3,4-tetrazole,
(105) 1-(2-benzyloxy-5-aminobenzyl)-5-(2,3-dichlorophenyl)-1H-1,2,3,4-tetrazole,
(106) 1-(2-aminobenzyl)-5-(2,3-dichlorophenyl)-1H-1,2,3,4-tetrazole,
(107) 5-(2-hydroxyphenyl)-1-(2-chloro-6-fluorobenzyl)-1H-1,2,3,4-tetrazole,
(108) 5-(2-hydroxymethylphenyl)-1-(2-chloro-6-fluorobenzyl)-1H-1,2,3,4-tetrazole,
(109) 1-(5-chloro-2-hydroxybenzyl)-5-(2,3-dichlorophenyl)-1H-1,2,3,4-tetrazole,
(110) 1-(4-fluoro-2-trifluoromethylbenzyl)-5-(2-hydroxy-3-methoxyphenyl)-1H-1,2,3,4-tetrazole,
(111) 1-(2-chloro-4-hydroxybenzyl)-5-(2,3-dichlorophenyl)-1H-1,2,3,4-tetrazole,
(112) 1-(2-benzyloxy-5-chlorobenzyl)-5-(2-chloro-4-hydroxyphenyl)-1H-1,2,3,4-tetrazole,
(113) 1-phenyl-5-(2,6-dimethoxyphenoxy)-1H-1,2,3,4-tetrazole,
(114) 1-phenyl-5-phenoxy-1H-1,2,3,4-tetrazole,
(115) 1-[5-chloro-2-(2,2-dimethyl-1,3-dioxolan-4-ylmethyloxy)benzyl]-5-(2,3-dichlorophenyl)-1H-1,2,3,4-tetrazole,
(116) 1-[5-chloro-2-(4-dimethylaminobenzyloxy)benzyl]-5-(2,3-dichlorophenyl)-1H-1,2,3,4-tetrazole,
(117) 1-[5-chloro-2-(2-morpholinoethyloxy)benzyl]-5-(2,3-dichlorophenyl)-1H-1,2,3,4-tetrazole,
(118) 1-(2-benzyloxy-5-chlorobenzyl)-5-(2,4-dichlorophenyl)-1H-1,2,3,4-tetrazole,
(119) 1-[5-chloro-2-(3-pyridylmethyloxy)benzyl]-5-(2,3-dichlorophenyl)-1H-1,2,3,4-tetrazole,
(120) 1-[5-chloro-2-(2-pyridylmethyloxy)benzyl]-5-(2,3-dichlorophenyl)-1H-1,2,3,4-tetrazole,
(121) 1-(2-benzyloxy-5-chlorobenzyl)-5-(2-chloro-3-nitrophenyl)-1H-1,2,3,4-tetrazole,
(122) 1-(2-benzyloxy-5-chlorobenzyl)-5-(2,5-dichlorophenyl)-1H-1,2,3,4-tetrazole,
(123) 1-(2-benzyloxy-5-chlorobenzyl)-5-(2,6-dichlorophenyl)-1H-1,2,3,4-tetrazole,
(124) 1-[5-chloro-2-(N-methyl-2,3,4,5,6,7-hexahydroazepin-3-yl)benzyl]-5-(2,3-dichlorophenyl)-1H-1,2,3,4-tetrazole,
(125) 1-[5-chloro-2-(N-methylpiperidin-2-ylmethyloxy)benzyl]-5-(2,3-dichlorophenyl)-1H-1,2,3,4-tetrazole,
(126) 1-(2-benzyloxy-5-chlorobenzyl)-5-(3-chloro-2-methylphenyl)-1H-1,2,3,4-tetrazole,
(127) 1-[5-chloro-2-(2-methylphenylamino)ethyloxybenzyl]-5-(2,3-dichlorophenyl)-1H-1,2,3,4-tetrazole,
(128) 1-(2-benzyloxy-5-chlorobenzyl)-5-(3-methoxymethoxy-2-methylphenyl)-1H-1,2,3,4-tetrazole,
(129) 1-[5-chloro-2-(tetrahydropyran-2-ylmethyloxy)-benzyl]-5-(2,3-dichlorophenyl)-1H-1,2,3,4-tetrazole,
(130) 1-(2-benzyloxy-5-chlorobenzyl)-5-(4-methoxymethoxyphenyl)-1H-1,2,3,4-tetrazole,
(131) 1-(2-benzyloxy-5-chlorobenzyl)-5-(4-chlorophenyl)-1H-1,2,3,4-tetrazole,
(132) 5-(2,3-dichlorophenyl)1-(2-phenethyloxybenzyl)-1H-1,2,3,4-tetrazole,
(133) 1-(2-cyclohexylmethyloxybenzyl)-5-(2,3-dichlorophenyl)-1H-1,2,3,4-tetrazole,
(134) 5-(2,3-dichlorophenyl)-1-[2-(4-pyridylmethyloxy)benzyl]-1H-1,2,3,4-tetrazole,
(135) 5-(2,3-dichlorophenyl)-1-[2-(3-pyridylmethyloxy)benzyl]-1H-1,2,3,4-tetrazole,
(136) 1-(2,3-dichlorophenyl)-5-[2-(2-morpholinoethyloxy)benzyl]-1H-1,2,3,4-tetrazole,
(137) 1-[2-(2H,3H-benzo[e] 1,4-dioxan-2-ylmethyloxy)benzyl]-5-(2,3-dichlorophenyl)-1H-1,2,3,4-tetrazole,
(138) 5-(2,3-dichlorophenyl)-1-[2-(2-morpholinoethyloxy)benzyl]-1H-1,2,3,4-tetrazole,
(139) 5-(2,3-dichlorophenyl)-1-[2-(2-pyridylmethyloxy)benzyl]-1H-1,2,3,4-tetrazole,
(140) 1-(2-chloro-6-phenoxybenzyl)-5-(2,3-dimethylphenyl)-1H-1,2,3,4-tetrazole,
(141) 1-(2-benzyloxybenzyl)-5-(2,3-dimethylphenyl)-1H-1,2,3,4-tetrazole,
(142) 5-(2,3-dichlorophenyl)-1-[2-(2,2-dimethyl-1,3-dioxolan-4-ylmethyloxy)benzyl]-1H-1,2,3,4-tetrazole,
(143) 5-(2,3-dichlorophenyl)-1-[2-[2-(N-methyl-N-phenylamino)ethyloxy]benzyl]-1H-1,2,3,4-tetrazole,
(144) 1-(2-cyclohexyloxybenzyl)-5-(2,3-dichlorophenyl)-1H-1,2,3,4-tetrazole,
(145) 1-(2-benzyloxyphenethyl)-5-(2,3-dimethylphenyl)-1H-1,2,3,4-tetrazole,
(146) 1-(4-benzyloxy-2-chlorobenzyl)-5-(2,3-dichlorophenyl)-1H-1,2,3,4-tetrazole,
(147) 1-[5-chloro-2-(4-methoxymethyloxybenzyloxy)benzyl]-5-(2,3-dichlorophenyl)-1H-1,2,3,4-tetrazole,
(148) 5-(2,3-dichlorophenyl)-1-(2-hydroxybenzyl)-1H-1,2,3,4-tetrazole,
(149) 1-(2,3-dichlorophenyl)-5-(2-hydroxybenzyl)-1H-1,2,3,4-tetrazole,
(150) 1-(2-benzyloxy-5-chlorobenzyl)-5-(3-hydroxy-2-methylphenyl)-1H-1,2,3,4-tetrazole,
(151) 1-(2-benzyloxy-5-chlorobenzyl)-5-(4-hydroxyphenyl)-1H-1,2,3,4-tetrazole, and
(152) 5-(3-amino-2-chlorophenyl)-1-(2-benzyloxy-5-chlorobenzyl)-1H-1,2,3,4-tetrazole, or a non-toxic salt thereof.

16. The compound according to claim 11, which is
(1) 5-(2-chlorophenyl)-1-(pyridin-2-ylmethyl)-1H-1,2,3,4-tetrazole,
(2) 5-(2-chlorophenyl)-1-(pyridin-3-ylmethyl)-1H-1,2,3,4-tetrazole,
(3) 5-(2-chlorophenyl)-1-(pyridin-4-ylmethyl)-1H-1,2,3,4-tetrazole,
(4) 1-(2,6-dichlorobenzyl)-5-(2-pyridyl)-1H-1,2,3,4-tetrazole,
(5) 1-(2-chlorobenzyl)-5-(4-pyridyl)-1H-1,2,3,4-tetrazole,
(6) 1-(2-chloro-6-fluorobenzyl)-5-(2-furyl)-1H-1,2,3,4-tetrazole,
(7) 1-(4-fluorobenzyl)-5-(3-thienyl)-1H-1,2,3,4-tetrazole,
(8) 5-[2-(5-chlorothienyl)]-1-(4-fluorobenzyl)-1H-1,2,3,4-tetrazole, or
(9) 1-(2-benzyloxy-5-chlorobenzyl)-5-(2,3-dihydrobenzofuran-7-yl)-1H-1,2,3,4-tetrazole, or
a non-toxic salt thereof.

17. The mitochondrial benzodiazepine receptor antagonist according to claim 6, wherein the compound of formula (I-a') or the non-toxic salt thereof is selected from
(1) 1-(2-chlorobenzyl)-5-(2-methoxyphenyl)-1H-1,2,3,4-tetrazole,
(2) 1-(3-methoxybenzyl)-5-phenyl-1H-1,2,3,4-tetrazole,
(3) 1-(2,6-dichlorobenzyl)-5-phenyl-1H-1,2,3,4-tetrazole,
(4) 1-benzyl-5-phenyl-1H-1,2,3,4-tetrazole,
(5) 1-benzyl-5-(2-trifluoromethylphenyl)-1H-1,2,3,4-tetrazole,
(6) 1-benzyl-5-(2-chlorophenyl)-1H-1,2,3,4-tetrazole,
(7) 5-(2-chlorophenyl)-1-(2,6-dichlorobenzyl)-1H-1,2,3,4-tetrazole,
(8) 5-(2-chlorophenyl)-1-(pyridin-2-ylmethyl)-1H-1,2,3,4-tetrazole,
(9) 5-(2-chlorophenyl)-1-(pyridin-3-ylmethyl)-1H-1,2,3,4-tetrazole,
(10) 5-(2-chlorophenyl)-1-(pyridin-4-ylmethyl)-1H-1,2,3,4-tetrazole,
(11) 1-(4-methylbenzyl)-5-(2-trifluoromethylphenyl)-1H-1,2,3,4-tetrazole,
(12) 1-(2,6-dichlorobenzyl)-5-(2-pyridyl)-1H-1,2,3,4-tetrazole,
(13) 1-(2-chlorobenzyl)-5-phenyl-1H-1,2,3,4-tetrazole,
(14) 1-(2-chlorobenzyl)-5-(3-pyridyl)-1H-1,2,3,4-tetrazole,
(15) 1-(2-chloro-6-fluorobenzyl)-5-(2-trifluoromethylphenyl)-1H-1,2,3,4-tetrazole,
(16) 1-(2-chlorobenzyl)-5-(3-methoxyphenyl)-1H-1,2,3,4-tetrazole,
(17) 1-(2-chlorobenzyl)-5-(4-methoxyphenyl)-1H-1,2,3,4-tetrazole,
(18) 1-(2-methoxybenzyl)-5-phenyl-1H-1,2,3,4-tetrazole,
(19) 1-(4-methoxybenzyl)-5-phenyl-1H-1,2,3,4-tetrazole,
(20) 1-(2-chlorobenzyl)-5-(4-dimethylaminophenyl)-1H-1,2,3,4-tetrazole,
(21) 1-(2-chlorobenzyl)-5-(3-dimethylaminophenyl)-1H-1,2,3,4-tetrazole,
(22) 1-(2-chlorobenzyl)-5-(2-dimethylaminophenyl)-1H-1,2,3,4-tetrazole,
(23) 5-(2-chlorophenyl)-1-(2-methylbenzyl)-1H-1,2,3,4-tetrazole,
(24) 5-(2-chlorophenyl)-1-(2,6-difluorobenzyl)-1H-1,2,3,4-tetrazole,
(25) 1-(3-dimethylaminobenzyl)-5-phenyl-1H-1,2,3,4-tetrazole,
(26) 1-(2-chloro-6-fluorobenzyl)-5-(2-methylphenyl)-1H-1,2,3,4-tetrazole,
(28) 1-(2-chloro-6-fluorobenzyl)-5-(2-methoxyphenyl)-1H-1,2,3,4-tetrazole,
(29) 1-(2-chloro-6-fluorobenzyl)-5-(2-furyl)-1H-1,2,3,4-tetrazole,
(30) 5-(2-chlorophenyl)-1-(3,4-dichlorobenzyl)-1H-1,2,3,4-tetrazole,
(31) 5-(2-chlorophenyl)-1-(4-trifluoromethylbenzyl)-1H-1,2,3,4-tetrazole,
(32) 1-(2-chloro-6-fluorobenzyl)-5-[2-(4-methoxyphenyloxymethyl)phenyl]-1H-1,2,3,4-tetrazole,
(33) 5-(2-benzyloxyphenyl)-1-(2-chloro-6-fluorobenzyl)-1H-1,2,3,4-tetrazole,
(34) 1-(2-chloro-6-fluorobenzyl)-5-(2,3-dichlorophenyl)-1H-1,2,3,4-tetrazole,
(35) 1-(4-fluorobenzyl)-5-(4-fluorophenyl)-1H-1,2,3,4-tetrazole,
(36) 5-(2,6-dichlorophenyl)-1-(4-fluorobenzyl)-1H-1,2,3,4-tetrazole,
(37) 5-(2,5-dichlorophenyl)-1-(4-fluorobenzyl)-1H-1,2,3,4-tetrazole,
(38) 5-(2-chloro-4-fluorophenyl)-1-(4-fluorobenzyl)-1H-1,2,3,4-tetrazole,
(39) 5-(2,3-dichlorophenyl)-1-cyclohexylmethyl-1H-1,2,3,4-tetrazole,
(40) 5-(2,3-dichlorophenyl)-1-(4-fluorobenzyl)-1H-1,2,3,4-tetrazole,
(41) 1-(2-chloro-6-fluorobenzyl)-5-cyclohexyl-1H-1,2,3,4-tetrazole,
(42) 1-(2-chloro-6-fluorobenzyl)-5-(1-naphthyl)-1H-1,2,3,4-tetrazole,
(43) 1-benzyl-5-(2,5-dichlorophenyl)-1H-1,2,3,4-tetrazole,
(44) 1-(2-chloro-6-fluorobenzyl)-5-(2,5-dichlorophenyl)-1H-1,2,3,4-tetrazole,
(45) 1-(2-chloro-6-phenoxybenzyl)-5-(2,3-dichlorophenyl)-1H-1,2,3,4-tetrazole,
(46) 1-(2-chloro-6-fluorobenzyl)-5-(2-naphthyl)-1H-1,2,3,4-tetrazole,
(47) 5-(2,4-dichlorophenyl)-1-(4-fluorobenzyl)-1H-1,2,3,4-tetrazole,
(48) 5-(2-chloro-4-nitrophenyl)-1-(4-fluorobenzyl)-1H-1,2,3,4-tetrazole,
(49) 5-(4-cyanophenyl)-1-(4-fluorobenzyl)-1H-1,2,3,4-tetrazole,
(50) 1-(4-fluorobenzyl)-5-(4-methylphenyl)-1H-1,2,3,4-tetrazole,
(51) 5-(2,5-dichlorophenyl)- 1-(4-fluoro-2-trifluoromethylbenzyl)-1H-1,2,3,4-tetrazole,
(52) 5-(2,3-dichlorophenyl)-1-(4-fluoro-2-trifluoromethylbenzyl)-1H-1,2,3,4-tetrazole,
(53) 1-(2-chloro-4-fluorobenzyl)-5-(2,5-dichlorophenyl)-1H-1,2,3,4-tetrazole,
(54) 1-(2-chloro-4-fluorobenzyl)-5-(2,3-dichlorophenyl)-1H-1,2,3,4-tetrazole,
(55) 5-(2,5-dichlorophenyl)-1-(2,5-difluorobenzyl)-1H-1,2,3,4-tetrazole,
(56) 5-(2-chlorophenyl)-1-(2,5-dichlorobenzyl)-1H-1,2,3,4-tetrazole,
(57) 5-(2-chlorophenyl)-1-(2,5-difluorobenzyl)-1H-1,2,3,4-tetrazole,
(58) 1-(4-fluorobenzyl)-5-(3-thienyl)-1H-1,2,3,4-tetrazole,
(59) 5-[2-(5-chlorothienyl)]-1-(4-fluorobenzyl)-1H-1,2,3,4-tetrazole,
(60) 1-(2-chloro-4-fluorobenzyl)-5-(2-trifluoromethylphenyl)-1H-1,2,3,4-tetrazole,
(61) 1-(4-fluoro-2-trifluoromethylbenzyl)-5-(5-fluoro-2-trifluoromethylphenyl)-1H-1,2,3,4-tetrazole,
(62) 1-(2-chloro-4-fluorobenzyl)-5-(5-fluoro-2-trifluoromethylphenyl)-1H-1,2,3,4-tetrazole,
(63) 1-(2,4-dichlorobenzyl)-5-(2,3-dichlorophenyl)-1H-1,2,3,4-tetrazole,
(64) 1-(2,5-dichlorobenzyl)-5-(2,3-dichlorophenyl)-1H-1,2,3,4-tetrazole,
(65) 5-(2,3-dichlorophenyl)-1-(2-phenoxybenzyl)-1H-1,2,3,4-tetrazole,
(66) 1-(2-benzyloxybenzyl)-5-(2,3-dichlorophenyl)-1H-1,2,3,4-tetrazole,
(67) 1-(2-benzyloxy-5-chlorobenzyl)-5-(2,3-dichlorophenyl)-1H-1,2,3,4-tetrazole,
(68) 1-(5-chloro-2-methoxybenzyl)-5-(2,3-dichlorophenyl)-1H-1,2,3,4-tetrazole,
(69) 1-(5-chloro-2-isopropyloxybenzyl)-5-(2,3-dichlorophenyl)-1H-1,2,3,4-tetrazole,
(70) 1-(5-chloro-2-ethoxycarbonylmethyloxybenzyl)-5-(2,3-dichlorophenyl)-1H-1,2,3,4-tetrazole,
(71) 1-(2,3-dichlorobenzyl)-5-(2,3-dichlorophenyl)-1H-1,2,3,4-tetrazole,
(72) 5-(2,5-dichlorobenzyl)-1-(2,3-dichlorophenyl)-1H-1,2,3,4-tetrazole,
(73) 1-(2-benzylbenzyl)-5-(2,3-dichlorophenyl)-1H-1,2,3,4-tetrazole,
(74) 1-(2,3-dichlorobenzyl)-5-(2,3-dichlorobenryl)-1H-1,2,3,4-tetrazole,
(75) 5-(2,3-dichlorobenzyl)-1-(2,3-dichlorophenyl)-1H-1,2,3,4-tetrazole,
(76) 5-(2,3-dichlorophenyl)-1-(2-phenoxymethylbenzyl)-1H-1,2,3,4-tetrazole,
(77) 5-(2,3-dichlorophenyl)-1-(2-phenethylbenzyl)-1H-1,2,3,4-tetrazole,
(78) 1-(2-chloro-6-methoxybenzyl)-5-(2,3-dichlorophenyl)-1H-1,2,3,4-tetrazole,
(79) 5-(2,3-dichlorophenyl)-1-[2-(N-methyl-N-phenylamino)benzyl]-1H-1,2,3,4-tetrazole,
(80) 1-(2-benzyloxy-6-chlorobenzyl)-5-(2,3-dichlorophenyl)-1H-1,2,3,4-tetrazole,
(81) 1-(2-benzyloxy-4-chlorobenzyl)-5-(2,3-dichlorophenyl)-1H-1,2,3,4-tetrazole,
(82) 1-(benzocyclohepten-1-yl)-5-(2,3-dichlorophenyl)-1H-1,2,3,4-tetrazole,
(83) 1-(2-benzyloxy-5-nitrobenzyl)-5-(2,3-dichlorophenyl)-1H-1,2,3,4-tetrazole,
(84) 1-(2-benzyloxy-5-fluorobenzyl)-5-(2,3-dichlorophenyl)-1H-1,2,3,4-tetrazole,
(85) 1-(2-benzyloxy-5-methylbenzyl)-5-(2,3-dichlorophenyl)-1H-1,2,3,4-tetrazole,
(86) 1-(2-benzyloxy-5-dimethylaminobenzyl)-5-(2,3-dichlorophenyl)-1H-1,2,3,4-tetrazole,
(87) 5-(2-benzyloxybenzyl)-1-(2,3-dichlorophenyl)-1H-1,2,3,4-tetrazole,
(88) 5-(2,3-dichlorophenyl)-1-(2-nitrobenzyl)-1H-1,2,3,4-tetrazole,
(89) 5-(2-benzyloxy-5-chlorophenyl)-1-(2,3-dichlorophenyl)-1H-1,2,3,4-tetrazole,
(90) 1-(2-benzyloxy-5-chlorobenzyl)-5-(2,3-dimethylphenyl)-1H-1,2,3,4-tetrazole,
(91) 5-(2,3-dichlorophenyl)-1-(2,5-dimethylbenzyl)-1H-1,2,3,4-tetrazole,
(92) 1-(2-benzyloxy-5-chlorobenzyl)-5-(2-chlorophenyl)-1H-1,2,3,4-tetrazole,
(93) 1-(2-benzyloxy-5-chlorobenzyl)-5-(2-methylphenyl)-1H-1,2,3,4-tetrazole,
(94) 1-(2-benzylaminobenzyl)-5-(2,3-dichlorophenyl)-1H-1,2,3,4-tetrazole,
(95) 5-(2,3-dichlorophenyl)-1-(2,4-dimethylbenzyl)-1H-1,2,3,4-tetrazole,
(96) 1-(2-benzyloxy-5-chlorobenzyl)-5-(3-methylphenyl)-1H-1,2,3,4-tetrazole,
(97) 1-(2-chloro-6-fluorobenzyl)-5-(2-nitrophenyl)-1H-1,2,3,4-tetrazole,
(98) 1-(4-fluorobenzyl)-5-(4-nitrophenyl)-1H-1,2,3,4-tetrazole,
(99) 1-(4-fluorobenzyl)-5-(2-nitrophenyl)-1H-1,2,3,4-tetrazole,
(100) 5-(2-benzyloxy-3-methoxyphenyl)-1-(4-fluoro-2-trifluoromethylbenzyl)-1H-1,2,3,4-tetrazole,
(101) 5-(2,3 -dichlorophenyl)-1-(2-phenylbenzyl)-1H-1,2,3,4-tetrazole,
(102) 5-(2,4-dichlorophenyl)-1-(2-phenoxybenzyl)-1H-1,2,3,4-tetrazole,
(103) 5-(2,5-dichlorophenyl)-1-(2-phenoxybenzyl)-1H-1,2,3,4-tetrazole,
(104) 5-(2-chloro-5-methoxymethoxyphenyl)-1-(5-chloro-2-benzyloxybenzyl)-1H-1,2,3,4-tetrazole,
(105) 1-(2-benzyloxy-5-chlorobenzyl)-5-phenyl-1H-1,2,3,4-tetrazole,
(106) 1-(2-benzyloxy-5-chlorobenzyl)-5-(3-chlorophenyl)-1H-1,2,3,4-tetrazole,
(107) 1-(5-chloro-2-cyclohexylmethyloxybenzyl)-5-(2,3-dichlorophenyl)-1H-1,2,3,4-tetrazole,
(108) 1-[5-chloro-2-(4-pyridylmethyloxy)benzyl]-5-(2,3-dichlorophenyl)-1H-1,2,3,4-tetrazole,
(109) 1-[2-(2H,3H-benzo[e]1,4-dioxan-2-ylmethyloxy)-5-chlorobenzyl]-5-(2,3-dichlorophenyl)-1H-1,2,3,4-tetrazole,
(110) 1-(2-benzyloxy-5-chlorobenzyl)-5-(2-chloro-3-methylphenyl)-1H-1,2,3,4-tetrazole,
(111) 5-(2,3-chlorophenyl)-1-(1-phenylethyl)-1H-1,2,3,4-tetrazole,
(112) 1-(5-chloro-2-phenethyloxybenzyl)-5-(2,3-dichlorophenyl)-1H-1,2,3,4-tetrazole,
(113) 5-(2,3-dichlorophenyl)-1-(2-cyclohexyloxy-5-chlorobenzyl)-1H-1,2,3,4-tetrazole,
(114) 5-(2-aminophenyl)-1-(2-chloro-6-fluorobenzyl)-1H-1,2,3,4-tetrazole,
(115) 1-(5-amino-2-benzyloxybenzyl)-5-(2,3-dichlorophenyl)-1H-1,2,3,4-tetrazole,
(116) 1-(2-aminobenzyl)-5-(2,3-dichlorophenyl)-1H-1,2,3,4-tetrazole,
(117) 1-(2-chloro-6-fluorobenzyl)-5-(2-hydroxyphenyl)-1H-1,2,3,4-tetrazole,
(118) 1-(2-chloro-6-fluorobenzyl)-5-(2-hydroxymethylphenyl)-1H-1,2,3,4-tetrazole,
(119) 1-(5-chloro-2-hydroxybenzyl)-5-(2,3-dichlorophenyl)-1H-1,2,3,4-tetrazole,
(120) 5-(2-hydroxy-3-methoxyphenyl)-1-(4-fluoro-2-trifluoromethylbenzyl)-1H-1,2,3,4-tetrazole,
(121) 1-(2-chloro-4-hydroxybenzyl)-5-(2,3-dichlorophenyl)-1H-1,2,3,4-tetrazole,
(122) 1-(2-benzyloxy-5-chlorobenzyl)-5-(2-chloro-4-hydroxyphenyl)-1H-1,2,3,4-tetrazole,
(123) 5-(2,6-dimethoxyphenoxy)-1-phenyl-1H-1,2,3,4-tetrazole,
(124) 5-phenoxy-1-phenyl-1H-1,2,3,4-tetrazole,
(125) 1-[5-chloro-2-(2,2-dimethyl-1,3-dioxolan-4-ylmethyloxy)benzyl]-5-(2,3-dichlorophenyl)-1H-1,2,3,4-tetrazole,
(126) 1-[5-chloro-2-(4-dimethylaminobenzyloxy)benzyl]-5-(2,3-dichlorophenyl)-1H-1,2,3,4-tetrazole,
(127) 1-[5-chloro-2-(2-morpholinoethyloxy)benzyl]-5-(2,3-dichlorophenyl)-1H-1,2,3,4-tetrazole,
(128) 1-(2-benzyloxy-5-chlorobenzyl)-5-(2,4-dichlorophenyl)-1H-1,2,3,4-tetrazole,
(129) 1-[5-chloro-2-(3-pyridylmethyloxy)benzyl]-5-(2,3-dichlorophenyl)-1H-1,2,3,4-tetrazolc,
(130) 1-[5-chloro-2-(2-pyridylmethyloxy)benzyl]-5-(2,3-dichlorophenyl)-1H-1,2,3,4-tetrazole,
(131) 1-(2-benzyloxy-5-chlorobenzyl)-5-(2-chloro-3-nitrophenyl)-1H-1,2,3,4-tetrazole,
(132) 1-(2-benzyloxy-5-chlorobenzyl)-5-(2,5-dichlorophenyl)-1H-1,2,3,4-tetrazole,
(133) 1-(2-benzyloxy-5-chlorobenzyl)-5-(2,6-dichlorophenyl)-1H-1,2,3,4-tetrazole,
(134) 1-[5-chloro-2-(N-methyl-2,3,4,5,6,7-hexahydroazepin-3-yl)benzyl]-5-(2,3-dichlorophenyl)-1H-1,2,3,4-tetrazole,
(135) 1-[5-chloro-2-(N-methylpiperidin-2-ylmethyloxy)benzyl]-5-(2,3-dichlorophenyl)-1H-1,2,3,4-tetrazole,
(136) 1-(2-benzyloxy-5-chlorobenzyl)-5-(3-chloro-2-methylphenyl)-1H-1,2,3,4-tetrazole,
(137) 1-[5-chloro-2-(2-methylphenylamino)ethyloxybenzyl]-5-(2,3-dichlorophenyl)-1H-1,2,3,4-tetrazole,
(138) 1-(2-benzyloxy-5-chlorobenzyl)-5-(3-methoxymethoxy-2-methylphenyl)-1H-1,2,3,4-tetrazole,
(139) 1-[5-chloro-2-(tetrahydropyran-2-ylmethyloxy)-benzyl]-5-(2,3-dichlorophenyl)-1H-1,2,3,4-tetrazole,
(140) 1-(2-benzyloxy-5-chlorobenzyl)-5-(4-methoxymethoxyphenyl)-1H-1,2,3,4-tetrazole,
(141) 1-(2-benzyloxy-5-chlorobenzyl)-5-(4-chlorophenyl)-1H-1,2,3,4-tetrazole,
(142) 5-(2,3-dichlorophenyl)1-(2-phenethyloxybenzyl)-1H-1,2,3,4-tetrazole,
(143) 1-(2-cyclohexylmethyloxybenzyl)-5-(2,3-dichlorophenyl)-1H-1,2,3,4-tetrazole,
(144) 5-(2,3-dichlorophenyl)-1-[2-(4-pyridylmethyloxy)benzyl]-1H-1,2,3,4-tetrazole,
(145) 5-(2,3-dichlorophenyl)-1-[2-(3-pyridylmethyloxy)benzyl]-1H-1,2,3,4-tetrazole,
(146) 1-(2,3-dichlorophenyl)-5-[2-(2-morpholinoethyloxy)benzyl]-1H-1,2,3,4-tetrazole,
(147) 1-[2-(2H,3H-benzo[e]1,4-dioxan-2-ylmethyloxy)benzyl]-5-(2,3-dichlorophenyl)-1H-1,2,3,4-tetrazole,
(148) 5-(2,3-dichlorophenyl)-1-[2-(2-morpholinoethyloxy)benzyl]-1H-1,2,3,4-tetrazole,
(149) 5-(2,3-dichlorophenyl)-1-[2-(2-pyridylmethyloxy)benzyl]-1H-1,2,3,4-tetrazole,
(150) 1-(2-chloro-6-phenoxybenzyl)-5-(2,3-dimethylphenyl)-1H-1,2,3,4-tetrazole,
(151) 1-(2-benzyloxybenzyl)-5-(2,3-dimethylphenyl)-1H-1,2,3,4-tetrazole,
(152) 5-(2,3-dichlorophenyl)-1-[2-(2,2-dimethyl-1,3-dioxolan-4-ylmethyloxy)benzyl]-1H-1,2,3,4-tetrazole,
(153) 5-(2,3-dichlorophenyl)-1-[2-[2-(N-methyl-N-phenylamino)ethyloxy]benzyl]-1H-1,2,3,4-tetrazole,
(154) 1-(2-cyclohexyloxybenzyl)-5-(2,3-dichlorophenyl)-1H-1,2,3,4-tetrazole,
(155) 1-(2-benzyloxyphenethyl)-5-(2,3-dimethylphenyl)-1H-1,2,3,4-tetrazole,
(156) 1-(4-benzyloxy-2-chlorobenzyl)-5-(2,3-dichlorophenyl)-1H-1,2,3,4-tetrazole,
(157) 1-[5-chloro-2-(4-methoxymethyloxybenzyloxy)benzyl]-5-(2,3-dichlorophenyl)-1H-1,2,3,4-tetrazole,
(158) 1-(2-benzyloxy-5-chlorobenzyl)-5-(2,3-dihydrobenzofuran-7-yl)-1H-1,2,3,4-tetrazole,
(159) 5-(2,3-dichlorophenyl)-1-(2-hydroxybenzyl)-1H-1,2,3,4-tetrazole,
(160) 1-(2,3-dichlorophenyl)-5-(2-hydroxybenzyl)-1H-1,2,3,4-tetrazole,
(161) 1-(2-benzyloxy-5-chlorobenzyl)-5-(3-hydroxy-2-methylphenyl)-1H-1,2,3,4-tetrazole,
(162) 1-(2-benzyloxy-5-chlorobenzyl)-5-(4-hydroxyphenyl)-1H-1,2,3,4-tetrazole, and
(163) 5-(3-amino-2-chlorophenyl)-1-(2-benzyloxy-5-chlorobenzyl)-1H-1,2,3,4-tetrazole, or a non-toxic salt thereof.

18. The mitochondrial benzodiazepine receptor antagonist according to claim 6, wherein the compound of formula (I-a') is selected from
(1) N-(2-chlorobenzyl)-2-methoxybenzamide,
(2) N-(2-chloro-6-fluorobenzyl)-2-trifluoromethylbenzamide,
(3) N-(2-chloro-6-fluorobenzyl)-N-methyl-2-chlorobenzamide,
(4) N-benzyl-N-(2-chloro-6-fluorobenzyl)-2-chlorobenzamide,
(5) N-(2-methylbenzyl)-2,6-dichlorobenzamide,
(6) N-(4-fluorobenzyl)-2-chloro-4-fluorobenzamide,
(7) N-(4-fluorobenzyl)-2,3-dichlorobenzamide,
(8) 2,5-dichlorophenyl-N-(4-fluorobenzyl)carboxamide,
(9) N-benzyl-2,5-dichlorobenzamide,
(10) 2-(4-fluorobenzylcarbamoyl)benzoic acid,
(11) 2-(2-methylbenzylcarbamoyl)benzoic acid,
(12) N-(2-methylbenzyl)-2-benzyloxybenzamide,
(13) N-(2-phenylbenzyl)-2,6-dichlorobenzamide,
(14) N-(2-chloro-6-fluorobenzyl)-2-benzyloxybenzamide,
(15) N-(1,2,3,4-tetrahydronaphthalen-1-yl)-2, 6-dichlorobenzamide,
(16) N-(4-fluoro-2-trifluoromethylbenzyl)-2,5-dichlorobenzamide,
(17) N-(4-fluoro-2-trifluoromethylbenzyl)-2,3-dichlorobenzamide,
(18) N-(2-chloro-4-fluorobenzyl)-2,5-dichlorobenzamide,
(19) N-(2-chloro-4-fluorobenzyl)-2,3-dichlorobenzamide,
(20) N-(2,5-dichlorobenzyl)-2,5-dichlorobenzamide,
(21) N-(2,5-difluorobenzyl)-2,5-dichlorobenzamide,
(22) N-(2,5-dichlorobenzyl)-2-chlorobenzamide,
(23) N-(2,5-difluorobenzyl)-2-chlorobenzamide,
(24) N-(4-fluoro-2-trifluoromethylbenzyl)-2-chlorobenzamide,
(25) N-(2-chloro-4-fluorobenzyl)-2-chlorobenzamide,
(26) N-(4-fluoro-2-trifluoromethylbenzyl)-5-fluoro-2-trifluoromethylbenzamide,
(27) N-(2-chloro-4-fluorobenzyl)-5-fluoro-2-trifluoromethylbenzamide,
(28) N-(3,5-dimethoxybenzyl)-2-chlorobenzamide,
(29) N-phenethyl-2-chlorobenzamide,
(30) N-(1-phenylethyl)-2-chlorobenzamide,
(31) N-(indan-1-yl)-2-chlorobenzamide,
(32) N-(2-methylbenzyl)-2-chlorobenzamide,
(33) N-(2,6-difluorobenzyl)-2-chlorobenzamide,
(34) N-(2-chloro-6-fluorobenzyl)-2-chlorobenzamide,
(35) N-(2-chloro-6-fluorobenzyl)-2-methylbenzamide,
(36) N-(2,6-dimethylbenzyl)-2-chlorobenzamide,
(37) N-(2-chloro-6-fluorobenzyl)-2-methoxybenzamide,
(38) N-(2-chloro-6-fluorobenzyl)-2-furylcarboxamide,
(39) N-(2,4-dichlorobenzyl)-2-chlorobenzamide,
(40) N-(3,4-dichlorobenzyl)-2-chlorobenzamide,
(41) N-(4-trifluoromethylbenzyl)-2-chlorobenzamide,
(42) N-(2-chloro-6-fluorobenzyl)phenylacetamide,
(43) N-(4-chlorobenzyl)-2-chlorobenzamide,
(44) N-(1-naphthyl)-2-chlorobenzamide,
(45) N-(2-chloro-6-fluorobenzyl)-2,6-dimethoxybenzamide,
(46) N-(2-chloro-6-fluorobenzyl)-2,6-dichlorobenzamide,
(47) N-(2-chloro-6-fluorobenzyl)-2-nitrobenzamide,
(48) N-(2-chloro-6-fluorobenzyl)-2-benzoyloxymethylbenzamide,
(49) N-(2-chloro-6-fluorobenzyl)-2-dimethylaminobenzamide,
(50) N-(2-chloro-6-fluorobenzyl)-2-(4-methoxyphenoxymethyl)benzamide,
(51) N-(2-chloro-6-fluorobenzyl)-2,6-dimethylbenzamide,
(52) N-(2-chloro-6-fluorobenzyl)-1-naphthylcarboxamide,
(53) 2-(2,6-dichlorobenzoyl)-1,2,3,4-tetrahydroisoquinoline,
(54) N-(2-chloro-6-fluorobenzyl)-2,3-dichlorobenzamide,
(55) N-(2-chloro-6-fluorobenzyl)-2,4-dichlorobenzamide,
(56) N-(2-methoxybenzyl)-2,6-dichlorobenzamide,
(57) N-(4-methylbenzyl)-2,6-dichlorobenzamide,
(58) N-(2-trifluoromethylbenzyl)-2,6-dichlorobenzamide,
(59) N-(2-methoxybenzyl)-2-chloro-6-fluorobenzamide,
(60) N-(2-methoxybenzyl)-2,6-difluorobenzamide,
(61) N-(3-methoxybenzyl)-2,6-dichlorobenzamide,
(62) N-(4-methoxybenzyl)-2,6-dichlorobenzamide,
(63) N-(2-chloro-6-fluorobenzyl)-2,5-dichlorobenzamide,
(64) N-(4-fluorobenzyl)-2,6-dichlorobenzamide,
(65) N-(2-chloro-6-fluorobenzyl)-2,4,6-trichlorobenzamide,
(66) N-(2-chloro-6-fluorobenzyl)-4-fluorobenzamide,
(67) N-(4-fluorobenzyl)-4-fluorobenzamide,
(68) N-(2-chloro-6-fluorobenzyl)cyclohexylcarboxamide,
(69) N-(2-chloro-6-fluorobenzyl)cyclopentylcarboxamide,
(70) N-cyclohexylmethyl-2,3-dichlorobenzamide,
(71) N-(2-furylmethyl)-2,3-dichlorobenzamide,
(72) N-(1-adamantylmethyl)-2,3-dichlorobenzamide,
(73) N-((2R)-2-norbornyl)-2,3-dichlorobenzamide,
(74) N-(2-chloro-6-phenoxybenzyl)-2,3-dichlorobenzamide,
(75) N-(2-chloro-6-fluorobenzyl)-2-naphthylcarboxamide,
(76) N-(4-fluorobenzyl)-2,4-dichlorobenzamide,
(77) N-(4-fluorobenzyl)-2-chloro-4-nitrobenzamide,
(78) N-(4-fluorobenzyl)-4-cyanobenzamide,
(79) N-(4-fluorobenzyl)-4-methylbenzamide,
(80) N-(4-fluorobenzyl)-4-chlorobenzamide,
(81) N-(4-fluorobenzyl)-4-nitrobenzamide,
(82) N-(4-fluorobenzyl)-2-nitrobenzamide,
(83) N-(4-fluorobenzyl)-1-thienylcarboxamide,
(84) N-(4-fluorobenzyl)-[2-(3-chlorothienyl)]carboxamide,
(85) N-(4-fluorobenzyl)-3-thienylcarboxamide,
(86) N-(2-phenoxybenzyl)-2,3-dichlorobenzamide,
(87) N-(2-phenoxybenzyl)-2,4-dichlorobenzamide,
(88) N-(2-phenoxybenzyl)-2,5-dichlorobenzamide,
(89) N-(4-fluoro-2-trifluoromethylbenzyl)-2-trifluoromethylbenzamide,
(90) N-(2-chloro-4-fluorobenzyl)-2-trifluoromethylbenzamide,
(91) N-(4-fluoro-2-trifluoromethylbenzyl)-2-benzyloxy-3-methoxybenzamide,
(92) N-(2,4-dichlorobenzyl)-2,3-dichlorobenzamide,
(93) N-(2,5-dichlorobenzyl)-2,3-dichlorobenzamide,
(94) N-(2-benzyloxybenzyl)-2,3-dichlorobenzamide,
(95) N-(2-benzyloxy-5-chlorobenzyl)-2,3-dichlorobenzamide,
(96) N-(2,3-dichlorobenzyl)-2,3-dichlorobenzamide,
(97) N-(2,3-dichlorophenyl)-2,5-dichlorophenylacetamide,
(98) N-(2-benzylbenzyl)-2,3-dichlorobenzamide,
(99) N-(2,3-dichlorobenzyl)-2,3-dichlorophenylacetamide,
(100) N-(2,3-dichlorophenyl)-2,3-dichlorophenylacetamide,
(101) N-(2-phenylbenzyl)-2,3-dichlorobenzamide,
(102) N-(2-phenoxymethylbenzyl)-2,3-dichlorobenzamide,
(103) N-(2-phenethylbenzyl)-2,3-dichlorobenzamide,
(104) N-(2-phenylaminobenzyl)-2,3-dichlorobenzamide,
(105) N-(2-benzylaminobenzyl)-2,3-dichlorobenzamide,
(106) N-(2-chloro-6-methoxybenzyl)-2,3-dichlorobenzamide,
(107) N-[2-(N-methyl-N-phenylamino)benzyl]-2,3-dichlorobenzamide,
(108) N-(2-benzyloxy-4-chlorobenzyl)-2,3-dichlorobenzamide,
(109) N-(2-benzyloxy-6-chlorobenzyl)-2,3-dichlorobenzamide,
(110) N-(benzocycloheptan-1-yl)-2,3-dichlorobenzamide,
(111) N-(2-benzyloxy-5-fluorobenzyl)-2,3-dichlorobenzamide,
(112) N-(2-benzyloxy-5-methylbenzyl)-2,3-dichlorobenzamide,
(113) N-(2-benzyloxy-5-nitrobenzyl)-2,3-dichlorobenzamide,
(114) N-(2-benzyloxy-5-methoxybenzyl)-2,3-dichlorobenzamide,
(115) N-(2,3-dichlorophenyl)-2-benzyloxyphenylacetamide,
(116) N-(2,3-dichlorophenyl)-2-benzyloxy-5-chlorophenylacetamide,
(117) N-(5-chloro-2-methylbenzyl)-2,3-dichlorobenzamide,
(118) N-(2,4-dimethylbenzyl)-2,3-dichlorobenzamide,
(119) N-(2-benzyloxy-5-chlorobenzyl)benzamide,
(120) N-(2-benzyloxy-5-chlorobenzyl)-2-chlorobenzamide,
(121) N-(2-benzyloxy-5-chlorobenzyl)-2-methylbenzamide,
(122) N-(2-benzyloxy-5-chlorobenzyl)-3-methylbenzamide,
(123) N-(2-benzyloxy-5-chlorobenzyl)-2,3-dimethylbenzamide,
(124) N-(2-nitrobenzyl)-2,3-dichlorobenzamide,
(125) N-(2,5-dimethylbenzyl)-2,3-dichlorobenzamide,
(126) N-(2-benzyloxy-5-chlorobenzyl)-3-chlorobenzamide,
(127) N-(2-benzyloxy-5-chlorobenzyl)-2-chloro-4-methoxymethoxybenzamide,
(128) N-(4-benzyloxy-2-chlorobenzyl)-2,3-dichlorobenzamide,
(129) N-(2-chloro-6-fluorobenzyl)-2-hydroxymethylbenzamide,
(130) N-(2-chloro-6-hydroxybenzyl)-2,3-dichlorobenzamide,
(131) N-(4-fluoro-2-trifluoromethylbenzyl)-2-hydroxy-3-methoxybenzamide,
(132) N-(2-benzyloxy-5-chlorobenzyl)-2-chloro-3-methylbenzamide,
(133) N-(2-benzyloxy-5-chlorobenzyl)-2,4-dichlorobenzamide,
(134) N-(2-benzyloxy-5-chlorobenzyl)-2,5-dichlorobenzamide,
(135) N-(2-benzyloxy-5-chlorobenzyl)-2,6-dichlorobenzamide,
(136) 2,3-dichloro-N-(1-phenylethyl)benzamide,
(137) N-(2-benzyloxy-5-chlorobenzyl)-2-chloro-3-nitrobenzamide,
(138) N-(2-benzyloxy-5-chlorobenzyl)-2-methyl-3-nitrobenzamide,
(139) N-(2-benzyloxy-5-chlorobenzyl)-2,3-dihydrobenzofuran-7-ylcarbamide,
(140) N-(2-benzyloxy-5-chlorobenzyl)-3-chloro-2-methylbenzamide,
(141) N-(2-benzyloxy-5-chlorobenzyl)-3-methoxymethyloxy-2-methylbenzamide,
(142) N-(2-benzyloxy-5-chlorobenzyl)-4-methoxymethoxy-2-methylbenzamide,
(143) 2,3-dichloro-N-(2-phenethyloxybenzyl)benzamide,
(144) N-(2-benzyloxy-5-chlorobenzyl)-4-chlorobenzamide,
(145) N-(2-cyclohexylmethyloxybenzyl)-2,3-dichlorobenzamide,
(146) N-(2-cyclohexyloxybenzyl)-2,3-dichlorobenzamide,
(147) 2,3-dichloro-N-[2-(2-morpholinoethyloxy)benzyl]benzamide,
(148) N-(2-chloro-6-phenoxybenzyl)-2,3-dimethylbenzamide,
(149) N-(2-benzyloxybenzyl)-2,3-dimethylbenzamide,
(150) N-(2-benzyloxyphenethyl)-2,3-dimethylbenzamide,
(151) N-[2-(2H,3H-benzo[e]1,4-dioxan-2-ylmethyloxy)benzyl]-2,3-dichlorophenylbenzamide,
(152) 2,3-dichloro-N-[2-(2,2-dimethyl-1,3-dioxolan-4-ylmethyloxy)benzyl]benzamide,
(153) 2,3-dichloro-N-[2-[2-(N-methyl-N-phenylamino)ethyloxy]benzyl]benzamide, and
(154) 2,3-dichloro-N-(2-hydroxybenzyl)benzamide, or
a non-toxic salt thereof.

19. The pharmaceutical composition for the treatment and/or prophylaxis according to claim 1, wherein the disease induced, exacerbated or reignited by stressors is selected from digestive system disease, circulatory system disease, endocrine system or metabolic system disease, respiratory system disease, nervous system or muscular system disease, cutaneous disease, surgical disease, orthopaedic disease, urinary or genital system disease, gynecological disease, ophthalmological disease, otorhinolaryngological disease, dental or oral surgeory disease and cancer.

20. The pharmaceutical composition according to claim 19, wherein the digestive system disease is selected from functional digestive disturbance, gastric or duodenal ulcer, ulcerative colitis, irritable bowel syndrome, biliary dyskinesia, esophageal spasm, gastric atony, aerophagia, chronic hepatitis and chronic pancreatitis.

21. The pharmaceutical composition according to claim 20, wherein the digestive system diseases is irritable bowel syndrome.
